(19) Europäisches Patentamt
European Patent Office
Office européen des brevets

(11) Publication number: **0 003 663**
**A2**

# (12) EUROPEAN PATENT APPLICATION

(21) Application number: 79300167.8

(22) Date of filing: 02.02.79

(51) Int. Cl.[2]: **C 07 C 101/56**, C 07 C 101/44, C 07 C 103/00, C 07 C 95/08, C 07 C 97/10, C 07 D 307/68, C 07 D 333/38, C 07 D 207/34, A 61 K 31/00

(30) Priority: **02.02.78 US 874433**
**27.02.78 US 881456**
**27.02.78 US 881457**
**08.03.78 US 884673**
**09.03.78 US 884941**
**20.03.78 US 888268**
**20.03.78 US 890569**
**12.04.78 US 895574**

(43) Date of publication of application: **22.08.79**
**Bulletin 79/17**

(84) Designated Contracting States: **BE CH DE FR GB IT NL SE**

(71) Applicant: **AMERICAN CYANAMID COMPANY, Berdan Avenue, Wayne New Jersey 06904 (US)**

(72) Inventor: **Shepherd, Robert Gordon, 3-LD Salisbury Manor, South Nyack New York (US)**

(74) Representative: **Allam, Peter Clerk et al, TREGEAR, THIEMANN & BLEACH Enterprise House Isambard Brunel Road, Portsmouth, Hants PO1 2AN (GB)**

(54) **Amino-substituted phenyl and heteroaryl compounds, process for their preparation and pharmaceutical compositions containing them.**

(57) The invention provides novel amino-substituted phenyl and heteroaryl compounds which show pharmaceutically useful properties, particularly as hypolipidemic and anti-atherosclerotic agents, and processes for the preparation of the novel compounds. The novel compounds have the formula:

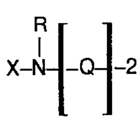

(I)

Q is

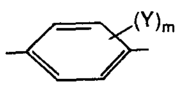

wherein Y is loweralkyl, loweralkoxy, hydroxy, nitro, acyloxy or halo, and m is an integer of from 0 to 4 inclusive, or a 5- or 6-membered heteroaryl ring containing 1 or more nitrogen, sulfur or oxygen atoms and which may be optionally substituted with one fluorine atom; R is hydrogen or a group convertible in vivo thereinto; Z is:

a) 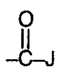 wherein J is selected from the group consisting of hydroxy, loweralkoxy, loweralkoxyloweralkyloxy, diloweralkylaminoloweralkoxy, hydrogen, loweralkyl, (mono- or polyhydroxy)loweralkoxy, (mono- or polycarboxy)loweralkoxy, (mono- or polycarboxy)hydroxyloweralkoxy, allyloxy, 2,3-epoxypropoxy, substituted or unsubstituted(phenoxy, benzyloxy and 3-pyridyloxy), pyridylmethoxy, tetrahydropyranyloxy, (mono- or polyhydroxyl)loweralkylamino, (mono- or polycarboxy)loweralkylamino, (mono- or polycarboalkoxy)loweralkylamino, allylamino, propargylamino, 2-sulfoethylamino, (substituted or unsubstituted aroyl)amino, loweralkanoylamino, loweralkanesulfonylamino, (substituted or unsubstituted arene)sulfonylamino, loweralkanoylhydrazino, hydroxylamino, polymethyleneimino, (4-carboxy- or 4-carboethoxy)thiazolidino, and loweralkyl bearing one or more carboxy, carboloweralkoxy, carbamoyl, acyl, sulfinyl or sulfonyl groups, or

b) 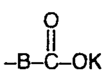 wherein B is a saturated or unsaturated loweralkylene group and K is selected from the group consisting of hydrogen, loweralkyl, loweralkoxyethyl, diloweralkyl aminoethyl, (mono- or polyhydroxy)loweralkyl, (mono- or polycarboxy)loweralkyl, (mono- or polycarboxy)hydroxyloweralkyl, allyl, 2,3-epoxypropyl, substituted or unsubstituted (phenyl, benzyl or 3-pyridyl) or pyridylmethyl and tetrahydropyranyl; and wherein Q, Y, m and X are defined as follows:

Formula I–A) Q is

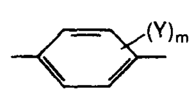

EP 0 003 663 A2

m is O; X is $S_a-T_a-$ wherein Sa is selected from the group consisting of $C_3-C_{16}$ cycloalkyl or $C_4-C_{17}$ cycloalkenyl and is either unsubstituted or substituted with at least one $C_1-C_{13}$ alkyl, $C_4-C_8$ cycloalkyl, decahydronaphthyl, methylene, ethylidene, or isopropylidene group; $T_a$ is a bond or a divalent radical selected from the group consisting of unbranched or branched $C_1-C_{13}$ alkylene or alkenylene and is either unsubstituted or substituted with at least one $C_1-C_4$ alkyl group; with the proviso that the total number of carbon atoms in $S_a$ and $T_a$ shall not exceed twenty; and with the further proviso that when $T_a$ is not a bond, $S_a$ is not an unsubstituted cyclopropyl nor a cyclopropyl substituted with at least one $C_1-C_{13}$ alkyl;

Formula I–B) Q is 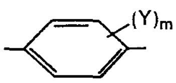

m is O; X is $S_b-$ wherein Sb is a branched or unbranched mono- or polyunsaturated or cyclopropylated $C_3-C_{22}$ alkyl group and can be represented by the formula:

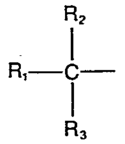

or

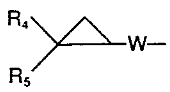

wherein $R_2$ and $R_3$ are the same or different and are hydrogen or a saturated or unsaturated $C_1-C_9$ alkyl group; $R_1$ is a:

Formula I–$B_a$) $C_2$ to $C_{21}$ E- or Z-alkenyl group unsubstituted or substituted with at least one loweralkyl group;

Formula I–$B_b$) $C_2$ to $C_{20}$ alkynyl group unsubstituted or substituted with at least one methyl or ethyl group;

Formula I–$B_c$) $C_4$ to $C_{20}$ alkyl group containing at least 2 non-cumulative double bonds, said group being unsubstituted or substituted with at least one methyl or ethyl group;

Formula I–$B_d$) $C_3$ to $C_{12}$ allenyl group unsubstituted or substituted with at least one methyl or ethyl group;

Formula I–$B_e$) vinyl or $C_3$ to $C_8$ E- or Z-alkenyl group said vinyl or alkenyl group being unsubstituted or substituted with at least one methyl group, and in this Formula I–$B_e$, $R_2$ is a vinyl or $C_3$ to $C_8$ E- or Z-alkenyl group unsubstituted or substituted with at least one methyl group;

Formula I–$B_f$) allyl or $C_4$ to $C_8$ E- or Z-alkenyl group said allyl or alkenyl group being substituted with at least one exo-(methylene, ethylidene, or isopropylidene), and either further unsubstituted or substituted with at least one methyl group;

Formula I–$B_g$) vinyl or $C_3$ to $C_8$ E- or Z-alkenyl group substituted between the nitrogen and the double bond with at least one loweralkenyl, said vinyl or alkenyl group being unsubstituted or substituted with at least one methyl group;

Formula I–$B_h$) vinyl or $C_3$ to $C_8$ E- or Z-alkenyl group said vinyl or alkenyl group being unsubstituted or substituted with at least one methyl and in this case, $R_2$ is a $C_2$ to $C_9$ alkynyl group unsubstituted or substituted with at least one methyl group;

Formula I–$B_i$) $C_4$ to $C_{20}$ alkyl group containing at least one carbon-carbon double bond and at least one carbon-carbon triple bond said group being unsubstituted or substituted with at least one loweralkyl group; or wherein $S_b$ is a;

Formula I–$B_j$) group of the formula 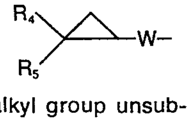

wherein $R_4$ is hydrogen or a $C_1$ to $C_{15}$ alkyl group unsubstituted or substituted with at least one methyl group, $R_5$ is hydrogen or methyl and W is a bond or a $C_1$ to $C_{15}$ branched or unbranched alkylene group unsubstituted or substituted with at least one methyl group;

Formula I–C) Q is 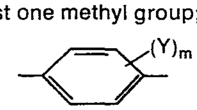

m is O; X is $S_c-T_c-$ wherein Sc is selected from the group consisting of mercapto, lowerakylthio, aralkylthio, arylthio, loweralkylsulinyl, loweralkylsulfonyl, aralkylsulfinyl, aralkylsulfonyl, arylsulfinyl, arylsulfonyl, arylamino, acylamino, loweralkylsulfonylamino, arylsulfonylamino, azido, oxo, oximino, chloro, bromo, and iodo; wherein the substituent $S_c$ may contain q carbon atoms with q being an integer from 0 to 8, inclusive; $T_c$ is an unbranched or branched alkylene group, optionally saturated or (mono- or poly-)unsaturated and containing or not containing a cycloalkyl group, represented by the formula $C_nH_{2(n-p)}$ with n being an integer from 7 to 18, inclusive, and p being an integer from 0 to 5, inclusive, both being selected in a manner such that the sum of n and q is greater than 6 but less than 19; provided that when $S_c$ is halogen $T_c$ must be a saturated alkylene group; J may not be loweralkyl bearing one or more carboxy, carboloweralkoxy, carbamoyl, acyl, sulfinyl or sulfonyl groups, and Z may not be 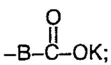;

Formula I–D) Q is 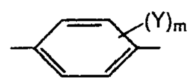

m is O; X is $S_d-T_d-$ wherein Sd is selected from the group consisting of substituted or unsubstituted aryl and heteroaryl groups; $T_d$ is a saturated or unsaturated alkylene group of 1–18 carbon atoms which may be branched or unbranched; such that the total number of carbon atoms in $S_d$ and $T_d$ shall not exceed 24; provided that J may not be hydroxyl, loweralkoxy, loweralkoxyloweralkyloxy, diloweralkylaminoloweralkoxy, or substituted or unsubstituted benzyloxy;

Formula I–E) Q is 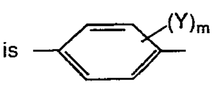

m is 1 to 4 inclusive; X is a saturated or unsaturated hydrocarbon radical of 7–19 carbon atoms which may be branched or unbranched and which may contain one or more radicals selected from the group consisting of saturated or unsaturated cycloalkyl, substituted or unsubstituted aryl and substituted or unsubstituted heteroaryl; Y is loweralkyl, loweralkoxy, hydroxy, nitro, acyloxy, or halo;

Formula I–F) Q is a 5- or 6-membered heteroaryl ring containing one or more nitrogen, sulfur, or oxygen atoms and optionally unsubstituted or substituted with one fluoro; X is $S_f-T_f-$ wherein $S_f$ is selected from the group consisting of hydrogen, $C_3-C_8$ cycloalkyl, and substituted or unsubstituted aryl; $T_f$ is a divalent radical selected from the group consisting of branched or unbranched $C_6-C_{19}$ alkylene, alkenylene, and alkynylene;

Formula I–G) Q is 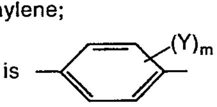

m is O; X is $S_g-T_g-$ wherein Sg is selected from the group consisting of carboxy, loweralkoxycarbonyl, aryloxycarbonyl, heteroaryloxycarbonyl, carbamyl, mono- or diloweralkylcarbamyl, cyano, sulfo, monoalkyl- or monoarylsulfamyl; $T_g$ is an unbranched or branched alkylene group, optionally saturated or mono- or polyunsaturated, and containing or not containing a cycloalkyl group, represented by the formula $C_rH_{2(r-p)}$ with r being an integer from 1 to 18, inclusive, and p being an integer from 0 to 5, inclusive; with the proviso that when $S_g$ is a carbamyl, cyano, or sulfo group, $S_g$ and the nitrogen atom in Formula I may not be bonded to the same carbon atom of the group $T_g$;

Formula I–H) Q is 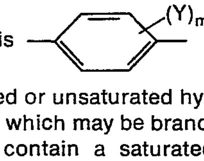

m is O; X is a saturated or unsaturated hydrocarbon radical of 7–19 carbon atoms which may be branched or unbranched and which may contain a saturated or unsaturated cycloalkyl group, said radical containing one or more perfluorinated ($-CF_2$ or $-CF_3$) carbon atoms excluding the carbon adjacent to the nitrogen atom;
and the pharmacologically acceptable acid-addition and cationic salts thereof.

0003663

TITLE MODIFIED
see front page

TITLE: NOVEL AMINO-SUBSTITUTED PHENYL AND HETEROARYL COMPOUNDS AND PHARMACEUTICAL COMPOSITIONS CONTAINING THEM.

This invention relates to new organic compounds, to processes for their preparation, and to pharmaceutical compositions comprising the novel compounds.

More particularly, the invention relates to new organic compounds of the following general formula:

$$X-\overset{R}{\overset{|}{N}}-\left[\;Q\;\right]-Z \qquad \ldots\ I$$

Q is 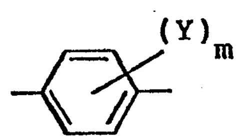

wherein Y is loweralkyl, loweralkoxy, hydroxy nitro, acyloxy or halo, and m is an integer of from 0 to 4 inclusive; or a 5- or 6-membered heteroaryl ring containing 1 or more nitrogen, sulfur or oxygen atoms and which may be optionally substituted with one fluorine atom;

R is hydrogen or a group convertible *in vivo* thereinto;

Z is:

a) $-\overset{O}{\overset{\|}{C}}-J$ wherein J is selected from the group consisting of hydroxy, loweralkoxy, loweralkoxyloweralkyloxy, diloweralkylaminoloweralkoxy, hydrogen, loweralkyl, (mono- or polyhydroxy)loweralkoxy, (mono- or polycarboxy)loweralkoxy, (mono- or polycarboxy)hydroxyloweralkoxy, allyloxy, 2,3-epoxypropoxy, substitued or unsubstituted(phenoxy, benzyloxy and 3-pyridyloxy), pyridylmethoxy, tetrahydropyranyloxy, (mono- or polyhydroxy)loweralkylamino, (mono- or polycarboxy)loweralkylamino, (mono- or polycarboalkoxy)loweralkylamino, allylamino, propargylamino, 2-sulfoethylamino,

(substituted or unsubstituted aroyl)amino, loweralkanoylamino, loweralkanesulfonylamino, (substituted or unsubstituted arene)sulfonylamino, loweralkanoylhydrazino, hydroxylamino, polymethyleneimino, (4-carboxy- or 4-carboethoxy) thiazolidino, and loweralkyl bearing one or more carboxy, carboloweralkoxy, carbamoyl, acyl, sulfinyl or sulfonyl groups, or

b) $-B-\overset{O}{\overset{\|}{C}}-OK$ wherein B is a saturated or unsaturated loweralkylene group and K is selected from the group consisting of hydrogen, loweralkyl, loweralkoxyethyl, diloweralkylaminoethyl, (mono- or polyhydroxy)loweralkyl, (mono- or polycarboxy)loweralkyl, (mono- or polycarboxy)hydroxyloweralkyl, allyl, 2,3-epoxypropyl, substituted or unsubstituted (phenyl, benzyl or 3-pyridyl) or pyridylmethyl and tetrahydropyranyl; and wherein Q, Y, m and X are defined as follows:

Formula I-A) Q is 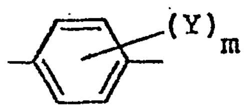 ; m is O; X is $S_a-T_a-$ wherein $S_a$ is selected from the group consisting of $C_3-C_{16}$ cycloalkyl or $C_4-C_{17}$ cycloalkenyl and is either unsubstituted or substituted with at least one $C_1-C_{13}$ alkyl, $C_4-C_8$ cycloalkyl, decahydronaphthyl, methylene, ethylidene, or isopropylidene group; $T_a$ is a bond or a divalent radical selected from the group consisting of unbranched or branched $C_1-C_{13}$ alkylene or alkenylene and is either unsubstituted or substituted with at least one $C_1-C_4$ alkyl group; with the proviso that the total number of carbon atoms in $S_a$ and $T_a$ shall not exceed twenty; and with the further proviso that when $T_a$ is not a bond, $S_a$ is not an unsubstituted cyclopropyl nor a cyclopropyl substituted with at least one $C_1-C_{13}$ alkyl;

Formula I-B) Q is 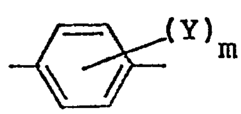 ; m is O; X is $S_b-$ wherein $S_b$ is a branched or unbranched mono- or polyunsaturated or cyclopropylated $C_3-C_{22}$ alkyl group and can be represented by the formula:

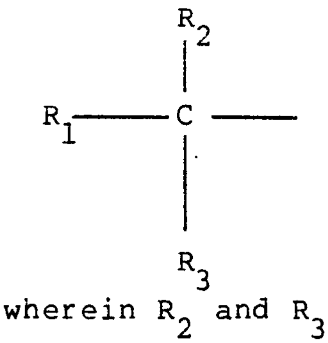

or

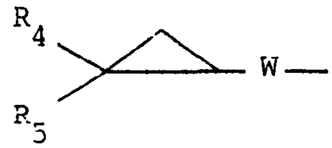

wherein $R_2$ and $R_3$ are the same or different and are hydrogen or a saturated or unsaturated $C_1$-$C_9$ alkyl group; $R_1$ is a·

Formula I-$B_a$) $C_2$ to $C_{21}$ E- or Z-alkenyl group unsubstituted or substituted with at least one loweralkyl group;

Formula I-$B_b$) $C_2$ to $C_{20}$ alkynyl group unsubstituted or substituted with at least one methyl or ethyl group;

Formula I-$B_c$) $C_4$ to $C_{20}$ alkyl group containing at least 2 non-cumulative double bonds, said group being unsubstituted or substituted with at least one methyl or ethyl group;

Formula I-$B_d$) $C_3$ to $C_{12}$ allenyl group unsubstituted or substituted with at least one methyl or ethyl group;

Formula I-$B_e$) vinyl or $C_3$ to $C_8$ E- or Z-alkenyl group said vinyl or alkenyl group being unsubstituted or substituted with at least one methyl group, and in this Formula I-$B_e$, $R_2$ is a vinyl or $C_3$ to $C_8$ E- or Z-alkenyl group unsubstituted or substituted with at least one methyl group;

Formula I-$B_f$) allyl or $C_4$ to $C_8$ E- or Z-alkenyl group said allyl or alkenyl group being substituted with at least one exo-(methylene, ethylidene, or isopropylidene), and either further unsubstituted or substituted with at least one methyl group;

Formula I-$B_g$) vinyl or $C_3$ to $C_8$ E- or Z-alkenyl group substituted between the nitrogen and the double bond with at least one loweralkenyl, said vinyl or alkenyl group

and either further unsubstituted or substituted with at least one methyl group;

Formula I-$B_g$) vinyl or $C_3$ to $C_8$ E- or Z-alkenyl group substituted between the nitrogen and the double bond with at least one loweralkenyl, said vinyl or alkenyl group being unsubstituted or substituted with at least one methyl group;

Formula I-$B_h$) vinyl or $C_3$ to $C_8$ E- or Z-alkenyl group said vinyl or alkenyl group being unsubstituted or substituted with at least one methyl and in this case, $R_2$ is a $C_2$ to $C_9$ alkynyl group unsubstituted or substituted with at least one methyl group;

Formula I-$B_i$) $C_4$ to $C_{20}$ alkyl group containing at least one carbon-carbon double bond and at least one carbon-carbon triple bond said group being unsubstituted or substituted with at least one loweralkyl group;

or wherein $S_b$ is a;

Formula I-$B_j$) group of the formula 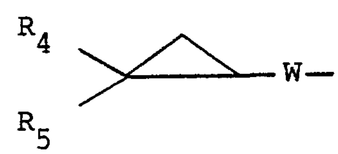

wherein $R_4$ is hydrogen or a $C_1$ to $C_{15}$ alkyl group unsubstituted or substituted with at least one methyl group, $R_5$ is hydrogen or methyl and W is a bond or a $C_1$ to $C_{15}$ branched or unbranched alkylene group unsubstituted or substituted with at least one methyl group;

Formula I-C) Q is 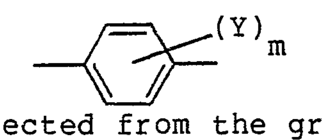 ; m is 0; X is $S_c$-$T_c$- wherein $S_c$ is selected from the group consisting of mercapto, lowerakylthio, aralkylthio, arylthio, loweralkylsulinyl, loweralkylsulfonyl, aralkylsulfinyl, aralkylsulfonyl, arysulfinyl, arylsulfonyl, arylamino, acylamino, loweralkylsulfonylamino, arylsulfonylamino, azido, oxo, oximino, chloro, bromo, and iodo; wherein the substituent $S_c$ may contain q carbon atoms with q being an integer from 0 to 8, inclusive; $T_c$ is an unbranched or branched alkylene group, optionally saturated or (mono- or poly-)unsaturated and containing or not containing a cycloalkyl group, represented by the formula $C_nH_{2(n-p)}$ with n being an integer from 7 to 18, inclusive, and p being an integer from 0 to 5 inclusive, both being selected in a manner such that the sum of n and q is greater than 6 but less than 19; provided that when $S_c$ is halogen $T_c$ must be a saturated alkylene group; J may not be loweralkyl bearing one or more carboxy, carboloweralkoxy, carbamoyl, acyl, sulfinyl or sulfonyl groups, and Z may not be

$-B-\overset{\overset{O}{||}}{C}-OK$ ;

Formula I-D) Q is 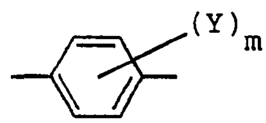 ; m is 0; X is $S_d$-$T_d$- wherein $S_d$ is selected from the group consisting of substituted or unsubstituted aryl and heteroaryl groups; $T_d$ is a saturated or unsaturated alkylene group of 1-18 carbon atoms which may be branched or unbranched; such that the total number of carbon atoms in $S_d$ and $T_d$ shall not exceed 24; provided that J may not be hydroxyl, loweralkoxy, loweralkoxyloweralkyloxy, diloweralkylaminoloweralkoxy, or substituted or unsubstituted benzyloxy;

Formula I-E) Q is 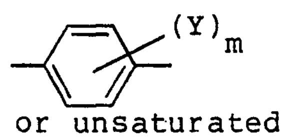 ; m is 1 to 4 inclusive; X is a saturated or unsaturated hydrocarbon radical of 7-19 carbon atoms which may be branched or unbranched and which may contain one or more radicals selected from the group consisting of saturated or unsaturated cycloalkyl, substituted or unsubstituted aryl and substituted or unsubstituted heteroaryl; Y is loweralkyl, loweralkoxy, hydroxy, nitro, acyloxy, or halo;

Formula I-F) Q is a 5- or 6-membered heteroaryl ring containing one or more nitrogen, sulfur, or oxygen atoms and optionally unsubstituted or substituted with one fluoro; X is $S_f$-$T_f$- wherein $S_f$ is selected from the group consisting of hydrogen, $C_3$-$C_8$ cycloalkyl, and substituted or unsubstituted aryl; $T_f$ is a divalent radical selected

from the group consisting of branched or unbranched $C_6$-$C_{19}$ alkylene, alkenylene, and alkynylene;

Formula I-G) Q is 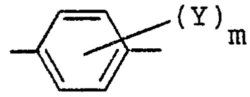 ; m is 0; X is $S_g$-$T_g$- wherein $S_g$ is selected from the group consisting of carboxy, loweralkoxycarbonyl, aryloxycarbonyl, heteroaryloxycarbonyl, carbamyl, mono- or diloweralkylcarbamyl, cyano, sulfo, monoalkyl- or monoarylsulfamyl; $T_g$ is an unbranched or branched alkylene group, optionally saturated or mono- or polyunsaturated, and containing or not containing a cycloalkyl group, represented by the formula $C_rH_{2(r-p)}$ with r being an integer from 1 to 18, inclusive, and p being an integer from 0 to 5, inclusive; with the proviso that when $S_g$ is a carbamyl, cyano, or sulfo group, $S_g$ and the nitrogen atom in Formula I may not be bonded to the same carbon atom of the group $T_g$;

Formula I-H) Q is 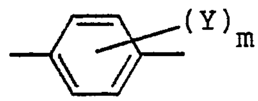 ; m is 0; X is a saturated or unsaturated hydrocarbon radical of 7-19 carbon atoms which may be branched or unbranched and which may contain a saturated or unsaturated cycloalkyl group, said radical containing one or more perfluorinated ($-CF_2-$ or $-CF_3$) carbon atoms excluding the carbon adjacent to the nitrogen atom;
and the pharmacologically acceptable acid-addition and cationic salts thereof.

The invention also relates to processes for preparing the novel compounds and salts of the invention. Thus, one process contemplated by the present invention comprises reacting a compound of the formula:

D-E

wherein D is X as defined above, or a group convertible into X;
E is halogen, alkanesulfonyloxy, arenesulfonyloxy, trifluoromethanesulfonyloxy, or $-\overset{O}{\overset{\|}{C}}-G$ wherein G is hydrogen, halogen, acyloxy, 1-imidazolyl, an activated ester moiety,

or a hydrocarbyl group provided that when G is a hydrocarbyl group the moieties D and G may in addition be directly bonded so that $D-\overset{O}{\overset{\|}{C}}-G$ forms a cyclic ketone, and further provided that $D-\overset{O}{\overset{\|}{C}}-G$ comprises the carbon skeleton of a desired X;
with a compound of the formula:

$$L-Q-M \qquad ....II$$

wherein Q is defined above;
L is -NHR, -NHV, -NXR or -NXV or a group convertible in situ thereinto wherein X and R are as defined above and V is an amine protecting group such as benzyl, trifluoroacetyl, carbo-*t*-butoxy, carbobenzyloxy, trialkylsilyl, and the like;
M is Z which is as defined above or a group convertible to Z; provided that when E is a bromine atom bonded to a saturated carbon atom and L is $-NH_2$ E and L may be interchanged, and further provided that when L is -NXR or -NXV then M is hydrogen and compound II is reacted in the presence of a suitable catalyst such as a Lewis acid, with a compound of the formula $J'-K'$ wherein $J'$ is a suitable leaving group such as halogen, sulfonyloxy, and the like, and $K'$ is Z or a group convertible thereto; and when required either during or after its formation, reducing an amide carbonyl or imino C=N group to produce the -NX group, and in any order or at anytime, before or after reacting compound II with $J'-K'$ or D-E, introducing or removing the protecting group V, or converting a specified M or X group to any other M or X; and/or forming the pharmaceutically acceptable acid addition or cationic salt thereof.

The invention also relates to the acid addition and cationic salts of the novel compounds of the invention.

The invention also relates to methods for using the compounds and pharmaceutical compositions and preparations of the invention, as hypolipidemic agents

and antiatherosclerotic agents which are useful for preventing or diminishing atherosclerotic lesion formation in mammals.

The invention also pertains to novel compositions of matter useful as antiatherosclerotic agents and to methods of ameliorating atherosclerosis by counteracting hyperlipemia and arterial plaque formation in mammals therewith; the active ingredients of said compositions of matter being the novel compounds of the present invention, the salts thereof and mixtures thereof. These compounds may be utilized either as the free bases or in the form of a pharmaceutically acceptable salt with an organic or norganic acid or base. The invention also contemplates a method for lowering serum lipids and for ameliorating atherosclerosis in mammals by the administration of said compositions, compounds, salts and mixtures thereof.

## BACKGROUND OF THE INVENTION

Considerable effort has been directed in recent years to obtain substances useful in counteracting the consequences of hyperlipidemia, a condition involving elevated cholesterol, phospholipid and/or triglyceride levels in the blood, and of hyperlipoproteinemia, involving an imbalance of the lipoproteins. The most serious condition associated with hyperlipidemia and hyperlipoproteinemia is atherosclerosis, a form of arteriosclerosis characterized by lipid accumulation and thickening of the walls of both medium-sized and large arteries such as the aorta. Their walls are thereby weakened and the elasticity and effective internal size of the arteries decreased. Atherosclerosis, the most common cause of coronary artery disease, is of great medical importance since it tends to occlude those arteries supplying blood to the heart muscles and brain, thereby producing permanent damage to these organs. Such damage, may lead to ischemic heart disease, congestive heart failure life-threatening arrhythmias, senility, or stroke. Involvement of leg arteries may lead to gangrene and loss of the limb. It has been known for more than 100 years that cholesterol is a major component of atherosclerotic lesions or plaques. Investigators have been trying to determine the role of cholesterol in lesion initiation and development and also, most importantly, whether lesion formation can be prevented or reversed and enlargement of lesions be slowed or stopped. The earliest lesions are now known to be fatty streaks, largely of chclesterol, which often progress in stages to plaques containing cellular, fibrous and calcified material in addition to the lipids.

The evidence that hyperlipidemia is one of the factors involved in coronary heart disease is very impressive. A most important study carried out in Framingham, Mass. (Gordon & Verter, 1969) in over 5,000 persons for more than 12 years established a correlation between high concentrations of blood cholesterol and increased risk of heart attack. Although the causes of coronary artery disease are

multiple, one of the most constant factors has been the elevated concentration of lipids in the blood plasma. A combined elevation of cholesterol and triglycerides has been shown (Carlson & Bottiger, 1972) to carry the highest risk of coronary heart disease. The majority of patients with ischemic heart disease or peripheral vascular disease were found to have hyperlipoproteinemia, involving very low-density and/or low-density lipoproteins (Lewis et al. 1974).

The reason for most treatment of hyperlipidemia or hyperlipoproteinemia is for arresting, reversing or preventing atherosclerosis. In the past, attempts have been made to lower the levels of cholesterol, phospholipids, and triglycerides in the blood by the oral feeding of various substances which have been generally referred to in the art as hypolipidemic agents or hypocholesteremic adjuvants. Typical of such substances are lecithin, pectin, cottonseed oil, and the mucilaginous substances listed in U.S. Patent No. 3,148,114. In addition, several synthetic hypolipidemic agents are now available, namely, clofibrate, D-thyroxine, cholestyramine and nicotinic acid [(Levy & Frederickson, Postgraduate Medicine 47, 130 (1970)]. Clofibrate has the undesirable side-effect of causing hypertrophy of the liver in some patients.

The development of agents capable of reducing elevated blood lipids and of favorably altering blood-lipoprotein patterns is considered by medical authorities to be extremely important for the treatment and prevention of atherosclerosis. Orally active agents are most desirable since patients usually continue therapy for a number of years.

DETAILED DESCRIPTION OF THE INVENTION

The new compounds of the present invention may be represented by the following general formula:

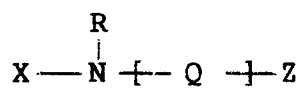

....I

Q is

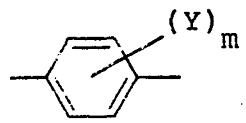

wherein Y is loweralkyl, loweralkoxy, hydroxy, nitro, acyloxy or halo, and m is an integer of from 0 to 4 inclusive, or a 5- or 6-membered heteroaryl ring containing 1 or more nitrogen, sulfur or oxygen atoms and which may be optionally substituted with one fluorine atom;

R is hydrogen or a group convertible in vivo thereinto;

Z is:

a) $-\overset{O}{\overset{\|}{C}}-J$ wherein J is selected from the group consisting of hydroxy, loweralkoxy, loweralkoxyloweralkyloxy, diloweralkylaminoloweralkoxy, hydrogen, loweralkyl, (mono- or polyhydroxy)loweralkoxy, (mono- or polycarboxy)loweralkoxy, (mono- or polycarboxy)hydroxyloweralkoxy, allyloxy, 2,3-epoxypropoxy, substitued or unsubstituted(phenoxy, benzyloxy and 3-pyridyloxy), pyridylmethoxy, tetrahydropyranyloxy, (mono- or polyhydroxy)loweralkylamino, (mono- or polycarboxy)loweralkylamino, (mono- or polycarboalkoxy)loweralkylamino, allylamino, propargylamino, 2-sulfoethylamino, (substituted or unsubstituted aroyl)amino, loweralkanoylamino, loweralkanesulfonylamino, (substituted or unsubstituted arene)sulfonylamino, loweralkanoylhydrazino, hydroxylamino, polymethyleneimino, (4-carboxy- or 4-carboethoxy) thiazolidino, and loweralkyl bearing one or more carboxy,

carboloweralkoxy, carbamoyl, acyl, sulfinyl or sulfonyl groups. or

b) $-B-\overset{O}{\overset{\|}{C}}-OK$ wherein B is a saturated or unsaturated loweralkylene group and K is selected from the group consisting of hydrogen, loweralkyl, loweralkoxyethyl, diloweralkylaminoethyl, (mono- or polyhydroxy)loweralkyl, (mono- or polycarboxy)loweralkyl, (mono- or polycarboxy)hydroxyloweralkyl, allyl, 2,3-epoxypropyl, substituted or unsubstituted (phenyl, benzyl or 3-pyridyl) or pyridylmethyl and tetrahydropyranyl; and wherein Q, Y, m and X are defined as follows:

Formula I-A) Q is 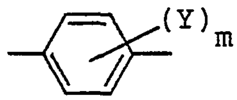 ; m is O; X is $S_a-T_a-$ wherein $S_a$ is selected from the group consisting of $C_3-C_{16}$ cycloalkyl or $C_4-C_{17}$ cycloalkenyl and is either unsubstituted or substituted with at least one $C_1-C_{13}$ alkyl, $C_4-C_8$ cycloalkyl, decahydronaphthyl, methylene, ethylidene, or isopropylidene group; $T_a$ is a bond or a divalent radical selected from the group consisting of unbranched or branched $C_1-C_{13}$ alkylene or alkenylene and is either unsubstituted or substituted with at least one $C_1-C_4$ alkyl group; with the proviso that the total number of carbon atoms in $S_a$ and $T_a$ shall not exceed twenty; and with the further proviso that when $T_a$ is not a bond, $S_a$ is not an unsubstituted cyclopropyl nor a cyclopropyl substituted with at least one $C_1-C_{13}$ alkyl;

Formula I-B) Q is 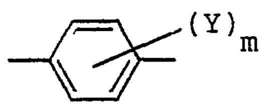 ; m is O; X is $S_b-$ wherein $S_b$ is a branched or unbranched mono- or polyunsaturated or cyclopropylated $C_3-C_{22}$ alkyl group and can be represented by the formula:

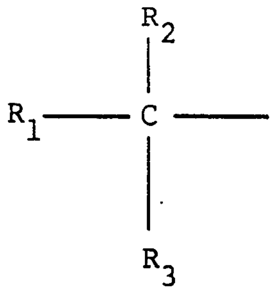

or

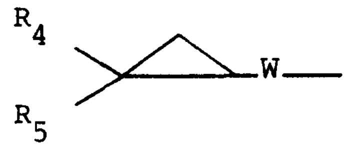

wherein $R_2$ and $R_3$ are the same or different and are hydrogen or a saturated or unsaturated $C_1$-$C_9$ alkyl group; $R_1$ is a:

Formula I-$B_a$) $C_2$ to $C_{21}$ E- or Z-alkenyl group unsubstituted or substituted with at least one loweralkyl group;

Formula I-$B_b$) $C_2$ to $C_{20}$ alkynyl group unsubstituted or substituted with at least one methyl or ethyl group;

Formula I-$B_c$) $C_4$ to $C_{20}$ alkyl group containing at least 2 non-cumulative double bonds, said group being unsubstituted or substituted with at least one methyl or ethyl group;

Formula I-$B_d$) $C_3$ to $C_{12}$ allenyl group unsubstituted or substituted with at least one methyl or ethyl group;

Formula I-$B_e$) vinyl or $C_3$ to $C_8$ E- or Z-alkenyl group said vinyl or alkenyl group being unsubstituted or substituted with at least one methyl group, and in this Formula I-$B_e$, $R_2$ is a vinyl or $C_3$ to $C_8$ E- or Z-alkenyl group unsubstituted or substituted with at least one methyl group;

Formula I-$B_f$) allyl or $C_4$ to $C_8$ E- or Z-alkenyl group said allyl or alkenyl group being substituted with at least one exo-(methylene, ethylidene, or isopropylidene),

and either further unsubstituted or substituted with at least one methyl group;

Formula I-$B_g$) vinyl or $C_3$ to $C_8$ E- or Z-alkenyl group substituted between the nitrogen and the double bond with at least one loweralkenyl, said vinyl or alkenyl group being unsubstituted or substituted with at least one methyl group;

Formula I-$B_h$) vinyl or $C_3$ to $C_8$ E- or Z-alkenyl group said vinyl or alkenyl group being unsubstituted or substituted with at least one methyl and in this case, $R_2$ is a $C_2$ to $C_9$ alkynyl group unsubstituted or substituted with at least one methyl group;

Formula I-$B_i$) $C_4$ to $C_{20}$ alkyl group containing at least one carbon-carbon double bond and at least one carbon-carbon triple bond said group being unsubstituted or substituted with at least one loweralkyl group;

or wherein $S_b$ is a;

Formula I-$B_j$) group of the formula 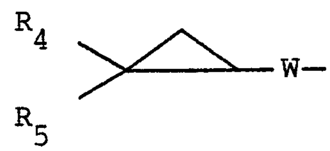

wherein $R_4$ is hydrogen or a $C_1$ to $C_{15}$ alkyl group unsubstituted or substituted with at least one methyl group, $R_5$ is hydrogen or methyl and W is a bond or a $C_1$ to $C_{15}$ branched or unbranched alkylene group unsubstituted or substituted with at least one methyl group;

Formula I-C) Q is 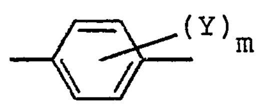 ; m is O; X is $S_c$-$T_c$- wherein $S_c$ is selected from the group consisting of mercapto, lowerakylthio, aralkylthio, arylthio, loweralkylsulinyl, loweralkylsulfonyl, aralkylsulfinyl, aralkylsulfonyl, arysulfinyl, arylsulfonyl, arylamino,

acylamino, loweralkylsulfonylamino, arylsulfonylamino, azido, oxo, oximino, chloro, bromo, and iodo; wherein the substituent $S_c$ may contain q carbon atoms with q being an integer from 0 to 8, inclusive; $T_c$ is an unbranched or branched alkylene group, optionally saturated or (mono- or poly-)unsaturated and containing or not containing a cycloalkyl group, represented by the formula $C_nH_{2(n-p)}$ with n being an integer from 7 to 18, inclusive, and p being an integer from 0 to 5, inclusive, both being selected in a manner such that the sum of n and q is greater than 6 but less than 19; provided that when $S_c$ is halogen $T_c$ must be a saturated alkylene group; J may not be loweralkyl bearing one or more carboxy, carboloweralkoxy, carbamoyl, acyl, sulfinyl or sulfonyl groups, and Z may not be $-B-\overset{O}{\overset{\|}{C}}-OK$;

Formula I-D) Q is 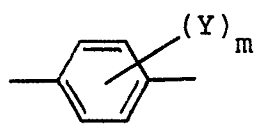 ; m is O; X is $S_d$-$T_d$- wherein $S_d$ is selected from the group consisting of substituted or unsubstituted aryl and heteroaryl groups; $T_d$ is a saturated or unsaturated alkylene group of 1-18 carbon atoms which may be branched or unbranched; such that the total number of carbon atoms in $S_d$ and $T_d$ shall not exceed 24; provided that J may not be hydroxyl, loweralkoxy, loweralkoxyloweralkyloxy, diloweralkylaminoloweralkoxy, or substituted or unsubstituted benzyloxy;

Formula I-E) Q is 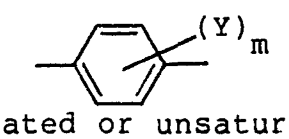 ; m is 1 to 4 inclusive; X is a saturated or unsaturated hydrocarbon radical of 7-19 carbon atoms which may be branched or unbranched and which may contain one or more radicals selected from the group consisting of saturated or unsaturated cycloalkyl, substituted or unsubstituted aryl and substituted or unsubstituted heteroaryl; Y is loweralkyl, loweralkoxy, hydroxy, nitro, acyloxy, or halo;

Formula I-F) Q is a 5- or 6-membered heteroaryl ring containing one or more nitrogen, sulfur, or oxygen atoms and optionally unsubstituted or substituted with one fluoro; X is $S_f$-$T_f$- wherein $S_f$ is selected from the group consisting of hydrogen, $C_3$-$C_8$ cycloalkyl, and substituted or unsubstituted aryl; $T_f$ is a divalent radical selected from the group consisting of branched or unbranched $C_6$-$C_{19}$ alkylene, alkenylene, and alkynylene;

Formula I-G) Q is 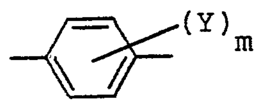 m is O; X is $S_g$-$T_g$- wherein $S_g$ is selected from the group consisting of carboxy, loweralkoxycarbonyl, arylcarbonyl, heteroaryloxycarbonyl, carbamyl, mono- or diloweralkylcarbamyl, cyano, sulfo, monoalkyl- or monoarylsulfamyl; $T_g$ is an unbranched or branched alkylene group, optionally saturated or mono- or polyunsaturated, and containing or not containing a cycloalkyl group, represented by the formula $C_rH_{2(r-p)}$ with r being an integer from 1 to 18, in clusive, and p being an integer from 0 to 5, inclusive; with the proviso that when $S_g$ is a carbamyl, cyano, or sulfo group, $S_g$ and the nitrogen atom in Formula I may not be bonded to the same carbon atom of the group $T_g$;

Formula I-H) Q is 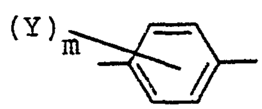 ; m is O; X is a saturated or unsaturated hydrocarbon radical of 7-19 carbon atoms which may be branched or unbranched and which may contain a saturated or unsaturated cycloalkyl group, said radical containing one or more perfluorinated ($-CF_2-$ or $-CF_3$) carbon atoms excluding the carbon adjacent to the nitrogen atom; and the pharmacologically acceptable acid-addition and cationic salts thereof.

The compounds of this invention are new and novel substituted amino compounds and have useful biological and pharmacological properties. No hypolipidemic activity has been reported in the literature for these compounds and they are different in structure from other hypolipidemic agents. The compounds of this invention lower serum lipid concentrations and also minimize atheroma formation in the aorta. These compounds provide the oral administration required of hypolipidemic agents, which patients usually take for many years. The novel compounds of this invention are adequately and reliably absorbed from the gastrointestinal tract with little, if any, gastrointestinal irritation.

It has now been found that certain members of this class of compound can safely and effectively lower both serum sterols and triglycerides in warm-blooded animals. Such actions on serum-lipid components are considered to be very useful in the treatment of atherosclerosis, especially in contrast to available drugs whose action is much more limited. For some time it has been considered desirable to lower serum-lipid levels and to correct lipoprotein imbalance in mammals as a preventive measure against atherosclerosis. The compounds of the present invention do not act by blocking late stages of cholesterol biosynthesis and thus do not produce accumulation of intermediates such a desmosterol, as equally undesirable as cholesterol itself. Compounds with the combination of therapeutically favorable characteristics possessed by those of the present invention can be safely administered to warm-blooded mammals for the treatment of hyperlipidemic and atherosclerotic states found in patients with or prone to heart attacks, to peripheral or cerebral vascular disease, and to stroke.

The novel compounds of the present invention are, in general, white crystalline solids having characteristic melting points and absorption spectra. They are soluble in organic solvents such as lower alkanols, chloroform, toluene, dimeathylformamide, and the like but are generally not very soluble in water.

The novel compounds of the present invention which are organic bases may be converted to their non-toxic acid-addition salts with a variety of pharmaceutically acceptable organic and inorganic salt-forming reagents. Thus, acid-addition salts may be formed by admixture of the organic free base in a natural solvent with one or two equivalents of an acid such as sulfuric, phosphoric, hydrochloric, hydrobromic, trifluroacetic, citric, tartaric, ascorbic, and the like.

The novel compounds of the present invention in their acidic forms or those which contain acidic substituents are converted to their organic or inorganic cationic salts for therapeutic use. The sodium or potassium salts which are formed in solution in the course of hydrolysis of their esters can be isolated as the solid alkali metal salts by cooling. Where it is desirable to purify a compound in the form of the acid, the salt is conveniently formed by treating its solution with exactly one equivalent of base and evaporation of lyophilization. Alkaline earth salts are prepared similarly, often using their acetate salts as a conveniently soluble form. Organic base salts such as those of N-methylglucamine are prepared by dissolving equimolar amounts of the acid and the base in hot ethanol or aqueous alcohols and cooling to crystalize.

Preferred embodiments contemplated by the composition aspect of the invention are as follows:

FORMULA I-A

Compounds of the formula:

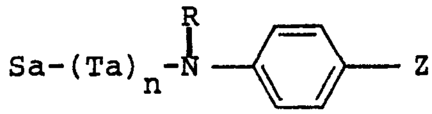

wherein Z is a moiety of the formula:

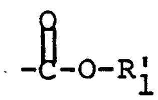

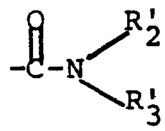

wherein $R_1'$ is selected from the group consisting of hydrogen, $C_1$-$C_6$ alkyl, loweralkoxyloweralkyl, (mono- or polyhydroxy)loweralkyl, allyl, 2,3-epoxypropyl, substituted or unsubstituted-(phenyl, benzyl, or 3-pyridyl), pyridylmethyl, mono- or polycarboxy(loweralkyl or hydroxyloweralkyl), and tetrahydropyranyl;

$R_2'$ is selected from the group consisting of hydrogen, carboxyloweralkyl, carboalkoxyloweralkyl, loweralkanoyl, loweralkanesulfonyl, benzenesulfonyl, sodium sulfo loweralkyl, sulfoloweralkyl, loweralkenyl, loweralkynyl, and phenylloweralkyl;

$R_3'$ is selected from the group consisting of loweralkyl, hydroxy, loweralkoxy, haloloweralkyl, phenyl, carboxyphenyl, chlorophenyl, sodium sulfophenyl, 3-pyridyl, pyridylloweralkyl, (mono- and polyhydroxy)loweralkyl, loweralkoxy loweralkyl, (di-loweralkyl)aminoloweralkyl, piperidino loweralkyl, pyrrolidino loweralkyl, amino, loweralkanoylamino, loweralkanesulfonylamino, N-piperidyl, benzenesulfonylamino, p-toluenesulfonylamino and 4-loweralkylpiperazino; $R_2'$ and $R_3'$ taken together with the associated nitrogen is selected from the group consisting of pyrrolidino, piperidino, morpholino, hexamethyleneimino, loweralkylpiperidino, 4-loweralkyl-1-piperazino, 4-phenylpiperazino, 3-pyrrolinyl, $\Delta^3$-piperidino, 4-(carboethoxy or carboxy)-3-thiazolidinyl, and 4-(carboethoxy)-3-oxazolidinyl;

R is selected from the group consisting of hydrogen, or a group convertible in vivo thereinto, such as methyl, carboxymethyl, acetyl, succinyl, 1-(sodium sulfo)loweralkyl, 1-(sodium sulfo)polyhydroxyalkyl and 1,3-bis-(sodium sulfo) aralkyl

n is either zero or one;

$T_a$ is a divalent radical selected from the group consisting of unbranched or branched $C_1$-$C_{13}$ alkylene or alkenylene and is either unsubstituted or substituted with at least one $C_1$-$C_4$ alkyl group;

and $S_a$ is selected from the group consisting of $C_3$-$C_{16}$ cycloalkyl or $C_4$-$C_{17}$ cycloalkenyl and is either unsubstituted or substituted with at least one $C_1$-$C_{13}$ alkyl, $C_4$-$C_8$ cycloalkyl, decahydronaphthyl, methylene, ethylidene, or isopropylidine group;

with the proviso that the total number of carbon atoms in $S_a$ and $T_a$ shall not exceed twenty; and with the further proviso that when n is 1, $S_a$ is not an unsubstituted cyclopropyl nor a cyclopropyl substituted with at least one $C_1$-$C_{13}$ alkyl;

and the pharmaceutically acceptably non-toxic acid-addition and cationic salts thereof.

More preferred compounds of formula I-A are when n is 1,

Formula I-$A_1$) $T_a$ is a divalent radical selected from the group consisting of branched or unbranched $C_1$-$C_{13}$ alkylene or alkenylene and is either unsubstituted or substituted with at least one $C_1$-$C_4$ alkyl; $S_a$ is a moiety selected from the group consisting of $C_3$-$C_8$ cycloalkyl which is either unsubstituted or substituted with at least one $C_1$-$C_{13}$ alkyl, a $C_5$-$C_7$ cycloalkyl, or a decahydronaphthyl group; with the proviso that D is not an unsubstituted cyclopropyl nor a cyclopropyl substituted with at least one $C_1$ to $C_{13}$ alkyl, or Formula I-$A_2$) $T_a$ is a divalent radical selected from the group consisting of branched or unbranched $C_1$-$C_{13}$ alkylene or alkenylene; and is either unsubstituted or substituted with at least one $C_1$-$C_2$ alkyl;

and $S_a$ is a moiety selected from the group consisting of $C_4$-$C_9$ cycloalkenyl and is either unsubstituted or substituted with at least one $C_1$-$C_{13}$ alkyl group; and $C_5$-$C_8$ cycloalkyl unsubstituted or substituted with at least one methylene moiety, and/or at least one $C_1$-$C_{13}$ alkyl;

and when n is 0,

Formula I-$A_3$) $S_a$ is a moiety selected from the group consisting of $C_4$-$C_7$ cycloalkyl and is either unsubstituted or substituted with at least one $C_4$-$C_7$ cycloalkyl, and decahydronaphthalene unsubstituted or substituted with at least one $C_1$ to $C_4$ alkyl; or

Formula I-$A_4$) $S_a$ is selected from the group consisting of $C_4$-$C_{16}$ cycloalkyl substituted with at least one $C_1$-$C_5$ alkyl; or

Formula I-$A_5$) $S_a$ is a moiety selected from the group consisting of $C_4$-$C_{17}$ cycloalkenyl which is either unsubstituted or substituted with at least one $C_1$-$C_4$ alkyl, and $C_4$-$C_{10}$ cycloalkyl substituted with a moiety selected from the group consisting of methylene, ethylidene, and isopropylidene and/or at least one $C_1$-$C_4$; with the proviso that the sum of the number of carbon atoms contained in Sa and Ta in Formula I-A shall not exceed twenty;

Also, preferred compounds of Formula I-A are those compounds wherein R, $S_a$ and $T_a$ are as previously defined and Z is

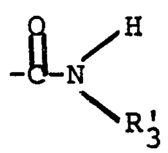 and 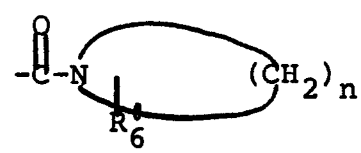

wherein $R_3'$ is a $C_2$-$C_4$ alkyl group unsubstituted or substituted with at least one hydroxyl group, $-CH_2-CH-CH_2$, $-CH_2-C-CH$, $-CH_2CH_2SO_3H$, $-(CH_2)m-COOR_7'$ wherein m is 2-4 and $R_7'$ is hydrogen or a $C_1$-$C_3$ alkyl group, $-\overset{O}{C}-R_8'$ wherein $R_8'$ is phenyl or a $C_1$-$C_3$ alkyl group, $-SO_2R_9'$ wherein $R_9'$ is a $C_1$-$C_3$ alkyl or phenyl or p-tolyl group, -OH, $OCH_3$ and $OCH_2CH_3$, $NHSO_2-R_8'$ wherein $R_8'$ is a previously defined, and wherein n is one of the

integers 4, 5 and 6 and $R_6'$ is hydrogen or represents at least one methyl group.

Preferred compounds of Formulas $I-A_1$ - $I-A_5$ are those wherein Z is the moiety $COOR_1'$ wherein $R_1$ is hydrogen, $C_1-C_6$ alkyl, a $C_1-C_6$ alkyl substituted with one or more carboxyl groups, a $C_1-C_3$ alkyl substituted with at lesat one hydroxyl or carboxyl group, a $C_1-C_3$ alkyl substituted with a dialkylamino moiety or a $C_1-C_4$ dialkylamino moiety and a hydroxyl group, a $C_1-C_3$ alkyl substituted with a polymethyleneimino (ring size 5-8) and a hydroxyl group; a $C_1-C_3$ alkyl substituted with a dialkylamino moiety and a hydroxy group; a benzyl group, a benzyl group optionally substituted with at least one fluorine, chlorine, bromine, iodine, or carboxyl group, a phenyl moiety, a phenyl group optionally substituted with at least one chlorine, bromine, iodine, fluorine or carboxyl group, and 3-pyridyl.

More preferred compounds of the Formula $I-A_1$ are those wherein $T_a$ is a divalent radical selected from those consisting of straight-chain $C_1-C_{13}$ alkylene; and still more preferred are the compounds of Formula $I-A_1$ wherein $S_a$ is a moiety selected from the group consisting of $C_5$ to $C_8$ cycloalkyl. The most preferred compounds of Formula $I-A_1$ are those where $T_a$ is a divalent radical selected from the group consisting of straight chain $C_6$ to $C_8$ alkylene.

More preferred compounds of Formula $I-A_2$ are those where $S_a$ is selected from the group consisting of $C_5-C_8$ cycloalkenyl unsubstituted or substituted with at least one $C_1-C_2$ alkyl and $T_a$ is a divalent radical selected from the group consisting of $C_1-C_{13}$ alkylene; and those compounds wherein Ta is a divalent radical selected from the group consisting of $C_4-C_{13}$ alkylene and/or Sa is $C_5$ or $C_6$ cycloalkyl are even more preferred. Additionally preferred embodiments of compounds of Formula $I-A_2$ are those where $S_a$ is selected from the group

consisting of $C_5$ to $C_8$ cycloalkyl substituted with a methylene moiety and/or at least one $C_1$-$C_2$ alkyl, and $T_a$ is $-CH_2-$ or $-CH(CH_3)-$.

Preferred embodiments of the compounds of Formula I-$A_3$ are those where $S_a$ is selected from the group consisting of $C_5$-$C_6$ cycloalkyl which is either unsubstituted or substituted with at least one $C_5$-$C_6$ cycloalkyl, and decahydronapthyl unsubstituted or substituted with at least one $C_1$-$C_4$ alkyl.

More preferred compounds of Formula I-$A_4$ are those where Sa is selected from the group consisting of $C_4$-$C_{16}$ cycloalkyls which may be unsubstituted or substituted with at least one $C_1$-$C_5$ alkyl and most preferred are those where $S_a$ is selected from the group consisting of $C_5$ to $C_{12}$ cycloalkyl.

More preferred compounds of Formula I-$A_5$ are those where Sa is $C_4$-$C_{17}$ cycloalkenyl or $C_4$-$C_8$ cycloalkenyl substituted with at least one $C_{1-4}$ alkyl group; and even more preferred of these is where $S_a$ is $C_5$-$C_{17}$ cycloalkenyl and more preferred of these is where Sa is $C_6$ to $C_{15}$ cycloalkenyl. Other preferred compounds of Formula I-$A_5$ are those where $S_a$ is $C_4$-$C_{10}$ cycloalkyl substituted with methylene, ethylidene or isopropylidene. Of these the most preferred are those in which Sa is a $C_5$-$C_{10}$ cycloalkyl substituted with a methylene.

FORMULA I-B

Preferred compounds of Formula I-B are those wherein Z is:

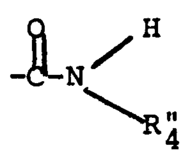 or 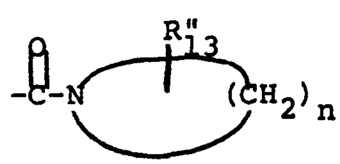

wherein $R''_4$ is a loweralkyl group substituted with at least one hydroxyl group, allyl, propargyl, 2-sulfoethyl, $-(CH_2)_m-COOR''_9$ wherein m is 2-4 and $R''_9$ is hydrogen or a loweralkyl group, $-\overset{O}{\overset{||}{C}}-R''_{12}$ wherein $R''_{12}$ is a loweralkyl or aryl group, $-SO_2R''_{12}$, $-NHCR''_{12}$, or a $-NHSO_2-R''_{12}$

group; n is one of the integers 4, 5 and 6 and $R''_{13}$ is hydrogen or at least one methyl group.

Additionally preferred compounds of Formula I-B are those where Z is the moiety $-COOR'_7$, wherein $R'_7$ is hydrogen; a lower alkyl group unsubstituted or substituted with one or more carboxyl, hydroxyl, diloweralkylamino or polymethyleneimino (ring size 5 - 8 ) groups; a benzyl or phenyl group which is unsubstituted or substituted with at least one halogen or carboxyl group, or 3-pyridyl.

The most preferred compounds of Formula $I-B_a$ are those defined above and those wherein $R_1$ is a vinyl or $C_3$-$C_{21}$ E- or Z- alkenyl group, said vinyl or alkenyl group being unsubstituted or substituted with at least one methyl graup; most preferred compounds of Formula $I-B_a$ are those wherein $R_1$ is a $C_7$ E- or Z- alkenyl group; and the most preferred of these are those in which R, $R_2$ and $R_3$ are hydrogen and J is hydroxy or a loweralkoxy bearing either one or two substituents selected from the group consisting of hydroxy, carboethoxy, diloweralkylamino, and 4-methyl-1-piperazino.

Preferred compounds of Formula $I-B_b$ are those as defined above and those wherein $R_2$ and $R_3$ methyl; even more preferred compounds of Formula $I-B_b$ are those wherein $R_1$ is $C_2$-$C_{18}$ alkenyl and most preferred are those wherein $R_1$ is $C_7$-$C_{18}$ alkenyl and the most preferred of these are those wherein R, $R_2$ and $R_3$ are hydrogen and J is as defined immediately above.

Preferred compounds of Formula $I-B_c$ are those as defined above and those wherein $R_2$ and $R_3$ are

hydrogen or a loweralkyl group and those compounds of Formula I-$B_c$ wherein $R_1$ is $C_4$-$C_{18}$ alkyl group containing at least two non-cumulative double bonds; and most preferred of these are those wherein R, $R_2$ and $R_3$ are hydrogen and J is as defined immediately above.

Preferred compounds of Formula I-$B_d$ are those as defined above and those wherein $R_2$ is methyl; more preferred compounds are those of Formula I-$B_d$ wherein $R_1$ is a $C_3$ to $C_{12}$ allenyl group and the other groups are as defined above; and the most preferred of these are those wherein R, $R_2$ and $R_3$ are hydrogen and J is as defined immediately above.

Preferred compounds of Formula I-$B_e$ are those as defined above and those wherein $R_1$ is a $C_2$ or $C_3$ alkenyl group; and the most preferred of these are those wherein R, $R_2$ and $R_3$ are hydrogen and J is as defined immediately above.

Particularly preferred compounds of Formula I-$B_f$ are those wherein the substituents are as defined immediately above except that R, $R_2$ and $R_3$ are hydrogen and J is as defined immediately above.

Particularly preferred compounds of Formula I-$B_g$ are those wherein R, $R_2$ and $R_3$ are hydrogen and J is as defined immediately above.

Particularly preferred compounds of Formula I-$B_h$ are those where R, $R_2$ and $R_3$ are hydrogen; more preferred compounds are those where $R_1$ is a loweralkenyl or loweralkynyl group; and J is as defined immediately above.

Particularly preferred compounds of Formula I-$B_i$ are those wherein $R_2$ and $R_3$ are the same or different and each is hydrogen or methyl. More preferred compounds of Formula I-$B_i$ are those wherein $R_3$ and/or $R_2$ is hydrogen; and other preferred compounds of Formula I-$B_i$ are those wherein $R_1$ is a $C_4$ to $C_{13}$ alkyl group containing at least one carbon-carbon double bond and at least one carbon-carbon triple bond. Most preferred compounds of Formula I-$B_i$ are those wherein $R_1$ is a $C_6$ to $C_{18}$ alkyl group containing at least one carbon-carbon double bond and at least one carbon-carbon triple bond and J is as defined immediately above.

Particularly preferred compounds of Formula I-$B_j$ are those wherein $R_5$ is hydrogen. Most preferred compounds

of Formula I-$B_j$ are those wherein R and $R_5$ are hydrogen and J is as defined immediately above.

FORMULA I-C

The preferred compounds of Formula I-C may be represented by the following structural formula:

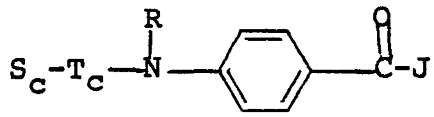

wherein R is hydrogen or is a group convertible *in vivo* thereinto such as methyl, ethyl, carboxymethyl, acetyl, trifluoroacetyl, succinyl, 1-(sodium sulfo)loweralkyl, 1-(sodium sulfo)polyhydroxyalkyl, and 1,3-*bis*(sodium sulfo) aralkyl; $S_C$ is methylthio, *n*-butylthio, benzylthio, *p*-chlorobenzylsufinyl, *p*-chlorophenylamino, phenylamino, acetamido, benzamido, methanesulfonamino, *p*-toluenesulfonamido;

$T_C$ is octamethylene, undecamethylene, tetradecamethylene, hexadecamethylene, 3-methylheptamethylene, 1,1,6-trimethylheptamethylene, 1-(*n*-butyl)pentamethylene, 1-ethyl-1-methylpentamethylene, 2,7-dimethyloctamethylene, 1-(*n*-octyl)decamethylene, 2-undecenylene, 1-(2-butenyl)decamethylene, 1-(1-methyl-2-propenyl)decamethylene, 1-(*n*-hexyl)-3-decenylene, 7-hexadecenylene, 9-hexadecenylene, 1-(*n*-butyl)-3-dodecenylene, 12-hexadecenylene, 3,7,7-trimethyl-2-heptenylene, 1-(*n*-propyl)-2-heptenylene, 2-decenylene, 9-methyl-2-nonenylene, 1-(*n*-pentyl)-2-undecynylene, 1-ethyl-2-tridecynylene, 1-(*n*-hexyl)-4-decynylene, 4-hexadecynylene, 2,6,6-trimethyl-1,3-cyclohexylenemethylene, 2,2,4-trimethyl-1,3-cyclopentylenemethylene; and

J is methoxy, isopropoxy, 2-ethoxyethoxy, 2-dimethylaminoethoxy, 1-methyl-4-piperidyloxy, 4-pyridylmethoxy, 2,3-dihydroxypropoxy, 2-hydroxypropoxy, 3-hydroxypropoxy, 4-chlorobenzyloxy, 3-methylbenzyloxy, 4-fluorophenoxy, 4-sulfophenoxy, 2,6-dichlorophenoxy, 3-carboxyphenoxy, 2,6-dimethyl-3-pyridyloxy, 6-methoxy-3-pyridyloxy, 2-hydroxy-3-pyridyloxy, 5-carboxy-3-pyridyloxy, carboxymethoxy, 1-methoxycarbonylpropoxy, 2-methoxycarbonyl-2-propoxy, 2,3-dihydroxypropylamino, carboxymethylamino, acetylamino, benzoylamino, 4-chlorobenzylamino, methanesulfonylamino, p-toluenesulfonylamino, 1-piperidyl, and the like.

FORMULA I-D

Preferred groups contemplated by the present invention for the aryl or heteroaryl moiety $S_d$ are phenyl, 4-methylphenyl, 3-methylphenyl, 4-decylphenyl, 3,4-dimethylphenyl, 4-methoxyphenyl, 4-hexylphenyl, 4-tridecyloxyphenyl, 4-chlorophenyl, 4-fluorophenyl, 2-chloro-6-fluorophenyl, pentafluorophenyl, 1-naphthyl, 4-biphenylyl, 2-furyl, 2-thienyl, 2-butyl-4-thiazolyl, 2-imidazolyl, 2-undecyl-r-imidazolyl, 5-ethyl-2-furyl, 4-propyl-2-oxazolyl, 5-methyl-2-thienyl, and the like.

Preferred alkylene, alkenylene, and alkynylene groups contemplated for $T_d$ are methylene, ethylene, hexamethylene, undecamethylene, hexadecamethylene, 3-methylheptamethylene, 2,7-dimethyloctamethylene, 9-decenylene, 4-hexenylene, 1-methyl-2-propenylene, 13-pentadecenylene, 18-methyl-18-octadecenylene, 6,8-nonadienylene, 10-undecenylene, 8-nonynylene, 16-heptadecynylene, 3-hexynylene, 1-ethyl-2-tridecynylene, and the like.

Preferred esters contemplated are those in which the group J is methoxy; isopropoxy; 2-ethoxyethoxy; 2-dimethylaminoethoxy; 1-methyl-4-piperidyloxy; 4-pyridylmethoxy; 2,3-dihydroxypropoxy; 2-hydroxypropoxy; 3-hydroxypropoxy; 4-chlorobenzyloxy; 3-methylbenzyloxy; 4-sulfophenoxy; 4-fluorophenoxy; 2,6-dichlorophenoxy; 3-carboxyphenoxy; 2,6-dimethyl-3-pyridyloxy; 6-methoxy-3-pyridyloxy; 2-hydroxy-3-pyridyloxy; 5-carboxy-3-pyridyloxy; 4-cyano-3-pyridyloxy; carboxymethoxy; 1-methoxycarbonylpropoxy; 2-methoxycarbonyl-2-propyl and the like.

Preferred amides contemplated are those in which the group J is 2,3-dihydroxypropylamino; carboxymethylamino, acetylamino, benzoylamino, 4-chlorobenzoylamino; methanesulfonylamino; phenylsulfonylamino, 1-piperidyl, and the like.

Preferred keto-acids and keto-esters contemplated

are those in which the group J is selected from the group consisting of carboxymethyl; carboxyethyl; 2-carboethoxy-2-propyl; dicarboethoxymethyl; carboethoxyvinyl and the like. Preferred alkanoic, alkenoic and alkynoic acids and esters are those in which the radical Z is selected from the group consisting of 4-carboxybutyl; 2-carboethoxyethyl; 2-carboxyvinyl, 2-carboethoxyethynyl, and the like.

FORMULA I-E

The preferred compounds of Formula I-E are ring-fluorinated 4-(disubstituted amino)phenyl compounds wherein the substituent R can be converted *in vivo* to hydrogen. Included among such substituents are, for example, methyl, carboxymethyl, acetyl, succinyl, 1-(sodium sulfo)lower alkyl, 1-(sodium-sulfo)polyhydroxy alkyl, and 1,3-*bis*(sodium sulfo) aralkyl.

Preferred groups contemplated by the present invention for the substituent X are saturated or mono- or polyolefinic or acetylenic such as n-octadecyl; 1-methylundecyl; 1-methylpentadecyl; 1-methylhexadecyl; 1-ethyltetradecyl; 2-methylundecyl; 2-methylpentadecyl; 6-methylhexadecyl; 14-methylpentadaceyl; 15-methylhexadecyl; 3,7,11-trimethyldodecyl; 3,7,11,15-tetramethylhexadecyl; 1-methyl-1-ethyltetradecyl; 1,1-dimethylundecyl; 1,1-dimethyltetradecyl; 13,13-dimethyltetradecyl; 15,15-dimethylhexadecyl; 10-undecenyl; 3,7-dimethyl-6-octenyl; 2,6,10-trimethyl-11-dodecenyl; 1-methyl-6-heptenyl; 1,1-diisopropyl-2-propenyl; 1,3-dimethyl-1-ethyl-2-pentenyl; 4,14-pentadecadienyl; 3,7,11-trimethyl-2,6,10-dodecatrienyl; 11-hexadecynyl; 6-methyl-6-hepten-2-ynyl; 1-isopropyl-1-methyl-2-heptynyl; 3-(1,3-dimethylcyclohexyl)-2-propyl; 13-cyclopentyltridecyl; 11-cyclohexylundecyl; cyclotetradecyl; 4-(1-cyclohexenyl)butyl; 4-cyclohexyl-2-butenyl; 11-phenylundecyl; 4-chlorobenzyl; 3-(4-fluorophenyl) propyl; 6-(4-methoxyphenyl)-hexyl; 4-(decyloxy)benzyl; 4-methylbenzyl; 3-(3-trifluoromethylphenyl)propyl; 3-fluoro-4-methylbenzyl; 3-(4-chloro-2-methoxyphenyl)propyl; cinnamyl; 4-(2-thienyl)butyl; 6-(2-furyl)hexyl; 3-(5-methyl-2-furyl)

propyl; 3-(2,4-dichlorophenyl)propyl; 3-(4-benzyloxyphenyl)propyl; 2-(1-naphthyl)ethyl and the like.

Various mono- and poly-substituted 4-(monosubstituted amino)phenyl compounds are contemplated by the present invention. These include phenyl compounds in which the group $(Y)_m$ represents 2-methyl; 3-methyl; 3,5-dimethyl; 2-chloro; 3-chloro; 3,5-dichloro; 3,5-dibromo; 3,5-diiodo; 2-hydroxy; 3-hydroxy; 2-acetoxy; 2-methoxy; 3,5-dimethoxy; 3-bromo; 3-nitro, 2-fluoro; 3-fluoro; 2,5-difluoro; 2,6-difluoro; 3,5-difluoro; 2,3,5-trifluoro; 2,3,6-trifluoro; or 2,3,5,6-tetrafluoro and the like.

Preferred esters contemplated by the present invention are those in which the group J is methoxy; isopropoxy; 2-ethoxy-ethoxy; 2-dimethylaminoethoxy; 1-methyl-4-piperidyloxy; 4-pyridylmethoxy; 2,3-dihydroxypropoxy; 2-hydroxypropoxy; 3-hydroxypropoxy; 4-chlorobenzyloxy; 3-methylbenzyloxy; 4-sulfophenoxy; 4-fluorophenoxy; 2,6-dichlorophenoxy; 3-carboxyphenoxy; 2,6-dimethyl-3-pyridyloxy; 6-methoxy-3-pyridyloxy; 2-hydroxy-3-pyridyloxy; 4-carboxy-3-pyridyloxy; 4-cyano-3-pyridyloxy; carboxymethoxy; 1-methoxycarbonylpropoxy; 2-methoxycarbonyl-2-propyl and the like.

Preferred amides contemplated are those in which the group J is 2,3-dihydroxypropylamino; carboxymethylamino, acetylamino, benzoylamino, 4-chlorobenzoylamino; methanesulfonylamino; phenylsulfonylamino, 1-piperidyl, and the like.

Preferred keto-acids and keto-esters contemplated by the present invention are those in which the radical J is selected from the group consisting of carboxymethyl; carboxyethyl; 2-carboethoxy-2-propyl; dicarboethoxymethyl; carboethoxyvinyl and the like. Preferred alkanoic, alkenoic and alkynoic acids and esters are those in which the radical Z is selected from the group consisting of 4-carboxybutyl; 2-carboethoxyethyl; 2-carboxyvinyl, 2-carboethoxyethynyl, and the like.

FORMULA I-F

Preferred groups contemplated by the present

invention for the substituent $S_f$ are cyclopropyl, cyclopentyl, cyclohexyl, phenyl, 4-chlorophenyl, 4-fluorophenyl, 3-methoxyphenyl, 4-methylphenyl, naphthyl, and the like.

Preferred alkylene, alkenylene and alkynylene groups contemplated by the present invention for the moiety $T_f$ are, for example, octamethylene, undecamethylene, tetradecamethylene, hexadecamethylene, 3-methylheptamethylene, 1,1,6-trimethylheptamethylene, 1-(n-butyl)pentamethylene, 1-ethyl-1-methylpentamethylene, 2,7-dimethyloctamethylene, 1-(n-octyl)decamethylene, 2-undecenylene, 1-(2-butenyl)decamethylene, 1-(1-methyl-2-propenyl)decamethylene, 1-(n-hexyl)-3-decenylene, 7-hexadecenylene, 9-hexadecenylene, 1-(n-butyl)-3-dodecenylene, 12-hexadecenylene, 3,7,7-trimethyl-2-heptenylene, 1-(n-propyl)-2-heptenylene, 2-decenylene, 9-methyl-2-nonenylene, 1-(n-pentyl)-2-undecynylene, 1-ethyl-2-tridecynylene, 1-(n-hexyl)-4-decynylene, 4-hexadecynylene, and the like.

Preferred heteroaryl rings contemplated by the present invention for the moiety Q are furan, pyrazole, pyridine, pyrimidine, oxazole, pyrrole, thiazole, thiophene, and the like.

Preferred groups contemplated by the present invention for the substituent J are, for example, methoxy, isopropoxy, 2-ethoxyethoxy, 2-dimethylaminoethoxy, 1-methyl-4-piperidyloxy, 4-pyridylmethoxy, 2,3-dihydroxypropoxy, 2-hydroxypropoxy, 3-hydroxypropoxy, 4-chlorobenzyloxy, 3-methylbenzyloxy, 4-fluorophenoxy, 4-sulfophenoxy, 2,6-dichlorophenoxy, 3-carboxyphenoxy, 2,6-dimethyl-3-pyridyloxy, 6-methoxy-3-pyridyloxy, 2-hydroxy-3-pyridyloxy, 5-carboxy-3-pyridyloxy, carboxymethoxy, 1-methoxycarbonylpropoxy, 2-methoxycarbonyl-2-propoxy, 2,3-dihydroxypropylamino, carboxymethylamino, acetylamino, benzoylamino, 4-chlorobenzoylamino, methanesulfonylamino, p-toluenesulfonylamino, 1-piperidyl, and the like.

A preferred embodiment of the present invention relates to those compounds of formula I-F wherein $S_f$, $T_f$, and J are as defined above and Q is selected from the group consisting of thiophene, furan, pyrrole, pyridine and pyrimidine. A more preferred embodiment of the present invention relates to those compounds in which $S_f$ is hydrogen; $T_f$ is as defined above; Q is furan, pyrrole, or thiophene; and J is hydroxy. The most preferred embodiment of the present invention relates to those compounds in which $S_f$ is hydrogen; $T_f$ is as defined above; and Q and J are selected such that formula I-F represents a 4- or 5-(monosubstituted-amino)-2-thiophenecarboxylic acid, a 5-(monosubstituted-amino)-2-furancarboxylic acid or a 4- or 5-(monosubstituted-amino)-1-methyl-2-pyrrolecarboxylic acid.

FORMULA I-G

Preferred grops contemplated by the present invention for the substiuent $S_g$ are, for example, carbomethoxy, carboethoxy, carbo-*tert*-butoxy, carbophenoxy, carbo-*p*-chlorophenoxy, carbo-4-pyridylmethoxy, carbo-3-pyridyloxy, N-methylcarbamyl, N,N-diethylcarbamyl, N-methylsulfamyl, N-phenylsulfamyl, N-(*p*-toluene)sulfamyl, and the like.

Preferred alkylene, alkenylene, alkynylene, and cycloalkylene groups contemplated by the present invention for the moiety $T_g$ are, for example, octamethylene, undecamethylene, tetradecamethylene, hexadecamethylene, 3-methylheptamethylene, 1,1,6-trimethylheptamethylene, 1-(*n*-butyl)-pentamethylene, 1-ethyl-1-methylpentamethylene, 2,7-dimethyloctamethylene, 1-(*n*-octyl)decamethylene, 2-undecenylene, 1-(2-butenyl)decamethylene, 1-(1-methyl-2-propenyl)decamethylene, 1-(*n*-hexyl)-3-decenylene, 7-hexadecenylene, 9-hexadecenylene, 1-(*n*-butyl)-3-dodecenylene, 12-hexadecenylene, 3,7,7-trimethyl-2-heptenylene, 1-(*n*-propyl)-2-heptenylene, 2-decenylene, 9-methyl-2-nonenylene, 1-(*n*-pentyl)-2-undecynylene, 1-ethyl-2-tridecynylene, 1-(*n*-hexyl)-4-decynylene, 4-hexadecynylene, 2,6,6-trimethyl-1,3-cyclohexylenemethylene, 2,2,4-trimethyl-1,3-cyclopentylenemethylene, and the like.

Preferred groups contemplated by the present invention for the substituent J are, for example, methoxy, isopropoxy, 2-ethoxyethoxy, 2-dimethylaminoethoxy, 1-methyl-4-piperidyloxy, 4-pyridylmethoxy, 2,3-dihydroxypropoxy, 2-hydroxypropoxy, 3-hydroxypropoxy, 4-chlorobenzyloxy, 3-methylbenzyloxy, 4-fluorophenoxy, 4-sulfophenoxy, 2,6-dichlorophenoxy, 3-carboxyphenoxy, 2,6-dimethyl-3-pyridyloxy, 6-methoxy-3-pyridyloxy, 2-hydroxy-3-pyridyloxy, 5-carboxy-3-pyridyloxy, carboxymethoxy, 1-methoxycarbonylpropoxy, 2-methoxycarbonyl-2-propoxy, 2,3-dihydroxypropylamino, carboxymethylamino, acetylamino, benzoylamino, 4-chlorobenzoylamino, methanesulfonylamino, p-toluenesulfonylamino, 1-piperidyl, and the like.

FORMULA I-H

Preferred groups contemplated by the present invention for the polyfluorinated substituent X are, for example, 6-(trifluoromethyl)hexyl, 8-(trifluoromethyl)octyl, 10-(trifluoromethyl)decyl, 11-(trifluoromethyl)undecyl, 13-(trifluoromethyl)tridecyl, 15-(trifluoromethyl)pentadecyl, 17-(trifluoromethyl)heptadecyl, 1-(2,2,2-trifluoroethyl)decyl, 1-(2,2,2-trifluoroethyl)pentadecyl, 7-(trifluoromethyl)undecyl, 1-hexyl-11-(trifluoromethyl)undecyl, 3,7-dimethyl-7-(trifluoromethyl)octyl, 11-(pentafluoroethyl)undecyl, 16-(pentafluoroethyl)hexadecyl, 11-(heptafluoropropyl)undecyl, 11-(heptafluoroisopropyl)undecyl, 11-(nonafluorobutyl)undecyl, 11-(undecafluoropentyl)undecyl, 12-fluoro-12-(heptafluoropropyl)-12-(trifluoromethyl)dodecyl, 5-(pentadecafluoroheptyl)pentyl, 7-(pentadecafluoroheptyl)heptyl, 3,3-difluorotetradecyl, 6,6-difluorohexadecyl, 11,11-difluorododecyl, 14,14-difluoropentadecyl, 15,15-difluorohexadecyl, 16,16-difluoroheptadecyl, 11,11-difluoro-16-(trifluoromethyl)hexadecyl, (pentadecafluoroheptyl)methyl, (hentricontafluoropentadecyl)methyl, 11-(trifluoromethyl)-9-undecenyl, 15-(trifluoromethyl)-9-pentadecenyl, 5-(pentadecafluoroheptyl)-4-pentenyl, 6-(pentadecafluoroheptyl)-5-hexenyl, 15-(trifluoromethyl)-7-pentadecenyl, 15-(trifluoromethyl)-12-pentadecenyl, 15-(trifluoromethyl)-4-pentadecynyl, 1-hexyl-9-(trifluoromethyl)-4-

nonynyl, 3-(2,2,2-trifluoroethyl)-2,4,4-trimethylcyclopentyl, 3-(trifluoromethyl)-2,4,4-trimethylcyclohexyl, and the like.

Preferred esters contemplated by the present invention are those in which the group J is methoxy; isopropoxy; 2-ethoxyethoxy; 2-dimethylaminoethoxy; 1-methyl-4-piperidyloxy; 4-pyridylmethoxy; 2,3-dihydroxypropoxy; 2-hydroxypropoxy; 3-hydroxypropoxy; 4-chlorobenzyloxy; 3-methylbenzyloxy; 4-sulfophenoxy; 4-fluorophenoxy; 2,6-dichlorophenoxy; 3-carboxyphenoxy; 2,6-dimethyl-3-pyridyloxy; 6-methoxy-3-pyridyloxy; 2-hydroxy-3-pyridyloxy; 5-carboxy-3-pyridyloxy; 4-cyano-3-pyridyloxy; carboxymethoxy; 1-methoxycarbonylpropoxy; 2-methoxycarbonyl-2-propyl and the like.

Preferred amides contemplated are those in which the group J is 2,3-dihydroxypropylamino; carboxymethylamino, acetylamino, benzoylamino, 4-chlorobenzoylamino; methanesulfonylamino; phenylsulfonylamino, 1-piperidyl, and the like.

Preferred keto-acids and keto-esters contemplated by the present invention are those in which the radical J is selected from the group consisting of carboxymethyl carboxyethyl; 2-carboethoxy-2-propyl; dicarboethoxymethyl; carboethoxyvinyl and the like. Suitable alkanoic, alkenoic and alkynoic acids and esters are those in which the radical Z is selected from the group consisting of 4-carboxybutyl; 2-carboethoxyethyl; 2-carboxyvinyl, 2-carboethoxyethynyl, and the like.

The compounds of the present invention may be readily prepared by the following general process:

A compound of the formula:

D-E

wherein D is X as defined above, or a group convertible into X;

E is halogen, alkanesulfonyloxy, arenesulfonyloxy, trifluoromethanesulfonyloxy, or $-\overset{O}{\overset{\|}{C}}-G$ wherein G is hydrogen, halogen, acyloxy, 1-imidazolyl, an activated ester moiety,

or a hydrocarbyl group provided that when G is a hydrocarbyl group the moieties D and G may in addition be directly bonded so that $D-\overset{\overset{O}{\|}}{C}-G$ forms a cyclic ketone, and further provided that $D-\overset{\overset{O}{\|}}{C}-G$ comprises the carbon skeleton of a desired X;
is reacted with a compound of the formula:

$$L-\!\!\!+\ Q\ +\!\!\!-M \qquad ....II$$

wherein Q is defined above;
L is -NHR, -NHV, -NXR or -NXV or a group convertible *in situ* thereinto wherein X and R are as defined above and V is an amine protecting group such as benzyl, trifluoroacetyl, carbo-*t*-butoxy, carbobenzyloxy, trialkylsilyl, and the like;
M is Z which is as defined above or a group convertible to Z; provided that when E is a bromine atom bonded to a saturated carbon atom and L is $-NH_2$ E and L may be interchanged, and further provided that when L is -NXR or -NXV then M is hydrogen and compound II is reacted in the presence of a suitable catalyst such as a Lewis acid, with a compound of the formula $J'-K'$ wherein $J'$ is a suitable leaving group such as halogen, sulfonyloxy, and the like, and K is Z or a group convertible thereto; and when required either during or after its formation, reducing an amide carbonyl or imino C=N group to produce the -NX group, and in any order or at anytime, before or after reacting compound II with $J'-K'$ or D-E, introducing or removing the protecting group V, or converting a specified M or X group to any other M or X; and/or forming the pharmaceutically acceptable acid addition or cationic salt thereof.

The substituted amino compounds of the present invention are preferably prepared by reaction of the appropriate unsubstituted amino compounds with suitable alkylating agents. For purposes of this invention, the term "alkylating agent" will be understood to include those agents which can introduce the moiety X or a group convertible to X when reacted with an amine under suitable conditions of substitution. Examples of such agents are halides, sulfates, alkanesulfonates, arenesulfonates and the like. Particularly preferred are the chlorides, bromides, tosylates, methanesulfonates, and the trifluoromethanesulfonates. The alkylation reaction may be carried out with or without solvent at from 30°-150°C. Suitable solvents are lower alkanols, N,N-dimethylformamide, N,N-dimethylacetamide; diglyme, acetonitrile, toluene, benzene, hexamethylphosphoramide and the like. The reaction may be carried out with 2 equivalents of the amino substrate or with one equivalent plus an equivalent of base such as an unreactive organic base such as diisopropylethylamine or an alkali carbonate or bicarbonate or with a catalytic amount of copper powder when a halide is used as the alkylating agent. When Q is substituted or unsubstituted p-phenylene, similar alkylation of the sodium salt of the anilide moiety of a 4-acylaminophenyl compound (formed by reaction with for example sodium hydride) yields the corresponding N-acyl compound which may also be prepared by acylation of (4-alkylamino)phenyl compounds. The acyl groups may subsequently be removed, for example, by basic hydrolysis. In a related procedure, a 4-(Z-substituted)-bromobenzene may be reacted with a suitably substituted primary amine, such as $X-NH_2$, to produce the compounds of the invention.

Alternative methods of preparation are by reductive alkylation of an amino compound which may also be generated *in situ* by reduction of an amino precursor such as a nitro group and the like or by hydride (diborane) reduction of an (acylamino)phenyl compound in a special procedure.

The reductive alkylation may be carried out, for example, by mixing equivalent amounts of a suitable amino compound and an aldehyde or ketone in a suitable solvent such as an alkanol and reducing with for example a metal borohydride such as sodium borohydride or with hydrogen at from 1-10 atmospheres pressure using a suitable catalyst, such as an activated metal catalyst. It will be understood

that the aldehyde or ketone utilized in a reductive alkylation procedure, will comprise the carbon skeleton of a desired group X. Alternatively, the amino compound may be reacted with a suitable aldehyde or ketone and the resulting imino (C=N) or Schiff base compound, may be isolated and subsequently reduced by, for example, treatment with sodium borohydride or $H_2$/catalyst as above. Those skilled in the art will understand what is meant by an aldehyde or ketone which comprises the carbon skeleton of a desired group X. Thus, for example, acetaldehyde would be utilized to provide an ethyl group, acetone would similarly be utilized to provide an i-propyl group, and cyclohexanone would provide a cyclohexyl group,and cyclopropanecarboxaldehyde would provide a cyclopropylmethyl substitutent.

When the aromatic reactant being utilized in the processes of the present invention comprises a particular class of sustituents or when it is desired that the product comprise a particular type of substituent, it has been observed that additional processes can be utlized to efficiently and effectively prepare the compounds of the invention.

These procedures are disclosed in addition to the main process of the invention for the convenience of those who may wish to practice the instant invention and are not intended to be used to delimit the scope of the invention in any way.

COMPOUNDS OF FORMULA I-A

The cycloalkylamino, cycloalkenylamino, cycloalkylalkylamino, cycloalkyl-alkenylamino, cycloalkenyl-alkylamino, cycloalkyl-cycloalkylamino benzoic acids of this invention are prepared by reaction of loweralkyl p-aminobenzoates with suitable alkylating agents, such as cycloalkyl, cycloalkenyl, cycloalkyl-alkyl, cycloalkyl-alkenyl, cycloalkenyl-alkyl and cycloalkyl-cycloalkyl halides, sulfates, tosylates, mesylates or tryfluoromethylsulfonates, with or without a solvent at 50-150°C. Suitable solvents are lower alkanols, chloroform N,N-dimethylformamide, N,N-dimethylacetamide, diglyme,

dimethylsulfoxide, acetonitrile, toluene, benzene, hexamethylphosphoramide and like solvents.

The reaction may be carried out with 2 equivalents of the alkyl aminobenzoate or with one equivalent of base, such as an unreactive organic base such as diisopropylethylamine or an alkali carbonate or bicarbonate, or with a catalytic amount of copper powder when an appropriate halide is used as the alkylating agent. The resulting benzoate esters are readily hydrolyzed to the acids in aqueous ethanolic alkali at 25°-100°C. for 1 to 24 hours.

The loweralkyl N-acetyl-4-(substituted amino)benzoates are prepared by reaction of a loweralkyl 4-(acetylamino)-benzoate with an appropriate alkylating agent in the presence of an equivalent of sodium hydride in an inert solvent such as N,N-dimethylformamide, N,N-dimethylacetamide or diglyme at 50°-150°C. The N-acetyl benzoate esters are readily hydrolyzed and deacetylated to the acids in boiling aqueous ethanolic dilute alkali or acid.

Alternative methods of preparation are by reductive alkylation of a 4-aminobenzoic ester or amide which may also be generated in situ by reduction of 4-amino precursors such as a 4-nitro group and the like, or by a borohydride reduction of the chloroimide formed with phosphorus oxychloride from a 4-(acylamino)benzoate ester. For example, a cycloalkyl aldehyde or ketone plus 4-aminobenzoyl piperidide are reduced under 1-10 atmospheres of hydrogen using a metal catalyst, forming the 4-(cycloalkyl-alkylamino)benzoyl piperidide.

Two types of substitution reactions also yield the compounds of the present invention, namely, reaction of esters or amides of 3,4-didehydrobenzoic acid with an appropriate amine (or its alkali metal salt) and amination of 4-fluorobenzoate esters or amides. The former type of reaction in which the 3,4-didehydrobenzoic acid derivative is generated in situ, is carried out by treating a 4-halo compound such as 4-bromobenzoyl piperidide with the lithium, potassium or sodium salt of an amine (in excess) such as

cyclohezylmethylamine in diethyl ether or other aprotic solvent. The latter type comprises reacting 2-cyclopentylethylamine of the like with 4-fluorobenzoyl piperidide at elevated temperature.

The novel compounds of formula I-A where J is other than OH may be readily prepared by treating an acid halide, mixed acid anhydride, or activated ester or amide of a compound of Formula I-A with an appropriate hydroxy compound, amine, or salt of a carboxamide or sulfonamide. These reactions are preferably carried out in an inert solvent at a temperature of 5°-125°C. for a period of time of from about 30 minutes to 18 hours or more. In the case of the acid halide and other acid-forming acylating agents, the reaction is carried out in the presence of an acid scavenger such as di*iso*propylethylamine, 4-dimethylaminopyridine, pyridine, triethylamine, finely powdered sodium carbonate, and the like. The acid halide and anhydride starting materials may be obtained from the corresponding 4-(substituted amino)benzoic acids by methods which are well-known in the art or described herein. However, a protecting group on the arylamino nitrogen is used for best results. The simplest protecting group is provided by protonation of the amine to give an anilinium salt prior to or during formation of the acylating agent. Acylation of this amino group by carefully selected acyl groups such as carbobenzyloxy, carbo-*t*-butoxy, and trifluoroacetyl provides protection of this group from self-acylation during amide or ester formation. These protecting groups are then removed by catalytic hydrogenation, mild acid treatment and mild alkali treatment, respectively.

Activated esters or amides, which are used to synthesize the esters of the present invention, are carboxymethyl, 4-nitrophenyl, N-oxysuccinimide, 1-imidazolyl and the like. In certain cases, treatment of acids or ordinary esters such as methyl or ethyl with an excess of an appropriate hydroxy-containing substrate in the presence of a Lewis or mineral acid such as boron trifluoride, sulfuric

acid, or hydrochloric acid is sufficient to convert the 4-(substituted amino) benzoic esters or acids to the appropriate esters.

With certain kinds of substrates for ester formation, it is necessary to form the alkali metal or strong organic base salts of the 4-(substituted amino)benzoic acids in order to react them with 2,3-dihydroxypropyl iodide, ethyl chloracetate and the like. Other esters are prepared from the avids themselves by reaction with diazoalkanes, ethyl diazoacetate or the like.

The 4-(substituted amino)benzoic acids and derivatives are prepared by de-acylation of the corresponding 4-N-(trifluoroacetyl N-substituted amino)benzoic ester or amide by reacting with an alkali hydroxide such as sodium or potassium hydroxide in a lower alkanol, water or an aqueous lower alkanol at 5°-50°C. Alternatively, these compounds may be prepared by de-acylation of the 4-(N-carbo-t-butoxy-N-substituted amino)benzamide and the like with mineral acids such as hydrochloric or hydrobromic acid, preferably in glacial acetic acid at 0°C. to 50°C. Also, they are prepared by removal of the carbobenzyloxy protecting group from the anilino nitrogen atom by means of mild catalytic hydrogenation or by treatment with a mineral acid such as hydrobromic acid in glacial acetic acid.

With certain kinds of substrates for amide formation, it is necessary to form the alkali metal or strong organic base salts of these substrates in order to react them with the various aforementioned acylating forms of the 4-(substituted amino)benzoic acids. The amino-alkanecarboxylic and aminoalkanesulfonic acids are zwitterionic and must be converted to their cationic salts, suitably in situ. They may also be used in the form of their esters and then hydrolyzed after amide formation. Certain substrates, which are neutral like the carboxamides or slightly acidic like the alkane or arene sulfonamides, are converted to reactive sodium sales by reaction with sodium hydride or other basic

reagents.

Alternatively, the free acids may be prepared by hydrolysis of the corresponding nitriles or various amides, imidates or oxazolines. The carboxylic acid moiety may also be generated by oxidation of the corresponding aldehydes, acetophenones, benzyl alcohols, or alkylbenzenes, most often with the use of an amine-protecting group such as trifluoroacetyl or t-butyloxycarbonyl.

COMPOUNDS OF FORMULA I-B

The 4-[(unsaturated or cyclopropylated alkyl)amino]-benzoic acids of this invention are prepared by reaction of loweralkyl 4-aminobenzoates with suitable alkylating agents, such as unsaturated or cyclopropylated alkyl halides, sulfates, tosylates, mesylates or trifluoromethylsulfonates, with or without a solvent at 25-150°C. Suitable solvents are lower alkanols, chloroform, N,N-dimethylformamide, N,N-dimethylacetamide, diglyme, dimethylsulfoxide, acetonitrile, toluene, benzene, hexamethylphosphoramide and like solvents. The reaction may be carried out with 2 equivalis of the loweralkyl aminobenzoate or with one equivalent of the loweralkyl aminobenzoate and one equivalent of base, such as an unreactive organic base such as diisopropylethylamine or an alkali carbonate or bicarbonate, or with a catalytic amount of copper powder when an appropriate halide is used as the alkylating agent

The resulting benzoate esters are readily hydrolyzed to the acids in boiling aqueous ethanolic alkali.

Alternative methods or preparation are by reductive alkylation of a 4-aminobenzoic ester or amide which may also be generated *in situ* by reduction of 4-amino precursors such as a 4-nitro group and the like, or by a borohydride reduction of the chloroimide formed with phosphorus oxychloride from 4-(acylamino)benzoate ester. For example, an unsaturated or cyclopropylated aldehyde or ketone plus 4-aminobenzoyl piperidide are reduced under 1-3 atmospheres of hydrogen using a metal catalyst, to form 4-([unsaturated or cyclopropylated alkyl]amino)benzoyl piperidide.

Two types of substitution reactions also yield the compounds of the present invention, namely reaction of esters or amides of 3,4-didehydrobenzoic acid with an (unsaturated or cyclopropylated alkyl)amine (or its alkali metal salt) and amination of 4-fluorobenzoate esters. The former type of reaction is carried out by treating a 4-halo compound such as 4-bromobenzoyl piperidide with the lithium, potassium or sodium salt of an amine (in excess) of such as pentadec-4-enylamine in diethyl ether or other aprotic solvent. The latter type comprises reacting pentadec-4-enylamine or the like with 4-fluorobenzoyl piperidide at elevated temperature.

The novel compounds of the present invention may be readily prepared by treating an acid halide, mixed acid anhydride, or activated ester or amide with a hydroxy compound, an amine, or a salt of a carboxamide or sulfonamide. These reactions are preferably carried out in an inert solvent at a temperature of 5°-125°C. for a period of time from about 30 minutes to 18 hours or more. In the case of the acid halide and other acid-forming acylating agents, the reaction is carried out in the presence of an acid scavenger such as di*iso*propylethylamine, 4-dimethylaminopyridine, pyridine, triethylamine, finely powdered sodium carbonate, and the like. The acid halide and anhydride starting materials may be obtained from the corresponding 4-(monoalkylamino)benzoic acids by methods which are well-known in the art or described herein. However, a protecting group on the arylamino nitrogen is used for best results. The simplest protecting group is provided by protonation of the amine to give an anilinium salt prior to or during formation of the acylating agent. Acylation of this amino group by carefully selected acyl groups such as carbobenzyloxy, carbo-*t*-butoxy, and trifluoroacetyl provides protection of this group from self-acylation during amide or ester formation. These protecting groups are then removed by catalytic hydrogenation, mild acid treatment and mild alkali treatment, respectively, Activated esters or amides, which are used to synthesize the

esters of the present invention, are carboxymethyl, 4-nitrophenyl, N-oxysuccinimide, 1-imidazolyl and the like. In certain cases, treatment of acids or ordinary esters such as methyl or ethyl with an excess of an appropriate hydroxy-containing substrate in the process of a Lewis or mineral acid such as boron trifluoride, sulfuric acid, or hydrochloric acid is sufficient to convert the 4-([unsaturated or cyclopropylated alkyl]amino)benzoic esters or acids to the appropriate esters.

With certain kinds of substrates for ester formation, it is necessary to form the alkali metal or strong organic base salts of the 4-(substituted amino)benzoic acids in order to react them with 2,3-dihydroxypropyl iodide, ethyl chloroacetate and the like. Other esters are prepared from the acids themselves by reaction with diazoalkanes, ethyl diazoacetate or the like.

The 4-[(unsaturated or cyclopropylated alkyl)amino]benzoic acids and derivatives are prepared by deacylation of the corresponding 4-(N-trifluoroacetyl-[unsaturated or cyclopropylated alkyl]amino)benzoic ester or amide by reacting with an alkali hydroxide such as sodium or potassium hydroxide in a lower alkanol, water or an aqueous lower alkanol at 5°C. to 50°C. Alternatively, these compounds may be prepared by deacylation of the 4-(N-carbo-*t*-butoxyalkenylamino)benzamide and the like with mineral acids such as hydrochloric or hydrobromic acid, preferably in glacial acetic acid at 0°C. to 50°C. Also, they are prepared by removal of the carbobenzyloxy protecting group from the anilino nitrogen atom by means of mild catalytic hydrogenation or by treatment with a mineral acid such as hydrobromic acid in glacial acetic acid.

With certain kinds of substrates for amide formation, it is necessary to form the alkali metal or strong organic base salts of these substrates in order to react them with the various aforementioned acylating forms of the 4-[(unsaturated or cyclopropylated alkyl)amino]benzoic acids. The aminoalkanecarboxylic and aminoalkanesulfonic

acids are zwitterionic and must be converted to their cationic salts, suitably in situ. They may also be used in the form of their esters and then hydrolyzed after amide formation. Certain substrates, which are neutral like the caroxamides, are converted to reactive salts by reaction with sodium hydride or other basic reagents.

Alternatively the free acids may be prepared by hydrolysis of the corresponding nitriles or various amides, imidates or oxazolines. The carboxylic acid moiety may also be generated by oxidation or the corresponding aldehydes, acetophenones, benzyl alcohols, ortoluenes, most often with the use of an amine-protecting group such as trifluoroacetyl or t-butyloxycarbonyl.

COMPOUNDS OF FORMULA I-C

Many of the novel 4-[(monosubstituted-alkyl)amino]-benzoic acids, esters, and amides of the present invention are prepared by reactions of the corresponding 4-aminobenzoic acid, ester, or amide with an appropriate alkylating or acylating agent followed by chemical modification of the monosubstitutedalkyl group. Alkylating and acylating agents especially suitable for these reactions are hydroxyalkyl halides and haloalkanoic acids, which are generally known in the chemical literature. Those which are not known are readily prepared from the corresponding hydroxyalkanoic acids either by reduction of the carboxylic acid group with reagents such as the alkali metal hydrides or by replacement of the hydroxy group with halogen with halogen with reagents such as hydrogen bromide. In certain cases, chemical modification of the monosubstituted-alkyl moiety before, rather than after the alkylation or acylation reaction as described above, is preferable. In these cases, reactions similar to those described subsequently for the interchange of substituents in 4-[(monosbstituted-alkyl)amino]phenyl compounds are employed.

Alkylation of the appropriate 4-aminophenyl compounds with suitable alkylating agents such as halides, sulfates, tosylates or trifluoromethanesulfonates is carried

out with or without solvent at 50°C.-150°C. Suitable solvents are lower alkanols, chloroform, N,N-dimethylformamide, N,N-dimethylacetamide, diglyme, dimethylulfoxide, acetonitrile, toluene, benzene, hexamethylphosphoramide and the like. The reaction may be carried out with an equivalent of base such as an alkali carbonate or bicarbonate, or with a catalytic amount of copper powder when substituted alkyl halides are used as the alkylating agent. Alternatively, alkylation of the sodim salt (formed with sodium hydride) of either amino group of a 4-aminophenyl compound or the anilide moiety of a 4-(acetylamino)phenyl compound yields the desired alkylation product or an N-acetyl derivative thereof. Other 4-aminophenyl compounds useful in these alkylation reactions, or in the subsequently described acylation reactions, are for example, 4-aminobenzonitrile, p-toluidine, and the acetal of 4-amionbenzaldehyde. In these cases, the desired alkylation product is obtained by a subsequent oxidative or hydrolytic transformation of the nitrile, methyl, or actal group.

Acylation of a 4-aminophenyl compound followed by diborane reduction of the resulting 4-(acylamino)phenyl compounds at room temperature of above for 1-16 hours also yields certain of the 4-[(monosubstituted-alkyl)amino]-phenyl compounds of the present invention. An alternative method for the reduction of 4-(acylamino)benzoate esters of 4-(acylamino)benzamides involves reaction of these intermediates with phosphorus oxychloride followed by reduction of the resulting chloroimides with sodium borohydride.

An alternative method for the preparation of certain of the desired alkylation products involves reductive alkylation of a 4-aminobenzoic ester or amide with a substituted aldehyde or ketone. The 4-aminobenzoic ester or amide itself may be used or it may be generated _in situ_ by reduction of a 4-amino group precursor such as the 4-nitro group.

Two types of substitution reactions also yield certain of the 4-[(monosubstituted-alkyl)amino]phenyl

compounds of the present invention. The reaction of esters of 3,4-didehydrobenzoic acid with an alkylamine 8or its alkali metal salt), or Friedel-Crafts acylation of an N-alkylaniline or N-acyl-N-alkylaniline yields certain of the desired compounds or intermediates thereto. The former type of reaction, in which the 3,4-didehydrobenzoic acid is generated in situ is carried out by treating a 4-halobenzoate ester or disubstituted amide such as phenyl 4-bromobenzoate with the lithium, potassium or sodium salt of excess alkylamine such as 11-(n-butylthio)undecylamine in diethyl ether or other aprotic solvent. The second method comprises reacting N-(11-methylthiohexadecyl)aniline and the like or its N-acetyl derivative with a carboalkoxy chloride or oxalyl chloride and anhydrous aluminum chloride in dry diethyl ether, halocarbon or hydrocarbon medium.

The 4-[(monosubstituted-alkyl)amino]benzoic acids of the present invention may be obtrained by hydrolysis of the correspoding 4-[(monosubstituted-alkyl)amino]benzoate essters in boiling aqueous ethanolic alkali followed by acidification. Alternatively, these benzoic acids may be isolated as their alkali metal salts.

The novel 4-[(monosubstituted-alkyl)amino]benzoic esters and amides of the present invention may be readily prepared by treating the corresponding acid halides, mixed acid anhydrides, or activated esters or amides with an appropriate hydroxy compound, amine, or salt of a carboxamide or sulfonamide. These reactions are preferably carried out in an inert solvent at a temperature of 25°-125°C. for a period of time from about 30 minutes to 18 hours or more. In the case of the acid halide and other acid-forming acylating agents, the reaction is carried out in the presence of an acid scavenger such as diisopropylethylamine, 4-dimethylaminopyridine, triethylamine, finely powdered sodium carbonate, and the like. The acid halide and anhydride starting materials may be obtained from the corresponding 4-[(monosubstitutedalkyl)amino]benzoic acids by methods

which are well-known in the art or described therein. However, a protecting group on the arylamino nitrogen is used for best results. The simplest protecting group is provided by protonation of the amine to give an anilinium salt prior to or during formation of the acylating agent. Acylation of this amino group by carefully selected acyl groups such as carbobenzyloxy, carbo-t-butoxy, and trifluoroacetyl provides protecting of this group from self-acylation during ester or amide formation. These protecting groups are then removed by catalytic hydrogenation, mild acid treatment, and mild alkali treatment, respectively. Activated esters or amides, which are used to synthesize the esters or amides of the present invention, are carboxymethyl, 4-nitrophenyl, N-oxysuccinimide, 1-imidazolyl and the like. In certain cases, treatment of acids or ordinary esters such as methyl or ethyl with an excess of an appropriate hydroxy-containing substrate in the presence of a Lewis or mineral acid such as boron trifluoride, sulfuric acid, or hydrochloric acid is sufficient to convert the 4-[(monosubstituted-alkyl)amino]benzoic esters or acids to the appropriate esters.

With certain kinds of substrates for ester formation, it is necessary to form the alkali metal or strong organic base salts of the 4-[(monosubstituted-alkyl)amino]-benzoic acids in order to react them with the various halo-, methanesulfonate- or p-toluenesfulfonate-containing substrates. Certain 4-[(monosubstituted-alkyl)amino]benzoate esters may be prepared by the reaction of 4-[(monosubstituted-alkyl)amino]benzoic acids with diazoalkyl compounds such as ethyl diazoacetate and the like, obtained by reaction of nitrous acid or alkyl nitrite on α- or β-aminoalkanoate esters.

The [(monosubstituted-alkyl)amino]benzoate esters or amides are also prepared by de-acylation of the corresponding 4-(N-trifluoroacetyl-monosubtituted-alkylamino)benzoate esters or amides by reaction with an alkali hydroxide such as sodium or potassium in a lower alkanol, water or an

aqueous lower alkanol at 5°C. to 50°C. Alternatively, the 4-[(monosubstituted-alkyl)amino]benzoate esters or amides may be prepared by de-acylation of the 4-(N-carbo-t-butoxy-N-alkyl-amino)benzoate esters or amides with mineral acids such as hydrochloric or hydrobromic acid, preferably in glacial acetic acid or with anhydrous trifluoroacetic acid at 0°C. to 50°C. Also these compounds are prepared by removal of the carbobenzyloxy protecting group from the anilino nitrogen atom by means of catalytic hydrogenation or treatment with a mineral acid such as hydrobromic acid, preferably in glacial acetic acid.

With certain kinds of substrates for amide formation, it is necessary to form the alkali metal or strong organic base salts of these substrates in order to react them with the various aforementioned acylating forms of the 4-[(monosubstituted-alkyl)amino]benzoic acids. The aminoalkanecarboxylic and aminoalkanesulfonic acids are zwitterionic and must be converted to their salts suitably <u>in situ</u>. They may also be used in the form of their esters and then hydrolyzed after amide formation. Certain substrates, which are neutral like the carboxamides or slightly acidic like th alkane or arenesulfonamides, are converted to reactive sodium salts by reaction with sodium hydride or other basic reagents.

In certain cases, unsaturation is introduced at a late stage of the preparation of 4-[(monosubstituted-unsaturatedalkyl)amino]benzoic acid derivatives. For example, an alkyl 4-[(monosubstituted-ω-haloalkyl)amino]benzoate is dehydrohalogenated to the corresponding olefinic compound or, alternatively, it is converted to a Wittig trialkylphosphonium reagent and reacted with an aldehyde to yield a product with an internal double bond. This ω-halo substrate is also reacted with an alkylacetylene sodium or lithium salt to form 4-[(monosubstituted-alkynylamino)benzoic acid derivatives. In other cases, a 4-[(monosubstituted-unsaturatedalkyl)amino]benzoic acid derivative is converted to the corresponding saturated derivative by catalytic hydrogenation.

In still other cases, a 4-(unsubstituted-unsaturatedalkyl-amino)benzoic acid derivative is converted to the desired 4-(monosubstituted-monoalkylamino)benzoic acid derivative by the addition of a reagent to the double or triple bond. An example of this type of reaction is the addition of hydrogen bromide to ethyl 4-(11-undecenylamino)benzoate to yield ethyl 4-(10-bromoundecylamino)benzoate.

Many of the novel compounds of formula I-C are prepared by chemical modifications of the substituent already present in a 4-[(monosubstituted-alkyl)amino]phenyl compound. An example of this type of modification is the conversion of a hydroxy substituent to a group activated for displacement, such as a methanesulfonate or p-toluenesulfonate ester. This modification is accomplished by methods well known to those skilled in the art. Alternatively, the hydroxy substituent may be converted to a halogen substituent, for example, the reaction of 4-(11-hydroxyundecylamino)-benzoic acid with hydrogen bromide affords 4-(11-bromoundecyl-amino)benzoic acid.

Displacement reactions of the above described sulfonate esters and halides may be carried out with a variety of nucleophiles, such as, for example, thiourea, potassium thioacetate, sodium thiophenoxide, the sodium or potassium salt of 2-mercaptopropane or 3-(p-chlorophenyl)propanethiol, the sodium salt of methanesulfonamide, sodium azide, potassium chloride, sodium iodide and the like. These displacement reactions yield 4-[(monosubstituted-monoalkyl) amino]phenyl compounds, monosubstituents such as, thioronium halide, acetylthio, phenylthio, 2-propylthio, 3-(p-chlorophenyl)propylthio, amino, anilino, azido, chloro, iodo, and the like. Hydrolysis of the thioacetates or thiuronium salts resulting from these displacements affords 4-[(mercapto-substituted-4-(alkyl)amino]phenyl compounds. The 4-[(mercapto-alkyl)amino]phenyl compounds may be alkylated with aklyl or aralkyl halides to yield 4-[(alkylthio- or aralkylthio-substitutedalkyl)amino]-phenyl compounds. Oxidation of these alkylthio- or aralkylthio-substituted derivatives or the previously mentioned 4-[(arylthio-substitutedalkyl)amino]phenyl compounds with one equivalent of an oxidizing agent such as m-chloroperbenzoic acid affords the corresponding sulfoxides. Oxidation of these derivatives with excess oxidizing agent yields the corresponding sulfones. These sulfinyl and sulfonylalkyl derivatives are also obtained by alkylation of aminophenyl compounds such as ethyl 4-aminobenzoate with sulfinyl or sulfonyl-substituted alkyl halides or sulfonates.

The alkylation of certain 4-aminophenyl compounds with haloketones yields 4-(alkanoylalkylamino)phenyl compounds, for example alkylation of ethyl 4-aminobenzoate with 1-bromo-6-hexadecanone (obtained by the reaction of 6-bromohexanoyl chloride with n-decyl magnesium bromide in the presence of cadmium chloride) yields ethyl 4-(6-oxohexadecylamino)benzoate. Carbonyl derivatives of this type may be reduced to the corresponding alcohols with diborane, converted to tertiary alcohols by reaction with Grignard or alkyllithium reagents, or converted to the corresponding oximes by reaction with hydroxylamine hydrochloride.

COMPOUNDS OF FORMULA I-D

Many of the novel 4-(aralkyl- and heteroarylalkylamino) phenly compounds of Formula I-D may be prepared by reaction of a 4-aminophenyl compound with a suitable alkylating agent such as an alkyl halide, sulfate, tosylate, or trifluoromethanesulfonate with or without a solvent at 30°C. to 150°C. Appropriate 4-aminophenyl compounds are, for example, ethyl 4-aminobenzoate; ethyl 3-(4-aminophenyl)propionate, 2,3-dihydroxypropyl 4-aminobenzoate; phenyl 4-aminobenzoate; 1-(4-aminobenzoyl)pyrrolidine; and ethyl 4-(4-aminophenyl)butyrate. Suitable solvents are lower alkanols, N,N-dimethylformamide, N,N-dimethylacetamide, 1,2-dimethoxyethane, acetonitrile, toluene, benzene, hexamethylphosphoramide and the like. The reaction may be carried out with two equivalents of the 4-aminophenyl compound or with one equivalent of the compound plus one equivalent of a base such as an alkali carbonate or bicarbonate or an unreactive

organic base such as diisopropylethylamine or alternatively with a catalytic amound of copper powder when an alkyl halide is used as the alkylating agent. Similarly, alkylation of the sodium salt (formed with sodium hydride) of either the amino group of a 4-aminophenyl compound or the anilide moiety of a 4-(acetylamino)phenyl compound yields the novel 4-(aralkyl- and heteroarylalkylamino)phenyl compounds or an N-acetyl derivative thereof. Removal of the N-acetyl group by conventional hydrolytic methods affords the desired amino compounds.

Alternative methods of preparation of these compounds are by reductive alkylation of a 4-aminophenyl compound, which may be generated *in situ* by reduction of a 4-aminophenyl precursor such as a 4-nitrophenyl compound and the like or by a metal hydride reduction of a 4-(acylamino)-phenyl)compound. For example, 10-phenyldecanal or another carbonylalkane and ethyl 4-aminophenylacetate are reduced under 1-10 atmospheres of hydrogen using an activated metal catalyst or with a metal hydride such as sodium borohydride forming 4-(10-phenyldecylamino)phenylacetic acid and the like. Diborane reduction of 4-(aralkanoylamino)phenyl compounds such as ethyl 4-(11-phenylundecanoylamino)phenylacetate at room temperature or above for 1-6 hour yields the corresponding 4-(aralkylamino)phenyl compounds such as ethyl 4-(11-phenylundecylamino)phenylacetate. The 4-(aralkanoylamino)phenyl compounds used in these reductions are prepared by acylation of the appropriate 4-aminophenyl compounds with suitable acylating agents, such as aralkanoyl halides. To prepare the 4-(aralkyl- and heteroarylamino)-phenyl alkenoic and alkynoic acids it is advantageous to form the corresponding alkylchloroimide from the 4-(alkanoylamino)phenyl compounds using phosphorus oxychloride and base, and then reduce the alkylchloroimide moiety to an alkylamino group with sodium borohydride.

The 4-(aralkyl- and heteroarylalkylamino)benzoic and phenyl alkanoic acids of this invention are often prepared from the corresponding p-aminobenzoic and phenylalkanoic acids by the sequence involving esterification of the amino acid with ethanol in the presence of boron trifluoride etherate, followed by alkylation of the amino function as described above. The free acids are then liberated by hydrolysis of the ester with aqueous alcoholic sodium hydroxide at 80° for 2-10 hours, followed by acidification. The acids obtained by this procedure may be converted to the corresponding cationic salts. For example, the sodium salt may be prepared by reaction of the benzoic acid with sodium hydroxide in a mixture of ethanol and water. Alternatively, the free acids may be prepared by hydrolysis of the corresponding nitriles or various amides, imidates or oxazolines. The carboxylic acid moiety may also be generated by oxidation of the corresponding aldehydes, acetophenones, benzyl alcohols, or toluenes, most often with the use of an amine-protecting group such as trifluoroacetyl or t-butyloxycarbonyl.

The carboxaldehydes of Formula I-D may be prepared by several methods among which is alkylation of the corresponding acetals as described above followed by hydrolysis of the resulting 4-(aralkylamino)phenyl compound to the desired aldehyde. Aldehydes may also be prepared by reduction of the appropriate nitriles. For example, treatment of 4-(16-phenylhexadecylamino)hydrocinnamonitrile with stannic chloride and anhydrous hydrogen chloride gas, followed by hydrolysis in hot water provides 4-(16-phenylhexadecylamino)-hydrocinnamaldehyde. These reductions are also conveniently carried out with hydrides such as diisobutylaluminum hydride.

The novel esters and amides of Formula I-D may readily be prepared by treating a derivative of the corresponding carboxylic acid, such as the acid halide, mixed acid anhydride or activated ester or amide with the appropriate alcohol or amine, respectively. These reactions may be carried out in an inert solvent at temperature of 50-125°C. for 30 minutes to 18 hours or more. In the case of the acid halide and other acid-forming acylating agents, the reaction is carried out in the presence of an acid scavenger such as

diisopropylethylamine; 4-dimethylaminopyridine; pyridine; triethylamine, finely powdered sodium carbonate and the like. A protecting group on the amino nitrogen is used for best results. The simplest protecting group is provided by protonation of the amine to yield an anilinium salt prior to or during formation of the acylating form of the carboxyl group. Acylation of the amino group by carefully selected acyl groups such as carbobenzyloxy, carbo-t-butoxy, and trifluoroacetyl provides protection of this group from self-acylation during amide formation. These protecting groups are then removed by catalytic hydrogenation, mild acid treatment and mild alkali treatment, respectively. Other N-acyl protecting groups such as acetyl and succinoyl may be used, and these are removed by conventional methods. Activated esters and amides, useful to synthesize the esters and amides of the present invention, are those containing carboxymethyl, 4-nitrophenyl, N-oxysuccinimide and 1-imidazolyl groups and the like. In certain cases, treatment of the acids with an excess of an appropriate hydroxy-containing substrate in the presence of a Lewis or mineral acid such as boron trifluoride, sulfuric acid or hydrochloric acid affords the corresponding esters. Ordinary esters such as the methyl and ethyl esters are sufficiently reactive to form the amides of the 4-(aralkyl- and heteroaryl-alkylamino)benzoic and phenylalkanoic acids and highly reactive amine substrates such as hydroxylamine, hydrazines and certain alkyl primary amines. In order to form amides from certain kinds of substrates, it is necessary first to form the alkali metal or strong organic base salts of these substrates prior to reacting them with the various aforementioned acylating forms of the 4-(aralkyl- and hetero-aryl-alkylamino)benzoic and phenylalkanoic acids. For example, the aminoalkanecarboxylic and aminoalkanesulfonic acids are zwitterionic and must be converted to their salts, suitable in situ. They may also be used in the form of their esters and then hydrolyzed after amide formation. Certain substrates which are neutral, like the carboxamides, or slightly

acidic, like the alkane or arene sulfonamides, are converted to acylatable sodium salts by reaction with sodium hydride or other basic reagents.

The substituted 4-(aralkyl- and heteroarylalkylamino)acetophenones of the invention are prepared by reaction of a derivative of the appropriate benzoic acid, such as 4-(11-phenylundecylamino)benzoyl chloride hydrochloride, with two or more equivalents of the reactive salt of an acidic methylene compound, for example the sodium salt of diethyl malonate. Other benzoic acid derivatives are also suitable for this reaction, such as an N-trifluoroacetyl or N-tert-butyloxycarbonyl acid chloride, or a methyl ester of the acid. In some cases the final step in the preparation of the substituted 4-(aralkyl- and heteroarylalkylamino)-acetophenone is the removal of the nitrogen-protecting group. In other cases, hydrolysis of one or more of the ester groups in the acylation product affords an unstable polycarboxylic acid which undergoes decarboxylation to allow the preparation of another acetophenone derivative. For example, the reaction of tertbutyl ethyl [4-(11-phenylundecylamino)benzoyl]malonate with trifluoroacetic acid affords ethyl [4-(11-phenylundecylamino)benzoyl]acetate. In other cases, hydrolysis of one or more of the ester groups allows the preparation of the corresponding acid derivative. For example, the hydrolysis of ethyl [4-(6-phenylhexylamino)-benzoyl]acetate yields [4-(6-phenylhexylamino)benzoyl]acetic acid.

An alternative procedure for preparing certain substituted 4-(aralkyl- and heteroarylalkylamino)acetophenones is alkylation of the corresponding 4-aminoacetophenone by the methods above. For example, alkylation of methyl 3-(4-aminobenzoyl)propionate with 11-phenylundec-10-enyl bromide yields methyl 3-[4-(11-phenylundec-10-enylamino)-benzoyl]propionate. The related carboxylic acids are then obtained by hydrolysis. Certain of these acids are particularly useful for the preparation of 4-(aryl- and heteroarylalkylamino)phenyl)alkanoic acids by reduction. For example,

the Clemensen or Wolff-Kishner reduction of 3-[(4-(6-phenylhexylamino)benzoyl propionic acid yields 4-[4-(6-phenylhexylamino)phenyl]butyric acid.

The 4-(aryl- and heteroarylalkylamino)phenylalkenoic acids may be prepared by condensation of the appropriate aldehydes or by dehydration of the corresponding substituted-phenylhydroxyalkanoic acids. For example, ethyl 5-[4-(benzylamino)phenyl]-2,4-pentadienoate is obtained by the Wittig reaction of 4-(benzylamino)benzaldehyde with the Wittig reagent, triethyl 4-phosphonocrotonate. Alternatively, these alkenoic acids are obtained by heating 4-[N-(10-phenyldecyl-N-methyl)amino]benzaldehyde and the like with the sodium salt of the carbanion of ethyl acetate or with a mixture of ethyl acetate, acetic anhydride and potassium acetate. The second method is illustrated by dehydration of ethyl 3-((4-benzylamino)phenyl)-3-hydroxypropionate to yield ethyl 4-benzylaminocinnamate.

The acetylenic analogs are prepared by dehydrobromination of of the side-chain vic-dibrominated alkanoic acid. For example, dehydrobromination of ethyl 3-[4-benzylamino)phenyl]-2,3,-dibromopropionate, its isomers or N-acyl analogs or of ethyl 3-[(4-benzylamino)phenyl]-3-bromoacrylate yields ethyl 4-(benzylamino)phenylpropiolate. The acetylenic acids are also formed from (4-benzylamino)phenylacetylene metal salts by carboxylation with carbon dioxide. The 4-(aryl and heteoarylalkylamine)phenylacetylenes are also used by N-acylating with _t_-butyl azidoformate followed by conversion to the lithium acetylide salt and the subsequent reaction of the lithium salt with boron trifluoride etherate in tetrahydrofuran at -20°C. to form _tris_-[(4-substituted-alkylamino)phenylethynyl]boranes. The tetrahydrofuran solution of the borane is in turn reacted with ethyl diazoacetate, followed by water to yield ethyl 4-[(4-monoalkylamino)phenyl]butynoate.

The 4-(aralkyl- and heteroarylalkylamino)phenylalkanoic acids, amides, or esters are also prepared by catalytic reduction at 1 to 10 atmospheres of hydrogen of

the corresponding alkenoic or alkynoic acid derivatives.

The 4-(aryl- and heteroarylalkylamino)phenylalkenoic acids and derivatives are prepared by Friedel-Crafts acylation of the N-acyl-N-alkylanilines with the appropriate dicarboxylic acid anhydride or half acid chloride. The 4-(aryl- and heteroarylalkylamino)benzoylalkanoic acids or esters, obtained by this and by other syntheses, may be converted to the corresponding 4-(substituted-amino)phenylalkanoic acids by reduction with: (a) hydrazine and alkali in diethylene glycol at 140 for 3 hours, (b) zinc amalgam and ethanolic hydrochloric acid at 60 for 5 hours, (c) red phosphorus and hydriodic acid, or (d) ketalization with 1,2-ethanedithiol followed by Raney nickel desulfurization. The amides of these 4-(substituted-amino)phenylalkanoic acids are prepared by heating the corresponding 4-(substituted-amino)phenylalkyl ketones with aqueous alcoholic ammonium polysulfide followed by hydrolysis to yield the acids with the same number of carbon atoms as the ketone. These acids are also prepared by reacting 4-(N-t-butyloxycarbonyl-N-aralkyl- and heteroarylalkylamino)phenylmagnesium halides with 2-(3-halopropyl)-2-oxazolines, followed by mild acid removal of 2-oxazolinyl and t-butoxycarbonyl protecting groups. Similarly, the above Grignard reagent can be reacted with 3-bromotriethylorthopropionate in the presence of dilithiumtetrachlorocuprate to yield the desired acids after removal of the protecting groups from the amino and carboxyl groups.

COMPOUNDS OF FORMULA I-E

(Mono- or poly-fluoro)-4-aminophenyl compounds required as intermediates for the preparation of the novel (mono- or poly-fluoro)-4-(monosubstituted amino)phenyl compounds of the present invention are obtained by a variety of methods. The 4-amino-(mono- or poly-fluoro)benzoic acids are obtained by oxidation of the corresponding N-protected toluenes, for example 4-acetamido-2-fluorotoluene, with potassium permanganate. The 4-amino-(mono- or poly-fluoro)-benzoic acids are obtained by oxidation of the corresponding N-protected toluenes, for example 4-acetamido-2-fluorotoluene,

with potassium permanganate. The 4-amino-(mono- or poly-fluoro)benzoate esters are obtained by reactions of ammonia or sodium amide with a 4-halo-benzoate ester. For example, the reaction of sodium amide with ethyl 2,3,4,5,6-pentafluoro benzoate yields ethyl 4-amino-2,3,5,6-tetrafluorobenzoate.

The [4-amino(mono- or poly-fluoro)phenyl]alkanoic acids and esters required as intermediates are prepared by a sequence of reactions involving nitration of a suitable phenyalkanoic acid followed by reduction of the nitro group. For example, 2,3,5,6-tetrafluorophenylacetic acid is converted to 4-amino-2,3,5,6-tetrafluorophenylacetic acid by this method. The [4-amino-(mono- or poly-fluoro)benzoyl]alkanoic acids and esters are prepared by the reaction of ammonia or sodium amide with (4-halobenzoyl)alkanoic acids. For example, the reaction of sodium amide with ethyl 2,3,4,5,6-pentafluorobenzoylacetate yields ethyl(4-amino-2,3,5,6-tetrafluorobenzoyl)acetate.

Many of the novel 4-(monosubstituted-amino)phenyl compounds of the present invention may be prepared by reaction of the appropriate 4-aminophenyl compound with a suitable alkylating agent such as an alkyl halide, sulfate, tosylate, or trifluoromethanesulfonate with or without a solvent at 30°C. to 150°C. Appropriate 4-aminophenyl compounds are, for example, ethyl 4-amino-2-fluorobenzoate; ethyl 4-amino-3-fluorobenzoate; 2,3-dihydroxypropyl 4-amino-3-fluorobenzoate; phenyl 2-fluorobenzoate; 1-(4-amino-3-fluorobenzoyl)pyrrolidine; and ethyl 4-(4-amino-2-fluorophenyl butyrate. Suitable solvents are lower alkanols, N,N-dimethylformamide, N,N-dimethylacetamide, 1,2-dimethoxyethane, acetonitrile, toluene, benzene, hexamethylphosphoramide and the like. The reaction may be carried out with two equivalents of the 4-aminophenyl compound or with one equivalent of the compound plus one equivalent of a base such as an alkali carbonate or bicarbonate or an unreactive organic base such as diisopropylethylamine or alternatively with a catalytic amount of copper powder when an alkyl halide is used as the alkylating agent. Similarly, alkyl

ation of the sodium salt (formed with sodium hydride) of either the amino group of a 4-aminophenyl compound or the anilide moiety of a 4-(acetylamino)phenyl compound yields the novel 4-(monosubstituted-amino)phenyl compounds or an N-acetyl derivative thereof. Removal of the N-acetyl group by conventional hydrolytic methods affords the desired 4-(monosubstituted amino) phenyl compounds.

Alternative methods of preparation of these compounds are by reductive alkylation of a 4-aminophenyl compound, which may be generated in situ by reduction of a 4-aminophenyl precursor such as a 4-nitrophenyl compound and the like or by a metal hydride reduction of a 4-(acylamino)-phenyl compound. For example, n-hexadecanal or another carbonylalkane and 4-amino-2-fluorobenzoic acid are reduced under 1-10 atmospheres of hydrogen using an activated metal catalyst or with a metal hydride such as sodium borohydride forming 4-(hexadecylamino)-2-fluorobenzoic acid and the like. Diborane reduction of 4-(alkanoylamino)phenyl compounds such as ethyl 4-(hexadecanoylamino)-3-fluorobenzoate at room temperature or above for 1-6 hours yields the corresponding 4-(alkylamino)phenyl compounds such as ethyl 4-(hexadecylamino)-3-fluorobenzoate. The 4-(alkanoylamino)-phenyl compounds used in these reductions are prepared by acylation of the appropriate 4-aminophenyl compounds with suitable acylating agents, such as alkanoyl halides. To prepare the ring-fluorinated 4-(alkylamino)phenyl alkenoic and alkynoic acids it is advantageous to form the corresponding ring-fluorinated alkylchlorimide from the 4-(alkanoylamino)phenyl compounds using phosphorus oxychloride and base, and then reduce the alkylchloroimide moeity to an alkylamino group with sodium borohydride.

A method particularly useful for the introduction of the monosubstituted-amino group into polyfluorinated aromatic compounds is nucleophilic aromatic substitution. An example of this method is the reaction of hexadecylamine (or the anion derived therefrom by treatment with a strong base) with ethyl pentafluorobenzoate to yield ethyl 4-(hexade-

cylamino)-2,3,5,6-tetrafluorobenzoate. In certain instances an amine such as hexadecylamine may be reacted with a benzyne such as that derived from ethyl 4-bromo-3-fluorobenzoate by treatment with sodium amide to yield the 4-(monosubstituted-amino)phenyl compound, in this case ethyl 3-fluoro-4-(hexadecylamino)benzoate.

The 4-(monosubstituted-amino)benzoic, benzoylalkanoic, and phenylalkanoic acids are often prepared from the corresponding p-aminobenzoic, benzoylalkanoic, and phenylalkanoic acids by the sequence involving esterification of the amino acid with ethanol in the presence of boron trifluoride etherate, followed by alkylation of the amino function by the methods above. The free acids are then liberated by hydrolysis of the ester with aqueous alcoholic sodium hydroxide at 80°C for 2-10 hours followed by acidification. The acids obtained by this procedure may be converted to the corresponding metallic cationic salts. For example, the sodium salt may be prepared by reaction of the acid with sodium hydroxide in a mixture of ethanol and water.

Alternatively, the acids may be prepared by hydrolysis of the corresponding nitriles or various amides, imidates or oxazolines. The carboxylic acid moiety may also be generated by oxidation of the corresopnding aldehydes, acetophenones, benzyl alcohols, or toluenes, most often with the use of an amine-protecting group such as trifluoroacetyl or t-butyloxycarbonyl.

The carboxaldehydes may be prepared by several methods among which is alkylation of the corresonding acetal by the several methods among which is alkylation of the corresponding acetal by the methods above followed by hydrolysis of the resulting 4-(monosubstituted-amino)phenyl acetal to the desired aldehyde. Aldehydes may also be prepared by reduction of the appropriate nitriles. For example, treatment of 3-fluoro-4-(hexadecylamino)hydrocinnamonitrile with stannic chloride and anhydrous hydrogen chloride gas, followed by hydrolysis in hot water provides 3-fluoro-4-(hexadecylamino)hydrocinnamaldehyde. These

reductions are also conveniently carried out with hydrides such as diisobutyl aluminum hydride.

The novel esters and amides of formula I-E may readily be prepared by treating a derivative of the corresponding carboxylic acid, such as the acid halide, mixed acid anhydride or activated ester or amide with the appropriate alcohol or amine, respectively. These reactions may be carried out in an inert solvent at a temperature of 50-120°C. for 30 minutes to 18 hours or more. In the case of the acid halide and other cid-forming acylating agents, the reaction is carried out in the presence of an acid scavenger such as diisopropylethylamine; 4-dimethylaminopyridine; pyridine; triethylamine; finely powddered sodium carbonate and the like. A protecting group on the amino nitrogen is used for best results. The simplest protecting group is provided by protonation of the amine to yield an anilinium salt prior to or during formation of the acylating form of the carboxyl group. Acylation of the amino group by carefully selected acyl groups such as carbobenzyloxy, carbo-*t*-butoxy, and trifluoroacetyl provides protection of this group from self-acylation during amide or ester formation. These protecting groups are then removed by catalytic hydrogenation, mild acid treatment, and mild alkali treatment, respectively. Other n-acyl protecting groups such as acetyl and succinoyl may be used and these are removed by conventional methods. Activated esters and amides, useful to synthesize the esters and amides of the present invention, are those containing carboxymethyl, 4-nitrophenyl, N-oxysuccinimide and 1-imidazolyl groups and the like. In certain cases, treatment of the acids with an excess of an appropriate hydroxy-containing substrate in the presence of a Lewis or mineral acid such as boron trifluoride, sulfuric acid or hydrochloric acid affords the corresponding esters. Ordinary esters such as the methyl and ethyl esters are sufficiently reactive to form the amides of the 4-(monosubstituted-amino)benzoic acids and highly reactive amine substrates such as hydroxylamine, hydrazines and certain

alkyl primary amines. With certain kinds of substrates in order to form amides it is necessary to first form the alkali metal or strong organic base salts of these substrates prior to reacting them with the various aforementioned acylating forms of the 4-(monosubstituted-amino)benzoic acids. E.g., the amino alkanecarboxylic and aminoalkanesulfonic acids are zwitterionic and must be converted to their salts, suitably in situ. They may also be used in the form of their esters and then hydrolyzed after amide formation. Certain substrates which are neutral, like the carboxamides, or slightly acidic, like the alkane or arene sulfonamides, are converted to reactive sodium salts by reaction with sodium hydride or other basic reagents.

The α-substituted ring-fluorinated 4-(monosubstituted-amino)acetophenones of formula I-E are prepared by reaction of a derivative of the appropriate benzoic acid, such as 2-fluoro-4(hexadecylamino)benzoyl chloride hydrochloride, with two or more equivalents of the reactive salt of an acidic methylene compound, for example the sodium salt of diethyl malonate. Other benzoic acid derivatives are also suitable for this reaction, such as a ring-flourinated 4-[N-trifluoroacetyl(monosubstituted)amino]benzoyl chloride, a ring-fluorinated 4-[N-trifluoroacetyl(monosubstituted)-amino]benzoyl chloride, a ring-fluorinated 4-[N-tert-butyloxycarbonyl(monosubstituted)amino]benzoyl chloride or a ring-fluorinated methyl 4-(monosubstituted-amino)benzoate ester. In some cases the final step in the preparation of the α-substituted 4-(monosubstituted-amino)acetophenonees is the removal of the nitrogen protecting group. In other cases, hydrolysis of one or more of the ester groups in the acylation product affords an unstable polycarboxylic acid which undergoes decarboxylation to allow the preparation of another acetophenone derivative. For example, the reaction of tert-butyl ethyl [3-fluoro-4-(hexadecylamino)benzoylmalonate with trifluoracetic acid affords ethyl 3-fluoro-4-(hexadecylaminobenzoyl)acetate. In other cases, hydrolysis of one or more of the ester groups allows the preparation of

the corresponding acid derivative. For example, the hydrolysis of ethyl [3-fluoro-4-(hexadecylamino)benzoyl]acetate yields [3-fluoro-4-(hexadecylamino)benzoyl]acetic acid.

An alternative procedure for preparing certain ring-fluorinated α-substituted-4-(monosubstituted-amino)acetophenones is alkylation of the corresponding 4-aminoacetophenone by the methods above. For example, alkylation of methyl 3-(3-fluoro-4-aminobenzoyl)propionate with hexadecyl bromide yields methyl 3-[3-fluoro-4-(hexadecylamino)benzoyl]-propionate The related carboxylic acids are then obtained by hydrolysis. Certain of these acids are particularly useful for the preparation of ring-fluorinated [4-(monosubtituted-amino)phenyl]alkanoic acids by reduction. For example, the Clemmensen or Wolff-Kishner reduction of 3-[3-fluoro-4-(hexadecylamino)benzoyl]propionic acid yields 4-[3-fluoro-4-(hexadecylamino)phenyl]butyric acid.

The ring-fluorinated 4-aminophenylalkenoic acids or 4-(monosubstituted-amino)phenyl]alkenoic acids may be prepared by condensation of the appropriate aldehydes or by dehydration of the corresponding substituted-phenyl-hydroxyalkanoic acid. For example, ethyl 5-[2-fluoro-4-(hexadecylamino)phenyl]-2,4-pentadienoate is obtained by the Wittig reaction of 2-fluoro-4-(hexadecylamino)benzaldehyde with the Wittig reagent, triethyl 4-(phosphonocrotonate. Alternatively, these alkenoic acids are obtained by heating 4-(N-decyl-N-[methyl or acetyl]amino)-3-fluorobenzaldehyde and the like with the sodium salt of the carbanion of ethyl acetate or with a mixture of ethyl acetate, acetic anhydride and potassium acetate. The second method is ullustraed by dehydration of ethyl 3-[(4-alkylamino-2-fluoro)phenyl]-3-hydroxypropionate to yeild ethyl 3-(4-alkylamino-2-fluoro)-cinnamate.

The acetylenic analogs are prepared by dehydrobromination of the side-chain vic-dibrominated alkanoic acid. For example, dehydrobromination of ethyl 3-[(4-alkylamino-2-fluoro)phenyl]-2,3-dibromopropionate, its isomers of N-acyl analogs or of ethyl 3-[(4-alkylamino-2-fluoro)-phenyl]-3-bromoacrylate yields ethyl 3-(4-alkylamino-2-fluoro)phenylpropiolate. The acetylenic acids are also formed from (4-alkylamino-2-fluoro)phenylacetylene metal salts by carboxylation with carbon dioxide. The (4-alkylamino-2-fluoro)phenylacetylenes are also used by N-acylating with t-butyl azidoformate followed by conversion to the lithium acetylide salt and subsequent reaction of the lithium salt with boron trifluoride etherate in tetrahydrofuran at -20°C. to form tris-[4-alkylamino-2-fluoro)phenylethynyl]boranes. The tetrahydrofuran solution of the borane is in turn reacted with ethyl diazoacetate, followed by water to yield ethyl 4-[(4-alkylamino-2-fluoro)phenyl]-butynoate.

The ring-fluorinated 4-(alkylamino)phenylalkanoic acids, amides, or esters are also prepared by catalytic reduction at 1 to 10 atmospheres of hydrogen of the corresponding alkenoic or alkynoic derivatives.

The ring-flourinated 4-(substituted-amino)phenylalkenoic acids and derivatives are prepared by Friedel-Crafts acylation of the N-acyl-N-alkylanilines with the appropriate dicarboxylic acid anhydride or half acid chloride. The 4-(substituted-amino)benzoylalkanoic acids or esters, obtained by this and by other syntheses, may be converted to the 4-(substituted-amino)phenylalkanoic acids by reduction with (a) hydrazine and alkali in diethylene glycol at 140° for 3 hours, (b) zinc amalgam and ethanolic hydrochloric acid at 60° for 5 hours, (c) red phosphorus and hydriodic acid, or (d) ketalization with 1,2-ethanedithiol followed by Raney nickel desulfurization. The amides of the 4-(substituted-amino)phenylalkanoic acids are prepared by heating the corresponding 4-(substituted-amino)phenyl alkyl ketones with aqueous alcoholic ammonium polysulfide followed by hydrolysis to yield the acids with the same number of carbon atoms as the ketone. These acids are also prepared by reacing 4-[N-t-butyloxycarbonyl-N-substituted-amino)phenylmagnesium halides with 2-(3-halopropyl)-2-oxazolines, followed by mild acid removal of 2-oxazolinyl and t-butoxycarbonyl protecting

groups. Similarly, the above Grignard reagent can be reacted with 3-bromotriethylorthopropionate in the presence of dilithiumtetrachlorocuprate to yield the desired acids after removal of the protecting groups from the amino and carboxyl groups.

COMPOUNDS OF FORMULA I-F

The (monosubstituted-amino)furan, pyrrole or thiophene carboxylic acids and analogs of this invention are prepared by reaction of loweralkyl aminoheteroaryl carboxylates with suitable alkylating agents such as alkyl, alkenyl, and alkynyl halides, sulfates, toylates, mesylates or trifluoromethane sulfonates, with or without solvent at 50-150°C. Suitable solvents are loweralkanols chloroform, N,N-dimethylformamide, N,N-dimethylacetamide, diglyme, dimethylsulfoxide, acetonitrile, toluene, benzene, hexamethylphosphoramide and like solvents.

The reaction may be carried out with 2 equivalents of these alkyl aminoheteroaryl carboxylates or with one equivalent of base, such as an unreactive organic base such as diisopropylethylamine or an alkali carbonate or bicarbonate or with a catalytic amount of copper powder when an appropriate halide is used as the alkylating agent. The resulting esters are readily hydrolyzed to the acids in aqueous ethanolic alkali at 25-100°C for 1 to 24 hours.

The loweralkyl N-acetyl-(substituted-amino)heteroaryl carboxylates are prepared by reaction of a loweralkyl-(acetylamino)heteroarylcarboylate with an appropriate alkylating agent in the presence of an equivalent of sodium hydride in an inert solvent such as N,N-dimethylformamide, N N,-dimethylacetamide or diglyme at 50-150°C. These N-acetyl esters are readily hydrolyzed and deacetylated to the acids in boiling aqueous ethanolic dilute alkali or acid.

Alternative methods of preparation are by reductive alkylation of a 4-aminoheteroaryl carboxylate ester which may also be generated in situ by reduction of 4-amino precursors such as a 4-nitro group and the like, or by a

borohydride reduction of the chlorimide formed with phosphorus oxychloride from an acylamino ester For example, an aldehyde plus an aminoheteroaryl carboxylate are reduced under 1-10 atmospheres of hydrogen using a metal catalyst forming the (monosubstituted-amino)hereroaryl carboxyiate ester. Diborane reduction of (alkanoylamino)heteroaryl carboxylate esters such as ethyl 5-hexadecanamido-2-thiophenecarboxylate at room temperature or above for 1-6 hours yields the corresponding (alkylamino)heteroaryl carboxylate esters in this case ethyl 5-hexadecylamino-2-thiophenecarboxylate. The requisite (alkanoylamino)heteroaryl carboxylate esters are obtained by acylation of the corresponding aminoheteroaryl carboxylate ester with an alkanoyl halide or anhydride. Alternatively, the reduction of the (alkanoylamino)heteroaryl carboxylate is carried out by first forming the corresponding alkylchloroimide with phosphorous oxychloride and base, and then reducing the alkylchloroimide moiety to the alkylamino group with sodium borohydride.

Two types of substitution reactions also yield the compounds of the present invention, namely, reaction of esters or amides of didehydroheteroarly carboxylic acids with an alkylamine (or its alkali metal salt) and amination of fluoroheteroaryl carboxylate esters. The former types of reaction is carried out by treating a haloheteroaryl compound with the lithium, potassium or sodium salt of an amine (in excess) such as hexadecylamine in diethyl ether or another aprotic solvent. The latter type comprises reacting hexadecylamine or the like with a fluoroheteroaryl carboxylate ester at elevated temperatures.

The alkyl halides, acyl halides, and alkyl amines required for the above-described alkylation, acylation, and displacement reactions, respectively, may be prepared from the appropriate carboxylic acids or alcohols which are described in the chemical literature, by methods well-known to those skilled in the art.

The novel compounds of Formula I-F where Z is

other than COOH may be readily prepared by treating an acid halide mixed acid anhydride, or activated ester or amide of a compound of Formula I-F with an appropriate hydroxy compound, amine, or salt of a carboxamide or sulfonamide. These reactions are preferably carried out in an inert solvent at a temperature of 5-125°C. for a period of time from about 30 minutes to 18 hours or more. In the case of the acid halide and other acid-forming acylating agents, the reaction is carried out in the presence of an acid scavenger such as diisopropylethylamine, 4-dimethylaminopyridine, pyridine, trfethylamine, finely powdered sodium carbonate, and the like. The acid halide and anhydride starting materials may be obtained from the corresponding (monosubstituted-amino)heteroaryl carboxylic acids by methods which are well-known in the art or described herein. However, a protecting group on the amino nitrogen is used for best results. The simplest protecting group is provided by protonation of the amine to give an ammonium salt prior to or during formation of the acylating agent. Acylation of this amino group by carefully selected acyl groups such as carbobenzyloxy, carbo-t-butoxy, and trifluoroacetyl provides protection of this group from self-acylation during amide or ester formation. These protecting groups are then removed by catalytic hydrogenation, mild acid treatment and mild alkali treatment, respectively.

Activated esters or amides, which are used to synthesize the esters of the present invention, are carboxymethyl, 4-nitrophenyl, N-oxysuccinimide, 1-imidazolyl and the like. In certain cases, treatment of acids or ordinary esters such as methyl or ethyl with an excess of an appropriate hydroxy-containing substrate in the presence of a Lewis or mineral acid such as boron trifluoride, sulfuric acid or hydrochloric acid is sufficient to convert the (monosubstitutedamino)heteroaryl carboxylic acids to the appropriate esters.

With certain kinds of substrates for ester formation, it is necessary to form the alkali metal or strong

organic base salts of the (monosubstituted-amino)heteroaryl carboxylic acid in order to react them with 2,3-dihydroxypropyl iodide, ethyl chloroacetate and the like. Other esters are prepared from the acids themselves by reaction with diazoalkanes ethyl diazoacetate or the like

The (monosubstituted-amino)heteroaryl carboxylic acids and derivatives are prepared by de-acylation of the corresponding N-tri-fluoroacetyl ester or amide by reacting with an alkali hydroxide such as sodium or potassium hydroxide in a lower alkanol, water or an aqueous lower alkanol at 5-50°C. Alternatively, certain of these compounds may be prepared by de-acylation of the N-carbo-t-butoxy derivative and the like with mineral acids such as hydrochloric or hydrobromic acid, preferably in glacial acetic acid at 0°C. to 50°C. Also, they are often prepared by removal of the carbobenzyloxy protecting group from the amino nitrogen atom by means of mild catalytic hydrogenation or by treatment with a mineral acid such as hydrobromic acid in glacial acetic acid.

With certain kinds of substrates for amide formation, it is necessary to form the alkali metal or strong organic base salts of these substrates in order to react them with the various aforementioned acylating forms of the (mono-substituted-amino)heteroaryl carboxylic acids. The aminoalkanecarboxylic and aminoalkanesulfonic acids are zwitterionic and must be converted to their cationic salts, suitably in situ. They may also be used in the form of their esters and then hydrolyzed after amide formation. Certain substrates, which are neutral like the carboxamides or slightly acidic like the alkane or arene sulfonamides, are converted to reactive sodium salts by reaction with sodium hydride or other basic reagents.

Alternatively, the free acids may be prepared by hydrolysis of the corresponding nitriles or various amides, imidates or oxazolines. The carboxylic acid moiety may also be generated by oxidation of the corresponding aldehydes, acetophenones, benzyl alcohols, or alkylbenzenes, most often

with the use of an amine-protecting group such as trifluoroacetyl or t-butyloxycarbonyl. The carboxaldehyde moiety may be generated by reduction of the corresponding (monosubstituted-amino) heteroaryl carbonitrile with diisobutylaluminum hydride.

COMPOUNDS OF FORMULA I-G

Many of the novel 4-[(carboxy- and sulfamyl-substituted alkyl)amino]benzoic acids, esters and amides of the present invention are prepared by alkylation of a 4-aminobenzoic acid, ester, or amide with the appropriate alkylating agent followed, if necessary, by chemical transformation of the substituent on the alkylamino group. Alkylating agents especially for these reactions include haloalkanoic acids, esters, and amides, haloalkanesulfonic acids and amides, and haloalkanonitriles. Many of these alkylating agents are obtained from the corresponding hydroxy compounds, for example, the reaction of 16-hydroxyhexadecanoic acid with hydrogen bromide affords 16-bromohexadecanoic acid. In other cases, these alkylating agents are obtained from the haloalcohols, for example, the reaction of 11-bromoundecanol with potassium cyanide affords 11-hydroxyundecanonitrile which in turn is converted to 11-bromoundecanonitrile by reaction with hydrogen bromide. Similarly, the reaction of 11-bromoundecanol with sodium sulfite yields 11-hydroxyundecanesulfonic acid which is converted to 11-bromoundecanesulfonic acid by reaction with hydrogen bromide. Other haloalkanesulfonic acids are prepared form the corresponding mercaptans, for example, oxidation of 6-bromohexanethiol (obtained by reaction of 6-bromo-1-methanusulfonyloxyhexane with potassium thioacetate followed by hydrolysis with sodium hydroxide) with nitric acid affords 6-bromohexanesulfonic acid. Still other haloalkanesulfonic acids are prepared by the addition of bisulfite salts to olefins in the presence of free-radical initiators. An example of this type of reaction is the addition of sodium bisulfite to 8-bromo-1-octene in the presence of dibenzoyl peroxide to yield 8-bromooctanesulfonic acid. These carboxylic and

sulfonic acids may be converted to the desired amides or esters by conventional methods. The compounds of this invention are also obtained by addition of ethyl p-aminobenzoate to $\alpha$-unsaturated alkanoic acids, amides or esters such as crotonic acid, ethyl acrylate, and 2-hexenoic amide and to $\alpha$-unsaturated alkanesulfonic acids or amides such as ethylenesulfonic acid and 2-butylenesulfonamide.

The alkylation of 4-aminophenylcompounds is carried out with the above-mentioned halides or alternatively with methanesulfonates, tosylates, trifluoromethanesulfonates or the like wiht or without solvent at 50-150°C. Suitable solvents are loweralkanols, chloroform, N,N-dimethylformamide, N,N-dimethylacetamide, diglyme, dimethylsulfixide, acetonitrile, toluene, benzene hexamethylphosphoramide and the like. The reaction may be carried out with an equivalent of base such as an alkali carbonate or bicarbonate, or with a catalytic amount of copper powder when alkyl halides are used as the alkylating agent. Alternatively, alkylation of the sodium salt (formed with sodium hydride of either the amino group of a 4-aminophenyl compound or the anilide moiety of a 4-(acetylamino)phenyl comound yields the desired alkylation product or an N-acetyl derivative thereof. Other 4-aminophenyl compounds useful in these alkylation reactions, or in the subsequently described acylation reactions, are, for example, 4-aminobenzonitrile, p-toluidine, and the acetal of 4-aminobenzaldehyde. In these cases, the desired alkylation product is obtained by a subsequent oxidative or hydrolytic transformation of the methyl, nitrile, or acetal group.

Two types of substitution reactions also yield the 4-(substituted-alkyl amino)benzoate esters, firstly, reaction of esters of 3,4-didehydrobenzoic acid with a (substituted-alkyl)amine (or its alkali metal salt). Friedel-Crafts acylation of an N-(substitutedalkyl)aniline or N-acyl-N-(substituted-alkyl)aniline yields certain intermediates thereto. The former type of reaction is carried out by treating a 4-halobenzoate ester such as phenyl 4-bromobenzoate with the

lithium, potassium or sodium salt of an alkylamine such as 16-carboethoxyhexadecylamine in diethyl ether or other aprotic solvent. The second method comprises reacting 16-carboethoxyhexadecylaniline and the like or its N-acetyl derivative with a carboalkoxy chloride and anhydrous aluminum chloride in dry diethyl ether, halocarbon or hydrocarbon medium.

The 4-[(substituted-alkyl)amino]benzoic acids may be obtained by hydrolysis of the correspondesters or amides in boiling aqueous ethanolic alkali, followed by acidification. Alternatively, these benzoic acids may be isolated as their alkali metal salts.

The novel esters and amides of Formula I-G may readily be prepared by treating a derivative of the corresponding carboxylic acid, such as the acid halide, mixed acid anhydride or activated ester or amide with the appropriate alcohol or amine, respectively. These reactions may be carried out in an inert solvent at a temperature of 50-150°C. for 30 minutes to 18 hours or more. In the case of the acid halide and other acid-forming acylating agents, the reaction is carried out in the presence of an acid scavenger such as diisopropylethylamine; 4-dimethylaminopyridine; pyridine; triethylamine; finely powdered sodium carbonate and the like. A protecting group on the amino nitrogen is used for best results. The simplest protecting group is provided by protonation of the amine to yield an anilinium salt prior to or during formation of the acylating form of the carboxyl group. Acylation of the amino group by carefully selected acyl groups such as carbobenzyloxy carbo-t-butoxy, and trifluoroacetyl provides protection of this group from self-acylation during amide formation. These protecting groups are then removed by catalytic hydrogenation, mild acid treatment and mild alkali treatment, respectively. Other N-acyl protecting groups such as acetyl and succinoyl may be used and these are removed by conventional methods. Activated esters and amides, useful to synthesize the esters and amides of the present invention, are those

containing carboxymethyl, 4-nitrophenyl, N-oxysuccinimide and 1-imidazolyl groups and the like. In certain cases, treatment of the acids with an excess of an appropriate hydroxy-containing substrate in the presence of a Lewis or mineral acid such as boron trifluoride, sulfuric acid or hydrochloric acid affords the corresponding esters. Ordinary esters such as the methyl and ethyl esters are sufficiently reactive to form the amides of the 4-[(substituted-alkyl)-amino]benzoic acids and highly reactive amine substrates such as hydroxylamine, hydrazines and certain alkyl primary amines. In order to form amides from certain kinds of substrates, it is necessary first to form the alkali metal or strong organic base salts of these substrates prior to reacting them with the various aforementioned acylating forms of the 4-[(substituted-alkyl)amino]phenyl compounds. For example, aminoalkanecarboxylic and aminoalkanesulfonic acids are zwitterionic and must be converted to their salts, suitable in situ. They may also be used in the form of their esters and then hydrolyzed after amide formation. Certain substrates which are neutral, like the carboxamides, or slightly acidic, like the alkane or arene sulfonamides, are converted to acylatable sodium salts by reaction with sodium hydride or other basic reagents.

The carboxaldehydes may be prepared by several methods, among which is alkylation of the corresponding acetal by the methods above followed by hydrolysis of the resulting 4-(substituted-amino)phenyl acetal to the aldehyde. Aldehydes may also be prepared by reduction of appropriate nitriles. For example, treatment of 4-(16-carboxyhexadecyl-amino)benzonitrile is conveniently carried out with diisobutyl aluminum hydride.

COMPOUNDS OF FORMULA I-H

Many of the polyfluorinated-alkyl halides required as intermediates for the syntheses of the novel 4-(polyfluoroalkylamino)phenyl compounds are prepared from the corresponding hydroxy acids, for example, reaction of 16-hydroxyhexadecanoic acid with hydrogen bromide followed

by sulfur tetrafluoride affords 15-(trifluoromethyl)pentadecyl bromide. Certain of the polyfluorinated-alkyl halides or methanesulfonates are prepared from the corresponding alcohols which in turn are obtained from the appropriate polyfluorinated carboxylic acids, for example, reduction of 11-(pentafluoroethyl)undecanoic acid with diborane or a metal hydride affords 11-(pentafluoroethyl)undecanol which may then be converted to 11-(pentafluoroethyl)undecyl bromide by reaction with hydrogen bromide or alternatively to 1-(methanesulfonyloxy)-11-(pentafluoroethyl)undecane by reaction with methanesulfonyl chloride. Other polyfluorinated-alkyl halides are prepared by the reaction of sulfur tetrafluoride with ketones, for example, reaction of 17-bromo-11-oxoheptadecanoic acid (obtained by the peroxidecatalyzed addition of hydrogen bromide to 11-oxo-16-heptadecenoic acid) with sulfur tetrafluoride affords 16-(trifluoromethyl)-11,11-difluorohexadecyl bromide. Similarly, 15-bromo-2-pentadecanone (obtained by alkylation of 14-bromotetradecanoyl chloride with dimethyl cadmium) yields 14,14-difluoropentadecyl bromide.

Many of the novel 4-(polyfluoroalkylamino)phenyl compounds of Formula I-H may be prepared by reaction of the appropriate 4-aminophenyl compound with a suitable alkylating agent such as an alkyl halide, sulfate, tosylate, or trifluoromethanesulfonate with or without a solvent at 30°C. to 150°C. Appropriate 4-aminophenyl compounds are, for example, ethyl 4-aminobenzoate; 2,3-dihydroxypropyl 4-aminobenzoate; phenyl 4-aminobenzoate; 1-(4-aminobenzoyl)pyrrolidine; and ethyl 4-(4-aminophenyl)butyrate. Suitable solvents are loweralkanols, N,N-dimethoxyethane, acetonitrile, toluene, benzene, hexamethylphosphoramide and the like. The reaction may be carried out with two equivalents of the 4-aminophenyl compound or with one equivalent of the compound plus one equivalent of a base such as an alkali carbonate or bicarbonate or an unreactive organic base such as diisopropylethylamine or alternatively with a catalytic amount of copper powder when an alkyl halide is used as the alkylating agent.

Similarly, alkylation of the sodium salt (formed with sodium hydride) of either the amino group of a 4-aminophenyl compound or the anilide moiety of a 4-(acetylamino)phenyl compound yields the novel 4-(polyfluoroalkylamino)phenyl compounds or an N-acetyl derivative thereof. Removal of the N-acetyl group by conventional hydrolytic methods affords the desired 4-(polyfluoroalkylamino)phenyl compounds.

Alternative methods of preparation of these compounds are by reductive alkylation of a 4-aminophenyl compound, which may be generated <u>in situ</u> by reduction of a 4-aminophenyl precursor such as a 4-nitrophenyl compound and the like or by a metal hydride reduction of a 4-(acylamino)-phenyl compound. For example, 15-(trifluoromethyl)pentadecanal or another carbonylalkane and 4-aminobenzoic acid are reduced under 1-10 atmospheres of hydrogen using an activated metal catalyst or with a metal hydride such as sodium borohydride forming 4-[ 15-(trifluoromethyl)pentadecylamino]benzoic acid and the like. Diborane reduction of 4-(polyfluoroalkanoylamino)phenyl compounds such as ethyl 4-[15-(trifluoromethyl)pentadecanoylamino]benzoate at room temperature or above for 1-6 hours yields the corresponding 4-(polyfluoroalkylamino)phenyl compounds such as ethyl 4-15-(trifluoromethyl)pentadecylamino benzoate. The 4-(polyfluoroalkanoylamino)phenyl compounds used in these reductions are prepared by acylation of the appropriate 4-aminophenyl compounds with suitable acylating agents, such as polyfluoroalkanoyl halides. To prepare the 4-(polyfluoroalkylamino)-phenyl alkenoic and alkynoic acids it is advantageous to form the corresponding polyfluoroalkylchloroimide from the 4-(polyfluoroalkanoylamino)phenyl compounds using phosphorus oxychloride and base, and then reduce the polyfluoroalkylchloroimide moiety to a polyfluoroalkylamino group with sodium borohydride.

A method useful for the introduction of the polyfluoroalkylamino group into aromatic compounds is nucleophilic aromatic substitution. An example of this method is the reaction of 15-(trifluoromethyl)pentadecylamine

(or the anion derived threrfrom by treatment with a strong base) with ethyl 4-fluorobenzoate to yeild ethyl 4-[15-(trifluoromethyl)pentadecylamino]benzoate. In certain instances an amine such as 15-(trifluoromethyl)pentadecylamine may be reacted with a benzyne such as that derived from ethyl 4-bromobenzoate by treatment with sodium amide to yield the 4-(polyfluoroalkylamino)-phenyl compound, in this case ethyl 4-[15-(trifluoro methyl)pentadecylamino]benzoate.

The 4-(polyfluoroalkylamino)benzoic, benzoylalkanoic, and phenylalkanoic acids are often prepared from the corresponding p-aminobenzoic, benzoylalkanoic, and phenylalkanoic acids by the sequence involving esterification of the amino acid with ethanol in the presence of boron trifluoride etherate, followed by alkylation of the amino function by the methods above. The free acids are then liberated by hydrolysis of the ester with aqueous alcoholic sodium hydroxide at 80°C for 2-10 hours followed by acidification. The acids obtained by this procedure may be converted to the corresponding metallic cationic salts. For example, the sodium salt may be prepared by reaction of the acid with sodium hydroxide in a mixture of ethanol and water.

Alternatively, the acids of Formula I-H may be prepared by hydrolysis of the corresponding nitriles or various amides, imidates or oxazolines. The carboxylic acid moiety may also be generated by oxidation of the corresponding aldehydes, acetophenones, benzyl alcohols, or toluenes, most often with the use of an amine-protecting group such as trifluoroacetyl or t-butyloxycarbonyl.

The carboxyldehydes may be prepared by several methods among which is alkylation of the corresponding acetal by the methods above followed by hydrolysis of the resulting 4-(polyfluoroalkylamino)phenyl acetal to the desired aldeyde. Aldehydes may also be prepared by reduction of the appropriate nitriles. For example, treatment of 4-[15-(trifluoromethyl)pentadecylamino]benzonitrile with stannic chloride and anhydrous hydrogen chloride gas,

followed by hydrolysis in hot water provides 4-[15(trifluoromethyl)pentadecylamino]benzaldehyde. These reductions are also conveniently carried out with hydrides such as di*iso*butyl aluminum hydride.

The novel esters and amides of Formula I-H may readily be prepared by treating a derivative of the corresponding carboxylic acid, such as the acid halide, mixed acid anhydride or activated ester or amide with the appropriate alcohol or amine, respectively. These reactions may be carried out in an inert solvent at a temperature of 50-125°C. for 30 minutes to 18 hours or more. In the case of the acid halide and other acid-forming acylating agents, the reaction is carried out in the presence of an acid scavenger such as di*iso*propylethylamine; 4-dimethylaminopyridine; pyridine; triethylamine; finely powdered sodium carbonate and the like. A protecting group on the amino nitrogen is used for best results. The simplest protecting group is provided by protonation of the amine to yield an anilinium salt prior to or during formation of the acylating form of the carboxyl group. Acylation of the amino group by carefully selected acyl groups such as carbobenzyloxy, carbo-*t*-butoxy, and trifluoroacetyl provides protection of this group from self-acylation during amide or ester formation. These protecting groups are then removed by catalytic hydrogenation, mild acid treatment, and mild alkali treatment, respectively. Other N-acyl protecting groups such as acetyl and succinoyl may be used and these are removed by conventional methods. Activated esters and amides, useful to synthesize the esters and amides useful to synthesize the esters and amides of the present invention, are those containing carboxymethyl, 4-nitrophenyl, N-oxysuccinimide and 1-imidazolyl groups and the like. In certain cases, treatment of the acids with an excess of an appropriate hydroxy-containing substrate in the presence of a Lewis or mineral acid such as boron trifluoride, sulfuric acid or hydrochloric acid affords the corresponding esters. Ordinary esters such as the methyl and ethyl esters are sufficiently

reactive to form the amides of the 4-(polyfluoroalkylamino)-benzoic acids and highly reactive amine substrates such as hydroxylamine, hydrazines and certain alkyl primary amines. With certain kinds of substrates in order to form amides it is necessary to first form the alkali metal or strong organic base salts of these substrates prior to reacting them with the various aforementioned acylating forms of the 4-(polyfluoroalkylamino)benzoic acids. For example, the aminoalkanecarboxylic and aminoalkanesulfonic acids are zwitterionic and must be converted to their salts, suitably *in situ*. They may also be used in the form of their esters and then hydrolyzed after amide formation. Certain substrates which are neutral, like the carboxamides, or slightly acidic, like the alkane or arene sulfonamides, are converted to reactive sodium salts by reaction with sodium hydride or other basic reagents.

The ω-substituted 4-(polyfluoroalkylamino)aceto-phenones of the invention are prepared by reaction of a derivative of the appropriate benzoic acid, such as 4-[15-(trifluoromethyl)pentadecylamino]benzoyl chloride hydrochloride, with two or more equivalents of the reactive salt of an acidic methylene compound, for example the sodium salt of diethyl malonate, Other benzoic acid derivatives are also suitable for this reaction, such as 4-[N-trifluoroacetyl-(polyfluoroalkyl)amino]benzoyl chloride, a 4-[N-trifluoroacetyl(polyfluoroalkyl)amino]benzoyl chloride, a 4-[N-*tert*-butyloxycarbonyl(polyfluoroalkyl)amino]benzoyl chloride or a methyl 4-(polyfluoroalkylamino)benzoate ester. In some cases the final step in the preparation of the -substituted 4-(polyfluoroalkylamino)acetophenones is the removal of the nitrogen protecting group. In other cases, hydrolysis of one or more of the ester groups in the acylation product affords an unstable polycarboxylic acid which undergoes decarboxylation to allow the preparation of another acetophenone derivative. For example, the reaction of *tert*-butyl ethyl 4-[15-(trifluoromethyl)pentadecylamino]benzoylmalonate with trifluoroacetic acid affords ethyl 4-[15-(trifluoromethyl)pentadecylamino]benzoylacetate. In other cases, hydrolysis of one or more of the ester groups allows the preparation of the corresponding acid derivative. For example, the hydrolysis of ethyl 4-[15-(trifluoromethyl)-pentadecylamino]benzoylacetate yields 4-[15-(trifluoromethyl)-pentadecylamino]benzoyl acetic acid.

An alternative procedure for preparing certain α--substituted-4-(polyfluoroalkylamino)acetophenones is alkylation of the corresponding 4-aminoacetophenone by the methods above. For example, alkylation of the methyl 3-(4-aminobenzoyl)-proprionate with 15-(trifluoromethyl)-bromide yields methyl 3-{4-[15-(trifluoromethyl)pentadecyl-amino]benzoyl} propionate. The related carboxylic acids are then obtained by hydrolysis. Certain of these acids are particularly useful for the preparation of [4-(polyfluoroalkylamino)phenyl]alkanoic acids by reduction. For example, the Clemmensen or Wolff-Kishner reduction of 3- 4-[15-(tri-fluoromethyl)pentadecylamino]benzoyl propionic acid yields 4-4-[15-(trifluoromethyl)pentadecylamino]phenyl butyric acid.

The [4-(polyfluoroalkylamino)phenyl]alkenoic acids may be prepared by condensation of the appropriate aldehydes or by dehydration of the corresponding substituted-phenyl-hydroxylakanoic acids. For example, ethyl 5-{4-[15-(tri-fluoromethyl)pentadecylamino)phenyl}-2,4-pentadienoate is obtained by the Wittig reaction of 4-[15-(trifluoromethyl)-pentadecylamino]benzaldehyde with the Wittig reagent, triethyl 4-phosphonocrotonate. Alternatively, these alkenoic acids are obtained by heating 4-[N-polyfluoroalkyl-N-(methyl or acetyl)amino]benzaldehydes with the sodium salt of the carbanion of ethyl acetate or with a mixture of ethyl acetate, acetic anhydride and potassium acetate. The second method is illustrated by dehydration of ethyl 3-{4-[15-trifluoromethyl)pentadecylamino]phenyl}-3-hydroxypropionate to yield ethyl 4-[15-(trifluoromethyl)pentadecyl-amino]cinnamate.

The acetylenic analogs are propared by dehydrobromination of the side-chain vic-dibrominated alkanoic acid. for example, dehydrobromination of ethyl 3-{4-[15-(trifluoromethyl)pentadecylamino]phenyl}-2,3-dibromopropionate, its isomers or N-acyl analogs yields ethyl 3- 4-[15-(trifluoromethyl)pentadecylamino]propiolate. The acetylenic acids are also formed from (4-polyfluoroalkylamino)phenylacetylene methyl salts by carboxylation with carbon dioxide. The 4-(polyfluoroalkylamino)phenylacetylenes are also used by N-acylating with t-butyl azidoformate followed by conversion to the lithium acetylide salt and subsequent reaction of the lithium salt with boron trifluoride etherate in tetrahydrofuran at -20°C. to form tris-[4-(polyfluoroalkylamino)phenylethynyl]boranes. The tetrahydrofuran solution of the borane is in turn reacted with ethyl diazoacetate, followed by water to yield ethyl 4-[4-(polyfluoroalkylamino)-phenyl]butynoate.

The 4-(polyfluoroalkylamino)phenylalkanoic acids, amides, or esters are also prepared by catalytic reduction at 1 to 10 atmospheres of hydrogen to the corresponding alkenoic or alkynoic derivatives.

The 4-(polyfluoroalkylamino)phenylalkenoic acids and derivatives are prepared by Friedel-Crafts acylation of the N-acyl-N-alkylanilines with the appropriate dicarboxylic acid anhydride or half acid chloride. The 4-(polyfluoroalkylamino)benzoylalkanoic acids or esters, obtained by this and by other syntheses, may be converted to the 4-(polyfluoroalkylamino)phenylalkanoic acids by reduction with (a) hydrazine and alkali in diethylene glycol at 140°C for 3 hours, (b) zinc amalgam and ethanolic hydrochloric acid at 60°C for 5 hours, (c) red phosphorus and hydriodic acid, or (d) ketalization with 1,2-ethanedithiol followed by Raney nickel desulfurization. The amides of the 4-(polyfluoroalkylamino)-phenylalkanoic acids are prepared by heating the corresponding 4-(polyfluoroalkylamino)phenyl alkyl ketones with aqueous alcoholic ammonium polysulfide followed by hydrolysis to yield the acids with the same number of carbon atoms as the ketone. These acids ae also prepared byreacting 4-(N-t-butyloxycarbonyl-N-polyfluoroalkylamino)phenylmagnesium halides with 2-(3-halopropyl)-2-oxaolines, followed by mild acid removal of 2-oxazolinyl and t-butoxycarbonyl protecting groups. Similarly, the above Grignard reagent can be reacted with 3-bromotriethylorthopropionate in the presence of dilithiumtetrachlorocuprate to yield the desired acids after removal of the protecting groups from the amino and carboxyl groups.

Certain derivatives $(-\overset{\overset{R}{|}}{N}-)$ of the aminobenzoyl nitrogen atom are useful for providing greater solubility, more uniform and reliable intestinal absorption, and for a certain degree of modification of the pharmacology of the compounds of the present invention. Some of these derivatives can be converted to the corresponding N-H forms by the acidity of the stomach or the alkalinity of the small intestine. Others are converted by metabolic processes. The methyl and carboxymethyl derivatives and the like are prepared by the alkylation, reductive alkylation, and acylamino reduction methods above. Derivatives such as the acetyl and succinyl compounds may be prepared using acetyl chloride, acetic anhydride, succinic anyhdride, etc. in the presence of pyridine, triethylamine or the like at temperatures moderate enough to avoid acylation of the amide moiety. The 1-(sodium sulfo)alkyl derivatives are obtained by reaction of the 4-(substituted amino)phenyl compound with sodium bisulfite and an aliphatic aldehyde, a polyhydroxyaldehyde such as glyceraldehyde or glucose, or cinnamaldehyde in a mixed organic-aqueous medium. In the case of cinnamaldehyde, the di-sulfonate salts result from addition of the bisulfite to the carbon-nitrogen double bond of the anil intermediate as well as to the carbon-carbon double bond of cinnamaldehyde itself.

The novel compounds of the present invention are not only potent hypolipidemic agents but also prevent or diminish the formation or enlargement of arterial plaques in mammals when administered in amounts ranging from about one

milligram to about 250 mg. per kilogram of body weight per day. A preferred dosage regimen for optimum results would be from about 5 mg. to about 100 mg. per kilogram of body weight per day, and such dosage units are employed that a total of from about 0.35 gram to about 7.0 grams of the active compound, for a subject of about 70 kg. of body weight, are administered in a 24 hour period. This dosage regimen may be adjusted to provide the optimum theraputic response. For example, several divided doses may be administered daily or the dose may be proportionally reduced as indicated by the exigencies of the therapeutic situation. A decided practical advantage of this invention is that the active compound may be administered in a convenient manner by the oral route. The compounds of the present invention exert a more powerful hypocholestermic and antiatherosclerotic effect than the aforementioned adjuvants and synthetic medicaments. It is not known how these novel compounds operate in the blood serum and no theory of why these compounds so operate is advanced. It is not intended that the present invention should be limited to any particular mechanism of action of lowering serum lipids or of ameliorating atherosclerosis, or be limited to compounds acting by only one mechanism.

The active compounds of the present invention may be orally administered, for example, with an inert diluent or with an assimilable edible carrier, or they may be enclosed in hard or soft shell gelatin capsules, or they may be compressed into tablets, or they may be incorporatd directly with the food of the diet. For oral therapeutic administration, the active compounds may be incorporated with excipients and used in the form of ingestible tablets, buccal tablets, troches, capsules, elixirs, suspensions, syrups, wafers, and the like. Such compositions and preparations should contain at least 0.1% of active compound. The percentage of the compositions and preparations may, of course, be varied and may conveniently be between about 2 to about 60% of the weight of the unit. The amount of active

ingredient in such therapeutically useful compositions is such that a suitable dosage will be obtained. Preferred compositions or preparations according to the present invention are prepared so that an oral dosage-unit form contains between about 50 and 250 milligrams of active compound.

The tablets, troches, pills, capsules and the like may also contain the following: a binder such as gum tragacanth, acacia, corn starch or gelatin; excipients such as dicalcium phosphate, a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage-unit form is a capsule, it may contain, in addition to materials of the above type, a liquid carrier. Various other materials may be present as coatings or to otherwise modify the physical form of the dosage unit. For instance, tablets, pills or capsules may be coated with shellac, sugar or both. A syrup or elixir may contain the active compound, sucrose as a sweetening agent, methyl an propyl parabens as preservatives, a dye, and flavoring such as cherry or orange flavor. Of course, any material used in preparing any dosage-unit form should be pharmaceutically pure and substantially non-toxic in the amounts employed. In addition, the active ingredients may be incorporated into sustained-release preparations and formulations.

The active ingredients of the therapeutic composition aspect of the present invention are chosen from the compounds of Formula I as defined above, and the pharmaceutically acceptable acid-addition and cationic salts thereof; and mixtures thereof.

The present invention also contemplates a method of inducing regression of atherosclerotic lesion development in a mammal which comprises administering to said mammal an effective lesion-regressive amount of a compound of Formula I, as defined above, and the pharmaceutically acceptable

acid-addition and cationic salts thereof; and mixtures thereof.

The present invention also contemplates a method of inhibiting atherosclerotic lesion development in a mammal which comprises administering to said mammal an effective lesion-development inhibiting amount of a compound of Formula I, as defined above, and the pharmaceutically acceptable acid-addition and cationic salts thereof; and mixtures thereof.

The invention will be described in greater detail in conjunction with the following specific Examples.

## EXAMPLE 1

Preparation of p-[(cyclohexylmethyl)amino]benzoic acid

A solution of 6 g. of cyclohexylmethyl bromide and 11.19 g. of ethyl p-aminobenzoate in 30ml. of hexamethylphosphoramide is heated in an oil bath for 20 hours. The solution is poured into ice-cold water and extracted several times with diethyl ether. The combined ether extracts are washed with water, dried with anhydrous magnesium sulfate, and evaporatee to dryness under reduced pressure to furnish ethyl p-cyclohexylmethylaminobenzoate as an oil.

The oil is dissolved in 250 ml. of ethanol:water (9:10) containing 9 g. of potassium hydroxide and the resulting solution is stirred at the reflux temperature for 3 hours. After chilling, the mixture is acidified with concentrated hydrochloric acid, diluted with water, and extracted twice with methylene chloride. The combined extracts are washed with water, dried with anhydrous magnesium sulfate, and evaporated to dryness under reduced pressure to furnish p-[(cyclohexylmethyl)amino]benzoic acid.

## EXAMPLES 2-108

Treatment of the indicated halide starting materials set forth in Table I below with ethyl p-aminobenzoate followed by saponification according to Example 1 is productive of the corresponding p-[substituted amino]benzoic acids listed in Table I.

TABLE I

| Example | Starting Material | Product |
|---|---|---|
| 2 | 1-iodomethyl-2-methyl cyclopentane Chem. Abst. 67,9041y | p-[(2-methylcyclopenthyl) methylamino]benzoic acid. |
| 3 | α-bromomethyl cyclopentane Chem. Abst. 66, 18472c | p-[(cyclopentyl)methylamino] benzoic acid |
| 4 | 1-bromomethyl-4-methylcyclohexane Chem. Abst. 70,2934b | p-[(4methylcyclohexyl)methylamino]-benzoic acid |

| Example | Starting Material | Product |
| --- | --- | --- |
| 5 | 1-chloromethyl-2-methyl-cyclohexane Chem. Abst. 68,88671h | p-[(2-methylcyclo-hexyl)methylamino]ben-zoic acid |
| 6 | 1-(1,2-dimethylcyclo-hexyl)-2-chloropropane Chem. Abst. 73,14272j | p-[1-(1,2-dimethyl-cyclohexyl)-2-propyl amino]benzoic acid |
| 7 | 1-(1,3-dimethylcyclo-hexyl)-2-chloropropane Chem. Abst. 73,14272j | p-[1,3-dimethyl-cyclohexyl)-2-propyl-amino]benzoic acid |
| 8 | 1-(1,4-dimethylcyclo-hexyl)-2-chloropropane Chem. Abst. 73,14171j | p-[1-(1,4-dimethyl-cyclohexyl)-2-propyl-amino[benzoic acid |
| 9 | α-bromomethyl cyclo-heptane Chem. Abst. 51,1049e | p-(cycloheptylmethyl-amino)benzoic acid |
| 10 | α-bromomethylcyclo-octane chem. Abst. 68,104595t | p-(cyclooctylmethyl-amino)benzoic acid |
| 11 | α-chloroethylcyclo-pentane Chem. Abst. 72,110862b | p-(1-cyclopentylethyl-amino)benzoic acid |
| 12 | 1-bromo-2-cyclo-pentylbutane | p-(2-cyclopentylbutyl-amino)benzoic acid |
| 13 | 1-bromo-2-cyclo-pentylhexane Ref. A | p-(2-cyclopentylhexyl-amino)benzoic acid |
| 14 | 2-chloroethylcyclo-hexane Chem. Abst. 68,86671h | p-(2-cyclohexylethyl-amino)benzoic acid |
| 15 | 1-(2-bromoethyl)-1-ethylcyclohexane Chem. Abst. 70,57233c | p-[2-1-(ethylcyclo-hexyl)ethylamino] benzoic acid |
| 16 | 1-bromo-2-(3-methyl-cyclohexyl)butane | p-[2-(3-methylcyclo-hexyl)butylamino] benzoic acid |
| 17 | 1-bromo-2-cyclohexyl-pentane Ref. A | p-[(2-cyclohexyl)pen-tylamino]benzoic acid |
| 18 | 1-bromo-2-cyclohexyl-butane Ref. A | p-[(2-cyclohexyl)-butylamino]benzoic acid |

| Example | Starting Material | Product |
|---|---|---|
| 19 | 1-bromo-2-cyclohexyl-propane Ref. A | p-[(2-cyclohexyl)prop-ylamino]benzoic acid |
| 20 | 1-(2-chloroethyl)-2,3-dimethylcyclohexane Chem. Abst. 69,56053n | p-[2-(2,3-dimethylcyclo-hexyl)ethylamino]-benzoic acid |
| 21 | 1-(2-chloroethyl)-3,5-dimethylcyclohexane Chem. Abst. 69,56053n | p-[2-(3,5-dimethylcyclo-hexyl)ethylamino]-benzoic acid |
| 22 | 2-(2-chloroethyl)-1,4-dimethylcyclohexane Chem. Abst. 69,56053n | p-[2-(2,5-dimethylcyclo-hexyl)ethylamino]-benzoic acid |
| 23 | 1-(2-chloroethyl)-2-ethylcyclohexane Chem. Abst. 69,56053n | p-[2-(2-ethylcyclo-hexyl)ethylamino]-benzoic acid |
| 24 | 1-(2-chloropropyl)-3-methylcyclohexane Chem. Abst. 67,53405a | p-[1-(3-methylcyclo-hexyl)-2-propylamino]-benzoic acid |
| 25 | 1-(2-bromoethyl)-1-methylcyclohexane Chem. Abst. 72,132133s | p-[2-(1-methylcyclo-hexyl)ethylamino]-benzoic acid |
| 26 | 1-(2-chloroethyl)-2-methylcyclohexane Chem. Abst. 69,56053n | p-[2-(2-methylcyclo-hexyl)ethylamino]-benzoic acid |
| 27 | 1-(2-chloroethyl)-3-methylcyclohexane Chem. Abst. 69,56053n | p-[2-(3-methylcyclo-hexyl)ethylamino]-benzoic acid |
| 28 | 1-(2-chloroethyl)-4-methylcyclohexane Chem. Abst. 69,56053n | p-[2-(4-methylcyclo-hexyl)ethylamino]-benzoic acid |
| 29 | 2-bromomethylcyclo-heptane Ref.A | p-(cycloheptylmethyl-amino)benzoic acid |
| 30 | 3-bromopropylcyclo-butane Ref. A | p-(3-cyclobutyl)propyl-amino)benzoic acid |
| 31 | 3-bromopropylcyclo-pentane Chem. Abst. 75,15138f | p-(3-cyclopentyl)propyl-aminobenzoic acid |

| Example | Starting Material | Product |
|---|---|---|
| 32 | 3-bromopropylcyclohexane Ref. A | p-(3-cyclohexyl)propylamino)benzoic acid |
| 33 | 1-(3-chloropropyl)-3-ethylcyclohexane Chem. Abst. 68,12589w | p-[3-(3-ethylcyclohexyl)propylamino]-benzoic acid |
| 34 | 1-(3-bromopropyl)-3-methylcyclohexane Chem. Abst. 75,151387f | p-[3-(3-methylcyclohexyl)propylamino]-benzoic acid |
| 35 | 1-(3-bromopropyl)-4-methylcyclohexane Chem. Abst. 75,151387f | p-[3-(4-methylcyclohexyl)propylamino]-benzoic acid |
| 36 | 1-bromo-3-cyclohexylpentane Ref. A | p-[(3-cyclohexyl)pentylamino]benzoic acid |
| 37 | (2-bromomethyl)butylcyclohexane Ref. A | p-[(3-cyclohexyl-2-ethyl)propylamino] benzoic acid |
| 38 | 1-bromo-2-methyl-3-(3-ethylcyclohexyl)-propane Chem. Abst. 68,12529w | p-[3-(3-ethylcyclohexyl)-2-methylpropylamino] benzoic acid |
| 39 | 4-bromobutylcyclopentane Chem. Abst. 69,18646z | p-(4-cyclopentylbutylamino)benzoic acid |
| 40 | 4-chlorobutylcyclohexane Ref. A | p-(4-cyclohexylbutylamino)benzoic acid |
| 41 | 5-bromo-2-cyclohexylpentane Ref. A | p-(4-cyclohexylpentylamino)benzoic acid |
| 42 | 1-bromo-4-cyclohexylhexane Ref. A Chem. Abst. 70,P87143r | p-(4-cyclohexylhexylamino)benzoic acid |
| 43 | 1-bromo-4-cyclohexyl-2-ethylbutane Ref. A | p-(4-cyclohexyl-2-ethylbutylamino)benzoic acid |
| 44 | 1-bromo-4-(3-methylcyclohexyl)butane Ref. A | p-[4-(3-methylcyclohexyl)butylamino]benzoic acid |

| Example | Starting Material | Product |
|---|---|---|
| 45 | 1-chloro-4-(4-methylcyclohexyl)butane | p-[4-(4-methylcyclohexyl)butylamino]benzoic acid |
| 46 | 1-chloro-4-(4-ethylcyclohexyl)butane Ref. A | p-[4-(4-ethylcyclohexyl)-butylamino]benzoic acid |
| 47 | 1-(4-chlorobutyl)-2,3-dimethylcyclohexane Chem. Abst. 70,P87143r; Ref. A | p-[4-(2,3-dimethylcyclohexyl)butylamino]-benzoic acid |
| 48 | 1-(4-chlorobutyl)-2,5-dimethylcyclohexane Ref. A | p-[4-(2,5-dimethylcyclohexyl)butylamino]-benzoic acid |
| 49 | 1-(4-chlorobutyl)-4-methoxycyclohexane Ref. A | p-[4-(4-methoxylcyclohexyl)butylamino]-benzoic acid |
| 50 | 1-(4-bromobutyl)-2-methoxycyclohexane Ref. A | p-[4-(2-methoxylcyclohexyl)butylamino]-benzoic acid |
| 51 | 4-bromobutyl)-cycloheptane Ref. A | p-(4-cycloheptylbutyl-amino)benzoic acid |
| 52 | 1-(4-chlorobutyl)-4-cyclohexylcyclohexane Ref. A | p-[4-(4-cyclohexyl)-cyclohexylbutylamino]-benzoic acid |
| 53 | 2-(4-chlorobutyl)-decahydronapthylene Ref. A | p-[4-(2-decahydronaphthyl)butylamino]-benzoic acid |
| 54 | 4-bromobutylcycloheptane Chem. Abst. 70,P87143r | p-(4-cycloheptylbutyl-amino) benzoic acid |
| 55 | 4-chloropentylcyclopropane Chem. Abst. 69,105732t, 74,31488x | p-[5-(cyclopropyl)-2-pentylamino]benzoic acid |
| 56 | 1-bromo-5-cyclobutylpentane Chem. Abst. 70,P87143r; Ref. A | p-[5-(cyclobutyl)pentyl-amino]benzoic acid |
| 57 | 1-chloro-5-cyclopentylpentane Chem. Abst. 70,P87143r; Ref. A | p-[5-(cyclopentyl)pentyl-amino]benzoic acid |

| Example | Starting Material | Product |
|---|---|---|
| 58 | 5-bromopentylcyclohexane Chem. Abst. 55,21016e | p-[5-(cyclohexyl)pentyl-amino]benzoic acid |
| 59 | 5-chloropentylcyclo-heptane Chem. Abst. 70,P87143r; Ref. A | p-[5-(cyclopentyl)pentyl-amino]benzoic acid |
| 60 | 6-chlorohexylcyclo-pentane Ref. A | p-[6-(cyclopentyl)hexyl-amino]benzoic acid |
| 61 | 6-chlorohexylcyclo-heptane heptane . Chem. Abst. 70,P87143r | p-[6-(cycloheptyl)hexyl-amino]benzoic acid |
| 62 | 1-chloro-7-cyclopentyl-heptane Chem. Abst. 75,P141605n | p-[7-(cyclopentyl)heptyl-amino]benzoic acid |
| 63 | 8-chlorooctylcyclo-pentane Ref. A; . Chem. Abst. 70,87143r | p-[8-(cyclopentyl)octyl-aminobenzoic acid |
| 64 | 8-bromooctylcyclohexane | p-[8-(cyclohexyl)octyl-aminobenzoic acid |
| 65 | 1-bromo-8-(3,3,5-tri-methyl-cyclohexyl)octane Chem. Abst. 75,P20026q | p-[8-(3,3,5-trimethyl-cyclohexyl)octylamino]-benzoic acid |
| 66 | 9-bromononylcyclopentane Ref. A; Chem. Abst. 70,P87143r | p-[9-(cyclopentyl)nonyl-amino]benzoic acid |
| 67 | 13-bromotridecylcyclo-pentane | p-[13-(cyclopentyl)tri-decylamino]benzoic acid |
| 68 | 1-(2-chlorocyclopropyl)-pentane Chem. Abst. 75,49270a | p-[2-pentyl)cyclopropyl-amino]benzoic acid |
| 69 | 1-bromocyclopropyl-pentane Chem. Abst. 75,76195n | p-[1-pentyl)cyclopropyl-amino]benzoic acid |
| 70 | 1-(2-bromocyclopropyl) butane Chem. Abst. 74,123924b | p-[2-butylcyclopropyl)-amino]benzoic acid |
| 71 | bromocyclopentane Ref. A | p-cyclopentylamino-benzoic acid |

| Example | Starting Material | Product |
|---|---|---|
| 72 | 1-chloro-1-propylcyclopentane Chem. Abst. 52, 8978a | p-(1-propylcyclopentylamino)benzoic acid |
| 73 | 4-bromo-1,1-dimethyl cyclohexane Chem. Abst. 71,11242c | p-(4,4-dimethylcyclohexylamino)benzoic acid |
| 74 | 1-chloro-4-propylcyclohexane Chem. Abst. 68,86671h; 75,128994t | p-(4-propylcyclohexylamino)benzoic acid |
| 75 | 2-chloro-1-methylethylcyclohexane Chem. Abst. 70,P87143r; 75,128994t | p-[2(1-methylethyl)-cyclohexylamino]benzoic acid |
| 76 | 4-(t-butyl)-1-chloro-1-methylcyclohexane Chem. Abst. 68, 113898w | p-[4-(t-butyl)-1-methylcyclohexylamino]-benzoic acid |
| 77 | bromocycloheptane Chem. Abst. 51,9505e; 67.9986s | p-cycloheptylaminobenzoic acid |
| 78 | bromocyclooctane Chem. Abst. 51,1049e; | p-cycloheptylaminobenzoic acid |
| 79 | bromocyclononane Chem. Abst. 54,4153f; 69,2306v | p-cyclononylaminobenzoic acid |
| 80 | bromocyclodecane Chem. Abst. 67,58849h; 69,2306c | p-cyclodecylaminobenzoic acid |
| 81 | bromocycloundecane Chem. Abst. 69,2306c | p-cycloundecylaminobenzoic acid |
| 82 | bromocyclododecane Chem. Abst. 54,4153f; 69,2306c | p-cyclododecylaminobenzoic acid |
| 83 | bromocyclotridecane Chem. Abst. 69,2306c | p-cyclotridecylaminobenzoic acid |
| 84 | bromocyclotetradecane Chem. Abst. 54,4153f; 54,16141i | p-cyclotetradecylaminobenzoic acid |
| 85 | bromocyclopentadecane Chem. Abst. 72,P100160g 69,2306c | p-cyclopentadecylaminobenzoic acid |

| Example | Starting Material | Product |
|---|---|---|
| 86 | bromocyclohexadecane Chem. Abst. 69,2306c | p-cyclohexadecylamino-benzoic acid |
| 87 | 3-bromobicyclopentyl Chem. Abst. 31, 7405[3]; 35, 2864 | p-(3-cyclopentyl)cyclo-pentylamino)benzoic acid |
| 88 | (3-bromocyclopentyl) cyclohexane Chem. Abst. 31,7405[4] | p-(3-cyclohexylcyclo-pentylamino)benzoic acid |
| 89 | 3-bromo-3'-ethylbi-cyclopentyl Chem. Abst. 36, 48089 | p-[3-(3-ethylcyclo-pentyl)cyclopentyl-amino] benzoic acid |
| 90 | 2-bromo-1-cyclopentyl-cyclopentane Chem. Abst. 51, 5712f | p-[2-(cyclopentyl)cyclo-pentylamino]benzoic acid |
| 91 | 1-chlorobicyclohexyl Chem. Abst. 30, 3807[1] | p-[1-(cyclohexyl)cyclo hexylamino]benzoic acid |
| 92 | 1-chlorobicyclopentyl Chem. Abst. 45, 6163b | p-[1-(cyclopentyl)cyclo-pentylamino]benzoic acid |
| 93 | 2-iodomethyldecahydro-napthalene Chem. Abst. 41, 116b | p-[(2-decahydronaph-thyl)methylamino]benzoic acid |
| 94 | 2-(2-iodoethyl)deca-hydronaphthalene Chem. Abst. 41, 116d | p-[2-(2-decahydronaph-thyl)ethylamino]benzoic acid |
| 95 | 1-(4-bromobutyl)deca-hydronaphthalene Chem. Abst. 45, p175d | p-[4-(1-decahydronaph-thyl)butylamino]benzoic acid |
| 96 | 1-bromo-1,1-dicyclo-pentylethane, Chem. Abst. 31, 5759[2] | p-[(1,1-dicyclopentyl) ethylamino]benzoic acid |
| 97 | 1-bromo-4- -methyldeca-hydronaphthalene Chem. Abst. 53, 3265f | p-[1-(4 -methyldeca-hydronaphthyl)amino] benzoic acid |
| 98 | 2-(bromomethyl)-1,3,3-trimethylcyclohexane Chem. Abst. 28, 2343[8] | p-[(1,3,3-trimethyl-cyclohexyl)methylamino]-benzoic acid |
| 99 | 6-(3-bromobutyl)-1,5,5-trimethylcyclohexene Chem. Abst. 66, 2658g | p-[4-(2,6,6-trimethyl-2-cyclohexenyl)-2-butyl amino]benzoic acid |

| Example | Starting Material | Product |
|---|---|---|
| 100 | 4-(3-chlorolpropyl)-cyclohexene Ref. A | p-[3-(3-cyclohexenyl)-propylamino]benzoic acid |
| 101 | 3-(4-chlorobutyl)-cyclopentene Ref. A | p-[4-(3-cyclopentenyl)-butylamino]benzoic acid |
| 104 | 3-(11-chloroundecyl)-cyclopentene Chem. Abst. 37, 3060f | p-[11-(3-cyclopentenyl)-undecylamino]benzoic acid |
| 105 | 1-(13 chlorotridecyl-cyclopentene Chem. Abst. 37, 5031b | p-[13-(1-cyclopentyl) tridecylamino]benzoic acid |
| 106 | 3-(13-chlorotridecyl)-cyclopentene Chem. Abst. 51, 7652a | p-[1-(cyclohexyl)cyclo-hexylamino]benzoic acid |
| 107 | 2-(4-chlorobutyl)deca-hydronapthalene Chem. Abst. 70, P87143r | p-[4-(2-decahydro-naphthyl)butylamino] benzoic acid |
| 108 | 4-bromo-1-(cyclohexyl)-cyclohexane Chem. Abst. 69, 103618m | p-[4-cyclohexyl)cyclo-hexylbutylamino]benzoic acid |

Ref. A=R.D. Westland, et al., J. Med Chem., 11,1190 (1968)

EXAMPLE 109

Preparation of p-[2-(cyclopentyl)ethylamino]benzoic acid

To a solution of 5 g. of 2-cyclopentylethanol and 9.15 ml. of triethylamine in 75 ml. of dry methylene chloride (cooled in an ice-bath) in an argon atmosphere, is added a solution of 8.39 g. of methanesulfonic anhydride in 50 ml. of methylene chloride dropwise over a period of 15 minutes. After stirring at -10°C. for 50 minutes the solution is washed successively with 30 ml. portions of ice-cold water, 5% hydrochloric acid, saturated sodium bicarbonate solution, saturated sodium chloride solution, dried with anhydrous magnesium sulfate, and taken to dryness under reduced pressure to provide 8.4 g. (99%) of 2-cyclopentylethyl methanesulfonate.

A solution of 5 g. of 2-cyclopentylethyl methanesulfonate and 8.59 g. of ethyl p-aminobenzoate in 30 ml. of hexamethylphosphoramide is stirred at 110°C. under argon atmosphere for 18 hours. The cooled solution is diluted with 30 ml. of water and the resulting tan solid is collected by filtration. Recrystallization from ethanol-water furnishes ethyl p-[2-cyclopentyl)ethyl]aminobenzoate as off-white crystals.

A solution of 2 g. of ethyl p-[2-(cyclopentyl)-ethylamino]benzoate in 50 ml. of ethanol:water (9:1) containing 2 g. of potassium hydroxide is stirred at the reflux temperature under argon atmosphere for 3 hours. The chilled solution is acidified with concentrated hydrochloric acid, diluted with 45 ml. of water, and filtered to furnish 1.52 g. of white crystals. Recrystallization from ethanol/hexane provided product as white crystals.

EXAMPLES 110-188

Treatment of the alcohols of Table II below with methane sulfonic anhydride to provide the corresponding mesylate followed by treatment with ethyl p-aminobenzoate followed by saponification and acidification of the resulting substituted aminobenzoate by the procedure of Example 109 produces the indicated p-(substituted amino)-benzoic acids shown in Table II.

TABLE II

| Example | Starting Material | Product |
|---|---|---|
| 110 | 2-isopropyl-5-methyl-enecyclopentanol Chem Abst. 66, 38074c | p-(2-isopropyl-5-methylene cyclopentyl-amino)benzoic acid |
| 111 | 2-cyclohexen-1-ol Aldrich Chem. Co. | p-(cyclohex-2-enyl-amino)benzoic acid |
| 112 | 4-isopropyl-2-cyclo-hexen-1-ol Chem. Abst. 69, 99290d | p-(4-isopropylcyclo-hex-2-enylamino) benzoic acid |
| 113 | 2-isopropyl-3-cyclo-hexen-1-ol Chem. Abst. 75, 55380m | p-(2-isopropylcyclo-hex-3-enylamino) benzoic acid |

| Example | Starting Material | Product |
|---|---|---|
| 114 | 2-methyl-2-cyclooctene-1-olopentane Chem. Abst. 69, 27127h | p-(2-(cyclopentyl)-cyclopentylamino) benzoic acid |
| 115 | 2-cyclononen-1-ol Chem. Abst. 72, 30882t | p-(cyclonon-2-enyl-amino)benzoic acid |
| 116 | 3-cyclononen-1-ol Chem. Abst. 75, 13957j | p-(cyclonon-3-enyl-amino)benzoic acid |
| 117 | 2-methylenecyclo-decanol Chem. Abst. 74, 75857w | p-(2-methylenecyclo-decylamino)benzoic acid |
| 118 | E-3-cyclodecen-1-ol Chem. Abst. 73, 87173n | p-(E-cyclodec-3-enyl-amino)benzoic acid |
| 119 | Z-3-cyclodecen-1-ol Chem. Abst. 73, 87173n | p-(z-cyclodec-3-enyl-amino)benzoic acid |
| 120 | 5-cyclodecen-1-ol Chem. Abst. 71, 60514w | p-(cyclodec-5-enyl-amino)benzoic acid |
| 121 | 4-ethyl-2-cyclododecen-1-ol Chem. Abst. 70, 114922c | p-(4-ethylcyclododec-2-enylamino)benzoic acid |
| 122 | 2-cyclotridecen-1-ol Chem. Abst. 70, 114922c | p-(cyclotridec-2-enyl-amino)benzoic acid |
| 123 | 8-cycloheptadecen-1-ol Chem. Abst. 66, 2658g | p-(cycloheptadec-8-enyl-amino)benzoic acid |
| 124 | 9-cycloheptadecen-1-ol Chem. Abst. 68, 49157z | p-(cycloheptadec-9-enyl-amino)benzoic acid |
| 125 | 2-cyclobutene-1-methanol Chem. Abst. 67, 32343p | p-[(cyclobut-2-enyl)-methylamino]benzoic acid |
| 126 | 1-cyclobutene-1-methanol Chem. Abst. 70, 10736d | p-[(cyclobut-1-enyl)-ethylamino]benzoic acid |
| 127 | 2-cyclopentene-1-methanol Chem. Abst. 71, 12650r | p-[(cyclopent-3-enyl)-methylamino]benzoic acid |
| 128 | 3-cyclopentene-1-methanol Chem. Abst. 73, 65783j | p-(4-isopropylcyclohex-2-enylamino]benzoic acid |

| Example | Starting Material | Product |
|---|---|---|
| 129 | 1-cyclopentene-1-propanol Chem. Abst. 73, 66113c | p-[1-(cyclopent-1-enyl)-propylamino]benzoic acid |
| 130 | 1-cyclohexene-1-methanol Chem. Abst. 70, 10773p | p-[(cyclohex-1-enyl)-methylamino]benzoic acid |
| 131 | 2-cyclohexene-1-ethanol 39882t Chem. Abst. 69, 26424r | p-[1-cyclohex-2-enyl)-ethylamino]benzoic acid |
| 132 | 1-3-cyclohexenyl)-1-propanol Chem. Abst. 67, 72634r | p-[1-cyclohex-3-enyl)-propylamino]benzoic acid |
| 133 | cis-5-ethyl-3-cyclohexene-1-methanol Chem. Abst. 69, 27575c | p-(cis-5-ethylenecyclohex-3-enyl)methylamino-benzoic acid |
| 134 | trans-5-ethyl-3-cyclohexene-1-methanol Chem. Abst. 69, 27575c | p-(trans-5-ethylenecyclohex-3-enyl)methylamino-benzoic acid |
| 135 | 1-cycloheptene-1-methanol Chem. Abst. 73, 14266k | p-(cyclohept-1-enyl)-ethylamino)benzoic acid |
| 136 | 1-cycloheptene-1-ethanol Chem. Abst. 70, 37270j | p-[1-(cyclohept-1-enyl)-ethylamino]benzoic acid |
| 137 | 2-cyclooctene-1-methanol Chem. Abst. 69, 36263b | p-(cyclooct-2-enylmethyl-amino)benzoic acid |
| 138 | 4-cyclooctene-1-methanol Chem. Abst. 66, 37492a | p-(cyclooct-4-enylmethyl-amino)benzoic acid |
| 139 | 1-cyclooctenylethanol Chem. Abst. 70. 37270j | p-(cyclooct-1-enylethyl-amino)benzoic acid |
| 140 | 2-(3-cyclopentenyl)-butanol Chem. Abst. 72, 133044a | p-[2-ethyl-2-cyclopent-3-enylethylamino]benzoic acid |
| 141 | 2,3-dimethyl-2-(2-cyclopentenyl)propanol Chem. Abst. 70, 11818u | p-[2-(2,3-dimethylcyclopent-2-enyl)propylamino]-benzoic acid |

| Example | Starting Material | Product |
|---|---|---|
| 142 | 4,6-dimethyl-3-cyclohexen-1-ethanol Chem. Abst. 74, 111623c | p-[2-(4,6-dimethylcyclohex-3-enyl)ethylamino]-benzoic acid |
| 143 | α-methyl-1-cyclohexene-1-ethanol Chem. Abst. 74, 42909v | p-[1-methyl-2-(cyclohex-1-enyl)ethylamino] benzoic acid |
| 144 | 4-methyl-3-cyclohexene-1-ethanol Chem. Abst. 75, 19601s | p-[2-(4-methylcyclohex-3-enyl)ethylamino] benzoic acid |
| 145 | 1-cyclooctene-1-ethanol Chem. Abst. 70, 37270j | p-(2-cyclooct-1-enylethylamino) benzoic acid |
| 146 | 1-cyclononene-1-ethanol | p-(2-cyclonon-1-enyl-ethylamino)benzoic acid |
| 147 | α,4-dimethyl-3-cyclohexene-1-propanol Chem. Abst. 68, 78427t | p-[1-methyl-3-(4-methyl-cyclohex-3-enyl)propyl-amino]benzoic acid |
| 148 | 1-cyclohexene-1-propanol Chem. Abst. 70, 87111d | p-(3-cyclohex-1-enyl-propylamino)benzoic acid |
| 149 | 3-cyclohexene-1-propanol Chem. Abst. 69, 43158z | p-(3-cyclohex-3-enyl-propylamino)benzoic acid |
| 150 | 3-cyclohexene-1-butanol Chem. Abst. 69, 49158z | p-(4-cyclohex-3-enyl-butylamino)benzoic acid |
| 151 | α,α-dimethyl-2-cyclopentene-1-undecanol Chem. Abst. 72, 110860z | p-[(1,1-dimethyl-11-cyclopent-2-enyl)undecyl amino]benzoic acid |
| 152 | 4-isopropylidene-2,2-dimethylcyclobutanol Chem. Abst. 73, 24996n | p-(2,2-dimethyl-4-isopropylidenecylobutyl-amino)benzoic acid |
| 153 | 2-cyclopenten-1-ol Chem. Abst. 68, 39177s | p-(cyclopent-2-enyl-amino)benzoic acid |
| 154 | 3-cyclopenten-1-ol Chem. Abst. 66, 11504r | p-(cyclopent-3-enyl-amino)benzoic acid |

| Example | Starting Material | Product |
|---|---|---|
| 155 | 3-cyclohexen-1-ol Chem. Abst. 69, 26837c | p-(cyclohex-3-enylamino)-benzoic acid |
| 156 | 2,2-dimethyl-6-methylenecyclohexanol | p-(2.2-dimethyl-6-enylcyclohexylamino)benzoic acid |
| 157 | 2-methylenecycloheptanol Chem. Abst. 69,27127h | p-(2-methylenecycloheptylamino)benzoic acid |
| 158 | 2-methyl-2-cyclohepten-1-ol Chem. Abst. 69,27127h | p-(2-methylcyclohept-2-enylamino)benzoic acid |
| 159 | 2-methyl-6-methylenecycloheptanol Chem. Abst. 67,11600e | p-(2-methyl-6-methylenylcycloheptylamino)-benzoic acid |
| 160 | 3,7-dimethyl-3-cyclohepten-1-ol Chem. Abst. 67, 11600e | p-(3,7-dimethylcyclohept-3-enylamino)benzoic acid |
| 161 | 4-cycloocten-1-ol Chem. Abst. 70,28287t | p-(cyclooct-4-enylamino) benzoic acid |
| 162 | 3-cycloocten-1-ol Chem. Abst. 66,104593z | p-(cyclooct-3-enylamino)-benzoic acid |
| 163 | 2-cycloocten-1-ol Chem. Abst. 68,39177s | p-(cyclooct-2-enylamino)-benzoic acid |
| 164 | 4-methylenecyclooctanol Chem. Abst. 70,28445t | p-(4-methylenecyclooctylamino)benzoic acid |
| 165 | α-methyl-5-methylenecyclooooctanemethanol Chem. Abst. 68,104595t | p-[1-(5-methylenecyclooctylethylamino]benzoic acid |
| 166 | 5-methylenecyclooctanemethanol Chem. Abst. 66,37492a | p-[5-methylenecyclooctylmethylamino]benzoic acid |
| 167 | 1,3-dimethyl-2-methylenecyclopentane-methanol Chem. Abst. 73,24996n | p-[(1,3-dimethyl-2-methylenecyclopentyl)methylaminobenzoic acid |
| 168 | E-4-cyclopropyl-3-buten-2-ol Chem. Abst. 70,3413t | p-[E-2-(4-cyclopropyl)but-3-enylamino]benzoic acid |

| Example | Starting Material | Product |
|---|---|---|
| 169 | Z-4-cyclopropyl-3-buten-2-ol Chem. Abst. 70,3413t | p-[Z-2-(4-cyclopropyl)but-3-enylamino]benzoic acid |
| 170 | α-methylenecyclohexaneethanol Chem. Abst. 66,45950p | p-[(1-methylene-2-cyclohexyl)ethylamino]-benzoic acid |
| 171 | β-methylenecyclohexaneethanol Chem. Abst. 75,139951c | p-[(2-methylene-2-cyclohexyl)ethylamino]-benzoic acid |
| 172 | E-2-(3,3-dimethylcyclohexylidenyl)ethanol Chem. Abst. 75,110431x | p-[E-2-(3,3-dimethylcyclohexylidenyl)ethyl]amino-benzoic acid |
| 173 | Z-2-(3,3-dimethylcyclohexylidenyl)ethanol Chem. Abst. 75,110431x | p-[Z-2-(3,3-dimethylcyclohexylidenyl)ethylamino]-benzoic acid |
| 174 | E-4-cyclopentyl-2-buten-1-ol Chem. Abst. 75,48349w | p-(4-cyclopentylbut-2-enyl-ylamino)benzoic acid |
| 175 | E-4-cyclohexyl-2-buten-1-ol Chem. Abst. 75,48349w | p-(E-4-cyclohexylbut-2-enylamino)benzoic acid. |
| 176 | 2-vinylcyclopentane-ethanol Chem. Abst. 66,104477q | p-[2-(2-vinylcyclopentyl)ethylamino]-benzoic acid |
| 177 | 3-isopropyl-1-methyl-cyclopentanemethanol Chem. Abst. 66,38061w | p-[(3-isopropyl-2-methyl-cyclopentyl)methylamino]-benzoic acid |
| 178 | 1-allyl-2-methylcyclohexanol Chem. Abst. 71,29919h | p-(1-allyl-2-methylcyclohexylamino)benzoic acid |
| 179 | 2-isopropenylcyclohexanol Chem. Abst. 72,12663t | p-(2-isopropenylcyclohexylamino)benzoic acid |
| 180 | 1-isopropenylcyclohexanol Chem. Abst. 75,139951c | p-(1-isopropenylcyclohexylamino)benzoic acid |
| 181 | 2-allylcyclohexanol Chem. Abst. 70,96517t | p-(2-allylcyclohexyl-amino)benzoic acid |

| Example | Starting Material | Product |
|---|---|---|
| 182 | 3-allylcyclohexanol Chem. Abst. 69,86453j | p-(3-allylcyclohexylamino)benzoic acid |
| 183 | 1-allylcyclohexanol Chem. Abst. 66,374866 | p-(1-allylcyclohexylamino)benzoic acid |
| 184 | 1-allylcycloheptanol | p-(1-allycycloheptylamino)benzoic acid |
| 185 | 1-allylcyclooctanol | p-(1-allycyclooctylamino)benzoic acid |
| 186 | 1-(3-butenyl)-2-methylcycloheptanol Chem. Abst. 69,106892g | p-[1-(3-butenyl)-2-methylcycloheptylamino]-benzoic acid |
| 187 | 1-allylcyclododecanol Chem. Abst. 68,95381r | p-(1-allylcyclododecylamino)benzoic acid |
| 188 | 2-butyl-2-cyclopenten-1-ol Chem. Abst. 71,38404p | p-(2-butylcyclopent-2-enylamino)benzoic acid |

EXAMPLE 189

Preparation of methyl esters

Treatment of a methylene chloride solution of the 4-(substituted amino)benzoic acids of Examples 1-188 with a solution of diazomethane in ethanol until a yellow color persists (excess) followed by concentration and purification, if necessary is productive of the corresponding methyl 4-(substituted amino)benzoate.

EXAMPLE 190

Preparation of hexyl esters

Treatment of a methylene chloride solution of the 4-(substituted amino)benzoic acids of Examples 1-188 with excess diazohexane in dietyl ether, followed by concentration and purification, is productive of the corresponding hexyl 4-(substituted amino)benzoate.

EXAMPLE 191

Preparation of p-cyclohexylaminobenzhydroxamic acid

To a suspension of p-cyclohexylaminobenzoic acid (44 g.) in glyme (350 ml.) and pyridine (70 ml.) at 0°C. is

added trifluoroacetic anhydride (67 ml., 100 g.) at such a rate as to maintain the temperature at 20-30°C. The resulting solution is stirred at 10-15°C. for 2 hours, then diluted with ether (400 ml.), cooled in an ice-bath and ice (100 gm.) is added. The mixture is stirred vigorously at ambient temperature for 1 hour. The aqueous layer is extracted with ether and the combined ether extracts are washed with water, brine, dried over sodium sulfate and concentrated *in vacuo* to provide *p*-[N-trifluoroacetyl]-*N*-cyclohexylamino]benzoic acid as an oil that solidifies upon standing.

The *N*-trifluoroacetyl derivative (57 g.) is dissolved in thionyl chloride (300 ml.) and refluxed for 3 hours. After cooling, the mixture is diluted with toluene and concentrated *in vacuo*. The residue is diluted again with toulene and filtered. The toulene is concentrated *in vacuo* to provide *N*-trifluoroacetyl-*p*-cyclohexylaminobenzoyl-chloride as an oil.

To a stirred solution of hydroxylamine hydrochloride (16 g.) in pyridine (75 ml.) and dichloromethane (35 ml.) is added, dropwise, the previously prepared benzoyl chloride derivative (11.6 g.) in dichloromethane (20 ml.). After 1 hour, the mixture is diluted with water and extracted with ether. The combined organic extract is washed with 5% hydrochloric acid until the aqeous wash remains acidic. The ether extract is then washed with water, brine, dried with sodium sulfate and concentrated *in vacuo* to afford *N*-trifluoroacetyl-*p*-cyclohexylaminobenzhydroxamic acid as an oil.

This oil (0.9 g.) is dissolved in ethanol (20 ml.) and 1N sodium hydroxide (2 ml.) is added. After 12 hours the solution is chilled and filtered to provide a white solid that is washed with ether.

The white solid is recrystallized from hot ethanol to provide p-(cyclohexylamino)benzhydroxamic acid.

EXAMPLE 192

Preparation of Esters

Treatment of the acids of Examples 1-188 with trifluoroacetic anhydride to provide the N-$COCF_3$ derivatives, followed by treatment with thionyl chloride to provide the N-$COCF_3$ acid chloride, followed by treatment with one of the following alcohols, followed by removal of the N-$COCF_3$ group with sodium hydroxide, by the method of Example 191, provides the corresponding esters of the starting acid.

Alcohols: methanol, ethanol, 2-methoxyethanol, butanol, pentanol, hexanol, cyclopentanol, cyclohexanol, 1,2-propanediol, 1,3-propanediol, ethylene glycol, glycerol, glycidol, glycolic acid, citric acid, tartaric acid, malic acid, methyl glycolate, 2-hydroxypropionic acid, 3-hydroxybutyric acid, 4-hydroxybutyric acid, glyceric acid, 3-diethylamino-1-propanol, 1-diethylamino-2-propanol, 1-diethylamino-2-propanol, 3-diethylamino-1-propanol, 2-diisopropylaminoethanol, 3-dimethylamino-1,2-propanediol, N piperidineethanol, N,N-diethylethanolamine, benzyl alcohol, p-fluorobenzyl alcohol, p-bromobenzyl alcohol, p-chlorobenzyl alcohol, p-methoxybenzyl alcohol, m-(trifluoromethyl)benzyl alcohol, p-carboxybenzyl alcohol, phenol, p-fluorophenol, p-bromophenol, p-methoxyphenol, p-carboxyphenol, m-(trifluoromethyl)phenol, 4-cyanophenol, 3-hydroxypyridine, 2-chloro-3-hydroxypyridine, and 5-carboxy-3-hydroxypyridine.

## EXAMPLE 193

Preparation of Amides

Treatment of the acids of Examples 1-188 with trifluoroacetic anhydride to provide the N-$COCF_3$ derivatives, followed by treatment with thionyl chloride to provide the N-$COCF_3$ acid chloride, followed by treatment with one of the amines of the list below, by the method of Example 191, provides the corresponding amides of the starting acid.

Amines: β-alanine, allylamine, N-allylaniline, allylcyclohexylamine, aminoacetonitrile, α-aminoacetophenone, 2-amino-1-butanol, 3-aminobutyric acid, 4-aminobutyric acid, 1-amino-1-cyclopentanemethanol, 2-amino-5-diethylaminopentane, N-(2-aminoethyl)morphonine, N-(2-aminoethyl)piperazine, N-(2-aminoethyl)piperidine, 2-amino-2-ethyl-1,3-propanediol, 2-(2-aminoethyl)pyridine, N-(2-aminoethyl)pyrrolidine, DL-4-amino-3-hydroxybutyric acid, 5-aminolevulinic acid, p-aminomethylcyclobutane, 4-(aminomethyl)cyclohexanecarbonitrile, 1-aminomethyl-1-cyclohexanol, aminomethylcyclopropane, 4-(aminomethyl)piperidine, 2-amino-2-methyl-1,3-propanediol, 2-amino-2-methyl-1-propanol, 2(aminomethyl)-2-propanol, 2-aminomethylpyridine, 3-aminomethylpyridine, 4-aminomethylpyridine, 2-amino-1-phenylethanol, 2-amino-3-phenyl-1-propanol, 3-amino-3-phenylpropionic acid, 3-amino-1,2 propanediol, 1-amino-2-propanol, L-2-amino-1-propanol, 3-amino-1-propanol, N-(3-aminopropyl)diethanolamine, N-(3-aminopropyl)morpholine, 1-(3-aminopropyl)-2-pipecoline, N-(3-aminopropyl)-2-pyrrolidinone, 5-aminovaleric acid, bis-(2-ethoxyethyl)amine, bis-(2-methylallyl)amine, p-bromophenethylamine-3-bromopropylamine, *n*-butylamine, *sec*-butylamine, *tert*-butylamine, 2-chlorobenzylamine, 3-chlorobenzylamine, 4-chlorobenzylamine, 2-chloroethylamine, 3-chloropropylamine, cyclobutylamine, cycloheptylamine, 1,3-cyclohexanebis(methylamine), cyclohexanemethylamine, cyclohexylamine, cyclopentylamine, cyclopropylamine, 3-(di-*n*-butylamineo)propylamine, 1,5-dimethylhexylamine, α,4-dimethyl-3-hydroxyphenethylamine,

1,2-dimethylpropylamine, 1,2-diphenylethylamine, ethylamine, ethyl 3-aminobutyrate, ethyl 4-aminobutyrate, 2-(ethylamino)ethanol, 1-ethylpropylamine, 1-ethynylcyclohexylamine, _m_-fluorobenzylamine, _p_-fluorobenzylamine, 2-fluoroethylamine, furfurylamine, _n_-heptylamine, propylamine, _m_-iodobenzylamine, isoamylamine, isopropylamine, _m_-methoxybenzylamine, _p_-methoxybenzylamine, 2-methoxyethylamine, _o_-methoxyphenethylamine, _p_-methoxyphene-thylamine, N-methyl-β-alaninenitrile, 2-methylallylamine, methylamine, methylaminoacetonitrile, 2-(methylamino)ethanol, 2-methylbenzylamine, 3-methylbenzylamine, 4-methylbenzylamine, 1-methylbutylamine, 4-methylcyclohexylamine, 1-norepinephrine, 4-phenylbutylamine, 1-phenylcyclopropanemethylamine, 2-phenylcyclopropylamine, N-phenylethylenediamine, α-phenylglycinol, 2-phenylglycinonitrile, phenylpropanolamine, 3-phenyl-1-propylamine, mono-propargylamine, propylamine, taurine, tetrahydrofurfurylamine, 1,2,3,4-tetrahydro-1-naphtyylamine, 2-(_p_-tolyl)ethylamine, veratrylamine, _m_-xylylenediamine, _m_-aminobenzoic acid, _p_-aminobenzoic acid, _o_-aminobenzyl alcohol, _m_-aminobenzyl alcohol, _p_aminobenzyl alcohol, 4-benzylpiperidine, 2,6-dimethylpiperidine, 2-ethylpiperidine, 3-hydroxypiperidine, 4-hydroxypiperidine, 2-methylpiperidine, 3-methylpiperidine, 4-methylpiperidine, 4-phenylpiperidine, piperidine, morpholine, hexamethyleneimine, heptamethylenimine, pyrrolidine, N-methylpiperazine, _dl_-alanine, hydrazine, N-acetylhydrazine, _dl_-valine, $\Delta^3$piperidine, _dl_-leucine, 2-aminoisobutyric acid, glycine, ethyl glycinate.

## EXAMPLE 194

## Preparation of N-(_p_-[(cyclohexylmethyl)amino]benzoyl)-benzamide

1.0 g. of sodium hydride (50% dispersion in mineral oil) under nitrogen is washed with hexane three times. To the dry sodium hydride is added 5 ml. of freshly distilled tetrahydrofuran. To this suspension is added a solution of 2.4 g. of benzamide in 5 ml. of tetrahydrofuran.

After complete reaction (30 minutes) a solution of 0.9 g. p-[N-trifluoroacetyl-N-(cyclohexylmethyl)amino]benzoyl chloride in 3 ml. of tetrahydrofuran is added. After stirring at ambient temperature for 1 hour, the reaction mixture is poured into water and extracted twice with ether. The ether extracts are washed with water, brine, dired with sodium sulfate, and concentrated in vacuo. The residue is recrystallized from hot ether-acetonitrile (1:1) to provide the amide.

EXAMPLE 195

Preparation of N-(p-[substituted amino]benzoyl)benzamides

Treatment of the N-$COCF_3$ acid halides (prepared from the corresponding acid of Examples 1-188 by the method of Example 191) with benzamide and sodium hydride by the method of Example 194 followed by removal of the N-$COCF_3$ group by the method of Example 191 is productive of the corresponding N-(p-[substituted amino]benzoyl)benzamides.

EXAMPLE 196

Preparation of 4-(8-cyclohexyloctylamino)benzoyl chloride hydrochloride

A cold solution of 25 g. 4-(8-cyclohexyloctyl-amino)benzoic acid in 500 ml. dimethoxyethane-methylene chloride (4:1) is prepared and dry hydrochloric acid is bubbled through the solution until no more precipitate forms. The solution is treated with 25 ml. thionyl chloride and refluxed until all of the precipitate has dissolved. The solvents are evaporated to yield an orange, semi-crystalline mass.

EXAMPLE 197

4-[N-carbobenzyloxy-N-(8-cyclohexyloctyl)amino]benzoyl chloride

To 15 g. 4-(8-cyclohexyloctylamino)benzoic acid in 200 ml. warm chloroform is added 15 g. sodium carbonate in 150 ml. water. To the vigorously stirred solution is added 10 g. carbobenzyloxy chloride. After 2 hours stirring at 40° C., the layers are separated, washed three times with 1N

hydrochloric acid, dried, and evaporated to an oil. The oil is dissolved in 300 ml. toluene, treated with 15 ml. thionyl chloride and the solution is refluxed for 5 hours. The solvents are evaporated and the residue is dissolved three times in toluene, evaporating each time to yield a viscous, orange oil.

EXAMPLE 198

Preparation of 1-{4-[N-(t-butyloxycarbonyl)-N-(8-cyclohexyloctyl)amino]benzoyl} imidazole

A solution of 10 g. 4-(8-cyclohexyloctylamino)-benzoic acid in 100 ml. dioxane is treated with 4.0 g. t-butylazidoformate and 10 ml. pyridine. After stirring at room temperature for 18 hours, the protected amido-acid is precipitated from solution by addition of 150 ml. water. The product is collected and thoroughly dried. The crude product is dissolved in 200 ml. of a mixture consisting of methylene chloride/dimethoxyethane/pyridine (1:4:1), and to this is added 5.4 g. 1,1'-carbonyldiimidazole. The solution is stirred overnight at room temperature and the solvents are evaporated to yield the title compound as a thick, orange oil.

EXAMPLE 199

Preparation of 1-(methoxycarbonyl)propyl 4-(8-cyclohexyloctylamino)benzoate

To a solution of 10.0 g. 4-(8-cyclohexyloctyl-amino)benzoyl chloride hydrochloride in 200 ml. methylene chloride is added dropwise a solution of 3 g. methyl 2-hydroxy butyrate and 5 g. triethylamine in 100 ml. ether. After 17 hours stirring at room temperature, the precipitate is filtered and washed with several portions of ether. The ether solution is washed with water, dried, and condensed to the crystalline title compound.

EXAMPLE 200

Preparation of 1-carboxyetrhyl 4-(8-cyclohexyloctylamino)-benzoate

A flask containing 10.0 g. 4-(8-cyclohexyloctyl-amino)benzoic acid, 3.3 g. lactic acid, 500 mg. toluene-

sulfonic acid and 500 ml. toluene is equipped with a Soxhlet extractor charged with activated A Linde molecular sieves. The solution is refluxed for 24 hours, during which time the Soxhlet extractor is charged twice more with fresh sieves. The hot solution is filtered and left to cool, whereupon the product separates as off-white crystals.

EXAMPLE 201

Preparation of O-[4-(8-cyclohexyloctylamino)benzoyl]malic acid

To a warm solution of N-carbobenzyloxy-4-(8-cyclohexyloctylamino)benzoyl chloride and 1.3 g. triethylamine in 100 ml. ether is treated with 2 g. malic acid. An immediate precipitate forms, but the mixture is refluxed for one hours and filtered while hot. The solid is washed several times with hot ehter, then the ehter is evaporated to yield a white solid. The product is dissolved in tetrahydrofuran (100 ml.) and hydrogenated over 600 mg. 10% Pd(C) at 50 psi until hydrogen uptake stops. The catalyst is filtered, the solution is evaporated, and the residue is crystallized from acetic acid to yield the title compound as a tan, crystalline mass.

EXAMPLE 202

Preparation of 2-(ethoxycarbonyl)vinyl 4-(8-cyclohexyloctyl amino)benzoate

To a mixture containing 4.3 g. 1-{4-[N-(*t*-butyloxycarbonyl)-N-(8-cyclohexyloctyl)amino]benzoyl} imidazole, 50 ml. chloroform, and 50 ml. 5*N* sodium hydroxide is added 3 g. ethyl 2-formyl acetate. The solution is vigorously stirred for 24 hours. The layers are separated, and the chloroform solution is washed once with 50 ml. 1*N* sodium hydroxide. The solvent is evaporated and the residue is heated for 30 minutes at 40° C. in 50 ml. anhydrous trifluoroacetic acid. The solvent is again evaporated and the oil is crystallized from acetone to yield light yellow crystals of the product.

EXAMPLE 203

Preparation of 3-O-[4-(8-cyclohexyloctylamino)benzoyl]-glyceric acid

To a solution of 3.6 g. glyceraldehyde and 2.44 g. 4-dimethylaminopyridine in an ice-water bath is added 11 mmoles of N-trifluoroacetyl-4-(8-cyclohexyloctylamino)-benzoyl chloride. Afater 4 hours, the product is partitioned between 50 ml. chloroform and 50 ml. water, and to the well-stirred solution is added 2 g. bromine and 1 g. soldium hydroxide. After thin-layer chromatography at intervals shows oxidation to be complete, the mixture is stirred 2 hours at 20° C. and the layers are separated. The organic layer is evaporated and the residue is crystallized from acetic acid to yield the title compound as a cream-colored, oily solid.

EXAMPLE 204

Preparation of 4-chlorophenyl 4-(8-cyclohexyloctylamino)-benzoate

To a solution of 6.4 g. 4-chlorophenol and 7.6 g. triethylamine in 500 ml. methylene chloride is added 10.4 g. 4-(8-cyclohexyloctylamino)benzoyl chloride hydrochloride in 250 ml. methylene chloride. After four hours at reflux, the solution is cooled, washed with water and dilute phosphoric acid, and dried. After passing the solution through a column of alumina, the solvent is evaporated and the residue is crystallized from diisopropyl ether.

EXAMPLE 205

Preparation of 2-tetrahydropyranyl 4-(8-cyclohexyloctyl amino)benzoate

A mixture of 7 g. 4-(8-cyclohexyloctylamino)-benzoic acid, 2 g. 2,3-dihydropyran and 100 mg. anhydrous p-toluene sulfonic acid in 50 ml. toluene is stirred at room temperature for 20 hours. The solution is washed with saturated sodium bicarbonate, dried, and concentrated. The residue is crystallized from methylcyclohexane to yield white crystals.

EXAMPLE 206

Preparation of 3-pyridyl 4-(8-cyclohexyloctylamino)benzoate

A 6 g. sample of 4-(8-cyclohexyloctylamino)benzoic acid and 2.7 g. 1,1'-carbonyldiimidazole in 50 ml. dry tetrahydrofuran is stirred for 2 hours. Then, 1.58 g. 3-hydroxypyridine and a trace of sodium hydride catalyst is added and the reaction is refluxed for 3 hours. The solution is cooled, filtered, and evaporated. The product is crystallized from isopropanol.

EXAMPLE 207

2,3-Dihydroxypropyl 4-(8-cyclohexyloctylamino)benzoate

A solution of 7.34 g. of 4-(8-cyclohexyloctylamino)benzoic acid, 4.80 g. of 25% aqueous sodium hydroxide, and 12.6 g. of 3-iodo-1,2-propanediol in 50 ml. of hexamethylphosphoramide is stirred for 24 hours at ambient temperature, diluted with 100 ml. of ether and stirred for 5 days at ambient temperature. The mixture is treated with water and extracted with ether. The dried extracts are evaporated to yield the product.

EXAMPLE 208

2,3-Dihydroxypropyl 4-(8-cyclohexyloctylamino)benzoate

A solution of 11.8 g. of 4-(8-cyclohexyloctylamino)benzoic acid, 1.00 g. of glycerol, and 5.35 ml. of boron trifluoride etherate in 200 ml. of toluene is stirred under reflux for 48 hours. The solution is treated with an additional 5.35 ml. of boron trifluoride etherate and refluxing is continued for 120 hours. Dilution with water and methylene chloride followed by filtration affords the product as a white solid.

EXAMPLE 209

2,3-Epoxypropyl 4-(8-cyclohexyloctylamino)benzoate

A mixture of 89.0 g. of epichlorohydrin, 92.0 g. of sodium 4-(8-cyclohexyloctylamino)benzoate, and 350 ml. of hexamethylphosphoramide is stirred at 105° C. for 5 hours, allowed to cool, and poured into 1.0 liter of water. The white solid is collected by filtration, recrystallized from

acetonitrile and then from hexane-methylene chloride to yield the product.

EXAMPLE 210

Preparation of N-[4-(8-cyclohexyloctylamino)benzoyl]-piperidide

To a chilled solution of 35 ml. of piperidine, 2.5 ml. of triethylamine and 0.6 g. of dimethylaminopyridine in 100 ml. of diethyl ether is added (1/2 hour) a solution of 8.3 g. of 4-(8-cyclohexyloctylamino)benzoyl chloride hydrochloride in 50 ml. of ether. The solution is warmed to room temperature and maintained there for two hours. The solution is heated to reflux for an additional two hours at which time the reaction was complete. The solution is cooled, extracted twice with 100 ml. portions of water and dried over $MgSO_4$. The solvent is removed *in vacuo* and the solid is recrystallized from ether to yield the product.

EXAMPLE 211

Preparation of ethyl 4-(8-cyclohexyloctylamino)hippurate

A solution of 18.0 g. of 4-(8-cyclohexyloctyl-amino)benzoyl chloride hydrochloride in 100 ml. of dioxane is added to freshly prepared ethyl glycinate in 300 ml. of $CH_2Cl_2$ containing 1 g. of dimethylaminopyridine and 10 ml. of triethylamine. After 16 hours at room temperature the reaction mixture was refluxed for 2 hours, cooled and filtered. The mother liquor is extracted with water and 10% hydrochloric acid. The solution is dried over magnesium sulfate and concentrated *in vacuo* to an amber liquid. A 6 g. sample is pre-absorbed on 30 g. of silica III and placed on 250 ml. of alumina and eluted with ether giving 4.2 g. of solid. Thin-layer chromatography (hexane:ethylacetate, 3:1) indicates three components so this material (3 g.) is chromatographed on silica giving a solid which is recrystallized from acetonitrile to yield the product.

EXAMPLE 212

Preparation of N-[4-(8-cyclohexyloctylamino)benzoyl]glycine

A mixture of 26.4 g. of ethyl N-[4-(8-cyclohexyl-octylamino)benzoyl]glycinate, 110 ml. of 1N sodium hydroxide

solution; and 100 ml. of ethanol is stirred at ambient temperature for 2 hours and then partially evaporated. The aqueous solution is washed with diethyl ether, acidified with 6N hydrochloric acid, and filtered. The white solid is dried in vacuo and recrystallized from acetone to yield the product.

EXAMPLE 213

Preparation of p-(8-cyclohexyloctylamino)-N-(phenylsulfonyl)benzamide

A solution of 31.4 g. of benzenesulfonamide in 250 ml. dry dimethylacetamide is added dropwise, with stirring and cooling, to a suspension of 5.5 g. of sodium hydride in 100 ml. of dry dimethylacetamide over 30 minutes at room temperature. Stirring is continued for a further 30 minutes. In the meantime, a mixture of 36.2 g. of p-(8-cyclohexyloctyl)aminobenzoic acid in 1200 ml. of methylene chloride, 300 ml. of dimethoxyethane, and 40 ml. of thionyl chloride is refluxed for 1 hour and 15 minutes. The solution is evaporated to an oil.

To the resulting oily residue of p-(8-cyclohexyloctyl)aminobenzoyl chloride is added, in one portion, the previously prepared mixture of sodium benzenesulfonamide in dimethylacetamide. The mixture is stirred for 30 minutes, without cooling, and is then filtered through a bed of diatomaceous earth. The filtrate is poured into 2 l. of water, and 250 ml. of saturated sodium chloride solution is added to coagulate the precipitate. The mixture is filtered and the product is washed with water and partially air dried. The product is dissolved in methylene chloride, the mixture is filtered through diatomaceous earth, and brine is added to break the emulsion. The layers are separated, the organic phase is dried over anhydrous sodium sulfate and filtered through a bed of 300 g. of hydrous magnesium silicate. The product is eluted with an additional 3 l. of methylene chloride. The first approx. 1 l. of filtrate is set aside and the remainder is evaporated to dryness. The

residue is crystallized three times from toluene to yield the product.

EXAMPLE 214

Preparation of 3-[4-(8-cyclohexyloctylamino)benzoyl]carboxythiazolidine

One-tenth mole of 4-(8-cyclohexyloctylamino)-benzoyl chloride hydrochloride in methylene chloride is added to a solution of 0.1 mole of ethyl thiazolidine-4-carboxylate in chloroform containing two equivalents of triethylamine. After 5 hours at 20° C. the solution is filtered and evaporated to a white solid which is recrystallized from acetonitrile. By means of the alkaline hydrolysis method of Example 1, the ethyl ester is converted to the subject carboxylic acid. This acid is also prepared using the procedure of this Example except that the acylation of the thiazolidine-4-carboxylic acid is carried out in aqueous acetone sodium bicarbonate solution.

EXAMPLE 215

Preparation of N-[4-(8-cyclohexyloctylamino)benzoyl)piperide

To a warm solution of 4-[N-carbobenzoyloxy-N-8-(cyclohexyl)octylamino]benzoyl chloride and 1.3 g. of triethylamine in 100 ml. ether is added 1.2 g. of piperidine. An immediate precipitate forms, but the mixture is refluxed for one hour and filtered while hot. The solid is washed several times with hot ether, then the ether is evaporated to yield a white solid. The product is dissolved in tetrahydrofuran (100 ml.) and hydrogenated over 600 mg. 10% Pd-on-carbon at 50 psi. until hydrogen up-take stops. The catalyst is filtered. The solution is evaporated, and the residue is crystallized from acetic acid to yield the title compound as a crystalline mass.

EXAMPLE 216

Preparation of 4-[N-(8-cyclohexyloctyl)-amino]-N-2,3-dihydroxypropylbenzamide

To a mixture containing 4.3 g. of 1- 4-[N-(t-butyloxycarbonyl)-N-(8-cyclohexyloctyl)amino]benzoyl imidazole, 50 ml. of chloroform and 50 ml. of 5N sodium hydroxide is added 1.1 g. of 3-amino-1,2-propanediol. The solution is vigorously stirred for 24 hours, the layers are separated, and the chloroform solution is washed once with 50 ml. of 1N sodium hydroxide. The solvent is evaporated and the residue is heated for 30 minutes at 40 C. in 50 ml. of anhydrous trifluoroacetic acid. The solvent is again evaporated and the oil is crystallized from acetone to yield light yellow crystals of the product.

EXAMPLE 217

Preparation of N-[4-(8-cyclohexyloctylamino)benzoyl]-2-aminoethanesulfonic acid

To a stirred solution of 2.50 g. of taurine and 5.6 ml. of triethylamine in 22.5 ml. of water is added 5.55 g. of N-{p-[2,2,2-trifluoro-N-(8-cyclohexyloctyl)acetamido]-benzoyloxy} succinimide as a solution in 45 ml. of ethanol. After 24 hours, the mixture is treated with 20 ml. of 2.0 M sodium hydroxide and 25 ml. of water. After stirring for 10 min., the mixture is acidified with dilute hydrochloric acid, and the crude product is collected by filtration. Recrystallization affords the title compound as a white solid.

EXAMPLE 218

Preparation of 4-[(8-cyclohexyloctyl)amino]-N-3-bromopropyl-benzamide

To a slurry of 21.80 g. of 2-bromopropylamine hydrobromide in 200 ml. of glyme at 3° C. is added a solution of 23.96 g. of 4-(8-cyclohexyloctylamino)benzoyl chloride hydrochloride in 65 ml. of glyme, concurrently with 26 ml. of triethylamine diluted to 39 ml. with glyme. The solution is warmed to reflux and 0.2 g. of 4-dimethylaminopyridine is added. The solution is heated for four hours and cooled overnight. The solid is removed by filtration and the filtrate diluted with 200 ml. of water. The precipitate is collected and crystallized from cyclohexane. The solid is recrystallized from acetonitrile to yield the product.

EXAMPLE 219

Preparation of 2-[4-(8-cyclohexyloctylamino)phenyl]-5,6-dihydro[4H]-1,3-oxazine

To 0.4 g. of NaH in 100 ml. of glyme is added 2.14 g. of N-(3-bromopropyl)-4-(8-cyclohexyloctylamino)benzamide and 12 ml. of triethylamine. The turbid solution is heated under reflux for 20 hours. The solution is diluted with 100 ml. of water and cooled overnight. The solid is collected, washed with water and dried to yield 2 g. of solid which gave a negative silver nitrate test and halogen flame test. This solid is recrystallized from cyclohexane to yield the product.

EXAMPLE 220

Preparation of 2-[4-(8-cyclohexyloctylamino)phenyl]oxazoline

To a slurry of 15 g. of 2-bromoethylamine hydrobromide in 150 ml. of glyme are added simultaneously solutions of 31 g. of 4-(8-cyclohexyloctylamino)benzoyl chloride hydrochloride in 60 ml. of glyme and 50 ml. of triethylamine (dropwise). After the addition of 0.5 g. of 4-dimethylaminopyridine the "solution" is stirred at room temperature overnight, refluxed for one hour and filtered. The solid is dried and partitioned between methylene chloride and water. The layers are separated and the methylene chloride solution is dried over magnesium sulfate, concentrated to a volume of about 100 ml., and diluted with an equal volume of hexane. The product is collected by filtration and recrystallized from cyclohexane and then from acetonitrile and methylene chloride hexane to yield the product.

EXAMPLE 221

Preparation of p-(allylamino)benzoic acid; (Method A)

To a solution of 18.2 g. of ethyl p-aminobenzoate in 100 ml. of dimethylformamide is added a solution of 4.7 ml. of allyl bromide in 60 ml. of dimethylformamide. The solution is heated at 60° C. for 5 hours and then cooled and partitioned between diethyl ether and water. The combined ether phases are washed with water, dried over magnesium

sulfate, and concentrated *in vacuo* to provide 16.5 g. of a semi-solid. A portion (6 g.) is absorbed onto 30 g. of silica gel and chromatographed on 475 g. of silica gel to provide the benzoate ester.

A mixture of the ester, 22.0 g. of potassium hydroxide and 200 ml. of ethanol-water (8:1) is stirred under reflux for 6 hours. Concentrated hydrochloric acid (about 80 ml.) is added to the warm mixture and cooling and dilution with water affords a white solid which is collected by filtration and recrystallized from ethanol to yield the product as a white solid.

EXAMPLE 222

Preparation of p-(1-pentadeca-4,14-dienylamino)benzoic acid;

(Method B)

To a solution of 4,14-pentadecadien-1-ol (15.0 g.) and triethylamine (14 ml.) in dry methylene chloride (320 ml.) at -8° C. is added methanesulfonylchloride (5.73 ml.), dropwise. The reaction mixture is stirred at -10° C. for 30 minutes and then diluted with methylene chloride, extracted with ice-water (250 ml.), followed by cold 10% hydrochloric acid (200 ml.); cold saturated sodium bicarbonate (200 ml.) and cold brine (200 ml.). The organic phase is dried over magnesium sulfate and the solvent removed *in vacuo* to provide the crude mesylate.

A solution of 18.1 g. of the above mesylate and 19.8 g. of ethyl p-aminobenzoate in hexamethylphosphoramide is heated at 120° C. for 20 hours. After cooling, the reaction mixture is diluted with 30 ml. of ethanol:water (1:1) (30 ml.) and chilled. More ethanol is added and the solid material is collected. This solid is recrystallized twice from ethanol to provide the benzoate ester.

A mixture of the ester, 22.0 g. of potassium hydroxide and 200 ml. of ethanol-water (8:1) is stirred under reflux for 6 hours. Concentrated hydrochloric acid (about 80 ml.) is added to the warm mixture and cooling and dilution with water affords a white solid which is collected

by filtration and recrystallized from ethanol to yield the product as a white solid.

EXAMPLES 223 - 537

Treatment of the indicated halide or carbinol starting material set forth in Table III below by the indicated method is productive of the product listed in the table. Reference B in Tables III and IV is J. Med. Chem. 11, 1190 (1968).

TABLE III

| Example | Starting Material | Method | Product |
|---|---|---|---|
| 223 | E-4-tetradecenol | B | p-(E-1-tetradec-4-enylamino)benzoic acid |
| 224 | Z-9-octadecen-1-ol | B | p-(Z-1-octadec-9-enylamino)benzoic acid |
| 225 | E-4-pentadecen-1-ol | B | p-(E-1-pentadec-4-enylamino)benzoic acid |
| 226 | 3-chloro-2,4,4-trimethyl-1-pentene Chem. Abst. 72, 111081h | A | p-[3-(2,4,4-trimethyl)-pent-1-enylamino]-benzoic acid |
| 227 | 3-bromo-3-isopropyl-4-methyl-1-pentene Chem. Abst. 54, 4355a | A | p-[3-(3-isopropyl-4-methyl)-pent-1-enylamino]benzoic acid |
| 228 | 4-bromo-2-heptene Chem. Abst. 70, 67482x | A | p-(4-hept-2-enylamino)-benzoic acid |
| 229 | 4-bromo-2,4-dimethyl-2-hexene | A | p-[4-(2,4-dimethylhex-2-enyl)amino]benzoic acid |
| 230 | 5-chloro-3,5-dimethyl-3-heptene Chem. Abst. 54, 1256e | A | p-[5-(3,5-dimethyl-hept-3-enyl)amino]-benzoic acid |
| 231 | Z-1-hydroxy-2-hexadecene Ref. B | B | p-(Z-1-hexadec-2-enylamino)benzoic acid |
| 232 | E-1-hydroxy-2-hexadecene Ref. B | B | p-(E-1-hexadec-2-enylamino)benzoic acid |

| Example | Starting Material | Method | Product |
|---|---|---|---|
| 233 | 1-bromo-4-methyl-3-heptene Chem. Abst. 71, 102020g | A | p-[1-(4-methylhept-3-enyl)amino]benzoic acid |
| 234 | 1-bromo-4-methyl-3-nonene Chem. Abst. 71, 101399h | A | p-[1-(4-methylnon-3-enyl)amino]benzoic acid |
| 235 | E-7-bromo-3-heptene Chem. Abst. 74, 99419f | A | p-(1-hept-4-enylamino)-benzoic acid |
| 236 | 1-bromo-5,9-dimethyl-4-decene Chem. Abst. 51, 8699g | A | p-[1-(5,9-dimethyl-dec-4-enyl)amino]-benzoic acid |
| 237 | 1-methanesulfonyl-oxy-4-tetradecene Ref. B. | B | p-(1-tetradec-4-enyl-amino)benzoic acid |
| 238 | 1-methanesulfonyl-oxy-4-hexadecene Ref. B. | B | p-(1-hexadec-4-enyl-amino)benzoic acid |
| 239 | 6-bromo-1-hexene Chem. Abst. 66, 2142j | A | p-(1-hex-5-enyl-amino)benzoic acid |
| 240 | 6-bromo-2-methyl-1-hexene Chem. Abst 75, 109624f | A | p-[1-(5-methylhex-5-enyl)amino]benzoic acid |
| 241 | 6-chloro-1-heptene-Chem. Abst. 72, 31877g | A | p-(2-hept-6-enylamino)-benzoic acid |
| 242 | 6-bromo-2-methyl-2-heptene Chem. Abst. 54, 13166f | A | p-[2-(2,6-dimethyl-hept-5-enyl)amino]-benzoic acid |
| 243 | 7-chloro-2-octene Chem. Abst. 75, 129245m | A | p-(2-oct-6-enylamino)-benzoic acid |
| 244 | E-1-chloro-4-nonene Chem. Abst. 67, 32294g | A | p-(E-1-non-5-enyl-amino)benzoic acid |
| 245 | 7-bromo-1-heptene | A | p-(1-hept-6-enyl-amino)benzoic acid |

| Example | Starting Material | Method | Product |
|---|---|---|---|
| 246 | 7-chloro-1-octene Chem. Abst. 75, 29245m | A | p-(2-oct-7-enylamino)-benzoic acid |
| 247 | 6-bromo-6-methyl-1-heptene Chem. Abst. 66, 94482w | A | p-[1-(2-methylhept-6-enyl)amino]benzoic acid |
| 248 | 6-chloro-6-methyl-1-heptene Chem. Abst. 75, 129245 | A | p-[1-(6-methylhept-6-enyl)amino]benzoic acid |
| 249 | E-8-bromo-2-octene Chem. Abst. 74, 99419f | A | p-(1-oct-6-enylamino)-benzoic acid |
| 250 | 8-bromo-2,6-dimethyl-2-octene Chem. Abst. 72, 90573c | A | p-[1-(3,7-dimethyl-oct-6-enyl)amino]-benzoic acid |
| 251 | 11-bromo-5-undecen-Chem. Abst. 67, 73101b | A | p-(1-undec-6-enyl-amino)benzoic acid |
| 252 | 8-bromo-1-octene Chem. Abst. 70 10990g | A | p-(1-oct-7-enylamino)-benzoic acid |
| 253 | R-8-iodo-7-methyl-1-octene Chem. Abst. 74, 12573e | A | p-[R-(2-methyloct-7-enyl)amino]benzoic acid |
| 254 | 1-chloro-7-tetra-decene Chem. Abst. 54, 22461h | A | p-(1-tetradec-7-enyl-amino)benzoic acid |
| 255 | 9-chloro-1-nonene Chem. Abst. 70, 11490k | A | p-(1-non-8-enylamino)-benzoic acid |
| 256 | 1-bromo-8-hepta-decene Chem. Abst. 52, 249d | A | p-(1-heptadec-8-enyl-amino)benzoic acid |
| 257 | E-1-bromo-9-octa-decene Chem. Abst. 70, 46779j | A | p-(E-1-octadec-9-enyl-amino)benzoic acid |
| 258 | Z-1-bromo-9-octa-decene Chem. Abst. 70, 46779j | A | p-(Z-1-octadec-9-enyl-amino)benzoic acid |

| Example | Starting Material | Method | Product |
|---|---|---|---|
| 259 | 11-chloro-1-undecene Chem. Abst. 66, P19046d | A | p-(1-undec-10-enyl-amino)benzoic acid |
| 260 | 12-iodo-3,7,11-trimethyl-1-dodecene | A | p-[1-(2,6,10-trimethyl-dodec-11-enyl)amino]-benzoic acid |
| 261 | 13-bromo-1-tridecene Chem. Abst. 67, 43348v | A | p-(1-tridec-12-enyl-amino)benzoic acid |
| 262 | 22-bromo-9-docosene Chem. Abst. 73, 44976j | A | p-[1-(2-methylhept-6-enyl)amino]benzoic acid |
| 263 | 16-methanesulfonyl-oxy-1-decene Ref. B | B | p-(1-hexadec-15-enyl-amino)benzoic acid |
| 264 | propargyl alcohol | B | p-(1-prop-2-ynyl-amino)benzoic acid |
| 265 | 3-chloro-1-butyne | A | p-(2-but-3-ynylamino-benzoic acid |
| 266 | 3-chloro-3-methyl-nonyne Chem. Abst. 55, 22090i | A | p-[3-(3-methylnon-1-ynyl)amino]benzoic acid |
| 267 | 3-bromo-1-pentadecyne Chem. Abst. 53, 21638c | A | p-(3-pentadec-1-ynyl-amino)benzoic acid |
| 268 | 1-octyn-3-ol Chem. Abst. 66, 85410n | B | p-(3-oct-1-ynylamino)-benzoic acid |
| 269 | 3,7,11,15-tetra-methyl-1-hexadecyn-3-ol | B | p-[3-(3,7,11,15-tetra-methylhexadec-1-ynyl)-amino]benzoic acid |
| 270 | 2-butyn-1-ol | B | p-(3-hex-4-ynylamino)-benzoic acid |
| 271 | 4-hexyn-3-ol | B | p-(3-hex-4-ynylamino)-benzoic acid |
| 272 | 2-methyl-3-pentyn-2-ol Chem. Abst. 69, 10496e | B | p-[2-(2-methylpent-3-ynyl)amino]benzoic acid |
| 273 | 2-octyn-1-ol | B | p-(1-oct-3-ynylamino)-benzoic acid |

| Example | Starting Material | Method | Product |
|---|---|---|---|
| 274 | 4-decyn-3-ol Chem. Abst. 69, P4630s | B | p-(3-dec-4-ynylamino)-benzoic acid |
| 275 | 1-bromo-2-dodecyne Chem. Abst. 28, 40345 | A | p-[1-dodec-2-ynylamino)-benzoic acid |
| 276 | 1-methanesulfonly-oxy-2-pentadecyne J. Med. Chem. 19, 946 (177) | B | p-(1-pentadec-2-ynyl-amino)benzoic acid |
| 277 | 3-butyne-1-ol Chem. Abst. 66, P2319a | B | p-(1-but-3-ynylamino)-benzoic acid |
| 278 | 1-undecyn-4-ol Chem. Abst. 70, 3219j | B | p-(4-undec-1-ynylamino)-benzoic acid |
| 279 | 2-methyl-4-pentyn-2-ol Chem. Abst. 68, 12200g | B | p-[2-(2-methylpent-4-ynyl)amino]benzoic acid |
| 280 | 4-pentyn-2-ol Chem. Abst. 64, 13537e | B | p-(2-pent-4-ynylamino)-benzoic acid |
| 281 | 3-pentyn-1-ol | B | p-(1-pent-3-ynylamino)-benzoic acid |
| 282 | 4-hexyn-2-ol | B | p-(2-hex-4-ynylamino)-benzoic acid |
| 283 | 2-methyl-3-pentyn-1-ol Chem. Abst. 66, 11524k | B | p-[1-(2-methyl-3-pent-3-ynyl)amino]benzoic acid |
| 284 | 2-(1-propynyl)-1-heptanol Chem. Abst. 66, 115242k | B | p-[1,2-(1-propynyl-heptyl)amino]benzoic acid |
| 285 | 2-methyl-4-nonyn-2-ol Chem. Abst. 68, 104593r | B | p-[2-(2-methylnon-4-ynyl)amino]benzoic acid |
| 286 | 2-methyl-3-nonyn-1-ol Chem. Abst. 66, 115242k | B | p-[1-(2-methylnon-3-ynyl)amino]benzoic acid |

| Example | Starting Material | Method | Product |
|---|---|---|---|
| 287 | 3-nonyn-1-ol Chem. Abst. 75, 5165r | B | p-(1-non-3-ynylamino)-benzoic acid |
| 288 | 2-methyl-3-decyn-1-ol Chem. Abst. 66,11524k | B | p-[1-(2-methyldec-3-ynyl)amino]benzoic acid |
| 289 | 5-chloro-1-pentyne | A | p-(1-pent-4-ynylamino)-benzoic acid |
| 290 | 4-hexyn-1-ol Chem. Abst. 74, 9800w | B | p-(1-hex-4-ynylamino)-benzoic acid |
| 291 | 1-chloro-4-nonyne | A | p-(1-non-4-ynylamino)-benzoic acid |
| 292 | 1-chloro-4-tri-decyne Chem. Abst. 32, 7426[3] | A | p-(1-pentadec-2-ynyl-amino)benzoic acid |
| 293 | 1-chloro-4-hexa-decyne | A | p-(1-hexadec-4-ynyl-amino)benzoic acid |
| 294 | 5-hexyn-1-ol Chem. Abst. 74, 9800w | B | p-(1-hex-5-ynylamino)-benzoic acid |
| 295 | 6-octyn-2-ol Chem. Abst. 71, 60300y | B | p-[2-methylocty-6-ynyl)amino]benzoic acid |
| 296 | 1-iodo-5-decyne Chem. Abst. 51, 12817f | A | p-(1-dec-5-ynylamino)-benzoic acid |
| 297 | 5-tetradecyn-1-ol Chem. Abst. 71, 120523k | B | p-(1-tetradec-5-ynyl-amino)benzoic acid |
| 298 | 5-octadecyn-1-ol Chem. Abst. 72, 42686v | B | p-(1-octadec-5-ynyl-amino)benzoic acid |
| 299 | 6-octadecyn-1-ol Chem. Abst. 72, 42686v | B | p-(1-octadec-6-ynyl-amino)benzoic acid |

| Example | Starting Material | Method | Product |
|---|---|---|---|
| 300 | 10-chloro-3-decyne Chem. Abst. 67, 108147a | A | p-(1-dec-7-ynylamino)-benzoic acid |
| 301 | 1-chloro-7-tetra-decyne Chem. Abst. 54, 22461e | A | p-(1-tetradec-7-ynyl-amino)benzoic acid |
| 302 | 7-hexadecyn-1-ol Chem. Abst. 71, 19554w | B | p-(1-hexadec-7-ynyl-amino)benzoic acid |
| 303 | 7-octadecyn-1-ol Chem. Abst. 67, 81784w | B | p-(1-octadec-7-ynyl-amino)benzoic acid |
| 304 | 8-octadecyn-1-ol Chem. Abst. 72, 42686v | B | p-(1-octadec-8-ynyl-amino)benzoic acid |
| 305 | 9-decyn-1-ol Chem. Abst. 67, 81784s | B | p-(1-decyn-9-ynylamino)-benzoic acid |
| 306 | 10-octadecyn-1-ol Chem. Abst. 67, 81784s | B | p-(1-octadec-10-ynyl-amino)benzoic acid |
| 307 | 1-tricosyn-1-ol Chem. Abst. 67, 81787s | B | p-(1-non-4-ynylamino)-benzoic acid |
| 308 | 11-dodecyn-1-ol Chem. Abst. 68, 39015n | B | p-(1-dodec-11-ynyl-amino)benzoic acid |
| 309 | 11-tetradecyn-1-ol Chem. Abst. 75, 16792u | B | p-(1-tetradec-11-ynyl-amino)benzoic acid |
| 310 | 11-tridecyn-1-ol Chem. Abst. 75, 167927u | B | p-(1-tridec-11-ynyl-amino)benzoic acid |
| 311 | 16-bromo-5-hexa-decyne Chem. Abst. 68, 2536g | A | p-(1-hexadec-11-ynyl-amino)benzoic acid |
| 312 | 12-tridecyn-1-ol Chem. Abst. 68, 39015n | B | p-(1-tridec-12-ynyl-amino)benzoic acid |

| Example | Starting Material | Method | Product |
|---|---|---|---|
| 313 | 12-octadecyn-1-ol Chem. Abst. 72, 42686v | B | p-(1-octadec-12-ynyl-amino)benzoic acid |
| 314 | 13-tetradecyn-1-ol Chem. Abst. 68, 390115n | B | p-(1-tetradec-13-ynyl-amino)benzoic acid |
| 315 | 14-pentadecyn-1-ol Chem. Abst. 68, 39015n | B | p-(1-pentadec-14-ynyl-amino)benzoic acid |
| 316 | 5,7-octadien-4-ol Chem. Abst. 72, 42686v | B | p-(4-octa-5,7-dienyl-amino)benzoic acid |
| 317 | 4-ethyl-3,5-hexa-decadien-2-ol Chem. Abst. 70, 20116r | B | p-[2-(4-ethylhexa-3,5-dienyl)amino] benzoic acid |
| 318 | 2-methyl-4,6-hepta-dien-3-ol Chem. Abst. 71, 112534z | B | p-[3-(2-methylhepta-4,6-dienyl)amino benzoic acid |
| 319 | E-2,4,6,trimethyl-3,5-heptadien-2-ol | B | p-[E-2-(2,4,6-tri-methylhepta-3,5-di-enyl)amino] benzoic acid |
| 320 | 4,6-octadien-3-ol Chem. Abst. 69, 58770s | B | p-(1-hexa-2,4-dienyl-amino)benzoic acid |
| 321 | 2,4-hexadien-1-ol Chem. Abst. 67, 81787s | B | p-(1-hexa-2,4-dienyl-amino)benzoic acid |
| 322 | 6-isopropyl-7,7-dimethyl-3,5-octa-dien-2-ol Chem. Abst. 75, 36362g | B | p-[2-(6-isopropyl-7,7-dimethylocta-3,5-dienyl)amino]benzoic acid |
| 323 | 3,5,7-trimethyl-4,6-nonadien-3-ol Chem. Abst. 74, 42252s | B | p-[3-(3,5,7-trimethyl-nona-4,6-dienyl)amino] benzoic acid |
| 324 | 2,6-dimethyl-3,5-octadien-2-ol Chem. Abst. 68, 114750d | B | p-[2-(2,6-dimethyl-octa-3,5-dienyl)amino] benzoic acid |

| Example | Starting Material | Method | Product |
|---|---|---|---|
| 325 | E,E-6-ethyl-4,6-decadien-3-ol Chem. Abst. 73, 130585n | B | p-[E,E-3-(6-ethyl-deca-4,6-dienyl)amino]-benzoic acid |
| 326 | 5-ethyl-3,5-nona-dien-2-ol | B | p-[2-(5-ethylnona-3,5-dienyl)amino]benzoic acid |
| 327 | E,E-2,4-octadien-1-ol Chem. Abst. 67, 104696n | B | p-(E,E-1-octa-2,4-di-enylamino)benzoic acid |
| 328 | E,Z-2,4-nonadien-1-ol Chem. Abst. 67, 99692w | B | p-(E,Z-1-nona-2,4-di-enylamino)benzoic acid |
| 329 | E,E-2,4-decadien-1-ol Chem. Abst. 67, 104946N | B | p-(E,E-1-deca-2,4-di-enylamino)benzoic acid |
| 330 | E,Z-2,4-decadien-1-ol Chem. Abst. 67, 99692v | B | p-(E,Z-1-deca-2,4-di-enylamino)benzoic acid |
| 331 | E-2,5-hexadien-1-ol Chem. Abst. 73, P55709a | B | p-(E-1-hexa-2,5-dienyl-amino)benzoic acid |
| 332 | Z-2,5-hexadienyl-1-ol Chem. Abst. 73, P55709a | B | p-(Z-1-hexa-2,5-dienyl-amino)benzoic acid |
| 333 | E(+)-4-ethyl-2,5-di-methyl-2,5,hexadien-1-ol Chem. Abst. 71, 70152m | B | p-[E(+)-1-(2,5-di-methylhexa-2,5-dienyl)-amino]benzoic acid |
| 334 | E-2,5,5-trimethyl-3,6-heptadien-10-ol Chem. Abst. 74 52962n | B | p-[E-2-(2,5,5-tri-methylhepta-3,6-di-enyl)amino]benzoic acid |
| 335 | Z-2,5,5-trimethyl-3,6-heptadien-1-ol Chem. Abst. 74, 52962n | B | p-[Z-2-(2,5,5-tri-methylhepta-3,6-di-enyl)amino]benzoic acid |
| 336 | Z,E-3,7-dimethyl-2,5-octadien-1-ol Chem. Abst. 71, 61565a | B | p-[Z-E-1-(3,7-dimethyl-octa-2,5-dienyl)amino]-benzoic acid |

| Example | Starting Material | Method | Product |
|---|---|---|---|
| 337 | E-2,6-dimethyl-2,6-heptadien-1-ol Chem. Abst. 72, 32036u | B | p-[E-1-(2,6-dimethyl-hepta-2,6-dienyl)-amino]benzoic acid |
| 338 | E,E-2,6-octadien-1-ol | B | p-(E,E-1-octa-2,6-di-enylamino)benzoic acid |
| 339 | E,E-2,6-dimethyl-2,6-octadien-1-ol Chem. Abst. 71,22204N | B | p-[E,E-1-(2,6-dimethyl-octa-2,6-dienyl)amino]-benzoic acid |
| 340 | Z,E-2,6-dimethyl-octadien-1-ol Chem. Abst. 71, 22204n | B | p-(Z,E-1-(2,6-dimethyl-octa-2,6-dienylamino)-benzoic acid |
| 341 | E,Z-2,6-nonadien-1-ol Chem. Abst. 72, 24504e | B | p-(E,Z-1-nona-2,6-di-enylamino)benzoic acid |
| 342 | E,Z-3-ethyl-7-methyl-2,6-nonadien-1-ol Chem. Abst. 75, 63019g | B | p-[E,Z-1-(3-ethyl-7-methylnona-2,6-dienyl)-amino]benzoic acid |
| 343 | 3,7,11,11-tetramethyl-2,6-dodecadien-1-ol Chem. Abst. 75, P117969n | B | p-[1-(3,7,11,11-tetra-methyldodeca-2,5-di-enyl)amino]benzoic acid |
| 344 | E,E-3,7,1-trimethyl-2,6-dodecadien-1-ol Chem. Abst. 69, 978z | B | p-[E,E-1-(3,7,11-tri-methyldodeca-2,6-di-enyl)amino]benzoic acid |
| 345 | E,Z-3,7,11-trimethyl-2,6-dodecadien-1-ol Chem. Abst. 69, 978g | B | p-(E,Z-1-(3,7,11-tri-methyldodeca-2,6-di-enyl)amino]benzoic acid |
| 346 | 2,7-octadien-1-ol Chem. Abst. 74, P41892p | B | p-(1-octa-2,7-dienyl-amino)benzoic acid |
| 347 | E-3,7-dimethyl-2,7-octadien-1-ol Chem.Abst. 68, 114750d | B | p-[E-1-(3,7,dimethyl-octa-2,7-dienyl)amino]-benzoic acid |

| Example | Starting Material | Method | Product |
|---|---|---|---|
| 348 | Z-3,7-dimethyl-2,7-octadien-1-ol Chem. Abst. 68, 114750d | B | p-[Z-1-(3,7-dimethyl-octa-2,7-dienyl)amino]-benzoic acid |
| 349 | 3,4,8-trimethyl-2,7-nonadien-1-ol Chem. Abst. 68, 9184c | B | p-[-(3,4,8-trimethyl-nona-2,7-dienyl)amino]-benzoic acid |
| 350 | 3,4,8-trimethyl-2,8-(-nonadien-1-ol Chem. Abst. 68,29184c | B | p-[1-(3,4,8-trimethyl-nona-2,8-dienyl)amino]-benzoic acid |
| 351 | 2,9-decadien-1-ol Chem. Abst. 68, 68373h | B | p-(1-deca-2,9-dienyl-amino)benzoic acid |
| 352 | E-3,7,11-trimethyl-2,10-dodecadien-1-ol Chem.Abst. 69, 978z | B | p-[E-1-(3,7,11-tri-methyldodeca-2,10-di-enyl)amino]benzoic acid |
| 353 | Z-3,7,11-trimethyl-2,10-dodecadien-1-ol | B | p-[Z-1-(3,7,11-tri-methyldodeca-2,10-di-enyl)amino]benzoic acid |
| 354 | E,E-4-methyl-3,5-heptadien-1-ol Chem. Abst. 6,104730s | B | p-[E,E-1-(4-methyl-hepta-3,5-dienyl)-amino]benzoic acid |
| 355 | E,Z-4-methyl-3,5-heptadien-1-ol Chem. Abst. 66 104730s | B | p-[E,Z-1-(4-methyl-hepta-3,5-dienyl)-amino]benzoic acid |
| 356 | Z,E-4-methyl-3,5-heptadien-1-ol Chem. Abst. 66, 104730s | B | p-[Z,E-1-(4-methyl-hepta-3,5-dienyl)-amino]benzoic acid |
| 357 | Z,Z-4-methyl-3,5-heptadien-1-ol Chem. Abst. 66, 104730s | B | p-[Z,Z-1-(4-methyl-hepta-3,5-dienyl)-amino]benzoic acid |
| 358 | E-4,6-dimethyl-3,5-heptadien-1-ol Chem. Abst. 66, 104730s | B | p-[E-1-(4,6-dimethyl-hepta-3,5-dienyl)-amino]benzoic acid |

| Example | Starting Material | Method | Product |
|---|---|---|---|
| 359 | Z-4,6-dimethyl-3,5-heptadien-1-ol Chem. Abst. 66, 104730s | B | p-[Z-1-(4,6-dimethylhepta-3,5-dienylamino]-benzoic acid |
| 360 | E,Z-2,6-dimethyl-4,6-octadien-2-ol Chem. Abst. 68, 114750d | B | p-[E,Z-2-(2,6-dimethylocta-4,6-dienyl)amino]-benzoic acid |
| 361 | 1-hydroxy-3,7,11-trimethyl-2,6,10-dodecatriene | B | p-[1-(3,7,11-trimethyldodeca-2,6,10-trienyl)-amino]benzoic acid |
| 362 | 5-methyl-3,5-octadien-1-ol Chem. Abst. 69, 18519k | B | p-[1-(5-methylocta-3,5-dienyl)amino]benzoic acid |
| 363 | E-4-methyl-3,6-heptadien-1-ol Chem. Abst. 66, 10473s | B | p-[E-1-(4-methylhepta-3,6,dienyl)amino]-benzoic acid |
| 364 | Z-4-methyl-3,6-heptadien-1-ol Chem. Abst. 66, 104730s | B | p-[Z-1-(4-methylhepta-3,6-dienyl)amino]-benzoic acid |
| 365 | E(+)-2,6-dimethyl-4,7-octadien-2-ol Chem. Abst. 75, 49345d | B | p-[E(+)-2-(2,6-dimethylocta-4,7-dienyl)-amino]benzoic acid |
| 366 | Z(+)-2,6-dimethyl-4,7-octadien-2-ol Chem. Abst. 75, 49345d | B | p-[Z(+)-2-(2,6-dimethylocta-4,7-dienyl)-amino]benzoic acid |
| 367 | E-3,7-dimethyl-3,6-octadien-1-ol Chem. Abst. 67, 64554z | B | p-[E-1-(3,7-dimethylocta-3,6-dienyl)amino]-benzoic acid |
| 368 | Z-3,7-dimethyl-3,6-octadien-1-ol | B | p-[Z-1-(3,7-dimethylocta-3,6-dienyl)-amino]benzoic acid |
| 369 | E-3-methyl-3,7-octadien-1-ol Chem. Abst. 69, 65681x | B | p-[E-1-(3-methylocta-3,7-dienyl)amino]-benzoic acid |

| Example | Starting Material | Method | Product |
|---|---|---|---|
| 370 | Z-3-methyl-3,7-octadien-1-ol Chem. Abst. 69 26681x | B | p-[Z-1-(3-methyl-octa-3,7-dienyl)amino]-benzoic acid |
| 371 | E-2-methyl-4,8-nona-dien-1-ol Chem. Abst. 73, 87253P | B | p-[Z-1-(4,6-dimethyl-hepta-3,5-dienyl)-amino]benzoic acid |
| 372 | E-3,7-dimethyl-3,7-octadien-1-ol Chem. Abst. 69, 26681x | B | p-[E-1-(3,7-dimethyl-octa-3,7-dienyl)amino]-benzoic acid |
| 373 | E-8-methyl-7-methylene-3-nonen-1-ol Chem. Abst. 70, 29098u | B | p-[E-1-(8-methyl-7-methylenenon-3-enyl)-amino]benzoic acid |
| 374 | 2,4,9-trimethyl-4,8-decadien-1-ol Chem. Abst. 74, 99342s | B | p-[2-(2,4,9-trimethyl-deca-4,8-dienyl)amino]-benzoic acid |
| 375 | E-2-methyl-4,9-decadien-1-ol Chem. Abst. 73, 87254g | B | p-[E-2-(2-methyldeca-4,9-dienyl)amino]-benzoic acid |
| 376 | 2,6-dimethyl-5,7-octadien-2-ol Chem. Abst. 72, 37616t | B | p-[2-(2,6-dimethylocta-5,7-dienyl)amino]-benzoic acid |
| 377 | E-3,7-dimethyl-4,6-octadien-1-ol Chem. Abst. 72, 121291r | B | p-[E-1-(3,7-dimethyl-octa-4,6-dienyl)-amino]benzoic acid |
| 378 | Z-3,7-dimethyl-4,6-octadien-1-ol Chem. Abst. 72, 121291r | B | p-[Z-1-(3,7-dimethyl-octa-4,6-dienyl)-amino-benzoic acid |
| 379 | E-4,7-octadien-1-ol Chem. Abst. 66, 28610k | B | p-(E-1-octa-4,7-dienyl-amino)benzoic acid |
| 380 | 5,8-nonadien-2-ol Chem. Abst. 68, 28610k | B | p-(2-nona-5,8-dienyl-amino)benzoic acid |

| Example | Starting Material | Method | Product |
|---|---|---|---|
| 381 | E-7-methyl-4,7-octadien-1-ol Chem. Abst. 66, 28610k | B | p-[E-1-(7-methyl-octa-4,7-dienyl)-amiono]benzoic acid |
| 382 | E-8-methyl-5,8-nonadien-2-ol Chem. Abst. 66, 28610k | B | p-[E-2-(8-methyl-nonal-5,8-dienyl)-amino]benzoic acid |
| 383 | E-6,10-dimethyl-5,9-undecadien-2-ol Chem. Abst. 73, 131152f | B | p-[E-2-(6,10-dimethyl-undeca-5,9-dienyl)-amino]benzoic acid |
| 384 | 5,9,13-trimethyl-8,13-tetradecadien-2-ol Chem. Abst. 70, 28303v | B | p-[6-(5,9,13-trimethyl-tetradeca-8,12-dienyl)-amino]benzoic acid |
| 385 | E-3,7,11-trimethyl-6,10-dodecadien-3-ol Chem. Abst. 69, 8333f | B | p-[E-3-(3,7,11-tri-methyldodeca-6,10-di-enyl)amino]benzoic acid |
| 386 | Z-3,7,11-trimethyl-6,10-dodecadien-3-ol Chem. Abst. 69, 8333f | | p-[Z-3-(3,7,11-tri-methyldodeca-6,10-di-enyl)amino]benzoic acid |
| 387 | 5,9-dimethyl-4,8-decadien-1-ol Chem. Abst. 74, 112233n | B | p-[1-(5,9-dimethyl-deca-4,8-dienyl)amino]-benzoic acid |
| 388 | 15-methanesulfonyl-oxy-1,11-pentadeca-diene Ref. B | B | p-(1-pentadeca-4,14-dienylamino)benzoic acid |
| 389 | 5,7-octadien-1-ol Chem. Abst. 68, 68503a | B | p-(1-octa-5,7-dienyl-amino)benzoic acid |
| 390 | E-3,7-dimethyl-5,7-octadien-1-ol Chem. Abst. 72, P90672j | B | p-[E-1-(3,7-dimeth-octa-5,7-dienyl)-amino]benzoic acid |
| 391 | 6,10-dimethyl-5,9-undecadien-1-ol Chem. Abst. 71, 50248y | B | p-[1-(6,10-dimethyl-undeca-5,9-dienyl)-amino]benzoic acid |

| Example | Starting Material | Method | Product |
|---|---|---|---|
| 392 | 2,6,10-trimethyl-5,9-undecadien-1-ol Chem. Abst. 71, 50243t | B | p-[1-(2,6,10-dimethyl-undeca-5,9-dienyl)-amino]benzoic acid |
| 393 | 10-propyl-5,9-tri-decadien-1-ol Chem. Abst. 68, 39028u | B | p-[1-(10-propyl-trideca-5,9-dienyl)-amino]benzoic acid |
| 394 | 5,13-tetradecadien-2-ol Chem. Abst. 66, 35672k | B | p-[1-tetradeca-5,13-dienylamino]benzoic acid |
| 395 | E-3,7,11-trimethyl-6,10-dodecadien-1-ol Chem. Abst. 69, 978z | B | p-[1-(3,7,11-trimethyl-dodeca-6,10-dienyl)-amino]benzoic acid |
| 396 | E-6,10,14-trimethyl-9,13-pentadecadien-2-ol Chem. Abst. 74, 53992j | B | p-[E-2-(6,10,14-tri-methylylpentadeca-9,13-dienyl)amino]-benzoic acid |
| 397 | Z-6,10,14-trimethyl-9,13-pentadecadien-2-ol Chem. Abst. 74, 53992j | B | p-[Z-2-(6,10,14-tri-methylylpentadeca-9,13-dienyl)amino]-benzoic acid |
| 398 | Z,Z-9,12-octadeca-dien-2-ol Chem. Abst. 68, 92804v | B | p-[1-octadeca-9,12-dienylamino]benzoic acid |
| 399 | E,Z-10,12-hexadeca-dien-1-ol Chem. Abst. 66, 106133y | B | p-(E,Z-1-hexadeca-10,12-dienylamino)-benzoic acid |
| 400 | Z,E-10,12-hexadeca-dien-1-ol Chem. Abst. 66, P79564f | B | p-(Z,E-1-hexadeca-10,12-dienylamino)-benzoic acid |
| 401 | 2-methyl-2,3-buta-dien-1-ol Chem. Abst. 71, 30229n | B | p-[1-(2-methylbuta-2,3-dienyl)amino]-benzoic acid |
| 402 | 2-ethyl-2,3-buta-dien-1-ol Chem. Abst. 67, 53567e | B | p-[1-(2-ethylbuta-2,3-dienyl)amino]-benzoic acid |

| Example | Starting Material | Method | Product |
|---|---|---|---|
| 403 | 2,3,5-trimethyl-3,4-hexadien-2-ol Chem. Abst. 72, 131953x | B | p-[2-(2,3,5-trimethylhexa-3,4-dienyl)-amino]benzoic acid |
| 404 | 3-isopropyl-2,4-dimethyl-4,5-hexadien-3-ol | B | p-[3-(2,4-dimethylhexa-4,5-dienyl)amino]-benzoic acid |
| 405 | 2,5-dimethyl-3,4-hexadien-2-ol Chem. Abst. 68, 39152e | B | p-[2-(2,5-dimethylhexa-3,4-dienyl)amino]-benzoic acid |
| 406 | 3,5-dimethyl-3,4-heptadien-2-ol Chem. Abst. 72, 131953x | B | p-[2-(3,5-dimethylhepta-3,4-dienyl)-amino]benzoic acid |
| 407 | 2-methyl-3,4-heptadien-2-ol Chem. Abst. 71, 38219g | B | p-[2-(2-methylhepta-3,4-dienyl)amino]-benzoic acid |
| 408 | 2,3,5-trimethyl-3,4-heptadien-2-ol Chem. Abst. 72, 131953x | B | p-[2-(2,3,5-trimethylhepta-3,4-dienyl)-amino]benzoic acid |
| 409 | 3-t-butyl-2-methyl-3,4-octadien-2-ol Chem. Abst. 66, 75593s | B | p-[2-(3-t-butyl-2-methylocta-3,4-dienyl)-amino]benzoic acid |
| 410 | 2-ethyl-2,3-heptadien-1-ol Chem. Abst. 75, 63063s | B | p-[1-(2-ethylhepta-2,3-dienyl)amino]-benzoic acid |
| 411 | 2-methyl-3,4-octadien-2-ol Chem. Abst. 75, 140175g | B | p-[1-(2-methylocta-3,4-dienyl)amino]-benzoic acid |
| 412 | 3-methyl-3,4-octadien-2-ol Chem. Abst. 66, 75593s | B | p-[2-(3-methylocta-3,4-dienyl)amino]-benzoic acid |
| 413 | 5,6-decadien-4-ol Chem. Abst. 75, 76898t | B | p-(4-deca-5,6-dienyl-amino)benzoic acid |

| Example | Starting Material | Method | Product |
|---|---|---|---|
| 414 | 2,3-dimethyl-3,4-octadien-2-ol Chem. Abst. 66, 75593s | B | p-[2,3-dimethylocta-3,4-dienyl)amino]-benzoic acid |
| 415 | 4-ethyl-4,5-nonadien-3-ol Chem. Abst. 75, 63063s | B | p-[3-(4-ethylnona-4,5-dienyl)amino]-benzoic acid |
| 416 | 2-methyl-3-propyl-3,4-octadien-2-ol Chem. Abst. 66, 65592v | B | p-[2-(2-methyl-3-propylocta-3,4-dienyl)-amino]benzoic acid |
| 417 | 2-methyl-3,4-nonadien-2-ol Chem. Abst. 75, 140173g | B | p-[2-(2-methylnona-3,4-dienyl)amino]-benzoic acid |
| 418 | 2,8-dimethyl-3,4-nonadien-2-ol Chem. Abst. 71, 38219g | B | p-[2-(2,8-dimethylnona-3,4-dienyl)amino]-benzoic acid |
| 419 | 2-methyl-3,4-decadien-2-ol Chem. Abst. 75, 140173g | B | p-[2-(2-methyldeca-3,4-dienyl)amino]-benzoic acid |
| 420 | 2,9-dimethyl-3,4-decadien-2-ol | B | p-[2-(2,9-dimethyl-deca-3,4-dienyl)amino]-benzoic acid |
| 421 | 2-methyl-3,4-dodecadien-2-ol Chem. Abst. 71, 38219g | B | p-[2-(2-methyldodeca-3,4-dienyl)amino]-benzoic acid |
| 422 | 2-methyl-3,4-tridecadien-1-ol Chem. Abst. 71, 38219g | B | p-[2-(2-methyltri-deca-3,4-dienyl)amino]-benzoic acid |
| 423 | 4,5-hexadien-2-ol Chem. Abst. 75, 5152j | B | p-(2-hexa-4,5-dienyl-amino)benzoic acid |
| 424 | 2-methyl-5,6-heptadien-3-ol Chem. Abst. 75, 5152j | B | p-[2-(2-methylhepta-5,6-dienyl)amino]-benzoic acid |

| Example | Starting Material | Method | Product |
|---|---|---|---|
| 425 | 3,3-dimethyl-4,5-hexadien-2-ol Chem. Abst. 69, 86256x | B | p-[2-(3,3-dimethylhexa-4,5-dienyl)-amino]benzoic acid |
| 426 | 2,5-dimethyl-5,6-heptadien-3-ol Chem. Abst. 68, 86855w | B | p-[2-(2,5-dimethylhepta-5,6-dienyl)-amino]benzoic acid |
| 427 | 2,2,5-trimethyl-3,4-hexadien-1-ol Chem. Abst. 71, 2)767g | B | p-[1-(2,2,5-trimethylhexa-3,4-dienyl)amino]-benzoic acid |
| 428 | (+)2,2-dimethyl-3,4-hexadien-1-ol Chem. Abst. 68, 58831s | B | p-[1-(2,2-dimethylhexa-3,4-dienyl)-amino]benzoic acid |
| 429 | 3,4-hexadien-1-ol Chem. Abst. 66, 54943r | B | p-(1-hexa-3,4-dienylamino)benzoic acid |
| 430 | 2,2,3,5-tetramethyl-3,4-hexadien-1-ol Chem. Abst. 71, 29767g | B | p-[1-(2,2,3,5-tetramethylhexa-3,4-dienyl)-amino]benzoic acid |
| 431 | 3,3,6-trimethyl-4,5-octadien-2-ol Chem. Abst. 69, 86256x | B | p-[2-(3,3,6-trimethylocta-4,5-dienyl)-amino]benzoic acid |
| 432 | 2,5-dimethyl-5,6-heptadien-2-ol Chem. Abst. 69, 86256x | B | p-[2-(2,5-dimethylhepta-5,6-dienyl)-amino]benzoic acid |
| 433 | 4-methyl-4-penten 2-yn-1-ol | B | p-[1-(4-methylpent-4-en-2-ynyl)amino]benzoic acid |
| 434 | 2-methyl-2-penten 2-yn-1-ol | B | p-[1-(2-methylpent-2-en-4-ynyl)amino]benzoic acid |
| 435 | 5-hexen-3-yn-2-ol | B | p-(2-hex-5-en-3-ynylamino)benzoic acid |
| 436 | 7-octen-5-yn-4-ol Chem. Abst. 67, 113544g | B | p-(4-oct-7-en-5-ynylamino)benzoic acid |

| Example | Starting Material | Method | Product |
|---|---|---|---|
| 437 | 5-methyl-5-hexen-3-yn-2-ol | B | p-[2-(5-methylhex-5-en-3-ynyl)amino]benzoic acid |
| 438 | 2,5-dimethyl-1-nonen-3-yn-5-ol Chem. Abst. 69, 2433s | B | p-[5-(2,5-dimethylnon-1-en-3-ynyl)amino]-benzoic acid |
| 439 | E-3-decen-1-yn-5-ol Chem. Abst. 75, 35042r | B | p-(5-dec-3-en-1-ynyl-amino)benzoic acid |
| 440 | E-3-dodecen-1-yn-5-ol Chem. Abst. 75, 35042r | B | p-(5-dodec-3-en-1-ynyl-amino)benzoic acid |
| 441 | 2-methyl-5-hexen-3-yn-2-ol Chem. Abst. 66, 75760v | B | p-[2-(2-methylhex-5-en-3-ynyl)amino]-benzoic acid |
| 442 | 3-methyl-6-hepten-4-yn-3-ol Chem. Abst. 67, 113544g | B | p-[3-(3-methylhept-6-en-4-ynyl)amino]-benzoic acid |
| 443 | 5-methyl-1-nonen-3-yn-5-ol Chem. Abst. 67, 43345s | B | p-[5-(5-methylnon-1-en-3-ynyl)amino]-benzoic acid |
| 444 | 3-ethyl-6-hepten-4-yn-3-ol Chem. Abst. 71, 112202g | B | p-[3-(3-ethylhept-6-en-4-ynyl)amino] benzoic acid |
| 445 | 5-ethyl-1-nonen-3-yn-5-ol Chem. Abst. 72, 31124j | B | p-[5-(5-ethylnon-1-en-3-ynyl)amino]-benzoic acid |
| 446 | 2,5-dimethyl-5-hexen-3-yn-2-ol Chem. Abst. 71, 112202g | B | p-[2-(2,5-dimethyl-hex-5-en-3-ynyl)amino]-benzoic acid |
| 447 | 3,6-dimethyl-6-hepten-4-yn-3-ol Chem. Abst. 69, 96881m | B | p-[2-(2,5-dimethyl-hex-5-en-3-ynyl)amino]-benzoic acid |
| 448 | 3-ethyl-6-methyl-6-hepten-4-yn-3-ol Chem. Abst. 71, 1122026 | B | p-[3-(3-ethyl-6-methyl-hept-6-en-4-ynyl)-amino]benzoic acid |
| 449 | 5-ethyl-2-methyl-3-yn-5-ol Chem. Abst. 69, 2433s | B | p-[5-(5-ethyl-2-methyl-n-1-en-3-ynyl)amino]-benzoic acid |

| Example | Starting Material | Method | Product |
|---|---|---|---|
| 450 | 2-hexen-4-yn-1-ol Chem. Abst. 69, 77868a | B | p-(1-hex-2-en-4-ynylamino)benzoic acid |
| 451 | 4-methyl-4-hexen-2-yn-1-ol Chem. Abst. 66, 115247r | B | p-[1-(4-methylhex-4-en-2-ynyl)amino]benzoic acid |
| 452 | 5-hexen-2-yn-1-ol Chem. Abst. 71, 80580t | B | p-(1-hex-5-en-2-ynylamino)benzoic acid |
| 453 | 5-methyl-5-hexen-2-yn-1-ol Chem. Abst. 70, 28152v | B | p-[1-(5-methylhex-5-en-2-ynyl)amino]benzoic |
| 454 | 5-hexen-3-yn-1-ol Chem. Abst. 73, 109833g | B | p-(1-hex-5-en-3-ynylamino)benzoic acid |
| 455 | E-4-methyl-5-yn-1-ol Chem. Abst. 66, 104730s | B | p-[E-1-1-(4-methylhept-3-en-5-ynyl)amino]- |
| 456 | Z-4-methyl-5-yn-1-ol Chem. Abst. 66, 104730s | B | p-[Z-1-(4-methylhept-3-en-5-ynyl)amino]-benzoic acid |
| 457 | 3-hexen-5-ynl-2-ol Chem. Abst. 75, 5152j | B | p-(2-hex-3-en-5-ynylamino)benzoic acid |
| 458 | 6-methyl-6-hepten-4-yn-2-ol | B | p-[2-(6-methylhept-6-en-4-ynyl)amino]-benzoic acid |
| 459 | 7-methyl-7-octen-5-yn-3-ol | B | p-[3-(7-methyloct-7-en-5-ynyl)amino]-benzoic acid |
| 460 | 2,5-dimethyl-5-hexen-3-yn-2-ol | B | p-[2-(2-dimethylhex-5-en-3-ynyl)amino]-benzoic acid |
| 461 | 3-methyl-6-octen-4-yn-3-ol Chem. Abst. 74, 41795j | B | p-[3-(3-methyloct-6-en-4-ynyl)amino]-benzoic acid |
| 462 | 5-methyl-9-decen-6-yn-5-ol Chem. Abst. 73, 12527 lc | B | p-[5-(5-methyldec-9-en-6-ynyl)amino]-benzoic acid |

| Example | Starting Material | Method | Product |
|---|---|---|---|
| 463 | 2,5,5,6-tetramethyl-6-hepten-3-yn-2-ol Chem. Abst. 70, 10801w | B | p-[2-(2,5,5,6-tetramethylhept-6-en-3-ynyl)amino]benzoic acid |
| 464 | 3-ethyl-7-octen-4-yn-3-ol Chem. Abst. 73, 3762t | B | p-[3-(3-ethyloct-7-en-4-ynyl)amino]-benzoic acid |
| 465 | 3-methyl-7-octen-4-yn-3-ol Chem. Abst. 73, 3762t | B | p-[3-(3-methyloct-7-en-4-ynyl)amino]-benzoic acid |
| 466 | 2,6-dimethyl-6-hepten-3-yn-2-ol Chem. Abst. 73, 55677p | B | p-[2-(2,6-dimethyl-hept-6-en-3-ynyl)-amino]benzoic acid |
| 467 | 3,6-diethyl-6-octen-4-yn-3-ol Chem. Abst. 68, 104850x | B | p-[3-(3,6-diethyl-oct-6-en-4-ynyl)-amino]benzoic acid |
| 468 | 6-ethyl-6-octen-4-yn-2-ol Chem. Abst. 73, 19632z | B | p-[2-(6-ethyloct-6-en-4-ynyl)amino]-benzoic acid |
| 469 | 6-methyl-t-hepten-2-yn-1-ol Chem. Abst. 72, 3587u | B | p-[1-(6-methylhept-6-en-2-ynyl)amino]-benzoic acid |
| 470 | E-4-methyl-3-hept-en-6-yn-1-ol Chem. Abst. 66, 104730s | B | p-[E-1-(4-methyl-hept-3-en-4-ynyl)-amino]benzoic acid |
| 471 | Z-4-methyl-3-hept-en-6-yn-1-ol Chem. Abst. 66, 104'30s | B | p-[Z-1-(4-methyl-hept-3-en-6-ynyl)-amino]benzoic acid |
| 472 | E-2-octen-6-yn-1-ol Chem. Abst. 75, 63019g | B | p-(E-1-oct-2-en-6-ynylamino)benzoic acid |
| 473 | Z-7-methyl-6-non-en-2-yn-1-ol Chem. Abst. 70, 78718j | B | p-[Z-1-(7-methyl-non-6-en-2-ynyl)-amino]benzoic acid |
| 474 | 8-methyl-7-methyl-ene-3-nonyn-1-ol Chem. Abst. 66, 29098u | B | p-[1-(8-methyl-1-methylenenon-3-ynyl)-amino]benzoic acid |

| Example | Starting Material | Method | Product |
|---|---|---|---|
| 475 | E-2-decen-4-yn-1-ol Chem. Abst. 67, 99692v | B | p-(1-dec-2-en-4-ynyl-amino)benzoic acid |
| 476 | 4-methyl-4-decen-8-yn-1-ol Chem. Abst. 75, 110510x | B | p-[1-(4-methyldec-4-en-8-ynyl)amino]-benzoic acid |
| 477 | E-2-undecen-4-yn-1-ol. Chem. Abst. 66, 28594h | B | p-(1-undec-2-en-4-ynyl-amino)benzoic acid |
| 478 | 6-methyl-5-undecen-2-yn-1-ol Chem. Abst. 71, P101399h | B | p-[1-(6-methylundec-5-en-2-ynyl)amino]-benzoic acid |
| 479 | E-5-tetradecen-3-yn-1-ol Chem. Abst. 73, 87370z | B | p-(E-1-tetradec-5-en 3-ynylamino)benzoic acid |
| 480 | Z-5-tetradecen-3-yn-1-ol Chem. Abst. 73, 87370z | B | p-(Z-1-tetradec-5-en-3-ynylamino)benzoic acid |
| 481 | 10-propyl-9-tridec-en-5-yn-1-ol Chem. Abst. 72, 12017K | B | p-[1-(10-propyltridec-9-en-5-ynyl)amino]-benzoic acid |
| 482 | 17-octadecen-14-yn-1-ol Chem. Abst. 68, 39015n | B | p-(1-octadec-17-en-14-ynylamino)benzoic acid |
| 483 | 2,4-dimethyl-1,4-hexadien-3-ol Chem. Abst. 74 87269u | B | p-[3-(2,4-dimethylhexa-1,4-dienyl)amino]-benzoic acid |
| 484 | 1,5-hexadien-3-ol Chem. Abst. 73, 44822f | B | p-(3-hexa-1,5-dienyl-amino)benzoic acid |
| 485 | 3,5-dimethyl-1,5-hexadien-3-ol Chem. Abst. 67, 53415d | B | p-[3-(3,5-dimethylhexa-1,5-dienyl)amino]-benzoic acid |
| 486 | 2,6-dimethyl-1,6-heptadien-3-ol Chem. Abst. 73, 87490p | B | p-[3-(2,6-dimethyl-hepta-1,6-dienyl)-amino]benzoic acid |

| Example | Starting Material | Method | Product |
|---|---|---|---|
| 487 | E-2,6-dimethyl-1,6-octadien-3-ol Chem. Abst. 71, 61565a | B | p-[E-3-(2,6-dimethylocta-1,6-dienyl)amino]-benzoic acid |
| 488 | Z-2,6-dimethyl-1,6-octadien-3-ol Chem. Abst. 71, 6165a | B | p-[Z-3-(2,6-dimethylocta-1,6-dienyl)amino]-benzoic acid |
| 489 | 3-ethyl-7-methyl-1,6-octadien-3-ol Chem. Abst. 66, P76192x | B | p-[3-(3-ethyl-7-methylocta-1,6-dienyl)amino]-benzoic acid |
| 490 | 3-t-butyl-7-methyl 1,6-octadien-3-ol Chem. Abst. 66, P76192x | B | p-[3-(3-t-butyl-7-methylocta-1,6-dienyl)-amino]benzoic acid |
| 491 | 7,9-dimethyl-1,6-decadien-3-ol Chem. Abst. 71, P60078c | B | p-[3-(7,9-dimethyldeca-1,6-dienyl)amino]-benzoic acid |
| 492 | 3,7-dimethyl-1,6-decadien-3-ol Chem. Abst. 67, 2688n | B | p-[3-(3,7-dimethyldeca-1,6-dienyl)amino]-benzoic acid |
| 493 | 2-methyl-1,5-heptadien-4-ol Chem. Abst. 67, 53415d | B | p-[4-(2-methylhepta-1,5-dienyl)amino]-benzoic acid |
| 494 | 4,6-dimethyl-1,5-heptadien-4-ol Chem. Abst. 67, 53415d | B | p-[4-(4,6-dimethylhepta-1,5-dienyl)-amino]benzoic acid |
| 495 | E,E-3,4,5-trimethyl-2,5-heptadien-4-ol Chem. Abst. 72, | B | p-[E,E-4-(3,4,5-trimethylhepta-2,5-dienyl)amino]benzoic acid |
| 496 | E,Z-3,4,5-trimethyl-2,5-heptadien-4-ol Chem. Abst. 72, 2823f | B | p-[E,Z-4-(3,4,5-trimethylhepta-2,5-dienyl)amino]benzoic acid |
| 497 | Z,Z-3,4,5-trimethyl-2,5-heptadien-4-ol Chem. Abst. 72, 2823f | B | p-[Z,Z-4-(3,4,5-trimethylhepta-2,5-dienyl)amino]benzoic acid |

| Example | Starting Material | Method | Product |
|---|---|---|---|
| 498 | 2-methyl-2,9-decadien-5-ol Chem. Abst. 73, 87254g | B | p-[5-(2-methyldeca-2,9-dienyl)amino]-benzoic acid |
| 499 | 8-methyl-1,7-nona-dien-5-ol Chem. Abst. 73, 87253p | B | p-[5-(8-methylnona-1,7-dienyl)amino]benzoic acid |
| 500 | 3,4,7,7-tetramethyl-1,5-octadien-4-ol Chem. Abst. 67, 63514z | B | p-[5-(3,4,7,7-tetra-methylocta-1,5-dienyl)-amino]benzoic acid |
| 501 | 3,4-dimethylene-2-hexanol Chem. Abst. 69,106850s | B | p-[2-(3,4-dimethylene-hexyl)amino]benzoic acid |
| 502 | 3-methyl-2-methyl-ene-3-butene-1-ol Chem. Abst. 74, 42503z | B | p-[1-(3-methyl-2-methylenebut-3-enyl-amino]benzoic acid |
| 503 | 2-methylene-3-buten-1-ol Chem. Abst. 73, P67478n | B | p-[1-(2-methylenebut-3-enyl)amino]benzoic acid |
| 504 | 3,3-dimethyl-2-methylene-4-penten-1-ol Chem. Abst. 74, 52962n | B | p-[1-(3,3-dimethyl-2-methylenepent-4-enyl)-amino]benzoic acid |
| 505 | 2-methylene-3-meth-yl-4-hexen-1-ol Chem. Abst. 68, 59021q | B | p[1-(2-methylene-3-methylhex-4-enyl)-amino]benzoic acid |
| 506 | 2,4-dimethyl-3-methylene-5-hexen-2-ol Chem. Abst. 72, 66299x | B | p-[2-(2,4-dimethyl-3-methylenehex-5-enyl)-amino]benzoic acid |
| 507 | 2,4,4-trimethyl-3-methylene-5-hexen-2-ol Chem. Abst. 74, 52)62n | B | p-[2-(2,4,4-trimethyl-3-methylenehex-5-enyl)amino]benzoic acid |
| 508 | E-2-methyl-3-methyl-ene-5-hepten-2-ol Chem. Abst. 72, 132999d | B | p-[E-2-(2-methyl-3-methylenehept-5-enyl)-amino]benzoic acid |

| Example | Starting Material | Method | Product |
|---|---|---|---|
| 509 | Z-2-methyl-3-methyl-ene-5-hepten-2-ol Chem. Abst. 72, 132999d | B | p-[Z-2-(2-methyl-3-methylenehept-5-enyl)amino]benzoic acid |
| 510 | 2,6-dimethyl-1,3-methylene-5-hepten-2-ol Chem. Abst. 72, 132999d | B | p-[2-(2,6-dimethyl-3-methylenehept-5-enyl)amino]benzoic acid |
| 511 | 3,7-dimethyl-2-methylene-6-octen-1-ol Chem. Abst. 68, 114750d | B | p-[1-(3,7-dimethyl-2-methyleneoct-6-enyl)-amino]benzoic acid |
| 512 | 3,7-dimethyl-2-methylene-7-octen-1-ol Chem. Abst. 70, P3'191J | B | p-[1-(3,7-dimethyl-2-methyleneoct-7-enyl)-amino]benzoic acid |
| 513 | 3-isopropylidene-2,5-dimethyl-4-hex-en-2-ol Chem. Abst. 74, 53105x | B | p-[2-(3-isopropylidene-2,5-dimethylhex-4-enyl)amino]benzoic acid |
| 514 | 2-methyl-6-methyl-ene-7-octen-4-ol Chem. Abst. 69, 10548y | B | p-[4-(2-methyl-6-methyleneoct-7-enyl)-amino]benzoic acid |
| 515 | 2-isopropylidene-5-methyl-4-hexene-1-ol Chem. Abst. 72, 132999d | B | p-[1-(2-isopropylidene-5-methylhex-4-enyl)-amino]benzoic acid |
| 516 | 7-methyl-3-methyl-ene-6-octen-1-ol 131140a | B | p-[1-(7-methyl-3-methyleneoct-6-enyl)-amino]benzoic acid |
| 517 | 2-methyl-6-methyl-ene-7-octen-2-ol Chem. Abst. 71, P61600h | B | p-[2-(2-methyl-6-methyleneoct-7-enyl)-amino]benzoic acid |
| 518 | E-2-ethylidene-6-methyl-5-hepten-1-ol Chem. Abst. 68, 114750d | B | p-[E-1-(2-ethylidene-6-methylhept-5-enyl)-amino]benzoic acid |

| Example | Starting Material | Method | Product |
|---|---|---|---|
| 519 | Z-2-ethylidene-6-methyl-5-hepten-1-ol Chem. Abst. 68, 114750d | B | p-[Z-1-(2-ethylidene-6-methylhept-5-enyl)-amino]benzoic acid |
| 520 | 2,5-dimethyl-3-vinyl-4-hexen-2-ol Chem. Abst. 69, 45983p | B | p-[2-(2,5-dimethyl-3-vinylhex-4-enyl)amino]-benzoic acid |
| 521 | 2-isopropenyl-5-methyl-4-hexen-1-ol Chem. Abst. 68, 111190k | B | p[1-(2-isopropenyl-5-methylhex-4-enyl)-amino]benzoic acid |
| 522 | 2-vinyl-5-hepten-1-ol Chem. Abst. 75, P151673w | B | p-[1-(2-vinylhept-5-enyl)amino]benzoic acid |
| 523 | 2-vinyl-6-hepten-1-ol Chem. Abst. 75, P151673w | B | p-[1-(2-vinylhept-6-enyl)amino]benzoic acid |
| 524 | 2-(2-methylpropenyl)-5-hexen-1-ol Chem. Abst. 68, 114750d | B | p-[1-(2-methyl-propenylhex-5-enyl)amino]benzoic acid |
| 525 | 7-methyl-3-vinyl-6-octen-1-ol Chem. Abst. 66, P115838j | B | p-[1-(7-methyl-3-vinyloct-6-enyl)amino]-benzoic acid |
| 526 | 2-allyl-4-methyl-4-penten-1-ol Chem. Abst. 72, 21731r | B | p-[1-(2-allyl-4-methyl-pent-4-enyl)amino]-benzoic acid |
| 527 | 3-methyl-5-undecen-1-yn-3-ol Chem. Abst. 71, P101399h | B | p-[3-(3-methylundec-5-en-1-ynyl)amino]benzoic acid |
| 528 | 3,4,8-trimethyl-8-nonen-1-yn-3-ol Chem. Abst. 68, 29184r | B | p-[3-(3,4,8-trimethyl-non-8-en-1-ynyl)amino]-benzoic acid |
| 529 | 1-dodecen-4-yn-3-ol Chem. Abst. 66, 75603v | B | p-(3-dodec-1-en-4-ynyl-amino)benzoic acid |
| 530 | 11-dodecen-1-yn-3-ol Chem. Abst. 73, 120015N | B | p-(3-dodec-11-en-1-ynylamino)benzoic acid |

| Example | Starting Material | Method | Product |
|---|---|---|---|
| 531 | 1-undecen-5-yn-ol Chem. Abst. 69, 2432r | B | p-(4-undec-1-en-5-ynyl-amino)benzoic acid |
| 532 | 3,7-dimethyl-6-nonen-1-yn-3-ol Chem. Abst. 71, P91265v | B | p-[3-(3,7-dimethylnon-6-en-1-ynyl)amino]-benzoic acid |
| 533 | 7,7-dimethyl-1-non-en-8-yn-5-ol Chem. Abst. 74, 22939t | B | p-[5-(7,7-dimethylnon-1-en-8-ynyl)amino]-benzoic acid |
| 534 | 2,3-dimethyl-1-non-en-4-yn-3-ol Chem. Abst. 68, 39161g | B | p-[3-(2,5-dimethylnon-1-en-4-ynyl)amino]-benzoic acid |
| 535 | 3,7-dimethyl-7-octen-1-yn-3-ol Chem. Abst. 73, p7095a | B | p-[3-(3,7-dimethyloct-7-en-1-ynyl)amino]-benzoic acid |
| 536 | 4,6-dimethyl-5-hept-en-1-yn-4-ol Chem. Abst. 66, 95156e | B | p-[3-(4,6-dimethylhept-5-en-1-ynyl)amino]-benzoic acid |
| 537 | 1-penten-4-yn-3-ol Chem. Abst. 74, P140935m | B | p-(3-pent-1-en-4-ynyl amino)benzoic acid |

## EXAMPLE 538

### Preparation of 4-(pentadec-4-enylamino)benzhydroxamic acid

To a suspension of 4-(pentadec-4-enylamino)benzoic acid (44 g.) in glyme (350 ml.) and pyridine (70 ml.) at 0°C. is added trifluoroacetic anhydride (67 ml., 100 g.) at such a rate as to maintain the temperature at 20-30°C. The resulting solution is stirred at 10-15°C. for 2 hours, then diluted with ether (400 ml.), cooled in an ice-bath and ice (100 gm.) is added. The mixture is stirred vigorously at ambient temperature for 1 hour. The aqueous layer is extracted with ether and the combined ether extracts are washed with water, brine, dried over sodium sulfate and concentrated *in vacuo* to provide 57 g. of 4-[N-trifluoroacetyl-N-(pentadec-4-enyl)amino]benzoic acid as an oil that solidifies upon standing.

The N-trifluoroacetyl derivative (57 g.) is dissolved in thionyl chloride (300 ml.) and refluxed for 3 hours. After cooling, the mixture is diluted with toluene and concentrated in vacuo. The residue is diluted again with hot toluene and filtered. The toluene is concentrated in vacuo to provide 61 g. or 4-[N-trifluoroacetyl-N-(pentadec-4-enyl)amino]benzoyl chloride as an oil.

To a stirred solution of hydroxylamine hydrochloride (16 g.) in pyridine (75 ml.) and dichloromethane (35 ml.) is added dropwise the previously prepared benzoyl chloride derivative (11.5g.) in dichloromethane (20 ml.). After 1 hour, the mixture is diluted with water and extracted with ether. The combined organic extract is washed with 5% hydrochloric acid until the aqueous wash remains acidic. The ether extract is then washed with water, brine, dried with sodium sulfate and concentrated in vacuo to afford 4-[N-trifluoroacetyl]-N-(pentadec-4-enyl)amino]benzhydroxamic acid as an oil.

A portion of this oil (0.9 g.) is dissolved in ethanol (20 ml.), 1N sodium hydroxide solution is added, and the mixture is stirred at ambient tewmperature. The mixture is chilled and filtered to provide a white solid that is washed with ethanol. The white solid is recrystallized from hot ethanol to provide 4-(pentadec-4-enylamino)-benzhydroxamic acid.

EXAMPLE 539

Preparation of esters

Treatment of the acids of Examples 221-537 and 547-585 with trifluoracetic anhydride to provide the N-$COCF_3$ derivative, followed by treatment with thionyl chloride to provide the N-$COCF_3$ acid chloride, followed by treatment with one of the following alcohols, followed by removal of the N-$COCF_3$ group with sodium hydroxide by the method of Example 538 provides the corresponding ester of the starting acid.

Alcohols: methanol, ethanol, 2-methoxyethanol, butanol, pentanol, cyclopentanol, cyclohexanol, 1,2,-propanediol, 1,3-propanediol, ethylene glycol, glycerol, glycidol, methyl glycolage, ethyl glycolate, glycolic acid, 2-hydroxypropionic acid, 3-hydroxybutyric acid, 4-hydroxybutyric acid, glyceric acid, 3-diethylamino-1-propanol, 1-diethylamino-2-propanol, 3-diemethylamino-1-propanol, 2-diisopropylaminoethanol, 3-(4-methyl-1-piperazino)-1,2-propanediol,3-methoxy-1,2-propanediol, N-piperidineethanol, N,N-diethylethanolamine, benzyl alcohol, p-fluorobenzyl alcohol, p-bromobenzyl alcohol, p-chlorobenzyl alcohol, p-methoxybenzyl alcohol, m-chlorobenzyl alcohol, p-carboxybenzyl alcohol, phenol, p-fluorophenol, p-bromophenol, p-chlorophenol, p-methoxyphenol,p-carboxyphenol, 4-cyanophenol,3-hydroxypyridine, 2-chloro-3-hydroxypyridine, 5-carboxy-3-hydroxypyridine.

## EXAMPLE 540

### Preparation of amides

Treatment of the acids of Examples 221-539 and 547-585 with trifluoroacetic anhydride to provide the $N-COCF_3$ derivative, followed by treatment with thionyl chloride to provide the $N-COCF_3$ acid chloride, followed by treatment with one of the amines of the list below, followed by removal of the $N-COCF_3$ group with sodium hydroxide by the method of Example 538 provides the corresponding amides of the starting acid.

Amines: β-alanine, allylamine, allycyclohexylamine, aminoacetonitrile, α-aminoacetophenone, 2-amino-1-butanol, 3-aminobutyric acid, 4-aminobutyric acid, 1-amino-1-cyclopentanemethanol, 2-amino-5-diethylaminopentane, N-(2-aminoethyl)morpholine, N-(2-aminoethyl)piperazine, N-(2-aminoethyl)piperidine, 2-amino-2-ethyl-1,3-propanediol, 2-(2-aminoethyl)pyridine, N-(2-aminoethyl)pyrrolidine, DL-4-amino-3-hydroxybutyric acid, 5-aminolevulinic acid, aminomethanesulfonnic acid, p-aminomethylbenzenesulfonamide, 2-amino-3-methyl-1-butanol, aminomethylcyclobutane, 4-(aminomethyl)cyclohexanecarbonitrile, 1-aminomethyl-1-cyclohexanol, aminomethylcyclopropane, 4-(aminomethyl)piperdine, 2-amino-2-methyl-1,3-propanediol, 2-amino-2-methyl-1-prop-

anol, 2-(aminomethyl)-2-propanol, 2-aminomethylpyridine, 3-aminomethylpyridine, 4-aminomethylpyridine, 2-amino-1-phenylethanol, 2-amino-3-phenyl-1-propanol, 3-amino-3-phenylpropionic acid, 3-amino-1,2-propanediol, 1-amino-2-propanol, N-(3-aminopropyl)diethanolamine, N-(3-aminopropyl)morpholine, 1-(3-aminopropyl) -2-pipecoline, N-(3-aminopropyl)-2-pyrrolidinone, 5-aminovaleric acid, bis-(2-ethanolethyl)amine, bis-(2-methylallyl)amine, p-bromophenethylamine, 3-bromoppropylamine hydrobromide, n-butylamine, sec-butylamine, tert-butylamine, 2-cholrobenzylamine, 3-chlorobenzylamine, 5-chlorobenzylamine, 2-chloroethylamine, 3-chloropropylamine, cyclobutylamine, cycloheptylamine, 1,3-cyclohexanebis(methylamine), cyclohexanemethylamine, cyclohexylamine, cyclopentylamine, cyclopropylamine, 3-(di-n-butylamino)propylamine, 1,5-dimethylhexylamine, α,4-dimethyl-3-hydroxyphenethylamine, 1,1-dimethylpropargylamine, 1,2-dimethylpropylamine, 1,2-diphenylethylamine, ethylamine, ethyl-3-aminobutyrate, ethyl-4-aminobutyrate, 2-(ethylamino)ethanol, 1-ethylpropylamine, 1-ethynlcyclohexylamine, m-fluorobenzylamine, p-fluorobenzylamine, 2-fluoroethylamine, furfurylamine, n-heptylamine, isoamylamine, isopropylamine, m-methobenzylamine, p-methoxybenzylamine, 2-methoxyethylamine, o-methoxyphenethylamine, p-methoxyphenethylamine, N-methyl-β-alaninenitrile, 2-methylallylamine, methylamine, methylaminoacetonitrile, 2-(methylamino)ethanol, 2-methylbanylamine, 3-methylbanzylamine, 4-methylbenzylamine, 1-methylbutylamine, 4-methylcyclohexylamine, 1-norepinephrine, 4-phenylbutylamine, 1-phenylcyclopropanemethylamine, trans-2-pheynlcyclopropylamine, D(-)-α-phenylglycinol, 2-phenylglycinonitrile, phenylpropanolamine, 3-phenyl-1-propylamine, monopropargylamine, propylamine, taurine, tetrahydrofurfurylamine, 1,2,3,4-tetrahydro-1-naphthylamine, 2-(p-tolyl)ethylamine, m-aminobenzoic acid, p-aminobenzoic acid, o-aminobenzyl alcohol, p-aminobenzyl alcohol, 3,5-dimethylpiperidine, 2-ethylpiperidine, 3-hydroxypiperidine, 4-hydroxypiperidine, 2-iminopiperidine, isonipecotamide, isonipecotic acid, methyl-4-oxo-3-piper-

idinecarboxylate, 2-methylpiperidine, 3-methylpiperidine, 4-methylpiperidine, nipecotamide, 4-phenylpiperidine, 4-phenyl-1,2,3,5-tetrahydropyridine, pipecolinic acid, piperidine, 2-piperidineethanol, 2-piperidinemethanol, 3-piperidinemethanol, 4-piperidinopiperidine, 4-piperidine, 1,2,3,6-tertahydropyridine, 2,2,6,6-tetramethylpiperidine, 2,2,6,6-tetra methyl-4-piperidinol, 2,2,6,6-tetramethyl-4-piperidone, 4,4-trimethylenedipiperidine, 2-methylpiperidine, 3-methylpiperidine, 4-methylpiperidine, 4-phenylpiperidine, piperidine, morpholine, hexamethyleneimine, heptamethyleneimine, pentamethyleneimine, pyrrolidine, N-methylpiperazine, dl-alinine, hydrazine, N-acetylhydrazine, dl-valine, $\Delta^3$-piperidine, dl-leucine, 2-aminoisobutyric acid.

## EXAMPLE 541

### Preparation of N-[4-(9-Octadecenylamino)benzoyl]benzamide

Sodium hydride (1.0 g., 50% dispersion in mineral oil) is washed with hexane three times under nitrogen. To the dry sodium hydride is added 5 ml. of freshly distilled tetryhydrofuran. To this suspension is added a solution of 2.4 g. of benzamide in 5 ml. of tetrahydrofuran. After complete reaction (30 min.), a solution of 0.9 g. 4-[N-trifluoroacetyl-N-(9-octadecenyl)amino]benzoyl chloride (Example 538) in 3 ml. of tetrahydrofuran is added. After stirring at ambient temperature for 1 hour, the reaction mixture is poured into water and extracted twice with ether. The ether extracts are washed with water, brine, dried with sodium sulfate, and concentrated *in vacuo*. The residue is recrystallized from ether-acetonitrile (1:1) to provide N-{4-[N-trifluoroacetyl-N-(9-octadecenyl)amino]benzoyl}-benzamide.

The N-trifluoroacetyl compound is in turn treated with ethanol and 1N sodium hydroxide and the mixture is stirred at ambient temperature for 6 hours. Chilling and filtration affords a white solid which is recrystallized from ethanol to yield N-[4-(9-octadecenylamino)benzoyl]-benzamide.

EXAMPLE 542

Preparation of N-[4-(substituted amino)benzoyl]benzamides

Treatment of the N-$COCF_3$ acid halides (prepared by the method of Example 538 from the corresponding acids of Examples 221-537 and 547-586) with benzamide and sodium hydride followed by removal of the N-$COCF_3$ group by the method of Example 541 is productive of the corresponding benzamides.

EXAMPLE 543

Preparation of p-(4-pentadecenylamino)-N-(phenylsulfonyl)-benzamide

A solution of 31.4 g. of benzenesulfonamide in 250 ml. of dry dimethylacetamide is added dropwise, with stirring and cooling, to a suspension of 5.5 g. of sodium hydride in 100 ml. of dry dimethylacetamide over 30 minutes at room temperature. Stirring is continued for a further 30 minutes. In the meantime, a mixture of 36.2 g. of 4-(4-pentadecenylamino)benzoic acid in 1200 ml. of methylene chloride, 300 ml. of dimethoxyethane, and 400 ml. of thionyl chloride is refluxed for 1 hour and 15 minutes. The solution is evaporated to an oil which is co-evaporated twice with added dioxane to remove excess thionyl chloride. To the resulting oily residue of 4-(4-pentadecenylamino)benzoyl chloride hydrochloride is added, in one portion, the previously prepared mixture of sodium benzenesulfonamide in dimethylacetamide. The mixture is stirred for 30 minutes, without cooling, and is then filtered through a bed of diatomaceous earth. the filtrate is poured into 2 l. of water, and 250 ml. of saturated sodium chloride solution is added to coagulate the precipitate. The mixture is filtered and the product is washed with water and partially air dried. The product is dissolved in methylene chloride, the mixture is filtered through diatomaceous earth, and brine is added to break the emulsion. The layers are separated, the organic phase is dried over anhydrous sodium sulfate and filtered through a bed of 300 g. of hydrous magnesium

silicate. The product is eluted with an additional 3L. of methylene chloride. The first approximately 1 l. of filtrate is set aside and the remainder is evaporated to dryness. The residue is crystallized three times from toluene and the product is dried in the Abderhalden at 65° C. to provide the title compound as colorless crystals.

EXAMPLE 544

Preparation of p-(substituted amino)-N-(sulfonyl)benzamides

Treatment of the acid chloride hydrochloride (prepared from the corresponding carboxylic acids of Examples 221-537 and 547-586 by the procedure of Example 587) with the following sulfonamides by the procedure of Example 543 is productive of the corresponding p-(substituted amino)-N-sulfonyl)banzamide. The sulfonamide starting materials are benzenesulfonamide, methanesulfonamide, p-methylphenylsulfonamide, p-nitrophenylsulfonamide, p-chlorophenylsulfonamide.

EXAMPLE 545

Preparation of methyl esters

Treatment of the p-(substituted amino)benzoic acids of Examples 221-537 and 547-536 with excess diazomethane is productive of the corresponding methyl ester.

EXAMPLE 546

Preparation of hexyl esters

Treatment of the p-(substituted amino)benzoic acids of Examples 221-537 and 547-585 with excess diazohexane is productive of the corresponding hexyl esters.

EXAMPLE 547

Preparation of p-[1-(2,3-methano)octylamino]benzoic acid

To a stirred mixture of 5 g. of zinc-copper couple prepared by the Shank-Shechter Method [R.S. Shank and H. Shechter, J. Org. Chem., 24, 1825 (1959)], 0.02 g. of iodine and 100 ml. of anhydrous ether is added to 8.7 g. (0.05 mol) of methylene iodide. The mixture is heated in the absence of atmospheric moisture until a spontaneous reaction begins as evidenced by continued refluxing of the

ether when the heat source is removed. Upon completion of the exothermic reaction, the mixture is refluxed for 30 minutes, the heat is removed and to this mixture is added a solution of 5.4 g. (0.04 mol) of cis-2-octenol [L.P. Paquette and R.F. Eizember, J. Am. Chem. Soc., 91, 7110 (1969)] in 10 ml. of anhydrous ether at a rate sufficient to maintain constant reflux. When the addition is complete, the mixture is refluxed for 3 hours. The flask is cooled and the mixture is filtered and the filtrate is washed with cold dilute hydrochloric acid and saturated sodium bicarbonate, dried and evaporated to give Z-1-hydroxymethyl-2-n-pentylcyclopropane.

Preparation of the tosylate by the method of Example 222, followed by condensation with ethyl p-aminobenzoate by the method of Example 222, followed by saponification by the method of Example 222 provides p-[1-(2,3-methano)octylamino]benzoic acid.

EXAMPLE 548

Treatment of the indicated olefin of the table with zinc and diiodomethane by the method of Example 547 to produce the corresponding cyclopropyl compound followed by treatment of the halide with ethyl-p-aminobenzoate by the method of Example 221, followed by saponification of the resulting ester by the method of Example 221, is productive of the p-(methanoalkyl)amino benzoic acid of Table IV (Method C).

Treatment of the olefinic alcohols of Table II with zinc and diiodomethane by the method of Example 547 will produce the corresponding cyclopropyl alkanols which upon treatment with methanesulfonic anhydride (Method of Example 222) will produce the corresponding methanesulfonate ester which upon treatment with ethyl p-aminobenzoate by the procedure of Example 222 followed by saponification will produce the p-(methano-alkyl)aminobenzoic acids of Table IV (Method D).

| Example | Starting Material | Method | Product |
|---|---|---|---|
| 549 | 3-bromo-3-isopropyl-4-methyl-1-pentene Chem. Abst. 54, 4355a | C | p-[3-(3-isopropyl-4-methyl-1,2-methanopentyl)-amino]benzoic acid |
| 550 | 4-bromo-2-heptene Chem. Abst. 70, 6748x | C | p-[4-(2,3-methanoheptyl)-amino]benzoic acid |
| 551 | 4-bromo-2,4-dimethyl-2-hexane Chem. Abst. 70, 3169t | C | p-[4-(2,4-dimethyl-2,3-methanohexyl)amino]-benzoic acid |
| 552 | 5-chloro-3,5-dimethyl-3-heptene Chem. Abst. 54, 1256e | C | p-[5-(3,5-dimethy-3,4-methanoheptyl)amino]-benzoic acid |
| 553 | Z-1-hydroxy-2-hexadecane Ref. B | D | p-[Z-1-(2,3-methanohexadecyl)amino]benzoic acid |
| 554 | E-1-hydroxy-2-hexadecane Ref. B | D | p-[E-1-(2,3-methanohexadecyl)amino]benzoic acid |
| 555 | 1-bromo-4-methyl-3-heptene Chem. Abst. 71, 102020q | C | p-[1-(4-methy-3,4-methanoheptyl)amino]-benzoic acid |
| 556 | 1-bromo-4-methyl-3-heptene Chem. Abst. 71, 102020q | C | p-[1-(4-methy-3,4-methanoheptyl)amino]-benzoic acid |
| 557 | E-7-bromo-3-heptene Chem. Abst. 74, 99419f | C | p-[E-1-(4,5-methanoheptyl)amino]benzoic acid |
| 558 | 1-bromo-5,9-dimethyl-4-decane Chem. Abst. 51, 8699g | C | p-[1-(5,9-dimethyl-4,5-methanodecyl)-amino]benzoic acid |
| 559 | 1-methanesulfonyloxy-4-tetradecene Ref. B. | C | p-[1-(4,5-methanotetradecyl)amino]-benzoic acid |
| 560 | 1-methanesulfonyloxy-4-hexadecene Ref. B. | C | p-[1-(4,5-methanohexadecyl)amino]-benzoic acid |
| 561 | 6-bromo-1-hexene Chem. Abst. 66, 2142j | C | p-[1-(5,6-methanohexyl)-amino]benzoic acid |

| Example | Starting Material | Method | Product |
|---|---|---|---|
| 562 | 6-bromo-2-methyl-1-hexene Chem. Abst. 75, 109624f | C | p-[1-(5,6-methano-5-methylhexyl)amino]benzoic acid |
| 563 | 6-chloro-1-heptene Chem. Abst. 72, 31877g | C | p-[2-(6,7-methanoheptyl)-amino]benzoic acid |
| 564 | 6-bromo-2-methyl-2-heptene Chem. Abst. 54, 13166f | C | p-[2-(5,6-methano-6-methylheptyl)amino]benzoic acid |
| 565 | 7-chloro-2-octene Chem. Abst. 75, 129245m | C | p-[2-(6,7-methanooctyl)-amino]benzoic acid |
| 566 | E-1-chloro-4-nonene Chem. Abst. 67, 32294y | C | p-[E-1-(4,5-methanononyl)-amino]benzoic acid |
| 567 | 7-bromo-1-heptene | C | p-[1-(6,7-methanoheptyl)-amino]benzoic acid |
| 568 | 7-chloro-1-octene Chem. Abst. 75, 129245m | C | p-[2-(7,8-methanooctyl)-amino]benzoic acid |
| 569 | 6-bromo-6-methyl-1-heptene Chem. Abst. 66, 94482w | C | p-[1-(2-methyl-6,7-methanoheptyl)amino]-benzoic acid |
| 570 | 6-chloro-2-methyl 1-heptene Chem. Abst. 75, 129245 | C | p-[1-(6-methyl-6,7-methanoheptyl)amino]-benzoic acid |
| 571 | E-8-bromo-2-octene Chem. Abst. 74, 99419f | C | p-[1-(6,7-methanooctyl)-amino]benzoic acid |
| 572 | 8-bromo-2,6-dimethyl-2-octene Chem. Abst. 72, 90573c | C | p-[1-(3,7-dimethyl-6,7-methanooctyl)amino]-benzoic acid |
| 573 | 11-bromo-5-undecene Chem. Abst. 67, 73101b | C | p-[1-(6,7-methanoundecyl)amino]benzoic acid |
| 574 | 8-bromo-1-octene Chem. Abst. 70, 10990g | C | p-[1-(7,8-methanooctyl)-amino]benzoic acid |

| Example | Starting Material | Method | Product |
|---|---|---|---|
| 575 | R-8-iodo-7-methyl-1-octene Chem. Abst. 74, 12573e | C | p-[1-(2-methyl-7,8-methanooctyl)amino]-benzoic acid |
| 576 | 1-chloro-7-tetradecene Chem. Abst. 54, 22461h | C | p-[1-(7,8-methanotetradecyl)amino]benzoic acid |
| 577 | 9-chloro-1-nonene Chem. Abst. 70, 114490k | C | p-[1-(8,9-methanonoyl)-amino]benzoic acid |
| 578 | 1-bromo-8-heptadecene Chem. Abst. 52, 249d | C | p-E-1-(9,10-methano-octadecyl)amino]benzoic |
| 579 | E-1-bromo-9-octadecene Chem. Abst. 70, 46779j | C | p-[E-1-(9,10-methano-octadecyl)amino]benzoic acid |
| 580 | Z-1-bromo-9-octadecene Chem. Abst. 70, 46779j | C | p-[Z-1-(9,10-methanooctadecyl)amino]benzoic acid |
| 581 | 11-chloro-1-undecene Chem. Abst. 66, P19046d | C | p-1-(2,6,10-trimethyl-11,12-methanododecyl)-amino]benzoic acid |
| 582 | 12-iodo-3,7,11-trimethyl-1-dodecene | C | p-1-(2,6,10-trimethyl-11,12-methanododecyl)-amino]benzoic acid |
| 583 | 13-bromo-1-tridecene Chem. Abst. 67, 43348v | C | p-1-(12,13-methanotridecyl)amino]benzoic acid |
| 584 | 22-bromo-9-docosene Chem. Abst. 73, 44976j | C | p-1-(13,14-methanodocosyl)amino]benzoic acid |
| 585 | 16-methanesulfonyloxy-1-hexadecene Ref. B | C | p-1-(15,16-methanohexadecyl)amino]benzoic acid |
| 586 | 3-chloro-2,4,4-trimethyl-1-pentene Chem. Abst. 72, 1110811 | C | p-3-(2,4,4-trimethyl-1,2-methanopentyl)amino]-benzoic acid |

EXAMPLE 587

Preparation of 4-(4-tetradecenylamino)benzoyl chloride

A cold solution of 25 g. 4-(4-tetradecenylamino)-benzoic acid in 500 ml. dimethoxyethane-methylenechloride (4:1) is prepared and dry hydrochloric acid is bubbled through the solution until no more percipitate forms.

The solution is treated with 25 ml. thionyl chloride and refluxed until all of the precipitate has dissolved. The solvents are evaporated to yield an orange, semi-crystalline mass.

EXAMPLE 588

Preparation of N-trifluoroacetyl-4-(4-tetradecenylamino)-benzoyl chloride

To a stirred, ice-cold suspension of 9 g. (24.9 m moles) 4-(4-tetradecenylamino)benzoic acid in 100 ml. dimethoxy ethane and 16 ml. pyridine is treated with 18 ml. trifluoracetic anhydride. The solution is stirrd at 0°C. for 30 minutes at room temperature. The solution is diluted with 300 ml. ether and 100 g. ice. After stirring vigorously for 15 minutes, the phases are separated, the ether solution is washed with brine, dried and evaporated to a white amorphous solid.

To 9.2 g. of the above product in 30 ml. methyene chloride and 0.5 ml. dimethylformamide is added 5.7 ml. thionyl chloride. After 20 hours at reflux, the solvents are evaporated to yield the product as light yellow mobile oil.

EXAMPLE 589

Preparation of O-[4-(4-tetradecenylamino)benzoyl]malic acid

A warm solution of N-carbobenzyloxy-4-(4-tetradecenylamino)benzoyl chloride and 1.3 g triethylamine in 100 ml. at room temperature. The solution is diluted with 300 ml. ether and 100 g. ice. After stirring vigorously for 15 minutes, the phases are separated, the ether solution is washed with brine, dried and evaporated to a white amorphous solid.

To 9.2 g. of the above product in 30 ml. methyene chloride and 0.5 ml. dimethylformamide is added 5.7 ml. thionyl chloride. After 20 hours at reflux, the solvents are evaporated to yield the product as light yellow mobile

oil.

EXAMPLE 589

Preparation of O-[4-(4-tetradecenylamino)benzoyl]malic acid

A warm solution of N-carbobenzyloxy-4-(4-tetradecenylamino)benzoyl chloride and 1.3 g triethylamine in 100 ml. ether is treated with 2 g. malic acid. An immediate precipitate forms, but the mixture is refluxed for one hour and filtered while hot. The solid is washed several times with hot ether, then the ether is evaporated to yield a white solid. The product is dissolved in tetrahydrofuran (100 ml.) and hydrogenated over 600 mg. 10% palladium-on-carbon at 20 psi until hydrogen uptake stops. The catalyst is filtered, the solution is evaporated, and the residue is crystallized from acetic acid to yield the title compound as a tan, crystalline mass.

EXAMPLE 590

Preparation of diethyl O-[4-(4-tetradecenylamino)benzoyl]-malate

In a manner similar to Example 589, a solution of 6.0 g. N-carbobenzyloxy-4-(4-tetradecenylamino)benzoyl chloride and 1.2 g. triethylamine in 100 ml warm ether is treated with 2.3 g. diethylmalate. After one hour at reflux, the percipitate is filtered off and washed with warm ether. After evaporation to dryness the residue is dissolved in 50 ml. 30% hydrobromic acid in acetic acid and warmed at 50°C. for 2 hours. The solvents are evaporated and the product is partitioned between methylene chloride and water. The layers are separated and methylene chloride layer is evaporated. The residue is crystallized from acetone to yield the product as colorless crystals.

EXAMPLE 591

Preparation of diethyl O-[4-(4-tetradecenylamino)benzoyl]-tartrate

N-trifluoroacetyl-4-(4-tetradecenylamino)benzoyl chloride and 1.2 g. triethylamine in 100 ml. warm ether is treated with 2.5 g. diethyl tartrate and refluxed for 24 hours. The solution is filtered, the residue is washed with

hot ether, and the solvent evaporated from the combined filtrate and washings to yield a solid residue. After dissolving in aqueous methanolic potassium carbonate, the product is precipitated by acidifiction, filtered, and dried. Crystallization from acetone yields the diethyl tartrate as a white, crystalline solid.

EXAMPLE 592

Preparation of 1-[4-(N-*t*-butyloxycarbonyl-4-tetradecenyl-amino)benzoyl]imidazole

A solution of 10 g. 4-(4-tetradecenylamino)benzoic acid in 100 ml. dioxane is treated with 4.0 g. *t*-butylazidoformate and 10 ml. pyridine. After stirring at room temperature for 18 hours, the protected amido-acid is precipitated from solution by addition of 150 ml. water. The product is collected and thoroughly dried. The crude product is dissolved in 200 ml. of a mixture consisting of methylene chloride/dimethoxyethane/pyridine (1:4:1), and to this is added 5.4 g. 1,1'-carbonyldiimidazole. The solution is stirred overnight at room temperature and the solvents are evaporated to yield the title compound as a thick, orange oil.

EXAMPLE 593

2,3-Dihydroxypropyl 4-(4-pentadecenyl)benzoate

A solution of 7.35 g. of 4-(4-pentadecenyl)benzoic acid in 50 ml. of hexamethylphosphoramide is treated with 4.80 g. of 25% aqueous sodium hydroxide followed by 11.0 g. of 3-chloro-1,2-propanediol and then is heated at 140°C. for 6 hours. The mixture is diluted with water and ether and filtered to yield a white solid. Recrystallization from acetonitrile and then from carbon tetrachloride affords the product as a white solid.

EXAMPLE 594

2,3-Dihydroxypropyl 4-(4-tetradecenylamino)benzoate

A mixture of 2.25 g. of methyl 4-(4-tetradecenyl-amino)benzoate, 280 mg. glycerol, and 1.37 g. of p-toluene-sulfonic acid is heated at 180°C. for 18 hours and then is partitioned between ether and 3% aqueous sodium carbonate

solution. The ether layer is separated, dried, and evaporated to yield the product as a white solid.

EXAMPLE 595

2,3-Dihydroxypropyl 4-(4-tetradecenylamino)benzoate

A solution of 11.8 g. of 4-(4-tetradecenylamino)-benzoic acid, 1.00 g. of glycerol, and 5.35 ml. of boron trifluoride etherate in 200 ml. of toluene is stirred under reflux for 48 hours. The solution is treated with an additional 5.35 ml. of boron trifluoride ethrate and refluxing is continued for 120 hours. Dilution with water and methylene chloride followed by filtration affords the benzoate as a white solid.

EXAMPLE 596

Preparation of N-[4-(4-tetradecenylamino)benzoyl]alanine

A solution of 4.75 g. of N-trifluoroacetyl-4-(tetradecenylamino)benzoyl chloride and 1.2 g. of triethylamine in 200 ml. of warm ether is treatd with 1.55 g. alanine ethyl ester and refluxed for 24 hours. The hot solution is filtered, the residue is washed with hot ether, and the solvent is evaporated from the combined filtrate and washings. After treatment of the residue with aqueous methanolic potassium carbonate, the product is precipitated by acidification, filtered, and dried. Crystallization from acetone yields the product as a white, crystalline solid.

EXAMPLE 597

Preparation of 1-{4-[N-(t-butyloxycarbonyl)-N-(4-tetradecenylamino)benzoyl} imidazole

A solution of 10 g. of 4-(4-tetradecenylamino)-benzoic acid in 100 ml. of dioxane is treated with 4.0 g. of *t*-butylazidoformate and 10 ml. pyridine. After stirring at room temperature for 18 hours, the protected amido-acid is precipitated from solution by addition of 150 ml. of water. The product is collected and thoroughly dried. The crude product is dissolved in 200 ml. of a mixture consisting of methylene chloride/dimethoxy ethane/pyridine (1:4:1), and to this is added 5.4 g. of 1,1'-carbonyldiimazole. The solution is stirred overnight at room temperature and the

solvents are evaporated to yield the title compound as a thick orange oil.

EXAMPLE 598

Preparation of 2-(ethoxycarbonyl)vinyl 4-(4-tetradecenyl-amino)benzoate

To a mixture containing 4.3 g. 1-[4-(N-*t*-butyl-oxycarbonylamino)benzoyl]imidazole, 50 ml. chloroform, and 50 ml. 5N sodium hdroxide is added 3 g. ethyl 2-formyl acetate. The mixture is vigorously stilled for 24 hours. The layers are separated, and the chloroform solution is washed once with 50 ml. 1N sodium hydroxide. The solvent is evaporated from the chloroform layer and the residue is heated for 30 minutes at 40°C. in 50 ml. anhydrous trifluoroacetic acid. The solvent is again evaporated and the oil is crystallized from acetone to yield the product as light yellow crystals.

EXAMPLE 599

Preparation of N-carbobenzyloxy-4-(4,14-pentadecadienyl-amino)benzoyl chloride

To 15 g. of 4-(4,14-pentadecadienylamino)benzoic acid in 200 ml. warm chloroform is added 15 g. sodium carbonate in 150 ml. water. To the vigorously stirred mixture is added 10 g. carbobenzoyl chloride. After 2 hours stirring at 40°C., the layers are separated, the chloroform layer is washed three times with 1N hydrochloric acid, dried, and the solvent evaporated to yield oil. The oil is dissolved in 300 ml. toluene, treated with 15 ml. of thionyl chloride, and the solution is refluxed for 5 hours. The solvents are evaporated and the residue is dissolved three times in toluene, evaporating each time to yield a viscous, orange oil.

EXAMPLE 600

Preparation of 1-[4-(4,14-pentadecadienylamino)benzoyl]-piperdine

To a warm solution of 4-[N-carbobenzoyloxy-4,14-pentadecadienylamino)benzoyl chloride and 1.3 g. of triethylamine in 100 ml. ether is added 1.2 g. of piperdine. An

immediate precipitate forms, but the mixture is refluxed for one hour and filtered while hot. The solid is washed several times with hot ether, then the ether is evaporated from the combined filtrate and washings to yield a white solid. The product is dissolved in tetrahydrofuran (100 ml.) and hydrogenated over 600 mg. 10% palladium on carbon at 20 psi until hydrogen uptake stops. The catylist is filtered. The solution is evaporated, and the residue is crystallized from acetic acid to yield the title compound as a crystalline mass.

## EXAMPLE 601

### Preparation of 1-[4-(4,14-pentadecadienylamino)benzoyl]-pyrrolidine

A solution of 6.0 g. of 4-[N-carbobenzyloxy-N-(4,14-pentadecadienylamino)]benzoyl chloride and 1.2 g.triethylamine in 100 ml. warm ether is treated with 1.1 g. of pyrrolidine. After 1 hour at reflux, the percipitate is filtered off and washed with warm ether. After evaporation of the combined filtrate and washings to dryness, the residue is dissolved in 50 ml. 30% hydrogen bromide in acetic acid and warmed at 50° for 2 hours. The solvents are evaporated and the product is partitioned between methylene chloride and water. The layers are separated and the methylene chloride is evaporated. The residue is crystallized from acetone to yield colorless crystals.

## EXAMPLE 602

### Preparation of O-[4-(4,14-pentadecadienylamino)benzoyl]-malic acid

To a warm solution of N-carbobenzyloxy-4-(4,14-pentadecadienylamino)benzoyl chloride and 1.3 g. triethylamine in 100 ml. ether is treated with 2 g. malic acid. An immediate precipitate forms, but the mixture is refluxed for one hour and fitered while hot. The solid is washed several times with hot ether, then the ether is evaporated to yield a white solid. The product is dissolved in tetrahydrofuran (100 ml.) and hydrogenated over 600 mg. 10% palladium-on-carbon at 50 psi until hydrogen uptake stops. The catalyst is

filtered. The solution is evaporated and the residue is crystallized from acetic acid to yield the title compound as white crystals.

EXAMPLE 603

Preparation of N-[4-($-tetradecenylamino)benzoyl]alanine

A solution of 4.75 g. of N-trifluoroacetyl-4-(4-tetradecenylamino)benzoyl chloride and 1.2 g. of triethylamine in 200 ml. of warm ether is treated with 1.55 g. alanine ethyl ester and refluxed for 24 hours. The hot solution is filtered, the residue is washed with hot ether, and the combined filtrate and washings are evaporated. After treatment of the residue with aqueous methanolic potassium carbonate, the product is precipitated by acidification, filtered, and dried. Crystallization from acetone yields the product a white, crystalline solid.

EXAMPLE 604

Preparation of 4-chlorophenyl 4-(4-tetradecenylamino)-benzoate

To a solution of 6.4 g. 4-chlorophenol and 7.6 g. triethylamine in 500 ml. methylene chloride is added 10.4 g. 4-(4-tetradecenylamino)benzoyl chloride hydrochloride in 250 ml. methylene chloride. After four hours at reflux, the solution is cooled, washed with water and dilute phosphoric acid, and dried. After passing the solution through a column of alumina, the solvent is evaporated and the residue is crystallized from diisopropyl ether.

EXAMPLE 605

Preparation of N- 4-[4- tetradecenylamino]benzoyl -2-amino ethanesulfonic acid

To a stirred solution of 2.50 g. of taurine and 5.6 ml. of triethylamine in 22.5 ml. of water is added 5.55 g. of N- p-[2,2,2-trifluoro-N-(4-tetradecenyl)acetamido]-benzoyloxy succinimide as a solution in 45 ml. of ethanol. After 24 hour, the mixture is treated with 20 ml. of 2.0 M sodium hydroxide and 25 ml. of water. After stirring for 10 minutes, the mixture is acidified with dilute hydrochloric acid, and the crude product is collected by filtration.

Recrystallization affords the title compound as a white solid.

EXAMPLE 606

Preparation of 3-[4-(4-pentadecenylamino)benzoyl]-4-carboethoxythiazolidine

One-tenth mole of 4-(4-pentadecenylamino)benzoyl chloride hydrochloride in methylene chloride is added to a solution of 0.1 mole of ethyl thiazolidine-4-carboxylate in chloroform containing two equivalents of triethylamine. After 5 hours at 20° C. the solution is filtered and evaporated to a white solid which is recrystallized from acetonitrile.

EXAMPLE 607

Preparation of 3-[4-(4-pentadecenylamino)benzoyl]-4-carboxythiazolidine

By means of the alkaline hydrolysis method of Example 222 the ethyl ester of Example 606 is converted to the subject carboxylic acid. This acid is also prepared using the procedure of Example 606 except that the acylation of the thiazolidine-4-carboxylic acid is carried out in aqueous acetone sodium bicarbonate solution.

EXAMPLE 608

Preparation of N-[4-(4-pentadecenylamino)benzoyl]glycine

A mixture of 26.4 g. of ethyl N- 4-(4-pentadecenylamino) benzoyl glycinate, 110 ml. of 1N sodium hydroxide solution, and 100 ml. of ethanol is stirred at ambient temperature for 2 hours and then partially evaporated. The gaseous solution is washed with diethyl ether, acidified with 6N hydrochloric acid, and filtered. The white solid is dried *in vacuo* and recrystallized from acetone.

EXAMPLE 609

Preparation of N-[4-(4-pentadecenylamino)benzoyl]-2,3-dihydroxypropylamine

To a mixture containing 4.3 g. of [N-(*t*-butyloxy carbonyl)-4-(4-pentadecenylamino)benzoyl]imidazole, 50 ml. of chloroform, and 50 ml. of 5N sodium hydroxide is added 1.1 g. of 3-amino-1,2-propanediol. The mixture is vigorously stirred for 24 hours, the layers are separated, and the chloroform solution is washed once with 50 ml. of 1N sodium hydroxide. The solvent is evaporated and the residue is heated for 30 minutes at 40° C. in 50 ml. of anhydrous trifluoroacetic acid. The solvent is again evaporated and the resulting oil is crystallized from acetone to yield the product as light yellow crystals.

EXAMPLE 610

Preparation of N-(3-bromopropyl)-4-(4-tetradecenylamino)-benzamide

To a slurry of 21.80 g. of 3-bromopropylamine hydrobromide in 200 ml. of glyme at 3° C. is added a solution of 23.9 g. of 4-(4-tetradecenylamino)benzoyl chloride hydrochloride in 65 ml. of glyme, concurrently with 26 ml. of triethylamine diluted to 39 ml with 1,2-dimethoxyethane. The solution is warmed to reflux and 0.2 g. of 4-dimethylaminopyridine is added. The solution was heated for four hours and cooled overnight. The solid is removed and the mother liquor diluted with 200 ml. of water to yield a solid which is crystallized from cyclohexane and recrystallized from acetonitrile to yield the product.

EXAMPLE 611

Preparation of 2-[4-(4-tetradecenylamino)phenyl]-5,6-dihydro-[4H]1,3-oxazine

To 0.4 g. of sodium hydride in 100 ml. of 1,2-dimethoxyethane is added 2.14 g. of N-(3-bromopropyl)-4-(4-tetradecenylamino) benzamide and 12 ml. of triethylamine. The turbid solution is heated to reflux for 20 hours. The solution is diluted with 100 ml. of water and cooled overnight. The solid is collected, washed with water, crystallized from cyclohexane, and recrystallized from acetonitrile to yield the product.

EXAMPLE 612

Preparation of 2-[4-(4-tetradecenylamino)phenyl]oxazoline

To a slurry of 15 g. of 2-bromoethylamine hydrobromide in 150 ml. of 1,2-dimethoxyethane are added simultaneously solutions of 31 g. of 4-(4-tetradecenylamino)benzoyl chloride hycrochloride in 60 ml. of 1,2-dimethoxyethane and 50 cc. of triethylamine (dropwise). Upon addition of 0.5 g. of 4-dimethylaminopyridine the mixture is stirred at room temperature overnight. The solution is refluxed for one hour and filtered. The solid is oven dried and partitioned between methylene chloride and water. The layers are separated and the organic phase dried over magnesium sulfate. The organic layer is concentrated and the residue collected and crystallized from cyclohexane and recrystallized from acetonitrile to yield the product.

EXAMPLE 613

Preparation of tetrahydropyranyl 4-(4-tetradecenylamino)-benzoate

A mixture of 7 g. 4-(4-tetradecenylamino)benzoic acid, 2 g. dihydropyran and 100 mg. anhydrous p-toluenesulfonic acid in 50 ml. toluene is stirred at room temperature for 20 hours. The solution is washed with saturated sodium bicarbonate, dried, and evaporated. The residue is collected and crystallized from methylcyclohexane to yield the product as white crystals.

EXAMPLE 614

Preparation of pyridin-3-yl 4-(4-tetradecenylamino)benzoate

A 6 g. sample of 4-(4-tetradecenylamino)benzoic acid and 2.7 g. 1,1'-carbonyldiimidazole in 50 ml. dry tetrahydrofuran is stirred for 2 hours. Then, 1.58 g. 3-hydroxypyridine and a trace of sodium hydride catalyst is added and the reaction is refluxed for 3 hours. The solution is cooled, filtered, and evaporated. The product is crystallized from isopropanol.

EXAMPLE 615

Preparation of 16-bromohexadecanoic acid

A mixture of 18 g. of 16-hydroxyhexadecanoic acid and 160 g. of 30-32% hydrogen bromide in acetic acid is treated with 32 ml. of concentrated sulfuric acid and stirred at ambient temperature for 18 hours. The solution is stirred under reflux for 7 hours and then poured into 500 ml. of ice-water and filtered. A methylene chloride solution of the product is Darco clarified, dried over magnesium sulfate, and evaporated. Crystallization of the residue from ether-petroleum ether and then acetonitrile affords 10-bromohexadecanoic acid as a white solid.

EXAMPLE 616

Preparation of 11-bromoundecanol

A solution of 5 g. of 11-bromoundecanoic acid and 20 ml. of tetrahydrofuran is stirred at 0°C. while 16 ml. of 1M borane in tetrahydrofuan is added during 15 minutes. The mixture is stirrd 18 hours at ambient temperature, poured into ice-water, and extracted with ether. The dried extract is evaporated and residual oil crystallized from hexane to yield 11-bromoundecanol as a white solid.

EXAMPLE 617

Preparation of 1-methanesulfonyloxy-10-undecane

To a solution of 10-undecen-1-ol (15.0 g.) and triethylamine (14 ml.) in dry methylene chloride (320 ml.) at -8°C. is added methanesulfonylchloride (5.73 ml.), dropwise. The reaction mixture is stirrd at -10°C. for 30 minutes and then diluted with methylene chloride, extracted with ice-water (250 ml.), followed by cold 10% hydrochloric acid (200 ml.); cold saturated sodium bicarbonate (200 ml.) and cold brine (200 ml.). The organic phase is dried over magnesium sulfate and the solvent removed *in vacuo* to provide the mesylate as an oil.

EXAMPLE 618

Preparation of 6-oxohexadecyl bromide

The Grignard reagent prepaed from 60 g. of decyl bromide, 5.6 of magnesium, and 200 ml. of ether is treated with 27 g. of cadmium chloride and the solution is stirred under reflux for 30 minutes. The solvent is replaced by distillation with toluene, and the toluene solution is treated with 29 g. of 6-bromohexanoyl chloride and stirred under reflux for 30 minutes. The mixture is cooled, diluted with 200 ml. of 10% sulfuric acid, and extracted with ether. The extracts are dried and evaporated and the residual oil distilled *in vacuo* to yield 6-oxohexadecyl bromide as a

light yellow oil.

EXAMPLE 619

Preparation of ethyl 4-(11-hydroxyundecylamino)benzoate

A solution of 16 g. of 11-bromoundecanol, 9.9 g. of ethyl 4-aminobenzoate, and 6.0 g. of triethylamine in 50 ml. of hexamethylphosphoramide is stirred at 70°C. under argon for 128 hours. The resulting solution is cooled, diluted with water, and filtered. Recrystallization of the solid from acetonitrile affords ethyl 4-(11-hydroxyundecylamino)benzoate as a white, crystalline solid.

EXAMPLE 620

Preparation of ethyl 4-(10-undecenylamino)benzoate

A solution of 14.0 g. of 1-methanesulfonyloxy-10-undecane and 19.8 g. of ethyl p-aminobenzoate in hexamethylphosphoramide is heated at 120°C. for 20 hours. After cooling the reaction mixture is diluted with ethanol:water (1:1) (30 ml.) and chilled. More ethanol is added and the solid material is collected. This solid is recrystallized from ethanol to yield ethyl 4-(10-undecenylamino)benzoate as a white solid.

EXAMPLE 621

Preparation of ethyl 4-(6-oxohexadecylamino)benzoate

A solution of 15 g. of 6-oxohexadecyl bromide and 15.6 g. of ethyl 4-aminobenzoate in 60 ml. of hexamethylphosphoramide is stirred at 120°C. for 18 hours, allowed to cool, and diluted with water. The mixture is filtered and the resulting solid is dissolved in methylene chloride. The solution is washed with dilute hydrochloric acid, dried, and evaporated to yield a brown solid. Crystallization from ethanol yields ethyl 4-(6-oxohexadecylamino)benzoate as a white solid.

EXAMPLE 622

Preparation of 4-(16-hydroxyhexadecylamino)benzonitrile

p-Aminobenzonitrile (12 g.) and 15-bromo-1-hexadecanol (15 g.) are dissolved in hexamethylposphoramide (200 ml.) and heated under nitrogen in an oil bath maintained at 120°C for 22 hours. The reaction mixture is cooled to room

temperature and water (50 ml.) is added gradually. The mixture is then chilled in an ice-bath. The precipitate separated is filtered, washed thoroughly with water, and dried. It is then washed repeatedly with hexane and dried. The pale brownish yellow granular solid is obtained as a homogeneous product. Recrystallization from ether-hexane affords 4-(16-hydroxyhexadeclyamino)benzonitrile as pale yellow prisms.

EXAMPLE 623

Preparation of 1-[4-hydroxytetradecylamino)benzoyl]pyrrolidine

A solution of 10 g. of 14-hydroxytertadecyl bromide and 10.8 g. of ethyl 1-(4-amoniobenzoyl)pyrrolidine in 75 ml. hexamethylphosphoramide is heated at 110°C. for 17 hours. The cooled solution is diluted with 100 ml. water, filtered, and the residue is washed in portions with 100 ml. 50% ethanol-water. After drying the product is crystallized from ethanol to yield 1-[4-(14-hydroxytertadecylamino)benzoyl]-pyrrolidine as a white solid.

EXAMPLE 624

Preparation of 4-(11-Hydroxyundeclyamino)benzoic acid

A solution of 5 g. of ethyl 4-(11-hydroxyundecylamino)benzoate in 75 ml. 95% ethanol is saponified with 2.5 g. of 85% potassium hydroxide by refluxing for 5 hours. The warm solution is diluted with 150 ml. water and adjusted to pH 5 with 37% hydrochloric acid. The precipitate is filtered, washed with water, dried *in vacuo* and crystallized from acetic acid to yield 4-(11-hydroxyundecylamino)benzoic acid as a white solid.

EXAMPLE 625

Preparation of 4-(16-bromohexadecylamino)benzaldehyde

Di-*iso*-butylaluminum hydride (54 ml., 25% solution in toluene) is added with stirring to a solution of 4-(16-bromohexadecylamino)benzonitrile (11.4g) under a nitrogen atmosphere. The temperature rises to 40°C. during the 30 minute addition and the reaction is then stirred for 1 hour. A solution of methanol in toluene (50 ml., 1:1) is

added over 30 minutes and the mixture is poured into vigorously stirred ice-cold aqueous sulfuric acid (50 ml., 5%). After 10 minutes diatomaceous earth (39 g.) is added, the mixture filtered and the organic layer separated. The aqueous solution is extracted twice with toluene (100 ml.) and the combined organic layers are washed with aqueous sodium bicarbonate, dried over magnesium sulfate, decolorized with charcoal, filtered and evaporated _in vacuo_ to give a light yellow crystalline solid. The crude product is recrystallized from dichloromethane-hexane to yield 4-(16-bromohexadecylamino)benzaldehyde as a white solid.

EXAMPLE 626

Preparation of ethyl 4-(11-bromoundecanamido)benzoate

A solution of 20 g. of 11-bromoundecanoyl chloride in 100 ml. of methylene chloride is slowly added with stirring to a solution of 20 g. of ethyl 4-aminobenzoate in 100 ml. of methylene chloride. The mixture is filtered and the filtrate is washed with dilute hydrochloric acid and water, dried, and evaporated. Crystallization from acetonitrile affords ethyl 4-(11-bromoundecanamido)benzoate as a white solid.

EXAMPLE 627

Preparation of ethyl 4-(11-bromoundecylamino)benzoate

A solution of 5 g. of ethyl 4-(11-bromoundecanamido)benzoate in 50 ml. of tetrahydrofuran is slowly added with stirring to 13 ml. of 1M borane in tetrahydrofuran. The solution is poured into 50 ml. of dilute hydrochloric acid and the resulting mixture is filtered. The solid is crystallized from acetonitrile to yield ethyl 4-(11-bromoundecylamino)benzoate as a white, crystalline solid.

EXAMPLE 628

Preparation of ethyl 4-(3-benzylthiopropylamino)benzoate

A mixture of 17 g. of ethyl 4-aminobenzoate and 14 g. of 3-benzylthiopropionaldehyde in 100 ml. of absolute ethanol is heated on a steam bath for 10 minutes. The solid which separates upon cooling the mixture is collected, dried, and redissolved in 200 ml. of hot ethanol. The solution is treated portion-wise with 4 g. of sodium borohydride and the

stirred mixture is refluxed for 3 hours. Dilution with water and filtration yields ethyl 4-(3-benzylthiopropylamino)benzoate as a white, crystalline solid.

EXAMPLE 629

Preparation of sodium 4-(11-hydroxyundecylamino)benzoate

A mixture of 3.62 g. of 4-(11-hydroxyundecylamino)benzoic acid and 25 ml. of ethanol-water (9:1) containing 400 mg. of sodium hydroxide is stirred for 4 hours. The mixture is filtered and the solid washed with ethanol-water and dried *in vacuo* to yield sodium 4-(11-hydroxyundecylamino)benzoate as a white, amorphous solid.

TABLE V

Acyl halides required for the preparation of compounds shown in Table V are obtained from the corresponding carboxylic acids, some of which are prepared by the method of Example 615, by treatment with thionyl chloride. Many of the alkyl halides and sulfonate esters required for the preparation of these compounds are prepared by the methods of Examples 616-618. The 4-aminophenyl compounds shown in Table V are prepared by reactions of these materials with appropriate 4-aminophenyl derivatives by the methods of Examples 619-629 as shown in Table V.

| Example No. | Methods of Examples | Product |
|---|---|---|
| 630 | 619, 624 | 4-(16-Hydroxyhexadecylamino)-benzoic acid |
| 631 | 619, 624 | 4-(14-Hydroxytetradecylamino)-benzoic acid |
| 632 | 619, 624 | 4-(8-Hydroxyloctylamino)benzoic acid |
| 633 | 619, 624 | 4-[5-Hydroxy-1-(n-butyl)pentyl-amino]-benzoic acid |
| 634 | 619, 624 | 4-(11-Hydroxyundec-2-enylamino)-benzoic acid |
| 635 | 619, 624 | 4-(16-Hydroxyhexadec-7-enyl-amino)-benzoic acid |
| 636 | 619, 624 | 4-[11-Hydroxy-1-(n-pentyl)undec-2-nylamino]benzoic acid |

TABLE V (cont'd)

| Example No. | Methods of Examples | Product |
|---|---|---|
| 637 | 619, 624 | 4-[(3-Hydroxy-2,6,6-trimethlyl-cyclohexyl)methylamino)]benzoic acid |
| 638 | 620 | Ethyl 4-(4-pentadecenylamino)-benzoate |
| 639 | 620 | Ethyl 4-(2-hexadecenylamino)-benzoate |
| 640 | 620 | Ethyl 4-(9-octadecenylamino)-benzoate |
| 641 | 620 | Ethyl 4-(15-hexadecenylamino)-benzoate |
| 642 | 621, 624 | 4-(11-Oxohexadecylamino)benzoic acid |
| 643 | 621,624 | 4-(6-Oxotetradecylamino)benzoic acid |
| 644 | 621, 624 | 4-(3-Oxoundecylamino)benzoic acid |
| 645 | 622, 625 | 4-(14-Chlorotetradecylamino)-benzaldehyde |
| 646 | 622, 625 | 4-[11-(*n*-Butylthio)undecylamino]-benzaldehyde |
| 647 | 622,625 | 4-(2-Benzylthioundecylamino)-benzaldehyde |
| 648 | 623 | 1-[4-(11-Benzylthioundecylamino)-benzoyl]pyrrolidine |
| 649 | 623 | 1-[4-(6-Oxoundecylamino)benzoyl]-piperidine |
| 650 | 623 | 1-[4-(16-Hydroxyhexadecylamino)-benzoyl]pyrrolidine |
| 651 | 626, 627, 624 | 4-(16-Bromohexadecylamino)benzoic acid |
| 652 | 626, 627, 624 | 4-[(3-Bromo-2,2,4-trimethylcyclopentyl)methylamino]benzoic acid |

TABLE V (cont'd)

| Example No. | Methods of Examples | Product |
|---|---|---|
| 653 | 626, 627, 624 | 4-(2-Benzylthiooctylamino)benzoic acid |
| 654 | 626, 627, 624 | 4-(11-Methylthioundecylamino)-benzoic acid |
| 655 | 626, 627, 624 | 4-(18-Bromooctadecylamino)benzoic acid |
| 656 | 626, 627, 624 | 4-[8-Bromo-2-(n-butyl)octyl-amino]benzoic acid |
| 657 | 626, 627, 624 | 4-(12-Bromooctadecylamino)-benzoic acid |
| 658 | 628, 624 | 4-[4-(4-Chlorophenylthio)octyl-amino]benzoic acid |
| 659 | 628, 624 | 4-(2-Methylthiotetradecylamino)-benzoic acid |
| 660 | 628, 624 | 4-(11-Phenylthioundec-2-enyl-amino)benzoic acid |
| 661 | 629 | Sodium 4-(16-bromohexadecyl-amino)benzoate |
| 662 | 629 | Sodium 4-(16-hydroxyhexadecyl-amino)benzoate |
| 663 | 629 | Sodium 4-(11-methylthioundecyl-amino)benzoate |
| 664 | 619, 624 | 4-(3-Hydroxytetradecylamino)-benzoic acid |
| 665 | 620 | Ethyl 4-(9-hexadecenylamino)-benzoate |
| 666 | 621, 624 | 4-(3-Oxotetradecylamino)benzoic acid |
| 667 | 622, 625 | 4-(16-Methylthioundecylamino)-benzaldehyde |
| 668 | 623 | 1-[4-(11-Benzylthioundecyl-amino)benzoyl]morpholine |

EXAMPLE 669

Preparation of 4-(16-bromohexadecylamino)benzoyl chloride hydrochloride

A cold solution of 25 g. 4-(16-bromohexadecylamino)-benzoic acid in 500 ml. dimethoxyethane-methylene chloride (4:1) is prepared and dry hydrochloric acid is bubbled through the solution until no more precipitate forms. The solution is treated with 25 ml. thionyl chloride and refluxed until all of the precipitate has dissolved. The solvents are evaporated to yield an orange, semi-crystalline mass.

EXAMPLE 670

Preparation of N-trifluoroacetyl-4-(16-bromohexadecylamino)-benzoyl chloride

A stirred, ice-cold suspension of 9 g. 4-(16-bromohexadecylamino)benzoic acid in 100 ml. dimethoxyethane and 16 ml. pyridine is treated with 18 ml. trifluoroacetic anhydride. The solution is stirred at 0°C. for 30 minutes and at room temperature. The solution is diluted with 300 ml. ether and 100 g. ice. After stirring vigorously for 15 minutes, the phases are separated, the ether solution is washed with brine, dried and evaporated to a white, amorphous solid.

To 9.2 g. of the above product in 30 ml. methylene chloride and 0.5 ml. dimethylformamide is added 5.7 ml. thionyl chloride. After 20 hours, at reflux, the solvents are evaporated to yield a light yellow, mobile oil.

EXAMPLE 671

N-Carbobenzyloxy-4-(bromohexadecylamino)benzoyl choride

To 15 g. 4-(16-bromohexadecylamino)benzoic acid in 200 ml. warm chloroform is added 15 g. sodium carbonate in 150 ml. water. To the vigorously stirred solution is added 10 g. carbobenzyloxy chloride. After 2 hours stirring at 40°C., the layers are separated, washed three times with 1N hydrochloric acid dried, and evaporated to an oil. The oil is dissolved in 300 ml. toluene, treated with 15 ml. thionyl chloride and the solution is refluxed for 5 hours. The solvents are evaporated and the residue is dissolved

three times in toluene, evaporating each time to yield a viscous, orange oil.

EXAMPLE 672

Preparation of 1-{4-[N-t-butyloxycarbonyl)-N-(16-bromohexadecyl)amino]benzoyl}imidazole

A solution of 10 g. 4-(16-bromohexadecylamino)-benzoic acid in 100 ml. dioxane is treated with 4.0 g. t-butylazidoformate and 10 ml. pyridine. After stirring at room temperature for 18 hours, the protected amido-acid is precipitated from solution by addition of 150 ml. water. The product is collected and thoroughly dried. The crude product is dissolved in 200 ml. of a mixture consisting of methylene chloride/dimethoxyethane/pyridine (1:4:1), and to this is added 5.4 g. 1,1'-carbonyldiimidazole. The solution is stirred overnight at room temperature and the solvents are evaporated to yield the title compound as a thick, orange oil.

EXAMPLE 673

Preparation of potassium sulfomethyl 4-(11-hydroxyundecylamino)benzoate

To 39.3 g. of potassium 4-(11-hydroxyundecylamino)-benzoate in 125 ml. of hexamethylphosphoramide is added 26.0 g. of powdered potassium iodomethanesulfonate. After stirring for 24 hours at 25°C., the product is obtained by careful dilution with water or alcohol to the crystallization point.

When this reaction is carried out with potassium 2-iodoethansulfonate, the potassium sulfoethyl ester is obtained.

EXAMPLE 674

2,3-Dihydroxypropyl 4-(11-hydroxyundecylamino)benzoate

A solution of 7.34 g. of 4-(11-hydroxyundecylamino)-benzoic acid, 4.80 g. of 25% aqueous sodium hydroxide, and 12.6 g. of 3-iodo-1,2-propanediol in 50 ml. of hexamethylphosphoramide is stirred for 24 hours at ambient temperature, diluted with 100 ml. of ether and stirred for 5 days at ambient temperature. The mixture is treated with water and

extracted with ether. The dried extracts are evaporated to yield, 2,3-dihydroxypropyl 4-(11-hydroxyundecylamino)benzoate.

EXAMPLE 675

2,3-Dihydroxypropyl 4-(16-bromohexadecylamino)benzoate

A solution of 11.8 g. of 4-(16-bromohexadecylamino)benzoic acid, 1.00 g. of glycerol, and 5.35 ml. of boron trifluoride etherate in 200 ml. of toluene is stirred under reflux for 48 hours. The solution is treated with an additional 5.35 ml. of boron trifluoride etherate and refluxed for 120 hours. Dilution with water and methylene chloride followed by filtration affords 2,3-dihydroxypropyl 4-(16-bromohexadecylamino)benzoate as a white solid.

EXAMPLE 676

2,3-Dihydroxypropyl 4-(11-bromoundecylamino)benzoate

A mixture of 2.25 g. of methyl 4-(11-bromoundecylamino)benzoate, 280 mg. of glycerol, and 1.37 g. of p-toluenesulfonic acid is heated at 180°C. for 18 hours and then is partitioned between ether and 3% aqueous sodium carbonate solution. The ether layer is separated, dried, and evaporated to yield 2,3-dihydroxypropyl 4-(11-bromoundecylamino)benzoate.

EXAMPLE 677

Methyl 4-(16-bromohexadecylamino)benzoate

A solution of 50.5 g. of 4-(16-bromohexadecylamino)benzoic acid and 34.4 ml. of boron trifluoride etherate in 200 ml. of methanol is stirred under reflux for 44 hours, allowed to cool, and is poured into 1.20 litres of ice-cold 5% aqueous sodium carbonate solution. The white solid is collected by filtration and recrystallized from benzene-ethanol to yield methyl 4-(16-bromohexadecylamino)benzoate, m.p. 92°-93°C.

EXAMPLE 678

Preparation of 1-(methoxycarbonyl)propyl 4-(16-bromohexadecylamino)benzoate

To a solution of 10.0 g. 4-(16-bromohexadecyl=amino)benzoyl chloride hydrochloride in 200 ml. methylene chloride is added dropwise a solution of 3 g. methyl 2-hydroxy butyrate and 5 g. triethylamine in 100 ml. ether. After 17 hours stirring at room temperature, the precipitate is filtered and washed with several portions of ether. The ether solution is washed with water, dried and condensed to the crystalline title compound.

EXAMPLE 679

Preparation of 1-carboxyethyl 4-(16-bromohexadecylamino)-benzoate

A flask containing 10.0 g. 4-(16-bromohexadecylamino)benzoic acid, 3.3 g. lactic acid, 500 mg. toluenesulfonic acid and 500 ml. toluene is equipped with a Soxhlet extractor charged with activated 4A Linde molecular sieves. The solution is refluxed for 24 hours, during which time the Soxhlet extractor is charged twice more with fresh sieves. The hot solution is filtered and left to cool, whereupon the product separates as off-white crystals.

EXAMPLE 680

Preparation diethyl O-[4-16-bromohexadecylamino)benzoyl]tartrate

N-Trifluoroacetyl-4-(16-bromohexadecylamino)benzoyl chloride and 1.2 g. triethylamine in 100 ml. warm ether is treated with 2.5 g. diethyl tartrate and refluxed for 24 hours. The hot solution is filtered, the residue is washed with hot ether, and the solution is evaporated. After treatment with aqueous methanolic potassium carbonate, the product is precipitated by acidification, filtered and dried. Crystallization from acetone yields the diethyl tartrate as a white, crystalline solid.

EXAMPLE 681

Preparation of O-[4-(16-bromohexadecylamino)benzoyl]malic acid

A warm solution of N-carbobenzyloxy-4-(16-bromohexadecylamino)benzoyl chloride and 1.3 g. triethylamine in 100 ml. ether is treated with 2 g. malic acid. An immediate precipitate forms, but the mixture is refluxed for one hour and filtered while hot. The solid is washed several times with hot ether, then the ether is evaporated to

yield a white solid. The product is dissolved in tetrahydrofuran (100 ml.) and hydrogenated over 600 mg. 10% palladium-on-carbon at 50 psi until hydrogen uptake stops. The catalyst is filtered, the solution is evaporated, and the residue is crystallized from acetic acid to yield the title compound as a tan, crystalline mass.

EXAMPLE 682

Preparation of 2-(ethoxycarbonyl)vinyl 4-(16-bromohexadecylamino)benzoate

To a mixture containing 4.3 g. 1-{4[N-(t-butyloxycarbonyl)-N-(16-bromohexadecyl)amino]benzoyl} imidazole, 50 ml.chloroform, in 50 ml. 5N sodium hydroxide is added 3 g. ethyl 2-formyl acetate. The solution is vigorously stirred for 24 hours. The layers are separated, and the chloroform solution is washed once with 50 ml. 1N sodium hydroxide. The solvent is evaporated and the residue is heated for 30 minutes at 40°C. in 50 ml. anhydrous trifluoroacetic acid. The solvent is again evaporated and the oil is crystallized from acetone to yield light yellow crystals of 2-ethoxycarbonyl)vinyl 4-(16-bromohexadecylamino)benzoate.

EXAMPLE 683

Preparation of 2-(ethoxycarbonyl)ethyl 4-(16-bromohexadecylamino)benzoate

A solution of 4 g. 2-(ethoxycarbonyl)vinyl 4-(16-bromohexadecylamino)benzoate and 400 mg 10% palladium-on-carbon in 100 ml. tetrahydrofuran is hydrogenated at 50 psi until hydrogen uptake stops. The catalyst is filtered, the solution is evaporated, and the residue is crystallized from acetonitrile to yield 2-(ethoxycarbonyl)ethyl 4-(16-bromohexadecylamino)benzoate.

TABLE VI

The benzoic acid derivatives required for the preparation of the benzoate esters of the Table are prepared from the corresponding acids by the methods of Examples 669-672. The carboxylic acids required are prepared by the methods of Examples 619-629 or 762-775. The benzoate

esters of the table are prepared from these carboxylic acids or derivatives thereof by the methods of Examples 673-683.

| Example No. | Method of Example | Product |
|---|---|---|
| 684 | 673 | Potassium sulfomethyl 4-(2-hydroxytetradecylamino)benzoate |
| 685 | 673 | Ethyl O-[4-(11-hydroxyundec-2-enylamino)benzoyl]glycolate |
| 686 | 674 | 2,3-Dihydroxypropyl 4-[11-hydroxy-1-(n-pentyl)undec-2-ynylamino]benzoate |
| 687 | 674 | 2,3-Dihydroxypropyl 4-(6-oxotetradecylamino)benzoate |
| 688 | 674 | 2,3-Dihydroxypropyl 4-(11-benzylthioundecylamino)benzoate |
| 689 | 674 | 3-Hydroxypropyl 4-(11-methylsulfonylundecylamino)benzoate |
| 690 | 674 | 2-Hydroxypropyl 4-(16-azidohexadecylamino)benzoate |
| 691 | 673 | 2- 4-[(11-benzylsulfinyl)undecylamino]benzoyl ethanesulfonic acid |
| 692 | 673 | Potassium sulfomethyl 4-[(3-acetamido-2,6,6-trimethylcyclohexyl)methylamino]benzoate |
| 693 | 675 | 2-Ethoxyethyl 4-(11-oxohexadecylamino)benzoate |
| 694 | 675 | Isopropyl 4-[11-(n-butylsulfonyl)undecylamino]benzoate |
| 695 | 675 | 2-Dimethylaminoethyl 4-(3-oxoundecylamino)benzoate |
| 696 | 675 | 2-Ethoxyethyl 4-(20-methylsulfinyltetradecylamino)benzoate |
| 697 | 677 | Methyl 4-(16-chlorohexadecylamino)benzoate |
| 698 | 677 | Methyl 4-(2-benzylsulfonyloctylamino)benzoate |

TABLE VI (cont'd)

| Example No. | Method of Example | Product |
|---|---|---|
| 699 | 677 | Methyl 4-(2-benzamidooctylamino)benzoate |
| 700 | 677 | Methyl 4-(11-phenylaminoundecylamino)benzoate |
| 701 | 676 | 3-Hydroxypropyl 4-(16-iodohexadecylamino)benzoate |
| 702 | 676 | 4-Hydroxybutyl 4-(2-azidotetradecylamino]benzoate |
| 703 | 678 | 4-Fluorophenyl 4-[11-(n-butylsulfonyl)undecylamino]benzoate |
| 704 | 678 | 3-Methylbenzyl 4-[(8-benzylsulfinyl)octylamino]benzoate |
| 705 | 678 | 4-Pyridylmethyl 4-(2-chlorotetradecylamino)benzoate |
| 706 | 678 | 2,6-Dichlorophenyl 4-(10-methylthiodec-2-enylamino)benzoate |
| 707 | 678 | 1-Methyl-4-piperidyl 4-(16-chlorohexadecylamino)benzoate |
| 708 | 678 | Allyl 4-(6-oxoundecylamino)-benzoate |
| 709 | 678 | 2,3-Epoxypropyl 4-(11-chlorohexadecylamino)benzoate |
| 710 | 679 | 1-Carboxyethyl 4-[(3-bromo-2,-2,4-trimethylcyclopentyl)methylamino]benzoate |
| 711 | 679 | 1-Carboxymethyl 4-(2-methylsulfonyltetradecylamino)benzoate |
| 712 | 679 | 1-Carboxyethyl 4-[11-chlorophenylsulphonyl)undec 2 enylamino)benzoate |
| 713 | 679 | Tetrahydropyranyl 4-(14-chlorotetradecylamino)benzoate |
| 714 | 679 | Tetrahydropyranyl 4-(2-oxotetradecylamino)benzoate |
| 715 | 680 | Diethyl O-[4-(6-oxoundecylamino)benzoyltartarate |

TABLE VI (cont'd)

| Example No. | Method of Example | Product |
|---|---|---|
| 716 | 680 | Diethyl O-[4-(16-chlorohexadec-7-enylamino)benzoyl]tartrate |
| 717 | 680 | Diethyl O-[4-(11-iodo-1-methyl-undec-2-ynylamino)benzoyl]tartrate |
| 718 | 681 | O-[4-(8-chlorooctylamino)benzoyl]malic acid |
| 719 | 681 | O-4[-(11-methansulfonamidoundec-2-enylamino)benzoyl]malic acid |
| 720 | 682 | 2-(Ethoxycarbonyl)vinyl 4-(10-benzylthiodecylamino)benzoate |
| 721 | 682 | 2-(Ethoxycarbonyl)vinyl 4-(3-bromo-2,2,4-trimethylcyclopentyl)methylamino]benzoate |
| 722 | 683 | 2-(Ethoxycarbonyl)ethyl 4-(12-chlorooctadecylamino)benzoate |
| 723 | 676 | 2,3-Dihydroxypropyl 4-(14-bromotetradecylamino)benzoate |
| 724 | 676 | 2,3-Dihydroxypropl 4-(11-chloroundecylamino)benzoate |
| 725 | 676 | 2-Hydroxypropyl 4-(11-bromoundecylamino)benzoate |

EXAMPLE 726

Preparation of 1-[4-(16-bromohexadecylamino)benzoyl]-piperidine

To a chilled solution of 35 ml. of piperidine, 2.5 ml. of triethylamine and 0.6 g. of dimethylaminopyridine in 100 ml. of diethyl ether is added (1/2 hour) a solution of 8.3 g. of 4-(16-bromohexadecylamino)benzoyl chloride hydrochloride in 50 ml of ether. The solution is warmed to room temperature and maintained there for two hours. The solution is heated to reflux for an additional 2 hours at which time the reaction is complete. The solution is cooled, extracted twice with 100 ml. portions of water and dried ovr magnesium sulfate. The solvent is removed *in*

vacuo and the solid recrystallized in 50 ml. of diethyl ether to yield the product.

EXAMPLE 727

Preparation of ethyl 4-(16-bromohexadecylamino)hippurate

To a solution of 18.08 g. of 4-(16-bromohexadecylamino)benzoic acid in a mixture of dioxane and methylene chloride (40 ml./160 ml.) is added gaseous hydrogen chloride for 10 minutes. The slurry is cooled and 18 ml of thionyl chloride added. The slurry is broughy to reflux for 2 hours and then concentrated under vacuum (thrice diluting with dioxane each time). The final amber solution is diluted with 100 ml. of dioxane and this solution added to freshly prepared ethyl glycinate in 300 ml. of methylene chloride containing 1 g. of dimethylaminopyridine and 10 ml. of triethylamine. After 16 hours at room temperature the reaction is refluxed for 2 hours, cooled and filtered. The mother liquor is extracted with water and 10% hydrochloric acid. The solution is dried over magnesium sulfate and concentrated in vacuo to an amber liquid. A sample is preabsorbed on silica and eluted with ether giving a solid. This material is placed on a second silica column giving a second solid which is recrystallized from acetonitrile to yield the product.

EXAMPLE 728

Preparation of N-[4-(16-bromohexadecylamino)benzoyl]glycine

A mixture of 26.4 g. of ethyl N-[4-(16-bromohexadecylamino)benzoyl]glycinate, 110 ml. of 1N sodium hydroxide solution; and 100 ml. of ethanol is stirred at ambient temperature for 2 hours and then partially evaporated. The aqueous solution is washed with diethyl ether, acidified with 6N hydrochloric acid, and filtered. The white solid is dried in vacuo and recrystallized from acetone to yield the product.

EXAMPLE 729

Preparation of 4-(16-bromohexadecylamino)-N-(methylsulfonyl)-benzamide

A solution of 19.0 g. of methanesulfonamide in 150

ml. of dry dimethyacetamide is added dropwise over 15 minutes to a stirred and cooled (water bath) suspension of 5.5 g. of sodium hydride in 100 ml. of dry dimethylacetamide. The mixture is then stirred and heated at 60°-80°C. for 2 hours. In the meantime, a mixture of 36.2 g. of 4-(16-bromohexadecylamino)benzoic acid in 1200 ml. of methylene chloride, 300 ml. of dimethoxyethane, and 40 ml of thionyl chloride is refluxed for 1 hour and 15 minutes. The solution is evaporated to an oil which is co-evaporated twice with added dioxane to remove excess thionyl chloride. To the resulting oily residue of 4-(16-bromohexadecylamino)benzoyl chloride hydrochloride is added, in one portion, the previously prepared mixture of sodium methansulfonamide in dimethylacetamide. The mixture becomes very hot and is cooled briefly in a water bath and then is stirred at room temperature for 30 minutes. The mixture is filtered through a bed of diatomaceous earth and the filtrate is poured into 2 l. of water. The resulting suspension is coagulated by the addition of 250 ml. of saturated sodium chloride solution and the mixture is filtered. The product is washed with water, partially air dried and then crystallized from 1.5 l. of ethanol. The product is recrystallized twice from p-dioxane and dried in the Abderhalden at 65°C. to give the title compound as tan crystals.

EXAMPLE 730

Preparation of [4-16-bromohexadecylamino)benzoyl]hydroxamic acid

16.7 g. of 4-(16-bromohexadecylamino)benzoyl chloride hydrochloride in methylene chloride is added at 5°C. to a mixture of 2.8 g. of hydroxylamine hydrochloride, 4.2 g. of sodium carbonate, and 120 ml. of ether. The mixture is stirred 3 hours after adding 10 ml. of water. After the addition of 150 ml. of water, the solvents are aerated off. The crystalline product is recrystallized from acetonitrile.

EXAMPLE 731

Preparation of 3-[4-(16-bromohexadecylamino)benzoyl]-4-carboxythiazolidine

One-tenth mole of 4-(16-bromohexadecylamino)benzoylchloride hydrochloride in methylene chloride is added to a solution of 0.1 mole of ethyl thiazolidine-4-carboxylate in chloroform containing two equivalents of triethylamine. After 5 hours at 20°C. the solution is filtered and evaporated to a white solid which is recrystallized from acetonitrile to yield 3-[4-(16-bromohexadecylamino)benzoyl]-4-carbethoxythiazolidine. By means of the alkaline hydrolysis method of Example 624, the ethyl ester is converted to the subject carboxylic acid. This acid is also prepared using the procedure of this Example except that the acylation of the thiazolidine-4-carboxylic acid is carried out in aqueous acetone sodium bicarbonate solution.

EXAMPLE 733

Preparation of 1-[4-(16-bromohexadecylamino)benzoyl]-pyrrolidine

A solution of 6.0 g. of 4-[N-carbobenzyloxy-N-(16-bromohexadecyl)amino]benzoyl chloride and 1.2 g. triethylamine in 100 ml. warm ether is treated with 1.1 g. of pyrrolidine. After 1 hour at reflux, the precipitate is filtered off and washed with warm ether. After evaporation to dryness, the intermediate is dissolved in 50 ml. 30% hydrobromic/acetic acid and warmed at 50° for 2 hours. The solvents are evaporated and the product is partitioned between methylene chloride and water. The layers are separated and the methylene chloride is evaporated. The residue is crystallized from acetone to yield colorless crystals

EXAMPLE 734

Preparation of 4-(16-bromohexadecylamino)-N-(2,3-dihydroxypropyl)benzamide

To a mixture containing 4.3 g. of 1-{4-[N-(*t*-butyloxycarbonyl)-N-(16-bromohexadecyl)amino]benzoyl} imidazole, 50 ml. of chloroform, and 50 ml. of 5N sodium hydroxide is added 1.1 g. of 3-amino-1,2-propanediol. The solution is vigorously stirred for 24 hours, the layers are separated, and the chloroform solution is washed once with 50 ml. of 1N sodium hydroxide. The solid is evaporated and the residue is heated for 30 minutes at 40°C. in 50 ml. of anhydrous trifluoroacetic acid. The solvent is again evaporated and the oil is crystallized from acetone to yield the product as a light yellow crystalline solid.

TABLE VII

The benzoic acid derivatives required for the preparation of the benzamides of the table are prepared from the corresponding acids by the methods of Examples 669-672. The requisite carboxylic acids are prepared by the methods of Examples 619-629 or 762-775. The benzamides of the table are prepared from these carboxylic acids or derivatives thereof by the methods of Examples 726-734.

| Example No. | Method of Example | Product |
|---|---|---|
| 735 | 726 | 1-[4-(11-Chloroundecylamino)benzoyl]morpholine |
| 736 | 726 | 1-[4-(11-Benzylsulfonylundec-2-enylamino)benzoyl]pyrrolidine |
| 737 | 726 | 1-[4-(2-Iodotetradecylamino)benzoyl]piperidine |
| 738 | 726 | N,N-Diethyl 4-[(3-methylthio-2,-2,4-trimethylcyclopentyl)methylamino]benzamide |
| 739 | 727 | Ethyl 4-(13-acetamido-1-ethyltridec-2-ynlamino)hippurate |
| 740 | 727 | Ethyl 4-[(3-chloro-2,6,6-trimethylcyclohexylmethyl)amino]-hippurate |
| 741 | 727 | Ethyl 4-(7-phenylsulfonyl-3-methylheptylamino)hippurate |
| 742 | 728 | N-{4-[10-Methylsulfonyl-2-(2-butenyl)decylamino]benzoyl} glycine |
| 743 | 728 | N-[4-(8-Benzamidooctylamino)benzoyl]glycine |

| | | |
|---|---|---|
| 744 | 729 | N-[4-(11-Chloroundecylamino)benzoyl]methanesulfonamide |
| 745 | 729 | N-[4-(2-Methylsulfinyltetradecylamino)benzoyl]benzamide |
| 746 | 729 | N-[4-(16-Methanesulfonamidohexadec-4-ynylamino)benzoyl]benzenesulfonamide |
| 747 | 730 | 4-[11-(p-Toluenesulfonamido)undec-2-enylamino]benzoylhydroxamic acid |
| 748 | 730 | 4-[10-(Chlorodecylamino)benzoyl] hydroxamic acid |
| 749 | 731 | 3-{4-[7-(p-Chlorophenylsulfonyl)3,7,7-trimethylhept-2-enylamino]benzoyl}-4-carboxythiazolidine |
| 750 | 731 | 3-{4-[10-Azido-1-(n-octyl)-decylamino]benzoyl}-4-carboxythiazolidine |
| 751 | 732 | N-[4-(16-Iodohexadecylamino)benzoyl]alanine |
| 752 | 732 | N- 4-[(16-Methylsulfinyl)hexadec-4-ynylamino]benzoyl glycine |
| 753 | 733 | 4-(8-Phenylthio-2,7-dimethyloctylamino)benzhydrazide |
| 754 | 733 | 1-[4-(16-Methylthiohexadecylamino)benzoyl]morpholine |
| 755 | 734 | N-[4-(11-Ethylsulfonylundec-2-enylamino)benzoyl]piperidine |
| 756 | 734 | N-[4-(16-Chlorohexadec-7-enylamino)benzoyl]morpholine |
| 757 | 734 | 4-[8-(p-Chlorophenylsulfonyl)-2,7-dimethyloctylamino]-N-(2,3-dihydroxypropyl)benzamide |
| 758 | 734 | 4-[10-Methylsulfonyl-2-(n-hexyl)dec-4-ynylamino]-N-(2,3-dihydroxypropyl)benzamide |
| 759 | 728 | N-{4-[11-(4-Chlorobenzamido)undecylamino]benzoyl} alanine |
| 760 | 731 | 3-{4-[11-(Methylsulfonyl)undec-2-enylamino]benzoyl}-4-carboxythiazolidine |

| Example No. | Method of Example | Product |
|---|---|---|
| 761 | 732 | N-[4-(11-Chloroundec-2-enylamino]benzoyl]glycine |

EXAMPLE 762

Preparation of 4-(16-bromohexadecylamino)benzoic acid

A mixture of 16 g. of 4-(16-bromohexadecylamino)-benzoic acid and 150 g. of 30-32% hydrogen bromide in acetic acid is treated with 30 ml. of concentrated sulfuric acid, stirred under reflux for 8 hours, poured into ice-water, and filtered. The product is washed with water, dried and recrystallized from ether-petroleum ether to yield 4-(16-bromohexadecylamino)benzoic acid, as a white, crystalline solid.

EXAMPLE 763

Preparation of Ethyl 4-(16-methanesulfonyloxyhexadecylamino)benzoate

A solution of 14 g. of ethyl 4-(16-hydroxyhexadecylamino)benzoate and 14 ml. of triethylamine in 320 ml. of methylene chloride at -10°C. is added to 6.0 ml. of methanesulfonyl chloride, dropwise. After 30 minutes at -10°C., the mixture is diluted with methylene chloride, extracted with ice-water and cold dilute hydrochloric acid, and dried over magnesium sulfate. Evaporation of the solution affords the methanesulfonate ester as an oil.

EXAMPLE 764

Preparation of 4-(11-Mercaptoundecylamino)benzoic acid

A solution of 10 g. of ethyl 4-[(11-bromoundecyl)amino]benzoate and 2.0 g. of thiourea in 80ml of 95% ethanol is stirred under reflux for 3 hours, allowed to cool, treated with 12 ml. of 10N aqueous sodium hydroxide solution and again stirred under reflux for 2 hours. The mixture is acidified with dilute hydrochloric acid and filtered. The solid is recrysallized from ethanol-water to yield the product as a white solid.

EXAMPLE 765

Preparation of Ethyl 4-(6-acetylthiohexadecylamino)benzoate

A mixture of 7.6 g. ethyl 4-(6-methanesulfonyloxyhexadecylamino)benzoate and 5.5 g. of potassium thioacetate in 200 ml of acetone is stirred under reflux for 3

hours and evaporated. The residue is partitioned between methylene chloride and water and the organic layer is separated, dried and evaporated. Recrystallization of the residual solid from hexane affords the product as a light, tan solid. Hydrolysis of the product by the method of Example 624 affords 4-(6-mercaptohexadecylamino)benzoic acid

EXAMPLE 766

Preparation of Ethyl 4-(11-benzylthioundecylamino)benzoate

A mixture of 8.0 g. of ethyl 4-(11-bromoundecylamino)benzoate, 4.0 g. of sodium methoxide and 5.3 g. of benzylthiol, and 100 ml. of methanol is stirred under reflux for 16 hours, cooled, diluted with 5% aqueous hydrochloric acid, and filtered. Recrystallization from ether-petroleum ether affords the product as a white solid. The procedure of Example 624 is used to convert the ester to 4-(11-benzylthioundecylamino)benzoic acid.

EXAMPLE 767

Preparation of ethyl 4-(6-aziodohexadecylamino)benzoate

A solution of 6.4 g. of ethyl 4-(6-methanesulfonyloxyhexadecylamino)benzoate and 1.9 g. of sodium azide in 65 ml. of dimethylformamide is stirred at ambient temperature for 16 hours, poured into 200 ml. of water, and extrated with chloroform. The organic extract is washed with saturated sodium chloride solution, dried over sodium sulfate, and evaporated. Crystallization of the residue from hexane yields the product. The ester is hydrolyzed to 4-(6-azidohexadecylamino)benzoic acid using the procedure of Example 624.

EXAMPLE 768

Preparation of ethyl 4-(6-chlorohexadecylamino)benzoate

A solution of 10 g. of ethyl 4-(6-hydroxyhexadecylamino)benzoate and 2.4 ml. of pyridine in 50 ml. of chloroform is treated with 2.0 ml. of thionyl chloride, stirred under reflux for 1 hour, washed with 1N hydrochloric acid and saturated aqueous sodium bicarbonate, dried over magnesium sulfate, and evaporated. The residue is crystallized from aqueous ethanol to yield the product as a white solid.

EXAMPLE 769

Preparation of ethyl 4-[11-(n-butylsulfinyl)undecylamino]-benzoic acid

A solution of 5.2 g. of 4-[11-(n-butylthio)undecylamino]benzoic acid in 35 ml. of acetone and 20 ml. of tetrahydrofuran is treated with a solution of 3.7 g. of sodium metaperiodate in 3.5 ml. of water and 20 ml. of tetrahydrofuran and the mixture is stirred under reflux for 2 hours and then filtered. The filtrate is extracted with saturated aqueous sodium chloride solution, dried over magnesium sulfate, and evaporated. Crystallization from acetone-chloroform affords the product as a white solid.

EXAMPLE 770

Preparation of 4-[11-(n-butylsulfonyl)undecylamino]-benzoic acid

A mixture of 3.8 g. of 4-(11-(n-butylthio)undecylamino]benzoic acid and 11 g. of sodium metaperiodate in 50 ml. of water and 200 ml. of methanol is stirred under reflux for 24 hours and filtered while hot. The filtrate was allowed to cool and then extracted with methylene chloride. The extract is dried over magnesium sulfate and evaporated to yield a solid. Recrystallization from acetone-chloroform affords the product as a white solid.

EXAMPLE 771

Preparation of ethyl 4-(3-oxotetradecylamino)benzoate

A solution of 1.8 g. of ethyl 4-(3-hydroxytetradecylamino)benzoate in 10 ml. of methylene chloride is added slowly to a stirred solution of 3.0 g. of chromium trioxide and 5.0 ml. of pyridine in 150 ml. of methylene chloride at ambient temperature and the mixture is stirred for 15 minutes. The supernatant liquid is decanted, washed with 1N sodium hydroxide solution and 1N hydrochloric acid, dried over magnesium sulfate, and evaporated. Crystallization of the residue from ether-petroleum ether yields the product as a white solid. Oxidation of the corresponding benzoic acid

by this procedure affords 4-(3-oxotetradecylamino)benzoic acid.

EXAMPLE 772

Preparation of ethyl 4-(6-hydrohexadecylamino)benzoate

A suspension of 10 g. of ethyl 4-(6-oxohexadecylamino)benzoate and 2.8 g. of sodium borohydride in 250 ml. of ethanol is stirred at ambient temperature for 3 hours, acidified with glacial acetic acid, and poured into water. The solid is collected by filtration, dried, and recrystallized from hexane to yield the product as a white solid. Alkaline hydrolysis of this ester by the method of Example 624 affords 4-(6-hydroxyhexadecylamino)benzoic acid.

EXAMPLE 773

Preparation of 4-(6-oximinohexadecylamino)benzoic acid

A mixture of 1.0 g. of ethyl 4-(6-oxohexadecylamino)benzoate, 207 mg. of hydroxylamine hydrochloride, 244 mg. of sodium acetate and 20 ml. of 95% ethanol is stirred under reflux for 3 hours and filtered. The filtrate is evaporated and a methylene chloride solution of the residue is washed with water, dried over magnesium sulfate, and evaporated to yield ethyl 4-(6-oximinohexadecylamino)benzoate as a white solid.

A solution of the ethyl 4-(6-oximinohexadecylamino)-benzoate, 1.o g. of potassium hydroxide and 15 ml. of ethanol in 15 ml. of water is stirred under reflux for 2 hours, evaporated, and then treated with 15 ml. of water and 2.3 ml. of concentrated hydrochloric acid. The mixture is extracted with methylene chloride and the dried extract is evaporated. Crystallization from ethanol-water affords the product as a white solid.

EXAMPLE 774

Preparation of ethyl 4-(11-anilinoundecylamino)benzoate

A solution of 6.0 g. of ethyl 4-(11-bromoundecylamino)benzoate and 3.0 g. of aniline in 15. ml. of hexamethylphosphoramide is stirred at 115°C for 18 hours, cooled, diluted with water and filtered. Recrystallization of the solid from ethanol yields the product as an off-white solid.

The ester is hydrolyzed to 4-(11-anilinoundecylamino)benzoic acid by the method of Example 624.

## EXAMPLE 775

Preparation of ethyl 4-(11-benzenesulfonamidoundecylamino)-benzoate

A solution of 6.0 g. of ethyl 4-(11-bromoundecylamino)benzoate, 5.0 g. of benzenesulfonamide, and 2.0 g of sodium hydride (50% suspension in mineral oil) in 25 ml. of hexamethylphosphoramide is stirred at 155° for 18 hours, cooled, diluted cautiously with water, and filtered. The solid is recrystallized from ethanol to yield the product as a white solid. This ester is converted to 4-(11-benzenesulfonamidoundecylamino)benzoic acid by the method of Example 624.

## EXAMPLE 776

Preparation of 4-(11-bromoundecylamino)benzoic acid

Anhydrous hydrogen bromide is passed rapidly into a solution of 5 g. of 4-(10-undecenylamino)benzoic acid and 1 g. of dibenzoyl peroxide in 300 ml. of 1,2-dimethoxyethane for 1 hour. Partial evaporation of the solvent affords a crystalline solid which is separated by filtration and recrystallized from ether-petroleum ether to yield the product as a white solid.

## TABLE VIII

Benzoic acids, esters, and amides required for the preparation of the 4-aminophenyl compounds of the Table are prepared from the appropriate starting materials by the methods of Examples 619-621, 624, 626-628, 673-683, 726-734, 762 and 763. The 4-aminophenyl compounds of the Table are prepared from these acids, esters and amides by the methods of Examples 763-774.

| Example No. | Method of Example | Product |
|---|---|---|
| 777 | 763 | 4-(16-Mercaptohexadecylamino)-benzoic acid |
| 778 | 763 | 4-(2-Mercaptotetradecylamino)-benzoic acid |

| Example No. | Method of Example | Product |
|---|---|---|
| 779 | 763 | Ethyl 4-(11-mercaptoundec-2-enylamino)benzoate |
| 780 | 764 | Ethyl 4-[10-mercapto-1-methyl-2-(2-hexyl)methylamino]benzoic acid |
| 781 | 764 | 4-[12-Mercapto-1-(n-butyl)dodec-3-enylamino]benzoic acid |
| 782 | 764 | 4-(16-(Mercaptohexadec-4-ynylamino)-benzoic acid |
| 783 | 764 | 4-[(3-Marcapto-2,6,6-trimethylcyclohexyl)methylamino]benzoic acid |
| 784 | 765 | N-[4-(16-Methylthiohexadecylamino)-benzoyl]pyrrolidine |
| 785 | 765 | 4-(16-Phenylthiohexadec-9-enyl- |
| 786 | 765 | 4-(7-Benzylthio-3-methylheptylamino)benzoic acid |
| 787 | 766 | 4-(16-Azidohexadecylamino)benzoic acid |
| 788 | 766 | 4-(14-Azidotetradecylamino)benzoic acid |
| 789 | 766 | 4-(2-Azidotetradecylamino)benzoic acid |
| 790 | 767 | Ethyl 4-[(3-chloro-2,2,4-trimethylcyclopentyl)methylamino]-benzoate |
| 791 | 767 | Ethyl 4-(16-chlorohexadec-4--ynylamino)benzoate |
| 792 | 767 | Ethyl 4-(16-Chlorohexadec-7-enylamino)benzoate |
| 793 | 768 | 4-[11-(n-Butylsulfinyl)undec-2-enylamino]benzoic acid |
| 794 | 768 | 4-(16-Methylsulfinylhexadecylamino)benzoic acid |

| Example No. | Method of Example | Product |
|---|---|---|
| 795 | 768 | 4-(8-Benzylsulfinyloctylamino)-benzoic acid |
| 796 | 768 | 4-(10-Phenylsulfinyldecylamino)-benzoic acid |
| 797 | 768 | 4-(2-Methylsulfinyltetradecyl-amino)benzoic acid |
| 798 | 769 | 4-[11-(n-Butylsulfonyl)undec-2-enyl-amino benzoic acid |
| 799 | 769 | 4-(16-Methylsulfonylhexadecyl-amino)benzoic acid |
| 800 | 769 | 4-(10-Phenylsulfonyldecylami-no)-benzoic acid |
| 801 | 770 | Ethyl 4-(3-oxododecylamino)ben-zoate |
| 802 | 770 | Ethyl 4-(3-oxodecylamino)ben-zoate |
| 803 | 771 | 4-(6-Hydroxyundecylamino)ben-zoic acid |
| 804 | 771 | 4-(3-Hydroxyundecylamino)ben-zoic acid |
| 805 | 771 | 4-(6-Hydroxytetradecylamino)ben-zoic acid |
| 806 | 771 | 4-(11-Hydroxyhexadecylamino)-benzoic acid |
| 807 | 772 | 4-(2-Oximinotetradecylamino)-benzoic acid |
| 808 | 772 | 4-(3-Oximinodecylamino)benzoic acid |
| 809 | 773 | 4-[8-(4-Chlorophenylamino)octyl-amino]benzoic acid |
| 810 | 773 | 4-[10-(p-Tolylamino)decylamino]-benzoic acid |
| 811 | 774 | 4-(16-Methanesulfonamidohexa-decylamino)benzoic acid |

| Example No. | Method of Example | Product |
|---|---|---|
| 812 | 774 | 4-(2-Methanesulfonamidotetra-decylamino)benzoic acid |
| 813 | 774 | 4-(11-Benzenesulfonamidoundec-2-enylamino)benzoic acid |
| 814 | 774 | 4-(11-Benzamidoundecylamino)-benzoic acid |
| 815 | 774 | 4-(16-Acetamidohexadec-4-ynyl-amino)benzoic acid |
| 816 | 775 | 4-(2-Acetamidotetradecylamino)-benzoic acid |

EXAMPLE 817

Preparation of 4-(4-methoxyphenyl)-1-butyne

A solution of 39.2 g. of zinc dust, 157 g. of triplhenylphosphene and 200 g. of carbon tetrabromide in 500 ml. methylene chloride is stirred at room temperature for 25 hours. To the resultant solution is added 50 g. of 4-methoxyhydrocinnamaldehyde and stirring is continued for an additional 2 hours. The solution is diluted with 2 l. of pentane and the precipitate is filtered. The precipitate is stirred with 100 ml. methylene chloride, diluted with 400 ml. pentene, and again filtered. The organic solutions are combined and evaporated to yield, 1,1-dibromo-4-(4-methoxyphenyl)-1-butene as a light yellow oil.

A solution of 40 g. of the yellow oil in 700 ml. dry tetrahydrofuran is cooled to -78° in a dry ice-acetone bath and treated with 220 ml. of 1.16M n-butyllithium in pentane. The solution is stirred at -78° for 1 hour, warmed, and stirred an additional hour at room temperature. The reaction is poured slowly into 1 l. water and the mixture is extracted twice with 500 ml. methylene chloride. The solution is dried with magnesium sulfate and evaporated and the residue is distilled to yield the product as a colorless crystalline mass.

EXAMPLE 818

Preparation of 16-bromohexadecanoic acid

To a stirred solution of 102 gm. 16-hydroxyhexadecanoic acid in 720 ml. 32% hydrobromic acid in acetic acid is slowly added 180 ml. sulfuric acid. After stirring at room temperature overnight the solution is refluxed for 3 hours. An additional 180 ml. solvent is added and reflux is resumed for an additional 4 hours. The warm solution is slowly poured into 2 l. of stirred ice-water, the precipitate is collected and thoroughly washed with water. Crystallization from acetonitrile yields 16-bromohexadecanoic acid as white crystals.

EXAMPLE 819

Preparation of 11-bromoundecanol

A 250 ml. solution of 1M borane in tetrahydrofuran is cooled in an ice-water bath, and to this is slowly added 50 g. 11-bromoundecanoic acid in 150 ml. dry tetrahydrofuran. After stirring at room temperature for 19 hours the reaction is quenched by the addition of 300 ml. 10% hydrochloric acid. The aqueous solution is extracted three times with 150 ml. portions of ether, the organic layers are combined, washed with saturated brine, dried with anhydrous magnesium sulfate and evaporated. Distillation of the residue yields 11-bromoundecanol as a colorless liquid.

EXAMPLE 820

Preparation of 16-bromo-1-(p-toluenesulfonyloxy)undecane

A solution of 30 g. 11-bromoundecyl alcohol and 25.0 g. 4-toluenesulfonyl chloride in 250 ml. dry ether is cooled with stirring in an ice-water bath. To this is added dropwise 13.2 triethylamine in 50 ml. ether. After addition, the solution is stirred in the cold for 1 hour and filtered. The ether is washed with 100 ml. 10% hydrochloric acid solution, 100 ml. brine, dried and evaporated to yield the title compound as an oily solid.

EXAMPLE 821

Preparation of 13-(4-chlorophenyl)-12-tridecynyl bromide

To a stirred solution of 200 ml. 1M ethyl magnesium bromide in ether under a stream of argon is added dropwise a solution of 25.9 g. 4-chlorophenylacetylene in 100 ml. ether. The solution is stirred at reflux for 3 hours (until gas evolution ceases) and most of the ether is distilled. Toluene (500 ml.) is added and distillation of ether is continued until the reaction temperature reaches 80°C. Heating is discontinued and a solution of 77 g. 11-bromo-1-(p-toluenesulfonyloxy)undecane in 50 ml. toluene is slowly added. The solution is stirred at 80° for 16 hours, cooled, and quenched by the addition of 200 ml. 50% acetic acid. The layers are separated and the organic layer

is washed with 200 ml. saturated bicarbonate solution, 200 ml. water, dried and evaporated. Distillation of the residue yields the title compound as a colorless liquid.

EXAMPLE 822

Preparation of ethyl 4-[13-(4-chlorophenyl)-12-tridecynyl-amino]benzoate

A solution of 20 g. 13-(4-chlorophenyl)-12-tri-decynyl bromide and 17.8 g. ethyl 4-aminobenzoate in 75 ml. hexamethylphosphoramide is stirred at 115° C. for 17 hours. The cooled solution is diluted with 150 ml. water, the precipitate is filtered, washed in portions with 250 ml. 50% ethanol and dried to a tan solid. The product is dissolved in 200 ml.
warm methylene chloride, passed through a pad of magnesol, and the solution is evaporated. Crystallization from 300 ml. acetonitrile yields colorless crystals of the title compound.

EXAMPLE 823

Preparation of 4-[13-(4-chlorophenyl)-12-tridecynylamino]-benzoic acid

A 4 g. sample of ethyl 4-[13-(4-chlorophenyl)-12-tridecylamino]benzoate is hydrolyzed with 1.6 g. 85% potassium hydroxide in 60 ml. 95% ethanol by refluxing the solution for 5 hours. The solution is cooled, diluted with 100 ml. water and acidified to pH 4.5 with 37% hydrochloric acid. The precipitate is collected, dried *in vacuo* and crystallized from acetone to yield the title compound as white powder.

EXAMPLE 824

Preparation of 4-(4-fluorophenyl)-3-butyn-1-ol

A solution of ethylmagnesium bromide is prepared by reacting 68 g. ethyl bromide with 16.2 g. magnesium ribbon in 200 ml. dry ether. After preparation of the Grignard reagent is completed, 200 ml. dry toluene is added and the ether is distilled, the solution is cooled and 60 g. 4-fluorophenyl acetylene in 30 ml. toluene is added dropwise at a rate such that the reaction temperature does not exceed 10°C. After addition is completed, the solution is warmed

at 90°C. for 5 hours. The reaction is again cooled in an ice bath and 31 g. ethylene oxide is passed into the stirred gray solution and stirring is continued at ambient temperature for 18 hours. After 4 hours of reflux, most of the toluene is distilled. The cooled solution is treated with 300 ml. water and 300 ml. 20% sulfuric acid, extracted twice with 500 ml. portions methylene chloride, and the combined organic layers are washed with 200 ml. saturated bicarbonate solution. After drying and evaporation, the residue is distilled to yield the title compound as a colorless liquid.

## EXAMPLE 825

### Preparation of 4-(4-fluorophenyl)-1-metanesulfonyoxy-3-butyne

To a mixture of 250 ml. of dichloromethane, 25 g. 4-(4-fluorophenyl)-3-butyn-1-ol and 16.7 g. of triethylamine cooled in an ice-salt bath at -10°C. is added dropwise, over 15 minutes, 18.9 g. of methanesulfonyl chloride. The mixture is cooled at -10°C. to -15°C. for 30 minutes and then washed with 300 ml. each of cold water, 10% hydrochloric acid, sodium carbonate solution and with saturated sodium chloride solution. The organic layer is dried with magnesium sulfate and conentrated *in vacuo* to give a pale yellow oil.

## EXAMPLE 826

### Preparation of ethyl 4-[4-(4-fluorophenyl)-3-butynylamino]-benzoate

A solution of 10 g. 4-(4-fluorophenyl)-1-methanesulfonyloxy-3-butyne and 13.6 g. ethyl 4-aminobenzoate in 50 ml. hexamethylphosphoramide is heated at 120°C. for 18 hours. The partially-cooled (about 50°) solution is diluted with 100 ml. water and the resultant precipitate is collected. After washing in portions with 200 ml. 50% ethanol, the tan product is dried *in* vacuo and crystallized three times from ethanol to yield the title compound as colorless crystals.

EXAMPLE 827

Preparation of ethyl 4-[3-(4-chlorophenyl)prop-2-enylamino]-phenylacetate

To a cold (-20°) stirred solution of 10 8 g.4-chlorocinnamyl alcohol prepared by lithium aluminum hydride reduction of methyl 4-chlorocinnamate and 13.4 ml. triethylamine in 300 ml. ether is added dropwise 5 6 ml. methanesulfonyl chloride in 5 ml of ether After addition is completed the solution is warmed to room temperature, stirred for 30 minutes and filtered directly into a solution of 23 1 g ethyl 4-aminobenzoate in 100 ml. ether After 17 hours at room temperature, the precipitate is filtered and washed with several portions of methylene chloride The organic solution is washed twice with 100 ml. water 100 ml. brine dried and evaporated. The tan residue is crystallized from ethanol and from acetonitrile to yield the title compound as white crystals.

EXAMPLE 828

Preparation of 5-octyl-2-thiophenecarboxaldehyde

To a stirred solution of 80.1 g of 2-octylthiophene in 70 ml diemethylformamide is added dropwise 42.4 ml of phosphorous oxychloride After addition the solution is warmed to 100°C. for 1 hour, then poured into 600 ml ice. After neutralization with sodium acetate the resulting oil is extracted with ether The ether is washed with water and aqueous potassium carbonate dried, and distilled in vacuo to give the title compound as an oil.

EXAMPLE 829

Preparation of ethyl 4-[2-(5-octylthienyl)methylamino]-hydrocinnamate

A solution of 8.6 ethyl 4-aminohydrocinnamate 9 77 g. 5-octyl-2-thiophenecarboxaldehyde and a few crystals of 2.4-dinitrobenzenesulfonic acid in 250 ml. toluene is refluxed under a Dean-Stark trap for 17 hours whereupon the theoretical amount (0.8 ml.) water has been collected. The toluene is evaporated to yield ethyl 3-[4-(5-octyl-2-thienylideneamino)phenyl]propionate as a crystalline mass.

To a mixture of 17 8 g of the above compound in 250 ml ethanol is added 1 68 g sodium borohydride and the mixture is stirred at room temperature for 18 hours Excess reagent is decomposed by addition of 10 ml. acetic acid The solution is concentrated *in vacuo* and the residue is partitioned betweed toluene and aqueous potassium carbonate After drying the toluene is evaporated to yield a solid Crystallization from acetonitrile and from ethanol affords the title compound as white crystals

EXAMPLE 830

Preparation of ethyl 4-aminophenylpropiolate

A sample of 50 g. of ethyl p-aminocinnamate is dissolved in 500 ml. of ethyl ether and a solution of 28 g. of acetic anhydride in 30 ml of ether is added dropwise. When the addition is complete the reaction is allowed to stir for another hour The mixture is then diluted with hexane and filtered providing ethyl p-acetamidocinnamate

A solution of 40 g. of ethyl p-acetamidocinnamate in 200 ml. of carbon tetrachloride is cooled in ice. Bromine (28 g.) is added dropwise, the reaction is allowed to stir for one additional hour and then the solvent is evaporated. The crystalline residue is the dibromo ester

A solution of 11.4 g of potassium hydroxide in 300 ml. of 95% ethanol is cooled to 40°C and 20 g. of the crude dibromo ester above is added After 30 minutes the reaction is heated to reflux for five hours. The solution is then cooled and filtered. The filtrate is treated with acetic acid until the solvent is neutral to litmus then concentrated, chilled and filtered to yield ethyl 4-aminophenylpropiolate.

EXAMPLE 831

Preparation of ethyl 5-octylthiophene-2-acrylate

A solution of 75 4 g. 5-octyl-2-thiophenecarboxaldehyde and 117 6 g. ethyl (triphenylphosphoranylidene)acetate in 400 ml. tetrahydrofuran is refluxed for 20 hours. The solution is concentrated, the semi-solid residue is slurried with ether and filtered. Concentration followed by

distillation of the resultant oil yields ethyl 5-octylthiophene-2-acrylate as an oil

EXAMPLE 832

Preparation of (10-carboxydecyl)triphenylphosphonium bromide

A solution of 81 4 g. 11-bromoundecanoic acid and 85.0 g triphenylphosphine in 600 ml acetonitrile is refluxed for 64 hours then concentrated *in vacuo*. The resultant oil is dissolved in 200 ml ether, whereupon crystals of the title compound form.

EXAMPLE 833

Preparation of 12-(2-thienyl)-11-dodecenol

To a stirred slurry of 18 1 g. lithium aluminum hydride in 460 ml. ether is added dropwise a solution of 61 3 g. 12-(2-thienyl)-11-dodecenoic acid in 140 ml ether at a rate such that gentle reflux is maintained After four additional hours at reflux. the solution is cooled in ice and treated sequentially with 18 1 ml. water 18.1 ml 15% sodium hydroxide, solution and 54.3 ml water. The precipitate is filtered and washed several times with ether The combined ether fractions are washed with brine, dried and evaporated. Distillation of the residue yields 12-(2-thienyl)-11-dodecenol as a semi-crystalline mass.

EXAMPLE 834

Preparation of 6-(2-furyl)-5-hexenoic acid

To a cooled solution of dimsyl sodium (prepared by heating 4.35 g. of 50% oil dispersion of sodium hydride in 50 ml dimethylsulfoxide at 60° for 2 hours under argon) is added dropwise 20.0 g. of (4-carboxybutyl)triphenylphosphonium bromide in 90 ml. dimethylsulfoxide. After the solution has stirred at room temperature for one hour a solution of 4.34 g. furfural in 20 ml. dimethylsulfoxide is added dropwise to the cool (15°C) solution over 20 minutes. After 2 hours the dimethylsulfoxide is evaporated the residue is dissolved in 100 ml water filtered and extracted four times with 150 ml. portions of ethyl acetate. The aqueous solution is acidified to pH 1 with 1N hydrochloric acid, saturated with sodium chloride and extracted twice

 

with 150 ml. portions of ethyl ether. The ether is dried and evaporated to yield the title compound as a yellow oil

EXAMPLE 835

Preparation of 6-(2-furyl)hexanoic acid

Nine grams 6-(2-furyl)-5-hexenoic acid is dissolved in 50 ml. acetic acid 1.8 g. 10% palladium-on-charcoal is added, and the solution is hydrogenated at 40 p s.i. for 16 hours. The catalyst is filtered the solvent evaporated, and the residue is dissolved in 200 ml. ether. The solution is washed twice with 25 ml. portions of saturated sodium bicarbonate dried, and evaporated to a light yellow oil.

In some cases when reduction is incomplete under the above conditions, fresh catalyst is added and hydrogenation is continued. For reduction of substrates containing a sulfur moiety, an equal weight of palladium-on-barium sulfate is used as a catalyst.

EXAMPLE 836

Preparation of ethyl 4-[11-(1-imidazolyl)undecylamino]-phenylacetate

To a stirred mixture of 4.84 g of hexane-washed 50% sodium hydride dispersion in 70 ml hexamethylphosphoramide is added a solution of 7.23 g. imidazole in 20 ml. hexamethylphosphoramide and the mixture is stirred at room temperature for 2 hours. A solution of 20.7 g. ethyl 4-(11bromoundecylamino)phenylacetate in 20 ml. hexamethylphosphoramide is then added and the reaction mixture is stirred for 64 hours at ambient temperature the solution is poured into 1.5 l. water and extracted twice with 1.5 l. portions of ethyl acetate. The organic layers are combined, washed twice with water dried with magnesium sulfate and evaporated. The product is purified by crystallization from acetonitrile.

EXAMPLE 837

Preparation of 2-undecyl-4-hydroxymethylimidazole

A solution of 100 g. undecylcyanide in 100 ml dry

ethanol and 400 ml. ether is cooled in an ice bath and 60 g. hydrogen chloride is bubbled in. After 16 hours at 0°C. the solvents are evaporated and 1 l. ether is added. The resultant white precipitate is collected and placed into a bomb reactor The bomb is charged with 46 9 g. dihydroxyacetone and 500 ml liquid ammonia. After heating at 60° for 5 hours, the ammonia is evaporated and 500 ml. saturated aqueous potassium carbonate is added The precipitate is collected and crystallized from acetonitrile-methanol and from ethanol to yield the title compound as white crystals.

EXAMPLE 838

Preparation of 2-undecyl-4-chloromethylimidazole hydrochloride

Into a solution of 2.5 g. 2-undecyl-4-hydroxymethylimidazole in 25 ml. ethanol is bubbled hydrogen chloride gas to saturation. The ethanol is evaporated and the residue is re-dissolved in 25 ml. toluene, 2 ml. thionyl chloride is added and the solution is refluxed for 2 hours. Evaporation yields the title compound as an oil.

EXAMPLE 839

Preparation of ethyl 4-[6-(4-chlorophenyl)hexa-5-enylamino]-phenylacetate

To a solution of 10.0 g ethyl 4-[6-(4-chlorophenyl)hexa-5-ynylamino]phenylacetate in 75 ml. acetone is added 1.0 ml. quinoline and 0.2 g. of palladium-on-calcium carbonate. The solution is hydrogenated at slightly above atmospheric pressure until 600 ml. hydrogen has been absorbed. The catylyst is filtered and washed with several small portions of acetone. The organic solution is diluted with 200 ml. water, the precipitate is collected, dried, and recrystallized from acetonitrile to yield the title compound as white crystals.

EXAMPLE 840

Preparation of 4-chlorocinnamyl alcohol

A solution of 30 g. methyl 4-chlorocinnamate in 300 ml. dry ether is cooled to -20°C in a dry ice-acetone bath, then with stirring 3.3 g. lithium aluminum hydride

is added in portions at a rate such that the reaction temperature does not exceed -10°C. After addition is complete, the reaction is allowed to warm slowly and stirring is continued for 18 hours at room temperature. The solution is treated in sequence with 3.3 ml. water, 3.3 ml. 15% aqueous sodium hydroxide and 9.9 ml. water. After stirring for one hour the yellow precipitate is filtered and washed with several small portions of ether Evaporation of solvent yields the product as a light yellow oil which on distillation affords 4-chlorocinnamyl alcohol as a white solid.

EXAMPLE 841

Preparation of 4-(4-methoxyphenyl)-1-butanol

A flask containing 250 ml. 1M diborane in tetrahydrofuran is thoroughly cooled in an ice-water bath. To the stirred solution is slowly added 38 8 g. of 4-(4-methoxyphenyl)butyric acid in 100 ml. tetrahydrofuran. After addition is completed the reaction is stirred for 16 hours at room temperature, then poured slowly into 1 l. stirred ice-water. The aqueous solution is extracted three times with 300 ml. portions of ether. The combined organic extracts are washed with water and brine, dried, and evaporated to a nearly colorless oil of 4-(4-methoxyphenyl)-1-butanol.

EXAMPLE 842

Preparation of 13-(4-methylphenyl)tridecyl bromide

A solution of 3-(4-methylphenyl)propylmagnesium bromide is prepared by reacting 20°g. 3-(4-methylphenyl)propyl bromide with 2.5 g. of magnesium turnings in 50 ml. dry tetrahydrofuran. The solution of Grignard reagent is added dropwise to a stirred, cold (-10°C.) solution of 30 g. of 1,10-dibromodecane and 0.2 g. of lithium tetrachlorocuprate in 75 ml. dry tetrahydrofuran at a rate such that the reaction temperature does not exceed -5°C. After one addition hour of stirring at -10°C. the solvent is evaporated and the resultant liquid is fractioned in vacuo to yield 13-(4-methylphenyl)tridecyl bromide as a colorless liquid.

EXAMPLE 843

Preparation of ethyl 3-{4-[7-(3-bromophenyl)hepta-6-enylamino]phenyl}propionate

A mixture of 5.0 g. of ethyl 4-aminohydrocinnamate 10.0 g. of 7-(3-bromophenyl)-1-methanesulfonyloxy-6-heptene (prepared by the method of Example 825), 4.2 g. of anhydrous powdered potassium carbonate and 40 ml. hexamethylphosphoramide is heated to 80°C for 17 hours. The mixture is then cooled, diluted with water and extracted with ethyl ether. The ether extracts are washed with water, dried and evaporated. The residue is recrystallized from ethanol yielding the title compound as white crystals.

EXAMPLE 844

Preparation of ethyl 4-[13-(4-chlorophenyl)tridecylamino]-cinnamate

A mixture of ethyl p-aminocinnamate, 5.9 g. 13-(4-chlorophenyl)tridecyl bromide and one equivalent of anhydrous powdered potassium carbonate in 50 ml. hexamethylphosphoramide is heated for 20 hours at 60°C. The mixture is then cooled, diluted with water and extracted with ether. The combined ether extracts are dried, filtered and evaporated. Crystallization from acetonitrile provides the title compound as white crystals.

EXAMPLE 845

Preparation of 4-[10-(2-thienyl)deca-9-enylamino]acetophenone

p-Aminoacetophenone is heated with 5 g. 10-(2-thienyl)-1-methanesulfonyloxy-9-decene (prepared by the method of Example 825) in 50 ml. hexamethylphosphoramide containing anhydrous potassium carbonate (1.9 g.) for 16 hours a 100°C. The solution is cooled to room temperature, filtered to remove solids, and the filtrate is diluted with cold water (50 ml.). The amber solid so obtained is collected and washed with water Recrystallization from ethanol followed by dichloromethane provides the title compound.

EXAMPLE 846

Preparation of 4-[10-(4-methoxyphenyl)decylamino]benzonitrile

4-Aminobenzonitrile (11.8g.) and 10-(4-methoxyphenyl) decylbromide (16.3 g.) are dissolved in hexamethylphosphoramide (100 ml.) and heated under nitrogen in an oil bath maintained at 120°C. for 22 hours. The reaction mixture is cooled to room temperature and water (100 ml.) is added gradually. The mixture is then chilled in an ice bath. The precipitate separated is filtered, washed thoroughly with water, and dried. It is then washed repeatedly with hexane and dried. Recystallization from ether-hexane affords 4-[10-(4-methoxyphenyl)decylamino]benzonitrile as pale yellow crystals.

EXAMPLE 847

Preparation of 4-[10-(4-methoxyphenyl)decylamino]benzaldehyde

Di-isobutylaluminum hydride (54 ml. 25% solution in toluene) is added with stirring to a solution of 12.1 g. of 4-[10-(4-methoxyphenyl)decylamino]benzonitrile under a nitrogen atmosphere After addition is completed, the solution stirred for one hour. A solution of methanol in toluene (50 ml., 1:1) is added over 30 minutes and the mixture is poured into 500 ml. vigorously stirred ice-cold 50% aqueous sulfuric acid. The mixture is filtered and the organic layer separated. The aqueous solution is extracted twice with toluene (100 ml.) and the combined organic layers are washed with aqueous sodium bicarbonate, dried over magnesium sulfate, decolorized with charcoal, filtered and evaporated in vacuo to give a light yellow crystalline solid. The product is recrystallized from dichloromethane/hexane giving colorless needles.

EXAMPLE 848

Preparation of sodium 4-[13-(4-chlorophenyl)tridec-12-ynylamino]benzoate

A mixture of 3.62 g. of 4-[13-(4-chlorophenyl)tridec-12-ynylamino]benzoic acid (Example 823) and 25 ml. of

ethanol water (9:1) containing 0.400 g. of sodium hydroxide is stirred for 4 hours. The mixture is filtered and the residue washed with 10 ml. of ethanol-water (9:1) and dried *in vacuo* for 24 hours to yield sodium 4-[13-(4-chlorophenyl)tridec-12-ynylamino]benzoate as a white solid.

## EXAMPLE 849

Preparation of 4-[13-(4-chlorophenyl)tridec-12-ynylamino benzoyl]chloride

A cold solution of 25 g. 4-[13-(4-chlorophenyl)tridec-12-ynylamino]benzoic acid in 500 ml. dimethoxyethane= methylene chloride (4:1) is prepared and dry hydrochloric acid is bubbled through the solution until no more precipi= tate forms. The solution is treated with 25 ml. thionyl chloride and refluxed until all of the precipitate has dissolved. The solvents are evaporated to yield the acid chloride hydrochloride as an orange, semi-crystalline mass.

## EXAMPLE 850

Preparation of 4-[N-trifluoroacetyl-13-(4-chlorophenyl)tridec-12-ynylamino benzoyl]chloride

A stirred ice-cold suspension of 9 g. 4-[13-(4-chlorophenyl)tridec-12-ynylamino]benzoic acid in 100 ml. of dimethoxy ethane and 16 ml. of pyridine is treated with 18 ml. of trifluoroacetic anhydride at 0°C. The solution is stirred for 30 minutes at room temperature and then diluted with 300 ml. ether and 100 g. ice. After stirring vigorously for 15 minutes, the phases are separated, the ether solution is washed with brine, dried and evaporated to a white, amorphous solid.

To a solution of 9.2 g of the above solid in 30 ml. methylene chloride and 0.5 ml. dimethylformamide is added 5.7 ml. thionyl chloride. After 20 hours at reflux, the solvents are evaporated to yield 4-[N-trifluoroacetyl-13-(4-chlorophenyl)tridec-12-ynylamino]benzoyl chloride as a light yellow, mobile oil.

EXAMPLE 851

Preparation of 4-[N-carbobenzyloxy-13-(4-chlorophenyl)tridec-12-ynylamino]benzoyl chloride

To 15 g. 4-[13-(4-chlorophenyl)tridec-12-ynylamino]benzoic acid in 200 ml. warm chloroform is added a solution of 12 g. sodium carbonate in 150 ml. water. To the vigorously stirred solution is added 10 g. carbobenzyloxy chloride. After 2 hours stirring at 40°C. the layers are separated, washed three times with 1N hydrochloric acid, dried, and evaporated to an oil. The oil is dissolved in 300 ml. toluene, treated with 15 ml. thionyl chloride and the solution is refluxed for 5 hours. The solvents are evaporated and the residue is dissolved three times in toluene, evaporating each time ultimately to yield 4-[N-carbobenzyloxy-13-(chlorophenyl)tridec-12-ynylamino]benzoyl chloride as a viscous, orange oil.

EXAMPLE 852

Preparation of 1-{4[(N-*tert*-butyloxycarbonyl)-13-(4-chlorophenyl)tridec-12-ynylamino]benzoyl}imidazole

A solution of 10 g. 4-[13-(4-chlorophenyl)tridec-12-ynylamino]benzoic acid in 100 ml. dioxane is treated with 4.0 g. *tert*-butylazidoformate and 10 ml. pyridine. After stirring at room temperature for 18 hours, the protected amidoacid is precipitated from solution by the addition of 150 ml. water. The solid is collected, thoroughly dried, and dissolved in 200 ml. of a mixture consisting of methylene chloride/dimethoxyethane/pyridine (1:4:1). This solution is stirred overnight at room temperature and the solvents are evaporated to yield 1- 4-[(N-*tert*-butyloxycarbonyl)-13-(4-chlorophenyl)tridec-12-ynylamino]benzoyl imidazole as an orange oil.

EXAMPLE 853

Preparation of 2,3-dihydroxypropyl 4-[13-(4-chlorophenyl)-tridec-12-ynylamino]benzoate

A solution of 7.34 g. of 4-[13-(4-chlorophenyl)-tridec-12-ynylamino]benzoic acid, 4.80 g. of 25% aqueous sodium hydroxid, and 1.6 of iodo-1,2-propanediol in 50 ml.

of hexamethylphosphoramide is stirred for 24 hours at ambient temperature, diluted with 100 ml. of ether and stirred for 5 days at ambient temperature. The mixture is treated with water and extracted with ether. The dried extracts are evaporated to yield 2,3-dihydroxypropyl 4-[13-(4-chlorophenyl)tridec-12-ynylamino]benzoate.

EXAMPLE 854

Preparation of methyl 4- 13-(4-chlorophenyl)tridec-12-ynylamino benzoate

A solution of 20.7 g. of 4-[13-(4-chlorophenyltri-dec-12-ynylamino]benzoic acid in 25 ml. of hexamethylphosphoramide is added to a stirred mixture of 0.800 g. of sodium hydride (57% in mineral oil) and 25 ml. of hexamethylphosphoramide. The solution which forms after one hour is treated with 11.0 g. of methyl iodide and is then stirred at 25°C. for 18 hours. Dilution with water followed by filtration affords a white solid which is crystallized from ethanol to yield methyl 4-[13-(4-chlorophenyl)tridec-12-ynylamino]benzoate as a white solid.

EXAMPLE 855

Preparation of 3-hydroxypropyl 4-[13-(4-chlorophenyl)tridec-12-ynylamino]benzoate

A mixture of 2.25 g. of methyl 4-[13-(4-chlorophenyl)-tridec-12-ynylamino]benzoate, 280 mg. of 1,3-propanediol and 1.37 g. p-toluenesulfonic acid is heated at 180°C. for 18 hours and then is partitioned between ether and 3% aqueous sodium carbonate solution. The ether layer is separated, dried, and evaporated to yield 3-hydroxypropyl 4-[13-(4-chlorophenyl)tridec-12-ynylamino]benzoate.

EXAMPLE 856

Preparation of 2-ethoxyethyl 4-[13-(4-chlorophenyl)tridec-12-ynylamino]benzoate

A solution of 11.8 g. of 4-[13-(4-chlorophenyl)-tridec-12-ynylamino]benzoic acid, 1.00 g. of 2-ethoxyethanol and 5.35 ml. of boron trifluoride etherate in 200 ml. toluene is stirred under reflux for 48 hours. The solution is treated with an additional 5.35 ml. of boron trifluoride

etherate and refluxing is continued for 120 hours. Dilution with water and methylene chloride followed by filtration affords 2-ethoxy ethyl 4-[13-(4-chlorophenyl)tridec-12-ynylamino]benzoate as a white solid.

EXAMPLE 857

Preparation of methyl 4-[13-(4-chlorophenyl)tridec-12-ynylamino]benzoate

A solution of 50.5 g. of 4-[13-(4-chlorophenyl)-tridec-12-ynylamino]benzoic acid and 34.4 ml. of boron trifluoride etherate in 200 ml. of methanol is stirred under reflux for 44 hours, allowed to cool, and poured into 1.20 liters of ice-cold 5% aqueous sodium carbonate solution. The white solid is collected by filtration and recrystallized from benzene-ethanol to yield methyl 4-[13-(4-chlorophenyl)tridec-12-ynylamino benzoate.

EXAMPLE 858

Preparation of 1-(methoxycarbonyl)propyl 4-[13-(4-chlorophenyl)tridec-12-ynylamino]benzoate

To a solution of 10.0 g. 4-[13-(4-chlorophenyl)-tridec-12-ynylamino]benzoyl chloride hydrochloride in 200 ml. methylene chloride added dropwise a solution of 3 g. methyl -hydroxybutyrate and 5 g. triethylamine in 100 ml. ether. After 17 hours stirring at room temperature, the precipitate is collected and washed with several portions of ether. The ether solution is washed with water, dried and evaporated to yield 1-(methoxycarbonyl)propyl 4-[13-(4-chlorophenyl)tri dec-12-ynylamino]benzoate as a white solid.

EXAMPLE 859

Preparation of 1-(ethoxycarbonyl)ethyl 4-[13-(4-chlorophenyl)tridec-12-ynylamino]benzoate

To a warm mixture of 7 g. sodium 4-[13-(4-chlorophenyl) tridec-12-ynylamino]benzoate in 100 ml. ethanol is added 4.7 g. ethyl tosyloxypropionate. After 17 hours at reflux, the cooled solution is diluted with an equal volume of water and the resultant precipitate is filtered. After washing with cold ethanol and drying, the product is crystallized from acetonitrile to yield 1-(ethoxycarbonyl)ethyl

4-[13-(4-chlorophenyl)tridec-12-ynylamino]benzoate as colorless crystals.

EXAMPLE 860

Preparation of 1-carboxyethyl 4-[13-(4-chlorophenyl)tridec-12-ynylamino]benzoate

A flask containing 10.0 g. 4-[13-(4-chlorophenyl)-tridec-12-ynylamino]benzoic acid, 3.3 g. lactic acid, 500 mg. toluene sulfonic acid and 500 ml. toluene equipped with a Soxhlet extractor charged with activated 4Å Linde molecular sieves. The solution is refluxed for 24 hours during which time the Soxhlet extractor is charged twice more with fresh sieves. The hot solution is filtered and left to cool, whereupon 1-carboxyethyl 4-[13-(4-chlorophenyl)tridec-12-ynylamino]benzoate separates as off-white crystals.

EXAMPLE 861

Preparation of diethyl O-{4-[13-(4-chlorophenyl)tridec-12-ynylamino]benzoyl} tartrate

A mixture of 4-[N-trifluoroacetyl-13-(4-chlorophenyl)tridec-12-ynylamino benzoyl chloride and 1.2 g. triethylamine in 100 ml. warm ether is treated with 2.5 g. diethyl tartrate and refluxed for 24 hours. The hot solution is filtered, the residue is washed with hot ether, and the solution is evaporated. After treatment with aqueous methanolic potassium carbonate, the product is precipitated by acidification, filtered, and dried. Crystallization from acetone yields diethyl O-{4-[13-(4-chlorophenyl)tridec-12-ynylamino]benzoyl} tartrate as a white, crystalline solid.

EXAMPLE 862

Preparation of O-{4-[13-(4-chlorophenyl)tridec-12-ynylamino]benzoyl} malic acid

A warm solution of 4-[N-carbobenzyloxy-13-(4-chlorophenyl)tridec-12-ynylamino]benzoyl chloride and 1.3 g. triethylamine in 100 ml. ether is treated with 2 g. malic acid. An immediate precipitate forms, but the mixture is refluxed for one hour and filtered while hot. The solid is washed several times with hot ether, then the ether is evaporated to yield a white solid. The product is dissolved in

tetrahydrofuran (100 ml.) and hydrogenated over 600 mg. 10% palladium-on-carbon at 50 psi until hydrogen uptake stops. The catylist is filtered, and the solution is evaporated. The residue is crystallized from acetic acid to yield O-{4-[13-(4-chlorophenyl)tridec-12-ynylaminio]benzoyl}malic acid.

## EXAMPLE 863

Preparation of 2-(ethoxycarbonyl)vinyl 4-[13-(4-chlorophenyl)tridec-12-ynylamino]benzoate

To a mixture containing 4.3 g. 1-{4-[N-(t-butyloxycarbonyl)-13-(4-chlorophenyl)tridec-12-ynylamino]benzoyl}-imidazole, 50 ml. 5N sodium hydroxide is added 3 g. ethyl 2-formyl acetate. The solution is vigorously stirred for 24 hours. The layers are separated, and the chloroform solution is washed once with 50 ml. 1N sodium hydroxide. The solvent is evaporated and the residue is heated for 30 minutes at 40°C. in 50 ml. anhydrous trifluoracetic acid. The solvent is again evaporated and the oil is crystallized from acetone to yield light yellow crystals of 2-(ethoxycarbonyl)vinyl 4-[13-(4-chlorophenyl)tridec-12-ynylamino]benzoate.

## EXAMPLE 864

Preparation of 1-{4-[13-(4-chlorophenyl)tridec-12-ynylamino]benzoyl}piperidine

To a chilled solution of 35 ml. of piperidine, 2.5 ml. of triethylamine and 0.6 g. of dimethylaminopyridine in 100 ml. of diethyl ether is added (1/2 hour) a solution of 8.3 g. of 4-[13-(4-chlorophenyl)tridec-12-ynylamino]benzoyl chloride hydrochloride in 50 ml of ether. The solution is warmed to room temperature and maintained there for two hours. The solution is heated to reflux for an additional 2 hours at which time the reaction is complete. The solution is cooled, extracted twice with water and dried. The solvent is removed *in vacuo* and the solid is recrystallized from diethyl ether to yield 1-{4-[13-(4-chlorophenyl)tridec-12-ynylamino]benzoyl}piperidine.

EXAMPLE 865

Preparation of ethyl 4-[13-(4-chlorophenyl)tridec-12-ynylamino]hippurate

To a solution of 18.0 g. of 4-[13-(4-chlorophenyl) tridec-12-ynylamino]benzoic acid in a mixture of dioxane and methylene chloride is added gaseous hydrogen chloride for 10 minutes. The slurry is cooled and 18 ml. of thionyl chloride added. The slurry is brought to reflux for 2 hours and then concentrated under vacuum (thrice diluting with dioxane each time). The final amber solution is diluted with 100 ml. of dioxane and this solution added to freshly prepared ethyl glycinate in 300 ml. of methylene chloride containing 1 g. of dimethylaminopyridine and 10 ml. of triethylamine. After 16 hours at room temperature the reaction appeared complete but was refluxed for 2 hours, cooled and filtered. The mother liquor is extrated with water and 10% hydrochloric acid. The solution is dried and concentrated *in vacuo* to an amber liquid. A sample is pre-absorbed on silica and eluted with ether. Evaporation of the eluate yields a solid, which is recrystallized from acetonitrile to yield ethyl 4-[13-(4-chlorophenyl)tridec-12-ynylamino]hippurate as a white solid.

EXAMPLE 866

Preparation of N-{4-[13-(4-chlorophenyl)tridec-12-ynylamino]benzoyl}glycine

A mixture of 26.4 g. of ethyl N- 4-[13-(4-chlorophenyl)tridec-12-ynylamio]benzoyl glycinate, 110 ml. of 1*N* sodium hydroxide solution; and 100 ml. of ethanol is stirred at ambient temperature for 2 hours and then partially evaporated. The aqueous solution is washed with diethyl ether, acidified with 6*N* hydrochloric acid, and filtered. The white solid is dried *in vacuo* and recrystallized from acetone to yield N-{4-[13-(4-chlorophenyl)tridec-12-ynylamino]-benzoyl}glycine.

EXAMPLE 867

Preparation of 4-[13-(4-chlorophenyl)tridec-12-ynylamino]-N-(phenylsulfonyl)benzamide

A solution of 31.4 g. of benzenesulfonamide in 250 ml. of dry dimethylacetamide is added dropwise, with stirring and cooling, to a suspension of 5.5 g. of sodium hydride in 100 ml. of dry dimethylacetamide during 30 minutes at room temperature. Stirring is continued for a further 30 minutes. In the meantime, a mixture of 36.5 g. of 4-[13-(4-chlorophenyl)tridec-12-ynylamino]benzoic acid in 100 ml. of methylene chloride, 300 ml. of dimethoxyethane, and 40 ml. of thionyl chloride is refluxed for 1 hour and 15 minutes. The solution is evaporated and to the resulting oil residue is added, in one portion, the previously prepared mixture of sodium benzenesulfonamide in dimethylacetamide. The mixture is stirred for 30 minutes, without cooling, and then filtered. The filtrate is poured into 2 l. of water and 250 ml. of saturated sodium chloride solution. The product is collected by filtration and then dissolved in methylene chloride, the mixture is filtered through diatomaceous earth, and brine is added to break the emulsion. The layers are separated, the organic phase is dried and evaporated. The residue is crystallized from toluene to yield 4-[13-(4-chlorophenyl) tridec-12-ynylamino]-N-(phenylsulfonyl)-benzamide.

EXAMPLE 868

Preparation of N-{4-[13-(4-chlorophenyl)tridec-12-ynylamino]benzoyl} methanesulfonamide

A solution of 25.2 g. of 4-[13-(4-chlorophenyl)tridec- 12-ynylamino]benzoyl chloride hydrochloride and 5.6 g. of methanesulfonamide in 250 ml. of pyridine is stirred under reflux for 2 hours and then concentrated *in vacuo*. The residue is partitioned between water and diethyl ether: the aqueous layer acidified with 1N HCl, and the organic layer separated, dried and evaporated. Crystallization of the residual white solid from 60% aqueous acetic acid and then from methylene chloride-hexane affords N-{4-[13-(4-chlorophenyl)tridec-12-ynylamino benzoyl} methanesulfonamide as a white solid.

EXAMPLE 869

Preparation of N-{4-[13-(4-chlorophenyl)tridec-12-ynylamino]benzoyl}alanine

A solution of 4.75 g. of 4-[N-trifluoroacetyl-13-(4-chlorophenyl)tridec-12-ynylamino]benzoyl chloride and 1.2 g. of triethylamine in 200 ml. of warm ether is treated with 1.55 g. alanine ethyl ester and refluxed for 24 hours. The hot solution is filtered, the residue is washed with hot ether, and the solution is evaporated. After treatment with aqueous methanolic potassium carbonate, the product is precipitated by acidification, filtered, and dried. Crystallization from acetone yields the N-{4-[13-(4-chlorophenyl)tridec-12-ynylamino]benzoyl}alanine as a white, crystalline solid.

EXAMPLE 870

Preparation of N-{4-[13-(4-chlorophenyl)tridec-12-ynylamino]benzoyl}benzamide

One gram of a 50% oil dispersion of sodium hydride is washed with petroleum ether by decantation, dried, and suspended in 5 ml. of tetrahydrofuran. To this stirred mixture is added a solution of 2.42 g. of benzamide in 5 ml. of tetrahydrofuran in one portion. An initial hydrogen evolution is observed. While stirring (30 min.), the sodium hydride gradually disappears and a white, milky, turbid mixture forms. A solution of 0.9 g. of 4-[N-trifluoroacetyl-13-(4-chlorophenyl)]tridec-12-ynylamino]benzoyl chloride in 3 ml. of tetrahydrofuran is added dropwise during 5 minutes to the mixture. The whole milky mixture is stirred at room temperature under nitrogen for one hour. The mixture is then poured into water and extracted with ether. The ether extract is washed with water and brine and dried over sodium sulfate. Evaporation of the solvent affords a pale yellow solid. The solid is recrystallized from ether/acetonitrile (50/50) and then from acetonitrile to yield N-{4-[13-(4-chlorophenyl)tridec-12-ynylamino]benzoyl} benzamide

EXAMPLE 871

Preparation of N-{4-[13-(4-chlorophenyl)tridec-12-ynyl-amino]benzoyl}piperidine

To a warm solution of 4-[N-carbobenzoyloxy-13-(4-chlorophenyl)tridec-12-ynylamino]benzoyl chloride and 1.3 g. of triethylamine in 100 ml. ether is added 1.2 g. of piperidine. An immediate precipitate forms, the mixture is refluxed for one hour and then filtered. The solid is extracted several times with hot ether and the ether is evaporated to yield a white solid. The solid is dissolved in tetrahydrofuran (100 ml.) and hydrogenated over 600 mg. 10% palladiumon-carbon at 50 psi until hydrogen uptake stops. The catalyst is filtered and the filtrate evaporated. The residue is crystallized from acetic acid to yield N-{4-[13-(4-chlorophenyl)tridec-12-ynylamino]benzoyl} piperidine as a crystalline mass.

EXAMPLE 872

Preparation of N-(2,3-dihydroxypropyl)-4-[13-(4-chlorophenyl)tridec-12-ynylamino]benzamide

To a mixture containing 4.3 g. of 1- 4-(*tert*-butyloxy carbonyl)-4-[13-(4-chlorophenyl)tridec-12-ynylamino]benzoyl imidazole 50 ml. of chloroform, and 50 ml. of 5N sodium hydroxide is added 1.1 g. of 3-amino-1,2-propanediol. The solution is vigorously stirred for 24 hours, the layers are separated, and the chloroform solution is washed once with 50 ml. of 1N sodium hydroxide. The solvent is evaporated and the residue is heated for 30 minutes at 40°C. in 50 ml. of anhydrous trifluoroacetic acid. The solvent is again evaporated and the oil is crystallized from acetone to yield light yellow crystals of N-(2,3-dihydroxypropyl)-4-[13-(4-chlorophenyl) tridec-12-ynylamino]benzamide.

EXAMPLE 873

Preparation of diethyl 4-[13-(4-chlorophenyl)tridec-12-ynyl amino]benzoylmalonate

A solution of 26.6 g. of diethyl malonate and 10 ml. of 1,2-dimethoxyethane is added to a suspension of 4.0 g. of sodium hydride in 1,2-dimethoxyethane under argon.

A solution of 17.3 g. of 4-[13-(4-chlorophenyl)tridec-12-ynylamino]benzoyl chloride hydrochloride in 1,2-dimethoxyethane is then added. The reaction mixture is refluxed for 4.5 hours, cooled, poured on ice, acidified, and extracted with ether. The ether solution is washed with water and saturated sodium chloride solution, dried over anhydrous sodium sulfate and concentrated to dryness. Addition of a small amount of ethanol to the residue gives a solid which is filtered and discarded. The ethanol filtrate is concentrated and the residue is recrystallized from ether to yield diethyl 4-[13-(4-chlorophenyl)tridec12-ynylamino]benzoylmalonate.

EXAMPLE 874

Preparation of tert-butyl ethyl 4-[13-(4-chlorophenyl)tridec 12-ynylamino]benzoylmalonate

A solution of 28.0 g. of tert-butyl ethyl malonate in 10 ml. of 1,2-dimethoxyethane is added to a suspension of 4.0 g. of sodium hydride in 1,2-dimethoxyethane under argon. A solution of 17.3 g. of 4-[13-(4-chlorophenyl)tridec-12-ynylamino]benzoyl chloride hydrochloride in 1,2-dimethoxyethane is then added. The reaction mixture is refluxed for 5 hours, cooled, poured on ice and extracted with ether. The ether solution is washed with water and saturated sodium chloride solution, dried over anhydrous sodium sulfate and concentrated to dryness The residue is then recrystallized from ether to yield tert-butyl ethyl 4-[13-(4-chlorophenyl)-tridec-12-ynyl amino]benzoyl malonate.

EXAMPLE 875

Preparation of ethyl 2-{4-[13-(4-chlorophenyl)tridec-12-ynylamino]benzoyl}acetoacetate

A solution of 21.6 g. of ethyl acetoacetate and 10 ml. of 1,2-dimethoxyethane is added to a suspension of 4.0 g. of sodium hydride in 1,2-dimethoxyethane under argon. A solution of 17.3 g. of 4-[13-(4-chlorophenyl)tridec-12-ynylamino]benzoyl chloride hydrochloride in 1,2-dimethoxyethane is then added. The reaction mixture is refluxed for 5 hours, cooled, poured on ice and extracted with ether. The

ether solution is washed with water and saturated sodium chloride solution, dried over anhydrous sodium sulfate and concentrated to dryness. Recrystallization from ether affords ethyl 2- 4[13-(4-chlorophen yl)tridec-12-ynylamino]-benzoyl acetoacetate as a white solid.

EXAMPLE 876

Preparation of ethyl 4-[13-(4-chlorophenyl)tridec-12-ynyl amino]benzoyl acetate

A solution of 3.0 g. of tert-butyl ethyl 4-[13-(4-chlorophenyl) tridec-12-ynylamino]benzoylmalonate in 10 ml. of trifluoroacetic acid is warmed with stirring for 3 hours. The solution is poured onto ice and neutralized with potassium hydroxide. The resulting precipitate is collected by filtration, washed with water and dried. Recrystallization from chloroform affords ethyl 4-[13-(4-chlorophenyl)tridec-12-ynylamino]benzoylacetate.

EXAMPLE 877

Preparation of 4-[13-(4-chlorophenyl)tridec-12-ynylamino]-benzoylacetic acid

Two grams of ethyl 4-[13-(4-chlorophenyl)tridec-12-ynylamino]benzoylacetate is added to a solution of potassium hydroxide in 50 ml. of 1:9 water-ethanol. The reaction mixture is stirred for 24 hours at room temperature. Careful neutralization with sulfuric acid gave a precipitate which is filtered, washed with water, and dried to yield 4-[13-(4-chlorophenyl)tridec-12-ynylamino]benzoylacetic acid.

EXAMPLE 878

Preparation of 4'-[13-(4-chlorophenyl)tridec-12-ynylamino]-2-(methylsulfinyl)acetophenone

To a solution of 5.8 g. of dimethyl sulfoxide, dried over sieves, and 50 ml. of tetrahydrofuran is slowly aaded 28 ml. of n-butyllithium (2.4 M in hexane). To this mixture is added 10 g. of methyl 4-[13-(4-chlorophenyl)-trioec-12-ynylamino]benzoate in 200 ml. of tetrahydrohydrofuran. After two hours, the reaction mixture is poured onto ice, acidified with dilute hydrochloric acid and

quickly extracted with chloroform. The chloroform extract is washed with water and saturated sodium chloride solution, and dried over anhydrous sodium sulfate. Concentration affords a solid which is washed with 500 ml. of hot hexane, filtered while hot and then washed with hexane. The white solid is dried in vacuo to yield 4'-[13-(4-chlorophenyl)tridec-12-ynylamino]-2-(methylsulfinyl)acetophenone.

EXAMPLE 879

Preparation of 4'-[13-(4-chlorophenyl)tridec-12-ynylamino]-2-(phenylsulfonyl)acetophenone

A solution of 864 mg. of sodium hydride and 5.3 g. of methylphenylsulfone in 20 ml. of 1,2-dimethoxyethane is stirred at 6°C. for one hour under an atmosphere of argon. To this solution is added a solution of 5.0 g. of methyl 4-[13-4-chlorophenyl)tridec-12-ynylamino]benzoate in 50 ml. of tetrahydrofuran and the reaction mixture is stirred at 60°C. for 1.5 hours. The mixture is cooled, poured onto ice, acidified with dilute hydrochloric acid to pH 3 and then extracted with chloroform. The organic layer is separated, washed with water and saturated sodium chloride solution, dried over anhydrous sodium sulfate and then concentrated to dryness. The crude solid is chromatographed on silica gel, eluting with methylene chloride to yield 4'-[13-(4-chlorophenyl) tridec-12-ynylamino]-2-(phenylsulfonyl)acetophenone.

EXAMPLE 880

Preparation of 4'-[13-(4-chlorophenyl)tridec-12-ynylamino-2-phenylsulfinyl)acetophenone

To a solution of 6.2 g. of methylphenylsulfoxide, dried over sieves, and 50 ml. of tetrahydrofuran is slowly added 28 ml. of n-butyllithium (2.4 M in hexane). To this mixture is added 10 g. of a solution of methyl 4-[13-(4-chlorophenyl)tridec-12-ynylamino]benzoate in 200 ml. of tetrahydrofuran. After two hours, the reaction mixture is poured into ice, acidified with diluted hydrochloric acid and quickly extracted with chloroform. The chloroform layer is washed with water and saturated sodium chloride solution

and dried over anhydrous sodium sulfate. Concentration affords a solid which is washed with 500 ml. of hot hexane, filtered while hot, and then washed with 50 ml. of hexane. The white solid is dried _in vacuo_ yielding 4'-[13-(4-chlorophenyl)tridec-12-ynylamino]-2-(phenylsulfinyl)acetophenone.

EXAMPLE 881

Preparation of 3-{4'-[13-(4-chlorophenyl)tridec-12-ynylamino]benzoyl}-2,4-pentanedione

A solution of 28.4 g. of 2,4-pentanedione and 20 ml. of 1,2-dimethoxyethane is added to a suspension of 13.6 g. of sodium hydride in 220 ml. of 1,2-dimethoxyethane under argon. A solution of 28.7 g. of 4-[13-(4-chlorophenyl)tridec-12-ynylamino]benzoyl chloride hydrochloride in 1,2-dimethoxyethane is then added. The reaction mixture is stirred at room temperature for 12 hours, cooled, poured on ice and extracted with ether. The ether solution is washed with water and saturated sodium chloride solution, dried over anhydrous sodium sulfate and concentrated. The residue is then chromatographed over silica gel to yield 3-{4'-[13-(4-chlorophenyl)tridec-12-ynylamino]benzoyl} 2,4-pentanedione.

EXAMPLE 882

Preparation of methyl 3-{4-[13-(4-chlorophenyl)tridec-12-ynylamino]benzoyl}propionate

A mixture of 35 g. of 3-(4-acetamidobenzoyl)propionic acid, 700 ml. of methanol and 1.4 ml. of concentrated sulfuric acid is refluxed for 76 hours. The solution is cooled to 35° C. and poured onto 7 g. of anhydrous sodium acetate while stirring. The reaction mixture is stirred in an ice-bath. The solid is collected and washed with cold methanol to yield 3-(4-aminobenzoyl)propionate as a white solid. A mixture of this solid, 9.2 g. of 13-(4-chlorophenyl)tridec-12-ynylbromide and 4.2 g of potassium carbonate is stirred for 20 hours at 125°C. under nitrogen. The mixture is then cooled to 25°C. and 30 ml. of water is added. After stirring, the product is filtered and washed with water. Recrystallization from methanol affords methyl 3-{4-[13-(chlorophenyl)tridec-12-ynylamino]benzoyl}propionate as a white solid.

EXAMPLE 883

Preparation of 3-{4-[13-(4-chlorophenyl)tridec-12-ynylamino]benzoyl}propionic acid

A solution of 5.4 g. of methyl 3- 4-[13-(4-chlorophen yl)tridec-12-ynylamino]benzoyl propionate is stirred with 5.4 g. of potassium hydroxide in 100 ml. of 95% ethanol for 3 hours at reflux. The reaction mixture is cooled, diluted with 50 ml. cf ethanol and 100 ml. of water, neutralized with hydrochloric acid. The solution is cooled to room temperature and filtered. The white solid is washed with 50% aqueous ethanol and dried. The product is recrystallized from ethanol to yield 3-{4-[13-(4-chlorophenyl)-tridec-12-ynylamino] benzoyl}propionic acid as a white crystalline solid.

TABLE IX

The following terminal acetylenic compounds are prepared following the method of Example 817, using the appropriate aldehyde.

| Example No. | Aldehyde | Aryl or Heteroaryl Acetylenic Compound |
|---|---|---|
| 884 | 2,4-Dichlorobenzaldehyde | 2,4-Dichlorophenylacetylene |
| 885 | 4-Methoxybenzaldehyde | 4-Methoxyphenylacetylene |
| 886 | 3-(2-Pyridyl)propionaldehyde | 4-(2-Pyridyl)-4-butyne |
| 887 | 3-(2-Furyl)propionaldehyde | 4-(2-Furyl)-4-butyne |
| 888 | 2-Chloro-6-fluorobenzaldehyde | 2-Chloro-6-fluorophenylacetylene |
| 889 | 5-Methylfurfuryl | 2-Ethynyl-5-methylfuran |
| 890 | 4-Chlororphenylacetaldehyde | 3-(4-Chlorophenyl)-1-propyne |
| 891 | 2-Methyl-3-(4-chlorophenyl)-propionaldehyde | 3-Methyl-4-(4-chlorophenyl)-1-butyne |
| 892 | 4-Methoxyphenylacetaldehyde | 4-(4-Methoxyphenyl)-1-butyne |
| 893 | 4-Methoxyhydrocinnamaldehyde | 4-(4-Methoxyphenyl)-1-butyne |
| 894 | 4-(2-Pyridyl)butyraldehyde | 5-(2-Pyridyl)-1-pentyne |
| 895 | 4-(6-Methyl-2-pyridyl)butyraldehyde | 5-(6-Methyl-2-pyridyl)-1-pentyne |
| 896 | 2-Furylacetaldehyde | 3-(2-Furyl)-1-propyne |
| 897 | 5-Ethyl-2-furaldehyde | 5-Methyl-2-ethynylfuran |

The following aryl- or heteroarylalkynyl bromides are prepared by the method of Example 821. The required -bromoalkyl toluene sulfonates are prepared by the method of Examples 818, 819 and 820.

| Example No. | Acetylene | Bromoalkyl tosylate | Aryl- or Heteroarylalkynyl Bromide |
|---|---|---|---|
| 898 | 2-Chloro-6-fluoro-phenyl acetylene | 9-Bromononyl tosylate | 11-(2-Chloro-6-fluorophenyl)undec-10-ynyl bromide |
| 899 | 3,4-Dichloropentyl-acetylene | 11-Bromoundecyl tosylate | 13-(3,4-Dichlorophenyl)tridec-12-ynylbromide |
| 900 | 4-Chlorophenyl-acetylene | 7-Bromoheptyl tosylate | 9-(4-Chlorophenyl)non-8-ynyl bromide |
| 901 | 4-Chlorophenyl-acetylene | 15-Bromopentadecyl tosylate | 17-(4-Chlorophenyl)heptadec-16-ynylbromide |
| 902 | 4-Methoxyphenyl-acetylene | 5-Bromopentyl tosylate | 7-(4-Methoxyphenyl)hept-6-ynyl bromide |
| 903 | 4-Fluorophenyl-acetylene | 10-Bromodecyl tosylate | 12-(4-Fluorophenyl)dodec-11-ynyl bromide |
| 904 | 3-Methylphenyl-acetylene | 6-Bromohexyl tosylate | 8-(3-Methylphenyl)oct-7-ynyl bromide |
| 905 | Pentafluorophenyl-acetylene | 14-Bromotetradecyl tosylate | 16-(Pentafluorophenyl)hexadec-15-ynylbromide |
| 906 | 4-Phenyl-1-butyne | 8-Bromooctyl tosylate | 12-Phenyldodec-9-ynyl bromide |
| 907 | 5-Phenyl-1-pentyne | 4-Bromobutyl tosylate | 9-Phenylnon-5-ynyl bromide |

| Example No. | Acetylene | Bromoalkyl tosylate | Aryl- or Heteroarylalkynyl Bromide |
|---|---|---|---|
| 908 | 5-Phenyl-1-pentyne | 11-Bromoundecyl tosylate | 16-Phenylhexadec-12-ynyl bromide |
| 909 | 3-(3-Methylphenyl)-1-propyne | 9-Bromononyl tosylate | 12-(3-Methylphenyl)dodec-10-ynyl bromide |
| 910 | 3-(4-Methoxyphenyl)- | 9-Bromononyl tosylate | 12-(4-Methoxyphenyl)dodec-10-ynyl bromide |
| 911 | 4-(4-Methoxyphenyl)-1-butyne | 3-Bromopropyl tosylate | 7-(4-Methoxyphenyl)hept-4-ynyl bromide |
| 912 | 3-methyl-4-(4-chlorophenyl)-1-butyne | 4-Bromobutyl tosylate | 8-(4-Chlorophenyl)-7-methyloct-5-ynylbromide |
| 913 | 3-Methyl-4-(4-chlorophenyl)-1-butyne | 14-Bromotetradecyl tosylate | 18-(4-Chlorophenyl)-17-methyloctadec-15-ynyl bromide |
| 914 | 4-Bromophenylacetylene | 13-Bromodecyl tosylate | 15-(4-Bromophenyl)-pentadec-14-ynyl bromide |
| 915 | 6-Pheynl-1-hexyne | 7-Bromoheptyl tosylate | 13-phenyltridec-8-ynyl bromide |
| 916 | 4-Chlorophenyl- | 4-Bromobutyl tosylate | 6(4-Chlorophenyl)hex-5-ynyl bromide |
| 917 | 4-Chlorophenylacetylene | 16-Bromohexadecyl tosylate | 18-(4-Chlorophenyl)octadec-17-ynyl bromide |
| 918 | Phenylacetylene | 5-Bromopentyl tosylate | 7-Phenylhept-6-ynyl bromide |
| 919 | Phenylacetylene | 12-Bromododecyl tosylate | 14-Phenyltetradec-13-ynyl bromide |

TABLE X (cont'd)

| Example No. | Acetylene | Bromoalkyl tosylate | Aryl- or Heteroarylalkynyl Bromide |
|---|---|---|---|
| 920 | 4-(2-Propyl)phenyl-acetylene | 8-Bromooctyl tosylate | 10-[4-(2-Propyl)phenyl]dec-9-ynyl bromide |
| 921 | 2-Ethynylthiophene | 11-Bromoundecyl tosylate | 13-(2-Thienyl)tridec-12-ynyl bromide |
| 922 | 2-Ethynylfuran | 6-Bromohexyl tosylate | 8-(2-Furyl)oct-7-ynyl bromide |
| 923 | 2-Ethynylpyridine | 4-Bromobutyl tosylate | 6-(2-Pyridyl)hex-5-ynyl bromide |
| 924 | 5-Ethyl-2-ethynyl-furan | 5-Bromopentyl tosylate | 7-(5-Ethyl-2-furyl)hept-6-ynyl bromide |
| 925 | 3-(2-Furyl)-1-propyne | 10-Bromodecyl tosylate | 13-(2-Furyl)triadec-11-ynyl bromide |
| 926 | 5-(2-Pyridyl)-1-pentyne | 4-Bromobutyl tosylate | 9-(2-Pyridyl)non-5-ynyl bromide |
| 927 | 5-(6-Methyl-2-pyridyl)-1-pentyne | 12-Bromododecyl tosylate | 17-(6-Methyl-2-pyridyl)heptadec-13-ynyl bromide |
| 928 | 5-Methyl-2-ethynyl-thiophene | 8-Bromooctyl tosylate | 10-(5-Methyl-2-thienyl)dec-9-ynyl bromide |

TABLE XI

The following -aryl- or heteroaryl-3-alkynyl alcohols are prepared by the method of Example 824 from the appropriate acetylenes.

| Example No. | Acetylene | Aryl- or Heteroaryl Alcohol |
|---|---|---|
| 929 | 3-(4-Chlorophenyl)-1-propyne | 5-(4-Chlorophenyl)pent-3-ynyl alcohol |
| 930 | 4-(4-Methoxyphenyl)-1-butyne | 6-(4-Methoxyphenyl)hex-3-ynyl alcohol |
| 931 | 3-Methylphenylacetylene | 4-(3-Methylphenyl)but-3-ynyl alcohol |
| 932 | 2-Pyridylacetylene | 4-(2-Pyridyl)but-3-ynyl alcohol |
| 933 | 5-Phenyl-1-pentyne | 7-Phenylhept-3-ynyl alcohol |
| 934 | 4-Chlorophenylacetylene | 4-(40Chlorophenyl)but-3-ynyl alcohol |
| 935 | Phenylacetylene | 4-Phenylbut-3-ynyl alcohol |
| 936 | 3-(2-Thienyl)-1-propyne | 5-(2-Thienyl)pent-3-ynyl alcohol |
| 937 | (2-Furyl)acetylene | 4-(2-Furyl-but-3-ynyl alcohol |
| 938 | Pentafluorophenylacetylene | 4-(Pentafluorophenyl)but-3-ynyl alcohol |

TABLE XII

The following ethyl 4-(aryl- or heteroarylalkynylamino)benzoates are prepared by the methods of Examples 822 or 826. The methanesulfonates required are prepared as described in Example 825.

| Example No. | 4-(aryl- or heteroarylalkynylamino)benzoate |
|---|---|
| 939 | Ethyl 4-[11-(2-chloro-6-fluoropheynl)undec-10-ynylamino]benzoate |
| 940 | Ethyl 4-]13-(3,4-dichlorophenyl)tridec-12-ynylamino]benzoate |
| 941 | Ethyl 4-[9-(4-chlorophenyl)non-8-ynylamino]benzoate |
| 942 | Ethyl 4-[17-(4-chlorophenyl)heptadec-16-ynylamino]benzoate |
| 943 | Ethyl 4-[7-(4-methoxyphenyl)hept-6-ynylamino]benzoate |
| 944 | Ethyl 4-[12-(4-fluorophenyl)dodec-11-ynylamino]benzoate |
| 945 | Ethyl 4-[8-(3-methylphenyl)oct-7-ynylamino]benzoate |
| 946 | Ethyl 4-[16-(pentafluorophenyl)hexadec-15-ynylamino]benzoate |
| 947 | Ethyl 4-(12-phenyldodec-9-ynylamino)benzoate |
| 948 | Ethyl 4-(9-phenylnon-5-ynylamino)benzoate |
| 949 | Ethyl 4-(16-phenylhexadec-12-ynylamino)benzoate |
| 950 | Ethyl 4-[12-(3-methylphenyl)dodec-10-ynylamino]benzoate |
| 951 | Ethyl 4-[12-(3-methoxyphenyl)dodec-1-ynylamino]benzoate |
| 952 | Ethyl 4-[7-(4-methoxyphenyl)hept-4-ynylamino]benzoate |
| 953 | Ethyl 4-[8-(4-chlorophenyl)-7-methyloct-5-ynylamino]benzoate |
| 954 | Ethyl 4-[18-(4-chlorophenyl)-17-methyloctadec-15-ynylamino]benzoate |
| 955 | Ethyl 4-[15-(4-bromophenyl)pentadec-14-ynylamino]benzoate |
| 956 | Ethyl 4-(13-phenyltridec-8-ynylamino)benzoate |

TABLE XII (cont'd)

| Example No. | 4-(aryl- or heteroarylalkynylamino)benzoate |
|---|---|
| 957 | Ethyl 4-[6-(4-chlorophenyl)hex-5-ynylamino]benzoate |
| 958 | Ethyl 4-[18-(4-chlorophenyl)octadec-17-ynylamino]benzoate |
| 959 | Ethyl 4-(7-phenylhept-6-ynylamino)benzoate |
| 960 | Ethyl 4-(14-phenyltetradec-13-ynylamino)benzoate |
| 961 | Ethyl 4-{10-[4-(2-propyl)phenyl]dec-9-ynylamino} benzoate |
| 962 | Ethyl 4-[13-(2-thienyl)tridec-12-ynylamino]benzoate |
| 963 | Ethyl 4-[8-(2-furyl)oct-7-ynylamino]benzoate |
| 964 | Ethyl 4-[6-(2-pyridyl)hex-5-ynylamino]benzoate |
| 965 | Ethyl 4-[7-(5-ethyl-2-furyl)hept-6-ynylamino]benzoate |
| 966 | Ethyl 4-[13-(2-furyl)tridec-11-ynylamino]benzoate |
| 967 | Ethyl 4-[9-(2-pyridyl)non-5-ynylamino]benzoate |
| 968 | Ethyl 4-[17-(6-methyl-2-pyridyl)heptadec-13-ynylamino]benzoate |
| 969 | Ethyl 4-[10-(5-methyl-2-thienyl)dec-9-ynylamino]benzoate |
| 970 | Ethyl 4-[5-(4-chlorophenyl)pent-3-ynylamino]benzoate |
| 971 | Ethyl 4-[6-(4-methoxyphenyl)hex-3-ynylamino]benzoate |
| 972 | Ethyl 4-[4-(3-methylphenyl)but-3-ynylamino]benzoate |
| 973 | Ethyl 4-[4-(2-pyridyl)but-3-ynylamino]benzoate |
| 974 | Ethyl 4-(7-phenylhept-3-ynylamino)benzoate |
| 975 | Ethyl 4-[4-(4-chlorophenyl)but-3-ynylamino]benzoate |
| 976 | Ethyl 4-(4-phenylbut-3-ynylamino)benzoate |

TABLE XII (cont'd)

| Example No. | 4-(aryl- or heteroarylalkynylamino)benzoate |
|---|---|
| 977 | Ethyl 4-[5-(3-thienyl)pent-3-ynylamino]benzoate |
| 978 | Ethyl 4-(4-furylbut-3-ynylamino)benzoate |
| 979 | Ethyl 4-[4-(pentafluorophenyl)but-3-ynylamino]benzoate |

TABLE XIII

The following 4-(aryl- or heteroarylalkynylamino)benzoic acids are prepared from esters of Table XII by the method of Example 823.

| Example No. | 4-(Aryl- or heteroarylalkynylamino)benzoic acid |
|---|---|
| 980 | 4-[11-(2-Chloro-6-fluorophenyl)undec-10-ynylamino]benzoic acid |
| 981 | 4-[13-(3,4-Dichlorophenyl)tridec-12-ynylamino]benzoic acid |
| 982 | 4-[9-(4-Chlorophenyl)non-8-ynylamino)benzoic acid |
| 983 | 4-[17-(4-Chlorophenyl)heptadec-16-ynylamino]benzoic acid |
| 984 | 4-[7-(4-Methoxyphenyl)hept-6-ynylamino]benzoic acid |
| 985 | 4-[12-(4-Fluorophenyl)dodec-11-ynylamino]benzoic acid |
| 986 | 4-[8-(3-Methylphenyl)cot-7-ynylamino]benzoic acid |
| 987 | 4-[16-(Pentafluorophenyl)hexadec-15-ynylamino]benzoic acid |
| 988 | 4-(12-Phenyldodec-9-ynylamino)benzoic acid |
| 989 | 4-(9-Phenylnon-5-ynylamino)benzoic acid |
| 990 | 4-(16-Phenylhexadec-12-ynylamino)benzoic acid |
| 991 | 4-[12-(3-Methylphenyl)dodec-10-ynylamino]benzoic acid |
| 992 | 4-[12-(4-Methoxyphenyl)dodec-10-ynylamino]benzoic acid |
| 993 | 4-[7-(4-Methoxyphenyl)hept-4-ynylamino]benzoic acid |
| 994 | 4-[8-(4-Chlorophenyl)-7-methyloct-5-ynylamino]benzoic acid |
| 995 | 4-[18-(4-Chlorophenyl)-17-methyloctadec-15-ynylamino]benzoic acid |
| 996 | 4-[15-(4-Bromophenyl)pentadec-14-ynylamino]benzoic acid |
| 997 | 4-[13-(Phenyltridec-8-ynylamino)benzoic acid |
| 998 | 4-[6-(4-Chlorophenyl)hex-5-ynylamino]benzoic acid |
| 999 | 4-[18-(4-Chlorophenyl)octadec-17-ynylamino]benzoic acid |

TABLE XIII (cont'd)

| Example No. | 4-(Aryl- or heteroarylalkynylamino)benzoic acid |
|---|---|
| 1000 | 4-(7-Phenylhept-6-ynylamino)benzoic acid |
| 1001 | 4-(14-Phenyltetradec-13-ynylamino)benzoic acid |
| 1002 | 4-{10-[4-(2-Propyl)phenyl]dec-9-ynylamino}benzoic acid |
| 1003 | 4-[13-(2-Thienyl)tridec-12-ynylamino]benzoic acid |
| 1004 | 4-[8-(2-Furyl)oct-7-ynylamino]benzoic acid |
| 1005 | 4-[6-(2-Pyridyl)hex-5-ynylamino]benzoic acid |
| 1006 | 4-[7-(5-Ethyl-2-furyl)hept-6-ynylamino]benzoic acid |
| 1007 | 4-[13-(2-Furyl)tridec-11-ynylamino]benzoic acid |
| 1008 | 4-[9-(2-Pyriudyl)non-5-ynylamino]benzoic acid |
| 1009 | 4-[17-(6-Methyl-2-pyridyl)heptadec-13-ynylamino]benzoic acid |
| 1010 | 4-[10-(5-Methyl-2-thienyl)dec-9-ynylamino]benzoic acid |
| 1011 | 4-[5-(4-Chlorophenyl)pent-3-ynylamino]benzoic acid |
| 1012 | 4-[6-(4-Methoxyphenyl)hex-3-ynylamino]benzoic acid |
| 1013 | 4-[4-(3-Methylphenyl)but-3-ynylamino]benzoic acid |
| 1014 | 4-[4-(2-Pyridyl)but-3-ynylamino]benzoic acid |
| 1015 | 4-(7-Phenylhept-3-ynylamino)benzoic acid |
| 1016 | 4-[4-(4-Chlorophenyl)but-3-ynylamino]benzoic acid |
| 1017 | 4-(4-Phenylbut-3-ynylamino)benzoic acid |
| 1018 | 4-[5-(2-Thienyl)pent-3-ynylamino]benzoic acid |
| 1019 | 4-[4-(2-Furyl)but-3-ynylamino]benzoic acid |
| 1020 | 4-[4-(Pentafluorophenyl)but-3-ynylamino]benzoic acid |

TABLE XIV

The following aryl- or heteroarylalkenyl halides or alcohols are prepared from the aldehydes or esters shown by the methods listed in the Table. The requisite aldehydes are prepared by the methods of Example 828 and the requisite phosphonium salts are obtained by the method of Example 832.

| Example No. | Method Example | Starting Material | Aryl- or Heteroarylalkenyl halide or alcohol |
|---|---|---|---|
| 1021 | 834, 833 | 4-Chlorobenzaldehyde | 10(4-Chlorophenyl)dec-9-enyl alcohol |
| 1022 | 834, 833 | Phenylacetaldehyde | 6-Phenylhex-4-enyl alcohol |
| 1023 | 834, 833 | Phenylacetaldehyde | 15-Phenylpentadec-13-enyl alcohol |
| 1024 | 834, 833 | 2-Methylcinnamaldehyde | 9-Phenylnona-6,8-dienyl alcohol |
| 1025 | 834, 833 | 2-Phenylpropionaldehyde | 5-Methyl-5-phenylpent-3-enyl alcohol |
| 1026 | 834, 833 | 2-Phenylpropionaldehyde | 18-Methyl-18-phenyloctadec-16-enyl alcohol |
| 1027 | 834, 833 | 4-Fluorobenzaldehyde | 11-(4-Fluorophenyl)undec-10-enyl alcohol |
| 1028 | 834, 833 | 3-Bromobenzaldehyde | 7-(3-Bromophenyl)hept-6-enyl alcohol |
| 1029 | 834, 833 | 2-Thiophenecarboxaldehyde | 10-(2-Thienyl)dec-9-enyl alcohol |
| 1030 | 834, 833 | 2-Pyridinecarboxaldehyde | 13-(2-Pyridyl)tridec-12-enyl alcohol |
| 1031 | 834, 833 | Indole-3-carboxaldehyde | 12-(3-indoyl)dodec-11-enyl alcohol |
| 1032 | 840 | Methylcinnamate | Cinnamyl alcohol |
| 1033 | 840 | Methyl 4-fluorocinnamate | 4-Fluorocinnamyl alcohol |
| 1034 | 840 | Methyl 3-(trifluoromethyl)- | 3-(Trifluoromethyl)cinnamyl alcohol |
| 1035 | 837, 838 | 4,8-Dimethyl-3,7-nonadieno-nitrile | 2-(3,7-Dimethyl-2,6-octadienyl)-4-chloro-methylimidazole hydrochloride |

TABLE XIV (cont'd)

| Example No. | Method Example | Starting Material | Aryl- or Heteroarylalkenyl halide or alcohol |
|---|---|---|---|
| 1036 | 840 | Ethyl 5-octyl-2-thienyl-acrylate | 3-[(5-Octyl)-2-thienyl]prop-2-ene-1-ol |
| 1037 | 831, 833 | 2-Ethylthiophene | 9-[2-(5-Ethyl)thienyl]non-8-ene-1-ol |
| 1038 | 834, 833 | 5-Methylfurfural | 15-[2-(5-Methyl)furyl]pentadec-14-ene-1-ol |

TABLE XV

The following aryl- or heteroarylalkyl halides or alcohols are prepared from the appropriate starting materials by the methods shown. The requisite aryl- or heteroarylalkyl bromides are prepared by the method of Example 818. Saturated acids are prepared from the corresponding unsaturated acids by method of Example 835.

| Example No. | Method Example(s) | Starting Material | Aryl- or Heteroarylalkenyl halide or alcohol |
|---|---|---|---|
| 1039 | 833 | 4-Chlorocinnamic acid | 3-(4-Chlorophenyl)propanol |
| 1040 | 842 | 3-(4-Chlorophenyl)propylbromide and 1,10-dibromodecane | 13-(4-Chlorophenyl)tridecyl bromide |
| 1041 | 842 | 4-(4-Methoxyphenyl)butylbromide and 1,6-dibromodecane | 10-(4-Methoxyphenyl)decyl bromide |
| 1042 | 833 | Ethyl 3-[(5-octyl)-2-thienyl]acrylate | 3-[(5-Octyl)-2-thienyl]propanol |
| 1043 | 842 | 4-Decyloxybenzyl bromide | 12-(4-Decyloxyphenyl)dodecyl bromide |
| 1044 | 842 | 1-Bromoethylnapthalene and 1,6-dibromodecane | 8-(1-Naphthyl)octyl bromide |
| 1045 | 842 | 4-Benzyloxyphenethyl bromide | 14-(4-Benzyloxyphenyl)tetradecyl bromide |
| 1046 | 837, 838 | Benzyl cyanide | 2-Benzyl-4-chloromethylimidazole hydrochloride |
| 1047 | 837, 838 | Cycloyheptyl cyanide | 2-Chcloheptyl-4-chloromethylimidazole hydrochloride |

TABLE XV (cont'd)

| Example No. | Method Example(s) | Starting Material | Aryl- or Heteroarylalkenyl halide or alcohol |
| --- | --- | --- | --- |
| 1048 | 837, 838 | Butyronitrile | 2-Propyl-4-chloromethylimidazole hydrochloride |
| 1049 | 842 | 3-(2-Thienyl)propyl bromide and 1,6-dibromohexane | 9-(2-Thienyl)nonyl bromide |
| 1050 | 833 | 6-(2-Furyl)hexanoic acid | 6-(2-Furyl)hexanol |
| 1051 | 841 | 4-(2,5-Dimethyl-3-thienyl)-butyric acid | 4-(2,5-Dimethyl-3-thienyl)butanol |

TABLE XVI

The following aryl- or heteroarylalkynyl, alkenyl or alkylaminophenyl acetates are prepared from the appropriate alcohols or alkyl halides by the method shown. The requisite mesylates are prepared by the method of Example 825; -bromoalkylaminophenyl acetates are prepared by the method of Example 843.

| Example No. | Method of Example | 4-(Substituted-amino)phenylacetate esters |
|---|---|---|
| 1052 | 844 | Ethyl 4-(14-phenyltetradec-13-ynylamino)phenylacetate |
| 1053 | 844 | Ethyl 4-[8-(4-chlorophenyl)-7-methyloct-5-ynylamino]phenylacetate |
| 1054 | 844 | Ethyl 4-[17-(4-chlorophenyl)heptadec-16-ynylamino]phenylacetate |
| 1055 | 844 | Ethyl 4-[12-(4-methoxyphenyl)dodec-10-ynylamino]phenylacetate |
| 1056 | 844 | Ethyl 4-[8-(2-furyl)oct-7-ynylamino]phenylacetate |
| 1057 | 843 | Ethyl 4-[4-(2-pyridyl)but-3-ynylamino]phenylacetate |
| 1058 | 843 | Ethyl 4-[10-(4-chlorophenyl)dec-9-enylamino]phenylacetate |
| 1059 | 843 | Ethyl 4-(9-phenylnona-6,8-dienylamino)phenylacetate |
| 1060 | 843 | Ethyl 4-(18-methyl-18-phenyloctadec-16-ynylamino)phenylacetate |
| 1061 | 843 | Ethyl 4-[10-(2-thienyl)dec-9-enylamino]phenylacetate |
| 1062 | 827 | Ethyl 4-[4-Fluorophenylprop-2-enylamino]phenylacetate |
| 1063 | 827 | Ethyl 4-{3-[(5-octyl)-2-thienyl]prop-2-enylamino}phenylacetate |
| 1064 | 839 | Ethyl 4-[12-(4-chlorophenyl)heptadec-16-enylamino]phenylacetate |
| 1065 | 839 | Ethyl 4-[8-(2-furyl)oct-7-ynylamino]phenylacetate |
| 1066 | 827 | Ethyl 4-(1-methyl-3-phenylprop-2-enylamino)phenylacetate |
| 1067 | 843 | Ethyl 4-[3-(4-chlorophenyl)propylamino]phenylacetate |
| 1068 | 843 | Ethyl 4-{3-[(5-octyl)-2-thienyl]propylamino}phenylacetate |

TABLE XVI (cont'd)

| Example No. | Method of Example | 4-(Substituted-amino)phenylacetate esters |
|---|---|---|
| 1069 | 844 | Ethyl 4-[8-(1-naphthyl)octylamino]phenylacetate |
| 1070 | 844 | Ethyl 4-[(2-cycloheptyl-4-imidazolylmethyl)amino]phenylacetate |
| 1071 | 844 | Ethyl 4-[(2-propyl-4-imidazolylmethyl)amino]phenylacetate |
| 1072 | 829 | Ethyl 4-[(4-chlorophenyl)methylamino]phenylacetate |
| 1073 | 829 | Ethyl 4-[(4-hydroxy-3-methoxyphenyl)methylamino]phenylacetate |
| 1074 | 829 | Ethyl 4-[(5-octyl-2-thienylmethyl)amino]phenylacetate |
| 1075 | 836 | Ethyl 4-[6-(1-imidazolyl)hexylamino]phenylacetate |
| 1076 | 843 | Ethyl 4-[4-(2-thienyl)butylamino]phenylacetate |
| 1077 | 843 | Ethyl 4-(11-phenylundecylamino)phenylacetate |

TABLE XVII

The following aryl- or heteroarylalkynyl, alkenyl or alkylaminophenylacetic acids are prepared from the esters of Table XVI by the method of Example 823.

| Example No. | 4-(Substituted-amino)phenylacetic acids |
|---|---|
| 1078 | 4-(14-Phenyltetradec-13-ynylamino)phenylacetic acid |
| 1079 | 4-[8-(4-Chlorophenyl)-7-methyloct-5-ynylamino]phenylacetic acid |
| 1080 | 4-[17-(4-Chlorophenyl)heptadec-16-ynylamino]phenylacetic acid |
| 1081 | 4-[12-(4-Methoxyphenyl)dodec-10-ynylamino]phenylacetic acid |
| 1082 | 4-[8-(2-Furyl)oct-7-ynylamino]phenylacetic acid |
| 1083 | 4-[4-(2-Pyridyl)but-3-ynylamino]phenylacetic acid |
| 1084 | 4-[10-(Chlorophenyl)dec-9-enylamino]phenylacetic acid |
| 1085 | 4-(9-Phenylnona-6,8-dienylamino)phenylacetic acid |
| 1086 | 4-(18-Methyl-18-phenyloctadec-16-ynylamino)phenylacetic acid |
| 1087 | 4-[10-(2-Thienyl)dec-9-enylamino]phenylacetic acid |
| 1088 | 4-[4-Fluorophenylprop-2-enylamino]phenylacetic acid |
| 1089 | 4-{3-[(5-Octyl)-2-thienyl]prop-2-enylamino}phenylacetic acid |
| 1090 | 4-[17-(4-Chlorophenyl)heptadec-16-enylamino)]phenylacetic acid |
| 1091 | 4-[8-(2-Furyl)oct-7-ynylamino]phenylacetic acid |
| 1092 | 4-(1-Methyl-3-phenylprop-2-enylamino)phenylacetic acid |
| 1093 | 4-[3-(4-Chlorophenyl)propylamino]phenylacetic acid |
| 1094 | 4-{3-[(5-Octyl)-2-thienyl]propylamino}phenylacetic acid |
| 1095 | 4-[8-(1-Naphthyl)octylamino]phenylacetic acid |
| 1096 | 4-[(2-Cycloheptyl-4-imidazolyl)methylamino]phenylacetic acid |
| 1097 | 4-[(2-Propyl-4-imidazolyl)methylamino]phenylacetic acid |

TABLE XVII (cont'd)

| Example No. | 4-(Substituted-amino)phenylacetic acids |
|---|---|
| 1098 | 4-[(4-Chlorophenyl)methylamino]phenylacetic acid |
| 1099 | 4-[(4-Hydroxy-3-methoxyphenyl)methylamino]phenylacetic acid |
| 1100 | 4-[(5-Octyl-2-thienyl)methylamino]phenylacetic acid |
| 1101 | 4-[6-(1-imidazolyl)hexylamino]phenylacetic acid |
| 1102 | 4-[4-(2-Thienyl)butylamino]phenylacetic acid |
| 1103 | 4-(11-Phenylundecylamino)phenylacetic acid |
| 1104 | 4-[11-(1-Imidazolyl)undecylamino]phenylacetic acid |
| 1105 | 4-[6-(4-Chlorophenyl)hex-5-enylamino]phenylacetic acid |

## TABLE XVIII

The following aryl- or heteroarylalkynyl, alkenyl or alkylamino hydrocinnamates are prepared from the appropriate alcohols or alkyl halides by the method shown. Alcohols are converted to their corresponding mesylates by method of Example 825; ethyl -bromo-alkylaminohydrocinnamates are prepared according to the method of Example 843.

| Example No. | Method of Example | 4-(Substituted-amino)hydrocinnamate esters |
|---|---|---|
| 1106 | 844 | Ethyl 4-[7-(4-methoxyphenyl)hept-6-ynylamino]hydrocinnamate |
| 1107 | 844 | Ethyl 4-[12-(4-fluorophenyl)dodec-11-ynylamino]hydrocinnamate |
| 1108 | 844 | Ethyl 4-{10-[4-(2-propyl)phenyl]dec-9-ynylamino}hydrocinnamate |
| 1109 | 844 | Ethyl 4-[6-(2-pyridyl)hex-5-ynylamino]hydrocinnamate |
| 1110 | 843 | Ethyl 4-[5-(2-thienyl)pent-3-ynylamino]hydrocinnamate |
| 1111 | 827 | Ethyl 4-(3-phenylprop-2-enylamino)hydrocinnamate |
| 1112 | 843 | Ethyl 4-[10-(2-thienyl)dec-9-enylamino]hydrocinnamate |
| 1113 | 843 | Ethyl 4-(15-phenylpentadec-13-enylamino]hydrocinnamate |
| 1114 | 844 | Ethyl 4-[2-(317-dimethyl-2,6-octadienyl)-4-imidazolylmethyl-amino]hydrocinnamate |
| 1115 | 827 | Ethyl 4-[3-(3-trifluoromethylphenyl)prop-2-enylamino]hydrocin-namate |
| 1116 | 843 | Ethyl 4-{9-[2-(5-ethyl)thienyl)non-8-enylamino}hydrocinnamate |
| 1117 | 839 | Ethyl 4-[6-(2-pyridyl)hex-5-enylamino]hydrocinnamate |
| 1118 | 843 | Ethyl 4-[3-(4-chlorophenyl)propylamino]hydrocinnamate |
| 1119 | 844 | Ethyl 4-[10-(4-methoxyphenyl)decylamino]hydrocinnamate |
| 1120 | 844 | Ethyl 4-[12-(4-decyloxyphenyl)dodecylamino]hydrocinnamate |

TABLE XVIII (cont'd)

| Example No. | Method of Example | 4-(Substituted-amino)hydrocinnamate esters |
| --- | --- | --- |
| 1121 | 844 | Ethyl 4-[4-(2-benzyl)imidazolylmethylamino]hydrocinnamate |
| 1122 | 843 | Ethyl 4-[6-(2-furyl)hexylamino]hydrocinnamate |
| 1123 | 843 | Ethyl 4-(4-phenylbutylamino)hydrocinnamate |
| 1124 | 843 | Ethyl 4-(2-phenylbutylamino)hydrocinnamate |
| 1125 | 829 | Ethyl 4-(4-chlorophenylmethylamino)hydrocinnamate |
| 1126 | 836 | Ethyl 4-[12-(1-imidazolyl)dodecylamino]hydrocinnamate |
| 1127 | 836 | Ethyl 4-[6-(1-imidazolyl)hexylamino]hydrocinnamate |

## TABLE XIX

The following aryl- or heteroarylalkynyl, alkenyl or alkylaminohydrocinnamic acids are prepared from the esters of Table XVIII by the method of Example 823.

| Example No. | Hydrocinnamic Acids |
|---|---|
| 1128 | 4-[7-(4-Methoxyphenyl)hept-6-ynylamino]hydrocinnamic acid |
| 1129 | 4-[12-(4-Fluorophenyl)dodec-11-ynylamino]hydrocinnamic acid |
| 1130 | 4-{10-[4-(12-(4-Flurophenyl)dodec-11-ynylamino}hydrocinnamic acid |
| 1131 | 4-[6-(2-Pyridyl)hex-5-ynylamino]hydrocinnamic acid |
| 1132 | 4-[5-(2-Thienyl)pent-3-ynylamino]hydrocinnamic acid |
| 1133 | 4-[3-Phenylprop-2-enylamino]hydrocinnamic acid |
| 1134 | 4-[10-(2-Thienyl)dec-9-enylamino]hydrocinnamic acid |
| 1135 | 4-(15-Phenylpentadec-13-enylamino)hydrocinnamic acid |
| 1136 | 4-[2-(3,7-Dimethyl-2,6-octadienyl)-4-imidazolylmethylamino]hydrocinnamic acid |
| 1137 | 4-[7-(3-Bromophenyl)hept-6-enylamino]hydrocinnamic acid |
| 1138 | 4-[3-(3-Trifluoromethyphenyl)prop-2-enylamino]hydrocinnamic acid |
| 1139 | 4-{9-[2-(5-Ethyl)thienyl]non-8-enylamino} hydrocinnamic acid |
| 1140 | 4-[6-(2-Pyridyl)hex-5-enylamino]hydrocinnamic acid |
| 1141 | 4-[3-(4-Chlorophenyl)propylamino]hydrocinnamic acid |
| 1142 | 4-[10-(4-Methoxyphenyl)decylamino]hydrocinnamic acid |
| 1143 | 4-[12-(4-Decyloxyphenyl)dodecylamino]hydrocinnamic acid |
| 1144 | 4-[4-(2-Benzyl-4-imidazolylmethyl)amino]hydrocinnamic acid |
| 1145 | 4-[6-(2-Furyl)hexylamino]hydrocinnamic acid |
| 1146 | 4-(4-Phenylbutylamino)hydrocinnamic acid |

TABLE XIX (cont'd)

The following aryl- or heteroarylalkynyl, alkenyl or alkylaminohydrocinnamic acids are prepared from the esters of Table XVIII by the method of Example 823.

| Example No. | Hydrocinnamic Acids |
|---|---|
| 1147 | 4-(2-Phenylbutylamino)hydrocinnamic acid |
| 1148 | 4-(4-Chlorophenylmethylamino)hydrocinnamic acid |
| 1149 | 4-[12-(1-Imidazolyl)dodecylamino]hydrocinnamic acid |
| 1150 | 4-[6-(1-Imidazolyl)hexylamino]hydrocinnamic acid |
| 1151 | 4-[2-(5-Octylthienyl)methylamino]hydrocinnamic acid |

TABLE XX

The following aryl- or heteroarylalkynyl, alkenyl or alkylaminocinnamates are prepared from the appropriate alcohols or alkyl halides by the method shown. Alcohols are converted to the required mesylates by method of Example 825.

| Example No. | Example | Cinnamate Esters |
|---|---|---|
| 1152 | 843 | Ethyl 4-[6-(4-methoxyphenyl)hex-3-ynylamino]cinnamate |
| 1153 | 844 | Ethyl 4-[13-(2-thienyl)tridec-12-ynylamino]cinnamate |
| 1154 | 844 | Ethyl 4-[4-(2-furyl)but-3-ynylamino]cinnamate |
| 1155 | 843 | Ethyl 4-(5-methyl-5-phenylpent-3-enylamino]cinnamate |
| 1156 | 843 | Ethyl 4-[11-(4-fluorophenyl)undec-10-enylamino]cinnamate |
| 1157 | 843 | Ethyl 4-[12-(3-indolyl)dodec-11-enylamino]cinnamate |
| 1158 | 827 | Ethyl 4-[3-(4-chlorophenyl)prop-2-enylamino]cinnamate |
| 1159 | 827 | Ethyl 4-(1-methyl-3-phenylprop-2-enylamino]cinnamate |
| 1160 | 844 | Ethyl 4-[14-(4-benzyloxyphenyl)tetradecylamino]cinnamate |
| 1161 | 843 | Ethyl 4-[4-(2,5-dimethyl-3-thienyl)butylamino]cinnamate |
| 1162 | 843 | Ethyl 4-(10-phenyldecylamino)cinnamate |
| 1163 | 829 | Ethyl 4-[2-(5-octylthienyl)methylamino]cinnamate |
| 1164 | 844 | Ethyl 4-(4-heptyloxyphenylmethylamino)cinnamate |

TABLE XXI

The following aryl- or heteroarylalkynyl alkenyl or alkylaminocinnamic acids are prepared from esters of Table XX by the method of Example 823.

| Example No. | Cinnamic Acids |
|---|---|
| 1165 | 4-[6-(4-methoxyphenyl)hex-3-ynylamino]cinnamic acid |
| 1166 | 4-[13-(2-thienyl)tridec-12-ynylamino]cinnamic acid |
| 1167 | 4-[4-(2-furyl)but-3-ynylamino]cinnamic acid |
| 1168 | 4-(5-methyl-5-phenylpent-3-enylamino]cinnamic acid |
| 1169 | 4-[11-(4-fluorophenyl)undec-10-enylamino]cinnamic acid |
| 1170 | 4-[12-(3-indolyl)dodec-11-enylamino]cinnamic acid |
| 1171 | 4-[3-(4-chlorophenyl)prop-2-enylamino]cinnamic acid |
| 1172 | 4-(1-methyl-3-phenylprop-2-enylamino)cinnamic acid |
| 1173 | 4-[14-(4-benzyloxyphenyl)tetradecylamino]cinnamic acid |
| 1174 | 4-[4-(2,5-dimethyl-3-thienyl)butylamino]cinnamic acid |
| 1175 | 4-(10-phenyldecylamino)cinnamic acid |
| 1176 | 4-[2-(5-octyl-2-thienyl)ethylamino]cinnamic acid |
| 1177 | 4-(4-heptyloxyphenylmethylamino)cinnamic acid |
| 1178 | 4-[13-(4-chlorophenyl)tridecylamino]cinnamic acid |

TABLE XXII.

The following aryl- or heteroarylalkynyl, alkenyl, or alkylaminophenylpropiolates are prepared from the appropriate alcohols or alkyl halides by the method shown. The requisite mesylate are prepared by the method of Example 825.

| Example No. | Method of Example | 4-(Substituted-amino)phenylpropiolate esters |
|---|---|---|
| 1179 | 844 | Ethyl 4-[13-(3,4-dichlorophenyl)tridec-12-ynylamino]propiolate |
| 1180 | 843 | Ethyl 4-[4-(2-pyridyl)but-3-ynylamino]propiolate |
| 1181 | 843 | Ethyl 4-(6-phenylhex-4-enylamino)propiolate |
| 1182 | 843 | Ethyl 4-{15-[2-(5-methyl)furyl]pentadec-14-eynylamino}propiolate |
| 1183 | 827 | Ethyl 4-[3-(4-fluorophenyl)prop-2-enylamino]propiolate |
| 1184 | 844 | Ethyl 4-[9-(2-thienyl)nonylamino]propiolate |
| 1185 | 843 | Ethyl 4-(1-ethyl-2-phenylethylamino]propiolate |
| 1186 | 844 | Ethyl 4-[2-(2-naphthyl)ethylamino]propiolate |
| 1187 | 843 | Ethyl 4-[3-(4-chlorophenyl)propylamino propiolate |

TABLE XXIII

The following aryl- or heteroarylalkynyl, alkenyl, or alkylaminophenylpropiolic acids are prepared from the esters of Table XXII by the method of Example 823.

| Example No. | Phenylpropiolic Acids |
|---|---|
| 1188 | 4-[13-(3,4-dichlorophenyl)tridec-12-ynylamino]propiolic acid |
| 1189 | 4-[4-(2-pyridyl)but-3-ynylamino]propiolic acid |
| 1190 | 4-(6-phenylhex-4-enylamino)propiolic acid |
| 1191 | 4-{15-[2-(5-methyl)furyl]pentadec-14-enylamino}propiolic acid |
| 1192 | 4-[3-(4-fluorophenyl)prop-2-enylamino]propiolic acid |
| 1193 | 4-[9-(2-thienyl)nonylamino]propiolic acid |
| 1194 | 4-(1-ethyl-2-phenylethylamino]propiolic acid |
| 1195 | 4-(2-(2-naphthyl)ethylamino]propiolic acid |
| 1196 | 4-[3-(4-chlorophenyl)propylamino]propiolic acid |

TABLE XXIV

The following aryl- or heteroarylalkynyl, alkenyl, or alkylaminoacetophenones are prepared by the method of Example 845. The requisite mesylates are prepared by the method of Example 825.

| Example No. | 4-(Substituted-amino)acetophenones |
|---|---|
| 1197 | 4-[9-(4-Chlorophenyl)non-8-ynylamino]acetophenone |
| 1198 | 4-[16-(Pentafluorophenyl)hexadec-15-ynylamino]acetophenone |
| 1199 | 4-(4-(2-Pyridyl)but-3-ynylamino)acetophenone |
| 1200 | 4-[13-(2-Furyl)tridec-11-ynylamino]acetophenone |
| 1201 | 4-(9-Phenylnona-6,8-dienylamino)acetopheone |
| 1202 | 4-(18-Methyl-18-phenyloctadec-16-enylamino)acetophenone |
| 1203 | 4-[3-(4-Chlorophenyl)prop-2-enylamino]acetophenone |
| 1204 | 4-{3-[(5-Octyl)-2-thienyl]propylamino}acetophenone |
| 1205 | 4-[13-(Chlorophenyl)tridecylamino]acetophenone |
| 1206 | 4-[6-(2-Furyl)hexylamino]acetophenone |
| 1207 | 4-[2-(4-Fluorophenyl)ethylamino]acetophenone |
| 1208 | 4-(7-Phenylheptylamino)acetophenone |

## TABLE XXV

The following aryl- or heteroarylalkynyl, alkenyl, or alkylaminobenzonitriles are prepared from the appropriate halide or alcohol by the method of Example 846. The requisite mesylates are prepared by the method of Example 825.

| Example No. | 4-(Substituted-amino)benzonitriles |
|---|---|
| 1209 | 4-[10-(5-Methyl-2-thienyl)dec-9-ynylamino]benzonitrile |
| 1210 | 4-(7-Phenylhept-6-ynylamino)benzonitrile |
| 1211 | 4-[18-(4-Chlorophenyl)octadec-17-ynylamino]benzonitrile |
| 1212 | 4-[4-(3-Methylphenyl)but-3-ynylamino]benzonitrile |
| 1213 | 4-(4-(2-Furyl)but-3-ynylamino)benzonitrile |
| 1214 | 4-(9-Phenylonon-6,8-dienylamino)benzonitrile |
| 1215 | 4-[11-(4-Fluorophenyl)undec-10-enylamino]benzonitrile |
| 1216 | 4-[13-(2-Pyridyl)tridec-12-enylamino]benzonitrile |
| 1217 | 4-[12-(3-Indoyl)dodec-11-enylamino]benzonitrile |
| 1218 | 4-[10-(2-Thienyl)dec-9-enylamino]benzonitrile |
| 1219 | 4-[12-(4-Decyloxyphenyl)dodecylamino]benzonitrile |
| 1220 | 4-[8-(1-Naphthyl)octylamino]benzonitrile |
| 1221 | 4-[9-(2-Thienyl)nonylamino]benzonitrile |
| 1222 | 4-[4-(2,5-Dimethyl-3-thienyl)butylamino]benzonitrile |
| 1223 | 4-(1-Ethyl-2-phenylethylamino)benzonitrile |
| 1224 | 4-[14-(4-Benzyloxyphenyl)tetradecylamino]benzonitrile |

TABLE XXVI

The following aryl- or heteroarylakynyl, alkenyl or alkylaminobenzaldehyudes are prepared from the corresponding benzonitriles of Table XXV by the method of Example 847.

| Example No. | 4-(Substituted-amino)benzaldehydes |
|---|---|
| 1225 | 4-[10-(5-Methyl-2-thienyl)dec-9-ynylamino]benzaldehyde |
| 1226 | 4-(7-Phenylhept-6-ynylamino)benzaledhyde |
| 1227 | 4-[18-(4-Chlorphenyl)okctadec-17-ynylamino]benzaldehyde |
| 1228 | 4-[4-(3-Methylphenyl)but-3-ynylamino]benzaldehyde |
| 1229 | 4-(4-(2-Furyl)but-3-ynylamino]benzaldehyde |
| 1230 | 4-(9-Phenylnona-6,8-dienylamino)benzaldehyde |
| 1231 | 4-[11-(4-Fluorophenyl)undec-10-enylamino]benzaldehyde |
| 1232 | 4-[13-(2-Pyridyl)tridec-12-enylamino]benzaldehyde |
| 1233 | 4-[12-(3-Indolyl)dodec-11-enylamino]benzaldehyde |
| 1234 | 4-[10-(2-Thienyl)dec-9-enylamino]benzaldehyde |
| 1235 | 4-[12-(4-Decyloxyphenyl)dodecylamino]benzaldehyde |
| 1236 | 4-[8-(1-Napthyl)octylamino]benzaldehyde |
| 1237 | 4-[9-(2-Thienyl)nonylamino]benzaldehyde |
| 1238 | 4-[4-(2,5-Dimethyl-3-thienyl)butylamino]benzaldehyde |
| 1239 | 4-(1-Ethyl-2-phenylethylamino)benzaldehyde |
| 1240 | 4-[14-(4-Benzyloxyphenyl)tetradecylamino]benzaldehyde |

TABLE XXVII

The following aryl- or heteroarylakynyl, alkenyl or alkylaminophenolbutyrates are prepared from the appropriate mesylates by the method of Example 843. The requisite mesylates are prepared by the method of Example.

| Example No. | 4-(Substituted-amino)phenylbutyrate esters |
|---|---|
| 1241 | Ethyl 4-[4-(2-phenylbutylamino)phenyl]butyrate |
| 1242 | Ethyl 4-[4-(4-phenylbutylamino)phenyl]butyrate |
| 1243 | Ethyl 4-[4-(15-phenylpentadec-13-enylamino)phenyl]butyrate |
| 1244 | Ethyl 4-{4-[6-(2-furyl)hexylamino]phenyl}butyrate |
| 1245 | Ethyl 4-{4-[10-(2-thienyl)dec-9-enylamino]phenyl}butyrate |
| 1246 | Ethyl 4-{4-[3-(4-chlorophenyl)propylamino]phenyl}butyrate |
| 1247 | Ethyl 4-{4-[9-(5-ethyl-2-thienyl)non-8-enylamino]phenyl}butyrate |
| 1248 | Ethyl 4-{4-[5-(2-thienyl)pent-3-ynylamino]phenyl}butyrate |

TABLE XXVIII

The following aryl- or heteroarylakynyl, alkenyl or alkylaminophenylbutyric acids are prepared from the esters of Table XXVII by the method of Example 823.

| Example No. | 4-(Substituted-amino)phenylbutyric acids |
|---|---|
| 1249 | 4-[4-(2-Phenylbutylamino)phenyl]butyric acid |
| 1250 | 4-[4-(15-Phenylpentadec-13-enylamino)phenyl]butyric acid |
| 1251 | 4-{4-[6-(2-Furyl)hexylamino]phenyl}butyric acid |
| 1252 | 4-{4-[3-(4-Chlorophenyl)propylamino]phenyl}butyric acid |
| 1253 | 4-{4-[5-(2-Thienyl)pent-3-ynylamino]phenyl}butyric acid |

## TABLE XXIX

The following esters are prepared by the methods shown from the carboxylic acids of Tables VIII, XVII, XIX, XXI, XXIII and XXVIII or appropriate derivatives thereof obtained by the methods of Examples 848-852.

| Example No. | Method of Example | Ester |
|---|---|---|
| 1254 | 853 | 2,3-Dihydroxypropyl 4-[11-(2-chloro-6-fluorophenyl)undec-10-ynylamino]benzoate |
| 1255 | 853 | 2,3-Dihydroxyproyl 4-[(2-cycloheptyl)-4-imidaxolymethylamino]-phenylacetate |
| 1256 | 853 | 2,3-Dihydroxypropyl 4-[12-(4-ethyl-2-furyl)pentadec-14-enyl-amino]propiolate |
| 1259 | 853 | 2,3-Dihydroxypropyl 4-[4-(2-phenylbutylamino)phenyl]butyrate |
| 1260 | 854 | Methyl 4-[(4-chlorophenyl)methylamino)amino]phenylacetate |
| 1261 | 854 | Isopropyl 4-[5-(2-thienyl)pent-3-ynylamino]hydrocinnamate |
| 1262 | 854 | Methyl 4-[13-(2-thienyl)tridec-12-ylamino]cinnamate |
| 1263 | 854 | Isopropyl 4-[9-(furyl)nonylamino]propiolate |
| 1264 | 854 | Methyl 4-(15-phenylpentadec-13-enylamino)phenyl]butyrate |
| 1265 | 855 | 3-Hydroxypropyl 4-[16-(pentafluorophenyl)hexadec-15-ynylamino]-benzoate |

TABLE XXIX (cont'd)

| Example No. | Method of Example | Ester |
|---|---|---|
| 1266 | 855 | 2-Hydroxypropyl 4-[5-octyl-2-thienyl)methylamino]phenylacetate |
| 1267 | 855 | 4-Hydroxybutyl 4-[10-(2-thienyl)dec-9-enylamino]hydrocinnamate |
| 1268 | 855 | 2-Hydroxypropyl 4-[3-(4-chlorophenyl)prop-2-enylamino]cinnamate |
| 1269 | 855 | 3-Hydroxypropyl 4-[1-ethyl-2-phenylethylamino)propiolate |
| 1270 | 855 | 2-Hydroxyethyl 4-{4-[6-(2-furyl)hexylamino]phenyl}butyrate |
| 1271 | 856 | 2-Ethoxyethyl 4-[12-(3-methylphenyl)dodec-10-ynylamino]benzoate |
| 1272 | 856 | 2-Methoxyethyl 4-[4-(2-thienyl)butylamino]acetate |
| 1273 | 856 | 2-Ethoxyethyl 4-[7-(3-bromophenyl)hept-6-enylamino]hydrocinnamate |
| 1274 | 856 | 2-Methoxyethyl 4-[4-(4-benzyloxyphenyl)tetradecylamino]cinnamate |
| 1275 | 856 | 2-Ethoxyethyl 4-[3-(4-chlorophenyl)propylamino]propiolate |
| 1276 | 857 | Methyl 4-(11-phenylundecylamino)phenylacetate |
| 1277 | 857 | Methyl 4-[6-(2-pyridyl)hex-5-enylamino)hydrocinnamate |
| 1278 | 857 | Methyl 4-[13-(4-chlorophenyl)tridecylamino]cinnamate |
| 1279 | 858 | 1-Methoxycarbonylpropyl 4-[8-(4-cholorophenyl)-7-methyloct-5-ynylamino]benzoate |
| 1280 | 858 | 1-Ethoxycarbonylethyl 4-(13-phenyltridec-8-ynylamino)benzoate |
| 1281 | 858 | 1-Ethoxycarbonylpropyl 4-[(2-propyl-4-imidazolyl)methylamino]-phenylacetate |
| 1282 | 858 | 1-Methoxycarbonylpropyl 4-[3-(4-chlorophenyl)propylamino]hydro-cinnamate |

TABLE XXIX (cont'd)

| Example No. | Method of Example | Ester |
|---|---|---|
| 1283 | 858 | 1-Ethoxycarbonylpropyl 4-[4-(2,5-dimethyl-3-thienyl)butylamino]-cinnamate |
| 1284 | 858 | 1-Ethoxycarbonylpropyl 4-[2-(2-napthyl)ethylamino]propiolate |
| 1285 | 858 | 1-Methoxycarbonylethyl 4-{4-[3-(4-chlorphenyl)propylamino]-phenyl}butyrate |
| 1286 | 859 | 1-Ethoxycarbonylethyl 4-(7-phenylhept-6-ynylamino)benzoate |
| 1287 | 859 | 1-Ethoxycarbonylethyl 4-[18-(chlorophenyl)-17-methyloctadec-15-ynylamino]benzoate |
| 1288 | 860 | 1-Carboxyethyl 4-[13-(2-furyl)tridec-11-ynynlamino]benzoate |
| 1289 | 860 | 1-Carboxypropyl 4-[5-(4-chlorophenyl)pent-3-ynylamino]benzoate |
| 1290 | 860 | 1-Carboxyethyl 4-[6(4-chlorophenyl)hex-5-enylamino]phenylacetate |
| 1291 | 860 | 1-Carboxypropyl 4-[4-(2-benzyl-4-imidazolyl)methylamino]hydro-cinnamate |
| 1292 | 860 | 1-Carboxyethyl 4-(10-phenyldecylamino)cinnamate |
| 1293 | 860 | 1-Carboxybutyl 4-(6-phenylhex-4-enylamino)propiolate |
| 1294 | 860 | 1-Carboxyethyl 4-{4-[5-(2-thienyl)pent-3-ynylamino]phenyl}butyr-ate |
| 1295 | 861 | 4-Chlorophenyl 4-[4-(2-pyridylbut-3-ynylamino]benzoate |
| 1296 | 861 | 4-Methylphenyl 4-[6-(1-imidazolyl)hexylamino]phenylacetate |
| 1297 | 861 | 3-Pyridylmethyl 4-[10-(4-methoxyphenyl)dec-9-ynylamino]hydrocin-namate |

TABLE XXIX (cont'd)

| Example No. | Method of Example | Ester |
|---|---|---|
| 1298 | 861 | 4-Pyridyl 4-[2-(5-octyl-2-thienyl)ethylamino]cinnamate |
| 1299 | 861 | 2-Pryidyl 4-[3-(4-fluorophenyl)prop-2-enylamino]propiolate |
| 1300 | 862 | O-[4-(9-Phenylnon-5-ynylamino)benzoyl]malic acid |
| 1301 | 862 | O-{4-[4-(2-Furyl)but-3-ynylamino]benzoyl} malic acid |
| 1302 | 862 | O-{4-[10-(5-Methyl-2-thienyl)dec-9-ynylamino]benzoyl} malic acid |
| 1303 | 863 | 2-(Ethoxycarbonyl)vinyl 4-(4-phenylbut-3-ynylamino)benzoate |
| 1304 | 863 | 2-(Ethoxycarbonyl)vinyl 4-[6-(4-chlorophenyl)hex-5-enylamino]-phenylacetate |
| 1305 | 863 | 2-(Ethoxycarbonyl)vinyl 4-[6-(2-furyl)hexylamino]hydrocinnamate |
| 1306 | 863 | 2-(Ethoxycarbonyl)vinyl 4-(1-methyl-3-phenylprop-2-enylamino)-cinnamate |
| 1307 | 863 | 2-(Ethoxycarbonyl)vinyl 4-[13-(3,4-dichlorophenyl)tridec-12-ynylamino]propiolate |
| 1308 | 863 | 2-(Ethoxycarbonyl)vinyl 4-[4-(2-phenylbutylamino)phenyl]butyrate |

TABLE XXX

The following amides are prepared from the carboxylic acids of Table XIII or appropriate derivatives thereof obtained by the methods of Examples 848-852.

| Example No. | Method of Example | Ester |
|---|---|---|
| 1309 | 864 | 1-{4-[4-(4-Fluorophenyl)but-3-ynylamino]benzoyl} piperidine |
| 1310 | 864 | 1-{4-[5-(2-Thienyl)pent-3-ynylamino]benzoyl} pyrrolidine |
| 1311 | 865 | Ethyl 4-(7-phenylhept-3-ynylamino)hippurate |
| 1312 | 865 | Ethyl 4-[4-(2-pyridyl)but-3-ynylamino]hippurate |
| 1313 | 866 | N-{4-[13-(2-Furyl)tridec-11-ynylamino]benzoyl}glycine |
| 1314 | 866 | N-{4-[7-(5-Ethyl-2-furyl)hept-6-ynylamino]benzoyl} glycine |
| 1315 | 867 | 4-[17-(4-Chlorophenyl)heptadec-16-ynylamino]-N-(phenylsulfonyl)-benzamide |
| 1316 | 867 | 4-[13-(2-Furyl)tridec-11-ynylamino]-N-(phenylsulfonyl)benzamide |
| 1317 | 868 | 4-[5-(4-Chlorophenyl)pent-3-ynylamino]-N-(methylsulfonyl)benz-amide |
| 1318 | 868 | 4-[4-(2-Pyridy)but-3-ynylamino]-N-(methylsulfonyl)benzamide |
| 1319 | 869 | N-{4-[8-(3-Methylphenyl)oct-7-ynylamino]benzoyl} alanine |
| 1320 | 869 | N-{4-[5-(2-Thienyl)pent-3-ynylamino]benzoyl}alanine |
| 1321 | 870 | N-{4-[9-(4-Chlorophenyl)non-8-ynylamino]benzoyl}benzamide |
| 1322 | 870 | N-{4-[5-(2-Thienyl)pent-3-ynylamino]benzoyl}benzamide |
| 1323 | 871 | N-{4-[7-(4-Methoxyphenyl)hept-6-ynylamino]benzoyl}piperidine |
| 1324 | 871 | N-{4-[8-(2-Furyl)oct-7-ynylamino]benzoyl} pyrrolidine |

TABLE XXX (cont'd)

| Example No. | Method of Example | Ester |
|---|---|---|
| 1325 | 872 | N-(2,3-Dihydroxypropyl)octadex-7-ynylamino]benzamide |
| 1326 | 872 | N-(2,3-Dihydroxypropyl)-4-[9-(2-pyridyl)non-5-ynylamino]benzamide |

TABLE XXXI

The following acetophenones are prepared from the carboxylic acids of Table XIII or appropriate derivatives thereof obtained by the methods of Examples 848-852.

| Example No. | Method of Example | 4-(Substituted-amino)acetophenones |
|---|---|---|
| 1327 | 873 | Diethyl 4-[5-(2-thienyl)pent-3-ynylamino]benzoylmalonate |
| 1328 | 874 | *tert*-Butyl ethyl 4-[4-(2-pyridyl)but-3-ynylamino]benzoylmalonate |
| 1329 | 875 | Ethyl 2-{4-[13-(2-furyl)tridec-11-ynylamino]benzoyl} acetoacetate |
| 1330 | 876 | Ethyl 4-[17-(4-chloropenyl)heptadec-16-ynylamino]benzoylacetate |
| 1331 | 877 | 4-[8-(3-Methylphenyl)oct-7-ynylamino]benzoacetic acid |
| 1332 | 878 | 4'-[7-(4-Methoxyphenyl)hept-6-ynylamino]-2-(methylsulfinyl)-acetophenone |
| 1333 | 879 | 4'-[5-(2-Thienyl)pent-3-ynylamino]-2-(phenylsulfinyl)acetophenone |
| 1334 | 880 | 4'-[9-(4-Chlorophenyl)non-8-ynylamino]-2-(phenylsulfinyl)acetophenone |
| 1335 | 881 | 3-{4-[8-(2-Furyl)oct-7-ynylamino]benzoyl}-2,4-pentanedione |
| 1336 | 882 | Methyl 3-{4-[9-(2-pyridyl)non-5-ynylamino]benzoyl} propionate |
| 1337 | 883 | 3-{4-[9-(2-Pyridyl)non-5-ynylamino]benzoyl} propionic acid |

EXAMPLE 1338

3-Fluoro-4-methylacetanilide

To a solution of 22.1 g of 3-fluoro-4-methylaniline in 200 ml of ethyl ether is added 19 5 ml (21.1 g) of acetic anhydride at such a rate as to maintain gentle reflux When the addition is complete, the solution is stirred for another 15 minutes, then heated to reflux for 1 hour. The mixture is then concentrated and upon cooling a solid is obtained. Recrystallization from chroloform hexane yields 3-fluoro-4-methylacetanilide as white solid.

EXAMPLE 1339

4-Acetamido-2-fluorobenzoic acid

Solid 3-fluoro-4-methyl acetanilide (10.0 g) is slowly added to a solution of 29.4 g potassium permanganate and 20.40 g. magnesium sulfate in 1.0 l of water at 65°C. After the addition is complete, the mixture is heated under reflux for 6 hours, cooled and allowed to stand overnight. Solid sodium carbonate (29.0 g.) is then added, the reaction mixture is stirred for 15 minutes and filtered. The colorless filtrate is then acidified with 40 ml of concentrated hydrochloric acid and chilled. Filtration yields a white solid. Recrystallization from ethanol-water provides 4-acetamido-2-fluorobenzoic acid.

EXAMPLE 1340

Ethyl 4-amino-2-fluorobenzoate

A solution of 10.0 g of 4-acetamido-2-fluorobenzoic acid, 100 ml of ethyl alcohol and 2.0 ml of boron trifluoride etherate is heated under reflux for 22 hours. The solvent is slowly removed by distillation as an equal amount of fresh ethyl alcohol is added. After 200 ml of solvent has been removed, the remainder is evaporated at reduced pressure. The residue is dissolved in ethyl acetate, washed with saturated aqueous sodium bicarbonate, dried and the solvent then evaporated to yield a tolid. Recrystallization of the solid from ethanol-hexane yields ethyl 4-amino-2-fluorobenzoate.

EXAMPLE 1341

Ethyl 2-fluoro-4-(hexadecylamino) benzoate

A mixture of 2.6 g of ethyl 4-amino-2-fluorobenzoate 4.5 g of hexadecylbromide, 2.0 g of anhydrous potassium carbonate and 20 ml of nexamethylphosphoramide is heated to 130°C. for 22 hours. The mixture is cooled, diluted with 200 ml of water, and extracted twice with ethyl ether. The combined extracts are washed with water, dried, and the ether evaporated and the residue upon recrystallization from hexane yields ethyl-2-fluoro-4-(hexadecylamino) benzoate as a white solid.

EXAMPLE 1342

Methyl 2-fluoro-4-(hexadecylamino)benzoate

A mixture of 8.3 g. of methyl 4-amino-2-fluorobenoate, 15.0 g. of hexadecylbromide, 6.8 g. of anhydrous potassium carbonate and 50 ml of hexamethylphosphamide is heated to 135° for 20 hours. Upon cooling, dilution with water and extraction with ether, the orange colored ethereal extract is washed with water, dried and evaporated to yield an off-white solid. Recrystallization of this material from hexane affords methyl 2-fluoro-4-(hexadecylamino)benzoate.

EXAMPLE 1343

2-Fluoro-4-(hexadeclamino)benzoic acid

A mixture of 3.5 g. of methyl 2-fluoro-4-hexadecylaminobenzoate, 40 ml of ethanol, 60 ml of water, and 3.8 g of potassium hydroxide is heated on a steam bath for 14 hours. The hot solution is acidified with concentrated hydrochloric acid. The reaction mixture is cooled and filtered. The colorless residue is recrystallized from chloroform-hexane to yield 2-fluoro-4-(hexadecylamino)benzoic acid as a white solid.

EXAMPLE 1344

Sodium 2-fluoro-4-(hexadecylamino)benzoate

A mixture of 3.62 g. of 2-fluoro-4-(hexadecylamino)benzoic acid and 25 ml of ethanol-water (9:1) containing 0.400 g. of sodium hydroxide is stirred for 4 hours. The mixture is filtered and the residue washed with 10 ml.

of ethanol-water (9:1) and dried, in *vacuo*, over $P_2O_5$ for 24 hours to yield sodium 2-fluoro-4-(hexadecylamino)benzoate as a white solid.

EXAMPLE 1345

Ethyl 4-amino-3-fluorobenzoate

A solution of 15.0 g of 4-amino-3-fluorobenzoic acid 150 ml of anhydrous ethyl alcohol and 5 ml of boron trifluoride ehterate is heated to reflux for 20 hours. Most of the solvent is then removed and 200 ml of water added and the mixture extracted with chloroform. The chloroform extract is washed with an aqueous solution of sodium bicarbonate dried over sodium sulfate and the solvent evaporated to yield an off-white solid which upoon recrystallization from ethanolhexane yields ethyl 4-amino-3-fluorobenzoate as a white solid.

EXAMPLE 1346

Ethyl 3-fluoro-4-(hexadecylamino)benzoate

A mixture of 4.0 g of ethyl 4-amino-3-fluorobenzoate, 6.66 g of hexadecyl bromide and 3.0 g of potassium carbonate in 25 ml of hexamethyl phosphamide is stirred at 150°C. for 20 hr. The mixture is cooled, diluted with water and extracted with ether. The ether extract is washed with water, dried, and the ether evaporated. The residue is recrystallized from hexane to yield ethyl 3-fluoro-4-(hexadecylamino)benzoate as a white solid.

EXAMPLE 1347

3-Fluoro-4-(hexadecylamino)benzoic acid

A solution of 3.8 g of potassium hydroxide in 15 ml of water is added to a mixture of 3.5 g of ethyl 3-fluoro-4-hexadecylaminobenzoate, 30 ml of water and 30 ml of ethanol. Upon heating, a clear solution is formed and after 20 hours, the hot solution is acidified with concetrated hydrochloric acid and cooled. The precipitate is collected and recrystallized from ethanol and then from chloroform to yield 3-fluoro-4-(hexadecylamino)benzoic acid as a colorless solid.

EXAMPLE 1348

Ethyl 2-fluoro-4-(octylamino)benzoate

A mixture of 33 g of ethyl 4-amino-2-fluorobenzoate, 44 ml of 1-bromooctane and 0 50 g of copper power is heated on a steam bath for 19 hours. The mixture is chilled, diluted with ethanol, filtered and the solid washed with cold ethanol and with water to give tan crystals. The filtrate is neutralized with 10N potassium hydroxide, chilled and filtered and the solid washed with water and with ethanol to give tan crystals. The two crops of crystals are combined and recrystallized from ethanol to yield ethyl 2-fluoro-4-(octylamino)benzoate as a white solid.

EXAMPLE 1349

Ethyl 3-fluoro-4-(tetradecylamino)benzoate

To a solution of 16.5 g. of ethyl 3-fluorobenzoate in 150 ml. of dry N N-dimethylformamide is added 4.12 g of sodium hydride (56% in oil) and 27.7 g. of 1-bromotetradecane The mixture is heated on a steam bath until hydrogen evolution began and then chilled briefly to control the reaction. After the sodium hydride has reacted, the mixture is heated on a steam bath under nitrogen for 6 hours. The mixture is chilled, filtered and the solid washed with ethanol and with water to give ethyl 3-fluoro-4-(tetradecylamino)benzoate.

EXAMPLE 1350

Ethyl 4-(p-chlorobenzylamino)-2-fluorobenzoate

Ethyl 4-amino-2-fluorobenzoate (17 g), 4-chlorobenzaldehyde (14 g), and 100 ml. of absolute alcohol are heated on a steam bath for 10 minutes Upon cooling, the mixture is filtered. The residue is dried and then dissolved in 200 ml of hot ethanol and 4 g. of sodium borohydride is added in portions with stirring. The mixture is then heated under reflux, with stirring, for 23 hours, cooled, added to ice water and filtered to yield ethyl 4-(p-chlorobenzylamino)-2-fluorobenzoate as a solid.

EXAMPLE 1351

Ethyl 3-fluoro-4-(hexadecanoylamino)benzoate

A solution of hexadecanoyl chloride (20 g) in 100 ml of methylene chloride is slowly added with stirring to a solution of 20 g of ethyl 4-amino-3-fluorobenzoate in 100 ml of methylene chloride. The mixture is filtered and the filtrate is washed with dilute hydrochloric acid and water, dried and the solvent evaporated. Crystallization of the residue from acetonitrile affords ethyl 3-fluoro-4-(hexadecanoylamino)benzoate.

EXAMPLE 1352

Ethyl 3-fluoro-4-(hexadecylamino)benzoate

A solution of 5 g of ethyl 3-fluoro-4-(hexadecanoylamino)benzoate in 50 ml of tetrahydrofuran is slowly added with stirring to 13 ml of 1M borane in tetrahydrofuran. The resulting solution is then poured into 50 ml of 10% aqueous hydrochloric acid and the resulting mixture is filtered. The residue is crystallized from acetonitrile to yield ethyl 3-fluoro-4-(hexadecylamino)benzoate as white crstals.

EXAMPLE 1353

4-Amino-2,3,5,6-tetrafluorobenzoic acid

A mixture of 10.0 g of 2,3,4,5,6-pentafluorobenzoic acid and 400 ml of nitromethane is saturated with anhydrous ammonia at room temperature and then placed in a bomb and heated at 100° for 20 hrs. The resulting solution is evaporated and the residue crustallized from chloroform-hexane to yield 4-amino-2,3,5,6-tetrafluorobenzoic acid as a white solid.

EXAMPLE 1354

4,14-Pentadecadienyl methanesulfonate

To a solution of 4,14-pentadecadien-1-ol (15.0 g) and triethylamine (14 ml.) in dry methylene chloride (320 ml.) at -8°C. is added methanesulfonylchloride (5.73 ml.), dropwise. The reaction mixture is stirred at -10°C. for 30 minutes and then diluted with methylene chloride, extracted with ice-water (250 ml.), followed by cold 10% HCl (200

ml.); cold saturated sodium bicarbonate (200 ml.) and cold brine (200 ml.). The organic phase is dried ($MgSO_4$) and the solvent removed *in vacuo* to provide 4,14-pentadecadien-1-yl-methanesulfonate as a clear oil.

## EXAMPLE 1355

### Ethyl 2-fluoro-4-[(4,14-pentadecadienyl)amino]benzoate

A solution of 28.2 g. of 4,14-pentadecadien-1-yl-methanesulfonate, as prepared in Example 1354 above and 19.8 g. of ethyl p-aminobenzoate in hexamethylphosphoramide is heated at 120°C for 20 hours. After cooling, the reaction mixture is diluted with ethanol:water (1·1) (30 ml.) and chilled. More ethanol is added, the mixture is filtered and the solid residue is recrystallized from ethanol to yield ethyl 2-fluoro-4-[(4,14-pentadecadienyl)amino]benzoate as a white solid.

### Table XXXII

The following benzoic acids are prepared from the corresponding ethyl esters by the hydrolysis procedure of Example 1343. The requisite esters are obtained by the reaction of the appropriate alkyl halide or mesylate with the appropriate ring-substituted ethyl 4-aminobenzoate by the methods of Examples 1341 and 1355.

| Example No. | Compound |
|---|---|
| 1356 | 3,5-Difluoro-4-(hexadecylamino)benzoic acid |
| 1357 | 4-(Hexadecylamino)-2,3,5-trifluorobenzoic acid |
| 1358 | 4-(Hexadecylamino)-2,3,5,6-tetrafluorobenzoic acid |
| 1359 | 4-(Hexadecylamino)-2-methylbenzoic acid |
| 1360 | 2-Chloro-4-(tetradecylamino)benzoic acid |
| 1361 | 3,5-Dibromo-4-(undecylamino)benzoic acid |
| 1362 | 3-Fluoro-4-(octadecylamino)benzoic acid |
| 1363 | 2-Fluoro-4-(1-methylpentadecylamino)benzoic acid |
| 1364 | 3-Bromo-4-(1-ethyltetradecylamino)benzoic acid |

1365 2-Methoxy-4-(6-methylhexadecylamino)benzoic acid

1366 2-Fluoro-4-(14-methylpentadecylamino)benzoic acid

1367 3,5-Diiodo-4-15-methylhexadecylamino)benzoic acid

1368 3-Chloro-4-(3,7,11-trimethyldodecylamino)-benzoic acid

1369 2-Methoxy-4-(3,7,11-trimethyldodecylamino)-benzoic acid

1370 2-Fluoro-4-(13,13-dimethyltetradecylamino)-benzoic acid

1371 3,5-Dimethyl-4-(15,15-dimethylhexadecrylamino)benzoic acid

1372 2-Hydrozy-4-(10-undecenylamino)benzoic acid

1373 3-Methyl-4-(4-hexadecenylamino)benzoic acid

1374 2-Fluoro-4-(2,6,10-trimethyl-11-dodecenylamino)benzoic acid

1375 3,5-Difluoro-4-(9-octadecenylamino)benzoic acid

1376 3-Nitro-4-(3,7-dimethyl-6-octenylamino)benzoic acid

1377 3,5-Dichloro-4-(1-methyl-6-heptenylamino)-benzoic acid

1378 3-Fluoro-4-(4,14-pentadecadienylamino)benzoic acid

1379 2-Fluoro-4-(3,7,11-trimethyl-2,6,10-dodecatrienylamino)benzoic acid

1380 2,6-Difluro-4-(11-hexadecynylamino)benzoic acid

1381 3,5-Dibromo-4-(6-methyl-6-hepten-2-ynylamino)benzoic acid

1382 2-Methyl-4-[3-(1,3-dimethylcyclohexyl-2-propyl)amino]benzoic acid

1383 2-Methoxy-4-(13-cyclopentyltridecylamino)-benzoic acid

| | |
|---|---|
| 1384 | 2-Fluoro-4-(11-cyclohexylundecylamino)benzoic acid |
| 1385 | 3-Fluoro-4-cyclotetradecylaminobenzoic acid |
| 1386 | 2-Chloro-4-[4-(1-cyclohexenyl)butylamino]-benzoic acid |
| 1387 | 2,6-Difluoro-4-[4-cyclohexyl-2-butenyl)-amino]benzoic acid |
| 1388 | 2-Fluoro-4-(11-phenylundecylamino)benzoic acid |
| 1389 | 3-Fluoro-4-(4-chlorobenzylmamino)benboic acid |
| 1390 | 2-Methyl-4-[2-(4-fluorophenyl)ethylamino]-benzoic acid |
| 1391 | 2-Acetoxy-4-(4-decyloxybenzylamino)benzoic acid |
| 1392 | 3-Fluoro-4-(4-methylbenzylamino)benzoic acid |
| 1393 | 3-Methyl-4-[6-(2-furyl)hexylamino]benzoic acid |
| 1394 | 3-Bromo-4-[3-(2,4-cichlorophenyl)propyl-amino]benzoic acid |
| 1395 | 2-Fluoro-4-[3-(4-benzylosyphenyl)propyl-amino]benzoic acid |
| 1396 | 3-Fluoro-4-[2-(1-napthyl)ethylamino]benzoic acid |
| 1397 | 2,5-Difluoro-4-(hexydecylamino)benzoic acid |
| 1398 | 2-Fluoro-4-(tetradecylamino)benzoic acid |
| 1399 | 3-Fluoro-4-(undec-10-enylamino)benzoic acid |

EXAMPLE 1400

2-Fluoro-4-(hesadecylamino)benzaldehyde

2-Fluoro-4-aminobenzonitrile (11.8 g.) and 1-bromohexadecane (15.3 g.) are dissolved in hexamethylphosphoramide (200 ml.) and heated under an atmosphere of nitrogen in an

oil bath maintained at 120°C. for 22 hours. The reaction mixture is cooled to room temperature and water is added gradually. The mixture is then chilled in an ice-bath and filtered. The solid is washed thoroughly with water and dried. The solid is recrystallized from ether-hexane to yield 2-fluoro-4-(hexadecylamino)benzonitrile as a pale yellow solid.

Di-*i*sobutylaluminum hydride (54 ml., 25% solution in toluene) is added with stirring to a solution of the 2-fluoro-4-(hexadecylamino)benzonitrile under a nitrogen atmosphere. The temperature rises to 40 C. during the addition which takes 30 minutes and the reaction is then stirred for 1 hour. A solution of methanol in toluene (50 ml., 1:1) is added during 30 minutes and the mixture is poured into vigorously stirred ice-cold aqueous sulfuric acid (500 ml., 5%). After 10 minutes, diatomaceous earth (30 g.) is added, the mixture filtered and the organic layer separated. The aqueous solution is extracted twice with toluene (100 ml.) and the combined organic layers are washed with aqueous sodium bicarbonate, dried over magnesium sulfate, decolorized with charcoal, filtered and evaporated *in vacuo* to give a light yellow crystalline solid. Recrystallization from hexane affords 2-fluoro-4(hexadecylamino)benzaldehyde as a white solid.

## EXAMPLE 1401

### 3-Fluoro-4-(hexadecylamino)acetophenone

3-Fluoro-4-aminoacetophenone (87.6 g.) is heated with 1-bromohexadecane (198 g.) in dry hexamethylphosphoramide (300 ml.) containing potassium carbonate (90 g. for 16 hours at 100°C. The solution is cooled to room temperature, filtered to remove solids, and the filtrate is diluted with cold water (20 ml.). The amber solid so obtained is collected and washed with water. Recrystallization of the solid from ethanol and then from dichloromethane affords 3-fluoro-4-(hexadecylamino)acetophenone.

EXAMPLE 1402

2-[2-Fluoro-4-(hexadecylamino)phenyl]-1,3-dioxolane

2-Fluoro-4-(hexadecylamino)benzaldehyde (1.7 g.) is dissolved in toluene (20 ml), and ethylene glycol (2.5 ml.) and p-toluenesulfonic acid (10 mg.) are added. The reaction mixture is heated under reflux for 16 hours while water is removed using a Dean-Stark trap. The reaction mixture is then cooled to room temperature, diluted with toluene (70 ml.), washed with aqueous sodium bicarbonate and then with water. The organic layer is separated dried over magnesium sulfate, decolorized with charcoal and filtered through anhydrous magnesium silicate. Removal of the solvent *in vacuo* gives a light yellow solid which is recrystallized from hexane to yield 2-[2-fluoro-4-(hexadecylamino)phenyl]1,3-dioxolane.

EXAMPLE 1403

3-Fluoro-4-(hexadecylamino)benzoyl chloride hydrochloride

A cold solution of 25 g. 3-fluoro-4-(hexadecylamino)benzoic acid in 500 ml. dimethoxyethane-methylene chloride (4:1) is prepared and dry hydrochloric acid is bubbled through the solution until no more precipitate forms. The solution is treated with 25 ml. thionyl chloride and refluxed until all of the precipitate has dissolved. The solvents are evaporated to yield 3-fluoro-4-(hexydecylamino)benzoyl chloride hydrochloride as an orange, semicrystalline mass.

EXAMPLE 1404

N-Trifluoroacetyl-3-fluoro-4-(hexadecylamino)benzoyl chloride

A stirred, ice-cold suspension of 9 g. 3-fluoro-4-(hexadecylamino)benzoic acid in 100 ml. of dimethoyethane and 16 ml. of pyridine is treated with 18 ml. trifluroacetic anhydride at 0°C. The solution is stirred at 0°C. for 30 minutes at room temperature and then diluted with 300 ml. ether and 100 g. ice. After stirring vigorously for 15 minutes, the phases are separated, the ether solution is washed with brine, dried and evaporated to a white, amorphous solid. To a souution of 9.2 g. of the above solid in 30 ml. methylene chloride and 0.5 ml. dimethylformamide is added 5.7 ml. thionyl chloride. After 20 hours at reflux, the solvents are evaporated to yield N-trifluoroacetyl-3-fluoro-4-(hexadecylamino)benzoyl chloride as a light yellow, mobile oil.

EXAMPLE 1405

N-Carbobenzyloxy-2-fluoro-4-(hexadecylamino)benzoyl chroride

To 15 g. 2-fluoro-4-(hexadecylamino)benzoic acid in 200 ml. warm chloroform is added a solution of 15 g. of sodium carbonate in 150 ml. water. To the vigorously stirred solution is added 10 g. carbobenzyloxy chloride. After 2 hours stirring at 40°C., the layers are separated, washed three times with 1N hydrochloric acid, dried, and evaporated to an oil. The oil is dissolved in 300 ml. toluene, treated with 15 ml. thionyl chloride and the solution is refluxed for 5 hours. The solvents are evaporated and the residue is dissolved three times in toluene, evaporating each time, ultimately to yield N-carbobenzyloxy-2-fluoro-4(hexadecylamino)benzoyl chloride as a viscuous, orange oil.

EXAMPLE 1406

4-N-tert-Butyloxcarbonyl-N-hexadecylamino)-3-fluorobenzoyl imidazole

A solution of 10 g. 3-fluoro-4-(hexadecylamino)-benzoic acid in 100 ml. dioxane is treated with 4.0 g. tert-butylazidoformate and 10 ml pyridine. After stirring at room temperature for 18 hours, the protected amino-acid is precipitated from solution by the addition of 150 ml. water. The solid is collected and thoroughly dried, and dissolved in 200 ml. of a mixture consisting of methlene chloride/dimethoxsyethane/pyridine (1:4:1). To this solution is added 5.4 g. 1,1'-carbonyldiimidazole. The solution is stirred overnight at room temperature and the solvents are evaporated to yield 4-(N-tert-butyloxycarbonyl-N-hexadecylamino)-3-fluoro-benzoylimidazole as an orange oil.

EXAMPLE 1407

2,3-Dihydroxypropyl 2-fluoro-4-(hexadecylamino)benzoate

A solution of 7.34 g. of 2-fluoro-4-(hexadecylamino)benzoic acid, 4.80 g. of 25% aqueous sodium hydroxide, and 12.6 g. of 3-iodo-1,2-propanediol in 50 ml of hexamethylphosphoramide is stirred for 24 hours at ambient temperature, diluted with 100 ml of ether and stirred for 5 days at ambient temperature. The mixture is treated with water and extracted with ether. The dried extracts are evaporated to yield 2,3-dihydroxypropyl 2-fluro-4(hexadecylamino)benzoate.

EXAMPLE 1408

Methyl 3-fluoro-4-(hexadecylamino)benzoate

A solution of 7.20 g. of 3-fluoro-4-(hexadecylamino)benzoic acid in 25 ml of hexamethylphosphoramide is added to a stirred mixture of 0.800 g of sodium hydride (57% in mineral oil) and 25 ml of he hexamethylphosphoramide. The solution which forms after one hour is treated with 11.0 g. of methyl iodide and is then stirred at 25°C. for 18 hours. Dilution with water folowed by filtration affords a white solid which is crystallized from ethanol to yield methyl 3-fluoro-4(hexadecylamino)benzoate as a white solid.

EXAMPLE 1409

3-Hydroxypropyl 3-fluoro-4-(hexadecylamino)benzoate

A mixture of 2.25 g. of methyl 3-fluoro-4-(hexadecylamino)benzoate, 280 mg. of 1,3-propanediol and 1.37 g of p-toluenesulfonic acid is heated at 180°C. for 18 hours and then is partitioned between ether and 3% aqueous sodium carbonate solution. The ether layer is separated, dried, and evaporated to yield 3-hydroxypropyl 3-fluoro-4-(hexadecylamino)benzoate.

EXAMPLE 1410

2-Ethanoxyethyl 2-fluoro-4-(hexadecylamino)benzoate

A solution of 11.8 g. of 2-fluoro-4-(hexadecylamino)benzoic acid, 1.00 g. of 2-ethoxyethanol and 5.35 ml. of boron trifluoride etherate in 200 ml. of toluene is

stirred under reflux for 48 hours. The solution is treated with an additional 5.35 ml. of boron trifluoride etherate and refluxing is continued for 120 hours. Dilution with water and methylene chloride followed by filtration affords 2-ethoxyethyl 2-fluoro-4-(hexadecylamino)benzoate as a white solid.

EXAMPLE 1411

Methyl 2-fluoro-4-(hexadecylamino)benzoate

A solution of 50.5 g. of 2-fluoro-4-(hexadecylamino)benzoic acid and 34.4 ml of boron trifluoride etherate in 200 ml of methanol is stirred under reflux for 44 hours, allowed to cool, and poured into 1.20 liters of ice cold 5% aqueous sodium carbonate solution. The white solid is collected by filtration and recrystallized from benzene-ethanol to yield methyl 2-fluoro-4-(hexadecylamino)benzoate.

EXAMPLE 1412

1-(Methoxycarbonyl)propyl 3-fluoro-4-(hexadecylamino)-benzoate

To a solution of 10.0 g. 3-fluoro-4-(hexadecylamino)benzoyl chloride hydrochloride in 200 ml methylene chloride is added dropwise a solution of 3 g. methyl 2-hydroxybutyrate and 5 g. triethylamine in 100 ml. ether. After 17 hours stirring at room temperature, the precipitate is collected and washed with several portions of ether. The ether solution is washed with water, dried and evaporated to yield 1-(methoxycarbonyl)propyl 3-fluro-4-(hexadecylamino)-benzoate as a white solid.

EXAMPLE 1413

1-(Ethoxycarbonyl)ethyl 3-fluoro-4-(hexadecylamino)benzoate

To a warm mixture of 7 g. sodium 3-fluoro-4-(hexadecylamino)benzoate in 100 ml. ethanol is added 4.7 g. ethyl 2-tosyloxypropionate. After 17 hours at reflux, the cooled solution is diluted with an equal volume of water and the resultant precipitate is filtered. After washing with cold ethanol and drying, the product is crystallized from acetonitrile to yield 1-(ethoxycarbonyl)ethyl 3-fluoro-4-(hexadecylamino)benzoate as colorless crystals.

EXAMPLE 1414

1-Carboxyethyl 2-fluoro-4-(hexadecylamino)benzoate

A flask containing 10.0 g. 2-fluoro-4-(hexadecylamino)benzoic acid, 3.3 g. lactic acid, 500 mg. toluenesulfonic acid and 500 ml. toluene is equipped with a Soxhlet extractor charged with activated 4A Linde molecular sieves. The solution is refluxed for 24 hours, during which time the Soxhlet extractor is charged twice more with fresh sieves. The hot solution is filtered and left to cool, whereupon 1-carboxyethl 2-fluoro-4-(hexadecylamino)benzoate separates as off-white crystals.

EXAMPLE 1415

Diethyl O-[2-fluoro-4-(hexadecylamino)benzoyl]tartrate

A mixture of 2-fluoro-N-trifluoracetyl-4-(hexadecylamino)benzoyl chloride and 1.2 g. triethylamine in 100 ml. warm ether is treated with 2.5 g. diethyl tartrate and refluxed for 24 hours. The hot solution is filtered, the residue is washed with hot ether, and the solution is evaporated. After treatment with aqueous methanolic potassium carbonate, the product is precipitated by acidification, filtered, and dried Crystallization from acetone yields diethyl O-[2-fluoro-4-(hexadecylamino)benzoyl]tartarate as a white, crystalline solid.

EXAMPLE 1416

O-[3-Fluoro-4-(hexadecylamino)benzoylmalic acid

A warm solution of N-carbobenzyloxy-3-fluoro-4-(hexadecylamino)benzoyl chloride and 1.3 g. triethylamine in 100 ml. ether is treated with 2 g. malic acid. An immediate precipitate forms, but the mixture is refluxed for one hour and filtered while hot. The solid is washed several times with hot ether, then the ether is evaporated to yield a white solid. The product is dissolved in tetrahydrofuran (100 ml.) and hydrogenated over 600 mg. 10% Pd(C) at 50 psi until hydrogen uptake stops. The catalyst is filtered, and the solution is evaporated. The residue is crystallized from acetic acid to yield O-[3-fluoro-4-(hexadecylamino)-benzoyl]malic acid.

EXAMPLE 1417

2-(Ethoxycarbonyl)vinyl 3-fluoro-4-(hexadecylamino)benzoate

To a mixture containing 4.3 g. 1-[3-fluoro-4-(N-*tert*-butyloxycarbonyl-N-hexadecylamino)benzoyl]imidazole, 50 ml. 5*N* sodium hydroxide is added 3 g. ethyl 2-formyl acetate. The solution is vigorously stirred for 24 hours. The layers are separated, and the chloroform solution is washed once with 50 ml. 1*N* sodium hydroxide. The solvent is evaporated and the residue is heated for 30 minutes at 40°C. in 50 ml. anhydrous trifluoroacetic acid. The solvent is again evaporated and the oil is crystallized from acetone to yield light yellow crystals of 2-(ethoxycarbonyl)vinyl 3-fluoro-4-(hexadecylamino)benzoate.

Table XXXIII

These benzoate esters are prepared from the corresponding halides, sulfonate esters, or hydroxy compounds by the methods of Examples 1407-1417 as shown in the table. The requisite carboxylic acids, carboxylate salts, carboxylate esters, N-protected carbonyl chlorides, or N-protected carbonyl imidazoles are prepared by the methods of Examples 1341-1355 and 1403-1406.

| Example No. | Method of Example | Compound |
|---|---|---|
| 1418 | 1409 | Isopropyl 3-fluoro-4-(hexadecylamino)benzoate |
| 1419 | 1412 | 2-Dimethylaminoethyl 2,5-difluoro-4-(undecylamino)benzoate |
| 1420 | 1407 | Allyl 3-chloro-4-(14-methylpentadecylamino)benzoate |
| 1421 | 1408 | 2,3-Epoxypropoxy 3-methoxy-4-(10-undecenylamino)benzoate |
| 1422 | 1407 | 4-Hydroxybutyl 2,6-difluoro-4-(4-hexadecenylamino)benzoate |
| 1423 | 1410 | 4-Chlorobenzyl 3-nitro-4-(4,14-pentadecadienylamino)benzoate |
| 1424 | 1412 | 4-(*tert*-Butyl)phenyl 3,5-dimethyl-4-(11-phenylundecylamino)benzoate |

| Example No. | Method of Example | Compound |
|---|---|---|
| 1425 | 1413 | Tetrahydropyranyl 2-fluoro-4-(4-chlorobenzylamino)benzoate |
| 1426 | 1412 | 3-Pyridyl 3,5-dichloro-4-(1-methyl-6-heptenylamino)benzoate |
| 1427 | 1408 | 3-Pyridylmethyl 2-fluoro-4-(cinnamylamino)benzoate |
| 1428 | 1415 | 3-O-[3-fluoro-4-(11-cyclohexylundecylamino)benzoyl]glyceric acid |
| 1429 | 1416 | 2,6-Dichlorophenyl 3,5-difluoro-4-[6-(2-furyl)hexylamino]benzoate |
| 1430 | 1412 | 5-Carboxy-3-pyridyl 2-methoxy-4-(3,7,11-trimethyldodecylamino) benzoate |
| 1431 | 1414 | Carboxymethyl 2-fluoro-4-[2-(1-naphthyl)ethylamino]benzoate |
| 1432 | 1417 | 2-Carboethoxy vinyl 3-fluoro-4-(9-octadecenylamino)benzoate |
| 1433 | 1411 | Methyl 2,4 6-trifluoro-4-(1-methyl-6-heptenylamino)benzoate |
| 1434 | 1415 | 2,6-Dimethyl-3-pyridyl 3,6-dimethyl-4-[3-(4-benzyloxyphenyl)-propylamino]benzoate |

EXAMPLE 1435

3-Fluoro-4-(hexadecylamino)benzoylpiperidine

To a chilled solution of 35 ml. of piperidine, 2.5 ml. of triethylamine and 0.6 g. of dimethylaminopyridine in 100 ml. of diethyl ether is added (1/2 hour) a solution of 8.3 g. of 3-fluoro-4-hexadecylaminobenzoylchloride hydrochloride in 50 ml. of ether. The solution is warmed to room temperature and maintained there for two hours. The solution is heated to reflux for an additional 2 hours at which time the reaction is complete. The solution is cooled, extracted twice with water and dried. The solvent is removed *in vacuo* and the solid is recrystallized from diethyl ether to yield 3-fluoro-4-(hexadecylamino)benzoyl-

piperidine.

EXAMPLE 1436

Ethyl 2-fluoro-4-(hexadecylamino)hippurate

To a solution of 18.0 g. of 2-fluoro-4-(hexadecylamino)benzoic acid in a mixture of dioxane and methylenechloride is added gaseous hydrogen chloride for 10 minutes. The slurry is cooled and 18 ml. of thionyl chloride added. The slurry is brought to reflux for 2 hours and then concentrated under vacuum (thrice diluting with dioxane each time). The final amber solution is diluted with 100 ml. of dioxane and this solution added to freshly prepared ethyl glycinate in 300 ml. of methylene chloride containing 1 g. of dimethylaminopyridine and 10 ml. of triethylamine. After 16 hours at room temperature the reaction is refluxed for 2 hours, cooled and filtered. The mother liquor is extracted with water and 10% hydrochloric acid. The solution is dried and concentrated in vacuo to an amber liquid. A sample is pre-absorbed on silica and eluted with ether. Evaporation of the eluate yields a solid which is recrystallized from acetonitrile to yield ethyl 2-fluoro-4-(hexadecylamino)hippurate as a white solid.

EXAMPLE 1437

N-[2-Fluoro-4-(hexadecylamino)benzoyl]glycine

A mixture of 26.4 g. of ethyl N-[2-fluoro-4-(hexadecylamino)benzoyl]glycinate, 110 ml. of 1N sodium hydroxide solution; and 100 ml. of ethanol is stirred at ambient temperature for 2 hours and then partially evaporated. The aqueous solution is washed with diethyl ether, acidified with 6N hydrochloric acid, and filtered. The white solid is dried in vacuo and recrystallized from acetone to yield N-[2-fluoro4-(hexadecylamino)benzoyl]glycine.

EXAMPLE 1438

2-Fluoro-4-(hexadecylamino)-N-(phenylsulfonyl)benzamide

A solution of 31.4 g. of benzenesulfonamide in 250 ml. of dry dimethylacetamide is added dropwise, with stirring and cooling, to a suspension of 5.5 g. of sodium hydride in 100 ml. of dry dimethylacetamide during 30

minutes at room temperature. Stirring is continued for 30 minutes. In the meantime, a mixture of 36.2 g. of 2-fluoro-4-(hexadecylamino)benzoic acid in 100 ml. of methylene chloride, 300 ml. of dimethoxyethane, and 40 ml. of thionyl chloride is refluxed for 1 hour and 15 minutes. The solution is evaporated and to the resulting oil residue is added, in one portion, the previously prepared mixture of sodium benzenesulfonamide in dimethylacetamide. The mixture is stirred for 30 minutes, without cooling, and then filtered. The filtrate is poured into 2 l. of water and 250 ml. of saturated sodium chloride solution. The product is collected by filtration and then dissolved in methylene chloride, the mixture is filtered through diatomaceous earth, and brine is added to break the emulsion. The layers are separated, the organic phase is dried and evaporated. The residue is crystallized from toluene to yield 2-fluoro-4-(hexadecylamino)-N-(phenylsulfonyl)benzamide.

EXAMPLE 1439

N-[3-Fluoro-4-(hexadecylamino)benzoyl]methanesulfonamide

A solution of 25.2 g. of 3-fluoro-4-(hexadecylamino)benzoyl chloride hydrochloride and 5.6 g. of methanesulfonamide in 250 ml. of pyridine is stirred under reflux for 2 hours and then concentrated _in vacuo_. The residue is partitioned between water and diethyl ether; the aqueous layer acidified with 1N HCl, and the organic layer separated, dried ($MgSO_4$), and evaporated. Crystallization of the residual white solid from 60% aqueous acetic acid and then from methylene chloridehexane affords N-[3-fluoro-4-(hexadecylamino)benzoyl]methanesulfonamide as a white solid.

EXAMPLE 1440

N-[3-Fluoro-4-(hexadecylamino)benzoyl]alanine

A solution of 4.75 g. of N-trifluoroacetyl-3-fluoro-4-(hexadecylamino)benzoyl chloride and 1.2 g. of triethylamine in 200 ml. of warm ether is treated with 1.55 g. alanine ethyl ester and refluxed for 24 hours. The hot solution is filtered, the residue is washed with hot ether, and the solution is evaporated. After treatment with

aqueous methanolic potassium carbonate, the product is precipitated by acidification, filtered, and dried. Crystallization from acetone yields the N-[3-fluoro-4-(hexadecylamino)benzoyl]alanine as a white, crystalline solid.

EXAMPLE 1441

N-[3-fluoro-4-(hexadecylamino)benzoyl]benzamide

One gram of a 50% oil dispersion of sodium hydride is washed with petroleum ether by decantation, dried, and suspended in 5 ml. of tetrahydrofuran. To this stirred mixture is added a solution of 2.42 g. of benzamide in 5 ml. of tetrahydrofuran in one portion. An initial hydrogen evolution is observed. While stirring (30 min.), the sodium hydride gradually disappears and a white, milky, turbid mixture forms. A solution of 0.9 g. of N-trifluoroacetyl-3-fluoro-4-(hexadecylamino)benzoyl chloride in 3 ml. of tetrahydrofuran is added dropwise during 5 minutes to the mixture. The whole milky mixture is stirred at room temperature under nitrogen for one hour. The mixture is then poured into water and extracted with ether. The ether extract is washed with water and brine and dried over sodium sulfate. Evporation of the solvent, affords a pale yellow solid. The solid is recrystallized from ether/acetonitrile (50/50) and then from acetonitrile to yield N-[3-fluoro-4-(hexadecylamino)benzoyl]benzamide.

EXAMPLE 1442

N-[2-Fluoro-4-(hexadecylamino)benzoyl]piperidine

To a warm solution of N-carbobenzoyloxy-[2-fluoro-4-(hexadeclyamino)]benzoyl chloride and 1.3 g. of triethylamine in 100 ml. ether is added 1.2 g. of piperidine. An immediate precipitate forms, the mixture is refluxed for one hour and then filtered. The solid is extracted several times with hot ether, and the ether is evaporated to yield a white solid. The solid is dissolved in tetrahydrofuran (100 ml.) and hydrogenated over 600 mg. 10% Pd-on-carbon at 50 psi. until hydrogen uptake stops. The catylist is filtered and the filtrate evaporated. The residue is crystallized from acetic acid to yield N-[2-fluoro-4-(hexadecylamino)-benzoyl]piperidine as a crystalline mass.

EXAMPLE 1443

N-(2,3-Dihydroxypropyl)-3-fluoro-4-(hexadecylamino)-benzamide

To a mixture containing 4.3 g. of 1-{3-fluoro-4-[N-(tert-butyloxycarbonyl)hexadecylamino]benzoyl}imidazole, 50 ml. of chloroform, and 50 ml. of 5N sodium hydroxide is added 1.1 g. of 3-amino-1,2-propanediol. The solution is vigorously stirred for 25 hours, the layers are separated, and the chloroform solution is washed once with 50 ml. of 1N sodium hydroxide. The solvent is evaporated and the residue is heated for 30 minutes at 40°C. in 50 ml. of anhydrous trifluoroacetic acid. The solvent is again evaporated and the oil is crystallized from acetone to yield light yellow crystals of N-(2,3-dihydroxypropyl)-3-fluoro-4-(hexadecylamino)benzamide.

TABLE XXXIV

These benzamides are prepared from the corresponding amines or amine derivatives by the methods shown in the Table. The requisite carboxylate esters, N-protected carbonyl chlorides, or N-protected carbonyl imidazoles are prepared by the methods of Examples 1403-1406 and 1436.

| Example No. | Method of Example | Compound |
|---|---|---|
| 1444 | 1437 | N-[2,5-Difluoro-4-(hexadecylamino)benzoyl]-2-methylalanine |
| 1445 | 1438 | 3-Chloro-4-(hexadecylamino)-N-(methylsulfonyl)benzamide |
| 1446 | 1438 | 2-Methyl-4-(hexadecylamino)-N-(p-tolylsulfonyl)benzamide |
| 1447 | 1436 | 3-[3-Methyl-4-(hexadecylamino)-benzoyl]-4-carbethoxythiazolidine |
| 1448 | 1437 | 3-[3-Methyl-4-(hexadecylamino) benzoyl]-4-carboxythiazolidine |
| 1449 | 1440 | N-[3-Methoxy-4-(hexadecylamino)-benzoyl]2-aminoethanesulfonic acid |

| | | |
|---|---|---|
| 1450 | 1441 | 3-Methoxy-4-hexadecylamino-N-acetylbenzamide |
| 1451 | 1441 | 3,5-Dichloro-4-(undecylamino)-N-(p-chlorobenozyl)benzamide |
| 1452 | 1435 | 2-Methoxy-4-(tetradecylamino)-N-allylbenzamide |
| 1453 | 1435 | 3,5-Dimethyl-4-(1-methylpentadecylamino)-N-propropargylbenzamide |
| 1454 | 1443 | 3,5-Dibromo-4-[3-(4-fluorophenyl)propylamino]-N-(2,3-dihydroxypropyl)benzamide |
| 1455 | 1442 | 1- 2-Methyl-4-[(11-cyclohexylundecyl)amino]benzoyl piperidine |
| 1456 | 1443 | 2-Chloro-4-(1-isopropyl-1-methyl-2-heptynylamino)-N-(2,3-dihydroxypropyl)benzamide |
| 1457 | 1439 | 3-Fluoro-4-(13,13-dimethyltetradecyl)amino-N-(methylsulfonyl)benzamide |
| 1458 | 1438 | 3,5-Difluoro-4-(4,14-pentadecadienylamino)-N-(phenylsulfonyl) benzamide |
| 1459 | 1439 | 3,5-Diiodo-4-(15,15-dimethylhexadecylamino)-N-(methylsulfonyl)benzamide |
| 1460 | 1437 | N-[3-Nitro-4-(15-methylhexadecylamino)benzoyl]glycine |
| 1461 | 1436 | 3-{2,3,5-Trifluoro-4-[3-(1,3-dimethylcyclohexyl)-2-propylamino]benzoyl-4}-carboethoxythiazolidine |
| 1462 | 1435 | 1-[2-Methyl-4-(3,7,11-trimethyldodecylamino)benzoyl]piperidine |
| 1463 | 1441 | 3-Methoxy-4-(6-methylhexadecylamino)-N-acetylbenzamide |
| 1464 | 1443 | 3-Chloro-4-(3,7,11-trimethyl-2,6,10-dodecatrienylamino)-N-(3-hydroxypropyl)benzamide |

| | | |
|---|---|---|
| 1465 | 1442 | 1-[3-Bromo-4-(1-ethyltetradecylamino)benzoyl]pyrrolidine |
| 1466 | 1438 | 2-Acetoxy-4-(4-chlorobenzylamino)-N-(p-tolylsulfonyl)benzamide |
| 1467 | 1435 | 1-(2,6-Difluoro-4-benzylaminobenzoyl)piperidine |
| 1468 | 1440 | N-[3,5-Difluoro-4-(4-methylbenzylamino)benzoyl]-2-aminoethanesulfonic acid |
| 1469 | 1435 | 1-[2-Fluoro-4-(2-thienylbutylamino)benzoyl]piperidine |
| 1470 | 1435 | 1-[3-Fluoro-4-(p-fluorophenethylamino)benzoyl]piperidine |

EXAMPLE 1471

Diethyl 2-fluoro-4-(hexadecylamino)benzoylmalonate

A solution of 26.6 g. of diethyl malonate and 10 ml. of 1,2-dimethoxyethane is added to a suspension of 4.0 g. of sodium hydride in 1,2-demethoxyethane under argon. A solution of 17.3 g. of 2-fluoro-4-(hexadecylamino)benzoyl chloride hydrochloride in 1,2-dimethoxyethane is then added. The reaction mixture is refluxed for 4.5 hours, cooled, poured on ice, acidified, and extracted with ether. The ether solution is washed with water and saturated sodium chloride solution, dried over anhydrous sodium sulfate and concentrated to dryness. Addition of a small amount of ethanol to the residue gives a solid which is filtered and discarded. The ethanol filtrate is concentrated and the residue is recrystallized from ether to yield diethyl 2-fluoro-4-(hexadecylamino)benzoylmalonate.

EXAMPLE 1472

tert-Butyl ethyl 3-fluoro-4-(hexadecylamino)benzoylmalonate

A solution of 28.0 g. of tert-butyl ethyl malonate in 10 ml. of 1,2-dimethoxyethane is added to a suspension of 4.0 g. of sodium hydride in 1,2-dimethoxyethane under argon. A solution of 17.3 g. of 3-fluoro-4-(hexadecyl-

amino)benzoyl chloride hydrochloride in 1,2-dimethoxyethane is then added. The reaction mixture is refluxed for 5 hours, cooled, poured on ice and extracted with ether. The ether solution is washed with water and saturated sodium chloride solution, dried over anhydrous sodium sulfate and concentrated to dryness. The residue is then recrystallized from ether to yield tert-butyl ethyl 3-fluoro-4-(hexadecylamino)benzoylmalonate.

EXAMPLE 1473

Ethyl 2-[2-fluoro-4-(hexadecylamino)benzoyl]acetoacetate

A solution of 21.6 g. of ethyl acetoacetate and 10 ml. of 1,2-dimethoxyethane is added to a suspension of 4.0 g. of sodium hydride in 1,2-dimethoxyethane under argon. A solution of 17.3 g. of 2-fluoro-4-(hexadecylamino)benzoyl chloride hydrochloride in 1,2-dimethoxyethane is then added. The reaction mixture is refluxed for 5 hours, cooled, poured on ice and extracted with ether. The ether solution is washed with water and saturated sodium chloride solution, dried over anhydrous sodium sulfate and concentrated to dryness. Recrystallization from ether affords ethyl 2-[2-fluoro-4-(hexadecylamino)benzoyl]acetoacetate as a white solid.

EXAMPLE 1474

Ethyl 3-fluoro-4-(hexadecylamino)benzoylmalonate

A solution of 3.0 g. of tert-butyl ethyl 3-fluoro-4-(hexadecylamino)benzoylmalonate 10 ml. of trifluoroacetic acid is warmed with stirring for 3 hours. The solution is poured onto ice and neutralized with potassium hydroxide. The resulting precipitate is collected by filtration, washed with water and dried. Recrystallization from chloroform affords ethyl 3-fluoro-4-(hexadecylamino)benzoylacetate.

EXAMPLE 1475

3-Fluoro-4-(hexadecylamino)benzoylacetic acid

Two grams of ethyl 3-fluoro-4-(hexadecylamino)-benzoylacetate is added to a solution of potassium hydroxide in 50 ml. of 1:9 water-ethanol The reaction mixture is stirred for 24 hours at room temperature. Careful neutralization with sulfuric acid gave a precipitate which is filtered, washed with water, and dried to yield 3-fluoro-4-(hexadecylamino)benzoylacetic acid.

EXAMPLE 1476

2'-Fluoro-4-(hexadecylamino)-2-(methylsulfinyl)acetophenone

To a solution of 5.8 g. of dimethyl sulfoxide, dried over sieves, and 50 ml. of tetrahydrofuran is slowly added 28 ml. of *n*-butyl lithium (2.42 M in hexane). To this mixture is added 10 g. of methyl 2-fluoro-4-(hexadecylamino)benzoate in 200 ml. of tetrahydrofuran. After two hours, the reaction mixture is poured onto ice, acidified with dilute hydrochloric acid and quickly extracted with chloroform. The chloroform extract is washed with water and saturated sodium chloride solution and dried over anhydrous sodium sulfate. Concentration affords a solid which is washed with 500 ml. of hot hexane, filtered while hot and then washed with hexane. The white solid is dried *in vacuo* to yield 2'-fluoro-4-(hexadecylamino)-2-(methylsulfinyl)-acetophenone.

EXAMPLE 1477

3'-Fluoro-4'-(hexadecylamino)-2-(phenylsulfonyl)acetophenone

A solution of 864 mg. of sodium hydride and 5.3 g. of methylphenylsulfone in 20 ml. of 1,2-dimethoxyethane is stirred at 60°C. for one hour under an atmosphere of argon. To this solution is then added a solution of 5.0 g. of methyl 3-fluoro-4-(hexadecylamino)benzoate and 50 ml. of tetrahydrofuran and the reaction mixture is stirred at 60°C. for 1.5 hours. The mixture is cooled, poured onto ice, acidified with dilute hydrochloric acid to pH 3 and then extracted with chloroform. The organic layer is separated, washed three times with water and saturated sodium chloride solution, dried over anhydrous sodium sulfate and then concentrated to dryness. The crude solid is chromatographed on silica gel, eluting with methylene chloride to yield 3'-fluoro-4'(hexadecylamino)-2-(phenylsulfonyl)acetophenone.

EXAMPLE 1478

3'-Fluoro-4'-(hexadecylamino)-2-(phenylsulfinyl)-acetophenone

To a solution of 6.2 g. of methylphenylsulfoxide, dried over sieves, and 50 ml. of tetrahydrofuran is slowly added 28 ml. of *n*-butyl lithium (2.42 M in hexane). To this mixture is added 10 g. of methyl 3-fluoro-4-(hexadecylamino)benzoate in 200 ml. of tetrahydrofuran. After two hours, the reaction mixture is poured onto ice. acidified with diluted hydrochloric acid and quickly extracted with chloroform. The chloroform layer is washed with water and saturated sodium chloride solution and dried over anhydrous sodium sulfate. Concentration affords a solid which is washed with 500 ml. of hot hexane, filtered while hot, and then washed with 50 ml. of hexane. The white solid is dried in vacuo yielding 3'-fluoro-4'-(hexadecylamino)-2-(phenylsulfinyl)acetophenone.

EXAMPLE 1479

3-[2-Fluoro-4-(hexadecylamino)benzoyl]-2,4-pentanedione

A solution of 28.4 g. of 2,4-pentanedione and 20 ml. of 1,2-dimethoxyethane is added to a suspension of 13.6 g. of sodium hydride in 220 ml. of 1,2-dimethoxyethane under argon. A solution of 28.7 g. of 3-fluoro-4-(hexadecylamino)benzoyl chloride hydrochloride in 1,2-dimethoxyethane is then added. The reaction mixture is stirred at room temperature for 12 hours, cooled, poured on ice and extracted with ether. The ether solution is washed with water and saturated sodium chloride solution, dried over anhydrous sodium sulfate and concentrated. The residue is then chromatographed over silica gel to yield 3-[2-fluoro-4-(hexadecylamino)benzoyl]-2,4-pentanedione.

EXAMPLE 1480

Methyl 3-[2-fluoro-4-(hexadecylamino)benzoyl]propionate

A mixture of 35 g. of 3-(2-fluoro-4-acetamidobenzoyl)propionic acid, 700 ml. of methanol and 1.4 ml. of concentrated sulfuric acid is refluxed for 76 hours. The solution is cooled to 35°C. and poured onto 7 g. of

anhydrous sodium acetate while stirring. The reaction mixture is stirred in an ice-bath. The solid is collected and washed with cold methanol to yield methyl 3-(2-fluoro-4-aminobenzoyl)propionate as a white solid. A mixture of this solid, 9.2 g. of hexadecyl bromide and 4.2 g. of potassium carbonate is stirred for 20 hours at 125°C. under nitrogen. The mixture is then cooled to 25°C. and 30 ml. of water is added. After stirring, the product is filtered and washed with water. Recrystallization from methanol affords methyl 3-[2-fluoro-4-(hexadecylamino)benzoyl]propionate as a white solid.

EXAMPLE 1481

3-[2-Fluoro-4-(hexadecylamino)benzoyl]propionic acid

A solution of 5.4 g. of methyl 3-[2-fluoro-4-(hexadecylamino)propionate is stirred with 5.4 g. of potassium hydroxide in 100 ml. of 95% ethanol for 3 hours at reflux. The reaction mixture is cooled, diluted with 50 ml. of ethanol and 100 ml. of water and neutralized with hydrochloric acid. The solution is cooled to room temperature and filtered. The white solid is washed with 50% aqueous ethanol and dried. The product is recrystallized from ethanol to yield 3-[2-fluoro-4(hexadecylamino)benzoyl]priopionic acid as a white crystalline solid.

Table XXXV

Some of these acetophenone derivatives are prepared by reactions of either acid chloride hydrochlorides obtained in the manner of Example 1403, or methyl esters, obtained in the manner of Example 1342, with appropriate anionic acylatable substrates. Others are obtained by alkylations of preformed acetophenones, in the manner of Example 1480, or by chemical transformations of various acetophenones, in the manner of Examples 1474, 1475 or 1481.

| Example No. | Method of Example | Compound |
|---|---|---|
| 1482 | 1471 | Diethyl 2,6-difluoro-4-(undecylamino)benzoylmalonate |

| | | |
|---|---|---|
| 1483 | 1473 | Ethyl 2,3.5-trifluoro-4-(tetradecylamino)benzoylacetoacetate |
| 1484 | 1474 | Ethyl 2-chloro-4-(1-methylpentadecylamino)benzoylacetate |
| 1485 | 1475 | 2-Chloro-4-(1-methylpentadecylamino)benzoylacetic acid |
| 1486 | 1472 | tert-Butyl ethyl 3-chloro-4-(3,7,11-trimethyldodecylamino)-benzoylmalonate |
| 1487 | 1477 | 3',5'-Dimethyl-4'-(15,15-dimethylhexadecylamino)-2-(p-tolylsulfonyl)acetophenone |
| 1488 | 1476 | 3',5'-Dichloro-4'-(1-methyl-6-heptenylamino)-2-(methylsulfinyl)acetophenone |
| 1489 | 1480 | Methyl 3-[3-methyl-4-(4-hexadecenylamino)benzoyl]propionate |
| 1490 | 1481 | 3-[3-Methyl-4-(4-hexadecenylamino)-benzoyl]propionic acid |
| 1491 | 1479 | 3-[3-Nitro-4-(9-octadecenylamino)-benzoyl]-2,4-pentanedione |
| 1492 | 1476 | 3'-Fluoro-4'-(4-chlorobenzylamino)-2-(methylsulfinyl)acetophenone |
| 1493 | 1474 | Ethyl 2-chloro-4-(11-cyclohexylundecylamino)benzoyl acetate |
| 1494 | 1479 | 3-[2,6-Difluoro-4-(4-decyloxybenzylamino)benzoyl]-2,4-pentanedione |
| 1495 | 1480 | Methyl 3-[2-methoxy-4-(4-methylbenzylamino)benzoyl]propionate |
| 1496 | 1481 | 3-[3,5-Dichloro-4-(cinnamylamino)-benzoyl]propionic acid |
| 1497 | 1477 | 3'-Fluoro-4'-[4-(2-thienyl)butylamino]-2-(phenylsulfonyl)acetophenone |
| 1498 | 1491 | Diethyl 3-bromo-4-[(4-cyclohexyl-2-butenyl)amino]benzoylmalonate |

| | | |
|---|---|---|
| 1499 | 1473 | Ethyl 2,3,5,6-tetrafluoro-4-[3-(3-trifluoromethylphenyl)propylamino]-benzoylmalonate |
| 1500 | 1472 | *tert*-Butyl ethyl 2-methoxy-4-(4-fluorobenzylamino)benzoylmalonate |
| 1501 | 1472 | tert-Butyl ethyl 3,5-dimethyl-4-[6-(2-furyl)hexylamino]benzoylmalonate |
| 1502 | 1474 | Ethyl 2,5-difluoro-4-[2-(1-naphthyl)ethylamino]benzoylacetate |
| 1503 | 1478 | 3',5'-Diiodo-4'-(cyclotetradecylamino)-2-(*p*-tolylsulfinyl)acetophenone |
| 1504 | 1481 | 4-[2-Fluoro-4-(tetradecylamino)-benzoyl]butyric acid |
| 1505 | 1476 | 2',3',6'-Trifluoro-4'-(3,7,11-trimethyldodecylamino)-2-(methylsulfinyl)acetophenone |
| 1506 | 1475 | 3-[4-(*tert*-butylbenzylamino)-2-fluoro]benzoylacetic acid |

## EXAMPLE 1507

Ethyl 2-fluoro-4-(hexadecylamino)phenylacetate

A solution of 8.2 g. or 2-fluoro-4-aminophenylacetic acid, 150 ml. of absolute ethanol, and 3 ml. of boron trifluoride etherate is heated to reflux for 15 hours. The solution is concentrated by distillation and then evaporated to dryness *in vacuo*. The residue is dissolved in ethyl ether, washed with aqueous sodium bicarbonate dried and evaporated to yield ethyl 2-fluoro-4-aminophenylacetate. A mixture of 5.0 g. of this amine, 9.4 g. of 1-bromohexadecane, 4.2 g. of anhydrous potassium carbonate and 40 ml. of hexamethylphosphoramide is heated at 80°C. for 7 hours. The mixture is then cooled, diluted with water, and extracted with ethyl ether. The ether extracts are washed with water, dried and evaporated. The residue is recrystallized from a mixture of chloroform and hexane, yielding ethyl 2-fluoro-4-(hexadecylamino)phenylacetate.

EXAMPLE 1508

2-Fluoro-4-(hexadecylamino)phenylacetic acid

A mixture of 6.0 g. of ethyl 2-fluoro-4-(hexadecylamino)phenylacetate, 7.0 g. of potassium hydroxide and 100 ml. of ethanol-water is heated to reflux for 4 hours. While hot, the mixture is adjusted to pH 7 with conc. hydrochloric acid. The mixture is diluted with water, cooled and filtered. Recrystallization of the precipitate yields 2-fluoro-4-(hexadecylamino)phenylacetic acid.

EXAMPLE 1509

Ethyl 3-fluoro-4-aminohydrocinnamate

A mixture of 5.0 g. of 3-fluoro-4-nitrocinnamic acid and 100 mg. of 10% palladium-on-carbon in 200 ml. of ethanol containing 5 drops of 5.5 N ethanolic hydrogen chloride is treated with hydrogen in a Parr apparatus at room temperature for 3 hours. The mixture is then filtered through Celite and the filtrate is concentrated, affording 3-fluoro-4-aminohydrocinnamic acid.

A solution of 3-fluoro-4-aminohydrocinnamic acid in 100 ml. of absolute ethanol containing 16 ml. of boron trifluoride etherate is heated to reflux for 48 hours. The solution is then cooled, poured into 5% aqueous sodium carbonate and extracted with methylene chloride. Evaporation of the organic extracts yields ethyl 3-fluoro-4-aminohydrocinnamate.

EXAMPLE 1510

3-Fluoro-4-(hexadecylamino)hydrocinnamic acid

In a manner directly analogous to that described in Example 1346, ethyl 3-fluoro-4-aminohydrocinnamate is alkylated with hexadecyl bromide to form ethyl 3-fluoro-4-(hexadecylamino)hydrocinnamate. Subsequently, in a manner directly analogous to that described in Example 1347, ethyl 3-fluoro-4-(hexadecylamino)hydrocinnamate is hydrolyzed to 3-fluoro-4(hexadecylamino)hydrocinnamic acid.

EXAMPLE 1511

2-Fluoro-4-(hexadecylamino)cinnamic acid

A mixture of ethyl 2-fluro-4-aminocinnamate, one

equivalent of 1-bromohexadecane and one equivalent of anhydrous potassium carbonate in hexamethylphosphoramide is heated for 20 hours at 60°C. The mixture is then cooled, diluted with water and extracted with ether. The combined ether extracts are dried, filtered and evaporated to provide ethyl 4-hexadecylaminocinnamate. The ester is hydrolyzed with sodium hydroxide in a 1:9 water·ethanol solution at steam bath temperatures for 10 hours. The hot solution is then acidified with acetic acid, cooled and filtered and the solid is washed with water. Recrystallization from chloroform yields 2-fluoro-4-(hexadecylamino)cinnamic acid.

EXAMPLE 1512

2-Fluoro-4-(hexadecylamino)phenylpropiolic acid

A sample of 50 g. of ethyl 2-fluoro-4-aminocinnamate is dissolved in 500 ml. of ethyl ether and a solution of 28 g. of trifluoroacetic anhydride in 30 ml. of ether is added dropwise. When the addition is complete, the reaction is allowed to stir for another hour. The mixture is then diluted with hexane and filtered, providing ethyl 2-fluoro-4-trifluoroacetamidocinnamate.

A solution of 40 g. of ethyl 2-fluoro-4-trifluoroacetamidocinnamate in 200 ml. of carbon tetrachloride is cooled in ice. Bromine (28 g.) is added dropwise, the reaction is allowed to stir for one additional hour and then the solvent is evaporated. The crystalline residue is the dibromo ester.

A solution of 11.4 g. of potassium hydroxide in 300 ml. of 95% ethanol is cooled to 40°C. and 20 g. of the crude dibromo ester above is added. After 30 minutes, the reaction is heated to reflux for five hours. The solution is then cooled and filtered. The filtrate is treated with acetic acid until the solution is neutral to litmus, then concentrated, chilled and filtered, to yield 2-fluro-4-aminophenylpropiolic acid.

Alternatively, the 2-fluoro-4-aminophenylpropiolic acid is prepared from 2-fluoro-4-nitrocinnamic acid by successive treatment with bromine in acetic acid, aqueous

sodium hydroxide, ferric sulfate and ammonium hydroxide, and ethanolic hydrogen chloride. The propiolic acid is then converted to the corresponding ethyl ester and alkylated with 1-bromohexadecane in the manner of Example 1346. The resulting ethyl 2-fluoro-4-(hexadecylamino)phenylpropiolate is hydrolyzed in the manner of Example 1347 to yield 2-fluoro-4-(hexadecylamino)phenylpropiolic acid.

EXAMPLE 1513

Preparation of 4-amino-2-cyanothiophene

A mixture of 15.4 g. of 2-cyano-4-nitrothiophene in 160 ml. of concentrated hydrochloric acid is cooled to 5°C. in an ice bath. To this mixture is added 12 g. of tin metal and the temperature rises to 12°C. When the temperature drops to 5°C. an additional 11.6 g. of tin metal is added. Stirring is continued until all the tin dissolves. At this time the reaction mixture is filtered and the solid washed with ether. The hydrochloride is obtained by recrystallization from methanol.

To 100 ml. of water is added 11.5 g. of 4-amino-2-cyanothiophene hydrochloride and this mixture is cooled in an ice bath. To the mixture is added 7.2 ml. of 10N sodium hydroxide dropwise. Upon stirring and continued cooling, crystalline product is obtained. The product is filtered and recrystallized from methylene chloride-hexane.

EXAMPLE 1514

Preparation of 4-hexadecylamino-2-cyanothiophene

To 13.6 g. 4-amino-2-cyanothiophene and 13.8 g. of potassium carbonate is added 50 ml. of hexamethylphosphoramide previously dried over molecular sieves. To this mixture is aded 31.4 g. of of 1-bromohexadecane and the reaction heated to 130°C. The temparature is maintained at 130° for 20 hours. After cooling, the reaction is poured into water and the solid filtered. The solid is dissolved in methylene chloride and this solution is then dried over sodium sulfate, filtered through Magnesol and treated with Darco. The filtrate is evaporated and the residue recrystallized from isopropanol to yield the product.

EXAMPLE 1515

Preparation of 4-hexadecylamino-2-formylthiophene

Di-*iso*butylaluminum hydride (14 ml., 25% solution in toluene) is added with stirring to a solution of 2.9 g. of 4-hexadecylamino-2-cyanothiophene in toluene under a nitrogen atmosphere. The temperature rises during the addition and the reaction is then stirred for 1 hour. A

solution of methanol in toluene (14 ml., 1:1) is added over 30 minutes and the mixture is poured into vigorously stirred ice-cold aqueous sulfuric acid (125 ml., 5%). After 10 minutes diatomaceous earth (7.5 g.) is added, the mixture filtered and the organic layer separated. The aqueous solution is extracted twice with toluene (25 ml.) and the combined organic layers are washed with aqueous sodium bicarbonate, dried over magnesium sulfate, decolorized with charcoal, filtered and evaporated in vacuo to give crude product. The product is obtained by recrystallization from dichloromethane-hexane.

EXAMPLE 1516

Preparation of 2-carbamyl-4-hexadecylaminothiophene

A mixture of 6.9 g. of 4-hexadecylamino-2-cyano-thiophene, 80 ml. of methyl cellosolve, and 80 ml. of 1N sodium hydroxide is refluxed for 21 hours. After cooling in an ice bath, the solid is filtered and thoroughly washed with methylene chloride. The solid is then added to 100 ml. of water and 5 ml. of concentrated hydrochloric acid and stirred for 8 hours. The solid is filtered and recrystallized from isopropanol.

EXAMPLE 1517

Preparation of 4-hexadecylamino-2-thiophenocarboxylic acid

A mixture of 4.4 g. of 2-carbamyl-4-hexadecylamino-thiophene, 50 ml. of methyl cellosolve, and 50 ml. of 6N hydrochloric acid is refluxed for 40 hours and then allowed to cool. The solid is filtered and recrystallized from methanol. The solid is then dissolved in hot sodium bicarbonate solution, filtered, and the filtrate is acidified with concentrated hydrochloric acid. The resulting precipitate is collected and recrystallized from isopropanol to yield the product.

EXAMPLE 1518

Preparation of ethyl 5-nitro-2-thiophenecarboxylate

To 160 ml. of acetic anhydride cooled to -5°C in an ice-salt bath is added 34 ml. of 90% nitric acid dropwise so that the temperature does not rise above 0° To this

mixture is added 62.7 g. of ethyl 2-thiophenecarboxylate dropwise so that the temperature remains below -5°. Reaction is stirred for 1 hour and poured onto ice. The product is filtered, dried, and recrystallized from hexane.

EXAMPLE 1519

Preparation of ethyl 5-amino-2-thiophenecarboxylate

A mixture of 60.4 g. of ethyl5-nitro-2-thiophenecarboxylate and 480 ml. of concentrated hydrochloric acid is cooled in an ice ice-salt bath to below -5°C. To this mixture is added 72 g. of granular tin in small amounts, keeping the temperature near -5°C. after stirring for 6 hours, 575 ml. of 10N sodium hydroxide is added to neutralize the mixture. Methylene chloride and Celite are added and the mixture is filtered. The methylene chloride layer of the filtrate is separated, dried over sodium sulfate, and filtered through Magnesol. Evaporation affords an oil which is distilled to yield the product.

EXAMPLE 1520

Preparation of ethyl 5-hexadecanamido-2-thiophenecarboxylate

To a solution of 8.6 g. of ethyl 5-amino-2-thiophenecarboxylate and 25 ml. of triethylamine in 100 ml. of methylene chloride is added dropwise 16.5 g. of palmitoyl chloride. The reaction mixture is stirred for 20 hours. Evaporation affords an oil. A methylene chloride solution of the oil is filtered and the filtrate passed through Magnesol. Removal of the solvent by evaporation yields a solid which is recrystallized from hexane. A second recrystallization from carbon tetrachloride affords the product.

EXAMPLE 1521

Preparation of ethyl 5-hexadecylamino-2-thiophenecarboxylate

To 1.1 g. of ethyl 5-hexadecanamido-2-thiophenecarboxylate is added 5 ml. 1M borane in tetrahydrofuran. The reaction mixture is refluxed for 1 hour. After cooling the reaction mixture is poured into 50 ml. of 1N hydrochloric acid. The solid is collected and then recrystallized from both carbon tetrachloride and ethanol to yield

the product.

EXAMPLE 1522

Preparation of 5-hexadecylamino-2-thiophenecarboxylic acid

A mixture of 0.1 g. of ethyl 5-hexadecylamino-2-thiophenecarboxylate, 10 ml. of absolute ethanol and 10 ml. of 0.5N sodium hydroxide is refluxed for 3 hours. The reaction mixture is cooled and then concentrated. The pH of the solution is adjusted to 5.3 and the resulting solid filtered and dried to yield the product.

EXAMPLE 1523

Preparation of ethyl 5-nitro-2-furancarboxylate

To a solution of 39 g. of 5-nitro-2-furancarboxylic acid in 200 ml. of ethanol is added 2 ml. of p-toluenesulfonic acid. The reaction is heated for 48 hours. The reaction mixture is evaporated and methylene chloride is added to the residue. The solution is extracted with 10% sodium bicarbonate and dried over anhydrous sodium sulfate. The solution is concentrated and then filtered through Magnesol. The product is obtained by recrystallization from methylene chloride and hexane.

EXAMPLE 1524

Preparation of ethyl 5-amino-2-furancarboxylate

A mixture of 53.3 g. of ethyl 5-nitro-2-furancarboxylate 5 g. of 10% palladium on carbon, and 250 ml. of absolute ethanol is shaken in an atmosphere of hydrogen until no more hydrogen is taken up. The catalyst is filtered and the filtrate evaporated. The thick oily residue is dissolved in a small amount of methylene chloride and the solution is filtered through Magnesol. The solvent is evaporated to yield the product.

EXAMPLE 1525

Preparation of ethyl 5-hexadecanamido-2-furancarboxylate

To a solution of 28.7 g. of ethyl 5-amino-2-furancarboxylate, 50 ml. of triethlamine and 300 ml. of methylene chloride is added 51.7 g. of palmitoyl chloride dropwise. The reaction mixture is allowed to stir for 48 hours. The solvent is evaporated and the residue dissolved in a small

amount of methylene chloride. The methylene chloride solution is filtered through Magnesol and concentrated to an oil. The product is obtained by crystallization of the residual oil from hexane.

EXAMPLE 1526

Preparation of ethyl 5-hexadecylamino-2-furancarboxylate

A mixture of 1.1 g of ethyl 5-hexadecanamido-2-furancarboxylate and 5 ml. of 1M borane in tetrahydrofuran is refluxed on the steam bath for 3 hours. After cooling, the reaction is poured into dilute hydrochloric acid and the resulting solid filtered. The solid is dissolved in methylene chloride and the solution is filtered through Magnesol. The solvent is evaporated to yield the product.

EXAMPLE 1527

Preparation of 5-hexadecylamino-2-furancarboxylic acid

A mixture of 2.0 g. of ethyl 5-hexadecylamino-2-furancarboxylate, 10 ml. of ethanol, and 2.0 ml. of 1N sodium hydroxide is stirred under reflux for 3 hours and then allowed to cool. The mixture is concentrated in vacuo, diluted with water, and adjusted to pH 5 with the dropwise addition of dilute hydrochloric acid. The solid is collected by filtration, washed with water, and dried to yield the product.

EXAMPLE 1528

Preparation of ethyl 1-methyl-4-nitro-2-pyrrolecarboxylate

A solution of 2.5 g. of sodium methoxide in 25 ml. of methanol is cooled to 10°C. and a mixture of 7.0 g. of ethyl 4-nitro-2-pyrrolecarboxylate in 15 ml. of methanol is added. After 10 minutes, 18.6 g. of methyl iodide is added. The dark-colored solution is warmed to 35°C. for 0.5 hours, then cooled and allowed to stir at room temperature for 16 hours. Chilling and filtration affords a solid which is collected by filtration and recrystallized from ethanol to yield the product.

EXAMPLE 1529

Preparation of ethyl 4-hexadecanamido-1-methyl-1-pyrrolecarboxylate

A solution of 1.5 g. of ethyl 1-methyl-4-nitro-1-pyrrolecarboxylate in 17 ml. of dry tetrahydrofuran is purged for 15 minutes with nitrogen. A catalytic quantity of 5% palladium on carbon is added and a slow stream of hydrogen gas is bubbled in. When the completion of the reaction is indicated by thin layer chromatography the system is again purged with nitrogen and solution is filtered through Celite. This filtrate is then added to a milky solution of 2.05 g. of palmitoyl chloride, 1 ml. of triethylamine and 10 ml. of dry tetrahydrofuran. The reaction is allowed to stir at 25°C. for 24 hours, diluted with water, and extracted with ether. The extract is dried over magnesium sulfate and evaporated to yield the product as a white solid.

EXAMPLE 1530

Preparation of ethyl 4-hexadecylamino-1-methyl-2-pyrrolecarboxylate

A solution of 1.0 g of ethyl 4-hexadecanamido-1-methyl-2-pyrrolecarboxylate in 10 ml. of dry tetrahydrofuran is added dropwise to a solution of 6 ml of 1M borane in tetrahydrofuran under nitrogen and the pale yellow solution is heated to reflux overnight. After cooling and acidification with 1N aqueous hydrochloric acid followed by neutralization with saturated sodium bicarbonate solution, the solution is extracted with ether. The extract is dried over anhydrous magnesium sulfate and evaporated. The residue is recrystallized from hexane to yield the product as a white solid.

EXAMPLE 1531

Preparation of 4-hexadecylamino-1-methyl-2-pyrrolecarboxylic acid

A mixture of 1.0 g. of ethyl 4-hexadecylamino-1-methyl-2-pyrrolecarboxylate and 10 ml. of 40% aqueous sulfuric acid is heated under reflux for 24 hours, allowed

to cool and neutralized with sodium acetate. The mixture is extracted with chloroform and the extract is dried over magnesium sulfate and evaporated. The residue is purified by chromatography on a silica gel column by elution with chloroform-acetic acid mixtures. Evaporation of the eluate affords the product.

EXAMPLE 1532

Preparation of 5-hexadecylamino-1-methyl-2-pyrrolecarboxylic acid

In a series of chemical reaction analogous to those described in Examples 1529-1531, methyl 1-methyl-5-nitropyrrolecarboxylate is converted to 5-hexadecylamino-1-methyl-2-pyrrolecarboxylic acid.

EXAMPLE 1533

Preparation of 2-hexadecylamino-5-pyrimidinecarboxylic acid hydrochloride

A mixture of 2.0 g of ethyl 2-methylthio-5-pyrimidinecarboxylate, 3.0 g. of hexadecylamine, and 20 ml. of hexamethylphosphoramide is heated at 110°C. for 10 hours, allowed to cool, and diluted with water. Filtration affords a solid which is crystallized from hexane to yield ethyl 2-hexadecylamino-5-pyrimidinecarboxylate as a tan solid.

The solid is added to a solution of 1.2 g. of potasium hydroxide in 50 ml. of 95% ethanol and the mixture is stirred under reflux for 3 hours, diluted with 50 ml. of water, treated with 1.0 ml. of concentrated hydrochloric acid. The warm mixture is then allowed to cool and the resulting precipitate is collected by filtration and dried to yield the product as a white solid.

EXAMPLE 1534

Preparation of 6-hexadecylamino-3-pyridinecarboxylic acid hydrochloride

A mixture of 5.13 g. of methyl 6-chloronicotinate, 7.23 g. of hexadecylamine and 150 ml. of hexamethylphosphoramide is stirred at 130°C for six hours. Cooling and dilution with water affords a precipitate which is collected by filtration and recystallized from ethyl acetate and then

from acetone to yield methyl 6-hexadecylamino-3-pyridinecarboxylate as a tan solid.

A mixture of the solid, 1.9 g. of potassium hydroxide, 5.0 ml. of water and 50 ml. of methanol is stirred under reflux for 3 hours, allowed to cool and filtered. The solid is warmed with 55 ml. of 10% hydrochloric acid for 2 hours. The mixture is chilled and filtered and the solid is recrystallized from acetone to yield the product as a white solid.

TABLE XXXVI

The heteroaryl carboxylic acids shown are prepared from the appropriate aminoheteroaryl compounds and alkyl halides, acyl halides, or alkyl amines using the methods of Examples 1513-1534 as listed in the table.

| Example No. | Methods of Examples | Compound |
|---|---|---|
| 1535 | 1513-1517 | 4-Hexylamino-2-thiophenecarboxylic acid |
| 1536 | 1513-1517 | 4-Octylamino-2-thiophenecarboxylic acid |
| 1537 | 1513-1517 | 4-Decylamino-2-thiophenecarboxylic acid |
| 1538 | 1513-1517 | 4-Undecylamino-2-thiophenecarboxylic acid |
| 1539 | 1513-1517 | 4-Tetradecylamino-2-thiophenecarboxylic acid |
| 1540 | 1513-1517 | 4-Octadecylamino-2-thiophenecarboxylic acid |
| 1541 | 1513-1517 | 4-(1-Methylpentadecylamino)-2-thiophenecarboxylic acid |
| 1542 | 1513-15]7 | 4-(15-Methylhexadecylamino)-2-thiophenecarboxylic acid |
| 1543 | 1513-1517 | 4-(13,13-Dimethyltetradecylamino)-2-thiophenecarboxylic acid |
| 1544 | 1513-1517 | 4-(15,15-Dimethylhexadecylamino)-2-thiophenecarboxylic acid |
| 1545 | 1513-1517 | 4-(10-Undecenylamino)-2-thiopehenecarboxylic acid |

| Example No. | Methods of Examples | Compound |
|---|---|---|
| 1546 | 1513-1517 | 4-(3,7-Dimethyl-6-octenylamino)-2-thiophenecarboxylic acid |
| 1547 | 1513-1517 | 4-(2,6,10-Trimethyl-11-dodecenylamino)-2-thiophenecarboxylic acid |
| 1548 | 1513-1517 | 4-(1-Methyl-6-heptenylamino)-2-thiophenecarboxylic acid |
| 1549 | 1513-1517 | 4-(1,1-Diisopropyl-2-propenylamino)-2-thiophenecarboxylic acid |
| 1550 | 1513-1517 | 4-(1,3-Dimethyl-1-ethyl-2-pentenylamino)-2-thiopenecarboxylic acid |
| 1551 | 1513-1517 | 4-(11-Hexadecynylamino)-2-thiophenecarboxylic acid |
| 1552 | 1513-1517 | 4-(6-Methyl-2-heptynylamino)-2-thiophenecarboxylic acid |
| 1553 | 1513-1517 | 4-(1-Isopropyl-1-methyl-2-heptynylamino)-2-thiophenecarboxylic acid |
| 1554 | 1513-1517 | 4-[3-(1,3-Dimethylcyclohexyl)-2-propylamino-2-thiophenecarboxylic acid |
| 1555 | 1513-1517 | 4-(13-Cyclopentyltridecylamino)-2-thiophenecarboxylic acid |
| 1556 | 1513-1517 | 4-(11-Cyclohexylundecylamino)-2-thiophenecarboxylic acid |
| 1557 | 1513-1517 | 4-(4-Cyclohexyl-2-butenylamino)-2-thiophenecarboxylic acid |
| 1558 | 1513-1517 | 4-(11-Phenylundecylamino)-2-thiophenecarboxylic acid |
| 1559 | 1513-1517 | 4-(4-Chlorobenzylamino)-2-thiophenecarboxlic acid |
| 1560 | 1513-1522 | 4-[3-(4-Fluorophenyl)propylamino-2-thiophenecarboxylic acid |

| | | |
|---|---|---|
| 1561 | 1513-1517 | 4-(4-Decyloxybenzylamino)-2-thiophenecarboxylic acid |
| 1562 | 1513-1517 | 4-(4-Methylbenzylamino)-2-thiophenecarboxylic acid |
| 1563 | 1517-1517 | 4-[3-(3-Trifluoromethyl)propylamino]-2-thiophenecarboxylic acid |
| 1564 | 1513-1517 | 4-Cinnamylamino-2-thiophenecarboxylic acid |
| 1565 | 1513-1517 | 4-[3-(2,4-Dichloropheneyl)propylamino]-2-thiophenecarboxylic acid |
| 1566 | 1513-1517 | 4-[3-(4-Benzyloxyphenyl)propylamino]-2-thiophenecarboxylic acid |
| 1567 | 1513-1517 | 4-[2-(1-Napthyl)ethylamino]-2-thiophenecarboxylic acid |
| 1568 | 1518-1522 | 5-Heptylamino-2-thiophenecarboxylic acid |
| 1569 | 1518-1522 | 5-Nonylamino-2-thiophenecarboxylic acid |
| 1570 | 1518-1522 | 5-Undecylamino-2-thiophenecarboxylic acid |
| 1571 | 1518-1522 | 5-Dodecylamino-2-thiophenecarboxylic acid |
| 1572 | 1518-1522 | 5-Tridecylamino-2-thiophenecarboxylic acid |
| 1573 | 1518-1522 | 5-Tetradecylamino-2-thiophenecarboxylic acid |
| 1574 | 1518-1522 | 5-Pentadecylamino-2-thiophenecarboxylic acid |
| 1575 | 1518-1522 | 5-Octadecylamino-2-thiophenecarboxylic acid |
| 1576 | 1518-1522 | 5-(3-Methylheptadecylamino)-2-thiophenecarboxylic acid |
| 1577 | 1518-1522 | 5-(15-Methylhexadecylamino)-2-thiophenecarboxylic acid |

| | | |
|---|---|---|
| 1578 | 1518-1522 | 5-(14-Methylpentadecylamino)-2-thiophenecarboxylic acid |
| 1579 | 1518-1522 | 5-(2,2,3-Trimethylnonylamino)-2-thiophenecarboxylic acid |
| 1580 | 1518-1522 | 5-(2-Methyloctylamino)-2-thiophenecarboxylic acid |
| 1581 | 1518-1522 | 5-(3,7-Dimethyl-7-octenylamino)-2-thiophenecarboxylic acid |
| 1582 | 1518-1522 | 5-(2-Tetradecenylamino)-2-thiophenecarboxylic acid |
| 1583 | 1518-1522 | 5-(6-Hexadecynylamino)-2-thiophenecarboxylic acid |
| 1584 | 1518-1522 | 5-(15-Hexadecenylamino)-2-thiophenecarboxylic acid |
| 1585 | 1518-1522 | 5-(11-Phenylundecylamino)-2-thiophenecarboxylic acid |
| 1586 | 1518-1522 | 5-[3-Isopropyl-3-(4-tert-butylphenyl)propylamino]-2-thiophenecarboxylic acid |
| 1587 | 1518-1522 | 5-[3-Ethyl-3-(4-tert-butylphenyl)propylamino]-2-thiophenecarboxylic acid |
| 1588 | 1518-1522 | 5-(11-Cyclopropylundecylamino)-2-thiophenecarboxylic acid |
| 1589 | 1518-1522 | 5-(8-Cyclopropyl-2-hexyloctylamino)-2-thiophenecarboxylic acid |
| 1590 | 1523-1527 | 5-Octylamino-2-furancarboxylic acid |
| 1591 | 1523-1527 | 5-Nonylamino-2-furancarboxylic acid |
| 1592 | 1523-1527 | 5-Decylamino-2-furancarboxylic acid |
| 1593 | 1523-1527 | 5-Undecylamino-2-furancarboxylic acid |
| 1594 | 1523-1527 | 5-Tridecylamino-2-furancarboxylic acid |

| | | |
|---|---|---|
| 1595 | 1523-1527 | 5-Tetradecylamino-2-furancarboxylic acid |
| 1596 | 1523-1527 | 5-Pentadecylamino-2-furancarboxylic acid |
| 1597 | 1523-1527 | 5-Heptadecylamino-2-furancarboxylic acid |
| 1598 | 1523-1527 | 5-Nonadecylamino-2-furancarboxylic acid |
| 1599 | 1523-1527 | 5-(2-Methyloctylamino)-2-furancarboxylic acid |
| 1600 | 1523-1527 | 5-(2,2,3-Trimethylnonylamino)-2-furancarboxylic acid |
| 1601 | 1523-1527 | 5-(14-Methylpentadecylamino)-2-furancarboxylic acid |
| 1602 | 1523-1527 | 5-(15-Methylhexadecylamino)-2-furancarboxylic acid |
| 1603 | 1523-1527 | 5-(3-Methylheptadecylamino)-2-furancarboxylic acid |
| 1604 | 1523-1527 | 5-(3,7-Dimethyl-7-octenylamino)-2-furancarboxylic acid |
| 1605 | 1523-1527 | 5-(2-Tetradecenylamino-2-furancarboxylic acid |
| 1606 | 1523-1527 | 5-(6-Hexadecynylamino)-2-furancarboxylic acid |
| 1607 | 1523-1527 | 5-(15-Hexadecenylamino)-2-furancarboxylic acid |
| 1608 | 1523-1527 | 5-(11-Phenylundecylamino)-2-furancarboxylic acid |
| 1609 | 1523-1527 | 5-[3-Isopropyl-3-(4-*tert*-butylphenyl)propylamino]-2-furancarboxylic acid |
| 1610 | 1523-1527 | 5-[3-Ethyl-3-(4-*tert*-butylphenyl)propylamino]-2-furancarboxylic acid |
| 1611 | 1523-1527 | 5-(11-Cyclopropylundecylamino)-2-furancarboxylic acid |

| | | |
|---|---|---|
| 1612 | 1523-1527 | 5-(8-Cyclopropyl-2-hexyloctyl-amino)-2-furancarboxylic acid |
| 1613 | 1528-1531 | 4-Hexylamino-1-methyl-2-pyrrole-carboxylic acid |
| 1614 | 1528-1531 | 4-Decylamino-1-methyl-2-pyrrole-carboxylic acid |
| 1615 | 1528-1531 | 4-Undecylamino-1-methyl-2-pyr-rolecarboxylic acid |
| 1616 | 1528-1531 | 4-Tetradecylamino-1-methyl-2-pyrrolecarboxylic acid |
| 1617 | 1528-1531 | 4-Pentadecylamino-1-methyl-2-pyrrolecarboxylic acid |
| 1618 | 1528-1531 | 4-Octadecylamino-1-methyl-2-pyrrolecarboxylic acid |
| 1619 | 1528-1531 | 4-(3-Methylheptadecylamino)-1-methyl-2-pyrrolecarboxylic acid |
| 1620 | 1528-1531 | 4-(14-Methylpentadecylamino)-1-methyl-2-pyrrolecarboxylic acid |
| 1621 | 1528-1531 | 4-(13,13-Dimethyltetradecyl-amino)-1-methyl-2-pyrrolecarbox-ylic acid |
| 1622 | 1528-1531 | 4-(10-Undecenylamino)-1-methyl-2-pyrrolecarboxylic acid |
| 1623 | 1528-1531 | 4-(1,1-Diisopropyl-2-propenyl-amino)-1-methyl-2-pyrrolecarb-oxylic acid |
| 1624 | 1528-1531 | 4-(11-Hexadecynylamino)-1-methyl-2-pyrrolecarboxylic acid |
| 1625 | 1528-1531 | 4-[3-(1,3-Dimethylcyclohexyl)-2-propylamino]-1-methyl-2-pyrrole-carboxylic acid |
| 1626 | 1528-1531 | 4-(13-Cyclopentyltridecylamino)-1-methyl-2-pyrrolecarboxylic acid |
| 1627 | 1528-1531 | 4-(11-Phenylundecylamino)-1-methyl-2-pyrrolecarboxylic acid |

| | | |
|---|---|---|
| 1628 | 1528-1531 | 4-[3-(4-Benzyloxyphenyl)propyl-amino]-1-methyl-2-pyrrolecarbox-ylic acid |
| 1629 | 1532 | 5-Heptylamino-1-methyl-2-pyr-rolecarboxylic acid |
| 1630 | 1532 | 5-Nonylamino-1-methyl-2-pyrrole-carboxylic acid |
| 1631 | 1532 | 5-Dodecylamino-1-methyl-2-pyr-rolecarboxylic acid |
| 1632 | 1532 | 2-Tridecylamino-1-methyl-2-pyr-rolecarboxylic acid |
| 1633 | 1532 | 5-Pentadecylamino-1-methyl-2-pyrrolecarboxylic acid |
| 1634 | 1532 | 5-Octadecylamino-1-methyl-2-pyr-rolecarboxylic |
| 1635 | 1532 | 5-(1-Methylpentadecylamino)-1-methyl-2-pyrrolecarboxylic acid |
| 1636 | 1532 | 5-(13,13-Dimethyltetradecyl-amino)-1-methyl-2-pyrrolecarbox-ylic acid |
| 1637 | 1532 | 5-(10-Undecencylamino)-1-methyl-2-pyrrolecarboxylic acid |
| 1638 | 1532 | 5-(6-Methyl-6-heptynylamino)-1-methyl-2-pyrrolecarboxylic acid |
| 1639 | 1532 | 5-(13-Cyclopentyltridecylamino)-1-methyl-2-pyrrolecarboxylic acid |
| 1640 | 1532 | 5-(11-Phenylundecylamino)-1-methyl-2-pyrolecarboxylic acid |
| 1641 | 1532 | 5-(4-Methylbenzylamino)-1-methyl-2-pyrrolecarboxylic acid |
| 1642 | 1533 | 2-Heptylamino-5-pyrimidinecar-boxylic acid |
| 1643 | 1533 | 2-Octylamino-5-pyrimidinecarb-oxylic acid |
| 1644 | 1533 | 2-Decylamino-5-pyrimidinecarb-oxylic acid |

| | | |
|---|---|---|
| 1645 | 1533 | 2-Tridecylamino-5-pyrimidine-carboxylic acid |
| 1646 | 1533 | 2-Tetradecylamino-5-pyrimidine-carboxylic acid |
| 1647 | 1533 | 2-Octadecylamino-5-pyrimidine-carboxylic acid |
| 1648 | 1533 | 2-(1-Methylpentadecylamino)-5-pyrimidinecarboxylic acid |
| 1649 | 1533 | 2-(15-Methylhexadecylamino)-5-pyrimidinecarboxylic acid |
| 1650 | 1533 | 2-(13,13-Dimethyltetradecyl-amino)-5-pyrimidinecarboxylic acid |
| 1651 | 1533 | 2-(10-Undecenylamion)-5-pyrim-idinecarboxylic acid |
| 1652 | 1533 | 2-(3,5-Dimethyl-6-octenylamino)-5-pyrimidinecarboxylic acid |
| 1653 | 1533 | 2-(6-Methyl-6-heptynylamino)-5-pyrimidinecarboxylic acid |
| 1654 | 1533 | 2-(13-Cyclopentyltridecylamino)-5-pyrimidinecarboxylic acid |
| 1655 | 1533 | 2-(11-Phenylundecylamino)-5-pyrimidinecarboxylic acid |
| 1656 | 1533 | 2-(4-Methylbenzylamino)-5-pyri-midinecarboxylic acid |
| 1657 | 1534 | 6-Hexylamino-3-pyridinecarbox-ylic acid |
| 1658 | 1533 | 6-Nonylamino-3-pyridinecarbox-ylic acid |
| 1659 | 1533 | 6-Undecylamino-3-pyridinecarbox-ylic acid |
| 1660 | 1534 | 6-Tetradecylamino-3-pyridine-carboxylic acid |
| 1661 | 1534 | 6-Pentadecylamino-3-pyridine-carboxylic acid |
| 1662 | 1534 | 6-(3-Methylheptadecylamino)-3-pyridinecarboxylic acid |

| | | |
|---|---|---|
| 1663 | 1534 | 6-(15-Methylhexadecylamino)-3-pyridinecarboxylic acid |
| 1664 | 1534 | 6-(14-Methylpentadecylamino)-3-pyridinecarboxylic acid |
| 1665 | 1534 | 6-(2,2,3-Trimethylnonylamino)-3-pyridiencarboxylic acid |
| 1666 | 1534 | 6-(1,1-Diisopropy)-2-(propenyl-amino)-3-pyridinecarboxylic acid |
| 1667 | 1534 | 6-(11-Hexadecynylamino)-3-pyridinecarboxylic acid |
| 1668 | 1534 | 6-[3-(1,3-Dimethylcyclohexyl)-2-propylamino]-3-pyridinecarboxylic acid |
| 1669 | 1534 | 6-(11-Phenylundecylamino)-3-pyridinecarboxylic acid |
| 1670 | 1534 | 6-[3-(4-Fluorophenyl)propyl-amino]-3-pyridinecarboxylic acid |
| 1671 | 1534 | 6-[3-(4-Benzyloxyphenyl)propyl-amino-3-pyridiencarboxylic acid |

## EXAMPLE 1672

Preparation of 4-hexadecylamino-2-thiophenecarbonyl chloride hydrochloride

A cold solution of 25 g. of 4-hexadecylamino-2-thiophenecarboxylic acid in 500 ml. of 1,2-dimethoxy ethanemethylene chloride (4:1) is prepared and dry hydrochloric acid is bubbled through the solution until no more precipitate forms. The solution is treated with 25 ml. thionyl chloride and refluxed until all of the precipitate has dissolved. The solvents are evaporated to yield the product as an orange semi-crystalline mass.

## EXAMPLE 1673

Preparation of 4-[N-(trifluoroacetyl)-N-(hexadecyl)-amino]-2-thiophenecarbonyl chloride

A stirred, ice-cold suspension of 9 g. 4-(hexadecylamino)-2-thiophenecarboxylic acid in 100 ml. of dimethoxyethane and 16 ml. of pyridine is treated with 18 ml. trifluoroacetic anhydride at 0°C. The solution is stirred

for 30 minutes at room temperature and then diluted with 300 ml. ether and 100 g. ice. After stirring vigorously for 15 minutes, the phases are separated. the ether solution is washed wiih brine, dried and evaporated to a white, amorphous solid.

To a solution of 9.2 g. of the above solid in 30 ml. methylene chloride and 0.5 ml. dimethylformamide is added 5.7 ml. thionyl chloride. After 20 hours at reflux, the solvents are evaporated to yield 4-[N-(trifluoroacetyl)-N-(hexadecyl)amino]-2-thiophenecarbonyl chloride as a light yellow, mobile oil.

EXAMPLE 1674

Preparation of 4-[N-(carbobenzyloxy)-N-(hexadecyl)amino]-2-thiophenecarbonyl chloride

To 15 g. 4-(hexadecylamino)-2-thiophenecarboxylic acid in 200 ml. warm chloroform is added a solution of 15 g. of sodium carbonate in 150 ml. water To the vigorously stirred solution is added 10 g. carbobenzyloxy chloride. After 2 hours stirring at 40°C. the organic layer is separated, washed three times with 1N hydrochloric acid, dried, and evaporated to yield an oil. The oil is dissolved in 300 ml. toluene, treated with 15 ml. thionyl chloride and the solution is refluxed for 5 hours. The solvents are evaporated and the residue is dissolved three times in toluene evaporating each time, ultimately to yield 4-[N-(carbobenzyloxy)-N-(hexadecyl)amino]-2-thiophenecarbonyl chloride as a viscous, orange oil.

EXAMPLE 1675

Preparation of 1-{4-[N-(*tert*-butyloxycarbonyl)-N-(hexadecyl)amino]-2-thiophenylcarbonyl} imidazole

A solution of 10 g. of 4-hexadecylamino-2-thiophenecarboxylic acid in 100 ml. dioxane is treated with 4.0 g. *tert*-butylazidoformate and 10 ml. pyridine. After stirring at room temperature for 18 hours, the protected amido-acid is precipitated from solution by the addition of 150 ml. water. The solid is collected, thoroughly dried, and dissolved in 200 ml. of a mixture consisting of methylene chloride/dimethoxyethane/pyridine (1:4:1). To this solution is added 5.4 g. 1,1'-carbonyldiimidazole. The solution is stirred overnight at room temperature and the solvents are evaporated to yield 1-{4-[N-(tert-butyloxycarbonyl)-N-(hexadecyl)amino]-2-thiophenecarbonyl}imidazole as an orange oil.

EXAMPLE 1676

Preparation of ethyl 4-hexadecylamino-2-thiophenecarboxylate

A solution of 7.20 g. of 4-(hexadecylamino)-2-thiophenecarboxylic acid in 25 ml. of hexamethylphosphoramide is added to a stirred mixture of 0.800 g. of sodium hydride (57% in mineral oil) and 25 ml. of hexamethylphosphoramide. The solution which forms after one hour is treated with 11.0 g. of ethyl iodide and is then stirred at 25°C. for 18 hours. Dilution with water followed by filtration affords a white solid which is crystallized from ethanol to yield ethyl 4-(hexadecylamino)-2-thiophenecarboxylate as a white solid.

EXAMPLE 1677

Preparation of methyl 4-hexadecylamino-2-thiophenecarboxylat

A solution of 50.5 g. of 4-(hexadecylamino)-2-thiophenecarboxylic acid and 34.4 ml. of boron triflouride etherate in 200 ml. of methanol is stirred under reflux for 44 hours, allowed to cool, and poured into 1.2 liters of ice-cold 5% aqueous sodium carbonate solution. The white solid is collected by filtration and recrystallized from benzene-ethanol to yield methyl 4-(hexadecylamino)-2-thiophenecarboxylate as a white solid.

EXAMPLE 1678

Preparation of 2,3-dihydroxypropyl 4-hexadecylamino-2-thiophenecarboxylate

A solution of 7.34 g. of 4-(hexadecylamino)-2-thiophenecarboxylic acid, 4.80 g. of 25% aqueous sodium hydroxide, and 12.6 g. of 3-iodo-1,2-propanediol in 50 ml. of hexamethylphosphoramide is stirred for 24 hours at ambient temperature, diluted with 100 ml. of ether and

stirred for 5 days at ambient temperature. The mixture is treated with water and extracted with ether. The dried extracts are evaporated to yield 2,3-dihydroxypropyl 4-(hexadecylamino)-2-thiophenecarboxylate as a white solid.

EXAMPLE 1679

Preparation of 2-ethoxyethyl 4-hexadecylamino-2-thiophenecarboxylate

A solution of 11.8 g. of 4-(hexadecylamino)-2-thiophenecarboxylic acid, 1.00 g. of 2-ethoxyethanol and 5.35 ml. of boron trifluoride etherate in 200 ml. of toluene is stirred under reflux for 48 hours. The solution is treated with an additional 5.35 ml. of boron trifluoride etherate and refluxing is continued for 120 hours. Dilution with water and methylene chloride followed by filtration affords 2-ethoxyethyl 4-(hexadecylamino)-2-thiophenecarboxylate as a white solid.

EXAMPLE 1680

Preparation of 1-(methoxycarbonyl)propyl 4-hexadecylamino-2-thiophenecarboxylate

To a solution of 10 g. of 4-hexadecylamino-2-thiophenecarbonyl chloride hydrochloride in 200 ml. methylene chloride is added dropwise a solution of 3 g. methyl 2-hydroxybutyrate and 5 g. triethylamine in 100 ml. ether. After 17 hours stirring at room temperature, the precipitate is collected and washed with several portions of ether. The ether solution is washed with water, dried and evaporated to yield 1-(methoxycarbonyl)propyl 4-(hexadecylamino)-2-thiophenecarboxylate as a white solid.

EXAMPLE 1681

Preparation of diethyl O-(4-hexadecylamino-2-thiophenecarbonyl)tartrate

A mixture of 4-[N-(trifluoroacetyl)-N-(hexadecyl)amino]-2-thiophenecarbonyl chloride and 1.2 g. of triethylamine in 100 ml of warm ether is treated with 2.5 g. of diethyl tartrate and refluxed for 24 hours. The hot solution is filtered, the residue is washed with hot ether, and the solution is evaporated. After treatment with

aqueous methanolic potassium carbonate the product is precipitated by acidification, filtered, and dried. Crystallization from acetone yields the product as a white solid.

EXAMPLE 1682

Preparation of O-(4-hexadecylamino-2-thiophene-carbonyl)-malic acid

A warm solution of 4-[N-(carbobenzyloxy)-N-(hexadecyl)amino]-2-thiophenecarbonyl chloride and 1.3 g. of triethylamine in 100 ml. ether is treated with 2 g. malic acid An immediate precipitate forms, but the mixture is refluxed for one hour and filtered while hot. The solid is washed several times with hot ether; then the ether is evaporated to yield a white solid. The product is dissolved in tetrahydrofuran (100 ml.) and hydrogenated over 600 mg. 10% palladium-on-carbon at 50 psi until hydrogen uptake stops. The catalyst is filtered and the solution is evaporated. The residue is crystallized from acetic acid to yield tb product as a white solid.

EXAMPLE 1683

Preparation of 2-(ethoxycarbonyl)vinyl 4-hexadecylamino-2-thiophenecarboxylate

To a mixture of 4.3 g. of 1- 4-[N-(tert-butyloxycarbonyl)-N-(hexadecyl)amino]-2-thiophenecarbonyl)imidazole and 50 ml. of 5N sodium hydroxide is added 3 g 2-ethyl formyl acetate. The solution is vigorously stirred for 24 hours. The layers are separated, and the chloroform solution is washed once with 50 ml. 1N sodium hydroxide. The solvent is evaporated and the residue is heated for 30 minutes at 40°C. in 50 ml. anhydrous trifluoroacetic acid. The solvent is again evaporated and the oil is crystallized from acetone to yield the product as a light yellow solid

TABLE XXXVII

| Example No. | Method of Example | Compound |
|---|---|---|
| 1684 | 1676 | Ethyl 4-undecylamino-2-thiophenecarboxylate |
| 1685 | 1677 | Methyl 4-(15-methylpentadecylamino)-2-thiophenecarboxylate |

| | | |
|---|---|---|
| 1686 | 1678 | 2,3-Dihydroxypropyl 4-(10-undecenylamino)-2-thiophenecarboxylate |
| 1687 | 1679 | 2-Ethoxyethyl 4-(11-hexadecynylamino)-2-thiophenecarboxylate |
| 1688 | 1680 | 1-(Carbomethoxy)propyl 4-(13-cyclopentyltridecylamino)-2-thiophenecarboxylate |
| 1689 | 1681 | Diethyl O- 4-[3-(1,3-dimethylcyclohexyl propylamino]-2-thiophenecarboxyl tartrate |
| 1690 | 1682 | O-[4-(11-Phenylundecylamino)-2-thiophenecarbonyl]malic acid |
| 1691 | 1683 | 2-(Carboethoxy)vinyl 4-(4-methylbenzylamino)-2-thiophenecarboxylate |
| 1692 | 1676 | Ethyl 4-(8-cyclopropyl-2-hexyloctylamino)-2-thiophenecarboxylate |
| 1693 | 1676 | Isopropyl 5-(15-hexadecenylamino)-2-thiophenecarboxylate |
| 1694 | 1677 | Methyl 5-(2,2,3-trimethylnonylamino)-2-thiophenecarboxylate |
| 1695 | 1678 | 3-Hydroxypropyl 5-pentadecylamino-2-thiophenecarboxylate |
| 1696 | 1679 | 2-Ethoxyethyl 5-octylamino-2-thiophenecarboxylate |
| 1697 | 1680 | 1-(Carboethoxy)ethyl 5-(3-methylheptadecylamino)-2-thiophenecarboxylate |
| 1698 | 1681 | Diethyl O-(5-hexadecylamino-2-thiophenecarbonyl)tartrate |
| 1699 | 1682 | O-[5-(6-Hexadecynylaino)-2-thiophenecarbonyl]malic acid |
| 1700 | 1683 | 2-(Carboethoxy)vinyl 5-(2-methyloctylamino)-2-thiophenecarboxylate |
| 1701 | 1676 | 3-Hydroxypropyl 5-hexadecylamino-2-furancarboxylate |

| | | |
|---|---|---|
| 1702 | 1676 | Methyl 5-undecylamino-2-furan-carboxylate |
| 1703 | 1678 | 2,3-Dihydroxypropyl 5-(11-phenylundecylamino)-2-furancarboxylate |
| 1704 | 1678 | 2-Ethoxyethyl 5-[3-isopropyl-3-(4-tert-butylphenyl)propyl-amino]-2-furancarboxylate |
| 1705 | 1681 | 1-(Carbomethoxy)propyl 5-(3 7-dimethyl-7-octenylamino)-2-furancarboxylate |
| 1706 | 1681 | Diethyl O-[5-(14-methylpentadecylamino)-2-furancarbonyl)-tartrate |
| 1707 | 1682 | O-[5-(2-Tetradecenylamino)-2-furancarbonyl)malic acid |
| 1708 | 1682 | 2-(Carboethoxy)vinyl 5-(15-hexadecenylamino)-2-furancarboxylate |
| 1709 | 1681 | 2-(Carboethoxy)vinyl 5-octyl-amino-2-furancarboxylate |
| 1710 | 1676 | Methyl 4-tetradecyl-1-methyl-2-pyrrolecarboxylate |
| 1711 | 1677 | Methyl 4-(13,13-dimethyltetradecylamino)-1-methyl-2-pyrrole-carboxylate |
| 1712 | 1678 | 2,3-Dihydroxypropyl 4-(11-hexadecynylamino)-1-methyl-2-pyrrolecarboxylate |
| 1713 | 1679 | 2-Ethoxyethyl 4-[3-(1,3-dimethylcyclohexyl)-2-propylamino]-1-methyl-2-pyrrolecarboxylate |
| 1714 | 1680 | Carbomethoxymethyl 4-[3-(4-benzyloxyphenyl)propylamino]-2-methyl-2-pyrrolecarboxylate |
| 1715 | 1681 | Diethyl O-[4-(13-cyclopentyl-tridecylamino)-1-methyl-2-pyrrolecarbonyltartrate |
| 1716 | 1682 | O-[4-(10-Undecenylamino)-1-methyl-2-pyrrolecarbonyl]malic acid |

| | | |
|---|---|---|
| 1717 | 1683 | 2-(Carboethoxy)vinyl 4-(hexadecylamino)-1-methyl-2-pyrrolecarboxylate |
| 1718 | 1676 | Methyl 5-pentadecyl-1-methyl-2-pyrrolecarboxylate |
| 1719 | 1677 | Methyl 5-(6-methyl-6-heptynylamino-1-methyl-2-pyrrolecarboxylate |
| 1720 | 1678 | 2,3-Dihydroxypropyl 5-hexadecylamino 1-methyl-2-pyrrolecarboxylate |
| 1721 | 1679 | 2-Ethoxyethyl 5-(10-undecnylamino)-1-methyl-2-pyrrolecarboxylate |
| 1722 | 1680 | 1-(Carbomethoxy)ethyl 5-(13-cyclopentyltridecylamino)-1-methyl-2-pyrrolecarboxylate |
| 1723 | 1681 | Diethyl O-[5-(13,13-dimethyltetradecylamino)-1-methyl-2-pyrrolecarbonyl]tartrate |
| 1724 | 1682 | O-[5-(4-Methylbenzyl)-1-methyl-2-pyrrolecarbonyl]malic acid |
| 1425 | 1683 | 2-(Carboethoxy)vinyl 5-hexadecylamino-1-methyl-2-pyrrolecarboxylate |
| 1726 | 1677 | Methyl 2-hexadecylamino-5-pyrimidinecarboxylate |
| 1727 | 1677 | Methyl 2-tetradecylamino-5-pyrimidinecarboxylate |
| 1728 | 1677 | 2,3-Dihydroxypropyl 6-pentadecylamino-3-pyridinecarboxylate |
| 1729 | 1677 | 2,3-Dihydroxypropyl 2-(11-phenylundecylamino)-5-pyrimidinecarboxylate |
| 1730 | 1679 | 2-Ethoxyethyl 6-(15-methylpentadecylamino)-3-pyridinecarboxylate |
| 1731 | 1680 | 1-(Carbomethoxy)ethyl 6-(2,2,3-trimethylnonylamino)-3-pyridinecarboxylate |

| | | |
|---|---|---|
| 1732 | 1681 | Diethyl O-{6-[3-(1,3-dimethylcyclohexyl)-2-propylamino]-3-pyridinecarbonyl}tartrate |
| 1733 | 1682 | O-[2-(6-Methyl-6-heptynylamino)-5-pyrimidinecarbonyl]malic acid |
| 1734 | 1683 | 2-(Carboethoxy)vinyl 6-[3-(4-fluorophenyl)proxylamino]-3-pyridinecarboxylate |
| 1735 | 1677 | 2,3-Dihydroxypropyl 6-(10-undecenylamino)-3-pyridinecarboxylate |
| 1736 | 1679 | 2,3-Dihydroxypropyl 2-(4-methylbenzylamino)-5-pyrimidinecarboxylate |

## EXAMPLE 1737

### Preparation of 1-(4-hexadecylamino-2-thiophenecarbonyl)-piperidine

To a chilled solution of 35 ml. of piperidine, 2.5 ml. of triethylamine and 0.6 g. of dimethylaminopyridine in 100 ml. of diethyl ether is added (1/2 hour) a solution of 8.3 g. of 4-hexadecylamino-2-thiophenecarbonyl chloride hydrochloride in 50 ml. of ether. The solution is warmed to room temperature and maintained there for two hours. The solution is heated to reflux for an additional 2 hours at which time the reaction is complete. The solution is cooled, extracted twice with water and dried. The solvent is removed *in vacuo* and the solid is recrystallized from ether to yield the product as a white solid.

## EXAMPLE 1738

### Ethyl N-(4-hexadecylamino-2-thiophenecarbonyl)glycinate

To a solution of 18 g. of 4-hexadecylamino-2-thiophenecarboxylic acid in dioxane-methylene chloride (1:1) is added gaseous hydrogen chloride for 10 minutes. The slurry is cooled and 18 ml. of thionyl chloride added. The slurry is brought to reflux for 2 hours and then concentrated under vacuum (thrice diluting with dioxane each time). The final amber solution is diluted with 100 ml. of dioxane and this solution added to freshly prepared ethyl glycinate in 300 ml. of methylene chloride containing 1 g. of dimethylaminopyridine and 10 ml. of triethylamine. After

16 hours at room temperature the reaction is refluxed for 2 hours, cooled and filtered. The mother liquor is extracted with water and 10% hydrochloric acid. The solution is dried and concentrated in vacuo to an amber liquid. A sample is pre-absorbed on silica and eluted with ether. Evaporation of the eluate yields a solid which is recrystallized from acetonitrile to yield the product as a white solid.

EXAMPLE 1739

Preparation of N-(4-hexadecylamino-2-thiophenecarbonyl)-glycine

A mixture of 26 g. of ethyl N-(4-hexadecylamino-2-thiophenecarbonyl)glycinate, 110 ml. 1N sodium hydroxide solution; and 100 ml. of ethanol is stirred at ambient temperature for 2 hours and then partially evaporated. The aqueous solution is washed with diethyl ether, acidified with 6N hydrochloric acid, and filtered. The white solid is dried in vacuo and recrystallized from acetone to yield the product as a white solid.

EXAMPLE 1740

Preparation of 4-(hexadecylamino)-N-(phenylsulfonyl)-2-thiophenecarboxamide

A solution of 31.4 g. of benzenesulfonamide in 250 ml. of dry dimethylacetamide is added dropwise, with stirring and cooling, to a suspension of 5.5 g. of sodium hydride in 100 ml. of dry dimethylacetamide during 30 minutes at room temperature. Stirring is continued for 30 minutes. In the meantime, a mixture of 36.2 g of 4-(hexadecylamino)-2-thiophenecarboxylic acid in 100 ml. of methylene chloride, 300 ml. of dimethoxyethane, and 40 ml. of thionyl chloride is refluxed for 1 hour and 15 minutes. The solution is evaporated and to the resulting oil residue is added, in one portion, the previously prepared mixture of sodium benzenesulfonamide in dimethylacetamide. The mixture is stirred for 30 minutes, without cooling and then filtered. The filtrate is poured into 2 l. of water and 250 ml. of saturated sodium chloride solution. The

product is collected by filtration and then dissolved in methylene chloride, the mixture is filtered through diatomaceous earth, and brine is added to break the emulsion. The layers are separated, the organic phase is dried and evaporated. The residue is crystallized from toluene to yield the product as a white solid.

EXAMPLE 1741

Preparation of N-(4-hexadecylamino-2-thiophenecarbonyl)methanesulfonamide

A solution of 25. g. of 4-hexadecylamino-2-thiophenecarbonyl chloride hydrochloride and 5.6 g. of methanesulfonamide in 250 ml. of pyridine is stirred under reflux for 2 hours and then concentrated in vacuo. The residue is partitioned between water and diethyl ether the aqueous layer acidified with 1N hydrochloric acid and the organic layer separated, dried over magnesium sulfate, and evaporated. Crystallization of the residual white solid from 60% aqueous acetic acid and then from methylene chloride-hexane affords the product as a white solid.

EXAMPLE 1742

Preparation of N-(4hexadecylamino-2-thiophenecarbonyl)-benzamide

One gram of a 50% oil dispersion of sodium hydride is washed with petroleum ether by decantation, dried, and suspended in 5 ml. of tetrahydrofuran. To this stirred mixture is added a solution of 2.42 g. of benzamide in 5 ml. of tetrahydrofuran in one portion. An initial hydrogen evolution is observed. While stirring (30 minutes), the sodium hydride gradually disappears and a white, milky turbid mixture forms. A solution of 0.9 g. of 4-[N-(trifluoroacetyl)-4-(hexadecyl)amino]-2-thiophenecarbonyl chloride in 3 ml. of tetrahydrofuran is added dropwise during 5 minutes to the mixture. The whole milky mixture is stirred at room temperature under nitrogen for one hour. The mixture is then poured into water and extracted with ether. The ether extract is washed with water and brine and dried over sodium sulfate Evaporation of the solvent

affords a pale yellow solid. The solid is recrystallized from ether/ acetonitrile (50/50) and then from acetonitrile to yield the product as a white solid.

EXAMPLE 1743

Preparation of N-(4-hexadecylamino-2-thiophenecarbonyl)-pyrrolidine

A warm solution of 1.0 g. of 4-[N-(carbobenzyloxy)N-hexadecylamino]-2-thiophenecarbonyl chloride and 1.3 g. of triethylamine in 100 ml. of ether is added to 1.2 g. of pyrrolidine. A precipitate forms the mixture is stirred under reflux for 1 hour, filtered, and extracted with hot ether. The extract is evaporated and a solution of the resulting residue in 100 ml. of tetrahydrofuran is shaken under 50 psi of hydrogen in the presence of 0.6 g. of 10% palladium on carbon. The mixture is filtered and the filtrate evaporated. The residue is crystallized from acetic acid to yield the product as a white solid.

EXAMPLE 1744

Preparation of N-(2,3-dihydroxypropyl)-4-hexadecylamino-2-thiophenecarboxamide

To a mixture containing 4.1 g. of 1-[4-[N-(*tert*-butyloxycarbonyl)-N-(hexadecyl)amino]-2-thiophenecarbonyl]-imidazole 50 ml. of chloroform, and 50 ml. of 1N sodium hydroxide is added 1.1 g. of 3-amino-1,2-propanediol. The solution is vigorously stirred for 24 hours, the layers are separated and the chloroform solution is washed once with 50 ml. of 1N sodium hydroxide. The solvent is evaporated and the residue is heated for 30 minutes at 40°C. in 50 ml. of anhydrous trifluoroacetic acid The solvent is again evaporated and the oil is crystallized from acetone to yield light yellow crystals of N-(2,3-dihydroxypropyl)-4-hexadecylamino-2-thio-phenecarboxamide.

Table XXXVIII

The heteroaryl carboxamides shown are prepared from the corresponding carboxylic acids (Table XXXVI) by the methods of Examples 1737-1744 as listed in the table. The requisite carboxylic acid derivatives are prepared by the

methods of Examples 1672-1675.

| Example No. | Method of Example | Compound |
|---|---|---|
| 1745 | 1737 | 1-[4-(13,13-Dimethyltetradecylamino)-2-thiophenecarbonyl]piperidine |
| 1746 | 1738 | Ethyl N-[4-(10-undecenylamino)-2-thiophenecarbonyl]glycinate |
| 1747 | 1739 | N-[4-(10-Undecenylamino)-2-thiophenecarbonyl]glycine |
| 1748 | 1740 | 4-(11-Cyclohexylundecylamino)-N-(phenylsulfonyl)-2-thiophenecarboxamide |
| 1749 | 1741 | N-[4-(6-Methyl-2-heptynylamino)-2-thiophenecarbonyl]methanesulfonamide |
| 1750 | 1742 | N-[4-(11-Phenylundecylamino)-2-thiophenecarbonyl]benzamide |
| 1751 | 1743 | 1-[4-(4-Methylbenzylamino)-2-thiophenecarbonyl]pyrrolidine |
| 1752 | 1744 | N-(2,3-Dihydroxyproply)-4-[2-(1-naphthyl)ethylamino]-2-thiophenecarboxamide |
| 1753 | 1737 | 1-[5-(15-Hexadecenylamino)-2-thiophenecarbonyl]pyrrolidine |
| 1754 | 1738 | Ethyl N-[5-(2,2,3-trimethylnonylamino)-2-thiophenecarbonyl]-glycinate |
| 1755 | 1738 | Ethyl N-(5-hexadecylamino-2-thiophenecarbonyl)glycinate |
| 1756 | 1739 | N-(5-Hexadecylamino-2-thiophenecarbonyl)glycine |
| 1757 | 1740 | 5-(11-Phenylundecyl)-N-(phenylsulfonyl)-2-thiophenecarboxamide |
| 1758 | 1741 | N-[5-(15-Hexadecenylamino)-2-thiophenecarbonyl]methanesulofonamide |

| | | |
|---|---|---|
| 1759 | 1742 | N-[5-(8-Cyclopropyl-2-hexyl-octylamino)-2-thiophenecarbonyl]benzamide |
| 1760 | 1743 | 1-[5-[3-Ethyl-3-(4-*tert*-butyl-phenyl)propylamino]-2-thiophenecarbonyl]pyrrolidine |
| 1761 | 1744 | 5-[3-Isopropyl-3-(4-*tert*-butylphenyl)propylamino]-N-(2,-3-dihydroxypropyl)-2-thiophenecarboxamide |
| 1762 | 1742 | 1-(5-Octylamino-2-furancarbonyl)-piperidine |
| 1763 | 1738 | Ethyl N-(5-hexadecylamino-2-furancarbonyl)glycinate |
| 1764 | 1743 | N-(5-Hexadecylamino-2-furan-carbonyl)glycine |
| 1765 | 1742 | 5-(15-Hexadecenyl)-N-(phenyl-sulfonyl)-2-furancarboxamide |
| 1766 | 1742 | N-[5-(11-Phenylundecylamino)-2-furancarbonyl]benzamide |
| 1767 | 1743 | 1-[5-(8-Cyclopropyl-2-hexyloctylamino)-2-furancarbonyl]piperdine |
| 1768 | 1737 | 1-(4-Hexadecylamino-1-methyl-2-pyrrolecarbonyl)piperidine |
| 1769 | 1738 | Ethyl N-[5-(10-undecenylamino)-1-methyl -2-pyrrolecarbonyl]glycinate |
| 1770 | 1739 | N-[5-(10-Undecenylamino)-1-methyl-2-pyrrolocarbonyl]glycine |
| 1771 | 1740 | 4-(14-Methylpentadecylamino)-1-meth-yl-N-(phenysulfonyl)-2-pyrrolecarboxamide |
| 1772 | 1741 | N-(5-Hexadecylamino-1-methyl-2-pyrrolecarbonyl)methanesulfonamide |
| 1773 | 1742 | N-[5-(11-Phenylundecylamino)-1-methyl-2-pyrrolecarbonyl]-benzamide |

| | | |
|---|---|---|
| 1774 | 1743 | 1-(4-Hexylamino-2-methyl-2-pyrrolecarbonyl)pyrrolidine |
| 1775 | 1744 | 5-(11-Hexadecynylamino)-1-methyl-N-(2,3-dihydroxypropyl)-2-pyrrolecarboxamide |
| 1776 | 1737 | 1-[2-(15-Methylhexadecylamino)-5-pyrimidinecarbonyl]pyrrolidine |
| 1777 | 1738 | Ethyl N-(2-hexadecylamino-5-pyrimidinecarbonyl)glycinate |
| 1778 | 1739 | N-(2-Hexadecylamino-5-pyridinecarbonyl)glycine |
| 1779 | 1740 | 6-(11-Phenylundecylamino)-N-(phenylsulfonyl)-3-pyridinecarboxamide |
| 1780 | 1741 | N-(6-Octylamino-3-pyridinecarbonyl)methanesulfonamide |
| 1781 | 1742 | N-[6-(11-Hexadecynylamino)-3-pyridinecarbonyl)benzamide |
| 1782 | 1743 | 1-[2-(13-Cyclopentyltridecylamino)-5-pyrimidinecarbonyl]-piperidine |
| 1783 | 1744 | 6-Tetradecyl-N-(2,3-dihydroxylpropyl)-3-pyridinecarboxamide |

Table XXXIX

The heteroaryl carboxaldehydes shown are prepared from the appropriate aminoheteroaryl carbonitrile and alkyl halide by the methods of Examples 1514 and 1515 except for Examples 1789, 1790, 1796 and 1797. Using 6-chloronicotinonitrile, the intermediate for Examples 1790 and 1797 is formed by the procedure of Example 1534. Employing 2-chloro-5-cyanopyrimidine, the intermediate for Examples 1789 and 1796 is prepared by the method of Example 1533.

| Example No. | Compound |
|---|---|
| 1784 | 4-Hexylamino-2-thiophenecarboxaldehyde |
| 1785 | 5-Heptylamino-2-thiophenecarboxaldehyde |

1786 5-Octylamino-2-furancarboxaldehyde

1787 4-Nonylamino-1-methyl-2-pyrrolecarboxaldehyde

1788 5-Decylamino-1-methyl-2-pyrrolecarboxaldehyde

1789 2-Undecylamino-5-pyrimidinecarboxaldehyde

1790 6-Dodecylamino-3-pyridinecarboxaldehyde

1791 4-Tridecylamino-2-thiophenecarboxaldehyde

1792 5-Tetradecylamino-2-thiophenecarboxaldehyde

1793 4-Pentadecyl-1-methyl-2-pyrrolecarboxaldehyde

1794 5-Hexadecylamino-2-furancarboxaldehyde

1795 5-Heptadecylamino-1-methyl-2-pyrrolecarboxaldehyde

1796 2-Octadecylamino-5-pyrimidinecarboxaldehyde

1797 6-Nonadecylamino-3-pyridinecarboxaldehyde

1798 4-(1-Methylpentadecylamino)-2-thiophenecarboxaldehyde

1799 5-(3-Methylheptadecylamino)-2-thiophenecarboxaldehyde

1800 5-(14-Methylpentadecylamino)-2-furancarboxaldehyde

1801 4-(13,13-Dimethyltetradecylamino)-1-methyl-2-pyrrolecarboxaldehyde

1802 5-(10-Undecenylamino)-2-furancarboxaldehyde

1803 5-(10-Undecenylamino)-2-thiophenecarboxaldehyde

1804 5-(6-Hexadecynylamino)-1-methyl-2-pyrrolecarboxaldehyde

| | |
|---|---|
| 1805 | 4-(3 7-Dimethyl-6-octenylamino)-2-thiophenecarboxaldehyde |
| 1806 | 5-(11-Phenylundecylamino)-2-furancarboxaldehyde |

EXAMPLE 1807

Preparation of methyl 16-bromohexadecanoate

A mixture of 22 g. of 16-bromohexadecanoic acid and 200 ml. of methanol is treated with 1.0 ml. of concentrated sulfuric acid and stirred under reflux for 24 hours. The mixture is treated with 10 g. of sodium acetate and the solvent is evaporated. The residue is partially soluble in 300 ml. of ether and the mixture is filtered. The ether solution is dried over magnesium sulfate and evaporated. The residue is crystallized from petroleum ether to yield methyl 16-bromohexadecanoate as a white solid.

EXAMPLE 1808

Preparation of 1-(16-bromohexadecanoyl)pyrrolidine

A solution of 20 g. of 16-bromohexadecanoic acid and 27.5 ml. of thionyl chloride in 200 ml. of toluene is stirred under reflux for 5 hours and evaporated to a light yellow oil. A solution of the oil in 200 ml. of tetrahydrofuran is added dropwise with stirring to a solution of 10 g. of pyrrolidine in 200 ml. of tetrahydrofuran at 0°C. The mixture is filtered and the filtrate is evaporated. The residue is crystallized from petroleum ether to yield 1-(16-bromohexadecanoyl)pyrrolidine as a white solid.

EXAMPLE 1809

Preparation of 12-hydroxydoecanonitrile

A solution of 4.6 g. of 11-bromoundecanol and 1.6 g. of sodium cyanide in 40 ml. of dimethylformamide is heated at 80°C. for 18 hours, cooled, and poured into ice-water. The mixture is extracted with methylene chloride and the extract is dried over anhydrous magnesium sulfate. Evaporation of the solution affords 12-hydroxydodecanonitrile as a yellow oil.

EXAMPLE 1810

Preparation of 12-(methanesulfonyloxy)dodecanonitrile

A solution of 15 g. of 12-hydroxydodecanonitrile and 14 ml. of triethylamine in 320 ml. of methylene chloride is stirred at 0°C. while 5.7 ml. of methanesulfonylchloride is added dropwise. The mixture is stirred at 0°C. for 1

hour, diluted with methylene chloride and extracted with water; cold, dilute hydrochloric acid; saturated aqueous sodium bicarbonate; and brine. The solution is dried over anhydrous magnesium sulfate and evaporated to yield 12-(methanesulfonyloxy)dodecanonitrile as a yellow oil.

EXAMPLE 1811

Preparation of sodium 2-bromoethanesulfonate

A mixture of 6.2 g. of 1,2-dibromoethane, 12.5 ml. of ethanol and 4.5 ml. of water is stirred under reflux while a solution of 1.25 g. of anhydrous sodium sulfite in 4.5 ml. of water is added dropwise and for an additional 2 hours after the addition is completed. The mixture is evaporated and the residual solid is extracted with 20 ml. of boiling 95% ethanol, repeatedly. Crystallization for ethanol affords sodium 2-bromoethanesulfonate as a white solid.

EXAMPLE 1812

Preparation of N-phenyl-2-bromoethanesulfonamide

A mixture of 10 g. of sodium 2-bromoethanesulfonate and 12 g. of finely powdered phosphorous pentachloride is heated on a steam bath until it liquefies and for 4 hours thereafter. The mixture is concentrated by distillation and the residue is extracted with chloroform. The extract is washed with water and 5% aqueous sodium bicarbonate solution, dried over calcium chloride and evaporated to yield a yellow oil.

A solution of the yellow oil in 100 ml. of tetrahydrofuran is added dropwise to a stirred solution of 18 g. of aniline in 150 ml. of tetrahydrofuran at 0°C. The mixture is filtered and the filtrate is evaporated. Crystallization of the residue from ether-petroleum ether yields N-phenyl-2-bromoethanesulfonamide as a white solid.

EXAMPLE 1813

Preparation of ethyl 4-(15-carbomethoxypentadecylamino) benzoate

A solution of 21 g of methyl 16-bromohexadecanoate and 20 g. of ethyl 4-aminobenzoate in 150 ml. of hexamethylphosphoramide is stirred at 120°C. for 20 hours, cooled, diluted with water and filtered. A methylene chloride solution of the solid is dried over anhydrous magnesium sulfate, clarified with activated charcoal, and filtered through silica gel. The filtrate is evaporated and the residue crystallized from acetonitrile to yield ethyl 4-(15-carbomethoxypentadecylamino)benzoate as a white crystalline solid.

EXAMPLE 1814

Preparation of 4-(15-carboxypentadecylamino)benzoic acid

A solution of 8.8 g. of ethyl 4-(15-carbomethoxypentadecylamino)benzoate, 4.5 g. of potassium hydroxide, and 10 ml. of water in 90 ml. of ethanol is stirred at 75° for 10 hours, cooled, diluted with water, brought to pH 4 with dilute hydrochloric acid, and filtered. The solid is dried and crystallized from acetic acid to yield 4-(15-carboxypentadecylamino)benzoic acid as a white crystalline solid.

EXAMPLE 1815

Preparation of the disodium salt of 4-(15-carboxypentadecylamino)benzoic acid

A solution of 4 g. of 4-(15-carboxypentadecylamino)benzoic acid, 25 ml. of 1N sodium hydroxide, and 75 ml. of water in 250 ml. of ethanol is formed by heating to reflux and then allowed to cool. The precipitate is collected by filtration and recrystallized from ethanol-water to yield the disodium salt of 4-(15-carboxypentadecylamino)benzoic acid as a white crystalline solid.

EXAMPLE 1816

Preparation of ethyl 4-(15-carboxypentadecylamino)benzoate

A solution of 5 g. of ethyl 4-(15-carbomethoxypentadecylamino)benzoate and 0.8 g. of potassium hydroxide in 50 ml. of 95% ethanol is stirred at 50°C. for 10 hours, diluted with 100 ml. of water and acidified with concentrated hydrochloric acid. The solid is collected by filtration, dried, and recrystallized from toluene to yield ethyl 4-(15-carboxypentadecylamino)benzoate as a white solid.

EXAMPLE 1817

Preparation of 4-(15-carbomethoxypentadecylamino) benzonitrile

A solution of 12 g. of 4-aminobenzonitrile and 15 g. of methyl 16-bromohexadecanoate in 200 ml. of hexamethylphosphoramide is heated at 120° for 22 hours, allowed to cool, and diluted with water. The mixture is chilled and the precipitate which forms is collected, dried, and recrystallized from etherhexane to yield 4-(15-carbomethoxypentadecylamino)benzonitrile as a white solid.

EXAMPLE 1818

Preparation of 4-(15-carboxypentadecylamino)benzonitrile

A solution of 3 g. of 4-(15-carbomethoxypentadecylamino)benzonitrile and 0.5 g. of potassium hydroxide in 50 ml. of 95% ethanol is stirred at 50°C. for 10 hours, diluted with water and acidified with dilute hydrochloric acid. The precipitate is collected, dried and recrystallized from ether-hexane to yield 4-(15-carboxypentadecylamino)benzonitrile as a white solid.

EXAMPLE 1819

Preparation of 4-(15-carboxypentadecylamino)benzaldehyde

Di-iso-butylaluminum hydride (54 ml., 25% solution in toluene) is added with stirring to a solution of 5.1 g. of 4-(15-carboxypentadecylamino)benzonitrile under a nitrogen atmosphere. The temperature rises to 40°C. during the addition which takes 30 minutes and the reaction is then stirred for 1 hour. A solution of methanol in toluene (50 ml., 1:1) is added over 30 minutes and the mixture is poured into vigorously stirred ice-cold aqueous sulfuric acid (500 ml., 5%). After 10 minutes diatomacious earth (30 g.) is added, the mixture filtered and the organic layer separated. The aqueous solution is extracted twice with toluene (100 ml.) and the combined organic layers are washed with aqueous sodium bicarbonate, dried over magnesium sulfate, decolorized with charcoal, filtered and evaporated *in vacuo* to give a light yellow crystalline solid. Recrystallization from hexane affords 4-(15-carboxypentadecylamino)benzaldehyde as

a white solid.

EXAMPLE 1820

Preparation of ethyl 4-[2-(N-phenylsulfonamido)ethylamino]-benzoate

A solution of 7.1 g. of N-phenyl-2-bromoethane-sulfonamide and 12.6 g. of ethyl 4-aminobenzoate in 100 ml. of hexamethylphosphoramide is stirred at 120° for 22 hours, cooled diluted with water and filtered. The solid is dried and recrystallized from acetonitrile to yield ethyl 4-[2-(N-phenylsulfonamido)ethylamino]benzoate as a white solid.

Hydrolysis of the product by the method of Example 9 affords 4-[2-(N-phenylsulfonamido)ethylamino]benzoic acid.

EXAMPLE 1821

Preparation of ethyl 4-(2-sulfoethylamino)benzoate

A solution of 4.1 g. of sodium 2-bromoethanesulfonate and 7.9 g. of ethyl 4-aminobenzoate in 110 ml. of hexamethylphosphoramide is stirred at 125° for 18 hours and then poured into dilute hydrochloric acid. The solid is collected by filtration, dried, and recrystallized from acetonitrile to yield ethyl 4-(2-sulfoethylamino)benzoate as a white solid.

EXAMPLE 1822

Preparation of 4-(2-sulfoethylamino)benzoic acid

A mixture of 4.4 g. of ethyl 4-(2-sulfoethylamino)-benzoate, 2.3 g. of potassium hydroxide, 10 ml. of water, and 90 ml. of ethanol is stirred under reflux for 8 hours, allowed to cool, diluted with water, and brought to pH 4 with concentrated hydrochloric acid. The precipitate is collected by filtration, dried, and recrystallized from acetic acid to yield 4-(2-sulfoethylamino)benzoic acid as a white solid.

EXAMPLE 1823

Preparation of 4-(15-carbomethoxypentadecylamino)benzoic acid

A mixture of 1.37 g. of 4-aminobenzoic acid,

3.50 g. of methyl 16-bromohexadecanoate, 2.12 g. of sodium carbonate and 140 ml. of hexamethylphosphoramide is stirred at 120°C. for 18 hours, allowed to cool, and poured into dilute hydrochloric acid. The precipitate is collected by filtration, dried, and crystallized from acetonitrile to yield 4-(15-carbomethoxypentadecylamino)benzoic acid as a white solid.

TABLE XL

The 4-[(substituted alkyl)amino]phenyl compounds shown in the Table were prepared by the methods of Examples 1813 to 1823. The requisite alkylating agents are prepared by the methods of Examples 615 and 1807 to 1813.

TABLE XL

| Example No. | Method of Example | Compound |
|---|---|---|
| 1824 | 1813 | Ethyl 4-(3-carbomethoxyprop-2-enylamino)benzoate |
| 1825 | 1813 | Ethyl 4-(15-carbomethoxypentadec-4-ynylamino)benzoate |
| 1826 | 1814 | 4-[(3-Carboxy-2,4,4-trimethylcyclohexyl)amino]benzoic acid |
| 1827 | 1823 | 4-(13-Carbomethoxytridecylamino)benzoic acid |
| 1828 | 1814 | 4-(Carboxymethylamino)benzoic acid |
| 1829 | 1814 | 4-(3-Carboxypropylamino)benzoic acid |
| 1830 | 1813 | Ethyl 4-(1-carboethoxybutylamino)benzoate |
| 1831 | 1814 | 4-(5-Carboxypentylamino)benzoic acid |
| 1832 | 1814 | 4-(10-Carboxydecylamino)benzoic acid |
| 1833 | 1815 | Disodium salt of 4-(5-carboxypentylamino)benzoic acid |
| 1834 | 1816 | Ethyl 4-(10-carboxydecylamino)benzoate |
| 1835 | 1813 | Ethyl 4-(2-carboethoxyethylamino)benzoate |
| 1836 | 1813 | Ethyl 4-(3-carboxypropylamino)benzoate |
| 1837 | 1823 | 4-(10-Carbomethoxydecylamino)benzoic acid |
| 1838 | 1813 | Ethyl 4-(carboethoxymethylamino)benzoate |
| 1839 | 1815 | Disodium salt of 4-(1-carboxybutylamino)benzoic acid |
| 1840 | 1814 | 4-(2-Carboxyethylamino)benzoic acid |
| 1841 | 1813 | Ethyl 4-(3-carboethoxypropylamino)benzoate |
| 1842 | 1813 | Ethyl 4-(1-carboethoxyethylamino)benzoate |
| 1843 | 1813 | Ethyl 4-(5-carboethoxypentylamino)benzoate |
| 1844 | 1814 | 4-(Carboxymethylamino)benzoic acid |
| 1845 | 1814 | 4-(1-Carboxybutylamino)benzoic acid |

TABLE XL (cont'd)

| Example No. | Method of Example | Compound |
|---|---|---|
| 1846 | 1815 | Disodium salt of 4-(carboxymethylamino)benzoic acid |
| 1847 | 1815 | Disodium salt of 4-(10-carboxydecylamino)benzoic acid |
| 1848 | 1815 | Disodium salt of 4-(13-carboxytridecylamino)benzoic acid |
| 1849 | 1813 | Methyl 4-(carboethoxymethylamino)benzoate |
| 1850 | 1816 | Ethyl 4-(13-carboxytridecylamino)benzoate |
| 1851 | 1816 | Ethyl 4-(carboxymethylamino)benzoate |
| 1852 | 1816 | Ethyl 4-(5-carboxypentylamino)benzoate |
| 1853 | 1823 | 4-(Carbomethoxymethylamino)benzoic acid |
| 1854 | 1823 | 4-(Carboethoxymethylamino)benzoic acid |
| 1855 | 1823 | 4-(5-Carbomethoxypentylamino)benzoic acid |
| 1856 | 1823 | 4-(1-Carbomethoybutylamino)benzoic acid |
| 1857 | 1816 | Ethyl 4-(18-carboxyoctadecylamino)benzoate |
| 1858 | 1814 | 4-(4-Carboxybutylamino)benzoic acid |
| 1859 | 1815 | Sodium 4-(5-carbomethoxypentylamino)benzoate |
| 1860 | 1815 | Sodium 4-(5-carboethoxypentylamino)benzoate |
| 1861 | 1817, 1818 | 4-(10-Carbomethoxydecylamino)benzaldehyde |
| 1862 | 1817, 1818, 1819 | 4-(2-Carboxyethylamino)benzaldehyde |
| 1863 | 1817, 1818, 1819 | 4-(Carboxymethylamino)benzaldehyde |
| 1864 | 1817, 1818 | 4-(5-Carbomethoxypentylamino)benzaldehyde |
| 1865 | 1817, 1818, 1819 | 4-(1-Carboxybutylamino)benzaldehyde |
| 1866 | 1817, 1818 | 4-(13-Carboethoxytridecylamino)benzaldehyde |
| 1867 | 1817, 1818 | 4-(2-Carbomethoxyethylamino)benzaldehyde |

TABLE XL (cont'd)

| Example No. | Method of Example | Compound |
|---|---|---|
| 1868 | 1817, 1818, 1819 | 4-(10-Carboxydecylamino)benzaldehyde |
| 1869 | 1821 | Ethyl 4-(5-sulfopentylamino)benzoate |
| 1870 | 1821, 1822 | 4-(11-Sulfoundecylamino)benzoic acid |
| 1871 | 1821, 1822 | 4-(6-Sulfohexylamino)benzoic acid |
| 1872 | 1821, 1822 | 4-(16-Sulfohexadecylamino)benzoic acid |
| 1873 | 1821 | Ethyl 4-(4-sulfoethylamino)benzoate |
| 1874 | 1820 | Ethyl 4-[6-(N-phenylsulfamyl)hexylamino]benzoate |
| 1875 | 1820 | Ethyl 4-[1-(N-methylsulfamyl)butylamino]benzoate |
| 1876 | 1820 | 4-[11-(N-ethylsulfamyl)undecylamino]benzoic acid |
| 1877 | 1820 | 4-[2-(N-methylsulfamyl)ethylamino]benzoic acid |
| 1878 | 1820 | 4-[6-(N-phenylsulfamyl)hexylamino]benzoic acid |
| 1879 | 1820 | Ethyl 4-[16-(N-methylsulfamyl)hexadecylamino]benzoate |
| 1880 | 1820 | Ethyl 4-{6-[N-(p-toluene)sulfamyl]hexylamino}benzoate |
| 1881 | 1820 | 4-[(N-methylsulfamyl)methylamino]benzoic acid |
| 1882 | 1817 | 4-(5-Carbomethoxyhexylamino)benzamide |
| 1883 | 1817 | 1-[4-(10-Carbomethoxydecylamino)benzoyl]pyrrolidine |
| 1884 | 1817 | 1-[4-(2-Carboethoxyethylamino)benzoyl]piperidine |
| 1885 | 1817 | 1-[4-(Carboethoxymethylamino)benzoyl]morpholine |
| 1886 | 1817 | 1-[4-(5-Carbomethoxypentylamino)benzoyl]pyrrolidine |
| 1887 | 1813 | Ethyl 4-(10-carbomethoxydec-9-enylamino)benzoate |
| 1888 | 1814 | 4-(15-Carboxypentadec-9-enylamino)benzoic acid |
| 1889 | 1823 | 4-(3-Carbomethoxyprop-2-enylamino)benzoic acid |

TABLE XL (cont'd)

| Example No. | Method of Example | Compound |
|---|---|---|
| 1890 | 1816 | Ethyl 4-(6-carboxyhex-5-enylamino)benzoate |
| 1891 | 1815 | Sodium 4-(15-carbomethoxypentadec-4-ynylamino)benzoate |
| 1892 | 1817, 1819 | 4-(10-Carboethoxydec-9-enylamino)benzaldehyde |
| 1893 | 1817, 1818, 1819 | 4-(3-Carboxyprop-2-enylamino)benzaldehyde |
| 1894 | 1821, 1822 | 4-(3-Sulfoprop-2-enylamino)benzoic acid |
| 1895 | 1821 | Ethyl 4-(11-sulfoundec-10-enylamino)benzoate |
| 1896 | 1820 | Ethyl 4-(15-methylsulfamylpentadec-4-ynylamino)benzoate |
| 1897 | 1820 | 4-[3-(N-Phenylsulfamyl)prop-2-enylamino)benzoic acid |
| 1898 | 1813 | Ethyl 4-[(3-carboethoxy-2,4,4-trimethylcyclohexyl)amino] benzoate |
| 1899 | 1816 | Ethyl 4-[(3-Carboxymethyl-2,2,4-trimethylcyclopentyl)-amino]benzoate |

EXAMPLE 1900

Preparation of 4-(15-carbomethoxypentadecylamino)benzoyl chloride hydrochloride

A cold solution of 25 g. of 4-(15-carbomethoxypentadecylamino)benzoic acid in 500 ml. of dimethoxyethane-methylene chloride (4:1) is prepared and dry hydrochloric acid is bubbled through the solution until no more precipitate forms. The solution is treated with 25 ml. of thionyl chloride and refluxed until all of the precipitate has redissolved. The solvents are evaporated to yield an orange, semi-crystalline mass.

EXAMPLE 1901

Preparation of N-triflouroacetyl-4-(15-carbomethoxypentadecylamino)benzoyl chloride

To a stirred, ice-cold suspension of 9 g. 4-(15-carbomethoxypentadecylamino)benzoic acid in 100 ml. dimethoxyethane and 16 ml. pyridine is added 18 ml. trifluoroacetic anhydride. The solution is stirred at 0°C. for 30 minutes. The solution is diluted with 300 ml. ether and 100 g. ice. After stirring vigorously for 15 minutes, the phases are separated, the ether solution is washed with brine, dried and evaporated to a white, amorphous solid.

To 9.2 g. of the above product in 30 ml. methylene chloride and 0.5 ml. dimethylformamide is added 5.7 ml. thionyl chloride. After 20 hours at reflux, the solvents are evaporated to yield a light yellow mobile oil.

EXAMPLE 1902

Preparation of N-carbobenzyloxy-4-(15-carbomethoxypentadecylamino)benzoyl chloride

To 15 g. of 4-(15-carbomethoxypentadecylamino)-benzoic acid in 200 ml. warm chloroform is added 15 g. sodium carbonate in 150 ml. water. To the vigorously stirred solution is added 10 g. carbobenzyloxy chloride. After 2 hours stirring at 40°C., the layers are separated, washed three times with 1N hydrochloric acid, dried, and evaporated to an oil. The oil is dissolved in 300 ml.

toluene, treated with 15 ml. of thionyl chloride, and the solution is refluxed for 5 hours. The solvents are evaporated and the residue is dissolved three times in toluene, evaporating each time to yield a viscous orange oil.

EXAMPLE 1903

Preparation of 1-{4-[N-(t-butyloxycarbonyl)-N-(15-carbomethoxypentadecyl)amino]benzoyl}imidazole

A solution of 10g. of 4-(15-carbomethoxypentadecylamino)benzoic acid in 100 ml. dioxane is treated with 4 0 g. of t-butylazidoformate and 10 ml. pyridine. After stirring at room temperature for 18 hours, the protected amido-acid is precipitated from solution by addition of 150 ml. of water. The product is collected and thoroughly dried. The crude product is dissolved in 200 ml. of a mixture consisting of methylene chloride/dimethoxyethane/pyridine (1:4:1), and to this is added 5.4 g. of 1,1'-carbonyldiimidazole. The solution is stirred overnight at room temperature and the solvents are evaporated to yield the title compound as a thick, orange oil.

EXAMPLE 1904

Preparation of potassium sulfomethyl 4-(15-carbomethoxypentamino)benzoate

To 39.3 g. of potassium 4-(15-carbomethoxypentadecylamino)benzoate in 125 ml. of hexamethylphosphoramide is added 26.0 g. of powdered potassium iodomethanesulfonate. After stirring for 24 hours at 25°C., the product is obtained by careful dilution with water or alcohol to the crystallization point.

When this reaction is carried out with potassium 2-iodoethanesulfonate, the potassium sulfoethyl ester is obtained.

EXAMPLE 1905

2,3-Dihydroxypropyl 4-(15-carbomethoxypentadecylamino)-benzoate

A solution of 7.34 g. of 4-(15-carbomethoxypentadecylamino)benzoic acid, 4.80 g. of 25% aqueous sodium

hydroxide, and 12.6 g. of 3-iodo-1,2-propanediol in 50 ml. of hexamethylphosphoramide is stirred for 24 hours at ambient temperature, diluted with 100 ml. of ether and stirred for 5 days at ambient temperature. The mixture is treated with water and extracted with ether. The dried extracts are evaporated to yield 2,3-dihydroxypropyl 4-(15-carbomethoxypentadecylamino)benzoate.

EXAMPLE 1906

2,3-Dihydroxypropyl 4-(15-carbomethoxypentadecylamino)-benzoate

A solution of 11.8 g. of 4-(15-carbomethoxypentadecylamino)benzoic acid 1.00 g. of glycerol, and 5.35 ml. of boron trifluoride etherate in 200 ml. of toluene is stirred under reflux for 4 hours. The solution is treated with an additional 5.35 ml. of boron trifluoride etherate and refluxing is continued for 120 hours. Dilution with water and methylene chloride followed by filtration affords 2,3-dihydroxypropyl 4-(15-carbomethoxypentadecylamino)benzoate as a white solid.

EXAMPLE 1907

2,3-Dihydroxypropyl 4-(15-carbomethoxypentadecylamino)-benzoate

A mixture of 2.25 g. of methyl 4-(15-carbomethoxypentadecylamino)benzoate, 280 mg. of glycerol, and 1.37 g. of p-toluene sulfonic acid is heated at 180°C. for 4 hours and then is partitioned between ether and 3% aqueous sodium carbonate solution. The ether layer is separated, dried, and evaporated to yield 2,3-dihydroxypropyl 4-(15-carbomethoxypentadecylamino) benzoate.

EXAMPLE 1908

Methyl 4-(15-carbomethoxypentadecylamino)benzoate

A solution of 50.5 g. of 4-(15-carbomethoxypentadecylamino)benzoic acid and 34.4 ml. of boron trifluoride etherate in 200 ml. of methanol is stirred under reflux for 4 hours, allowed to cool, and is poured into 1.20 liters of ice-cold, 5% aqueous sodium carbonate solution. The white solid is collected by filtration and recrystallized from benzene-ethanol to yield methyl 4-(15-carbomethoxypentadecylamino)benzoate. m.p. 92°-93°C.

EXAMPLE 1909

Preparation of 1-(methoxycarbonyl)propyl 4-(15-carbomethoxypentadecylamino)benzoate

To a solution of 10.0 g. 4-(15-carbomethoxypentadecylamino)benzoyl chloride hydrochloride in 200 ml. methylene chloride is added dropwise to a solution of 3 g. methyl -hydroxy butyrate and 5 g triethylamine in 100 ml. ether. After 17 hours stirring at room temperature, the precipitate is filtered and washed with several portions of ether. The ether solution is washed with water, dried and condensed to the crystalline title compound.

EXAMPLE 1910

Preparation of 1-carboxyethyl 4-(15-carbomethoxypentadecylamino)benzoate

A flask containing 10.0 g. 4-(15-carbomethoxypentadecylamino)benzoic acid, 3.3 g. lactic acid, 500 mg. toluene-sulfonic acid and 500 ml. toluene is equipped with a Soxhlet extractor charged with activated 4 Å Linde molecular sieves. The solution is refluxed for 4 hours, during which time the Soxhlet extractor is charged twice more with fresh sieves. The hot solution is filtered and left to cool whereupon the product separates as off-white crystals.

EXAMPLE 1911

Preparation of diethyl O-[4-(15-carbomethoxypentadecylamino)benzoyl]tartarate

N-Trifluoroacetyl-4-(15-carbomethoxypentadecylamino)benzoyl chloride and 1.2 g. triethylamine in 100 ml. warm ether is treated with 2.5 g. diethyl tartarate and refluxed for 24 hours. The hot solution is filtered, the residue is washed with hot ether, and the solution is evaporated. After treatment with aqueous methanolic potassium carbonate, the product is precipitated by acidification, filtered, and dried. Crystallization from acetone yields the diethyl tartarate as a white, crystalline solid.

EXAMPLE 1912

Preparation of O-[4-(15-carbomethoxypentadecylamino)-benzoyl]malic acid

To a warm solution of N-carbobenzyloxy-4-(15-carbomethoxypentadecylamino)benzoyl chloride and 1.3 g. triethylamine in 100 ml. ether is added 2 g. malic acid. An immediate precipitate forms, but the mixture is refluxed for one hour and filtered while hot. The solid is washed several times with hot ether, then the ether is evaporated to yield a white solid. The product is dissolved in tetrahydrofuran (100 ml.) and hydrogenated over 600 mg. 10% palladium-on-carbon at 50 psi until hydrogen uptake stops. The catalyst is filtered, the solution is evaporated, and the residue is crystallized from acetic acid to yield the title compound as a tan, crystalline mass.

EXAMPLE 1913

Preparation of 2-(ethoxycarbonyl)vinyl 4-(15-carbomethoxypentadecylamino)benzoate

To a mixture containing 4.3 g. 1-{4-[N-(t-butyloxycarbonyl)-N-(15-carbomethoxypentadecyl)amino]benzoyl}-imidazole, 50 ml. chloroform, and 50 ml. 5N sodium hydroxide is added 3 g. ethyl -formal acetate. The solution is vigorously stirred for 24 hours. The layers are separated, and the chloroform solution is washed once with 50 ml. 1N sodium hydroxide. The solvent is evaporated and the residue is heated for 30 minutes at 40°C. in 50 ml. anhydrous trifluoroacetic acid. The solvent is again evaporated and the oil is crystallized from acetone to yield light yellow crystals of 2-(ethoxycarbonyl)vinyl 4-(15-carbomethoxypentadecylamino)benzoate.

EXAMPLE 1914

Preparation of 2-(ethoxycarbonyl)ethyl 4-(15-carbomethoxypentadecylamino)benzoate

A solution of 4 g. 2-(ethoxycarbonyl)vinyl 4-(15-carbomethoxypentadecylamino)benzoate and 400 mg. 10% palladium-on-carbon in 100 ml. tetrahydrofuran is

hydrogenated at 50 psi until hydrogen uptake stops. The catalyst is filtered, the solution is evaporated, and the residue is crystallized from acetonitrile to yield 2-(ethoxycarbonyl)ethyl 4-(15-carbomethoxypentadecylamino)benzoate.

TABLE XLI

The 4-[(substituted-alkyl)amino]benzoate esters shown in the Table are prepared by the method of Examples 1904-1914. The requisite benzoic acids or their derivatives are prepared by the methods of Examples 1814-1823 and 1900-1903.

TABLE XLI

| Example Number | Method of Example | Compound |
|---|---|---|
| 1915 | 1905 | Isopropyl 4-(5-carbomethoxypentylamino)benzoate |
| 1916 | 1905 | 2-Ethoxyethyl 4-[11-(N-phenylsulfamyl)undecylamino)benzoate |
| 1917 | 1905 | Isopropyl 4-(13-carboethoxypentadecylamino)benzoate |
| 1918 | 1904 | Potassium sulfomethyl 4-(13-carbomethoxytridecylamino)benzoate |
| 1919 | 1904 | Ethyl O-[4-(10-carboethoxydec-2-enylamino)benzoyl]glycolate |
| 1920 | 1904 | 2-{4-[10-(Carbo-*tert*-butoxy)decylamino]benzoyl}ethanesulfonic acid |
| 1921 | 1904 | 2-[4-(5-carbomethoxypentylamino)benzoyl]ethanesulfonic acid |
| 1922 | 1904 | 2-[4-(2-carboethoxyethylamino)benzoyl]ethanesulfonic acid |
| 1923 | 1905 | 2,3-Dihydroxypropyl 4-(10-carbomethoxydecylamino)benzoate |
| 1924 | 1905 | 2,3-Dihydroxypropyl 4-(2-carbomethoxyethylamino)benzoate |
| 1925 | 1905 | 3-Hydroxypropyl 4-(2-carboethoxyethylamino)benzoate |
| 1926 | 1905 | 2,3-Dihydroxypropyl 4-(15-carbomethoxypentadec-4-ynylamino)benzoate |
| 1927 | 1905 | 2-Hydroxypropyl 4-[11-(N-phenylsulfamyl)undecylamino]benzoate |
| 1928 | 1905 | 2,3-Dihydroxypropyl 4-[2-(N-methylsulfamyl)ethylamino]benzoate |
| 1929 | 1906 | 2-Ethoxyethyl 4-(5-carbomethoxypentylamino)benzoate |
| 1930 | 1906 | Isopropyl 4-(15-carboethoxypentadecylamino)benzoate |
| 1931 | 1906 | 2-Dimethylaminoethyl 4-(10-carboethoxydec-9-enylamino)benzoate |
| 1932 | 1908 | Methyl 4-(2-carbophenoxyethylamino)benzoate |
| 1933 | 1908 | Methyl 4-(15-carbomethoxypentadec-4-ynylamino)benzoate |
| 1934 | 1908 | Methyl 4-[(3-carboxymethyl-2,2,4-trimethylcyclopentyl)amino]benzoate |
| 1935 | 1907 | 4-Hydroxybutyl 4-(2-carboethoxyethylamino)benzoate |

TABLE XLI (cont'd)

| Example Number | Method of Example | Compound |
|---|---|---|
| 1936 | 1907 | 2-Ethoxyethyl 4-(15-carboethoxypentadecylamino)benzoate |
| 1937 | 1907 | 4-Methylbenzyl 4-(11-methylsulfamylundecylamino)benzoate |
| 1938 | 1909 | 1-Carbomethoxypropyl 4-(10-carbomethoxydec-9-emylamino)benzoate |
| 1939 | 1909 | Carbomethoxymethyl 4-(15-carbomethoxypentadecylamino)benzoate |
| 1940 | 1909 | 1-Carbomethoxypropyl 4-(5-carbomethoxypentylamino)benzoate |
| 1941 | 1909 | Carbomethoxymethyl 4-[(3-carbomethoxy-2,4,4-trimethylcyclohexyl)-amino]benzoate |
| 1942 | 1910 | 1-Carboxyethyl 4-[11-(N-phenylsulfamyl)undecylamino]benzoate |
| 1943 | 1910 | Carboxymethyl 4-[16-(N-methylsulfamyl)hexadecylamino]benzoate |
| 1944 | 1910 | 1-Carboxypropyl 4-(2-carbomethoxyethylamino)benzoate |
| 1945 | 1911 | 4-Pyridylmethyl 4-(10-carboethoxydec-9-enylamino)benzoate |
| 1946 | 1911 | 1-Methyl-4-piperidyl 4-(5-carboethoxypentylamino)benzoate |
| 1947 | 1911 | Diethyl O-[4-(5-carbomethoxypentylamino)benzoyl]tartarate |
| 1948 | 1911 | Diethyl O-[4-(15-carboethoxypentadecylamino)benzoyl]tartarate |
| 1949 | 1912 | O-{4-[11-(N-Phenylsulfamyl)undec-2-enylamino]benzoyl} malic acid |
| 1950 | 1912 | O-[4-(1-Carbomethoxybutylamino)benzoyl]malic acid |
| 1951 | 1912 | O-{4-[2-(N-Methylsulfamyl)ethylamino]benzoyl} tartaric acid |
| 1952 | 1913 | 2-(Ethoxycarbonyl)vinyl 4-(10-carbophenoxydec-9-enylamino)benzoate |
| 1953 | 1913 | 2-(Methoxycarbonyl)vinyl 4-[16-(N-methylsulfamyl)hexadecylamino]-benzoate |
| 1954 | 1913 | 2-(Ethoxycarbonyl)vinyl 4-(5-carboethoxypentylamino)benzoate |

| Example Number | Method of Example | Compound |
|---|---|---|
| 1955 | 1914 | 2-(Ethoxycarbonyl)ethyl 4-(1-carboethoxybutylamino)benzoate |
| 1956 | 1914 | 2-(Ethoxycarbonyl)ethyl 4-[(3-carboethoxy-2,2,4-trimethylcyclopentyl)-amino]benzoate |

EXAMPLE 1957

Preparation of 1-[4-(15-carbomethoxypentadecylamino)-benzoyl]piperidine

To a chilled solution of 35 ml. of piperidine, 2.5 ml. of triethylamine and 0.6 g. of dimethylaminopyridine in 100 ml of diethyl ether is added (1/2 hour) a solution of 8.3 g. of 4-(15-carbomethoxypentadecylamino)benzoyl chloride hydrochloride in 50 ml. of ether. The solution is warmed to room temperature and maintained there for two hours. The solution is heated to reflux for an additional 2 hours at which time the reaction is complete. The solution is cooled, extracted twice with 100 ml. portions of water and dried over magnesium sulfate. The solvent is removed *in vacuo* and the solid is recrystallized in 50 ml of diethyl ether to yield the product.

EXAMPLE 1958

Preparation of ethyl 4-(15-carbomethoxypentadecylamino)-hippurate

To a solution of 18 08 g. of 4-(15-carbomethoxypentadecylamino)benzoic acid in a mixture of dioxane and methylene chloride (40 ml /160 ml.) is added gaseous hydrogen chloride for 10 minutes. The slurry is cooled and 18 ml of thionyl chloride added The slurry is heated under reflux for 2 hours and then concentrated. The amber solution is diluted with 100 ml of dioxane and then added to freshly prepared ethyl glycinate in 300 ml. of methylene chloride containing 1 g of dimethylaminopyridine and 10 ml. of triethylamine. After 16 hours at room temperature the mixture is refluxed for 2 hours cooled and filtered. The mother liquor is extracted with water and 10% hydrochloric acid. The solution is dried over magnesium chloride and concentreated in *vacuo* to an amber liquid. This material is chromatographed on a silica column and then recrystallized from acetonitrile to yield the product.

EXAMPLE 1959

Preparation of N-[4-(15-carbomethoxypentadecylamino)-

benzoyl]glycine

A mixture of 26 4 g. of ethyl N-[4-(15-carbomethoxypentadecylamino)benzoyl]glycinate, 110 ml. of 1N sodium hydroxide solution and 100 ml of ethanol is stirred at ambient temperature for 2 hours and then partially evaporated. The aqueous solution is washed with diethyl ether, acidified with 6N hydrochloric acid, and filtered. The white solid is dried *in vacuo* and recrystallized from acetone to yield the product

EXAMPLE 1960

Preparation of p-(15-carbomethoxypentadecylamino)-N-(methylsulfonyl)benzamide

A solution of 19 0 g. of methanesulfonamide in 150 ml. of dry dimethylacetamide is added dropwise during 15 minutes to a stirred and cooled (water bath) suspension of 5 5 g. of sodium hydride in 100 ml of dry dimethylacetamide. The mixture is then stirred and heated at 60°-80°C. for 2 hours. In the meantime, a mixture of 36 2 g. of p-(15-carbomethoxypentadecylamino)-benzoic acid in 1200 ml. of methylene chloride, 300 ml. of dimethoxyethane and 40 ml. of thionyl chloride is refluxed for 1 hour and 15 minutes. The solution is evaporated to an oil which is co-evaporated twice with added dioxane to remove excess thionyl chloride. To the resulting oily residue p-(15-carbomethoxypentadecylamino)benzoyl chloride hydrochloride is added, in one portion, the previously prepared mixture of sodium methanesulfonamide in dimethylacetamide The mixture becomes very hot and is cooled briefly in a water bath and then is stirred at room temperature for 30 minutes. The mixture is filtered through a bed of diatomaceous earth and the filtrate is poured into 2 l. of water. The resulting suspension is coagulated by the addition of 250 ml. of saturated sodium chloride solution and the mixture is filtered. The product is washed with water partially air dried and then crystallized from 1.5 l. of ethanol. The product

is recrystallized from p-dioxane and dried at 65°C to yield the title compound as tan crystals.

EXAMPLE 1961

Preparation of [4-(15-carbomethoxypentadecylamino)-benzoyl]hydroxamic acid

A solution of 16.7 g. of 4-(15-carbomethoxypentadecylamino)benzoyl chloride hydrochloride in methylene chloride is added at 5°C. to a mixture of 2.8 g. of hydroxyamine hydrochloride 4.2 g. of sodium carbonate, and 120 ml. of ether Water (10 ml.) is added and the mixture is stirred 3 hours. After the addition of 150 ml. of water, the solvents are evaporated. The crystalline residue is recrystallized from acetonitrile.

EXAMPLE 1962

Preparation of 3-[4-(15-carbomethoxypentadecylamino)-benzoyl] 4-carboxythiazolidine

One-tenth mole of 4-(15-carbomethoxypentadecylamino)benzoyl chloride hydrochloride in methylene chloride is added to a solution of 0.1 mole of ethyl thiazolidine-4-carboxylate in chloroform containing two equivalents of triethylamine After 5 hours at 20°C. the solution is filtered and evaporated to a white solid which is recrystallized from acetonitrile to yield 3-[4-(16-bromohexadecylamino)benzoyl]-4- carbethoxythiazolidine. By means of the alkaline hydrolysis method of Example 10, the ethyl ester is converted to the subject carboxylic acid. This acid is also prepared using the procedure of this Example except that the acylation of the thiazolidine-4-carboxylic acid is carried out in aqueous acetone sodium bicarbonate solution.

EXAMPLE 1963

Preparation of N-[4-(15-carbomethoxypentadecylamino)-benzoyl]alanine

A solution of 4.75 g. of N-trifluoroacetyl-4-(15-carbomethoxypentadecylamino)benzoyl chloride and

1 2 g. of triethylamine in 20 ml. of warm ether is treated with 1 55 g. alanine ethyl ester and refluxed for 24 hours. The hot solution is filtered the residue is washed with hot ether, and the solution is evaporated. After treatment with aqueous methanolic potassium carbonate, the product is precipitated by acidification, filtered, and dried. Crystallization from acetone yields the product as a white, crystalline solid.

EXAMPLE 1964

Preparation of 1-[4-(15-carbomethoxypentadecylamino)-benzoyl]pyrrolidine

A solution of 6.0 g. of 4-[N-carbobenzyloxy-N-(15-carbomethoxypentadecylamino)benzoyl chloride and 1.2 g. triethylamine in 100 ml. warm ether is treated with 1.1 g. of pyrrolidine After 1 hour at reflux the precipitate is filtered and washed with warm ether. After evaporation to dryness the intermediate is dissolved in 50 ml. 30% hydrobromic/acetic acid and warmed at 50° for 2 hours The solvents are evaporated and the product is partitioned between methylene chloride and water. The layers are separated and the methylene chloride is evaporated. The residue is crystallized from acetone to yield colorless crystals.

EXAMPLE 1965

Preparation of 4-(15-carbomethoxypentadecylamino)-N-(2,-3-dihydroxypropyl)benzamide

To a mixture containing 4.3 g. of 1- 4-[N-(t-butyloxycarbonyl)-N-(15-carbomethoxypentadecyl)amino]-benzoyl imidazole, 50 ml. of chloroform, and 50 ml. of 5N sodium hydroxide is added 1.1 g. of 3-amino-1,2-propanediol. The solution is vigorously stirred for 24 hours the layers are separated, and the chloroform solution is washed once with 50 ml. of 1N sodium hydroxide. The solvent is evaporated and the residue is heated for 30 minutes at 40°C. and 50 ml. of anhydrous trifluoroacetic acid. The solvent is again evaporated and the oil is crystallized from acetone to yield the product as a light yellow crystalline solid.

TABLE XLII

The 4-[(substituted-alkyl)amino]benzamides shown in the Table are prepared by the methods of Examples 1957-1965. The requisite benzoic acids or their derivatives are prepared by the methods of Examples 1814-1823 and 1900-1903.

TABLE XLII

| Example Number | Method of Example | Compound |
|---|---|---|
| 1966 | 1965 | N-(2 3-Dihydroxypropyl)-4-(13-carbomethoxytridecylamino)benzamide |
| 1967 | 1965 | N-(2,3-Dihydroxypropyl)-4-[11-(N-methylsulfamyl)undecylamino]benzamide |
| 1968 | 1966 | N-(2-Hydroxypropyl)-4-(16-carbomethoxyhexadecylamino)benzamide |
| 1969 | 1957 | 1-[4-(10-Carbomethoxydecylamino)benzoyl]piperidine |
| 1970 | 1957 | 1-[4-(15-Carbomethoxypentadec-4-ynylamino)benzoyl]pyrrolidine |
| 1971 | 1957 | 1-[4-(2-Carbomethoxyethylamino)benzoyl]morpholine |
| 1972 | 1957 | 1-[4-(6-Carboethoxypentylamino)benzoyl]pyrrolidine |
| 1973 | 1958 | Ethyl 4-[11-(N-methylsulfamyl)undec-2-enylamino]hippurate |
| 1974 | 1958 | Ethyl 4-(1-carboethoxybutylamino)hippurate |
| 1975 | 1958 | Ethyl 4-(2-carbomethoxyethylamino)hippurate |
| 1976 | 1959 | N-{4-[11-(N-phenylsulfamyl)undecylamino]benzoyl}glycine |
| 1977 | 1959 | N-[4-(15-carboxypentadecylamino)benzoyl]glycine |
| 1978 | 1959 | N-[4-(2-carboxyethylamino)benzoyl]glycine |
| 1979 | 1959 | N-[4-(1-carboxybutylamino)benzoyl]glycine |
| 1980 | 1959 | N-{4-[11-(N-Phenylsulfamyl)undec-2-enylamino]benzoyl}glycine |
| 1981 | 1960 | N-Methyl-4-(10-carbomethoxydecylamino)benzamide |
| 1982 | 1960 | N-Phenyl-4-(15-carbomethoxypentadecylamino)benzamide |
| 1983 | 1960 | N-(Methylsulfonyl)-4-(2-carbomethoxyethylamino)benzamide |
| 1984 | 1960 | N-(Phenylsulfonyl)-4-[(3-carbomethoxy-2,2,4-trimethylcyclopentyl)-amino]benzamide |
| 1985 | 1961 | 4-[16-(N-Methylsulfamyl)hexadecylamino]benzoylhydroxamic acid |

TABLE XLII (cont'd)

| Example Number | Method of Example | Compound |
|---|---|---|
| 1986 | 1961 | 4-(1-Carbomethoxybutylamino)benzoylhydroxamic acid |
| 1987 | 1961 | 4-(Carboxymethylamino)benzoylhydroxamic acid |
| 1988 | 1963 | 3-{4-[11-(N-Phenylsulfamyl)undec-2-enylamino]benzoyl}-4-carboxythiazolidine |
| 1989 | 1963 | 3-{4-[15-(Carboxypentadec-4-enyl)amino]benzoyl}-4-carboxythiazolidine |
| 1990 | 1963 | 3-[4-(5-Carboxypentylamino)benzoyl]-4-carboxythiazolidine |
| 1991 | 1964 | 1-[4-(16-Methylsulfamylhexadecylamino)benzoyl]piperidine |
| 1992 | 1964 | 1-[4-(Carboxymethylamino)benzoyl]pyrrolidine |
| 1993 | 1964 | 1-[4-(10-Carbomethoxydec-9-enylamino)benzoyl]pyrrolidine |
| 1994 | 1965 | N-(2,3-Dihydroxypropyl)-4-(10-carboethoxydecylamino)benzamide |
| 1995 | 1966 | N-(2,3-Dihydroxypropyl)-4-[11-(N-phenylsulfamyl)undec-2-enylamino]-benzamide |

EXAMPLE 1996

15-(Trifluoromethyl)pentadecyl bromide

A 25.0 g. quantity of 16-bromohexadecanoic acid is placed in a 2 liter stainless steel bomb and after flushing with nitrogen and cooling the bomb in dry-ice, 66 g. of sulfur tetrafluoride is introduced and the sealed bomb is heated with shaking at 118°C. for 6 hours. The bomb is allowed to cool and opened and volatile material is allowed to evaporate. The residue is partitioned between water and ether and the ether layer is separated, dried over anhydrous magnesium sulfate and evaporated. The residue is distilled to yield 15-(trifluoromethyl)pentadecyl bromide as a clear, colorless liquid.

EXAMPLE 1997

(Pentadecafluoroheptyl)(trifluoromethanesulfonyloxy)methane

A mixture of 40 g. of (pentadecafluoroheptyl)-methanol, 11 g. of triethylamine, and 200 ml. of methylene chloride is stirred at -20°C. while a solution of 18 g. of trifluoromethanesulfonyl chloride in 50 ml. of methylene chloride is added. The solution is stirred for one hour at room temperature and then washed with ice-cold 10% hydrochloric acid, saturated aqeous sodium bicarbonate solution, and water; dried over anhydrous magnesium sulfate; and evaporated. The residual brown liquid is distilled to yield the product as a colorless liquid.

EXAMPLE 1998

11-(Pentafluoroethyl)undecanol

A solution of 5 g. of 11-(pentafluoroethyl)undecanoic acid in 20 ml. of tetrahydrofuran is stirred at 0°C. while 16 ml. of 1M borane is tetrahydrofuran is added during 15 minutes. The mixture is stirred for 18 hours at ambient temperature, poured into ice-water, and extracted with ether. The dried extact is evaporated and the residual oil crystallized from hexane to yield 11-(pentadecafluoroethyl)undecanol as a white solid.

EXAMPLE 1999

11-(Pentafluoroethyl)-1-(methanesulfonyloxy)undecane

To a solution of 15 g. of 11-(pentafluoroethyl)-undecanol and triethylamine (14 ml.) in dry methylene chloride (320 ml.) at -8°C. is added methanesulfonylchloride (5.73 ml.), dropwise. The reaction mixture is stirred at -10°C. for 30 minutes and then diluted with methylene chloride, extracted with ice-water (250 ml.), followed by cold 10% hydrochloric acid (200 ml.); cold saturated sodium bicarbonate (200 ml.) and cold saturated sodium chloride solution (200 ml.). The organic layer is separated, dried over anhydrous magnesium sulfate, and evaporated to yield the methanesulfonate as a light yellow oil.

EXAMPLE 2000

6-Oxohexadecyl bromide

The Grinard reagent prepared from 60 g. of decyl bromide. 5.6 g. of magnesium, and 200 ml. of ether is treated with 27 g. of cadium chloride and the solution is stirred under reflux for 30 minutes. The solvent is replaced by distillation with toluene, and the toluene solution is treated with 29 g. of 6-bromohexanoyl chloride and stirred under reflux for 30 minutes. The mixture is cooled, diluted with 200 ml. of 10% sulfuric acid and extracted with ether. The extracts are dried and evaporated and the residual oil distilled in vacuo to yield 6-oxohexadecyl bromide as a light yellow oil.

EXAMPLE 2001

6,6-Difluorohexadecyl bromide

In the manner of Example 1996, 6-oxohexadecyl bromide is converted to 6,6-difluorohexadecyl bromide.

EXAMPLE 2002

Ethyl 4-[15-(trifluoromethyl)pentadecylamino]benzoate

A mixture of 18.5 g. of 15 (trifluoromethyl) pentadecyl bromide, 17.0 g. of ethyl 4-aminobenzoate, 7.1.g. of potassium carbonate, and 75 ml. of hexamethylphosphoramide is stirred for 16 hours at 120°C., allowed to cool, diluted with water, and filtered. The solid is dissolved in methylene

chloride and the solution is dried over anydrous magnesium sulfate and evaporated. The residual brown oil is crystallized from ethanol and recrystallized from acetonitrile to yield the product as a white solid.

EXAMPLE 2003

4-[15-(Trifluoromethyl)pentadecylamino]benzoic acid

A mixture of 6.0 g. of ethyl 4-[15-(trifluoromethyl)pentadecylamino]benzoate, 4.0 g. of potassium hydroxide and 50 ml. of ethanol is stirred at 80°C. for 4 hours. The mixture is diluted with water and adjusted to pH 5.5 by the addition of concentrated hydrochloric acid. Cooling and filtration affords a solid which is recrystallized from acetone to yield the product as a white solid.

EXAMPLE 2004

Ethyl 4-[pentadecafluoroheptyl)methylamino]benzoate

A solution of 45 g. of (pentadecafluoroheptyl)-(trifluoromethanesulfonyloxy)methane and 28 g. of ethyl 4-aminobenzoate in 50 ml. of hexamethylphosphoramide is stirred at 120°C. for 72 hours, diluted with water, and filtered. A methylene chloride solution of the solid is dried over anhydrous magnesium sulfate and evaporated. The residue is recrystallized fom acetonitrile to yield the product as a white solid.

EXAMPLE 2005

4-[(Pentadecafluorheptyl)methylamino]benzoic acid

Hydrolysis of ethyl 4-[(pentadecafluoroheptyl)-methylamino]benzoate by the method of Example 2003 affords 4-[(pentadecafluoroheptyl)methylamino]benzoic acid as a white solid.

EXAMPLE 2006

Ethyl 4-[11-(pentafluoroethyl)undecylamino]benzoate

A solution of 18.1 g. of 11-(pentafluoroethyl)-1-(methanesulfonyloxy)undecane and 19.8 g. of ethyl p-aminobenzoate in in hexamethylphosphoramide is heated at 120°C. for 20 hours. After cooling, the reaction mixture is diluted with ethanol:water (1:1)(30 ml.) and chilled. More ethanol is added, the mixture is filtered and the solid residue is

recrystallized from ethanol to yield the product as a white solid.

EXAMPLE 2007

4-[11-(Pentafluoroethyl)undecylamino]benzoic acid

Hydrolysis of ethyl 4-[11-(pentafluoroethyl)undecylamino]benzoate in the manner of Example 2003 affords 4-[11-(pentafluoroethyl)undecylamino]benzoic acid as a white solid.

Table XLIII

The following benzoic acids are prepared from the corresponding benzoate esters by the method of Example 2003. The requisite benzoate esters are prepared from 4-aminobenzoate esters and the appropriate halide, trifluoromethanesulfonate, or methanesulfonate by the methods of Examples 2002, 2004, and 2006, respectively. Polyfluoroalkyl halides, trifluoromethanesulfonates, and methanesulfonates required for these reactions are prepared by the methods of Examples 615 and 1996 to 2001.

| Example No. | Compound |
|---|---|
| 2008 | 4-[6-(Trifluoromethyl)hexylamino]benzoic acid |
| 2009 | 4-[7-(Trifluoromethyl)heptylamino]benzoic acid |
| 2010 | 4-[8-(Trifluoromethyl)octylamino]benzoic acid |
| 2011 | 4-[9-(Trifluoromethyl)nonylamino]benzoic acid |
| 2012 | 4-[10-(Trifluoromethyl)decylamino]benzoic acid |
| 2013 | 4-[11-(Trifluoromethyl)undecylamino]benzoic acid |
| 2014 | 4-[12-(Trifluoromethyl)dodecylamino]benzoic acid |
| 2015 | 4-[13-(Trifluoromethyl)tridecylamino]benzoic acid |
| 2016 | 4-[14-(Trifluoromethyl)tetradecylamino]benzoic acid |
| 2017 | 2-[16-(Trifluoromethyl)hexadecylamino]benzoic acid |

| Example No. | Compound |
|---|---|
| 2018 | 4-[17-Trifluoromethyl)heptadecylamino]benzoic acid |
| 2019 | 4-[18-(Trifluoromethyl)octadecylamino]benzoic acid |
| 2020 | 4-[1-(2,2,2-Trifluoroethyl)decylamino]bezoic acid |
| 2021 | 4-[1-(2,2,2-Trifluoroethyl)tridecylamino]benzoic acid |
| 2022 | 4-[1-(2,2,2-Trifluoroethyl)pentadecylamino]-benzoic acid |
| 2023 | 4-[7-(Trifluoromethyl)undecylamino]benzoic acid |
| 2024 | 4-1-Hexyl-11-(trifluoromethyl)undecylamino]-benzoic acid |
| 2025 | 4-[3,7-Dimethyl-7-(trifluoromethyl)octyl-amino]benzoic acid |
| 2026 | 4-[16-(Pentafluoroethyl)hexadecylamino]benzoic acid |
| 2027 | 4-[11-(Heptafluoropropyl)undecylamino]benzoic acid |
| 2028 | 4-[11-(Heptafluoroisopropyl)undecylamino]benzoic acid |
| 2029 | 4-[11-(Nonafluorobutyl)undecylamino]benzoic acid |
| 2030 | 4-[11-(Unecafluoropentyl)undecylamino]benzoic acid |
| 2031 | 4-[12-Fluoro-12-(heptafluoropropyl)-12-(trifluoromethyl)dodecylamino]benzoic acid |
| 2032 | 4-[5-(Pentadecafluoroheptyl)pentylamino]benzoic acid |
| 2033 | 4-[7-(Pentadecafluoroheptyl)heptylamino]benzoic acid |
| 2034 | 4-(3,3-Difluorotetradecylamino)benzoic acid |
| 2035 | 4-(6 6-Difluorohexadecylamino)benzoic acid |
| 2036 | 4-(11,11-Difluorododecylamino)benzoic acid |

| Example No. | Compound |
|---|---|
| 2037 | 4-(14,14-Difluoropentadecylamino)benzoic acid |
| 2038 | 4-(15,15-Difluorohexadecylamino)benzoic acid |
| 2039 | 4-(16,16-Difluoroheptadecylamino)benzoic acid |
| 2040 | 4-[11,11-Difluoro-16-(trifluoromethyl)hexadecylamino]benzoic acid |
| 2041 | 4-[(Hentricontafluoropentadecyl)methylamino]-benzoic acid |
| 2042 | 4-[11-(Trifluoromethyl)-9-undecenylamino]benzoic acid |
| 2043 | 4-[15-(Trifluoromethyl)-9-pentadecenylamino]-benzoic acid |
| 2044 | 4-[5-(Pentadecafluoroheptyl)-4-pentenylamino]-benzoic acid |
| 2045 | 4-[6-(Pentadecafluoroheptyl)-5-hexenylamino]-benzoic acid |
| 2046 | 4-[15-(Trifluoromethyl)-7-pentadecenylamino]-benzoic acid |
| 2047 | 4-[15-(Trifluoromethyl)-12-pentadecenylamino]-benzoic acid |
| 2048 | 4-[15-(Trifluoromethyl)-4-pentadecynylamino]-benzoic acid |
| 2049 | 4-[1-Hexyl-9-(trifluoromethyl)-4-nonynyl-amino]benzoic acid |
| 2050 | 4-[3-(2,2,2-Trifluoroethyl)-2,4,4-trimethyl-cyclopentylamino]benzoic acid |
| 2051 | 4-[3-(Trifluoromethyl)-2,4,4-trimethylcyclo-hexylamino]benzoic acid |

## EXAMPLE 2052

4-[15-(Trifluoromethyl)pentadecylamino)benzaldehyde

A solution of 4-aminobenzonitrile (11.8 g.) and 15-(trifluoromethyl)pentadecyl bromide (15.3 g.) in hexamethylphosphoramide (200 ml.) is heated under an atmosphere of nitrogen in an oil bath maintained at 120°C. for 22 hours. The reaction mixture is cooled to room temperature

and water is added gradually. The mixture is then chilled in an ice-bath and filtered. The solid is washed thoroughly with water and dried. The solid is recrystallized from etherhexane to yield 4-[(15-trifluoromethyl)pentadecylamino)benzonitrile as a pale yellow solid.

Di-isobutylaluminum hydride (54 ml., 25% solution in toluene) is added with stirring to a solution of the nitrile under a nitrogen atmosphere. The temperature rises to 40°C. during the addition which takes 30 minutes and the reaction is then stirred for 1 hour. A solution of methanol in toluene (50 ml., 1:1) is added during 30 minutes and the mixture is poured into vigorously stirred ice-cold aqueous sulfuric acid (500 ml., 5%). After 10 minutes, diatomaceous earth (30 g.) is added, the mixture filtered and the organic layer separated. The aqueous solution is extracted twice with toluene (100 ml.) and the combined organic layers are washed with aqueous sodium bicarbonate, dried over magnesium sulfate decolorized with charcoal, filtered and evaporated *in vacuo* to give a light yellow crystalline solid. Recrystallization from hexane affords 4-[15-(trifluoromethyl)-pentadecylamino]benzaldehyde as a white solid.

Table XLIV

The following benzaldehydes are prepared from 4-aminobenzonitrile and the appropriate polyfluoroalkyl halide by the method of Example 2052.

| Example No. | Compound |
|---|---|
| 2053 | 4-[6-(Trifluoromethyl)hexylamino]benzaldehyde |
| 2054 | 4-[11-(Trifluoromethyl)undecylamino]benzaldehyde |
| 2055 | 4-[17-(Trifluoromethyl)heptadecylamino]benzaldehyde |
| 2056 | 4-[7-(Trifluoromethyl)undecylamino]benzaldehyde |
| 2057 | 4-[11-(Pentafluoroethyl)undecylamino]benzaldehyde |

| Example No. | Compound |
|---|---|
| 2058 | 4-[16-(Pentadecafluoroheptyl)pentylamino]-benzaldehyde |
| 2059 | 4-[5-(Pentadecafluoroheptyl)pentylamino]benz-aldehyde |
| 2060 | 4-[6,6-Difluorohexadecylamino)benzaldehyde |
| 2061 | 4-(14,14-Difluoropentadecylamino)benzaldehyde |
| 2062 | 4-[(Pentadecafluoroheptyl)methylamino]benz-aldehyde |
| 2063 | 4-[(Hentricontafluoropentadecyl)methylamino]-benzaldehyde |
| 2064 | 4-[15-(Trifluoromethyl)-12-pentadecenyl-amino]benzaldehyde |
| 2065 | 4-[15-(Trifluoromethyl)-4-pentadecynylamino]-benzaldehyde |
| 2066 | 4-[3-(Trifluoromethyl)-2,4,4-trimethylcyclo-hexylamino]benzaldehyde |

## EXAMPLE 2067

### 4-[15-(Trifluoromethyl)pentadecylamino]benzoyl chloride hydrochloride

A cold solution of 25 g. of 4-[15-(trifluoromethyl)pentadecylamino]benzoic acid in 500 ml. dimethoxyethane-methylene chloride (4:1) is prepared and dry hydrochloric acid is bubbled through the solution until no more precipitate forms. The solution is treated with 25 ml. thionyl chloride and refluxed until all of the precipitate has dissolved. The solvents are evaporated to yield 4-[15-(trifluoromethyl)pentadecylamino)benzoyl chloride hydrochloride as an orange, semi-crystalline mass.

## EXAMPLE 2068

### 4-[N-Trifluoroacetyl-15-(trifluoromethyl)-pentadecylamino]-benzoyl chloride

A stirred, ice-cold suspension of 9 g. 4-[15-(trifluoromethyl)pentadecylamino]benzoic acid in 100 ml. of dimethoxyethane and 16 ml. of pyridine is treated with 18 ml. of trifluoracetic anhydride at 0°C. The solution is

stirred at 0°C. for 30 minutes then at room temperature and then diluted with 300 ml. ether and 100 g. ice. After stirring vigorously for 15 minutes, the phases are separated, the ether solution is washed with brine, dried and evaporated to a white, amorphous solid.

To a solution of 9.2 g. of the above solid in 30 ml. methylene chloride and 0.5 ml. dimethylformamide is added 5.7 ml. thionyl chloride. After 20 hours at reflux, the solvents are evaporated to yield 4-[N-trifluoroacetyl-15-(tri-fluoromethyl)pentadecylamino]benzoyl chloride as a light yellow, mobile oil.

## EXAMPLE 2069

### 4-[N-Carbobenzyloxy-15-(trifluoromethyl)pentadecylamino]-benzoyl chloride

To 15 g. 4-[15-(trifluoromethyl)pentadecylamino]-benzoic acid in 200 ml. warm chloroform is added a solution of 15 g. of sodium carbonate in 150 ml. water. To the vigorously stirred solution is added 10 g. carbobenzyloxy chloride. After 2 hours stirring at 40°C., the layers are separated, washed three times with 1N hydrochloric acid, dried, and evaporated to an oil. The oil is dissolved in 300 ml. toluene, treated with 15 ml. thionyl chloride and the solution is refluxed for 5 hours. The solvents are evaporated and the residue is dissolved three times in toluene, evaporating each time, ultimately to yield 4-[N-carbobenzyloxy-15-(trifluoromethyl)pentadecylamino]benzoyl chloride as a viscous, orange oil.

## EXAMPLE 2070

### 1-{4-[N-tertr-Butyloxycarbonyl-15-(trifluoromethyl)pentadecylamino]benzoyl} imidazole

A solution of 10 g. 4-[15-(trifluoromethyl)pentadecylamino]benzoic acid in 100 ml. dioxane is treated with 4.0 g. *tert*-butylazidoformate and 10 ml. pyridine. After stirring at room temperature for 18 hours, the protected amido-acid is precipitated from solution by the addition of 150 ml. water. The solid is collected, and thoroughly dried, and dissolved in 200 ml. of a mixture consisting of

methylene chloride/dimethoxyethane/pyridine (1:4:1). To this solution is added 5.4 g. 1,1'-carbonyldiimidazole. The solution is stirred overnight at room temperature and the solvents are evaporated to yield 1- 4-[N-*tert*-butyloxycarbonyl-15-(trifluoromethyl)pentadecylamino]benzoyl imidazole as an orange oil.

EXAMPLE 2071

2,3-Dihydroxypropyl 4-[15-(trifluoromethyl)pentadecylamino]benzoate

A solution of 7.34 g. of 4-[15-(trifluoromethyl)-pentadecylamino]benzoic acid, 4.80 g. of 25% aqueous sodium hydroxide, and 12.6 g. of 3-iodo-1,2-propanediol in 50 ml. of hexamethylphosphoramide is stirred for 24 hours at ambient temperature, diluted with 100 ml. of ether and stirred for 5 days at ambient temperature. The mixture is treated with water and extracted with ether. The dried extracts are evaporated to yield the product as a white solid.

EXAMPLE 2072

Methyl 4-[15-(trifluoromethyl)pentadecylamino]benzoate

A solution of 7.20 g. of 4-[15-(trifluoromethyl)-pentadecylamino)benzoic acid in 25 ml. of hexamethylphosphoramide is added to a stirred mixture of 0.800 g. of sodium hydride (57% in mineral oil) and 25 ml. of hexamethylphosphoramide. The solution which forms after one hour is treated with 11.0 g. of methyl iodide and is then stirred at 25°C. for 18 hours. Dilution with water followed by filtration affords a white solid which is crystallized from ethanol to yield the product as a white solid.

EXAMPLE 2073

3-Hydroxypropyl 4-[15-(trifluoromethyl)pentadecylamino]-benzoate

A mixture of 2.25 g. of methyl 4-[15-(trifluoromethyl)pentadecylamino]benzoate, 280 mg. of 1,3-propanediol and 1.37 g. of *p*-toluenesulfonic acid is heated at 180°C. for 18 hours and then is partitioned between ether and 3% aqueous sodium carbonate solution. The ether layer is

separated, dried, and evaporated to yield the product as a white solid.

EXAMPLE 2074

2-Ethoxyethyl 4-[15-(trifluoromethyl)pentadecylamino benzoate

A solution of 11.8 g. of 4-[15-(trifluoromethyl)-pentadecylamino]benzoic acid, 1.00 g. of 2-ethoxyethanol and 5.35 ml. of boron trifluoride etherate in 200 ml. of toluene is stirred under reflux for 48 hours. The solution is treated with an additional 5.35 ml. of boron trifluoride etherate and refluxing is continued for 120 hours. Dilution with water and methylene chloride followed by filtration affords the product as a white solid.

EXAMPLE 2075

Isopropyl 4-[15-(trifluoromethyl)pentadecylamino]benzoate

A solution of 50.5 g. of 4-[15-(trifluoromethyl)-pentadecylamino]benzoic acid and 34.4 ml. of boron trifluoride etherate in 200 ml. of isopropyl alcohol is stirred under reflux for 44 hours, allowed to cool, and poured into 1.20 liters of ice cold 5% aqueous sodium carbonate solution. The white solid is collected by filtration and recrystallized from benzene-ethanol to yield the product as a white solid.

EXAMPLE 2076

1-(Methoxycarbonyl)propyl 4-[15-(trifluoromethyl)pentadecylamino]benzoate

To a solution of 10.0 g. 4-[15-(trifluoromethyl)-pentadecylamino]benzoyl chloride hydrochloride in 200 ml. methylene chloride is added dropwise a solution of 3 g. methyl 2-hydroxybutyrate and 5 g. triethylamine in 100 ml. ether. After 17 hours stirring at room temperature, the precipitate is collected and washed with several portions of ether. The ether solution is washed with water, dried and evaporated to yield the product as a white solid.

EXAMPLE 2077

1-(Ethoxycarbonyl)ethyl 4-[15-(trifluoromethyl)pentadecylamino]benzoate

To a warm mixture of 7 g. sodium 4-[15-(trifluoromethyl)pentadecylamino)benzoate in 100 ml. ethanol is added 4.7 g. ethyl 2-tosyloxypropionate. After 17 hours at reflux, the cooled solution is diluted with an equal volume of water and the resultant precipitate is filtered. After washing with cold ethanol and drying, the product is crystallized from acetonitrile to yield the product as colorless crystals.

EXAMPLE 2078

1-Carboxyethyl 4-[15-(trifluoromethyl)pentadecylamino]-benzoate

A flask containing 10.0 g. 4-[15-(trifluoromethyl)pentadecylamino]benzoic acid, 3.3 g. lactic acid, and 500 ml. toluene is equipped with a Soxhlet extractor charged with activated 4A Linde molecular sieves. The solution is refluxed for 24 hours, during which time the Soxhlet extractor is charged twice more with fresh sieves. The hot solution is filtered and allowed to cool, whereupon the product separates as off-white crystals.

EXAMPLE 2079

Diethyl O-{4-[15-(trifluoromethyl)pentadecylamino]benzoyl}-tartrate

A mixture of 4-[N-trifluoroacetyl-15-(trifluoromethyl)pentadecylamino)benzoyl chloride and 1.2 g. triethylamine in 100 ml. warm ether is treated with 2.5 g. diethyl tartrate and refluxed for 24 hours. The hot solution is filtered, the residue is washed with hot ether, and the solution is evaporated. After treatment with aqueous methanolic potassium carbonate, the product is precipitated by acidification, filtered, and dried. Crystallization from acetone yields the product as a white, crystalline solid.

EXAMPLE 2080

O-{4-[15-(Trifluoromethyl)pentadecylamino]benzoyl}malic acid

A warm solution of 4-[N-carbobenzyloxy-15-(trifluoromethyl)pentadecylamino]benzoyl chloride and 1.3 g. triethylamine in 100 ml. ether is treated with 2 g. malic acid. An immediate precipitate forms, but the mixture is refluxed for one hour and filtered while hot. The solid is washed several times with hot ether, then the ether is evaporated to yield a white solid. The product is dissolved in tetrahydrofuran (100 ml.) and hydrogenated over 600 mg. 10% palladium-on-carbon at 50 psi until hydrogen uptake stops. The catalyst is filtered and the solution is evaporated. The residue is crystallized from acetic acid to yield the product as a white solid.

EXAMPLE 2081

2-(Ethoxycarbonyl)vinyl 4-[15-(trifluoromethyl)pentadecylamino]benzoate

To a mixture containing 4.3g. 1-{4-[N-*tert*-butyloxycarbonyl-15-(trifluoromethyl)pentadecylamino]benzoyl}-imidazole, in 50 ml. 5*N* sodium hydroxide is added 3 g. ethyl 2-formyl acetate. The solution is vigorously stirred for 24 hours. The layers are separated, and the chloroform solution is washed once with 50 ml. 1*N* sodium hydroxide. The solvent is evaporated and the residue is heated for 30 minutes at 40°C. in 50 ml. anhydrous trifluoroacetic acid. The solvent is again evaporated and the residue is heated for 30 minutes at 40°C. in 50 ml. anhydrous trifluoroacetic acid. The solvent is again evaporated and the oil is crystallized from acetone to yield light yellow crystals of the product.

Table XLV

The following benzoate esters are prepared from the carboxylic acids of Table XLIII (or activated derivatives thereof prepared by the methods of Examples 2067-2070 by the methods of Examples 2071-2081 as shown in the table.

| Example No. | Method of Example | Compound |
|---|---|---|
| 2082 | 2071 | 2,3-Dihydroxypropyl 4-[8-(tri-fluoromethyl)octylamino]benzoate |
| 2083 | 2071 | 2,3-Dihydroxypropyl 4-[16-(penta-fluoroethyl)hexadecylamino]benzoate |
| 2084 | 2071 | 2,3-Dihydroxypropyl 4-[15,15-(di-fluoro)hexadecylamino]benzoate |
| 2085 | 2071 | 2,3-Dihydroxypropyl 4-[6-(penta-decafluoroheptyl)-5-hexenylamino]-benzoate |
| 2086 | 2072 | Methyl 4-[12-(trifluoromethyl)dode-cylamino]benzoate |
| 2087 | 2072 | Methyl 4-[1-hexyl-9-(trifluoro-methyl)-4-nonynylamino]benzoate |
| 2088 | 2072 | Methyl 4-[11,11-difluoro-16-tri-fluoromethyl)hexadecylamino]ben-zoate |
| 2089 | 2072 | Methyl 4-[15,15-(difluoro)hexa-decylamino]benzoate |
| 2090 | 2073 | 3-Hydroxypropyl 4-[15-(trifluoro-methyl)-7-pentadecenylamino]ben-zoate |
| 2091 | 2073 | 2-Hydroxypropyl 4-[1-(2,2,2-tri-fluoroethyl)pentadecylamino]ben-zoate |
| 2092 | 2073 | 4-Hydroxybutyl 4-[12-(trifluoro-methyl)dodecylamino]benzoate |
| 2093 | 2073 | 3-Hydroxypropyl 4-[11,11-(difluoro)-dodecylamino]benzoate |
| 2094 | 2074 | 2-Ethoxyethyl 4-[7-(pentadecafluoro-heptyl)heptylamino]benzoate |
| 2095 | 2074 | 3-Methoxypropyl 4-[11-(trifluoro-methyl)undecylamino]benzoate |
| 2096 | 2074 | 2-Ethoxyethyl 4-[3-(2,2,2-trifluoroethyl)-2,4,4-trimethylcyclopentyl-amino]benzoate |

| Example No. | Method of Example | Compound |
|---|---|---|
| 2097 | 2075 | Isopropyl 4-[11-(trifluoromethyl)-undecylamino]benzoate |
| 2098 | 2076 | 1-(Methoxycarbonyl)propyl 4-[6-(trifluoromethyl)hexylamino]benzoate |
| 2099 | 2076 | 1-(Methoxycarbonyl)ethyl 4-[11-(heptafluoroisopropyl)undecylamino]benzoate |
| 2100 | 2076 | 1-(Ethoxycarbonyl)propyl 4-(15,15-difluorohexadecylamino)benzoate |
| 2101 | 2076 | 1-(Methoxycarbonyl)propyl 4-[3-(trifluoromethyl)-2,4,4-trimethylcyclohexylamino]benzoate |
| 2102 | 2077 | 1-(Ethoxycarbonyl)propyl 4-[11,11-difluoro-16-(trifluoromethyl)hexadecylamino]benzoate |
| 2103 | 2077 | 1-(Methoxycarbonyl)ethyl 4-[11-(trifluoromethyl)undecylamino]benzoate |
| 2104 | 2078 | 1-Carboxyethyl 4-[11-(heptafluoropropyl)undecylamino]benzoate |
| 2105 | 2078 | 1-Carboxyethyl 4-[5-(pentadecafluoroheptyl)-4-pentenylamino]benzoate |
| 2106 | 2079 | Diethyl O-{4-[(hentricontafluoropentadecyl)methylamino]benzoyl}tartrate |
| 2107 | 2079 | Diethyl O-{4-[12-fluoro-12-(heptafluoropropyl)-12-(trifluoromethyl)-dodecylamino]-benzoyl}tartrate |
| 2108 | 2079 | Diethyl O-[4-(6,6-difluorohexadecylamino)benzoyl]tartrate |
| 2109 | 2080 | O-{4-[3,7-Dimethyl-7-(trifluoromethyl)octylamino]benzoyl}malic acid |
| 2110 | 2080 | O-{4-[15-(trifluoromethyl)-7-pentadecenylamino]benzoyl}malic acid |
| 2111 | 2081 | 2-(Ethoxycarbonyl)vinyl 4-[11-(trifluoromethyl)-9-undecenylamino]benzoate |

| Example No. | Method of Example | Compound |
|---|---|---|
| 2097 | 2075 | Isopropyl 4-[11-(trifluoromethyl)-undecylamino]benzoate |
| 2112 | 2081 | 2-(Ethoxycarbonyl)vinyl 4-[11-(tri-fluoromethyl)undecylamino]benzoate |
| 2113 | 2081 | 2-(Ethoxycarbonyl)vinyl 4-[5-(penta-decafluoroheptyl)-4-pentenylamino]-benzoate |

EXAMPLE 2114

1-{4-[15-(Trifluoromethyl)pentadecylamino]benzoyl}-piperdine

To a chilled solution of 35 ml. of piperidine, 2.5 ml. of triethylamine and 0.6 g. of dimethylaminopyridine in 100 ml. of diethyl ether is added (1/2 hour) a solution of 8.3 g. of 4-[15-(trifluoromethyl)pentadecylamino]benzoyl chloride hydrochloride in 50 ml. of ether. The solution is warmed to room temperature and maintained there for two hours. The solution is heated to reflux for an aoditional 2 hours at which time the reaction is complete. The solution is cooled, extracted twice with water and dried. The solvent is removed *in vacuo* and the solid is recrystallized from diethyl ether to yield the product as a white solid.

EXAMPLE 2115

Ethyl 4-[15-(trifluoromethyl)pentadecylamino]hippurate

To a solution of 18.0 g. of 4-[15-(trifluoromethyl)pentadecylamino]benzoic acid in a mixture of dioxane and methylene chloride is added gaseous hydrogen chloride for 10 minutes. The slurry is cooled and 18 ml. of thionyl chloride is added. The slurry is brought to reflux for 2 hours and then concentrated under vacuum (thrice diluting with dioxane each time). The amber solution is diluted with 100 ml. of dioxane and this solution is added to freshly prepared ethyl glycinate in 300 ml. of methylene chloride containing 1 g. of dimethylaminopyridine and 10 ml. of triethylamine. After 16 hours at room temperature the reaction is refluxed for 2 hours, cooled and filtered. The

mother liquor is extracted with water and 10% hydrochloric acid. The solution is dried and concentrated in vacuo to an amber liquid. A sample is pre-absorbed on silica and eluted with ether. Evaporation of the eluate yields a solid which is recrystallized from acetonitrile to yield the product as a white solid.

EXAMPLE 2116

N-{4-[15-(Trifluoromethyl)pentadecylamino]benzoyl}glycine

A mixture of 26.4 g. of ethyl 4-[15-(trifluoromethyl)pentadecylamino]hippurate, 110 ml. of 1N sodium hydroxide solution, and 200 ml. of ethanol is stirred at ambient temperature for 2 hours and then partially evaporated. The aqueous solution is washed with diethyl ether, acidified with 6N hydrochloric acid, and filtered. The solid is dried in vacuo and recrystallized from acetone to yield the product as a white solid.

EXAMPLE 2117

4-[15-(Trifluoromethyl)pentadecylamino]-N-(phenylsulfonyl)-benzamide

A solution of 31.4 g. of benzenesulfonamide in 250 ml. of dry dimethylacetamide is added dropwise, with stirring and cooling, to a suspension of 5.5 g. of sodium hydride in 100 ml. of dry dimethylacetamide during 30 minutes at room temperature. Stirring is continued for 30 minutes. In the meantime, a mixture of 36.2 g. of 4-[15-(trifluoromethyl)pentadecylamino] benzoic acid in 100 ml. of methylene chloride, 300 ml. of dimethoxyethane, and 40 ml. of thionyl chloride is refluxed for 1 hour and 15 minutes. The solution is evaporated and to the resulting oil residue is added, in one portion, the previously prepared mixture of sodium benzenesulfonamide in dimethylacetamide. The mixture is stirred for 30 minutes, without cooling, and then filtered. The filtrate is poured into 2 liters of water and 250 ml. of saturated sodium chloride solution. The product is collected by filtration and then dissolved in methylene chloride, the mixture is filtered through diatomaceous earth, and brine is added to break the emulsion.

The layers are separated, the organic phase is dried and evaporated. The residue is crystallized from toluene to yield the product as a white solid.

EXAMPLE 2118

N-{4-[15-(Trifluoromethyl)pentadecylamino]benzoyl} methanesulfonamide

A solution of 25.2 g. of 4-[15-(trifluoromethyl)-pentadecylamino]benzoyl chloride hydrochloride and 5.6 g. of methanesulfonamide in 250 ml. of pyridine is stirred under reflux for 2 hours and then concentrated *in vacuo*. Tbe residue is partitioned between water and diethyl ether; tbe aqueous layer acidified with 1N hydrochloric acid, and the organic layer separated, dried over magnesium sulfate and evaporated. Crystallization of the residual white solid from 60% aqueous acetate acid and then from methylene chloride-hexane affords tbe product as a white solid.

EXAMPLE 2119

N-{4-[15-(Trifluoromethyl)pentadecylamino)benzoyl} alanine

A solution of 4.75 g. of 4-[N-trifluoroacetyl-15-(trifluoromethyl)pentadecylamino benzoyl chloride and 1.2 g. of triethylamine in 200 ml. of warm ether is treated with 1.55 g. alanine ethyl ester and refluxed for 24 hours. The hot solution is filtered, the residue is washed with hot ether, and the solution is evaporated. After treatment with aqueous methanolic potassium carbonate, the product is precipitated by acidification, filtered, and dried. Crystallization from acetone yields the product as a white, crystalline solid.

EXAMPLE 2120

N-{4-[15-(Trifluromethyl)pentadecylamino]benzoyl} benzamide

One gram of a 50% oil disperson of sodium hydride is washed with petroleum ether by decantation, dried, and suspended in 5 ml. of tetrahydrofuran. To this stirred mixture is added a solution of 2.42 g. of benzamide in 5 ml. of tetrahydrofuran in one portion. An initial hydrogen evolution is observed. While stirring (30 minutes), the sodium hydride gradually disappears and a white, milky,

turbid mixture forms. A solution of 0.9 g. of 4-[N-trifluoroacetyl-15-(trifluoromethyl)pentadecylamino]benzoyl chloride in 3 ml. of tetrahydrofuran is added dropwise during 5 minutes to the mixture. The whole milky mixture is stirred at room temperature under nitrogen for one hour. The mixture is then poured into water and extracted with ether. The ether is extracted with water and brine and dried over sodium sulfate. Evaporation of the solvent affords a pale yellow solid. The solid is recrystallized from ether/acetonitrile (50/50) and then from acetronitrile to yield the product as a white solid.

EXAMPLE 2121

1-{4-[15-(Trifluoromethyl)pentadecylamino]benzoyl}-pyrrolidine

To a warm solution of 4-[N-carbobenzoyloxy-15-(trifluoromethyl)pentadecylamino]benzoyl chloride and 1.3 g. of triethylamine in 100 ml. ether is added 1.2 g. of pyrrolidine. An immediate precipitate forms, the mixture is refluxed for one hour and then filtered. The solid is extracted several times with hot ether, and the ether is evaporated to yield a white solid. The solid is dissolved in tetrahydrofuran (100 ml.) and hydrogenated over 600 mg. 10% palladium-on-carbon at 50 psi. until hydrogen up-take stops. The catalyst is filtered and the filtrate evaporated. The residue is crystallized from acetic acid to yield the product as a white solid.

EXAMPLE 2122

N-(2,3-Dihydroxypropyl)-4-[15-(trifluoromethyl)pentadecylamino]benzamide

To a mixture containing 4.3 g. of 1- 4-[N-(tert-butyloxycarbonyl)-4-[15-(trifluoromethyl)pentadecylamino]-benzoyl imidazole, 50 ml. of chloroform, and 50 ml. of 5N sodium hydroxide is added 1.1 g. of 3-amino-1,2-propanediol. The solution is vigorously stirred for 24 hours, the layers are separated, and the chloroform solution is washed once with 50 ml. of 1N sodium hydroxide. The solvent is evaporated and the residue is heated for 30 minutes at 40°C. in

50 ml. of anhydrous trifluoroacetic acid. The solvent is again evaporated and the oil is crystallized from acetone to yield the product as a light yellow solid.

TABLE XLVI

The following benzamides are prepared from the carboxylic acids of Table XLIII (or activated derivatives thereof prepared by the methods of Examples 2067-2070) by the methods of Examples 2114-2122 as shown in the table.

| Example No. | Method of Example | Compound |
|---|---|---|
| 2123 | 2114 | 1- 4-[11-(Trifluoromethyl)undecylamino]benzoyl piperidine |
| 2124 | 2114 | 1- 3-[12-Fluoro-12-(heptafluoropropyl)-12-(trifluoromethyl)dodecylamino]benzoyl pyrrolidine |
| 2125 | 2114 | 1- 4-[5-(Pentadecafluoroheptyl)-4-pentenylamino]benzoyl -piperidine |
| 2126 | 2114 | 1- 4-[3-(2,2,2-Trifluoroethyl)-2,4,4-trimethylcyclopentylamino]benzoyl pyrrolidine |
| 2127 | 2115 | Ethyl 4-[5-(pentadecafluoroheptyl)pentylamino]hippurate |
| 2128 | 2115 | Ethyl 4-[7-(trifluoromethyl)-heptylamino]hippurate |
| 2129 | 2115 | Ethyl 4-[1-hexyl-9-(trifluoromethyl)-4-nonynylamino]hippurate |
| 2130 | 2115 | Ethyl 4-[3-(trifluoromethyl)-2,4,4-trimethylcyclohexylamino]hippurate |
| 2131 | 2116 | N-{4-[5-(pentadecafluoroheptyl)-pentylamino]benzoyl} glycine |
| 2132 | 2116 | N-{4-[7-(trifluoromethyl)heptylamino]benzoyl}glycine |
| 2133 | 2116 | N-{4-[1-hexyl-9-(trifluoromethyl)-4-nonynylamino]benzoyl} glycine |
| 2134 | 2116 | N-{4-[3-(trifluoroethyl)-2,4,4-trimethylcyclohexylamino]benzoyl}-glycine |

| Example No. | Method of Example | Compound |
|---|---|---|
| 2135 | 2117 | 4-[14,14-(difluoro)pentadecyl-amino]-N-(phenylsulfonyl)benzamide |
| 2136 | 2117 | 4-[15-triflurometbyl)-4-penta-decynylamino]-N-(phenylsulfonyl)-benzamide |
| 2137 | 2117 | 4-[18-(Trifluoromethyl)octadecyl-amino]-N-(phenysulfonyl)benzamide |
| 2138 | 2117 | 4-[11-(Nonafluorobuty)undecyl-amino]-N-(phenylsulfonyl)benzamide |
| 2139 | 2118 | N-{4-[11-(Undecafluoropentyl)un-decylamino]benzoyl}methanesulfon-amide |
| 2140 | 2118 | N-{4-[11,11-Difluoro-16-(tri-fluoromethyl)hexadecylamino]ben-zoyl}methanesulfonamide |
| 2141 | 2118 | N-[4-(15,15-Difluorohexadecyl-amino)benzoyl]methanesulfonamide |
| 2142 | 2119 | N-{4-[15-(Trifluoromethyl)-4-pentadecynylamino]benzoyl}alanine |
| 2143 | 2119 | N-{4-[7-(Trifluoromethyl)heptyl-amino]benzoyl}alanine |
| 2144 | 2120 | N-{4-[1-(2,2,2-Trifluoroethyl)-pentadecylamino]benzoyl}benzamide |
| 2145 | 2120 | N-[4-(11,11-Difluorododecylamino)-benzoyl]benzamide |
| 2146 | 2120 | N-{4-[(Hentricontafluoropenta-decyl)methylamino]benzoyl}benz-amide |
| 2147 | 2121 | 1-{4-[16-(Pentafluoroethyl)hexa-decylamino]benzoyl}pyrrolidine |
| 2148 | 2121 | 1-{4-[3-(Trifluoromethyl)-2,4,4-trimethylcyclohexylamino]benzoyl}-piperidine |
| 2149 | 2122 | N-(2,3-Dihydroxypropyl)-4-(16,16-difluoroheptadecylamino)benzamide |

| Example No. | Method of Example | Compound |
|---|---|---|
| 2150 | 2122 | N-(2,3-Dihydroxypropyl)-4-[1-(2,2,2-trifluoroethyl)pentadecylamino]-benzamide |

## EXAMPLE 2151

### Diethyl 4-[15-(trifluoromethyl)pentadecylamino]benzoylmalonate

A solution of 26.6 g. of diethyl malonate and 10 ml. of 1.2-dimethoxyethane is added to a suspension of 4.0 g. of sodium hydride in 1,2-dimethoxyethane under argon. A solution of 17.3 g. of 4-[15-(trifluoromethyl)pentadecylamino]benzoyl chloride hydrochloride in 1,2-dimethoxyethane is then added. The reaction mixture is refluxed for 4.5 hours, cooled, poured on ice, acidified, and extracted with ether. The ether solution is washed with water and saturated sodium chloride solution, dried over anhydrous sodium sulfate and concentrated to dryness. Addition of a small amount of ethanol to the residue gives a solid which is filtered and discarded. The ethanol filtrate is concentrated and the residue is recrystallized from ether to yield the product.

## EXAMPLE 2152

### *tert*-Butyl ethyl 4-[15-(trifluoromethyl)pentadecylamino]-benzoylmalonate

A solution of 28.0 g. of tert-butyl ethyl malonate in 10 ml. of 1,2-dimethoxyethane is added to a suspension of 4.0 g. of sodium hydride in 1,2-dimethoxyethane under argon. A solution of 17.3 g. of 4-[15-(trifluoromethyl)pentadecylamino]benzoyl chloride hydrochloride in 1,2-dimethoxyethane is then added. The reaction mixture is refluxed for 5 hours, cooled, poured on ice and extracted with ether. The ether solution is washed with water and saturated sodium chloride solution, dried over anhydrous sodium sulfate and concentrated to dryness. The residue is then recrystallized from ether to yield the product.

EXAMPLE 2153

Ethyl 2-{4-[15-(trifluoromethyl)pentadecylamino]benzoyl}-acetoacetate

A solution of 21.6 g. of ethyl acetoacetate and 10 ml. of 1,2-dimethoxyethane is added to a suspension of 4.0 g. of sodium hydride in 1,2-dimethoxyethane under argon. A solution of 17.3 g. of 4-[15-(trifluoromethyl)pentadecylamino]benzoyl chloride hydrochloride in 1,2-dimethoxyethane is then added. The reaction mixture is refluxed for 5 hours, cooled, poured on ice and extracted with ether. The ether solution is washed with water and saturated sodium chloride solution, dried over anhydrous sodium sulfate and concentrated to dryness. Recrystallization from ether affords the product as a white solid.

EXAMPLE 2154

Ethyl 4-[15-(trifluoromethyl)pentadecylamino]benzoylacetate

A solution of 3.0 g. of tert-butyl ethyl 4-[15-(triflurometnyl)pentadecylamino]benzoylmalonate and 10 ml. of trifluoroacetic acid is warmed with stirring for 3 hours. The solution is poured onto ice and neutralized with potassium hydroxide. Tbe resulting precipitate is collected by filtration, washed with water and dried. Recrystallization from chloroform affords the product as a white solid.

EXAMPLE 2155

4-[15-(Trifluoromethyl)pentadecylamino]benzoylacetic acid

Two grams of ethyl 4-[15-(trifluoromethyl)pentadecylamino]benzoylacetate is added to a solution of potassium hydroxide in 50 ml. of 1·9 water-ethanol. The reaction mixture is stirred for 24 hours at room temperature. Careful neutralization with sulfuric acid gives a precipitate which is filtered, washed with water, and dried to yield the product.

EXAMPLE 2156

4'-[15-(Trifluoromethyl)pentadecylamino]-2-(methylsulfinyl)-acetophenone

To a solution of 5.8 g. of dimethyl sulfoxide, dried over sieves, and 50 ml. of tetrahydrofuran is slowly

added 28 ml. of n-butyl lithium (2.42 M in hexane). To this mixture is added 10 g. of methyl 4-[15-(trifluoromethyl)pentadecylamino]benzoate in 200 ml. of tetrahydrofuran. After two hours, the reaction mixture is poured onto ice, acidified with dilute hydrochloric acid and quickly extracted with chloroform. The chloroform extract is washed with water and saturated sodium chloride solution and dried over anhydrous sodium sulfate. Concentration affords a solid which is washed with hexane. The white solid is dried in vacuo to yield the product.

## EXAMPLE 2157

### 4'-[15-(Trifluoromethyl)pentadecylamino]-2-(phenylsulfonyl)-acetophenone

A solution of 864 mg. of sodium hydride and 5.3 g. of methylphenylsulfone in 20 ml. of 1,2-dimethoxyethane is stirred at 60°C. for one hour under an atmosphere of argon. To this solution is then added a solution of 5.0 g. of methyl 4-[15-(trifluoromethyl)pentadecylamino]benzoate and 50 ml. of tetrahydrofuran and the reaction mixture is stirred at 60°C. for 1.5 hours. The mixture is cooled, poured onto ice, acidified with dilute hydrochloric acid to pH 3 and then extracted with chloroform. The organic layer is separated, washed three times with water and saturated sodium chloride solution, dried over anhydrous sodium sulfate and then concentrated to dryness. The crude solid is chromatographed on silica gel, eluting with methylene chloride to yield the product.

## EXAMPLE 2158

### 4'-[15-Trifluoromethyl)pentadecylamino]-2-(phenylsulfinyl)-acetophenone

To a solution of 6.2 g. of methylphenylsulfoxide, dried over sieves, and 50 ml. of tetrahydrofuran is slowly added 28 ml. of n-butyl lithium (2.42 M in hexane). To this mixture is added 10 g. of methyl 4-[15-(trifluoromethyl)pentadecylamino]benzoate in 200 ml. of tetrahydrofuran. After two hours, the reaction mixture is poured onto ice, acidified with diluted hydrochloric acid and quickly extracted

with chloroform. The chloroform layer is washed with water and saturated sodium chloride solution and dried over anhydrous sodium sulfate. Concentration affords a solid which is washed with 500 ml. of hot hexane, filtered while hot, and then washed with 50 ml. of hexane. The white solid is dried *in vacuo* yielding the product.

EXAMPLE 2159

3-{4-[15-(Trifluoromethyl)pentadecylamino]benzoyl}-2,4-pentanedione

A solution of 28.4 g. of 2,4-pentanedione and 20 ml. of 1,2-dimethoxyethane is added to a suspension of 13.6 g. of sodium hydride in 220 ml. of 1,2-dimethoxyethane under argon. A solution of 28.7 g. of 4-[15-(trifluoromethyl)pentadecylamino]benzoyl chloride hydrochloride in 1,2-dimethoxyethane is then added. The reaction mixture is stirred at room temperature for 12 hours, cooled, poured on ice and extracted with ether. The ether solution is washed with water and saturated sodium chloride solution, dried over anhydrous sodium sulfate and concentrated. The residue is then chromatographed over silica gel to yield the product as a white solid.

EXAMPLE 2160

3-{4-[15-(Trifluoromethyl)pentadecylamino]benzoyl}propionic acid

A mixture of 35 g. of 3-(4-acetamidobenzoyl)propionic acid, 700 ml. of methanol and 1.4 ml. of concentrated sulfuric acid is refluxed for 76 hours. The solution is cooled to 35°C. and poured onto 7 g. of anhydrous sodium acetate while stirring. The reaction mixture is stirred in an ice-bath. The solid is collected and washed with cold methanol to yield methyl 3-(4-aminobenzoyl)propionate as a white solid. A mixture of this solid, 9.2 g. of 15-(trifluoromethyl)pentadecyl bromide and 4.2 g. of potassium carbonate is stirred for 20 hours at 125°C. under nitrogen. The mixture is then cooled to 25°C. and 30 ml. of water is added. After stirring, the product is filtered and washed with water. Recrystallization from methanol affords

methyl 3-{4-[15-(trifluoromethyl)pentadecylamino]benzoyl}-propionate as a white solid.

A solution of 5.4 g. of methyl 3-{4-[15-(trifluoromethyl)pentadecylamino]benzoyl}propionate is stirred with 5.4 g. of potassium hydroxide in 100 ml. of 95% ethanol for 3 hours at reflux. The reaction mixture is cooled, diluted with 50 ml. of ethanol and 100 ml. of water, and neutralized with hydrochloric acid. The solution is cooled to room temperature and filtered. The white solid is washed with 50% aqueous ethanol and dried. The product is recrystallized from ethanol to yield 3-{4-[15-(trifluoromethyl)-pentadecylamino]benzoyl}propionic acid as a white crystalline solid.

TABLE XLVII

The following benzamides are prepared from the benzoic acids and benzoate esters of Tables XLIII and XLIV (or activated derivatives thereof prepared by the methods of Examples 2067-2070) by the methods of Examples 2151-2160 as shown in the table.

| Example No. | Method of Example | Compound |
|---|---|---|
| 2161 | 2151 | Diethyl 4-[(hentricontafluoropentadecyl)methylamino]benzoylmalonate |
| 2162 | 2151 | Diethyl 4-[15-(trifluromethyl)-12-pentadecenylamino]benzoylmalonate |
| 2163 | 2152 | *tert*-Butyl ethyl 4-(11,11-difluorododecylamino)benzoylmalonate |
| 2164 | 2152 | *tert*-Butyl ethyl 4-[12-fluoro-12-(heptafluoropropyl)-12-(trifluoromethyl)dodecylamino]benzoylmalonate |
| 2165 | 2153 | Ethyl 2- 4-[9-(trifluoromethyl)-nonylamino]benzoyl acetoacetate |
| 2166 | 2153 | Ethyl 2-{4-[11,11-difluoro-16-(trifluoromethyl)hexadecylamino]benzoyl}-acetoacetate |

| Example No. | Method of Example | Compound |
|---|---|---|
| 2167 | 2154 | Ethyl 4-(16,16-difluoroheptadecylamino)benzoylacetate |
| 2168 | 2154 | Ethyl 4-[12-fluoro-12-(heptafluoropropyl)-12-(trifluoromethyl)dodecylamino]benzoylacetate |
| 2169 | 2155 | 4-(16,16-Difluoroheptadecylamino)-benzoylacetic acid |
| 2170 | 2155 | 4-[12-Fluoro-12-(heptafluoropropyl)-12-(trifluoromethyl)dodecylamino]benzoylacetic acid |
| 2171 | 2156 | 4'-[11-(Nonafluorobutyl)undecylamino]-2-(methylsulfinyl)acetophenone |
| 2172 | 2157 | 4'-[12-(Trifluoromethyl)dodecylamino]-2-(methylsulfinyl)acetophenone |
| 2173 | 2157 | 4'-[3-(Trifluoromethyl)-2,4,4-trimethylcyclohexylamino]-2-(methylsulfiny)acetophenone |
| 2174 | 2158 | 4'-(14,14-Difluoropentadecylamino)-2-(phenylsulfonyl)acetophenone |
| 2175 | 2158 | 4'-[1-(2,2,2-Trifluoroethyl)decylamino]-2-(phenylsulfonyl)acetophenone |
| 2176 | 2159 | 4'-[7-(Trifluoromethyl)undecylamino]-2-(phenylsulfinyl)acetophenone |
| 2177 | 2159 | 4'-[1-Hexyl-11-(trifluoromethyl)undecylamino]-2-(phenylsulfiny)acetophenone |
| 2178 | 2160 | 3-{4-[13-(Trifluoromethyl)tridecylamino]benzoyl}-2,4-pentanedione |
| 2179 | 2160 | 3-[4-(15,15-Difluorohexadecylamino)benzoyl]-2,4-pentanedione |
| 2180 | 2161 | 3-{4-[3-(2,2,2-Trifluoroethyl)-2,4,4-trimethylcyclopentylamino]benzoyl}-propionic acid |

| Example No. | Method of Example | Compound |
|---|---|---|
| 2181 | 2161 | 3-[4-[12-Fluoro-12-heptafluoropropyl)-12-(trifluoromethyl)dodecylamino]benzoyl]propionic acid |

EXAMPLE 2182

4-[15-(Trifluoromethyl)pentadecylamino]phenylacetic acid

A solution of 8.2 g. of 4-aminophenylacetic acid, 150 ml. of absolute ethanol, and 3 ml. of boron trifluoride concentrated by distillation and then evaporated to dryness *in vacuo*. The residue is dissolved in ethyl ether, washed with aqueous sodium bicarbonate dried and evaporated to yield ethyl 4-aminophenylacetate. A mixture of 5.0 g. of this amine, 9.4 g. of 15-(trifluoromethyl)pentadecyl bromide, 4.2 g. of anhydrous potassium carbonate and 40 ml. of hexamethylphosphoramide is heated at 80°C. for 7 hours. The mixture is then cooled, diluted with water, and extracted with ethyl ether. The ether extracts are washed with water, dried and evaporated. The residue is recrystallized from a mixture of chloroform and hexane, yielding ethyl 4-[15-(trifluoromethyl)pentadecylamino]phenylacetate. A mixture of 6.0 g. of this ester, 7.0 g. of potassium hydroxide and 100 ml. of ethanol-water is heated to reflux for 4 hours. While hot, the mixture is adjusted to pH 7 with concentrated hydrochloric acid. The mixture is diluted with water, cooled and filtered. Recrystallization of the precipitate yields the product as a white solid.

EXAMPLE 2183

4-[15-trifluoromethyl)pentadecylamino]hydrocinnamic acid

A mixture of 5.0 g. of 4-nitrocinnamic acid and 100 mg. of 10% palladium-on-carbon in 200 ml. of ethanol containing 5 drops of 5.5*N* ethanolic hydrogen chloride is treated with hydrogen in a Parr apparatus at room temperature for 3 hours. The mixture is then filtered through celite and the filtrate is concentrated, affording 4-aminohydrocinnamic acid.

A solution of 10.0 g. 4-aminohydrocinnamic acid in 100 ml. of absolute ethanol containing 16 ml. of boron trifluoride etherate is heated to reflux for 48 hours. The solution is then cooled, poured into 5% aqueous sodium carbonate, and extracted with methylene chloride. Evaporation of the organic extracts yields ethyl 4-aminohydrocinnamate.

In a manner directly analogous to that described in Example 2002, ethyl 4-aminohydrocinnamate is alkylated with 15-(trifluoromethyl)pentadecyl bromide to form ethyl 4-[15-(trifluoromethyl)pentadecylamino]hydrocinnamate. Subsequently, in a manner directly analogous to that described in Example 2003, ethyl 4-[15-(trifluoromethyl)-pentadecylamino]hydrocinnamate is hydrolyzed to 4-[15-(trifluoromethyl)pentadecylamino hydrocinnamic acid.

EXAMPLE 2184

4-[15-(Trifluoromethyl)pentadecylamino]cinnamic acid

A mixture of ethyl 4-aminocinnamate, one equivalent of 15-(trifluoromethyl)pentadecyl bromide and one equivalent of anhydrous potassium carbonate in hexamethylphosphoramide is heated for 20 hours at 60°C. The mixture is then cooled, diluted with water and extracted with ether. The combined ether extracts are dried, filtered and evaporated to provide ethyl 4-[15-(trifluoromethyl)pentadecylamino]cinnamate. The ester is hydrolyzed with sodium hydroxide in a 1·9 water:ethanol solution at steam bath temperature for 10 hours. The hot solution is then acidified with acetic acid, cooled and filtered and the solid is washed with water. Recrystallization from chloroform yields the product as a white solid.

EXAMPLE 2185

4-[15-(Trifluoroethyl)pentadecylamino]phenylpropiolic acid

A sample of 50 g. of ethyl 4-aminocinnamate is dissolved in 500 ml. of ethyl ether and a solution of 28 g. of trifluoroacetic anhydride in 30 ml. of ether is added dropwise. When the addition is complete, the reaction is allowed to stir for another hour. The mixture is then

diluted with hexane and filtered, providing ethyl 4-trifluoroacetamidocinnamate.

A solution of 40 g. of ethyl 4-trifluoroacetamidocinnamate in 200 ml. of carbon tetrachloride is cooled in ice. Bromine (28 g.) is added dropwise, the reaction is allowed to stir for one additional hour and then the solvent is evaporated. The crystalline residue is the dibromo ester.

A solution of 11.4 g. of potassium hydroxide in 300 ml. of 95% ethanol is cooled to 40°C. and 20 g. of the crude dibromo ester above is added. After 30 minutes, the reaction is heated to reflux for five hours. The solution is then cooled and filtered. The filtrate is treated with acetic acid until the solution is neutral to litmus, then concentrated, chilled and filtered, to yield 4-aminophenylpropiolic acid.

The 4-aminophenylpropiolic acid is converted to 4-[15-(trifluoromethylpentadecylamino]phenylpropiolic acid in the manner of Example 2182.

TABLE XLVIII

The following carboxylic acids are prepared by alkylation of the corresponding 4-aminophenyl carboxylate ester with the appropriate polyfluoroalkyl halide, trifluoromethanesulfonate, or methanesulfonate followed by hydrolysis using the methods of Examples 2182-2185 as shown in the table.

| Example No. | Method of Example | Compound |
|---|---|---|
| 2186 | 2182 | 4-[7-(Trifluoromethyl)heptylamino]phenylacetic acid |
| 2187 | 2182 | 4-[16-(Trifluoromethyl)hexadecylamino]phenylacetic acid |
| 2188 | 2182 | 4-[1-Hexyl-11-(trifluoromethyl)octylamino]phenylacetic acid |

| Example No. | Method of Example | Compound |
|---|---|---|
| 2189 | 2182 | 4-(3,3-Difluorotetradecyl-amino)phenylacetic acid |
| 2190 | 2183 | 4-[(Hentricontafluoropenta-decyl)methylamino hydrocin-namic acid |
| 2191 | 2183 | 4-[15-[Trifluoromethyl)-4-pentadecynylamino]hydro-cinnamic acid |
| 2192 | 2183 | 4-[11-(Trifluoromethyl)un-decylamino]hydrocinnamic acid |
| 2193 | 2183 | 4-[1-2,2,2,-Trifluoroethyl)-tridecylamino]hydrocinnamic acid |
| 2194 | 2184 | 4-[11-(Heptafluoropropyl)-undecylamino]cinnamic acid |
| 2195 | 2184 | 4-[12-Fluoro-12-(heptafluoro-propyl)-12-(trifluormethyl)-dodecylamino]cinnamic acid |
| 2196 | 2184 | 4-[15-(Trifluoromethyl)-9-pentadecenylamino]cinnamic acid |
| 2197 | 2184 | 4-[1-Hexyl-9-(trifluorometh-yl)-4-nonynylamino]cinnamic acid |
| 2198 | 2185 | 4-[14-(Trifluoromethyl)te-tradecylamino]phenylpropio-lic acid |
| 2199 | 2185 | 4-[3,7-Dimethyl-7-(trifluoro-methyl)octylamino]phenylpro-piolic acid |
| 2200 | 2185 | 4-(11,11-Difluorododecyla-mino)phenylpropiolic acid |
| 2201 | 2185 | 4-[3-(Trifluoromethyl)-2,4,4-trimethylcyclohexylamino]-phenylpropiolic acid |

Example 2202

Preparation of p-[(cylohexylmethyl)amino]phenylacetic acid

A solution of 6 g. of cyclohexylmethyl bromide and 11.19 g. of ethyl p-aminophenyl acetate in 30 ml. of hexamethylphosphoramide is heated in an oil bath for 20 hours. The solution is poured into ice-cold water and extracted several times with diethyl ether. The combined ether extracts are washed with water, dried with anhydrous magnesium sulfate, and evaporated to dryness under reduced pressure to furnish ethyl p-cyclohexylmethylaminophenyl acetate as an oil.

The oil is dissolved in 250 ml. of ethanol:water (9:1) containing 9 g. of potassium hydroxide and the resulting solution is stirred at the reflux temperature for 3 hours. After chilling, the mixture is acidified with concentrated hydrochloric acid, diluted with water, and extraceted twice with methylene chloride. The combined extracts are washed with water, dried with anhydrous magnesium sulfate, and evaporated to dryness under reduced pressure to furnish p-[(cyclohexylmethyl)amino]phenylacetic acid.

Examples 2203-2308

Treatment of the indicated halide starting materials set forth in Table XLIX below with ethyl p-aminophenyl acetate followed by saponification according to Example 2202 is productive of the corresponding p-[substituted amino]phenylacetic acids listed in Table XLIX.

TABLE XLIX

| Example | Starting material | Product |
|---|---|---|
| 2203 | 1-iodomethyl-2-methyl cyclopentane Chem. Abst. 67,90421y | p-[(2-methycyclopen-tyl)methylamino]phenyl-acetic acid |
| 2204 | α-bromomethyl cyclopen-tane Chem. Abst. 66, 18472c | p-[(cyclopentyl)methyl-amino]phenylacetic acid |

TABLE XLIX

| Example | Starting material | Product |
|---|---|---|
| 2205 | 1-bromomethyl-4-methylcyclohexane Chem. Abst. 70, 2934b | p-[(4-methylcyclohexyl)methylamino]-phenylacetic acid |
| 2206 | 1-chloromethyl-2-methylcyclohexane Chem. Abst. 68,88671h | p-[(2-methylcyclohexyl)methylamino]-phenylacetic acid |
| 2207 | 1-(1,2-dimethylcyclohexyl)-2-chloropropane Chem. Abst. 73,14272j | p-[1-(1,2-dimethylcyclohexyl)-2-propylamino]phenylacetic acid |
| 2208 | 1-(1,3-dimethylcyclohexyl)-2-chloropropane Chem. Abst. 73,14272j | p-[1-(1,3-dimethylcyclohexyl)-2-propylamino]phenylacetic acid |
| 2209 | 1-(1,4-dimethylcyclohexyl)-2-chloropropane Chem. Abst. 73,14272j | p-[1-(1,4-dimethylcyclohexyl)-2-propylamino]phenylacetic acid |
| 2210 | α-bromomethylcycloheptane Chem. Abst. 51,1049e | p-(cycloheptylmethylamino)phenylacetic acid |
| 2211 | α-bromomethylcyclooctane Chem. Abst. 68,104595t | p-(cyclooctylmethylamino)phenylacetic acid |
| 2212 | α-chloroethylcyclopentane Chem. Abst. 72,110862b | p-(1-cyclopentylethylamino)phenylacetic acid |
| 2213 | 1-bromo-2-cyclopenytlbutane | p-(2-cyclopentylbutylamino)phenylacetic acid |
| 2214 | 1-bromo-2-cyclopentylhexane Ref. A | p-(2-cyclopentylhexylamino)phenylacetic acid |
| 2215 | 2-chloroethylcyclohexane Chem. Abst. 68,86671h | p-(2-cyclohexylethylamino)phenylacetic acid |
| 2216 | 1-(2-bromoethyl)-1-ethylcyclohexane Chem. Abst. 70,57233c | p-[2-(1-ethylcyclohexyl)ethylamino] phenylacetic acid |
| 2217 | 1-bromo-2-(3-methylcyclohexyl)butane Ref. A | p-[2-(3-methylcyclohexyl)butylamino] phenylacetic acid |
| 2218 | 1-bromo-2-cyclohexylpentane Ref. A | p-[(2-cyclohexyl)pentylamino]phenylacetic |

TABLE XLIX (cont'd)

| Example | Starting material | Product |
| --- | --- | --- |
| 2219 | 1-bromo-2-cyclohexyl-butane Ref. A | p-[(2-cyclohexyl)butyl-amino]phenylacetic acid |
| 2220 | 1-bromo-2-cyclohexyl-propane Ref. A | p-[(2-cyclohexyl)prop-ylamino]phenylacetic acid |
| 2221 | 1-(2-chloroethyl)-2,3-dimethylcyclohexane Chem. Abst. 69,56053n | p-[2-(2,3-dimethylcy-clohexyl)ethylamino]-phenylacetic acid |
| 2222 | 1-(2-chloroethyl)-3,5-dimethylcyclohexane Chem. Abst. 69,56053n | p-[2-(3,5-dimethylcy-clohexylyl)ethylamino]-phenylacetic acid |
| 2223 | 2-(2-chloroethyl)-1,4-dimethylcyclohexane Chem. Abst. 69,56053n | p-[2-(2,5-dimethylcy-clohexyl)ethylamino]-phenylacetic acid |
| 2224 | 1-(2-chloroethyl)-2-ethylcyclohexane Chem. Abst. 69,56053n | p-[2-(2-ethylcyclo-hexyl)ethylamino]-phenylacetic acid |
| 2225 | 1-(2-chloropropyl)-3-methylcyclohexane Chem. Abst. 67,53405a | p-[1-(3-methylcyclo-hexyl)-2-propylamino]-phenylacetic acid |
| 2226 | 1-(2-bromoethyl)-1-methylcyclohexane Chem. Abst. 72,132133s | p-[2-(1-methylcyclo-hexyl)ethylamino]-phenylacetic acid |
| 2227 | 1-(2-chloroethyl)-2-methylcyclohexane Chem. Abst. 69,56053n | p-[2-(2-methylcyclo-hexyl)ethylamino]-phenylacetic acid |
| 2228 | 1-(2-chloroethyl)-3-methylcyclohexane Chem. Abst. 69,56053n | p-[2-(3-methylcyclo-hexyl)ethylamino]-phenylacetic acid |
| 2229 | 1-(2-chloroethyl)-4-methylcyclohexane Chem. Abst. 69,56053n | p-[2-(4-methylcyclo-hexyl)ethylamino]-phenylacetic acid |
| 2230 | 2-bromomethylcyclo-heptane Ref. A | p-(cycloheptylmethyl-amino)phenylacetic acid |
| 2231 | 3-bromopropylcyclo-butane Ref. A | p-(3-cyclobutyl)propyl-amino)phenylacetic acid |

TABLE XLIX (cont'd)

| Example | Starting material | Product |
|---|---|---|
| 2232 | 3-bromopropylcyclopentane Chem. Abst. 75,15138f | p-(3-cyclopentyl)propylaminophenylacetic acid |
| 2233 | 3-bromopropylcyclohexane Ref. A | p-(3-cyclohexyl)propylamino)phenylacetic acid |
| 2234 | 1-(3-chloropropyl)-3-ethylcyclohexane Chem. Abst. 68,12589w | p-[3-(3-ethylcyclohexyl)propylamino]-phenylacetic acid |
| 2235 | 1-(3-bromopropyl)-3-methylcyclohexane Chem. Abst. 75,151387f | p-[3-(3-methylcyclohexyl)propylamino]-phenylacetic acid |
| 2236 | 1-(3-bromopropyl)-4-methylcyclohexane Chem. Abst. 75,151387f | p-[3-(4-methylcyclohexyl)propylamino]-phenylacetic acid |
| 2237 | 1-bromo-3-cyclohexylpentane Ref. A | p-[(3-cyclohexyl)pentylamino]phenylacetic acid |
| 2238 | (2-bromomethyl)butylcyclohexane Ref. A | p-[(3-cyclohexyl-2-ethyl)propylamino] phenylacetic acid |
| 2239 | 1-[1-bromo-2-methyl-3-(3-etylcyclohexyl]-propane Chem. Abst. 68,12529w | p-[3-(3-ethylcyclohexyl)-2-methyl]propylaminophenylacetic acid |
| 2240 | 4-bromobutylcyclopentane Chem. Abst. 69,18646z | p-(4-cyclopentyl)-butylaminophenylacetic acid |
| 2241 | 4-chlorobutylcyclohexane Ref. A | p-(4-cyclohexyl)butylaminophenylacetic acid |
| 2242 | 5-bromo-2-cyclohexylpentane Ref. A | p-(4-cyclohexyl)pentylaminophenylacetic acid |
| 2243 | 1-bromo-4-cyclohexylhexane Ref. A Chem. Abst. 70,P87143r | p-(4-cyclohexyl)hexylaminophenylacetic acid |
| 2244 | 1-bromo-4-cyclohexyl-2-ethylbutane Ref. A | p-(4-cyclohexyl-2-ethyl)butylaminophenyl acetic acid |

TABLE XLIX (cont'd)

| Example | Starting material | Product |
|---|---|---|
| 2245 | 1-bromo-4-(3-methyl-cyclohexyl)butane Ref. A | p-[4-(3-methylcyclo-hexyl)butylamino] phenylacetic acid |
| 2246 | 1-chloro-4-(4-methyl-cyclohexyl)butane Ref. A | p-[4-(4-methylcyclo-hexyl)butylamino] phenylacetic acid |
| 2247 | 1-chloro-4-(4-ethyl-cyclohexyl)butane Ref. A | p-[4-(4-ethylcyclo-hexyl)butylamino] phenylacetic acid |
| 2248 | 1-(4-chlorobutyl)-2,3-dimethylcyclo-hexane Chem. Abst. 70,P87143r; Ref. A | p-[4-(2,3-dimethylcy-clohexyl)butylamino]-phenylacetic acid |
| 2249 | 1-(4-chlorobutyl)-2,5-dimethylcyclo-hexane Ref. A | p-[4-(2,5-dimethyl-cyclohexyl)butyl-amino]phenylacetic acid |
| 2250 | 1-(4-chlorobutyl)-4-methoxycyclo-hexane Ref. A | p-[4-(4-methoxylcyclo-hexyl)butylamino]-phenylacetic acid |
| 2251 | 1-(4-bromobutyl)-2-methoxycyclo-hexane Ref. A | p-[4-(2-methoxylcyclo-hexyl)butylamino]-phenylacetic acid |
| 2252 | 4-bromobutyl)-cycloheptane Ref. A | p-(4-cycloheptyl)butyl-aminophenylacetic acid |
| 2253 | 1-(4-chlorobutyl)-4-cyclohexylcyclo-hexane Ref. A | p-[4-(4-cyclohexyl)-cyclohexyl]butylamino phenylacetic acid |
| 2254 | 2-(4-chlorobutyl)-decahydronapthylene Ref. A | p-[4-(2-decahydronaph-thyl]butylamino phenylacetic acid |
| 2255 | 4-bromobutylcyclo-heptane Chem. Abst. 70,P87143r | p-(4-cycloheptyl)-butylamino phenylacetic acid |
| 2256 | 4-chloropentylcyclopro-pane Chem. Abst. 69,105732t, 74,31488x | p-[5-(cyclopropyl)-2-pentylamino]phenyl-acetic acid |

TABLE XLIX (cont'd)

| Example | Starting material | Product |
|---|---|---|
| 2257 | 1-bromo-5-cyclobutyl-pentane Chem. Abst. 70,P87143r; Ref. A | p-[5-(cyclobutyl)pentylamino]phenylacetic acid |
| 2258 | 1-chloro-5-cyclopentyl-pentane Chem. Abst. 70,P87143r; Ref. A | p-[5-(cyclopentyl)-pentylamino]phenyl-acetic acid |
| 2259 | 5-bromopentylcyclohexane Chem. Abst. 55,21016e | p-[5-(cyclohexyl)pentyl amino]phenylacetic acid |
| 2260 | 5-chloropentylcycloheptane Chem. Abst. 70,P87143r Ref. A | p-[5-(cyclopentyl)pentylamino]phenylacetic acid |
| 2261 | 6-chlorohexylcyclopentane Ref. A | p-[6-(cyclopentyl)hexyl amino]phenylacetic acid |
| 2262 | 6-chlorohexylcycloheptane Chem. Abst. 70,P87143r | p-[6-(cycloheptyl)hexyl amino]phenylacetic acid |
| 2263 | 1-chloro-7-cyclopentyl-heptane Chem. Abst. 75,P141605n | p-[7-(cyclopentyl)-heptylamino]phenyl-acetic acid |
| 2264 | 8-chlorooctylcyclopentane Ref. A; Chem. Abst. 70,87143r | p-[8-(cyclopentyl)-octylaminophenyl-acetic acid |
| 2265 | 8-bromooctylcyclohexane | p-[8-(cyclohexyl)octyl-aminophenylacetic acid |
| 2266 | 1-bromo-8-(3,3,5-trimethyl-cyclohexyl)octane Chem. Abst. 75,P20026q | p-[8-(3,3,5-trimethyl-cyclohexyl)octylamino]-phenylacetic acid |
| 2267 | 9-bromononylcyclopentane Ref. A; Chem. Abst. 70,P87143r | p-[9-(cyclopentyl)-nonylamino]phenyl-acetic acid |
| 2268 | 13-bromotridecylcyclopentane | p-[13-(cyclopentyl)tri-decylamino]phenylacetic acid |
| 2269 | 1-(2-chlorocyclopropyl)-pentane Chem. Abst. 75,49270a | p-[2-pentyl)cyclo-propylamino]phenyl-acetic acid |
| 2270 | 1-bromocyclopropylpentane Chem. Abst. 75,76195n | p-[1-pentyl)cyclo-propylamino]phenyl- |

TABLE XLIX (cont'd)

| Example | Starting material | Product |
|---|---|---|
| 2271 | 1-(2-bromocyclopropyl)butane Chem. Abst. 74,124924b | p-[2-butylcyclopropyl)-amino]phenylacetic acid |
| 2272 | bromocyclopentane Ref. A | p-cyclopentylamino-phenylacetic acid |
| 2273 | 1-chloro-1-propylcyclo-pentane Chem. Abst. 52, | p-(1-propylcyclopentyl-amino)phenylacetic acid |
| 2274 | 4-bromo-1,1-dimethylcyclo-hexane Chem. Abst. 71, 11242c | p-(4,4-dimethylcyclo-hexylamino)phenyl-acetic acid |
| 2275 | 1-chloro-4-propylcyclo-hexane Chem. Abst. 68,86671h; 75,128994t | p-(4-propylcyclohexyl-amino)phenylacetic acid |
| 2276 | 2-chloro-1-methylethyl-cyclohexane Chem. Abst. 70,P87143r; 75,128994t | p-[2(1-methylethyl)-cyclohexylamino]phenyl-acetic acid |
| 2277 | 4-(t-butyl)-1-chloro-1-methylcyclohexane Chem. Abst. 68, 113898w | p-[4-(t-butyl)-1-methylcyclohexylamino]-phenylacetic acid |
| 2278 | bromocycloheptane Chem. Abst. 51,9505e; 67.9986s | p-cycloheptylamino-phenylacetic acid |
| 2279 | bromocyclooctane Chem. Abst. 51,1049e; | p-cycloheptylamino-phenylacetic acid |
| 2280 | bromocyclononane Chem. Abst. 54,4153f; 69,2306v | p-cyclononylamino-phenylacetic acid |
| 2281 | bromocyclodecane Chem. Abst. 67,58849h; 69,2306c | p-cyclodecylamino-phenylacetic acid |
| 2282 | bromocycloundecane | p-cycloundecylamino- |
| 2283 | bromocyclotridecane Chem. Abst. 69,2306c | p-cyclotridecylamino-phenylacetic acid |
| 2284 | bromocyclotetradecane Chem. Abst. 54,4153f; 54,16141i | p-cyclotetradecylamino-phenylacetic acid |
| 2285 | bromocyclopentadecane Chem. Abst. 72,P100160g 69,2306c | p-cyclopentadecylamino-phenylacetic acid |

TABLE XLIX (cont'd)

| Example | Starting material | Product |
|---|---|---|
| 2286 | bromocyclohexadecane Chem. Abst. 69,2306c | p-cyclohexadecylamino-phenylacetic acid |
| 2287 | 3-bromobicyclopentyl Chem. Abst. 31, 7405[3]; 35, 2864 | p-(3-cyclopentyl)cyclo-pentylamino)phenyl-acetic acid |
| 2288 | (3-bromocyclopentyl) cyclohexane Chem. Abst. 31,7405[4] | p-(3-cyclohexylcyclo-pentylamino)phenyl-acetic acid |
| 2289 | 3-bromo-3'-ethylbicyclo-pentyl Chem. Abst. 36, 48089 | p-[3-(3-ethylcyclo-pentyl)cyclopentyl-amino]phenylacetic acid |
| 2290 | 2-bromo-1-cyclopentyl-cyclopentane Chem. Abst. 51, 5712f | p-[2-(cyclopentyl)-cyclopentylamino]-phenylacetic acid |
| 2291 | 1-chlorobicyclohexyl Chem. Abst. 30, 3807[1] | p-[1-(cyclohexyl)-cyclohexylamino]-phenylacetic acid |
| 2292 | 1-chlorobicyclopentyl Chem. Abst. 45, 6163b | p-[1-(cyclopentyl)-cyclopentylamino]-phenylacetic acid |
| 2293 | 2-iodomethyldecahydro-napthalene Chem. Abst. 41, 116b | p-[(2-decahydro-naphthyl)methylamino]-phenylacetic acid |
| 2294 | 2-(2-iodoethyl)deca-hydronaphthalene Chem. Abst. 41, 116d | p-[2-(2-decahydro-naphthyl)ethylamino]-phenylacetic acid |
| 2295 | 1-(4-bromobutyl)deca-hydronaphthalene Chem. Abst. 45, p175d | p-[4-(1-decahydro-naphthyl)butylamino]-phenylacetic acid |
| 2296 | 1-bromo-1,1-dicyclo-pentylethane, Chem. Abst. 31, 5759[2] | p-[(1,1-dicyclopentyl)-ethylamino]phenyl-acetic acid |
| 2297 | 1-bromo-4 -methyldeca-hydronaphthalene Chem. Abst. 53, 3265f | p-[1-(4 -methyldeca-hydronaphthyl)amino] phenylacetic acid |
| 2298 | 2-(bromomethyl)-1,3,3-trimethylcyclohexane Chem. Abst. 28, 2343[8] | p-[(1,3,3-trimethyl-cyclohexyl)methyl amino]phenylacetic |

TABLE XLIX (cont'd)

| Example | Starting material | Product |
|---|---|---|
| 2299 | 6-(3-bromobutyl)-1,5,5-trimethylcyclohexene Chem. Abst. 66, 2658g | p-[4-(2,6,6-trimethyl-2-cyclohexenyl)-2-butylamino]phenylacetic acid |
| 2300 | 4-(3-chloropropyl)-cyclohexane Ref. A | p-[3-(3-cyclohexenyl)-propylamino]phenylacetic acid |
| 2301 | 3-(4-chlorobutyl)-cyclopentene Ref. A | p-[4-(3-cyclopentenyl)-butylamino]phenylacetic acid |
| 2302 | 1-(4-bromobutyl)cyclohexene Chem. Abst. 69, 76727n | p-[4-(1-cyclohexenyl)-butylamino]phenylacetic acid |
| 2303 | 1-(5-bromopentyl)-cyclopentene Chem. Abst. 55, 27129g | p-[5-(1-cyclopentenyl)-pentylamino]phenyl-acetic acid |
| 2304 | 3-(11-chloroundecyl)-cyclopentene Chem. Abst. 37, 3060f | p-[11-(3-cyclopentenyl)-undecylamino]phenylacetic acid |
| 2305 | 1-(13-chlorotridecyl-cyclopentene Chem. Abst. 37, 5031b | p-[13-(1-cyclopentenyl) tridecylamino]phenyl-acetic acid |
| 2306 | 3-(13-chlorotridecyl)-cyclopentene Chem. Abst. 51, 7652a | p-[13-(3-cyclopentenyl) tridecylamino]phenylacetic acid |
| 2307 | 2-(4-chlorobutyl)decahydronapthalene Chem. Abst. 70, P87143r | p-[4-(2-decahydro-naphthyl)butylamino] phenylacetic acid |
| 2308 | 4-bromo-1-(cyclohexyl)-cyclohexane Chem. Abst. 69, 103618m | p-[4-cyclohexyl)cyclohexylbutylamino]phenyl acetic acid |

Ref. A=R.D. Westland, et al., J. Med Chem., 11,1190 (1968)

## EXAMPLE 2309

### Preparation of p-[2-(cyclopentyl)ethylamino]phenylacetic acid

To a solution of cyclopentylethan-2-ol(15.0 g.) and triethylamine (14 ml.) in dry methylene chloride (320 ml.) at -8°C. is added methanesulfonylchloride (5.73 ml.), dropwise. The reaction mixture is stirred at -10°C. for 30

minutes and then diluted with methylene chloride, extracted with ice-water (250 ml.), followed by cold 10% hydrochloric acid (200 ml.); cold saturated sodium bicarbonate (200 ml.) and cold brine (200 ml.). The organic phase is dried over magnesium sulfate and the solvent removed *in vacuo* to provide crude mesylate.

A solution of 18.1 g. of the above mesylate and 19.8 g. of ethyl *p*-aminophenylacetate in hexamethylphosphoramide is heated at 120°C. for 20 hours. After cooling, the reaction mixture is diluted with 30 ml. of ethanol:water (1:1) (30 ml.) and chilled. More ethanol is added and the solid material is collected. This solid is recrystallized twice from ethanol to provide the ester.

A mixture of the ester, 22.0 g. of potassium hydroxide and 200 ml. of ethanol-water (8:1) is stirred under reflux for 6 hours. Concentrated hydrochloric acid (about 80 ml.) is added to the warm mixture and cooling and dilution with water affords a white solid which is collected by filtration and recrystallized from ethanol to yield the product as a white solid.

## EXAMPLES 2310-2385

Treatment of the alcohols of Table L below with methanesulfonylchloride to provide the corresponding mesylate followed by treatment with ethyl *p*-aminophenylacetate followed by saponification and acidification of the resulting substituted *p*-aminophenylacetate by the procedure of Example 2309 produces the indicated *p*-(substituted amino)phenylacetic acids shown in Table L.

### TABLE L

| Example | Starting material | Product |
|---|---|---|
| 2310 | 2-isopropyl-5-methylenecyclopentanol Chem. Abst. 66, 38074c | *p*-(2-isopropyl-5-methylene cyclopentylamino)-phenylacetic acid |
| 2311 | 2-cyclohexen-1-ol Aldrich Chem. Co. | *p*-(cyclohex-2-enylamino)-phenylacetic acid |

TABLE L(cont'd)

| Example | Starting material | Product |
|---|---|---|
| 2312 | 4-isopropyl-2-cyclohexen-1-ol Chem. Abst. 69, 99290d | p-(4-isopropylcyclohex-2-enylamino)phenylacetic acid |
| 2313 | 2-isopropyl-3-cyclohexen-1-ol Chem. Abst. 75, 55380m | p-(2-isopropylcyclox-hex-3-enylamino)phenyl acetic acid |
| 2314 | 2-methyl-2-cycloocten-1-olopentane Chem. Abst. 69, 27127h | p-(2-(cyclopentyl)cyclopentylamino)phenylacetic acid |
| 2315 | 2-cyclononen-1-ol Chem. Abst. 72, 30882t | p-(cyclonon-2-enylamino)-phenylacetic acid |
| 2316 | 3-cyclononen-1-ol Chem. Abst. 75, 13957j | p-(cyclonon-3-enylamino)-phenylacetic acid |
| 2317 | 2-methylenecyclodecanol Chem. Abst. 74, 75857w | p-(2-methylenecyclodecyl-amino)phenylacetic acid |
| 2318 | E-3-cyclodecen-1-ol Chem. Abst. 73, 87173n | p-(E-cyclodec-3-enyl-amino)phenylacetic acid |
| 2319 | Z-3-cyclodecen-1-ol Chem. Abst. 73, 87173n | p-(Z-cyclodec-3-enyl amino)phenylacetic acid |
| 2320 | 5-cyclodecen-1-ol Chem. Abst. 71, 60514w | p-(cyclodec-5-enyl-amino)phenylacetic acid |
| 2321 | 4-ethyl-2-cyclododecen-1-ol Chem. Abst. 70, 114922c | p-(4-ethylcyclododec-2-enylamino)phenylacetic acid |
| 2322 | 2-cyclotridecen-1-ol Chem. Abst. 70, 114922c | p-(cyclotridec-2-enyl-amino)phenylacetic acid |
| 2323 | 8-cycloheptadecen-1-ol Chem. Abst. 66, 2658g | p-(cycloheptadec-8-enyl-amino)phenylacetic acid |
| 2324 | 9-cycloheptadecen-1-ol Chem. Abst. 68, 49157z | p-(cycloheptadec-9-enyl-amino)phenylacetic acid |
| 2325 | 2-cyclobutene-1-methanol Chem. Abst. 67,32343p | p-[(cyclobut-2-enyl)meth-ylamino]phenylacetic acid |
| 2326 | 1-cyclobutene-1-methanol Chem. Abst. 71, 12650 r | p-[(cyclobut-1-enyl)-ethylamino]phenylacetic acid |

TABLE L(cont'd)

| Example | Starting material | Product |
|---|---|---|
| 2327 | 2-cyclopentene-1-methanol Chem. Abst. 71, 12650r | p-[(cyclopent-3-enyl)-methylamino]phenylacetic acid |
| 2328 | 3-cyclopentene-1-methanol Chem. Abst. 73, 65783j | p-(4-isopropylcyclohex-2-enylamino]phenylacetic acid |
| 2329 | 1-cyclopentene-1-propanol Chem. Abst. 73, 66113c | p-[1-(cyclopent-1-enyl)-propylamino]phenylacetic acid |
| 2330 | 1-cyclohexene-1-methanol Chem. Abst. 70, 10773p | p-[(cyclohex-1-enyl)-methylamino]phenylacetic acid |
| 2331 | 2-cyclohexene-1-ethanol, 30882t Chem. Abst. 69, 26424r | p-[(1-cyclohex-2-enyl)-ethylamino]phenylacetic acid |
| 2332 | 1-(3-cyclohexenyl)-1-propanol Chem. Abst. 67, 72634r | p-[(1-cyclohex-3-enyl)-propylamino]phenylacetic acid |
| 2333 | cis-5-ethyl-3-cyclohexene-1-methanol Chem. Abst. 69, 27575c | p-(cis-5-ethylcyclohex-3-enyl)methylaminophenyl-acetic acid |
| 2334 | trans-5-ethyl-3-cyclohexene-1-methanol Chem. Abst. 69, 27575c | p-(trans-5-ethylcyclohex-3-enyl)methylaminophenyl-acetic acid |
| 2335 | 1-cycloheptene-1-methanol Chem. Abst. 73, 14266k | p-(cyclohept-1-enylmethylamino)phenylacetic acid |
| 2336 | 1-cycloheptene-1-ethanol Chem. Abst. 70, 37270j | p-[1-(cyclohept-1-enyl)-ethylamino)phenylacetic acid |
| 2337 | 2-cyclooctene-1-methanol Chem. Abst. 69, 36263b | p-(cyclooct-2-enylmethyl-amino)phenylacetic acid |
| 2338 | 4-cyclooctene-1-methanol Chem. Abst. 66, 37492a | p-(cyclooct-4-enylmethyl-amino)phenylacetic acid |
| 2339 | 1-cyclooctenylethanol Chem. Abst. 70, 37270j | p-(cyclooct-1-enylethyl-amino)phenylacetic acid |

TABLE L(cont'd)

| Example | Starting material | Product |
|---|---|---|
| 2340 | 2-(3-cyclopentenyl) butanol Chem. Abst. 72, 133044a | p-[2-ethyl-2-cyclopent-3-enylethylamino]phenylacetic acid |
| 2341 | 2,3-dimethyl-2-(2-cyclopentenyl)propanol Chem. Abst. 70, 11818u | p-[2-(2,3-dimethylcyclopent-2-enyl)propylamino]-phenylacetic acid |
| 2342 | 4,6-dimethyl-3-cyclohexen-1-ethanol Chem. Abst. 111623c | p-[2-(4,6-dimethylcyclohex-3-enyl)ethylamino]-phenylacetic acid |
| 2343 | α-methyl-1-cyclohexene-1-ethanol Chem. Abst. 74, 42909v | p-[1-methyl-2-(cyclohex-1-enyl)ethylamino]phenylacetic acid |
| 2344 | 4-methyl-3-cyclohexene-1-ethanol Chem. Abst. 75, 19601s | p-[2-(4-methylcyclohex-3-enyl)ethylamino]phenyl acetic acid |
| 2345 | 1-cyclooctene-1-ethanol Chem. Abst. 70, 37270j | p-(2-cyclooct-1-enylethylamino)phenylacetic acid |
| 2346 | 1-cyclononene-1-ethanol | p-(2-cyclonon-1-enylethylamino)phenylacetic acid |
| 2347 | α,4-dimethyl-3-cyclohexene-1-propanol Chem. Abst. 68, 78427t | p-[1-methyl-3-(4-methylcyclohex-3-enyl)propylamino]phenylacetic acid |
| 2348 | 1-cyclohexene-1-propanol Chem. Abst. 70, 87111d | p-(3-cyclohex-1-enylpropylamino)phenylacetic acid |
| 2349 | 3-cyclohexene-1-propanol Chem. Abst. 69, 43158z | p-(3-cyclohex-3-enylpropylamino)phenylacetic acid |
| 2350 | 3-cyclohexene-1-butanol Chem. Abst. 69, 49158z | p-(4-cyclohex-3-enylbutylamino)phenylacetic acid |
| 2351 | α,α-dimethyl-2-cyclopentene-1-undecanol Chem. Abst. 72, 110860z | p-[(1,1-dimethyl-11-cyclopent-2-enyl)undecylamino]phenylacetic acid |
| 2352 | 4-isopropylidene-2,2-dimethylcyclo- | p-(2,2-dimethyl-4-isopropylidenecylobutyl- |

TABLE L(cont'd)

| Example | Starting material | Product |
|---|---|---|
| | butanol Chem. Abst. 73, 24996n | amino)phenylacetic acid |
| 2353 | 2-cyclopenten-1-ol Chem. Abst. 68, 39177s | p-(cyclopent-2-enylamino)-phenylacetic acid |
| 2354 | 3-cyclopenten-1-ol Chem. Abst. 66, 11504r | p-(cyclopent-3-enylamino)-phenylacetic acid |
| 2355 | 3-cyclohexen-1-ol Chem. Abst. 69, 26837c | p-(cyclohex-3-enylamino)-phenylacetic acid |
| 2356 | 2,2-dimethyl-6-methylenecyclohexanol | p-(2.2-dimethyl-6-enylcyclohexylamino)phenylacetic acid |
| 2357 | 2-methylenecycloheptanol Chem. Abst. 69,27127h | p-(2-methylenecycloheptylamino)phenylacetic acid |
| 2358 | 2-methyl-2-cyclohepten-1-ol Chem. Abst. 69,27127h | p-(2-methylcyclohept-2-enylamino)phenylacetic acid |
| 2359 | 2-methyl-6-methylenecycloheptanol Chem. Abst. 67,11600e | p-(2-methyl-6-methylenylcycloheptylamino)-phenylacetic acid |
| 2360 | 3,7-dimethyl-3-cyclohepten-1-ol Chem. Abst. 67,11600e | p-(3,7-dimethylcyclohept-3-enylamino)phenylacetic acid |
| 2361 | 4-cycloocten-1-ol Chem. Abst. 70,28287t | p-(cyclooct-4-enylamino) phenylacetic acid |
| 2362 | 3-cycloocten-1-ol Chem. Abst. 66,104593z | p-(cyclooct-3-enylamino)-phenylacetic acid |
| 2363 | 2-cycloocten-1-ol Chem. Abst. 68,39177s | p-(cyclooct-2-enylamino)-phenylacetic acid |
| 2364 | 4-methylenecyclooctanol Chem. Abst. 70,28445t | p-(4-methylenecyclooctylamino)phenylacetic acid |
| 2365 | α-methyl-5-methylenecycloooctanemethanol Chem. Abst. 68,104595t | p-[1-(5-methylenecyclooctylethylamino]phenylacetic acid |
| 2366 | 5-methylenecyclooctanemethanol | p-[5-methylenecyclooctylmethylamino]phenylacetic |

TABLE L(cont'd)

| Example | Starting material | Product |
|---|---|---|
| | Chem. Abst. 66,37492a | acid |
| 2367 | 1,3-dimethyl-2-methyl-enecyclopentane-methanol Chem. Abst. 73,24996n | p-[(1,3-dimethyl-2-methyl-enecyclopentyl)methyl-aminophenylacetic acid |
| 2368 | E-4-cyclopropyl-3-buten-2-ol | p-[E-2-(4-cyclopropyl) but-3-enylamino]phenyl- |
| 2369 | Z-4-cyclopropyl-3-buten-2-ol anol Chem. Abst. 70,3413t | p-[Z-2-(4-cyclopropyl)-but-3-enylamino]phenyl-acetic acid |
| 2370 | α-methylenecyclo-hexaneethanol Chem. Abst. 66,45950p | p-[(1-methylene-2-cyclohexyl)ethyl-amino]phenylacetic acid |
| 2371 | β-methylenecyclo-hexaneethanol Chem. Abst. 75,139951c | p-[(2-methylene-2-cyclo-hexyl)ethylamino] phenylacetic acid |
| 2372 | E-2-(3,3-dimethylcyclo-hexylidenyl)ethanol Chem. Abst. 75,110431x | p-[E-2-(3,3-dimethylcyclo-hexylidenyl)ethyl]amino phenylacetic acid |
| 2373 | Z-2-(3,3-dimethylcyclo-hexylidenyl)ethanol Chem. Abst. 75,110431x | p-[Z-2-(3,3-dimethylcyclo-hexylidenylethylamino]-phenylacetic acid |
| 2374 | E-4-cyclopentyl-2-buten-1-ol Chem. Abst. 75,48349w | p-(4-cyclopentylbut-2-enylamino]phenylacetic acid |
| 2375 | E-4-cyclohexyl-2-buten-1-ol Chem. Abst. 75,48349w | p-[E-4-cyclohexylbut-2-en-ylamino]phenylacetic acid |
| 2376 | 2-vinylcyclopentane-ethanol Chem. Abst. 66,104477q | p-[2-(2-vinylcyclopentyl)-ethylamino]phenylacetic acid |
| 2377 | 3-isopropyl-1-methyl-cyclopentanemethanol Chem. Abst. 66,38061w | p-[(3-isopropyl-2-methyl-cyclopentyl)methylamino]-phenylacetic acid |
| 2378 | 1-allyl-2-methylcyclo-hexanol Chem. Abst. 71,29919h | p-(1-allyl-2-methylcyclo-hexylamino)phenylacetic acid |

TABLE L(cont'd)

| Example | Starting material | Product |
|---|---|---|
| 2379 | 2-isopropenylcyclo-hexanol Chem. Abst. 72,12663t | p-(2-isopropenylcyclo-hexylamino)phenylacetic acid |
| 2380 | 1-(isopropenylcyclo-hexanol Chem. Abst. 75,139951c | p-(1-isopropenylcyclo-hexylamino)phenylacetic acid |
| 2381 | 2-allylcyclohexanol Chem. Abst. 70,96517t | p-(2-allylcyclohexyl-amino)phenylacetic acid |
| 2382 | 3-allylcyclohexanol Chem. Abst. 69, 86453j | p-(3-allylcyclohexyl-amino)phenylacetic acid |
| 2383 | 1-allylcyclohexanol Chem. Abst. 66,374866 | p-(1-allylcyclohexyl-amino)phenylacetic acid |
| 2384 | 1-(3-butenyl)-2-methyl cycloheptanol Chem. Abst. 69, 106892g | p-[1-(3-butenyl)-2-methyl-cycloheptylamino] phenylacetic acid |
| 2385 | 1-allylcyclododecanol Chem. Abst. 68, 95381r | p-(1-allylcyclododecyl-amino)phenylacetic acid |
| 2386 | 2-butyl-2-cyclopenten-1-ol Chem. Abst. 71, 38404p | p-(2-butycyclopent-2-en-ylamino)phenylacetic acid |

## EXAMPLE 2387

Preparation of Esters

Treatment of the acids of Examples 1-186 with trifluoroacetic anhydride to provide the N-$COCF_3$ derivatives, followed by treatment with thionyl chloride to provide the N-$COCF_3$ acid chloride, followed by treatment with one of the following alcohols, followed by removal of the N-$COCF_3$ group with sodium hydroxide, provides the corresponding esters of the starting acid.

Alcohols: methanol, ethanol, 2-methoxyethanol, butanol, pentanol, hexanol, cyclopentanol, cyclohexanol, 1,2-propanediol, 1,3-propanediol, ethylene glycol, glycerol, glycidol, glycolic acid, citric acid, tartaric acid, malid acid, methyl glycolate, 2-hydroxypropionic acid, 3-hydroxybutyhric acid 4-hydroxybutyric acid, glyceric acid,

3-diethylamino-1-propanol, 1-diethylamino-2-propanol, 1-dimethylamino-2-propanol, 3-dimethylamino-1-propanol, 2-diisopropylaminoethanol, 3-diethylamino-1,2-propanediol, N-piperidineethanol, N,N-diethylethanolamine, benzyl alcohol, p-fluorobenzyl alcohol, p-bromobenzyl alcohol, p-chlorobenzyl alcohol, p-methoxylbenzyl alcohol, m-chlorobenzyl alcohol, m-(trifluoromethyl)benzyl alcohol, p-carboxybenzyl alcohol, phenol p-fluorophenol, p-bromophenol, p-chlorophenol, p-methoxyphenol, p-carboxyphenol, m-(trifluoromethyl)phenol, 4-cyanophenol, 3-hydroxypyridine, 2-chloro-3-hydroxypyridine, and 5-carboxy-3-hydroxypyridine.

## EXAMPLE 2388

### Preparation of 4-[(cyclohexyl)methylamino]hydrocinnamic acid

A 4 g. sample of ethyl 4-[(cyclohexyl)methylamino]hydrocinnamate is hydrolyzed with 1.6 g. 85% potassium hydroxide in 60 ml. 95% ethanol by refluxing the solution for 5 hours. The solution is cooled, diluted with 100 ml. water and acidified to pH 4.5 with 37% hydrochloric acid. The precipitate is collected, dried *in vacuo* and crystallized from acetone to yield the title compound as white powder.

## EXAMPLE 2389

### Preparation of 1-methanesulfonyloxy-2-allylcyclohexane

To a mixture of 250 ml. of dichloromethane, 25 g. 2-allylcyclohexanol and 16.7 g. of triethylamine cooled in an ice-salt bath to -10°C. is added dropwise, over 15 minutes, 18.9 g. of methanesulfonyl chloride. The mixture is cooled at -10°C to -15°C. for 30 minutes and then washed with 300 ml. each of cold water, 10% hydrochloric acid, sodium carbonate solution and with saturated sodium chloride solution. The organic layer is dried with magnesium sulfate and concentrated *in vacuo* to give a pale yellow oil.

EXAMPLE 2390

Preparation of ethyl 4-[(2-methylcyclopentyl)methylamino]-phenylacetate

To a cold (-20°) stirred solution of 10.8 g. 1-hydroxymethyl-2-methylcyclopentane prepared by lithium aluminum hydride reduction of methyl 2-methylcyclopentane-carboxylate and 13.4 ml. triethylamine in 300 ml. ether is added dropwise 5.6 ml. methanesulfonyl chloride in 5 ml. of either. After addition is completed, the solution is warmed to room temperature, stirred for 30 minutes and filtered directly into a solution of 23.1 g. ethyl 4-aminophenyl acetate in 100 ml. ether. After 17 hours at room temperature, the precipitate is filtered and washed with several portions of methylene chloride. The organic solution is washed twice with 100 ml. water, 100 ml. brine, dried and evaporated. The tan residue is crystallized from ethanol and from acetonitrile to yield the title compound as white crystals.

EXAMPLE 2391

Preparation of ethyl 4-[(3-isopropyl-2-methylcyclopentyl) methylamino]hydrocinnamate

A solution of 8.6 ethyl 4-aminohydrocinnamate, 9.77 g. 3-isopropyl-2-methylcyclopentanecarboxaldehyde and a few crystals of 2,4-dinitrobenzenesulfonic acid in 250 ml. toluene is refluxed under a Dean-Stark trap for 17 hours, whereupon the theoretical amount (0.8 ml.) water has been collected. The toluene is evaporated to yield ethyl 3-[4-(3-isopropyl-2-methylcyclopentyl)methylene-amino)phenyl]propionate as a crystalline mass.

To a mixture of 17.8 g. of the above compound in 250 ml. ethanol is added 1.68 g. sodium borohydride and the mixture is stirred at room temperature for 18 hours. Excess reagent is decomposed by addition of 10 ml. acetic acid. The solution is concentrated in vacuo and the residue is partitioned betweed toluene and aqueous potassium carbonate. After drying, the toluene is evaporated to yield a solid. Crystallization from acetonitrile and from ethanol affords the title compound as white crystals.

EXAMPLE 2392

Preparation of ethyl 3-[4-(2-allylcyclohexylamino)phenyl] propionate

A mixture of 5.0 g. of ethyl 4-aminohydrocinnamate, 10.0 g. of 1-methanesulfonyloxy-2-allylcyclohexane (prepared by the method of Example 189), 4.2 g. of anhydrous powdered potassium carbonate and 40 ml. hexamethylphosphoramide is heated to 80 for 17 hours. The mixture is then cooled, diluted with water and extracted with ethyl ether. The ether extracts are washed with water, dried and evaporated. The residue is recrystallized from ethanol yielding the title compound as white crystals.

EXAMPLE 2393

Preparation of ethyl 4-[(4-cycloheptyl)butylamino]-cinnamate

A mixture of ethyl p-aminocinnamate, 5.9 g. 4-bromobutylcycloheptane and one equivalent of anhydrous powdered potassium carbonate in 50 ml. hexamethylphosphoramide is heated for 20 hours at 60°C. The mixture is then cooled, diluted with water and extracted with ether. The combined ether extracts are dried, filtered and evaporated. Crystallization from acetonitrile provides the title compound as white crystals.

TABLE LI

The following 4-[(cycloalkyl or cycloalkenyl substituted)amino, alkylamino or alkenylamino]hydrocinnamates are prepared from the appropriate starting material by the method shown. Alcohols are converted to the corresponding mesylate by the method of Example 2389.

| Example No. | Method of Example | 4-(Substituted-amino)hydrocinnamate |
|---|---|---|
| 2394 | 2390 | Ethyl 4-[(cyclopentyl)methylamino]-hydrocinnamate |

| Example No. | Method of Example | 4-(Substituted-amino)hydrocinnamate |
|---|---|---|
| 2395 | 2393 | Ethyl 4-[1-(1,4-dimethylcyclohexyl)-2-propylamino]hydrocinnamate |
| 2396 | 2391 | Ethyl 4-(2-cyclopentylbutylamino)-hydrocinnamate |
| 2397 | 2392 | Ethyl 4-(4-cyclopentylbutylamino)-hydrocinnamate |
| 2398 | 2393 | Ethyl 4-cyclodecylaminohydrocinnamate |
| 2399 | 2390 | Ethyl 4-(3-cyclohexylcyclopentyl-amino)hydrocinnamate |
| 2400 | 2392 | Ethyl 4-[2-(2-decahydronaphthyl)-ethylamino]hydrocinnamate |
| 2401 | 2390 | Ethyl 4-(2-isopropyl-5-methylene-cyclopentylamino)hydrocinnamate |
| 2402 | 2393 | Ethyl 4-(4-isopropylcyclohex-2-enyl-amino)hydrocinnamate |
| 2403 | 2390 | Ethyl 4-[2-(2,3-dimethylcyclopent-2-enyl)propylamino]hydrocinnamate |
| 2404 | 2392 | Ethyl 4-(cyclopent-2-enylamino)-hydrocinnamate |
| 2405 | 2392 | Ethyl 4-(1-allylcyclododecylamino)-hydrocinnamate |

TABLE LII

The following 4-[(cycloalkyl or cycloalkenyl substituted)amino, alkylamino or alkenylamino]hydrocinnamic acids are prepared from the esters of Table LI by the method of Example 2388.

| Example No. | 4-(Substituted-amino)hydrocinnamic acids |
|---|---|
| 2406 | 4-[(Cyclopentyl)methylamino]hydrocinnamic acid |
| 2407 | 4-[1-(1,4-Dimethylcyclohexyl)-2-propylamino]-hydrocinnamic acid |
| 2408 | 4-(2-Cyclopentylbutylamino)hydrocinnamic acid |
| 2409 | 4-(4-Cyclopentylbutylamino)hydrocinnamic acid |

| Example No. | 4-(Substituted-amino)hydrocinnamic acids |
|---|---|
| 2410 | 4-Cyclodecylaminohydrocinnamic acid |
| 2411 | 4-(3-Cyclohexylcyclopentylamino)hydrocinnamic acid |
| 2412 | 4-[2-(2-Decahydronaphthyl)ethylamino]hydrocinnamic acid |
| 2413 | 4-(2-Isopropyl-5-methylenecyclopentylamino)-hydrocinnamic acid |
| 2414 | 4-(4-Isopropylcyclohex-2-enylamino)hydrocinnamic acid |
| 2415 | 4-[2-(2,3-Dimethylcyclopent-2-enyl)propylamino]-hydrocinnamic acid |
| 2416 | 4-(Cyclopent-2-enylamino)hydrocinnamic acid |
| 2417 | 4-(1-Allylcyclododecylamino)hydrocinnamic acid |

TABLE LIII

The following 4-[(cycloalkyl or cycloalkenyl substituted)amino, alkylamino or alkenylamino]cinnamates are prepared from the appropriate starting materials by the methods shown. Alcohols are converted to their corresponding mesylate by the method of Example 2389.

| Example No. | Method of Example | 4-(Substituted-amino)cinnamate |
|---|---|---|
| 2418 | 2390 | Ethyl 4-[(cyclopentyl)methylamino]-cinnamate |
| 2419 | 2393 | Ethyl 4-[(4-methylcyclohexyl)methyl-amino]cinnamate |
| 2420 | 2392 | Ethyl 4-[1-(1,4-dimethylcyclohexyl)-2-propylamino]cinnamate |
| 2421 | 2392 | Ethyl 4-[2-(2-methylcyclohexyl)ethyl-amino]cinnamate |
| 2422 | 2393 | Ethyl 4-[3-(3-ethylcyclohexyl)-2-methyl]propylaminocinnamate |
| 2423 | 2392 | Ethyl 4-[5-(cyclopropyl)-2-pentyl-amino]cinnamate |

| Example No. | Method of Example | 4-(Substituted-amino)cinnamate |
|---|---|---|
| 2424 | 2391 | Ethyl 4-(3-cyclohexylcyclopentyl-amino)cinnamate |
| 2425 | 2390 | Ethyl 4-[4-(1-decahydronaphthyl)-butylamino]cinnamate |
| 2426 | 2393 | Ethyl 4-(cyclonon-2-enylamino)cin-namate |
| 2427 | 2390 | Ethyl 4-[(1-cyclohex-2-enyl)ethyl-amino]cinnamate |
| 2428 | 2392 | Ethyl 4-[1-methyl-2-(cyclohex-1-enyl)ethylamino]cinnamate |
| 2429 | 2393 | Ethyl 4-[(2-methylene-2-cyclohexyl-ethyl)amino]cinnamate |
| 2430 | 2392 | Ethyl 4-(1-isopropenylcyclohexyl-amino)cinnamate |

TABLE LIV

The following 4-[cycloalkyl or cycloalkenyl substituted)amino, alkylamino or alkenylamino]cinnamic acids are prepared from the esters of Table LII by the method of Example 2388.

| Example No. | 4-(Substituted-amino)cinnamic acid |
|---|---|
| 2431 | 4-[(Cyclopentyl)methylamino]cinnamic acid |
| 2432 | 4-[(4-Methylcyclohexyl)methylamino]cinnamic acid |
| 2433 | 4-[1-(1,4-Dimethylcyclohexyl)-2-propylamino]-cinnamic acid |
| 2434 | 4-[2-(2-Methylcyclohexyl)ethylamino]cinnamic acid |
| 2435 | 4-[3-(3-Ethylcyclohexyl)-2-methylpropylamino]-cinnamic acid |
| 2436 | 4-[5-(Cyclopropyl)-2-pentylamino]cinnamic acid |
| 2437 | 4-(3-Cyclohexylcyclopentylamino)cinnamic acid |
| 2438 | 4-[4-(1-Decahydronaphthyl)butylamino]cinnamic acid |
| 2439 | 4-(Cyclonon-2-enylamino)cinnamic acid |

2440 4-[(1-Cyclohex-2-enyl)ethylamino]cinnamic acid

2441 4-[1-Methyl-2-(cyclohex-1-enyl)ethylamino]-cinnamic acid

2442 4-[(2-Methylene-2-cyclohexylethyl)amino]-cinnamic acid

2443 4-(1-Isopropenylcyclohexylamino)cinnamic acid

TABLE LV

The following 4-[(cycloalkyl or cycloalkenyl substituted)amino, alkylamino or alkenylamino]phenyl-propiolates are prepared from the appropriate starting materials by the methods shown. Alcohols are converted to their corresponding mesylate by the method of Example 2389.

| Example No. | Method of Example | 4-(Substituted-amino)phenylpropiolate esters |
|---|---|---|
| 2444 | 2391 | Ethyl 4-[(cyclopentyl)methylamino]-phenylpropiolate |
| 2445 | 2393 | Ethyl 4-(2-cyclopentylbutylamino)-phenylpropiolate |
| 2446 | 2393 | Ethyl 4-(4-cyclopentyl)-butylamino-phenylpropiolate |
| 2447 | 2392 | Ethyl 4-[2-(2-decahydronaphthyl)-ethylamino]phenylpropiolate |
| 2448 | 2392 | Ethyl 4-(4-isopropylcyclohex-2-enyl-amino)phenylpropiolate |
| 2449 | 2390 | Ethyl 4-(cyclopent-2-enylamino)-phenylpropiolate |
| 2450 | 2390 | Ethyl 4-(1-allylcyclododecylamino)-phenylpropiolate |

TABLE LVI

The following 4-[cycloalkyl or cycloalkenyl substituted)amino, alkylamino, or alkenylamino]phenyl-propiolic acids are prepared from the esters of Table LI by the method of Example 2388.

| Example No. | 4-(Substituted-amino)phenylpropiolic acid |
|---|---|
| 2451 | 4-[(Cyclopentyl)methylamino]phenylpropiolic acid |
| 2452 | 4-(2-Cyclopentylbutylamino)phenylpropiolic acid |
| 2453 | 4-(4-Cyclopentylbutylamino)phenylpropiolic acid |
| 2454 | 4-[2-(2-Decahydronaphthyl)ethylamino]phenylpropiolic acid |
| 2455 | 4-(4-Isopropylcyclohex-2-enylamino)phenylpropiolic acid |
| 2456 | 4-(Cyclopent-2-enylamino)phenylpropiolic acid |
| 2457 | 4-(1-Allylcyclododecylamino)phenylpropiolic acid |

TABLE LVII

The following 4-[(cycloalkyl or cycloalkenyl substituted)amino, alkylamino or alkenylamino]phenylbutyrates are prepared from the appropriate mesylates by the method of Example 2392. The requisite mesylates are prepared by the method of Example 2389

| Example No. | 4-(Substituted-amino)phenylbutyrate esters |
|---|---|
| 2458 | Ethyl 4-[4-(2-butylcyclopent-2-enylamino)-phenyl]butyrate |
| 2459 | Ethyl 4-[4-(1-allylcyclohexylamino)phenyl]-butyrate |
| 2460 | Ethyl 4-[4-(cyclooct-2-enylamino)phenyl]-butyrate |
| 2461 | Ethyl 4-[4-(cycloheptyl)butylamino]phenyl-butyrate |
| 2462 | Ethyl 4-[4-(1-cyclopentylethylamino)phenyl]-butyrate |
| 2463 | Ethyl 4-[4-(cyclooctylmethylamino)phenyl]-butyrate |

TABLE LVIII

The following 4-[cycloalkyl or cycloalkenyl substituted amino, alkylamino or alkenylamino]phenylbuty-

ric acids are prepared from the esters of Table LVII by the method of Example 2388.

| Example No. | 4-(Substituted-amino)phenylbutyric acid |
|---|---|
| 2464 | 4-[4-(2-Butylcyclopent-2-enylamino)phenyl]-butyric acid |
| 2465 | 4-[4-(1-Allylcyclohexylamino)phenyl]butyric acid |
| 2466 | 4-[4-(Cyclooct-2-enylamino)phenyl]butyric acid |
| 2467 | 4-[4-(Cycloheptyl)butylamino]phenylbutyric acid |
| 2468 | 4-[4-(1-Cyclopentylethylamino)phenyl]butyric acid |
| 2469 | 4-[4-(Cyclooctylmethylamino)phenyl]butyric acid |

EXAMPLE 2470

Preparation of 4-(2-allylcyclohexylamino)acetophenone

p-Aminoacetophenone is heated with 5 g. 1-methanesulfonyloxy-2-allylcyclohexane (prepared by the method of Example 189) in 50 ml. hexamethylphosphoramide containing anhydrous potassium carbonate (1.9 g.) for 16 hours a 100°C. The solution is cooled to room temperature, filtered to remove solids, and the filtrate is diluted with cold water (50 ml.). The amber solid so obtained is collected and and washed with water. Recrystallization from ethanol followed by dichloromethane provides 4-(2-allylcyclohexylamino)acetophenone.

TABLE LIX

The following 4-[(cycloalkyl or cycloalkenyl substituted)amino, alkylamino or alkenylamino]acetophenones are prepared by the method of Example 2470. The requisiste mesylates are prepared by the method of Example 2389.

| Example No. | 4-(Substituted-amino)acetophenone |
|---|---|
| 2471 | 4-(2-Butylcyclopent-2-enylamino)acetophenone |

2472 4-[(1-Cyclohex-2-enyl)ethylamino]acetophenone

2473 4-(Cycloheptadec-8-enylamino)acetophenone

2474 4-(2-Cyclohexylethylamino)acetophenone

2475 4-[(Cyclopentyl)methylamino]acetophenone

EXAMPLE 2476

Preparation of sodium 4-(1-cyclopentylethylamino)phenylacetate

A mixture of 3.62 g. of 4-(1-cyclopentylethyl-amino)phenylacetic acid and 25 ml. of ethanol water (9:1) containing 0.400 g. of sodium hydroxide is stirred for 4 hours. The mixture is filtered and the residue washed with 10 ml. of ethanol-water (9:1) and dried *in vacuo* for 24 hours to yield 4-(1-cyclopentylethylamino)phenyl acetate as a white solid.

EXAMPLE 2477

Preparation of 4-(2-cyclopentylbutylamino)phenylacetyl chloride

A cold solution of 25 g. 4-(2-cyclopentylbutyl-amino)phenylacetic acid in 500 ml. dimethoxyethanemethylene chloride (4:1) is prepared and dry hydrochloric acid is bubbled through the solution until no more precipitate forms. The solution is treated with 25 ml. thionyl chloride and refluxed until all of the precipitate has dissolved. The solvents are evaporated to yield the acid chloride hydrochloride as an orange, semi-crystalline mass.

EXAMPLE 2478

Preparation of 4-(N-trifluoroacetyl-1-cyclopentylethyl-amino)phenylacetyl chloride

A stirred ice-cold suspension of 9 g. 4-(1-cyclo-pentylethylamino)phenylacetic acid in 100 ml. of dimethoxyethane and 161. of pyridine is treated with 18 ml. of trifluoroacetic anhydride at 0°C. The solution is stirred for 30 minutes at room temperature and then diluted with 300 ml. ether and 100 g. ice. After stirring vigorously

for 15 minutes, the phases are separated, the ehter solution is washed with brine, dried and evaporated to a white, amorphous solid.

To a solution of 9.2 g. of the above solid in 30 ml. methylene chloride and 0.5 ml. dimethylformamide is added 5.7 ml. thionyl chloride. After 20 hours at reflux, the solvents are evaporated to yield 4-[N-trifluoroacetyl-1-cyclopentylethylamino)phenylacetyl chloride as a light yellow, mobile oil.

EXAMPLE 2479

Preparation of 4-(N-carbobenzyloxy-N-cyclooctylmethyl-amino)phenylacetyl chloride

To 15 g. 4-(cyclooctylmethylamino)phenylacetic acid in 200 ml. warm chloroform is added a solution of 12 g. sodium carbonate in 150 ml. water. To the vigorously stirred solution is added 10 g. carbobenzyloxy chloride. After 2 hours stirring at 40°C., the layers are separated, washed three times with 1N hydrochloric acid, dried, and evaorated to an oil. The oil is dissolved in 30 ml. Prevaporated to an oil. The oil is dissolved in 300 ml. toluene, treated with 15 ml. thionyl chloride and the solution is refluxed for 5 hours. The solvents are evaporated and the residue is dissolved three times in toluene, evaporating each time ultimately to yield 4-(N-carbobenzyloxycyclooctylmethylamino)phenylacetyl chloride as a viscous, orange oil.

EXAMPLE 2480

Preparation of 1-[4-(N-*tert*-butyloxycarbonyl)cyclopentyl-ethylaminophenylacetyl]imidazole

To a solution of 10 g. 4-(cyclopentylethylamino)-phenylacetic acid in 100 ml. dioxane is treated with 4.0 g. *tert*-butylazidoformate and 10 ml. pyridine. After stirring at room temperature for 18 hours, the protected amidoacid is precipitated from solution by the addition of 150 ml. water. The solid is collected, thoroughly dried,

and dissolved in 200 ml. of a mixture consisting of methylene chloride/dimethoxyethane/pyridine (1:4:1). To this solution is stirred overnight at room temperature and the solvents are evaporated to yield 1-[4-(N-tert-butyloxycarbonyl)cyclopentylethylaminophenylacetyl]imidazole as an orange oil.

EXAMPLE 2481

Preparation of diethyl 4-(1-cyclopentylethylamino)benzoylmalonate

A solution of 26.6 g. of diethyl malonate and 10 ml. of 1,2-dimethoxyethane is added to a suspension of 4.0 g. of 4-(1-cyclopentylethylamino)benzoyl chloride hydrochloride in 1,2-dimethoxyethane under argon. A solution of 17.3 g. of 1,2-dimethoxyethane is then added. The reaction mixture is refluxed for 4.5 hours, cooled, poured on ice, acidified, and extracted with ether. The ether solution is washed with water and saturated sodium chloride solution, dried over anhydrous sodium sulfate and concentrated to dryness. Addition of a small amount of ethanol to the residue gives a solid which is filtered and discarded. The ethanol filtrate is concentrated and the residue is recrystallized from ether to yield diethyl 4-(1-cyclopentylethylamino)benzoylmalonate.

EXAMPLE 2482

Preparation of tert-butyl ethyl 4-(1-cyclopentylethylamino)benzoylmalonate

A solution of 28.0 g. of tert-butyl ethyl malonate in 10 ml. of 1,2-dimethoxyethane is added to a suspension of 4.0 g. of sodium hydride in 1,2-dimethoxyethane under argon. A solution of 17.3 g. of 4-(1-cyclopentylethylamino)benzoyl chloride hydrochloride in 1.2-dimethoxyethane is then added. The reaction mixture is refluxed for 5 hours, cooled, poured on ice and extracted with ether. The ether solution is washed with water and saturated sodium chloride solution, dried over anhydrous

sodium sulfate and concentrated to dryness. The residue is then recrystallized from ether to yield tert-butyl ethyl 4-(1-cyclopentylethylamino)benzoyl malonate.

EXAMPLE 2483

Preparation of ethyl 2-[4-(1-cyclopentylethylamino)benzoyl-acetoacetate

A solution of 21.6 g. of ethyl acetoacetate and 10 ml. of 1,2-dimethoxyethane is added to a suspension of 4.0 g. of sodium hydride in 1,2-dimethoxyethane under argon. A solution of 17.3 g. of 4-(1-cyclopentylethyl-amino)benzoyl chloride hydrochloride in 1,2-dimethoxyethane is then added. The reaction mixture is refluxed for 5 hours, cooled, poured on ice and extracted with ether. The ether solution is washed with water and saturated sodium chloride solution, dried over anhydrous sodium sulfate and concentrated to dryness. Recrystallization from ether affords ethyl 2-[4-(1-cyclopentylethylamino)-benzoylacetoacetate as a white solid.

EXAMPLE 2484

Preparation of ethyl 4-(1-cyclopentylethylamino)benzoyl-acetate

A solution of 3.0 g. of tert-butyl ethyl 4-(1-cyclopentylethylamino)benzoylmalonate 10 ml. of trifluoroacetic acid is warmed with stirring for 3 hours. The solution is poured onto ice and neutralized with potassium hydroxide. The resulting precipitate is collected by filtration, washed with water and dried. Recrystallization from chloroform affords ethyl 4-(1-cyclopentylethylamino)-benzoylacetate.

EXAMPLE 2485

Preparation of 4-(1-cyclopentylethylamino)benzoyl acetic acid

Two grams of ethyl 4-(1-cyclopentylethylamino)-benzoylacetate is added to a solution of potassium hydroxide in 50 ml. of 1:9 water-ethanol. The reaction of neutralization with sulfuric acid gave a precipitate which is filtered, washed with water, and dried to yield 4-(1-cyclopentylethylamino)benzoylacetic acid.

## EXAMPLE 2486

Preparation of 4'-(1-cyclopentylethylamino)-2-(methylsulfinyl)acetophenone

To a solution of 5.8 g. of dimethyl sulfoxide, dried over sieves, and 50 ml. of tetrahydrofuran is slowly aaded 28 ml. of n-butyllithium (2.4 M in hexane). To this mixture is added 10 g. of methyl 4-(1-cyclopentylethylamino)benzoate in 200 ml. of tetrahydrofuran. After two hours, the reaction mixture is poured onto ice, acidified with dilute hydrochloric acid and quickly extracted with chloroform. The chloroform extract is washed with water and saturated sodium chloride solutien, and dried over anhydrous sodium sulfate. Concentration affords a solid which is washed with 500 ml. of hot hexane, filtered while hot and then washed with hexane. The white solid is dried in vacuo to yield 4'-(1-cyclopentylethylamino)-2-(methylsulfinyl)acetophenone

## EXAMPLE 2487

Preparation of 4'-(1-cyclopentylethylamino)-2-(phenylsulfonyl)acetophenone

A solution of 864 mg. of sodium hydride and 5.3 g. of methylphenylsulfone in 20 ml. of 1, 2-dimethoxyethane is stirred at 6°C. for one hour under an atmosphere of argon. To this solution is added a solution of 5.0 g. of methyl (1-cyclopentylethylamino)benzoate in 50 ml. of tetrahydrofuran and the reaction mixture is stirred at 60°C. for 1.5 hours. The mixture is cooled, poured onto ice, acidified with dilute hodrochloric acid and pH 3 and then extracted with chloroform. The organic layer is separated, washed with water and saturated sodium chloride solution, dried over anhydrous sodium sulfate and then concentrated to dryness. The crude solid is chromatographed on silica gel, eluting with methylene chloride to yield 4'-(1-cyclopentylethylamino)-2-(phenylsulfonyl)acetophenone.

EXAMPLE 2488

Preparation of 4'-(1-cyclopentylethylamino)-2-(phenylsulfinyl)acetophenone

To a solution of 6.2 g. of methylphenylsulfoxide, dried over sieves, and 50 ml. of tetrahydrofuran is slowly added 28 ml. of n-butyllithium (2.4 M in hexane). To this mixture is added 10 g. of a solution of methyl 4-(1-cyclopentylethylamino)benzoate in 200 ml. of tetrahydrofuran. After two hours, the reaction mixture is poured into ice, acidified with diluted hydrochloric acid and quickly extracted with chloroform. The chloroform layer is washed with water and saturated sodium chloride solution and dried over anhydrous sodium sulfate. Concentration affords a solid which is washed with 500 ml. of hot hexane, filtered while hot, and then washed with 50 ml. of hexane. The white solid is dried *in vacuo* yielding 4'-(1-cyclopentylethylamino)-2-(phenylsulfinyl)acetophenone.

EXAMPLE 2489

Preparation of 3-[4'-(1-cyclopentylethylamino)benzoyl]-2,4-pentanedione

A solution of 28.4 g. of 2,4-pentanedione and 20 ml. of 1,2-dimethoxyethane is added to a suspension of 13.6 g. of sodium hydride in 220 ml. of 1,2-dimethoxyethane under argon. A solution of 28.7 g. of 4-(1-cyclopentylethylamino)benzoyl chloride hydrochloride in 1,2-dimethoxyethane is then added. The reaction mixture is stirred at room temperature for 12 hours, cooled, poured on ice and extracted with ether. The ether solution is washed with water and saturated sodium chloride solution, dried over anhydrous sodium sulfate and concentrated. The residue is then chromatographed over silica gel to yield 3-[4'-(1-cyclopentylethylamino)benzoyl]-2,4-pentanedione.

EXAMPLE 2490

Preparation of methyl 3-[4-(1-cyclopentylethylamino)-benzoyl]propionate

A mixture of 35 g. of 3-(4-acetamidobenzoyl)propionic acid, 700 ml. of methanol and 1.4 ml. of concentrated sulfuric acid is refluxed for 76 hours. The solution is cooled to 35° C. and poured onto 7 g. of anhydrous sodium acetate while stirring. The reaction mixture is stirred in an ice-bath. The solid is collected and washed with cold methanol to yield 3-(4-aminobenzoyl)-propionate as a white solid. A mixture of this solid, 9.2 g. of 1-cyclopentylethylbromide and 4.2 g of potassium carbonte is stirred for 20 hours at 125°C. under nitrogen. The mixture is then cooled to 25°C. and 30 ml. of water is added. After stirring, the product is filtered and washed with water. Recrystallization from methanol affords methyl 3-[4-(1-cyclopentylethylamino)benzoyl]propionate as a white solid.

EXAMPLE 2491

Preparation of 3-[4-(1-cyclopentylethylamino)benzoyl]-propionic acid

A solution of 5.4 g. of methyl 3-[4-(1-cyclopentylethylamino)benzoylpropionate is stirred with 5.4 g. of potassium hydroxide in 100 ml. of 95% ethanol for 3 hours at reflux. The reaction mixture is cooled, diluted with 50 ml. of ethanol and 100 ml. of water, neutralized with hydrochloric acid. The solution is cooled to room temperature and filtered. The white solid is washed with 50% aqueous ethanol and dried. The product is recrystallized from ethanol to yield 3-[4-(1-cyclopentylethylamino)benzoyl]propionic acid.

TABLE LX

The following acetophenones are prepared by the noted methods from the carboxylic acids of Tables XLIX or L or appropriate derivatives thereof which are obtained by the methods of Examples 2477-2480.

| Example No. | Method of Example | 4-(Substituted-amino)acetophenones |
|---|---|---|
| 2492 | 2481 | Diethyl 4-[(3-cyclohexylpentyl)-amino]benzoylmalonate |
| 2493 | 2482 | *tert*-Butyl ethyl 4-(1-cyclopent-3-enylmethylamino)benzoylmalonate |

| | | |
|---|---|---|
| 2494 | 2483 | Ethyl 2-[4-(cycloheptylmethylamino)-benzoyl]acetoacetate |
| 2495 | 2484 | Ethyl 4-[3-methyl-3-(4-methylcyclohex-3-enyl)propylamino]benzoylacetate |
| 2496 | 2485 | 4-[3-(Cyclohex-3-enyl)propylamino]-benzoylacetic acid |
| 2497 | 2486 | 4-[3-(3-Cyclohexenyl)propylamino]-2-(methylsulfonyl)acetophenone |
| 2498 | 2487 | 4'-(Cyclonon-3-enylamino)-2-(phenyl-sulfonyl)acetophenone |
| 2499 | 2488 | 4'-[(5-Ethylcyclohex-3-enyl)-methylamino]-2-(phenylsulfinyl)-acetophenone |
| 2500 | 2489 | 3'-[4-(4-Isopropylcyclohex-2-enylamino)-benzoyl]-2,4-pentanedione |
| 2501 | 2490 | Methyl 3-[4-(2-cylooct-1-enylethyl-amino)benzoyl]propionate |
| 2502 | 2491 | 3-[4-(2-Methyl-6-methylcycloheptyl-amino)benzoyl]propionic acid |

EXAMPLE 2503

Preparation of 4-(2-methylcyclopentylmethyl)amino]benzonitrile

4-Aminobenzonitrile (11.8g.) and 1-iodomethyl-2-methylcyclopentane (16.3 g.) are dissolved in hexamethylphosphoramide (100 ml.) and heated under nitrogen in an oil bath maintained at 120°C. for 22 hours. The reaction mixture is cooled to room temperature and water (100 ml.) is added gradually. The mixture is then chilled in an ice bath. The precipitate separated is filtered, washed thoroughly with water, and dried. It is then washed repeatedly with hexane and dried. Recrystallization from ether-hexane affords 4-(2-methylcyclopentylmethylamino)-benzonitrile as pale yellow crystals.

EXAMPLE 2504

Preparation of 4-(cyclohex-3-enylamino)benzaldehyde

Di-isobutylaluminum hydride (54 ml., 25% solution in toluene) is added with stirring to a solution of 12.1 g.

of 4-(cyclohex-3-enylamino)benzonitrile under a nitrogen atmosphere. After addition is completed, the solution stirred for one hour. A solution of methanol in toluene (50 ml., 1:1) is added over 30 minutes and the mixture is poured into 500 ml. vigorously stirred ice-cold 50% aqueous sulfuric acid. The mixture is filtered and the organic layer separated. The aqueous solution is extracted twice with toulene (100 ml.) and the combined organic layers are washed with acqueous sodium bicarbonate, dried over magnesium sulfate, decolorized with charcoal, filtered and evaporated in vacuo to give a light yellow crystalline solid. The product is recrystallized from dicloro-methane/hexane giving colorless needles.

TABLE LXI

The following 4-[(cycloalkyl or cycloalkenyl substituted)amino, alkylamino or alkenylamino]benzonitriles are prepared by the method of Example 2503.

| Example No. | 4-(Substituted-amino)benzonitrile |
|---|---|
| 2505 | 4-(Cyclohex-3-enylamino)benzonitrile |
| 2506 | 4-(Cyclohexylmethylamino)benzonitrile |
| 2507 | 4-(2-Methyl-6-methylenylcycloheptylamino) benzonitrile |
| 2508 | 4-[(4-Cyclopropyl)but-3-enylamino] benzonitrile |
| 2509 | 4-[1-(3-Butenyl)-2-methylcycloheptylamino] benzonitrile |
| 2510 | 4-[4-(2-Decahydronaphthyl)butylamino]benzoni-trile |
| 2511 | 4-[5-(1-Cyclopentenyl)pentylamino]benzonitrile |

TABLE LXII

The following 4-[(cycloalkyl or cycloalkenyl substituted amino, alkylamino or alkenylamino]benzaldehydes are prepared from the corresponding benzonitriles of Table LXI by the method of Example 2504.

TABLE LXII (cont'd.)

| Example No. | 4-(Substituted-amino)benzaldehydes |
|---|---|
| 2512 | 4-(Cyclohex-3-enylamino)benzaldehyde |
| 2513 | 4-(Cyclohexylmethylamino)benzaldehyde |
| 2514 | 4-(2-Methyl-6-methylenylcycloheptylamino) benzaldehyde |
| 2515 | 4-[(4-Cyclopropyl)but-3-enylamino] benzaldehyde |
| 2516 | 4-[1-(3-Butenyl)-2-methylcycloheptylamino] benzaldehyde |
| 2517 | 4-[4-(2-Decahydronaphthyl)butylamino benzaldehyde |
| 2518 | 4-[5-(1-Cyclopentenyl)pentylamino]benzaldehyde |

## EXAMPLE 2519

### Preparation of 2,3-dihydroxypropyl 4-(2-cyclohexylethylaminophenylacetate

A solution of 7.34 g. of 4-(2-cyclohexylethylamino)phenylacetic acid, 4.80 g. of 25% aqueous sodium hydroxide, and 12.6 g. of 3-iodo-1,2-propanediol in 50 ml. of hexamethylphosphoramide is stirred for 24 hours at ambient temperature, diluted with 100 ml. of ether and stirred for 5 days at ambient temperature. The mixture is treated with water and extracted with ether. The dried extracts are evaporated to yield 2,3-dihydroxypropyl 4-(2-cyclohexylethylaminophenylacetic acid.

## EXAMPLE 2520

### Preparation of methyl 4-(2-cyclohexylethylamino)phenylacetate

A solution of 20.7 g. of 4-(2-cyclohexylethylamino)phenylacetic acid in 25 ml. of hexamethylphosphoramide is added to a stirred mixture of 0.800 g. of sodium hydride (57% in mineral oil) and 25 ml. of hexamethylphosphoramide. The solution which forms after one hour is treated with 11.0 g. of methyl iodide and is then stirred at 25°C. for 18 hours. Dilution with water followed by

filtration affords a white solid which is crystallized from ethanol to yield methyl 4-(2-cyclohexylethylamino)-phenylacetate.

EXAMPLE 2521

Preparation of 3-hydroxypropyl 4-(2-cyclohexylethylamino)-phenylacetate

A mixture of 2.25 g. of methyl 4-(2-cyclohexyl-ethylamino)phenylacetate, 280 mg. of 1,3-propanediol and 1.37 g. p-toluenesulfonic acid is heated at 180°C. for 18 hours and then is partitioned between ether and 3% aqueous sodium carbonate solution. The ether layer is separated, dried, and evaporated to yield 3-hydroxypropyl 4-(2-cyclo-hexylethylamino)phenylacetate.

EXAMPLE 2522

Preparation of 2-ethoxyethyl 4-(cyclohex-2-enylmethyl-amino)phenylacetate

A solution of 11.8 g. of 4-(cyclohex-2-enylmethyl-amino)phenylacetic acid, 1.00 g. of 2-ethoxyethanol and 5.35 ml. of boron trifluoride etherate in 200 ml. toluene is stirred under reflux for 48 hours. The solution is treated with an additional 5.35 ml. of boron trifluoride etherate and refluxing is continued for 120 hours. Dilution with water and methylene chloride followed by filtration affords 2-ethoxyethyl 4-(cyclohex-2-enylmethyl-amino)phenylacetate.

EXAMPLE 2523

Preparation of methyl 4-(2-cyclohexylhept-3-enylamino)-hydrocinnamate

A solution of 50.5 g. of 4-(2-cyclohexylhept-3-enylamino)hydrocinnamic acid and 34.4 ml. of boron trifluoride etherate in 200 ml. of methanol is stirred under reflux for 44 hours, allowed to cool, and poured into 1.20 liters of ice-cold 5% aqueous sodium carbonate solution. The white solid is collected by filtration and recrystallized from benzene-ethanol to yield methyl 4-(2-cyclohexyl-hept-3-enyl-amino)hydrocinnamate.

EXAMPLE 2524

Preparation of 1-(methoxycarbonyl)propyl 4-(cyclohex-3-enylmethylamino)hydrocinnamate

To a solution of 10.0 g. 4-(cyclohex-3-enylmethylamino)hydrocinnamoyl chloride hydrochloride in 200 ml. methylene chloride is added dropwise a solution of 3 g. methyl 2-hydroxybutyrate and 5 g. triethylamine in 100 ml. ether. After 17 hours stirring at room temperature, the precipitate is collected and washed with several portions of ether. The ether solution is washed with water, dried and evaporated to yield 1-(methoxycarbonyl)propyl 4-(cyclohex-3-enylmethylamino)hydrocinnamate as a white solid.

EXAMPLE 2525

Preparation of 1-(ethoxycarbonyl)ethyl 4-(2-cyclohexylethyl amino)phenylacetate

To a warm mixture of 7 g. sodium 4-(2-cyclohexylethylamino)phenylacetate in 100 ml. ethanol is added 4.7 g. ethyl 2-tosyloxypropionate. After 17 hours at reflux, the cooled solution is diluted with an equal volume of water and the resultant precipitate is filtered. After washing with cold ethanol and drying, the product is crystallized from acetonitrile to yield 1-(ethoxycarbonyl)ethyl 4-(2-cyclohexylethylamino)phenylacetate as colorless crystals.

EXAMPLE 2526

Preparation of 1-carboxyethyl 4-(2-cyclohexylethylamino)-phenylacetate

A flask containing 10.0 g. 4-(2-cyclohexylethylamino)phenylacetic acid, 3.3 g. lactic acid, 500 mg. toluenesulfonic acid and 500 ml. toluene equipped with a Soxhlet extractor charged with activated 4Å Linde molecular sieves. The solution is refluxed for 24 hours during which time the Soxhlet extractor is charged twice more with fresh sieves. The hot solution is filtered and left to cool, whereupon 1-carboxyethyl 4-(2-cyclohexylethylamino)phenylacetate separates as off-white crystals.

EXAMPLE 2527

Preparation of diethyl O-[4-(2-cyclohexylethylamino)phenyl-acetyl]tartrate

A mixture of 4-[N-trifluoroacetyl-(2-cyclohexyl-ethylamino)]phenylacetyl chloride and 1.2 g. triethylamine in 100 ml. warm ether is treated with 2.5 g. diethyl tartrate and refluxed for 24 hours. The hot solution is filtered, the residue is washed with hot ether, and the solution is evaporated. After treatment with aqueous methanolic potassium carbonate, the product is precipitated by acidification, filtered, and dried. Crystallization from acetone yields diethyl O-[4-(2-cyclohexylethylamino)-phenylacetyl]tartrate as a white, crystalline solid.

EXAMPLE 2528

Preparation of O-[4-(2-cyclohexylethylamino)phenylacetyl]-malic acid

A warm solution of 4-[N-carbobenzyloxy-(2-cyclo-hexylethylamino)phenylacetyl chloride and 1.3 g. triethylamine in 100 ml. ether is treated with 2 g. malic acid. An immediate precipitate forms, but the mixture is refluxed for one hour and filtered while hot. The solid is washed several times with hot ether, then the ether is evaporated to yield a white solid. The product is dissolved in tetrahydrofuran (100 ml.) and hydrogenated over 600 mg. 10% palladium-on-carbon at 50 psi until hydrogen uptake stops. The catalyst is filtered, and the solution is evaporated. The residue is crystallized from acetic acid to yield O-[4-(2-cyclohexylethylamino)phenylacetyl]malic acid.

EXAMPLE 2529

Preparation of 2-(ethoxycarbonyl)vinyl 4-(2-cyclohexylethyl-amino)phenylacetate

To a mixture containing 4.3 g. 1-[4-(N-t-butyl-oxycarbonyl-2-cyclohexylethylamino)phenylacetyl]imidazole 50 ml. 5N sodium hydroxide is added 3 g. ethyl 2-formyl acetate. The solution is vigorously stirred for 24 hours. The layers are separated, and the chloroform solution

is washed once with 50 ml. 1N sodium hydroxide. The solvent is evaporated and the residue is heated for 30 minutes at 40°C. in 50 ml. anhydrous trifluoracetic acid. The solvent is again evaporated and the oil is crystallized from acetone to yield light yellow crystals of 2-(ethoxycarbonyl)vinyl-4-(2-cyclohexylethylamino)phenylacetate.

TABLE LXIII

The following esters are prepared by the methods shown from the carboxylic acids of Tables XLIX, L, LII, LIV, LVI and LVIII or appropriate derivatives thereof obtained by the methods of Examples 2476-2480.

| Example No. | Method of Example | Ester |
|---|---|---|
| 2530 | 2519 | 2,3-Dihydroxypropyl 4-(1-cyclopentylethylamino)phenylacetate |
| 2531 | 2519 | 2,3-Dihydroxypropyl 4-(cyclohex-2-enylmethylamino)hydrocinnamate |
| 2532 | 2519 | 2,3-Dihydroxypropyl 4-(cyclooct-4-enylamino)cinnamate |
| 2533 | 2519 | 2,3-Dihydroxypropyl 4-(1-allyl-2-methylcyclohexylamino)phenyl propiolate |
| 2534 | 2519 | 2,3-Dihydroxypropyl 4-(4-cyclopentylbut-2-enylamino)butyrate |
| 2535 | 2520 | Methyl 4-(cyclooctylmethylamino)-phenylacetate |
| 2536 | 2520 | Methyl 4-(cyclooct-2-enylamino)-hydrocinnamate |
| 2537 | 2520 | Methyl 4-(2-butylcyclopent-2-enylamino)cinnamate |
| 2538 | 2520 | Methyl 4-(cyclohex-3-enylamino)-phenylpropiolate |
| 2539 | 2520 | Methyl 4-[4-(cyclohexylmethylamino)-phenyl]butyric acid |
| 2540 | 2521 | 2-Hydroxypropyl 4-(3-cyclopentylpropylamino)phenylacetate |

TABLE LXIII (cont'd)

| Example No. | Method of Example | Ester |
|---|---|---|
| 2541 | 2521 | 4-Hydroxybutyl 4-[(2-cyclohexyl)hex-4-enylamino]hydrocinnamate |
| 2542 | 2521 | 2-Hydroxypropyl 4-(cyclopropyl-methylamino)cinnamate |
| 2543 | 2521 | 3-Hydroxypropyl 4-(cyclohexyl-methylamino)phenylpropiolate |
| 2544 | 2521 | 2-Hydroxyethyl 4-[(2-methylcyclo-hexylmethylamino)butyrate |
| 2545 | 2522 | 2-Methoxyethyl 4-(cyclohex-2-enyl-methylamino)phenylacetate |
| 2546 | 2522 | 2-Ethoxyethyl 4-(1-cyclopentylbut-2-enylamino)propiolate |
| 2547 | 2523 | Methyl 4-(2-cyclopentylhexyl-amino)hydrocinnamate |
| 2548 | 2523 | Methyl 4-(4-cycloheptylpentyl-amino)cinnamate |
| 2549 | 2524 | 1-Methoxycarbonylpropyl 4-(cyclo-hexylmethylamino)hydrocinnamate |
| 2550 | 2524 | 1-Ethoxycarbonylpropyl 4-[2-(cyclo-butylpropyl)amino]phenylpropiolate |
| 2551 | 2525 | 1-Ethoxycarbonylethyl 4-(cyclo-pentylmethylamino)phenylpropiolate |
| 2552 | 2526 | 1-Carboxyethyl 4-(2-cyclohexvl-propylamino)phenylacetate |
| 2553 | 2526 | 1-Carboxyethyl 4-[2-(2-ethylcyclo-hexyl)ethylamino]cinnamate |
| 2554 | 2526 | 1-Carboxybutyl 4-(2-cyclopentyl-ethylamino)propiolate |
| 2555 | 2526 | 1-Carboxyethyl 4-[4-(cyclopentyl-methylamino)phenyl]butyrate |
| 2556 | 2527 | 3-Pyridyl 4-(cyclooctylmethyl-amino)cinnamate |

TABLE LXIII (cont'd)

| Example No. | Method of Example | Ester |
|---|---|---|
| 2557 | 2528 | O-[4-(Cyclohexylmethylamino)-benzoylmalic acid |
| 2558 | 2528 | O-[4-(4-cyclopentylbut-3-ynyl)-benzoyl]malic acid |
| 2559 | 2529 | 2-(Ethoxycarbonyl)vinyl 4-(cyclohexylmethylamino)hydrocinnamate |
| 2560 | 2529 | 2-(Ethoxycarbonyl)vinyl 4-(3-cyclopentylpropylamino)cinnamate |
| 2561 | 2529 | 2-(Ethoxycarbonyl)vinyl 4-(cyclohex-3-enylmethylamino)propiolate |
| 2562 | 2529 | 2-(Ethoxycarbonyl)vinyl 4-[4-(1-cycloheptylpent-2-enylamino)-butyrate |

TABLE LXIV

The following compounds are prepared from the carboxylic acids which may be prepared from the corresponding nitriles and aldehydes which are obtained by the methods of Examples 2503 and 2504 or from appropriate acid derivatives obtained by the methods of Examples 2476-2480.

| Example No. | Method of Example | 4-(Substituted-amino)compounds |
|---|---|---|
| 2563 | 2481 | Diethyl 4-(cyclooctylmethylamino)-benzoylmalonate |
| 2564 | 2482 | tert-Butyl ethyl 4-(2-cyclopentyl-butylamino)benzoylmalonate |
| 2565 | 2483 | Ethyl 2-[4-(cyclopentylbut-2-enyl-aminobenzoyl]acetoacetate |
| 2566 | 2484 | Ethyl 4-[2-(2-methylcyclohexyl)-ethylamino]benzoylacetate |
| 2567 | 2485 | 4-(Cyclobutylmethylamino)benzoyl-acetic acid |
| 2568 | 2490 | Methyl 3-[4-(2-cyclohexylethyl amino)benzoyl]propionate |
| 2569 | 2491 | 3-[4-(2-Cyclooct-1-enylethylamino)-benzoyl]propionic acid |

EXAMPLE 2570

Preparation of p-(allylamino)phenylacetic acid; (Method A)

To a solution of 18.2 g. of ethyl p-aminophenylacetate in 100 ml. of dimethylformamide is added a solution of 4.7 ml. of allyl bromide in 60 ml. of dimethylformamide. The solution is heated at 60°C. for 5 hours and then cooled and partitioned between diethyl ether and water. The combined ether phases are washed with water, dried over magnesium sulfate, and concentrated in vacuo to provide 16.5 g. of a semi-solid. A portion (6 g.) is absorbed onto 30 g.

of silica gel and chromatographed on 475 g. of silica gel to provide ester.

A mixture of the ester, 22.0 g. of potassium hydroxide and 200 ml. of ethanol-water (8:1) is stirred under reflux for 6 hours. Concentrated hydrochloric acid (about 80 ml.) is added to the warm mixture and cooling and dilution with water affords a white solid which is collected by filtration and recrystallized from ethanol to yield the product as a white solid.

EXAMPLE 2571

Preparation of p-(1-pentadeca-4,14-dienylamino)-phenylacetic acid; (Method B)

To a solution of 4,14-pentadecadien-1-ol (15.0 g.) and triethylamine (14 ml.) in dry methylene chloride (320 ml.) at -8°C. is added methanesulfonylchloride (5.73 ml.), dropwise. The reaction mixture is stirred at -10°C. for 30 minutes and then diluted with methylene chloride, extracted with ice-water (250 ml.); followed by cold 10% hydrochloric acid (200 m.); cold saturated sodium bicarbonate (200 ml.) and cold brine (200 ml.). The organic phase is dried over magnesium sulfate and the solvent removed *in vacuo* to provide the crude mesylate.

A solution of 18.1 g. of the above mesylate and 19.8 g. of ethyl *p*-aminophenylacetate in hexamethylphosphoramide is heated at 120°C. for 20 hours. After cooling, the reaction mixture is diluted with 30 ml. of ethanol:water (1:1) (30 ml.) and chilled. More ethanol is added and the solid material is collected. This solid is recrystallized twice from ethanol to provide the ester.

A mixture of the ester, 22.0 g. of potassium hydroxide and 200 ml. of ethanol-water (8:1) is stirred under reflux for 6 hours. Concentrated hydrochloric acid (about 80 ml.) is added to the warm mixture and cooling and dilution with water affords a white solid which is collected by filtration and recrystallized from ethanol to yield the product as a white solid.

EXAMPLES 2572 - 2876

Treatment of the indicated halide or carbinol starting material set forth in Table I below by the indicated method is productive of the product listed in the table. Reference B in Tables LXV and LXVI is J. Med. Chem. 11, 1190 (1968).

TABLE LXV

| Example | Starting Material | Method | Product |
|---|---|---|---|
| 2572 | E-4-tetradecenol | B | p-(E-1-tetradec-4-enylamino)-phenylacetic acid |
| 2573 | Z-9-octadecen-1-o1 | B | p-(Z-1-octadec-9-enylamino)phenyl-acetic acid |
| 2574 | E-4-pentadecen-1-o1 | B | p-(E-1-pentadec-4-enylamino)-phenylacetic acid |
| 2575 | 3-chloro-2,4,4-tri-methyl-1-pentene Chem. Abst. 72, 111081h | A | p-[3-(2,4,4-tri-methyl)-pent-1-enylamino]phenyl-acetic acid |
| 2576 | 3-bromo-3-isoprop-yl-4-methyl-1-pent-ene Chem. Abst. 54, 4355a | A | p-[3-(3-isopro-pyl-4-methyl)-pent-1-enyl-ami- no]phen-ylacetic acid |
| 2577 | 4-bromo-2-heptene Chem. Abst. 70, 67482x | A | p-(4-hept-2-enyl-amino)phenylacetic acid |
| 2578 | 4-bromo-2,4-dimeth-yl-2-hexene | A | p-[4-(2,4-dimethyl-hex-2-enyl)amino]-phenylacetic acid |
| 2579 | 5-chloro-3,5-di-methyl-3-heptene Chem. Abst. 54, 1256e | A | p-[5-(3,5-dimethyl-hept-3-enyl)amino]-acid |
| 2580 | Z-1-hydroxy-2-hexa-decene Ref. B | B | p-(Z-1-hexadec-2--enylamino)phenyl-acetic acid |

| Example | Starting Material | Method | Product |
|---|---|---|---|
| 2581 | E-1-hydroxy-2-hexa decene Ref. B | B | p-(E-1-hexadec-2-enylamino)phenyl-acetic acid |
| 2582 | 1-bromo-4-methyl-3-heptene Chem. Abst. 71, 102020q | A | p-[1-(4-methylhept-3-enyl)amino]phenyl acetic acid |
| 2583 | 1-bromo-4-methyl-3-none Chem. Abst. 71, 101399h | A | p-[1-(4-methynon-3-enyl)amino]phenyl-acetic acid |
| 2584 | E-7-bromo-3-hept-ene Chem. Abst. 74, 99419f | A | p-(1-hept-4-enyl-amino)-phenylacetic acid |
| 2585 | 1-bromo-5,9-dimeth-yl-4-decene Chem. Abst. 51, 8699g | A | p-[1-(5,9-dimethyl-dec-4-enyl)amino]-phenylacetic acid |
| 2586 | 1-methanesulfonyl-oxy-4-tetradecene Ref. B. | B | p-(1-tetradec-4-enylamino)phenyl-acetic acid |
| 2587 | 1-methanesulfonyl-oxy-4-hexadecene Ref. B. | B | p-(1-hexadec-4-enylamino)phenyl-acetic acid |
| 2588 | 6-bromo-1-hexene Chem. Abst. 66, 2142j | A | p-(1-hex-5-enyl-amino)phenylacetic acid |
| 2589 | 6-bromo-2-methyl-1-hexene Chem. Abst. 75, 109624f | A | p-[1-(5-methylhex-5-enyl)amino]phenyl-acetic acid |
| 2590 | 6-chloro-1-heptene-Chem. Abst. 72, 31877g | A | p-)2-hept-6-enyl-amino)phenylacetic acid |
| 2591 | 6-bromo-2-methyl-2-heptene Chem. Abst. 54, 13166f | A | p-[2-(2,6-dimethyl-hept-5-enyl)amino]-phenylacetic acid |
| 2592 | 7-chloro-2-octene Chem. Abst. 75, 129245m | A | p-(2-oct-6-enyl-amino)-phenylacetic acid |

| Example | Starting Material | Method | Product |
|---|---|---|---|
| 2593 | E-1-chloro-4-nonene Chem. Abst. 67, 32294g | A | p-(E-1-non-5-enylamino)phenylacetic acid |
| 2594 | 7-bromo-1-heptene | A | p-(1-hept-6-enylamino)phenylacetic acid |
| 2595 | 7-chloro-1-octene Chem. Abst. 75, 29245m | A | p-(2-oct-7-enylamino)-phenylacetic acid |
| 2596 | 6-bromo-6-methyl-1-heptene Chem. Abst. 66, 94482w | A | p-[1-(2-methylhept-6-enyl)amino]phenylacetic acid |
| 2597 | 6-chloro-6-methyl-1-heptene Chem. Abst. 75, 129245 | A | p-[1-(6-methylhept-6-enyl)amino]phenylacetic acid |
| 2598 | E-8-bromo-2-octene Chem. Abst. 74, 99419f | A | p-(1-oct-6-enylamino)phenylacetic acid |
| 2599 | 8-bromo-2,6-dimethyl-2-octene Chem. Abst. 72, 90573c | A | p-[1-(3,7-dimethyloct-6-enyl)amino]-phenylacetic acid |
| 2600 | 11-bromo-5-undecene-Chem. Abst. 67, 73101b | A | p-(1-undec-6-enylamino)phenylacetic acid |
| 2601 | 8-bromo-1-octene Chem. Abst. 70, 10990g | A | p-(1-oct-7-enylamino)phenylacetic acid |
| 2602 | R-8-iodo-7-methyl-1-octene Chem. Abst. 74, 12573e | A | p-[R-(2-methyloct-7-enyl)amino]phenylacetic acid |
| 2603 | 1-chloro-7-tetradecene Chem. Abst. 54, 22461h | A | p-(1-tetradec-7-enylamino)phenylacetic acid |
| 2604 | 9-chloro-1-nonene Chem. Abst. 70, 114490k | A | p-(1-non-8-enylamino)phenylacetic acid |
| 2605 | 1-bromo-8-heptadecene Chem. Abst. 52, 249d | A | p-(1-heptadec-8-enylamino)phenylacetic acid |

| Example | Starting Material | Method | Product |
|---|---|---|---|
| 2606 | E-1-bromo-9-octadecene Chem. Abst. 70, 46779j | A | p-(E-1-octadec-9-enylamino)phenylacetic acid |
| 2607 | Z-1-bromo-9-octadecene Chem. Abst. 70, 46779j | A | p-(Z-1-octadec-9-enyl-amino)acid |
| 2608 | 11-chloro-1-undecene Chem. Abst. 66, P19046d | A | p-(1-undec-10-enylamino)phenylacetic acid |
| 2609 | 12-iodo-3,7,11-trimethyl 1-dodecene | A | p-[1-(2,6,10-trimethyldodec-11-enyl)amino]phenylacetic acid |
| 2610 | 13-bromo-1-tridecene Chem. Abst. 67, 43348v | A | p-(1-tridec-12-enylamino)phenylacetic acid |
| 2611 | 22-bromo-9-docosene Chem. Abst. 73, 44976j | A | p-[1-(2-methylhept-6-enyl)amino]-phenylacetic acid |
| 2612 | 1-hexadecene Ref. B. | B | p-(1-hexadec-15-enylamino)phenylacetic acid |
| 2613 | propargyl alcohol | B | p-(1-prop-2-ynylamino)phenylacetic acid |
| 2614 | 3-chloro-1-butyne | A | p-(2-but-3-ynylamino)phenylacetic acid |
| 2615 | 3-chloro-3-methylnonyne Chem. Abst. 55, 22090i | A | p-[3-(3-methylnon-1-ynyl)amino]phenylacetic acid |
| 2616 | 3-bromo-1-pentadecyne Chem. Abst. 53, 21638c | A | p-(3-pentadec-1-nylamino)phenylacetic acid |
| 2617 | 1-octyn-3-ol Chem. Abst. 66 85410n | B | p-(3-oct-1-ynylamino)phenylacetic acid |

| Example | Starting Material | Method | Product |
|---|---|---|---|
| 2618 | 3,7,11,15-tetra-methyl-1-hexadecyn-3-ol | B | p-[3-(3,7,11,15-tetramethylhexadec-1-ynyl)amino]-phenylacetic acid |
| 2619 | 2-butyn-1-ol | B | p-(1-but-2-ynyl-amino)phenylacetic acid |
| 2620 | 4-hexyn-3-ol | B | p-(3-hex-4-ynyl-amino)phenylacetic acid |
| 2621 | 2-methyl-3-pentyn-2-ol Chem. Abst. 69, 10496e | B | p-[2-(2-methylpent-3-ynyl)amino]phenyl-acetic acid |
| 2622 | 2-octyn-1-ol | B | p-(1-oct-3-ynyl-amino)phenylacetic acid |
| 2623 | 4-decyn-3-ol Chem. Abst. 69, P4630s | B | p-(3-dec-4-ynyl-amino)phenylacetic acid |
| 2624 | 1-bromo-2-do-decyne Chem. Abst. 28, 40345 | A | p-[1-dodec-2-ynyl-amino)phenylacetic acid |
| 2625 | 1-methanesulfonyl-oxy-2-pentadecyne J. Med. Chem 19, 946 (1977) | B | p-(1-pentadec-2-ynylamino)phenyl-acetic acid |
| 2626 | 3-butyne-1-ol Chem. Abst. 66, P2319a | B | p-(1-but-3-ynyl-amino)phenylacetic acid |
| 2627 | 1-undecyn-4-ol Chem. Abst. 70, 3219j | B | p-(4-undec-1-ynyl-amino)phenylacetic acid |
| 2628 | 2-methyl-4-pentyn-2-ol Chem. Abst. 68, 12200g | B | p-[2-(2-methylpent-4-ynyl)amino]-phenylacetic acid |
| 2629 | 4-pentyn-2-ol Chem. Abst. 64, 13537e | B | p-(2-pent-4-ynyl-amino)phenylacetic acid |

| Example | Starting Material | Method | Product |
|---|---|---|---|
| 2630 | 3-pentyl-1-ol | B | p-(1-hex-3-ynyl-amino)phenylacetic acid |
| 2631 | 4-hexyn-2-ol | B | p-(2-hex-4-ynyl-amino)phenylacetic acid |
| 2632 | 2-methyl-3-pentyn-1-ol Chem. Abst. 66, 115242k | B | p-[1-(2-methly-3-pent-3-ynyl)-amino]phenylacetic acid |
| 2633 | 2-(1-propynyl)-1-heptanol Chem. 66, 115242k | B | p-[2-(1-propynl-heptyl)amino]-phenylacetic acid |
| 2634 | 2-methyl-4-nonyn-2-ol Chem. Abst. 68, 104593r | B | p-[2-(2-methylnon-4-ynyl)amino]-phenylacetic acid |
| 2635 | 2-methyl-3-nonyn-1-ol Chem. Abst. 66, 115242k | B | p-[1-(2-methylnon-3-ynyl)amino]phenylacetic acid |
| 2636 | 3-nonyn-1-ol Chem Abst. 75 5165r | B | p-(1-non-3-ynyl-amino)phenylacetic acid |
| 2637 | 2-methyl-3-decyn-1-ol Chem. Abst. 66, 115242k | B | p-[1-(2-methlydec-3-ynyl)amino]-phenylacetic acid |
| 2638 | 5-chloro-1-pentyne | A | p-(1-pent-4-ynyl-amino)phenylacetic acid |
| 2639 | 4-hexyn-1-ol Chem. Abst. 74, 9800w | B | p-(1-hex-4-ynyl-amino)phenylacetic acid |
| 2640 | 1-chloro-4-nonyne | A | p-(1-non-4-ynyl-amino)phenylacetic acid |
| 2641 | 1-chloro-4-tri-decyne Chem. Abst. 32, 7426[3] | A | p-(1-pentadec-2-ynylamino)phenylacetic acid |

| Example | Starting Material | Method | Product |
|---|---|---|---|
| 2642 | 1-chloro-4-hexa-decyne | A | p-(1-hexadec-4-ynyl-amino)phenylacetic acid |
| 2643 | 5-hexyn-1-ol Chem. Abst. 74, 9800w | B | p-(1-hex-5-ynyl-amino)phenylacetic acid |
| 2644 | 6-octyn-2-ol Chem. Abst. 71, 60300y | B | p-[2-(2-methyloct-6-ynyl)amino]phenyl-acetic acid |
| 2645 | 1-iodo-5-decyne Chem. Abst. 51, 12817f | A | p-(1-dec-5-ynyl-amino)phenylacetic acid |
| 2646 | 5-tetradecyn-1-ol Chem. Abst. 71, 12053k | B | p-(1-tetradec-5-ynylamino)phenyl-acetic acid |
| 2647 | 5-octadecyn-1-ol Chem. Abst. 72, 42686v | B | p-(1-octadec-5-ynyl-amino)phenylacetic acid |
| 2648 | 6-octadecyn-1-ol Chem. Abst. 72, 42686v | B | p-(1-octadec-6-ynyl-amino)phenylacetic acid |
| 2649 | 10-chloro-3-decyne Chem. Abst. 67, 108147a | A | p-(1-dec-7-ynyl-aminophenylacetic acid |
| 2650 | 1-chloro-7-tetra-decyne Chem. Abst. 54, 2461e | A | p-(1-tetradec-7-ynylamino)phenyl-acetic acid |
| 2651 | 7-hexadecyn-1-ol Chem. Abst. 71, 19554w | B | p-(1-hexadec-7-ynyl-amino)phenylacetic acid |
| 2652 | 7-octadecyn-1-ol Chem. Abst. 67, 81784w | B | p-(1-octadec-7-ynyl-amino)phenylacetic acid |
| 2653 | 8-octadecyn-1-ol Chem. Abst. 72, | B | p-(1-octadec-8-ynyl-amino)phenylacetic acid |
| 2654 | 9-decyn-1-ol Chem. Abst. 67, 81787s | B | p-(1-octadec-9-ynyl-amino)phenylacetic acid |

| Example | Starting Material | Method | Product |
|---|---|---|---|
| 2655 | 10-octadecyn-1-ol Chem. Abst. 67, 81787s | B | p-(1-octadec-10-ynylamino)phenylacetic acid |
| 2656 | 4-nonyn-1-ol Chem. Abst. 67, | B | p-(1-non-4-ynylamino)phenylacetic acid |
| 2657 | 11-dodecyn-1-ol Chem. Abst. 68, 39015n | B | p-(1-dodec-11-ynylamino)phenylacetic acid |
| 2658 | 11-tetrdecyn-1-ol Chem. Abst. 75, 16792u | B | p-(1-tetradec-11-ynylamino)phenylacetic acid |
| 2659 | 11-tridecyn-1-ol Chem. Abst. 75, 1679u | B | p-(1-tridec-11-ynylamino)phenylacetic acid |
| 2660 | 16-bromo-5-hexadecyne Chem. Abst. 68, 39015n | A | p-(1-hexadec-11-ynylamino)phenylacetic acid |
| 2661 | 12-octadecyn-1-ol Chem. Abst. 68, 39015n | B | p-(1-octadec-12-ynylamino)-phenylacetic acid |
| 2662 | 12-octadecyn-1-ol Chem. Abst. 72, 42686v | B | p-(1-octadec-12-ynylamino)-phenylacetic acid |
| 2663 | 13-tetradecyn-1-ol Chem. Abst. 68, 39015n | B | p-(1-tetradec-13-ynylamino)phenylacetic acid |
| 2664 | 14-pentadecyn-1-ol Chem. Abst. 68, 39015n | B | p-(1-pentadec-14-ynylamino)phenylacetic acid |
| 2665 | 5,7-octadien-4-ol Chem. Abst. 72, 42686v | B | p-(4-octa-5,7-dienylamino)phenylacetic acid |
| 2666 | 4-ethyl-3,5-hexadecadien-2-ol Chem. Abst. 70 20116r | B | p-[2-(4-ethylhexa-3-5-dienyl)amino]-phenylacetic acid |
| 2667 | 2-methyl-4,6-heptadien-3-ol Chem. Abst. 71, 112534z | B | p-[3-(2-methylhepta-4,6-dienyl)amino-phenylacetic acid |

| Example | Starting Material | Method | Product |
|---|---|---|---|
| 2668 | E-2,4,6-trimethyl-3,5-heptadien-2-ol | B | p-[E-2(2,4,6-hepta-3,5-dienyl)amino]-phenylacetic acid |
| 2669 | 4,6-octadien-3-ol Chem. Abst. 69, 58770s | B | p-(1-hexa-2,4-dienylamino)phenylacetic acid |
| 2670 | 2,4-hexadien-1-ol Chem. Abst. 67, 81787s | B | p-[1-hexa-2,4-dienylamino)phenylacetic acid |
| 2671 | 6-isopropyl-7,7-dimethyl-3,5-octadien-2-ol Chem. Abst. 75, 36362g | B | p-[2-6-isopropyl-7,7-dimethylocta-3,5-dienyl)amino]phenylacetic acid |
| 2672 | 3,5,7-trimethyl-4,6-nonadien-3-ol Chem. Abst. 74, | B | p-[3-(3,5,7-trimethylnona-4,6-dienyl)amino]-phenylacetic acid |
| 2673 | 2,6-dimethyl-3,5-octadien-2-ol Chem. Abst. 68, 114750d | B | p-[2-(2,6-dimethylocta-3,5-dienyl)-amino]phenylacetic acid |
| 2674 | E,E-6-ethyl-4,6-decadien-3-ol Chem. Abst. 73, 130585n | B | p-[E,E-3-(6-ethyldeca-4,6-dienyl)-amino]phenylacetic acid |
| 2675 | 5-ethyl-3,5-nonadien-2-ol | B | p-[2-(5-ethylnona-3,5-dienyl)amino]-phenylacetic acid |
| 2676 | E,E-2,4-octadien-1-ol Chem. Abst. 67, 104946n | B | p-(E,E-1-octa-2-4-dienylamino)-phenylacetic acid |
| 2677 | E,Z-2,4-nonadien-1-ol Chem. Abst. 67, 99692w | B | p-(E,Z-1-nona-2-4-dienylamino)-phenylacetic acid |
| 2678 | E,E-2,4-decadien-1-ol Chem. Abst. 67, 104946n | B | p-(E,E-1-deca-2-4-dienylamino)-phenylacetic acid |
| 2679 | E,Z-2,4-decadien-1-ol Chem. Abst. 67, 99692v | B | p-E,Z-1-deca-2-4-dienylamino)-phenylacetic acid |

| Example | Starting Material | Method | Product |
|---|---|---|---|
| 2680 | E-2,5-hexadien-1-ol Chem. Abst. 73, P55709a | B | p-(E-1-hexa-2,5-dienylamino)phenylacetic acid |
| 2681 | Z-2,5-hexadienyl-1-ol Chem. Abst. 73, PS5709a | B | p-(Z-1-hexa-2,5-dienylamino)phenylacetic acid |
| 2682 | E(+)-4-ethyl-2,5-dimethyl-2,5-hexadien-1-ol Chem. Abst. 71, 70152m | B | p-(E(+)-1-(2,5-dimethylhexa-2,5-dienyl)amino]phenylacetic acid |
| 2683 | E-2,5,5-trimethyl-3,6-heptadien-1-ol Chem. Abst. 74, 52962n | B | p-[E-2-(2,5,5-trimethylhepta-3,6-dienylamino]phenylacetic acid |
| 2684 | Z-2,5,5-trimethyl-3,6-heptadien-1-ol Chem. Abst 74, 52962n | B | p-[Z-2-(2,5,5-triylhepta-3,6-dienylamino]phenylacetic acid |
| 2685 | Z,E-3,7-dimethyl-2,5-octadien-1-ol Chem. Abst. 71, 6156a | B | p-[Z-E-1-(3,7-dimethylocta-2,5-dienyl)amino]phenylacetic acid |
| 2686 | E-2,6-dimethyl-2,6-heptadien-1-ol Chem. Abst. 72, 32036u | B | p-[E-1-(2,6-dimethylhept-2,6-dienyl)-amino]phenylacetic acid |
| 2687 | E,E-2,6-octadien-1-ol | B | p-(E,E-1-octa-2,6-dienylamino)phenylacetic acid |
| 2688 | E,E-2,6-dimethyl-2,6-octadien-1-ol Chem. Abst. 71, 22204n | B | p-[E,E-1-(2,6-dimethylocta-2,6-dienyl)amino]phenylacetic acid |
| 2689 | Z,E-2,6-dimetyl-octadien-1-ol Chem. Abst. 71, 22204n | B | p-(Z,E-1-(2,6-dimethylocta-2,6-dienylamino)phenylacetic acid |
| 1290 | E,Z-2,6-nonadien-1-ol Chem. Abst. 72, 24504e | B | p-(E,Z-1-nona-2,6-dienylamino)phenylacetic acid |

| Example | Starting Material | Method | Product |
|---|---|---|---|
| 2691 | E,Z-3,ethyl-7-methyl-2,6-nonadien-1-ol Chem. Abst. 75, 63019g | B | p-[E,Z-1-(3-ethyl-7-methylnona-2,6-dienyl)amino]phenylacetic acid |
| 2692 | 3,7,11,11-tetramethyl-2,6-dodecadien-1-ol Chem. Abst. 75, P117969n | B | p-[1-(3,7,11,11-tetramethyldodeca-2,6-dienyl)amino]-phenylacetic acid |
| 2693 | E,E-3,7,11-trimethyl-2,6-dodecadien-1-ol Chem. Abst. 69, 978z | B | p-[E,E-1-(3,7,11-trimethyldodeca-2,6-dienyl)amino]-phenylacetic acid |
| 2694 | E-Z-3,7,11-trimethyl-2,6-dodecadien-1-ol Chem. Abst. 69, 978g | B | p-(E,Z-1-(3,7,11-trimethyldodeca-2,6-dienyl)amino]-phenylacetic acid |
| 2695 | 2,7-octadien-1-ol Chem. Abst. 74, P41892p | B | p-(1-octa-2,7-dienylamino)phenylacetic acid |
| 2696 | E-3,7-dimethyl-2,7-octadien-1-ol Chem. Abst. 68, 114750d | B | p-[E-1-(3,7-dimethylocta-2,7-dienyl)amino]phenylacetic acid |
| 2697 | Z,3,7-dimethyl-2,7-octadien-1-ol Chem. Abst. 68, 114750d | B | p-[Z-1-(3,7-dimethylocta-2,7-dienyl)amino]phenylacetic acid |
| 2698 | 3,4,8-trimethyl-2,7-nonadien-1-ol Chem. Abst. 68, 9184c | B | p-[1-(3,4,8-trimethylnona-2,7-dienyl)amino]phenylacetic acid |
| 2699 | 3,4,8-trimethyl-2,8-nonadien-1-ol Chem. Abst. 68, 29184c | B | p-[1-(3,4,8-trimethylnona-2,8-dienyl)amino]phenylacetic acid |
| 2700 | 2,9-decadien-1-ol Chem. Abst. 68, 68373h | B | p-(1-deca-2,9-dienylamino)phenylacetic acid |

| Example | Starting Material | Method | Product |
|---|---|---|---|
| 2701 | E,3,7,11-trimethyl-2,10dodecadien-1-ol Chem. Abst. 69, 978z | B | p-[E-1-(3,7,11-trimethyldodeca-2,10-dienylamino]-phenylacetic acid |
| 2702 | Z-3,7,11-trimethyl-2,10-dodecadien-1-ol | B | p-[Z-1-(3,7,11-trimethyldodeca-2,10-dienyl)amino]-phenylacetic acid |
| 2703 | E,E-4-methyl-3,5-heptadien-1-ol Chem. Abst. 66, 104730s | B | p-[E,E-1-(4-methylhepta-3,5-dienyl)-amino]phenylacetic acid |
| 2704 | E,Z-4-methyl-3,5-heptadien-1-ol Chem. Abst. 66, 104730s | B | p-[E,Z-1-(4-methylhepta-3,5-dienyl)-amino]phenylacetic acid |
| 2705 | Z-E-4-methyl-3,5-heptadien-1-ol Chem. Abst. 66, 104730s | B | p-[Z,E-1-(4-methylhepta-3,5-dienyl)-amino] phenylacetic acid |
| 2706 | Z,Z-4-methyl-3,5-heptadien-1-ol Chem. Abst. 66, 104730s | B | p-[Z,Z-1-(4-methylhepta-3,5-dienyl)-amino]phenylacetic acid |
| 2707 | E-4,6-dimethyl-3,5-heptadien-1-ol Chem. Abst. 66, 104730s | B | p-[E-1-(4,6-dimethylhepta-3,5-dienyl)amino]-phenylacetic acid |
| 2708 | Z-4,6-dimethyl-3,5-heptadien-1-ol Chem. Abst. 66, 104730s | B | p-[Z-1-(4,6-dimethylhepta-3,5-dienylamino]-phenylacetic acid |
| 2709 | E,Z-2,6-dimethyl-4,6-octadien-2-ol Chem. Abst. 68, 114750d | B | p-[E,Z-2-(2,6-dimethylocta-4,6-dienyl)amino]phenylacetic acid |
| 2710 | 1-hydroxy-3,7,11-trimethyl-2,6,10-dodecatriene | B | p-[1-(3,7,11-trimethyldodeca-2,6,10-trienyl)amino]-phenylacetic acid |

| Example | Starting Material | Method | Product |
|---|---|---|---|
| 2711 | 5-methyl-3,5-octa-dien-1-ol Chem. Abst. 69, 18519k | B | p-[1-(5-methylocta-3,5-dienyl)amino]-phenylacetic acid |
| 2712 | E-4-methyl-3,6-heptadien-1-ol Chem. Abst. 66, 104730s | B | p-[E-1-(4-methyl-hepta-3,6-dienyl)-amino]phenylacetic acid |
| 2713 | Z-4-methyl-3,6-heptadien-1-ol Chem. Abst. 66, 104730s | B | p-[Z-1-(4-methyl-hepta-3,6-dienyl)-amino]phenylacetic acid |
| 2714 | E(+)-2,6-dimethyl-4,7-octadien-2-ol Chem. Abst. 75, 49345d | B | p-[E(+)-2-(2,6-di-methylocta-4,7-di-enyl)amino]phenyl-acetic acid |
| 2715 | Z(+)-2,6-dimethyl-4,7-octadien-2-ol Chem. Abst. 75, 49345d | B | p-[Z(+)-2-(2,6-di-methylocata-4,7-di-enyl)phenylacetic acid |
| 2716 | E-3,7-dimethyl-3,6-octadien-1-ol Chem. Abst. 67, 64554z | B | p-[E-1-(3,7-di-methylocta-3,6-di-enyl)amino]phenyl-acetic acid |
| 2717 | E-3,7 dimethyl-3,6-octadien-1-ol | B | p-[Z-1-(3,7-di-methylocta-3,6-di-enyl)amino]phenyl-acetic acid |
| 2718 | Z-3-methyl-3,7-octadien-1-ol Chem. Abst. 69, 65681x | B | p-[E-1-(3-methyl-octa-3,7-dienyl)-amino]phenylacetic acid |
| 2719 | Z-3-methyl-3,7-octadien-1-ol Chem. Abst. 69, 26681x | B | p-[Z-1-(3-methyl-octa-3,7-dienyl)-amino]phenylacetic acid |
| 2720 | E-2-methyl-4,8-nona-dien-1-ol Chem. Abst. 73, 87253p | B | p-[Z-1-(4,6-di-methylhepta-3,5-di-enyl)amino]phenyl-acetic acid |

| Example | Starting Material | Method | Product |
|---|---|---|---|
| 2721 | E-3,7-dimethyl-3,7-octadien-1-ol Chem. Abst. 69, 26681x | B | p-[E-1-(3,7-dimethylocta-3,7-dienylamino]phenylacetic acid |
| 2722 | E-8-methyl-7-methylene-3-nonen-1-ol Chem. Abst. 70, 29098u | B | p-[E-1-(8-methyl-7-methylenenon-3-enyl)amino]phenylacetic acid |
| 2723 | 2,4,9-trimethyl-4,8-decadien-1-ol Chem. Abst. 74, 99342s | B | p-[2-(2,4,9-trimethyldeca-4,8-dienyl)amino]phenylacetic acid |
| 2724 | E-2-methyl-4,9-decadien-1-ol Chem. Abst. 73, 87254g | B | p-[E-2-(2-methyldeca-4,9-dienyl)-amino]phenylacetic acid |
| 2725 | 2,6-dimethyl-5,7-ocatadien-2-ol Chem. Abst. 72, 37616t | B | p-[2-(2,6-dimethylocta-5,7-dienyl)-amino]phenylacetic acid |
| 2726 | E-3,7-dimethyl-4,6-octadien-1-ol Chem. Abst. 72, 121291r | B | p-[E-1-(3,7-dimethylocta-4,6-dienyl)-amino]phenylacetic acid |
| 2727 | Z-3,7-dimethyl-4,6-octadien-1-ol Chem. Abst. 72, 121291r | B | p-[Z-1-(3,7-dimethylocta-4,6-dienyl)-amino]phenylacetic acid |
| 2728 | E-4,7-octadien-1-ol Chem. Abst. 66, 28610k | B | p-[E-1-(octa-4,7-dienyl)amino]phenylacetic acid |
| 2729 | 5,8-nonadien-2-ol Chem. Abst. 68 28610k | B | p-[2-(nona-5,8-dienyl)amino]phenylacetic acid |
| 2730 | E-7-methyl-4,7-octadien-1-ol Chem. Abst. 66, 28610k | B | p-[E-1-(7-methylocta-4,7-dienyl)-amino]phenylacetic acid |

| Example | Starting Material | Method | Product |
|---|---|---|---|
| 2731 | E-8-methyl-5,8-nonadien-2-ol Chem. Abst. 66, 28610k | B | p-[E-2-(8 methytl-nona-5,8-dienyl)-amino]phenylacetic acid |
| 2732 | E-6,10-dimethyl-5,9-undecadien-2-ol Chem. Abst. 73, 1331152f | B | p-[E-2-(6,10-dimethylundeca-5,9-dienyl)amino]phenylacetic acid |
| 2733 | 5,9,13-trimethyl-8,13-tetradecadien-2-ol Chem. Abst. 70, 28303v | B | p-[6-(5,9,13-trimethyltetradeca-8,12-dienyl)amino]-phenylacetic acid |
| 2734 | E-3,7,11-trimethyl-6,10-dodecadien-3-ol Chem. Abst. 69, 8333f | B | p-[E-3-(3,7,11-trimethyldodeca-6,10-dienyl)amino]-phenylacetic acid |
| 2735 | Z-3,7,11-trimethyl-6,10-dodecadien-3-ol Chem. Abst. 69, 8333f | B | p-[Z-3-(3,7,11-trimethyldodeca-6,10-dienyl)amino]-phenylacetic acid |
| 2736 | 5,9-dimethyl-4,8-decadien-1-ol Chem. Abst. 74, 112233n | B | p-[1-(5,9-dimethyldeca-4,8-dienyl)-amino]phenylacetic acid |
| 2737 | 15-methanesulfonyloxy-1,11-pentadecadiene Ref. B | B | p-[1-pentadeca-4,14-dienylamino)-phenylacetic acid |
| 2738 | 5,7-octadien-1-ol Chem. Abst. 68, 68503a | B | p-[1-octa-5,7-dienylamino)phenylacetic acid |
| 2739 | E-3,7-dimethyl-5,7-octadien-1-ol Chem. Abst. 72, P90672j | B | p-[E-1-(3,7-dimethocta-5,7-dienyl)-amino]phenylacetic acid |
| 2740 | 6,10-dimethyl-5,9-undecadien-1-ol Chem. Abst. 71, 50248y | B | p-[1-(6,10-dimethylundeca-5,9-dienylamino]phenylacetic acid |

| Example | Starting Material | Method | Product |
|---|---|---|---|
| 2741 | 2,6,10-trimethyl-5,9-undecadien-1-ol Chem. Abst. 71, 50243t | B | p-[1-(2,6,10-trimethylundeca-5,9-dienyl)amino]phenylacetic acid |
| 2742 | 10-propyl-5,9-tridecadien-1-ol Chem. Abst. 68, 39028u | B | p-[1-(10-propyltrideca-5,9-dienyl)amino]phenylacetic acid |
| 2743 | 5,13-tetradecadien-2-ol Chem. Abst. 66, 35672k | B | p-[1-tetradeca-5,13-dienylamino]phenylacetic acid |
| 2744 | E-3,7,11-trimethyl-6,10-dedecadien-1-ol Chem. Abst. 69, 978z | B | p-[1-(3,7,11-trimethyldodeca-6,-10-dienyl)amino]phenylacetic acid |
| 2745 | E-6,10,14-trimethyl-9,13-pendadecadien-2-ol Chem. Abst. 74, 53992j | B | p-[E-2-(6,10,14-trimethylylpentadeca-9,13-dienyl)amino]phenylacetic acid |
| 2746 | Z-6,10,14-trimethyl-9,13-pentadecadien-2-ol Chem. Abst. 74, 53992j | B | p-[Z-2-(6,10,14-trimethylylpentadeca-9,13-dienyl)amino]phenylacetic acid |
| 2747 | Z,Z-9,12-octadecadien-2-ol Chem. Abst. 68, 92804v | B | p-[1-octadeca-9,12-dienylamino]phenylacetic acid |
| 2748 | E,Z-10,12-hexadecadien-1-ol Chem. Abst. 66 106133y | B | p-[E,Z-1-hexadeca-10,12-dienylamino)phenylacetic acid |
| 2749 | Z,E-10,12-hexadecadien-1-ol Chem. Abst. 66, P7956f | B | p-[Z,E-1-hexadeca-10,12-dienylamino)phenylacetic acid |
| 2750 | 2-methyl-2,3-butadien-1-ol Chem. Abst. 71, 30229n | B | p-[1-(2-methylbuta-2,3-dienyl)amino]phenylacetic acid |
| 2751 | 2-ethyl-2,3-buta-2,4-hexadien-2-ol Chem. Abst. 67, 535673e | B | p-[1-(2-ethylbuta-2,3-dienyl)amino]phenylacetic acid |

| Example | Starting Material | Method | Product |
|---|---|---|---|
| 2752 | 2,3,5-trimethyl-3,4-hexadien-2-ol Chem. Abst. 72, 131953x | B | p-[2-(2,3,5-trimethylhexa-3,4-dienyl)amino]phenylacetic acid |
| 2753 | 3-isopropyl-2,4-dimethyl-4,5-hexadien-3-ol | B | p-[3-(2,4-dimethylhexa-4,5-dienyl)-amino]phenylacetic acid |
| 2754 | 2,5-dimethyl-3,4-hexadien-2-ol Chem. Abst. 68, 39152e | B | p-[2-(2,5-dimethylhexa-3,4-dienyl)-amino]phenylacetic acid |
| 2755 | 3,5-dimethyl-3,4-heptadien-2-ol Chem. Abst. 72, 131953x | B | p-[2-(3,5-dimethlylhepta-3,4-dienyl)-amino]phenylacetic acid |
| 2756 | 2-methyl-3,4-heptadien-2-ol Chem. Abst. 71, 38219g | B | p-[2-(2-methylhepta-3,4-dienyl)amino]-phenylacetic acid |
| 2757 | 2,3,5-trimethyl-3,4-heptadien-2-ol Chem. Abst. 72, 131953x | B | p-[2-(2,3,5-trimethylhepta-3,4-dienyl)amino]phenylacetic acid |
| 2758 | 3-t-butyl-2-methyl-3,4-octadien-2-ol Chem. Abst. 66, 75593s | B | p-[2-(3-t-butyl-2-methylocta-3,4-dientyl)amino]phenylacetic acid |
| 2759 | 2-ethyl-2,3-heptadien-1-ol Chem. Abst. 75, 63063s | B | p-[1-(2-ethylhepta-2,3-dienyl)amino]-phenylacetic acid |
| 2760 | 2-methyl-3,4-octadien-2-ol Chem. Abst. 75, 140175g | B | p-[1-(2-methylocta-3,4-dienyl)amino]-phenylacetic acid |
| 2761 | 3-methyl-3,4-octadien-2-ol Chem. Abst. 66, 75593s | B | p-[2-3-(3-methylocta-3,4-dienyl)-amino]phenylacetic acid |

| Example | Starting Material | Method | Product |
|---|---|---|---|
| 2762 | 5,6-decadien-4-ol Chem. Abst. 75, 76898t | B | p-(4-deca-5,6-dienylamino)phenylacetic acid |
| 2763 | 2,3-dimethyl-3,4-octadien-2-ol Chem. Abst. 66, 75593s | B | p-[2-(2,3-dimethylocta-3,4-dienyl)amino]phenylacetic acid |
| 2764 | 4-ethyl-4,5-nonadien-3-ol Chem. Abst. 75, 63063s | B | p-[3-(4-ethylnona-4,5-dienyl)amino]-phenylacetic acid |
| 2765 | 2-methyl-3-propyl-3,4-octadien-2-ol Chem. Abst. 66, 65592v | B | p-[2-(2-methyl-3-propylocta-3,4-dienyl)amino]phenylacetic acid |
| 2766 | 2-methyl-3,4-nonadien-2-ol Chem. Abst. 75, 140173g | B | p-[2-(2-methylnona-3,4-dienyl)amino]-phenylacetic acid |
| 2767 | 2,8-dimethyl-3,4-nonadien-2-ol Chem. Abst. 71, 38219g | B | p-[2-(2,8-dimethylnona-3,4-dienyl)-amino]phenylacetic acid |
| 2768 | 2-methyl-3,4-decadien-2-ol Chem. Abst. 75, 140173g | B | p-[2-(2-methyldeca-3,4-dienyl)amino]-phenylacetic acid |
| 2769 | 2,9-dimethyl-3,4-decadien-2-ol | B | p-[2-(2,9-dimethyldeca-3,4-dienyl)-amino]phenylacetic acid |
| 2770 | 2-methyl-3,4-dodecadien-2-ol Chem. Abst. 71, 38219g | B | p-[2-(2-methyldodeca-3,4-dienyl)-amino]phenylacetic acid |
| 2771 | 2-methyl-3,4-tridecadien-1-ol Chem. Abst. 71, 38219g | B | p-[2-(2-methyltrideca-3,4-dienlyl)-amino]phenylacetic acid |

| Example | Starting Material | Method | Product |
|---|---|---|---|
| 2772 | 4,5-hexadien-2-ol Chem. Abst. 75, 5152j | B | p-(2-hexa-4,5-dienylamino)phenylacetic acid |
| 2773 | 2-methyl-5,6-heptadien-3-ol Chem. Abst. 75, 5152j | B | p-[2-(2-methylhepta-5,6-dienyl)amino]-phenylacetic acid |
| 2774 | 3,3-dimethyl-4,5-hexadien-2-ol Chem. Abst. 69, 86256x | B | p-[2-(3,3-dimethyl-hexa-4,5-dienyl)-amino]phenylacetic acid |
| 2775 | 2,5-dimethyl-5,6-heptadien-3-ol Chem. Abst. 68, 86855w | B | p-[2-(2,5,dimethyl-hepta-5,6-dienyl)-amino]phenylacetic acid |
| 2776 | 2,2,5-trimethyl-3,4-hexadien-1-ol Chem. Abst. 71, 29767g | B | p-[1-(2,2,5-tri-methylhexa-3,4-dienyl)amino]phenyl-acetic acid |
| 2777 | (+) 2,2-dimethyl-3,4-hexadien-1-ol Chem. Abst. 68, 58831s | B | p-[1-(2,2-dimethyl-hexa-3,4-dienyl)-amino]phenylacetic acid |
| 2778 | 3,4-hexadien-1-ol Chem. Abst. 66, 54943r | B | p-[1-hexa-3,4-dienylamino)phenyl-acetic acid |
| 2779 | 2,2,3,5-tetramethyl-3,4-hexadien-1-ol Chem. Abst. 71, 29767g | B | p-[1-(2,2,3,5-tetra-methylhexa-3,4-dienyl)amino]phenyl-acetic acid |
| 2780 | 3,3,6-trimethyl-4,5-octadien-2-ol Chem. Abst. 69, 86256x | B | p-[2-(3,3,6-trimeth-ylocta-4,5-dienyl)-amino]phenylacetic acid |
| 2781 | 2,5-dimethyl-5,6-heptadien-2-ol Chem. Abst. 69, 86256x | B | p-[2-(2,5-dimethyl-hepta-5,6-dienyl)-amino]phenylacetic acid |

| Example | Starting Material | Method | Product |
| --- | --- | --- | --- |
| 2782 | 4-methyl-4-penten-2-yn-1-ol | B | p-[1-(4-methylpent-4-en-2-ynyl)amino]-phenylacetic acid |
| 2783 | 2-methyl-2-penten 2-yn-1-ol | B | p-[1-(2-methylpent-2-en-4-ynyl)amino]-phenylacetic acid |
| 2784 | 5-hexen-3-yn-2-ol | B | p-[2-hex-5-en-3-ynylamino)phenyl-acetic acid |
| 2785 | 7-octen-5-yn-4-ol Chem. Abst. 67, 113544g | B | p-[4-oct-7-en-5-ynylamino)phenyl-acetic acid |
| 2786 | 5-methyl-5-hexen-3-yn-2-ol | B | p-[2-(5-methylhex-5-en-3-ynyl)amino]-phenylacetic acid |
| 2787 | 2,5-dimethyl-1-nonen-3-yn-5-ol Chem. Abst. 69, 2433s | B | p-[5-(2,5-dimethyl-non-1-en-3-ynyl)-amino]phenylacetic acid |
| 2788 | E-3-decen-1-yn-5-ol Chem. Abst. 75, 35042r | B | p-(5-dec-3-en-1-ynylamino]phenyl-acetic acid |
| 2789 | E-3-dodecen-1-yn-5-ol Chem. Abst. 75, 35042r | B | p-(5-dodec-3-en-1-ynylamino)phenyl-acetic acid |
| 2790 | 2-methyl-5-hexen-3-yn-2-ol Chem. Abst. 66, 75760v | B | p-[2-(2-methylhex-5-en-3-ynyl)amino]-phenylacetic acid |
| 2791 | 3-methyl-6-hepten-4-yn-3-ol Chem. Abst. 67, 113544g | B | p-[3-(3-methylhept-6-en-4-ynyl)amino]-phenylacetic acid |
| 2792 | 5-methyl-1-nonen-3-yn-5-ol Chem. Abst. 67, 43345s | B | p-[5-(5-methylnon-1-en-3-ynyl)amino]-phenylacetic acid |
| 2793 | 3-ethyl-6-hepten-4-yn-3-ol Chem. Abst. 71, 112202g | B | p-[3-(3-ethylhept-6-en-4-ynyl)amino]-phenylacetic acid |

| Example | Starting Material | Method | Product |
|---|---|---|---|
| 2794 | 5-ethyl-1-nonen-3-yn-5-ol Chem. Abst. 72, 31124j | B | p-[5-(5-ethvlnon-1-en-3-ynyl)amino]-phenylacetic acid |
| 2795 | 2,5-dimethyl-5-hexen-3-yn-2-ol Chem. Abst. 71, 112202g | B | p-[2-(2,5-dimethyl-hex-5-en-3-ynyl)-amino]phenylacetic acid |
| 2796 | 3,6-dimethyl-6-hepten-4-yn-3-ol Chem. Abst. 69, 96881m | B | p-[2-(2,5-dimethyl-hex-5-en-3-ynyl)-amino]phenylacetic acid |
| 2797 | 3-ethyl-6-methyl-6-hepten-4-yn-3-ol Chem. Abst. 71, 1122026 | B | p-[3-(3-ethyl-6-methylhept-6-en-4-ynylamino]phenyl-acetic acid |
| 2798 | 5-ethyl-2-methyl-3-yn-5-ol Chem. Abst. 69, 2433s | B | p-[5-(5-ethyl-2-methyl-n-1-en-3-ynyl)amino]-phenylacetic acid |
| 2799 | 2-hexen-4-yn-1-ol Chem. Abst. 68, 77868a | B | p-(1-(hex-2-en-4-ynylamino)phenyl-acetic acid |
| 2800 | 4-methyl-4-hexen-2-yn-1-ol Chem. Abst. 66, 11524r | B | p-[1-(4-methylhex-4-en-2-ynyl)amino]-phenylacetic acid |
| 2801 | 5-hexen-2-yn-1-ol Chem. Abst. 71, 80580t | B | p-(1-hex-5-en-2-ynylamino)phenyl-acetic acid |
| 2802 | 5-methyl-5-hexen-2-yn-1-ol Chem. Abst. 70, 28152v | B | p-[1-(5-methylhex-5-en-2-ynyl)amino]-phenylacetic acid |
| 2803 | 5-hexen-3-yn-1-ol Chem. Abst. 73, 109833g | B | p-(1-hex-5-en-3-ynylamino)phenyl-acetic acid |
| 2804 | E-4-methyl-5-yn-1-ol Chem. Abst. 66, 104730s | B | p-[E-1-(4-methyl-hept-3-en-5-ynyl)-amino]phenylacetic acid |

| Example | Starting Material | Method | Product |
|---|---|---|---|
| 2805 | Z-4-methyl-5-yn-1-ol Chem. Abst. 66, 104730s | B | p-[Z-1-(4-methylhept-3-en-5-ynyl)-amino]phenylacetic acid |
| 2806 | 3-hexen-5-yn-2-ol Chem. Abst. 75, 5152j | B | p-(2-hex-3-en-5-ynylamino)phenylacetic acid |
| 2807 | 6-methyl-6-hepten-4-yn-2-ol | B | p-[2-(6-methylhept-6-en-4-ynyl)amino]-phenylacetic acid |
| 2808 | 7-methyl-7-octen-5-yn-3-ol | B | p-[3-(7-methyloct-7-en-5-ynyl)amino]-phenylacetic acid |
| 2809 | 2,5-dimethyl-5-hexen-3-yn-2-ol | B | p-[2-(2-dimethylhex-5-en-3-ynyl)amino]-phenylacetic acid |
| 2810 | 3-methyl-6-octen-4-yn-3-ol Chem. Abst. 74, 41795j | B | p-[3-(3-methyloct-6-en-4-ynyl)amino]-phenylacetic acid |
| 2811 | 5-methyl-9-decen-6-yn-5-ol Chem. Abst. 73, 125271c | B | p-[5-(5-methyldec-9-en-6-ynyl)amino]-phenylacetic acid |
| 2812 | 2,5,5,6-tetramethyl-6-hepten-3-yn-2-ol Chem. Abst. 70, 10801w | B | p-[2-(2,5,5,6-tetramethylhept-6-en-3-ynyl)amino]-phenylacetic acid |
| 2813 | 3-ethyl-7-octen-4-yn-3-ol Chem. Abst. 73, 3762t | B | p-[3-(3-ethyloct-7-en-4-ynyl)amino]-phenylacetic acid |
| 2814 | 3-methyl-7-octen-4-yn-3-ol Chem. Abst. 73, 3762t | B | p-[3-(3-methyloct-7-en-4-ynyl)amino]-phenylacetic acid |
| 2815 | 2,6-dimethyl-6-hepten-3-yn-2-ol Chem. Abst. 73, 55677p | B | p-[2-(2,6-dimethylhept-6-en-3-ynyl)-amino]phenylacetic acid |
| 2816 | 3,6-diethyl-6-octen-4-yn-3-ol Chem. Abst. 68, 104850x | B | p-[3-(3,6-diethyloct-6-en-4-ynyl)-amino]phenylacetic acid |

| Example | Starting Material | Method | Product |
|---|---|---|---|
| 2817 | 6-ethyl-6-octen-4-yn-2-ol Chem. Abst. 73, 19632z | B | p-[2-(6-ethyloct-6-en-4-ynyl)amino]-phenylacetic acid |
| 2818 | 6-methyl-6-hepten-2-yn-1-ol Chem. Abst. 72, 3587u | B | p-[1-6-methylhept-6-en-2-ynyl)amino]-phenylacetic acid |
| 2819 | E-4-methyl-3-hept-en-6-yn-1-ol Chem. Abst. 66, 104730s | B | p-[E-1-(4-methyl-hept-3-en-4-ynyl)-amino]phenylacetic acid |
| 2820 | Z-4-methyl-3-hept-en-5-yn-1-ol Chem. Abst. 66, 104730s | B | p-[Z-1-(4-methyl-hept-3-en-6-ynyl)-amino]phenylacetic acid |
| 2821 | E-2-octen-6-yn-1-ol Chem. Abst. 75, 63019g | B | p-(E-1-oct-2-en-6-ynylamino)-phenylacetic acid |
| 2822 | Z-7-methyl-6-non-en-2-yn-1-ol Chem. Abst. 70, 78718j | B | p-[Z-1-(7-methyl-non-6-en-2-ynyl)-amino]phenylacetic acid |
| 2823 | 8-methyl-7-methyl-ene-3-nonyn-1-ol Chem. Abst. 66, 29098u | B | p-[1-(8-methyl-1-methylenenon-3-ynyl)amino]phenyl-acetic acid |
| 2824 | E-2-decen-4-yn-1-ol Chem. Abst. 67, 99692v | B | p-(1-dec-2-en-4-ynylamino)phenyl-acetic acid |
| 2825 | 4-methyl-4-decen-8-yn-1-ol Chem. Abst. 75, 110510x | B | p-[1-(4-methyldec-4-en-8-ynyl)amino]-phenylacetic acid |
| 2826 | E-2-undecen-4-yn-1-ol Chem. Abst. 66, 28594h | B | p-(1-undec-2-en-4-ynylamino)phenyl-acetic acid |
| 2827 | 6-methyl-5-undecen-2-yn-1-ol Chem. Abst. 71, P101399h | B | p-[1-(6-methylundec-5-en-2-ynyl)amino]-phenylacetic acid |
| 2828 | E-5-tetradecen-3-yn-1-ol Chem. Abst. 73, 87370z | B | p-(E-1-tetradec-5-en-3-ynylamino)-phenylacetic acid |

| Example | Starting Material | Method | Product |
|---|---|---|---|
| 2829 | Z-5-tetradecen-3-yn-1-ol Chem. Abst. 73, 87370z | B | p-(Z-1-tetradec-5-en-3-ynylamino)-phenylacetic acid |
| 2830 | 10-propyl-9-tridec-en-5-yn-1-ol Chem. Abst. 72, 12017k | B | p-[1-(10-propyltri-dec-9-en-5-ynyl)-amino]phenylacetic acid |
| 2831 | 17-octadecen-14-yn-1-ol Chem. Abst. 68, 39015n | B | p-(1-octadec-17-en-14-ynylamino)phenyl-acetic acid |
| 2832 | 2,4-dimethyl-1,4-hexadien-3-ol Chem. Abst. 74, 87269u | B | p-[3-(2,4-dimethyl-hexa-1,4-dienyl)-amino]phenylacetic acid |
| 2833 | 1,5-hexadien-3-ol Chem. Abst. 73, 44822f | B | p-(3-hexa-1,5-di-enylamino)phenyl-acetic acid |
| 2834 | 3,5-dimethyl-1,5-hexadien-3-ol Chem. Abst. 67, 53415d | B | p-[3-(3,5-dimethyl-hexa-1,5-dienyl)-amino]phenylacetic acid |
| 2835 | 2,6-dimethyl-1,6-heptadien-3-ol Chem. Abst. 73, 87490p | B | p-[3-(2,6-dimethyl-hepta-1,6-dienyl)-amino]phenylacetic acid |
| 2836 | E-2,6-dimethyl-1,6-octadien-3-ol Chem. Abst. 71, 61565a | B | p-[E-3-(2,6-di-methylocta-1,6-di-enyl)amino]phenyl-acetic acid |
| 2837 | Z-2,6-dimethyl-1,6-octadien-3-ol Chem. Abst. 71, 61565a | B | p-[Z-3-(2,6-di-methylocta-1,6-di-enyl)amino]phenyl-acetic acid |
| 2838 | 3-ethyl-7-methyl-1,6-octadien-3-ol Chem. Abst. 66, P76192x | B | p-[3-(3-ethyl-7-methylocta-1,6-di-enyl)amino]phenyl-acetic acid |
| 2839 | 3-t-butyl-7-methyl-1,6-octadien-3-ol Chem. Abst. 66, P76192x | B | p-[3-(3-1-butyl-7-methylocta-1,6-di-enyl)amino]phenyl-acetic acid |

| Example | Starting Material | Method | Product |
|---|---|---|---|
| 2840 | 7,9-dimethyl-1,6-decadien-3-ol Chem. Abst. 71, P60078c | B | p-[3-(7,9-dimethyl-deca-1,6-dienyl)-amino]phenylacetic acid |
| 2841 | 3,7-dimethyl-1,6-decadien-3-ol Chem. Abst. 67, 2688n | B | p-[3-(3,7-dimethyl-deca-1,6-dienyl)-amino]phenylacetic acid |
| 2842 | 2-methyl-1,5-heptadien-4-ol Chem. Abst. 67, 53415d | B | p-[4-(2-methylhepta-1,5-dienyl)amino]-phenylacetic acid |
| 2843 | 4,6-dimethyl-1,5-heptadien-4-ol Chem. Abst. 67, 53415d | B | p-[4-(4,6-dimethyl-hepta-1,5-dienyl)-amino]phenylacetic acid |
| 2844 | E,E-3,4,5-trimethyl-2,5-heptadien-4-ol Chem. Abst. 72, 2823f | B | p-[E,E-4-(3,4,5-tri-methylhepta-2,5-di-enyl)amino]phenyl-acetic acid |
| 2845 | E,Z-3,4,5-trimethyl-2,5-heptadien-4-ol Chem. Abst. 72, 2823f | B | p-[E,Z-4-(3,4,5-tri-methylhepta-2,5-di-enyl)amino]phenyl-acetic acid |
| 2846 | Z,Z-3,4,5-trimethyl-2,5-heptadien-4-ol Chem. Abst. 72, 2823f | B | p-[Z,Z-4-(3,4,5-tri-methylhepta-2,5-di-enyl)amino]phenyl-acetic acid |
| 2847 | 2-methyl-2,9-decadien-5-ol Chem. Abst. 73, 87254q | B | p-[5-(2-methyldeca-2,9-dienyl)amino]-phenylacetic acid |
| 2848 | 8-methyl-1,7-nona-dien-5-ol Chem. Abst. 73, 87253p | B | p-[5-(8-methylnona-1,7-dienyl)amino]-phenylacetic acid |
| 2849 | 3,4,7,7-tetrameth-yl-1,5-octadien-4-ol Chem. Abst. 67, 63514z | B | p-[5-(3,4,7,7-tetra-methylocta-1,5-dien-yl)-enyl)amino]phe-nylacetic acid |
| 2850 | 3,4-dimethylene-2-hexanol Chem. Abst. 69, 106850s | B | p-[2-(3,4-dimeth-ylenehexyl)amino)-phenylacetic acid |

| Example | Starting Material | Method | Product |
|---|---|---|---|
| 2851 | 3-methyl-2-methyl-ene-3-butene-1-ol Chem. Abst. 74, 42503z | B | p-[1-(3-methyl-2-methylenebut-3-enyl)amino]phenyl-acetic acid |
| 2852 | 2-methylene-3-buten-1-ol Chem. Abst. 73, P67478n | B | p-[1-(2-methylene-but-3-enyl)amino]-phenylacetic acid |
| 2853 | 3,3-dimethyl-2-methylene-4-penten-1-ol Chem. Abst. 74, 52962n | B | p-[1-(3,3-demethyl-2-methylenepent-4-enyl)amino]phenyl-acetic acid |
| 2854 | 2-methylene-3-meth-yl-4-hexen-1-ol Chem. Abst. 68 59021q | B | p-[1-(2-methylene-3-methylhex-4-enyl)-amino]phenylacetic acid |
| 2855 | 2,4-dimethyl-3-methylene-5-hexen-2-ol Chem. Abst. 72, 66299x | B | p-[2-(2,4-dimethyl-3-methylenehex-5-enyl)amino]-phenylacetic acid |
| 2856 | 2,4,4-trimethyl-3-methylene-5-hexen-2-ol Chem. Abst. 74, 52962n | B | p-[2-(2,4,4-tri-methyl-3-methylene-hex-5-enyl)amino]-phenylacetic acid |
| 2857 | E-2-methyl-3-methyl-ene-5-hepten-2-ol Chem. Abst. 72, 132999d | B | p-[E-2-(2-methyl-3-methylenehept-5-enyl)amino]phenyl-acetic acid |
| 2858 | Z-2-methyl-3-methyl-ene-5-hepten-2-ol Chem. Abst. 72, 132999d | B | p-[Z-2-(2-methyl-3-methylenehept-5-enyl)amino]phenyl-acetic acid |
| 2859 | 2-dimethyl-1,3-methylene-5-hepten-2-ol Chem. Abst. 72, 132999d | B | p-[2-(2,6-dimethyl-3-methylenehpet-5-enyl)amino]phenyl-acetic acid |
| 2860 | 3,7-dimethyl-2-methylene-6-octen-1-ol Chem. Abst. 68, 1114750d | B | p-[1-(3,7-dimethyl-2-methyleneoct-6-enyl)amino]-phenylacetic acid |

| Example | Starting Material | Method | Product |
| --- | --- | --- | --- |
| 2861 | 3,7-dimethyl-2-methylene-7-octen-1-ol Chem. Abst. 70, P37191j | B | p-[1-(3,7-dimethyl-2-methyleneoct-7-enyl)amino]-phenylacetic acid |
| 2862 | 3-isopropylidene-2,5-dimethyl-4-hex-en-2-ol Chem. Abst. 74, 53105x | B | p-[2-(3-isopropyl-idene-2,5-dimethyl-hex-4-enyl)amino]-phenylacetic acid |
| 2863 | 2-methyl-6-methyl-ene-7-octen-4-ol Chem. Abst. 69, 10548y | B | p-[4-(2-methyl-6-methyleneoct-7-enyl)amino]phenyl-acetic acid |
| 2864 | 2-isopropylidene-5-methyl-4-hexene-1-ol Chem. Abst. 72, 132999d | B | p-[1-(2-isopropyl-idene-5-methylhex-4-enyl)amino]phenyl-acetic acid |
| 2865 | 7-methyl-3-methyl-ene-6-octen-1-ol Chem. Abst. 73, 131140a | B | p-[1-(7-methyl-3-methyleneoct-6-enyl)amino]-phenylacetic acid |
| 2866 | 2-methyl-6-methyl-ene-7-octen-2-ol Chem. Abst. 71, P61600h | B | p-[2-(2-methyl-6-methyleneoct-6-enyl)amino]-phenylacetic acid |
| 2867 | E-2-ethylidene-6-methyl-5-hepten-1-ol Chem. Abst. 68, 114750d | B | p-[E-1-(2-ethyl-idene-6-methylhept-5-enyl)amino]phenyl-acetic acid |
| 2868 | Z-2-ethylidene-6-methyl-5-hepten-1-ol Chem. Abst. 68, 114750d | B | p-[Z-1-(2-ethyl-idene-6-methylhept-5-enyl)amino]phenyl-acetic acid |
| 2869 | 2,5-dimethyl-3-vinyl-4-hexen-2-ol Chem. Abst. 69, 45983p | B | p-[2-(2,5-dimethyl-3-vinylhex-4-enyl)-amino]phenylacetic acid |
| 2870 | 2-isopropenyl-5-methyl-4-hexen-1-ol Chem. Abst. 68, 111190k | B | p-[1-(2-isopropenyl-5-methylhex-4-enyl)-amino]phenylacetic acid |

| Example | Starting Material | Method | Product |
|---|---|---|---|
| 2871 | 2-vinyl-5-hepten-1-ol Chem. Abst. 75, P151673w | B | p-[1-(2-vinylhept-5-enyl)amino]phenylacetic acid |
| 2872 | 2-vinyl-6-hepten-1-ol Chem. Abst. 75, P15167w | B | p-[1-(2-vinylhept-6-enyl)amino]phenylacetic acid |
| 2873 | 2-(2-methylpropenyl)-5-hexen-1-ol Chem. Abst. 68, 114750d | B | p-[1-(2-methylpropenylhex-5-enyl)-amino]phenylacetic acid |
| 2874 | 7-methyl-3-vinyl-6-octen-1-ol Chem. Abst. 66, P115838j | B | p-[1-7-methyl-3-vinyloct-6-enyl)-amino]phenylacetic acid |
| 2875 | 2-allyl-4-methyl-4-penten-1-ol Chem. Abst. 72, 21731r | B | p-[1-2-allyl-4-methylpent-4-enyl)amino]phenylacetic acid |
| 2876 | 3-methyl-5-undecen-1-yn-3-ol Chem. Abst. 71, P101399h | B | p-[3-(3-methylundec-5-en-1-ynyl)amino]-phenylacetic acid |
| 2877 | 3,4,8-trimethyl-8-nonen-1-yn-3-ol Chem. Abst. 68, 29184r | B | p-[3-(3,4,8-trimethylnon-8-en-1-ynylamino]phenylacetic acid |
| 2878 | 1-dodecen-4-yn-3-ol Chem. Abst. 66, 75603v | B | p-(3-dodec-1-en-4-ynylamino)phenylacetic acid |
| 2879 | 11-dedecen-1-yn-3-ol Chem. Abst. 73, 120015n | B | p-(3-dodec-11-en-1-ynylamino)phenylacetic acid |
| 2880 | 1-undecen-5-yn-4-ol Chem. Abst. 69, 2432r | B | p-(4-undec-1-en-5-ynylamino)phenylacetic acid |
| 2881 | 3,7-dimethyl-6-nonen-1-yn-3-ol Chem. Abst. 71, P91265v | B | p-[3-(3,7-dimethylnon-6-en-1-ynyl)-amino]phenylacetic acid |

| Example | Starting Material | Method | Product |
|---|---|---|---|
| 2882 | 7,7-dimethyl-1-non-en-8-yn-5-ol Chem. Abst. 74, 22939t | B | p-[5-(7,7-dimethyl-non-1-en-8-ynyl)-amino]phenylacetic acid |
| 2883 | 2,3-dimethyl-1-non-en-4-yn-3-ol Chem. Abst. 68, 39161g | B | p-[3-(2,5-dimethyl-non-1-en-4-ynyl)-amino]phenylacetic acid |
| 2884 | 3,7-dimethyl-7-octen-1-yn-3-ol Chem. Abst. 73, P7095a | B | p-[3-(3,7-dimethyl-oct-7-en-1-ynyl)-amino]phenylacetic acid |
| 2885 | 4,6-dimethyl-5-hept-en-1-yn-4-ol Chem. Abst. 66, 95156e | B | p-[3-(4,6-dimethyl-hept-5-en-1-ynyl)-amino]phenylacetic acid |
| 2886 | 1-penten-4-yn-3-ol Chem. Abst. 74, P140935m | B | p-(3-pent-1-en-4-ynylamino)phenyl-acetic acid |

## EXAMPLE 2887

### Preparation of esters

Treament of the acids of Examples 2570-2886 and 2890-2927 with trifluoracetic anhydride to provide the N-$COCF_3$ derivative, followed by treatment with thionyl chloride to provide the N-$COCF_3$ acid chloride, followed by treatment with one of the following alcohols, followed by removal of the N-$COCF_3$ group with IN sodium hydroxide solution at 20° provides the corresponding ester of the starting acid.

Alcohols: methanol, ethanol, 2-methoxyethanol, butanol, pentanol, cyclopentanol, cyclohexanol, 1,2-propanediol, 1,3-propanediol, ethylene glycol, glycerol, glycidol, methyl glycolate, ethyl glycolate, glycolic acid, 2-hyroxy-propionic acid, 3-hydroxybutyric acid, 4-hydroxybutyric acid, glyceric acid, 3-diethylamino-1-propanol, 1-diethylamino-2-propanol, 3-dimethylamino-1-propanol, 2-diisopropyl-

aminoethanol, 3-(4-methyl-1-piperazino)-1,2-propanediol, 3-methoxy-1,2-propanediol, N-piperidineethanol, N,N-diethylethanolamine, benzyl alcohol, p-fluorobenzyl alcohol, p-bromobenzyl alcohol, p-chlorobenzyl alcohol, p-methoxybenzyl-alcohol, m-chlorobenzyl alcohol, p-carboxybenzyl alcohol, phenol, p-fluorophenol, p bromophenol, p-chlorophenol p-methoxyphenol, p-carboxyphenol, 4-cyanophenol, 3-hydroxypyridine, 2-chloro-3-hydroxypyridine, 5-carboxy-3-hydroxypyridine.

## EXAMPLE 2888

### Preparation of p-[1-(2,3-methano)octylamino]phenylacetic acid

To a stirred mixture of 5 g. of zinc-copper couple prepared by the Shank-Shechter Method [R. S. Shank and H. Schehter, J. Org. Chem., 24, 1825 (1959)], 0.02 g. of iodine, and 100 ml. of anhydrous ether is added 8.7 g. of (0.05 mol) of methylene iodide. The mixture is heated in the absence of atmospheric moisture until a spontaneous reaction begins as evidenced by continued refluxing of the ether when the heat source is removed. Upon completion of the exothermic reaction, the mixture is refluxed for 30 minutes, the heat is removed and to this mixture is added a solution of 5.4 g. (0.04 mol) of cis-2-octenol [L. P. Paquette and R. F. Eizember, J. Am. Chem. Soc., 91, 7110 (1969)] in 10 ml. of anhydrous ether at a rate sufficient to maintain constant reflux. When the addition is complete, the mixture is refluxed for 3 hours. The flask is cooled and the mixture is filtered and the filtrate is washed with cold dilute hydrochloric acid and saturated sodium bicarbonate, dried and evaporated to give Z-1-hydroxymethyl-2-n-pentylcyclopropane.

Preparation of the tosylate by the method of Example 2571, followed by condensation with ethyl p-aminophenylacetate by the method of Example 2571, followed by saponification by the method of Example 2571 provides p-[1-(2,3-methano)octylamino]phenylacetic acid.

EXAMPLE 2889

Treatment of the indicated olefin of the Table LXVI with Zinc and diiodomethane by the method of Example 2888 to produce the corresponding cyclopropyl compound followed by treatment of the halide with ethyl p-aminophenylacetate by the method of Example 2570, followed by saponification of the resulting ester by the method of Example 2570, is productive of the p-(methanoalkyl)aminophenylacetic acid of Table LXVI (Method C).

Treatment of the olefinic alcohols of Table LXVI with Zinc and diiodomethane by the method of Example 2888 will produce the corresponding cyclopropyl alkanols which upon treatment with methanesulfonic anhydride (Method of Example 2571) will produce the corresponding methanesulfonate ester which upon treatment with ethyl p-aminophenylacetate by the procedure of Example 2571 followed by saponification will produce the p-(methanoalkyl)aminophenylacetic acids of Table LXVI (Method D).

TABLE LXVI

| Example | Starting Material | Method | Product |
|---|---|---|---|
| 2890 | 3-bromo-3-isopropyl-4-methyl-1-pentene Chem. Abst. 54, 4355a | C | p-[3-(3-isopropyl-4-methyl-1,2-methanopentyl)amino]-phenylacetic acid |
| 2891 | 4-bromo-2-heptene Chem. Abst. 70, 67482x | C | p-[4-(2,3-methanoheptyl)amino]phenylacetic acid |
| 2892 | 4-bromo-2,4-dimethyl-2-hexene Chem. Abst. 70, 3169t | C | p-[4-(2,4-dimethyl-2,3-methanohexylamino]phenylacetic acid |
| 2893 | 5-chloro-3,5-dimethyl-3-heptene Chem. Abst. 54, 1256e | C | p-[5-(3,5-dimethyl-3,4-methanoheptyl)-amino]phenylacetic acid |
| 2894 | Z-1-hydroxy-2-hexadecene Ref. B | D | p-[Z-1-(2,3-methanohexadecyl)amino]-phenylacetic acid |

| Example | Starting Material | Method | Product |
| --- | --- | --- | --- |
| 2895 | E-1-hydroxy-2-hexadecene Ref. B | D | p-[E-1-(2,3-methanohexadecyl)amino]-phenylacetic acid |
| 2896 | 1-bromo-4-methyl-3-heptene Chem. Abst. 71, 102020q | C | p-[1-(4-methyl-3,-4-methanoheptyl)-amino]phenylacetic acid |
| 2897 | 1-bromo-4-methyl-3-heptene Chem. Abst. 71, P101399h | C | p-[1-(4-methyl-3,4-methanononyl)amino]-phenylacetic acid |
| 2898 | E-7-bromo-3-heptene Chem. Abst. 74, 99419 f | C | p-[E-1-(4,5-methanoheptyl)amino]phenylacetic acid |
| 2899 | 1-bromo-5,9-dimethyl-4-decene Chem. Abst. 51, 8699g | C | p-[1-(5,9-dimethyl-4,5-methanodecyl)-amino]phenylacetic acid |
| 2900 | 1-methanesulfonyloxy-4-tetradecene Ref. B | C | p-[1-(4,5-methanotetradecyl)amino]-phenylacetic acid |
| 2901 | 1-methanesulfonyloxy-4-hexadecene Ref. B | C | p-[1-(4,5-methanohexadecyl)amino]-phenylacetic acid |
| 2902 | 6-bromo-1-hexene Chem. Abst. 66, 2142j | C | p-[1-(5,6-methanohexyl)amino]phenylacetic acid |
| 2903 | 6-bromo-2-methyl-1-hexene Chem. Abst. 75, 109624f | C | p-[1-(5,6-methano-5-methylhexyl)amino]-phenylacetic acid |
| 2904 | 6-chloro-1-heptene Chem. Abst. 72, 31877g | C | p-[2-(6,7-methanoheptyl)amino]phenylacetic acid |
| 2905 | 6-bromo-2-methyl-2-heptene Chem. Abst. 54, 13166f | C | p-[2-(5,6-methano-6-methylheptyl)-amino]phenylacetic acid |
| 2906 | 7-chloro-2-octene Chem. Abst. 75, 129245m | C | p-[2-(6,7,-methanooctyl)amino]phenylacetic acid |

| Example | Starting Material | Method | Product |
|---|---|---|---|
| 2907 | E-1-chloro-4-nonene Chem. Abst. 67, 32294y | C | p-[E-1-(4,5-methanononyl)amino]phenylacetic acid |
| 2908 | 7-bromo-1-heptene | C | p-[1-(6,7-methanoheptyl)amino]phenylacetic acid |
| 2909 | 7-chloro-1-octene Chem. Abst,75, 129245m | C | p-[2-(7,8-methanooctyl)amino]phenylacetic acid |
| 2910 | 6-bromo-6-methyl-1-heptene Chem. Abst. 66, 94482w | C | p-[1-(2-methyl-6,7-methanoheptyl)amino]-phenylacetic acid |
| 2911 | 6-chloro-2-methyl-1-heptene Chem. Abst. 75, 129245 | C | p-[1-(6-methyl-6,7-methanoheptyl)amino]-phenylacetic acid |
| 2912 | E-8-bromo-2-octene Chem. Abst. 74, 99419f | C | p-[1-(6,7-methanooctyl)phenylacetic acid |
| 2913 | 8-bromo-2,6-dimethyl-2-octene Chem. Abst. 72, 90573c | C | p-[1-(3,7-demethyl-6,7-methanooctyl)-amino]phenylacetic acid |
| 2914 | 11-bromo-5-undecene Chem. Abst. 67, 73101b | C | p-[1-(6,7-methanoundecyl)amino]phenylacetic acid |
| 2915 | 8-bromo-1-octene Chem. Abst. 70, 10990g | C | p-[1-(7,8-methanooctyl)amino]phenylacetic acid |
| 2916 | R-8-iodo-7-methyl-1-octene Chem. Abst. 74, 12573e | C | p-[1-(2-methyl-7,8-methanooctyl)amino]-phenylacetic acid |
| 2917 | 1-chloro-7-tetradecene Chem. Abst. 54, 22461h | C | p-[1-(7,8-methanotetradecyl)amino]-phenylacetic acid |
| 2918 | 9-chloro-1-nonene Chem. Abst. 70, 114490k | C | p-[1-(8,9-methanononyl)amino]phenylacetic acid |

| Example | Starting Material | Method | Product |
|---|---|---|---|
| 2919 | 1-bromo-8-heptadecene Chem. Abst. 52, 249d | C | p-1-(8,9-methanoheptadecyl)amino]-phenylacetic acid |
| 2920 | E-1-bromo-9-octadecene Chem. Abst. 70, 47799j | C | p-E-1-(9,10-methanooctadecyl)amino]-phenylacetic acid |
| 2921 | Z-1-bromo-9-octadecene Chem. Abst. 70, 46779j | C | p-Z-1-(9,10-methanooctadecyl)amino]-phenylacetic acid |
| 2922 | 11-chloro-1-undecene Chem. Abst. 66, P19046d | C | p-1-(10,11-methanoundecyl)amino]-phenylacetic acid |
| 2923 | 12-iodo-3,7,11-trimethyl-1-dodecene | C | p-1-(2,6,10-trimethyl-11,12-methanododecyl)-amino]phenylacetic acid |
| 2924 | 13-bromo-1-tridecene Chem. Abst. 67, 43348v | C | p-1-(12,13-methanotridecyl)amino]-phenylacetic acid |
| 2925 | 22-bromo-9-docosene Chem. Abst. 73, 44976j | C | p-1-(13,14-methanodocosyl)amino]-phenylacetic acid |
| 2926 | 16-methanesulfonyloxy-1-hexadecene Ref. B | C | p-1-(15,16-methanohexadecyl)amino]-phenylacetic acid |
| 2927 | 3-chloro-2,4,4-trimethyl-1-pentene Chem. Abst. 72, 1110811 | C | p-3-(2,4,4-trimethyl]-1,2-methanopentyl)amino]phenylacetic acid |

## EXAMPLE 2928

### 2,3-Dihydroxypropyl 4-(4-pentadecenyl)aminophenylacetate

A solution of 7.35 g. of 4-(4-pentadecenyl)aminophenylacetic acid in 50 ml. of hexamethylphosphoramide is treated with 4.80 g. of 25% aqueous sodium hydroxide followed by 11.0 g. of 3-chloro-1,2-propanediol and then is heated at 140°C. for 6 hours. The mixture is diluted with water and ether and filtered to yield a white solid.

Recrystallization from acetonitrile and then from carbon tetrachloride affords the product as a white solid.

EXAMPLE 2929

2,3-Dihydroxypropyl 4-(4-tetradecenylamino)phenylbutyrate

A mixture of 2.25 g. of methyl 4-(4-tetradecenylamino)phenylbutyrate, 280 mg. of glycerol, and 1.37 g. of p-touluenesulfonic acid is heated at 180°C. for 18 hours and then is partitioned between ether and 3% aqueous sodium carbonate solution. The ether layer is separated, dried, and evaporated to yield the product as a white solid.

EXAMPLE 2930

2,3-Dihydroxypropyl 4-(4-tetradecenylamino)phenylpropionate

A solution of 11.8 g. of 4-(4-tetradecenylamino)-phenylpropionic acid, 1.00 g. of glycerol, and 5.35 ml. of boron trifluoride etherate in 200 ml. of toluene is stirred under reflux for 48 hours. The solution is treated with an additional 5.35 ml. of boron trifluoride etherate and refluxing is continued for 120 hours. Dilution with water and methylene chloride followed by filtration affords the product as a white solid.

EXAMPLE 2931

Preparation of 4-(allylamino)hydrocinnamic acid

A 4 g. sample of ethyl 4-(allylamino)hydrocinnamate is hydrolyzed with 1.6 g. 85% potassium hydroxide in 60 ml. 95% ethanol by refluxing the solution for 5 hours. The solution is cooled, diluted with 100 ml. water and acidified to pH 4.5 with 37% hydrochloric acid. The precipitate is collected, dried *in vacuo* and crystallized from acetone to yield the title compound as white powder.

EXAMPLE 2932

Preparation of 1-methanesulfonyloxyoctadec-9-ene

To a mixture of 250 ml. of dichloromethane, 25 g. 1-hydroxyoctadec-9-ene and 16.7 g. of triethylamine cooled in an ice-salt bath to -10°C. is added dropwise, over 15 minutes, 18.9 g. of methanesulfonyl chloride. The mixture

is cooled at -10°C to -15°C. for 30 minutes and then washed with 300 ml. each of cold water, 10% hydrochloric acid, sodium carbonate solution and with saturated sodium chloride solution. The organic layer is dried with magnesium sulfate and concentrated in vacuo to give a pale yellow oil.

EXAMPLE 2933

Preparation of ethyl 4-(1-pentadec-4-enylamino)phenylacetate

To a cold (-20°) stirred solution of 10.8 g. 1-hydroxypentadec-4-ene prepared by lithium aluminum hydride reduction of methyl tetradec-3-encarboxylate and 13.4 ml. triethylamine in 300 ml. ether is added dropwise 5.6 ml. methanesulfonyl chloride in 5 ml. of either. After addition is completed, the solution is warmed to room temperature, stirred for 30 minutes and filtered directly into a solution of 23.1 g. ethyl 4-aminophenyl acetate in 100 ml. ether. After 17 hours at room temperature, the precipitate is filtered and washed with several portions of methylene chloride. The organic solution is washed twice with 100 ml. water, 100 ml. brine, dried and evaporated. The tan residue is crystallized from ethanol and from acetonitrile to yield the title compound as white crystals.

EXAMPLE 2934

Preparation of ethyl 4-(1-pentadeca-4,14-dienylamino)-hydrocinnamate

A solution of 8.6 ethyl 4-aminohydrocinnamate, 9.77 g. 3,13-tetradecadien-1-carboxaldehyde and a few crystals of 2,4-dinitrobenzenesulfonic acid in 250 ml. toluene is refluxed under a Dean-Stark trap for 17 hours, whereupon the theoretical amount (0.8 ml.) water has been collected. The toluene is evaporated to yield ethyl 3-[4-(1-pentadeca-4,14-dienylideneaminophenyl]propionate as a crystalline mass.

To a mixture of 17.8 g. of the above compound in 250 ml. ethanol is added 1.68 g. sodium borohydride and the mixture is stirred at room temperature for 18 hours. Excess reagent is decomposed by addition of 10 ml. acetic acid. The solution is concentrated in vacuo and the residue

is partitioned betweed toluene and aqueous potassium carbonate. After drying, the toluene is evaporated to yield a solid. Crystallization from acetonitrile and from ethanol affords the title compound as white crystals.

EXAMPLE 2935

Preparation of ethyl 3-[4-(3,7,11-trimethyldodeca-2,6,10-tri- enylamino)phenyl]propionate

A mixture of 5.0 g. of ethyl 4-aminohydrocinnamate, 10.0 g. of 1-methanesulfonyloxy-3,7,11-triemethyldodeca-2,-6,-10-triene (prepared by the method of Example 363), 4.2 g. of anhydrous powdered potassium carbonate and 40 ml. hexamethylphosphoramide is heated to 80°C for 17 hours. The mixture is then ccoled, diluted with water and extracted with ethyl ether. The ether extracts are washed with water, dried and evaporated. The residue is recrystallized from ethanol yielding the title compound as white crystals.

EXAMPLE 2936

Preparation of ethyl 4-[1-(6-decylhept-6-enyl)amino]-cinnamate

A mixture of ethyl p-aminocinnamate, 5.9 g. 6-decylhept-6-enylbromide and one equivalent of anhydrous powdered potassium carbonate in 50 ml. hexamethylphosphoramide is heated for 20 hours at 60°C. The mixture is then cooled, diluted with water and extracted with ether. The combined ether extracts are dried, filtered and evaporated. Crystallization from acetonitrile provides the title compound as white crystals.

TABLE LXVI

The following 4-(substituted-amino)hydrocinnamates are prepared from the appropriate starting material by the method shown. Alcohols are converted to the corresponding mesylates by the method of Example 2932.

| Example No. | Method of Example | 4-(Substituted-amino)hydrocinnamate |
|---|---|---|
| 2937 | 2933 | Ethyl 4-(allylamino)hydrocinnamate |
| 2938 | 2936 | Ethyl 4-(1-octadec-9-enylamino)hydrocinnamate |

| Example No. | Method of Example | 4-(Substituted-amino)hydrocinnamate |
|---|---|---|
| 2939 | 2934 | Ethyl 4-(1-pentadec-4-enylamino)-hydrocinnamate |
| 2940 | 2935 | Ethyl 4-(1-tetradec-4-enylamino)hydro-cinnamate |
| 2941 | 2936 | Ethyl 4-(1-pentadec-4,14-dienylamino)-hydrocinnamate |
| 2942 | 2933 | Ethyl 4-[1-(3,7,11-trimethyldodeca-2,-6,-10-trienyl)amino]hydrocinnamate |
| 2943 | 2933 | Ethyl 4-[1-(6-decylhept-6-enyl)amino]-hydrocinnamate |
| 2944 | 2931 | Ethyl 4-[4-(2,3-methanoheptyl)amino]-hydrocinnamate |

TABLE IV

The following 4-(substituted-amino)hydrocinnamic acids are prepared from the esters of Table III by the method of Example 2931.

| Example No. | 4-(Substituted-amino)hydrocinnamic acid |
|---|---|
| 2945 | 4-(Allylamino)hydrocinnamic acid |
| 2946 | 4-(1-Octadec-9-enylamino)hydrocinnamic acid |
| 2947 | 4-(1-Pentadec-4-enylamino)hydrocinnamic acid |
| 2948 | 4-(1-Tetradec-4-enylamino)hydrocinnamic acid |
| 2949 | 4-(1-Pentadec-4,14-dienylamino)hydrocinnamic acid |
| 2950 | 4-[1-(3,7,11-Trimethyldodeca-2,6,10-trienyl)-amino]hydrocinnamic acid |
| 2951 | 4-[1-(6-Decylhept-6-enyl)amino]hydrocinnamic acid |
| 2952 | 4-[4-(2,3-Methanoheptyl)amino]hydrocinnamic acid |

TABLE LXIX

The following 4-(substituted-amino)cinnamates are prepared from the appropriate starting materials by the

methods shown. Alcohols are converted to their corresponding mesylates by the method of Example 2932.

| Example No. | Method of Example | 4-(Substituted-amino)cinnamate |
|---|---|---|
| 2953 | 2933 | Ethyl 4-(allylamino)cinnamate |
| 2954 | 2936 | Ethyl 4-(1-octadec-9-enylamino)cinnamate |
| 2955 | 2934 | Ethyl 4-(1-pentadec-4-enylamino)-cinnamate |
| 2956 | 2935 | Ethyl 4-(1-tetradec-4-enylamino)-cinnamate |
| 2957 | 2936 | Ethyl 4-(1-pentadec-4,14-dienylamino)-cinnamate |
| 2958 | 2933 | Ethyl 4-[1-(3,7,11-trimethyldodeca-2,6,-10-trienyl)amino]cinnamate |
| 2959 | 2935 | Ethyl 4-[1-(6-decylhept-6-enyl)amino]-cinnamate |
| 2960 | 2933 | Ethyl 4-[4-(2,3-methanoheptyl)amino]-cinnamate |

TABLE LXIX

The following 4-(substituted-amino)cinnamic acids are prepared from the esters of Table LXIX by the method of Example 2931.

| Example No. | 4-(Substituted-amino)cinnamic acid |
|---|---|
| 2961 | 4-(Allylamino)cinnamic acid |
| 2962 | 4-(1-Octadec-9-enylamino)cinnamic acid |
| 2963 | 4-(1-Pentadec-4-enylamino)cinnamic acid |
| 2964 | 4-(1-Tetradec-4-enylamino)cinnamic acid |
| 2965 | 4-(1-Pentadec-4,14-dienylamino)cinnamic acid |
| 2966 | 4-[1-(3,7,11-Trimethyldodeca-2,6,10-trienyl-amino]cinnamic acid |
| 2967 | 4-[1-(6-Decylhept-6-enyl)amino]cinnamic acid |
| 2968 | 4-[4-(2,3-Methanoheptyl)amino]cinnamic acid |

TABLE LXXI

The following 4-(substituted-amino)phenylpropiolates are prepared from the appropriate starting materials by the methods shown. Alcohols are converted to their corresponding mesylates by the method of Example 2932.

| Example No. | Method of Example | 4-(Substituted-amino)phenylpropiolate esters |
|---|---|---|
| 2969 | 2934 | Ethyl 4-(allylamino)phenylpropiolate |
| 2970 | 2936 | Ethyl 4-(1-octadec-9-enylamino)phenylpropiolate |
| 2971 | 2936 | Ethyl 4-(1-pentadec-4-enylamino)phenylpropiolate |
| 2972 | 2935 | Ethyl 4-(1-tetradec-4-enylamino)-phenylpropiolate |
| 2973 | 2935 | Ethyl 4-(1-pentadeca-4,14-dienylamino)-phenylpropiolate |
| 2974 | 2933 | Ethyl 4-[1-(3,7,11-trimethyldodeca-2,-6,-10-trienyl)amino]phenylpropiolate |
| 2975 | 2933 | Ethyl 4-[1-(6-decylhept-6-enyl)amino]-phenylpropiolate |
| 2976 | 2934 | Ethyl 4-[4-(2,3-methanoheptyl)amino]-phenylpropiolate |

TABLE LXXII

The following 4-(substituted-amino)phenylpropiolic acids are prepared from the esters of Table LXXI by the method of Example 2931.

| Example No. | 4-(Substituted-amino)phenypropiolic acid |
|---|---|
| 2977 | 4-(Allylamino)phenylpropiolic acid |
| 2978 | 4-(1-Octadec-9-enylamino)phenylpropiolic acid |
| 2979 | 4-(1-Pentadec-4-enylamino)phenylpropiolic acid |
| 2980 | 4-(1-Tetradec-4-enylamino)phenylpropiolic acid |
| 2981 | 4-(1-Pentadeca-4,14-dienylamino)phenylpropiolic acid |

2982 4-[1-(3,7,11-Trimethyldodeca-2,6,10-trienyl)-amino]phenylpropiolic acid

2983 4-[1-(6-Decylhept-6-enyl)amino]phenylpropiolic acid

2984 4-[4-(2,3-Methanoheptyl)amino]phenylpropiolic acid

TABLE LXXIII

The following 4-(substituted-amino)phenylbutyrates are prepared from the appropriate mesylates by the method of Example 2935. The requisite mesylates are prepared by the method of Example 2932.

| Example No. | 4-(Substituted-amino)phenylbutyrate esters |
|---|---|
| 2985 | Ethyl 4-(allylamino)phenylbutyrate |
| 2986 | Ethyl 4-(1-octadec-9-enylamino)phenylbutyrate |
| 2987 | Ethyl 4-(1-pentadec-4-enylamino)phenylbutyrate |
| 2988 | Ethyl 4-(1-tetradec-4-enylamino)phenyl butyrate |
| 2989 | Ethyl 4-(1-pentadeca-4,14-dienylamino)-phenylbutyrate |
| 2990 | Ethyl 4-[1-(3,7,11-trimethyldodeca-2,6,10-trienyl)amino]phenylbutyrate |
| 2991 | Ethyl 4-[1-(6-decylhept-6-enyl)amino]-phenylbutyrate |
| 2992 | Ethyl 4-[4-(2,3-methanoheptyl)amino]-phenylbutyrate |

TABLE LXXIV

The following 4-(substituted-amino)phenylbutyric acids are prepared from the esters of Table LXXIII by the method of Example 2931.

| Example No. | 4-(Substituted-amino)phenybutyric acid |
|---|---|
| 2993 | 4-(Allylamino)phenylbutyric acid |
| 2994 | 4-(1-Octadec-9-enylamino)phenylbutyric acid |

| Example No. | 4-(Substituted-amino)phenybutyric acid |
|---|---|
| 2995 | 4-(1-Pentadec-4-enylamino)phenylbutyric acid |
| 2996 | 4-(1-Tetradec-4-enylamino)phenylbutyric acid |
| 2997 | 4-(1-Pentadeca-4,14-dienylamino)phenylbutyric acid |
| 2998 | 4-[1-(3,7,11-Trimethyldodeca-2,6,10-trienyl)-amino]phenylbutyric acid |
| 2999 | 4-[1-(6-Decylhept-6-enyl)amino]phenylbutyric acid |
| 3000 | 4-[4-(2,3-Methanoheptyl)amino]phenylbutyric acid |

EXAMPLE 3001

Preparation of 4-[4-(2,3-methanoheptyl)amino]acetophenone

*p*-Aminoacetophenone is heated with 5 g. 1-methane-sulfonyloxy-2,3-methanoheptane (prepared by the method of Example 2932) in 50 ml. hexamethylphosphoramide containing anhydrous potassium carbonate (1.9 g.) for 16 hours a 100°C. The solution is cooled to room temperature, filtered to remove solids, and the filtrate is diluted with cold water (50 ml.). The amber solid so obtained is collected and washed with water. Recrystallization from ethanol followed by dichloromethane provides 4-[4-(2,3-methano-heptyl)amino]acetophenone.

TABLE LXXV

The following 4-(substituted-amino)acetophenones are prepared by the method of Example 3001. The requisite mesylates are prepared by the method of Example 2932.

| Example No. | 4-(Substituted-amino)acetophenone |
|---|---|
| 3001 | 4-(Allylamino)acetophenone |
| 3002 | 4-(1-Octadec-9-enylamino)acetophenone |
| 3003 | 4-(1-Pentadec-4-enylamino)acetophenone |
| 3004 | 4-(1-Tetradec-4-enylamino)acetophenone |

3005 4-(1-Pentadeca-4,14-dienylamino)acetophenone

3006 4-[1-(3,7,11-Trimethyldodeca-2,6,10-trienyl)-amino]acetophenone

3007 4-[1-(6-Decylhept-6-enyl)amino]acetophenone

3008 4-[4-(2,3-Methanoheptyl)amino]acetophenone

EXAMPLE 3009

Preparation of sodium 4-(allylamino)phenylacetate

A mixture of 3.62 g. of 4-(allylamino)phenylacetic acid and 25 ml. of ethanol water (9:1) containing 0.400 g. of sodium hydroxide is stirred for 4 hours. The mixture is filtered and the residue washed with 10 ml. of ethanol-water (9:1) and dried in vacuo for 24 hours to yield sodium 4-(allylamino)phenylacetate as a white solid.

EXAMPLE 3010

Preparation of 4-(1-octadec-9-enylamino)phenylacetyl chloride

A cold solution of 25 g. 4-(1-octadec-9-enylamino)-phenylacetic acid in 500 ml. dimethoxyethane methylene chloride (4:1) is prepared and dry hydrochloric acid is bubbled through the solution until no more precipitate forms. The solution is treated with 25 ml. thionyl chloride and refluxed until all of the precipitate has dissolved. The solvents are evaporated to yield the acid chloride hydrochloride as an orange, semi-crystalline mass.

EXAMPLE 3011

Preparation of 4-(N-trifluoroacetyl-allylamino)-phenylacetyl chloride

A stirred ice-cold suspension of 9 g. 4-(allylamino)phenylacetic acid in 100 ml. of dimethoxyethane and 16 ml. of pyridine is treated with 18 ml. of trifluoroacetic anhydride at 0°C. The solution is stirred for 30 minutes at room temperature and then diluted with 300 ml. ether and 100 g. ice. After stirring vigorously for 15 minutes, the phases are separated, the ether solution is washed with brine, dried and evaporated to a white, amorphous solid.

To a solution of 9.2 g. of the above solid in 30 ml. methylene chloride and 0.5 ml. dimethylformamide is added 5.7 ml. thionyl chloride. After 20 hours at reflux, the solvents are evaporated to yield 4-(N-trifluoroacetyl-allylamino)phenylacetyl chloride as a light yellow, mobile oil.

EXAMPLE 3012

Preparation of 4-(N-carbobenzyloxy-allylamino)-phenylacetyl chloride

To 15 g. 4-(allylamino)phenylacetic acid in 200 ml. warm chloroform is added a solution of 12 g. sodium carbonate in 150 ml. water. To the vigorously stirred solution is added 10 g. carbobenzyloxy chloride. After 2 hours stirring at 40°C., the layers are separated, washed three times with 1N hydrochloric acid, dried, and evaorated to an oil. The oil is dissolved in 300 ml. toluene, treated with 15 ml. thionyl chloride and the solution is refluxed for 5 hours. The solvents are evaporated and the residue is dissolved three times in toluene, evaporating each time ultimately to yield 4-(N-carbobenzyloxy-allylamino)phenylacetyl chloride as a viscous, orange oil.

EXAMPLE 3013

Preparation of 1-[4-(N-*tert*-butyloxycarbonyl)-1-tetradec-4-enylamino]phenylacetyl imidazole

A solution of 10 g. 4-(1-tetradec-4-enylamino)-phenylacetic acid in 100 ml. dioxane is treated with 4.0 g. *tert*-butylazidoformate and 10 ml. pyridine. After stirring at room temperature for 18 hours, the protected amidoacid is precipitated from solution by the addition of 150 ml. water. The solid is collected, thoroughly dried, and dissolved in 200 ml. of a mixture consisting of methylene chloride/dimethoxyethane/pyridine (1:4:1). This solution is stirred overnight at room temperature and the solvents are evaporated to yield 1-[4-(N-*tert*-butyloxycarbonyl)-tetradec-4-enyl-amino]phenylacetyl imidazole as an orange oil.

EXAMPLE 3014

Preparation of diethyl 4-(1-tetradec-4-enylamino)-benzoylmalonate

A solution of 26.6 g. of diethyl malonate and 10 ml. of 1,2-dimethoxyethane is added to a suspension of 4.0 g. of sodium hydride in 1,2-dimethoxyethane under argon. A solution of 17.3 g.of 4-(1-tetradec-4-enylamino)benzoyl chloride hydrochloride in 1,2-dimethoxyethane is then added. The reaction mixture is refluxed for 4.5 hours, cooled, poured on ice, acidified, and extracted with ether. The ether solution is washed with water and saturated sodium chloride solution, dried over anhydrous sodium sulfate and concentrated to dryness. Addition of a small amount of ethanol to the residue gives a solid which is filtered and discarded. The ethanol filtrate is concentrated and the residue is recrystallized from ether to yield diethyl 4-(1-tetradec-4-enylamino)benzoylmalonate.

EXAMPLE 3015

Preparation of tert-butyl ethyl 4-(1-pentadeca-4,14-dienyl-amino)benzoylmalonate

A solution of 28.0 g. of tert-butyl ethyl malonate in 10 ml. of 1,2-dimethoxyethane is added to a suspension of 4.0 g. of sodium hydride in 1,2-dimethoxyethane under argon. A solution of 17.3 g. of 4-(1-pentadeca-4,14-dienylamino) benzoyl chloride hydrochloride in 1,2-dimethoxyethane is then added. The reaction mixture is refluxed for 5 hours, cooled, poured on ice and extracted with ether. The ether solution is washed with water and saturated sodium chloride solution, dried over anhydrous sodium sulfate and concentrated to dryness. The residue is then recrystallized from ether to yield tert-butyl ethyl 4-(1-pentadeca-4,14-dienylamino)benzoylmalonate.

EXAMPLE 3016

Preparation of ethyl 2-[4-(1-allylamino)benzoyl-acetoacetate

A solution of 21.6 g. of ethyl acetoacetate and 10 ml. of 1,2-dimethoxyethane is added to a suspension of 4.0 g. of

sodium hydride in 1,2-dimethoxyethane under argon. A solution of 17.3 g. of 4-(1-allylamino)benzoyl chloride hydrochloride in 1,2-dimethoxyethane is then added. The reaction mixture is refluxed for 5 hours, cooled, poured on ice and extracted with ether. The ether solution is washed with water and saturated sodium chloride solution, dried over anhydrous sodium sulfate and concentrated to dryness. Recrystallization from ether affords ethyl 2-[4-(1-allylamino)benzoyl]acetoacetate as a white solid.

EXAMPLE 3017

Preparation of ethyl 4-(1-tetradec-4-enylamino)-benzoylacetate

A solution of 3.0 g. of tert-butyl ethyl 4-(1-tetradec-4-enylamino)benzoyl malonate 10 ml. of trifluoroacetic acid is warmed with stirring for 3 hours. The solution is poured onto ice and neutralized with potassium hydroxide. The resulting precipitate is collected by filtration, washed with water and dried. Recrystallization from chloroform affords ethyl 4-(1-tetradec-4-enylamino)-benzoylacetate.

EXAMPLE 3018

Preparation of 4-(1-tetradec-4-enylamino)benzoyl-acetic acid

Two grams of ethyl 4-(1-tetradec-4-enylamino)-benzoylacetate is added to a solution of potassium hydroxide in 50 ml. of 1:9 water-ethanol. The reaction mixture is stirred for 24 hours at room temperature. Careful neutralization with sulfuric acid gave a precipitate which is filtered, washed with water, and dried to yield 4-(1-tetradec-4-enylamino)benzoylacetic acid.

EXAMPLE 3019

Preparation of 4'-(1-allylamino)-2-(methylsulfinyl)-acetophenone

To a solution of 5.8 g. of dimethyl sulfoxide, dried over sieves, and 50 ml. of tetrahydrofuran is slowly added 28 ml. of n-butyllithium (2.4 M in hexane). To this mixture is added 10 g. of methyl 4-(1-allylamino)benzoate in

200 ml. of tetrahydrofuran. After two hours, the reaction mixture is poured onto ice, acidified with dilute hydrochloric acid and quickly extracted with chloroform. The chloroform extract is washed with water and saturated sodium chloride solution, and dried over anhydrous sodium sulfate. Concentration affords a solid which is washed with 500 ml. of hot hexane, filtered while hot and then washed with hexane. The white solid is dried in vacuo to yield 4'-(1-allylamino)-2-(methylsulfinyl)acetophenone

EXAMPLE 3020

Preparation of 4'-(1-allylamino)-2-(phenylsulfonyl)-acetophenone

A solution of 864 mg. of sodium hydride and 5.3 g. of methylphenylsulfone in 20 ml. of 1,2-dimethoxyethane is stirred at 6°C. for one hour under an atmosphere of argon. To this solution is added a solution of 5.0 g. of methyl (1-allylamino)benzoate in 50 ml. of tetrahydrofuran and the reaction mixture is stirred at 60°C. for 1.5 hours. The mixture is cooled, poured onto ice, acidified with dilute hydrochloric acid to pH 3 and then extracted with chloroform. The organic layer is separated, washed with water and saturated sodium chloride solution, dried over anhydrous sodium sulfate and then concentrated to dryness. The crude solid is chromatographed on silica gel, eluting with methylene chloride to yield 4'-(1-allylamino)-2-(phenylsulfonyl)-acetophenone.

EXAMPLE 3021

Preparation of 4'-(1-cyclopentylethylamino)-2-(phenylsulfinyl)acetophenone

To a solution of 6.2 g. of methylphenylsulfoxide, dried over sieves, and 50 ml. of tetrahydrofuran is slowly added 28 ml. of n-butyllithium (2.4 M in hexane). To this mixture is added 10 g. of a solution of methyl 4-(1-allylamino)benzoate in 200 ml. of tetrahydrofuran. After two hours, the reaction mixture is poured onto ice, acidified with diluted hydrochloric acid and quickly extracted with

chloroform. The chloroform layer is washed with water and saturated sodium chloride solution and dried over anhydrous sodium sulfate. Concentration affords a solid which is washed with 500 ml. of hot hexane, filtered while hot, and then washed with 50 ml. of hexane. The white solid is dried in vacuo yielding 4'-(1-allylamino)-2-(phenylsulfinyl)acetophenone.

EXAMPLE 3022

Preparation of 3-[4'-(1-allylamino)benzoyl]-2,4-pentanedione

A solution of 28.4 g. of 2,4-pentanedione and 20 ml. of 1,2-dimethoxyethane is added to a suspension of 13.6 g. of sodium hydride in 220 ml. of 1,2-dimethoxyethane under argon. A solution of 28.7 g. of 4-(1-allylamino)benzoyl chloride hydrochloride in 1,2-dimethoxyethane is then added. The reaction mixture is stirred at room temperature for 12 hours, cooled, poured onto ice and extracted with ether. The ether solution is washed with water and saturated sodium chloride solution, dried over anhydrous sodium sulfate and concentrated. The residue is then chromatographed over silica gel to yield 3-[4'-(1-allylamino)benzoyl]-2,4-pentanedione.

EXAMPLE 3023

Preparation of methyl 3-[4-(1-allylamino)benzoyl]propionate

A mixture of 35 g. of 3-(4-acetamidobenzoyl)propionic acid, 700 ml. of methanol and 1.4 ml. of concentrated sulfuric acid is refluxed for 76 hours. The solution is cooled to 35° C. and poured onto 7 g. of anhydrous sodium acetate while stirring. The reaction mixture is stirred in an ice-bath. The solid is collected and washed with cold methanol to yield 3-(4-aminobenzoyl)propionate as a white solid. A mixture of this solid, 9.2 g. of 1-allylbromide and 4.2 g of potassium carbonte is stirred for 20 hours at 125°C. under nitrogen. The mixture is then cooled to 25°C. and 30 ml. of water is added. After stirring, the product is filtered and washed with water. Recrystallization

from methanol affords methyl 3-[4-(1-allylamino)benzoyl]-propionate as a white solid.

EXAMPLE 3024

Preparation of 3-[4-(1-allylamino)benzoyl]propionic acid

A solution of 5.4 g. of methyl 3-[4-(1-allylamino)benzoyl]propionate is stirred with 5.4 g. of potassium hydroxide in 100 ml. of 95% ethanol for 3 hours at reflux. The reaction mixture is cooled, diluted with 50 ml. of ethanol and 100 ml. of water, neutralized with hydrochloric acid. The solution is cooled to room temperature and filtered. The white solid is washed with 50% aqueous ethanol and dried. The product is recrystallized from ethanol to yield 3-[4-(1-allylamino)benzoyl]propionic acid.

TABLE LXXVI

The following acetophenones are prepared by the noted methods from the carboxylic acids of Tables LXV or LXVI or appropriate derivatives thereof which are obtained by the methods of Examples 3010-3013.

| Example No. | Method of Example | 4-(Substituted-amino)acetophenones |
|---|---|---|
| 3025 | 3014 | Diethyl 4-[(1-tetradec-4-enyl)amino]-benzoylmalonate |
| 3026 | 3015 | tert-Butyl ethyl 4-(1-allylamino)-benzoylmalonate |
| 3027 | 3016 | Ethyl 2-[4-(1-pentadec-4,14-dienyl-amino)benzoyl]acetoacetate |
| 3028 | 3017 | Ethyl 4-[1-(3,7,11-trimethyldodeca-2,-6,-10-trienyl)amino]benzoylacetate |
| 3029 | 3018 | 4-(2,3-Methanoheptylamino]benzoyl-acetic acid |
| 3030 | 3019 | 4-[1-(6-Decylhept-6-enyl)amino]-2-(methylsulfonyl)acetophenone |

| Example No. | Method of Example | 4-(Substituted-amino)acetophenones |
|---|---|---|
| 3031 | 302 | 4'-(Allylamino)-2-(phenylsulfonyl)-acetophenone |
| 3032 | 302 | 4'-(Proparygylamino)-2-(phenyl-sulfinyl)acetophenone |
| 3033 | 302 | 3'-[4-(2,3-Methanoheptylamino)benzoyl]-2,4-pentanedione |
| 3034 | 302 | Methyl 3-[4-(allylamino)benzoyl]-propionate |
| 3035 | 302 | 3-[4-(1-Pentadec-4-enylamino)benzoyl]-propionic acid |

EXAMPLE 3036

Preparation of 4-[1-(2,3-methanoheptyl)amino]benzonitrile

4-Aminobenzonitrile (11.8g.) and 1-iodo-2,3-methanoheptane (16.3 g.) are dissolved in hexamethylphosphoramide (100 ml.) and heated under nitrogen in an oil bath maintained at 120°C. for 22 hours. The reaction mixture is cooled to room temperature and water (100 ml.) is added gradually. The mixture is then chilled in an ice bath. The precipitate separated is filtered, washed thoroughly with water, and dried. It is then washed repeatedly with hexane and dried. Recystallization from ether-hexane affords 4-[1-(2,3-methanoheptyl)amino]benzonitrile as pale yellow crystals.

EXAMPLE 3037

Preparation of 4-(allylamino)benzaldehyde

Di-isobutylaluminum hydride (54 ml., 25% solution in toluene) is added with stirring to a solution of 12.1 g. of 4-(allylamino)benzonitrile under a nitrogen atmosphere. After addition is completed, the solution is stirred for one hour. A solution of methanol in toluene (50 ml., 1:1) is added over 30 minutes and the mixture is poured into 500 ml. vigorously stirred ice-cold 50% aqueous sulfuric acid. The mixture is filtered and the organic layer separated. The aqueous solution is extracted twice with toulene (100 ml.)

and the combined organic layers are washed with aqueous sodium bicarbonate, dried over magnesium sulfate, decolorized with charcoal, filtered and evaporated <u>in vacuo</u> to give a light yellow crystalline solid. The product is recrystallized from dichloromethane/hexane giving colorless needles.

TABLE LXXVII

The following 4-(substituted-amino)benzonitriles are prepared by the method of Example 3036.

| Example No. | 4-(Substituted-amino)benzonitrile |
|---|---|
| 3038 | 4-(Allylamino)benzonitrile |
| 3039 | 4-(1-Octadec-9-enylamino)benzonitrile |
| 3040 | 4-(1-Pentadec-4-enylamino)benzonitrile |
| 3041 | 4-(1-Tetradec-4-enylamino)benzonitrile |
| 3042 | 4-(1-Pentadeca-4,14-dienylamino)benzonitrile |
| 3043 | 4-[1-(3,7,11-Trimethyldodeca-2,6,10-trienyl)-amino]benzonitrile |
| 3044 | 4-[1-(6-Decylhept-6-enyl)amino]benzonitrile |
| 3045 | 4-[4-(2,3-Methanoheptyl)amino]benzonitrile |

TABLE LXXVIII

The following 4-(substituted-amino)benzaldehydes are prepared from the corresponding benzonitriles of Table LXXVII by the method of Example 3037.

| No. | 4-(Substituted-amino)benzaldehydes |
|---|---|
| 3046 | 4-(Allylamino)benzaldehyde |
| 3047 | 4-(1-Octadec-9-enylamino)benzaldehyde |
| 3048 | 4-(1-Pentadec-4-enylamino)benzaldehyde |
| 3049 | 4-(1-Tetradec-4-enylamino)benzaldehyde |
| 3050 | 4-(1-Pentadeca-4,14-dienylamino)benzaldehyde |
| 3051 | 4-[1-(3,7,11-Trimethyldodeca-2,6,10-trienyl)-amino]benzaldehyde |

3052 4-[1-(6-Decylhept-6-enyl)amino]benzaldehyde

3053 4-[4-(2,3-Methanoheptyl)amino]benzaldehyde

EXAMPLE 3054

Preparation of 2,3-dihydroxypropyl 4-(allylamino)-phenylacetate

A solution of 7.34 g. of 4-(allylamino)phenylacetic acid, 4.80 g. of 25% aqueous sodium hydroxide, and 12.6 g. of 3-iodo-1,2-propanediol in 50 ml. of hexamethylphosphoramide is stirred for 24 hours at ambient temperature, diluted with 100 ml. of ether and stirred for 5 days at ambient temperature. The mixture is treated with water and extracted with ether. The dried extracts are evaporated to yield 2,3-dihydroxypropyl 4-(allylamino)-phenylacetate.

EXAMPLE 3055

Preparation of methyl 4-(allylamino)phenylacetate

A solution of 20.7 g. of 4-(allylamino)phenylacetic acid in 25 ml. of hexamethylphosphoramide is added to a stirred mixture of 0.800 g. of sodium hydride (57% in mineral oil) and 25 ml. of hexamethylphosphoramide. The solution which forms after one hour is treated with 11.0 g. of methyl iodide and is then stirred at 25°C. for 18 hours. Dilution with water followed by filtration affords a white solid which is crystallized from ethanol to yield methyl 4-(allylamino)phenylacetate

EXAMPLE 3056

Preparation of 3-hydroxypropyl 4-(allylamino)-phenylacetate

A mixture of 2.25 g. of methyl 4-(allylamino)phenylacetate, 280 mg. of 1,3-propanediol and 1.37 g. p-toluenesulfonic acid is heated at 180°C. for 18 hours and then is partitioned between ether and 3% aqueous sodium carbonate solution. The ether layer is separated, dried, and evaporated to yield 3-hydroxypropyl 4-(allylamino)phenylacetate.

EXAMPLE 3057

Preparation of 2-ethoxyethyl 4-(allylamino)phenylacetate

A solution of 11.8 g. of 4-(allylamino)phenylacetic acid, 1.00 g. of 2-ethoxyethanol and 5.35 ml. of boron trifluoride etherate in 200 ml. toluene is stirred under reflux for 48 hours. The solution is treated with an additional 5.35 ml. of boron trifluoride etherate and refluxing is continued for 120 hours. Dilution with water and methylene chloride followed by filtration affords 2-ethoxyethyl 4-(allylamino)phenylacetate.

EXAMPLE 3058

Preparation of methyl 4-(allylamino)hydrocinnamate

A solution of 50.5 g. of 4-(allylamino)hydrocinnamic acid and 34.4 ml. of boron trifluoride etherate in 200 ml. of methanol is stirred under reflux for 44 hours, allowed to cool, and poured into 1.20 liters of ice-cold 5% aqueous sodium carbonate solution. The white solid is collected by filtration and recrystallized from benzene-ethanol to yield methyl 4-(allylamino)hydrocinnamate.

EXAMPLE 3059

Preparation of 1-(methoxycarbonyl)propyl 4-(allylamino)-hydrocinnamate

To a solution of 10.0 g. 4-(allylamino)hydrocinnamoyl chloride hydrochloride in 200 ml. methylene chloride is added dropwise a solution of 3 g. methyl 2-hydroxybutyrate and 5 g. triethylamine in 100 ml. ether. After 17 hours stirring at room temperature, the precipitate is collected and washed with several portions of ether. The ether solution is washed with water, dried and evaporated to yield 1-(methoxycarbonyl)propyl 4-(allylamino)hydrocinnamate as a white solid.

EXAMPLE 3060

Preparation of 1-(ethoxycarbonyl)ethyl 4-(allylamino)-phenylacetate

To a warm mixture of 7 g. sodium 4-(allylamino)-phenylacetate in 100 ml. ethanol is added 4.7 g. ethyl 2-tosyloxypropionate. After 17 hours at reflux, the cooled

solution is diluted with an equal volume of water and the resultant precipitate is filtered. After washing with cold ethanol and drying, the product is crystallized from acetonitrile to yield 1-(ethoxycarbonyl)ethyl 4-(allylamino)phenylacetate as colorless crystals.

EXAMPLE 3061

Preparation of 1-carboxyethyl 4-(allylamino)phenylacetate

A flask containing 10.0 g. 4-(allylamino)phenylacetic acid, 3.3 g. lactic acid, 500 mg. toluenesulfonic acid and 500 ml. toluene equipped with a Soxhlet extractor charged with activated 4Å Linde molecular sieves. The solution is refluxed for 24 hours during which time the Soxhlet extractor is charged twice more with fresh sieves. The hot solution is filtered and left to cool, whereupon 1-carboxyethyl 4-(allylamino)phenylacetate separates as off-white crystals.

EXAMPLE 3062

Preparation of diethyl O-[4-(allylamino)phenylacetyl]-tartrate

A mixture of 4-[N-trifluoroacetyl-(allylamino)]-phenylacetyl chloride and 1.2 g. triethylamine in 100 ml. warm ether is treated with 2.5 g. diethyl tartrate and refluxed for 24 hours. The hot solution is filtered, the residue is washed with hot ether, and the solution is evaporated. After treatment with aqueous methanolic potassium carbonate, the product is precipitated by acidification, filtered, and dried. Crystallization from acetone yields diethyl O-[4-(allylamino)phenylacetyl]tartrate as a white, crystalline solid.

EXAMPLE 3063

Preparation of O-[4-(allylamino)phenylacetyl]malic acid

A warm solution of 4-[N-carbobenzyloxy-(allylamino]phenylacetyl chloride and 1.3 g. triethylamine in 100 ml. ether is treated with 2 g. malic acid. An immediate precipitate forms, but the mixture is refluxed for one hour and filtered while hot. The solid is washed several times with hot ether, then the ether is evaporated to yield a

white solid. The product is dissolved in tetrahydrofuran (100 ml.) and hydrogenated over 600 mg. 10% palladium-on-carbon at 50 psi until hydrogen uptake stops. The catalyst is filtered, and the solution is evaporated. The residue is crystallized from acetic acid to yield O-[4-(allylamino)-phenylacetyl]malic acid.

## EXAMPLE 3064

### Preparation of 2-(ethoxycarbonyl)vinyl 4-(allylamino)-phenylacetate

To a mixture containing 4.3 g. 1-[4-(N-*t*-butyloxycarbonyl-allylamino)phenylacetyl]imidazole 50 ml. 5*N* sodium hydroxide is added 3 g. ethyl 2-formyl acetate. The solution is vigorously stirred for 24 hours. The layers are separated, and the chloroform solution is washed once with 50 ml. 1*N* sodium hydroxide. The solvent is evaporated and the residue is heated for 30 minutes at 40°C. in 50 ml. anhydrous trifluoracetic acid. The solvent is again evaporated and the oil is crystallized from acetone to yield light yellow crystals of 2-(ethoxycarbonyl)vinyl 4-(allylamino)-phenylacetate.

## TABLE LXXIX

The following esters are prepared by the methods shown from the carboxylic acids of Tables LXV, LXVI, LXVIII, LXX, LXXII and LXXIV or appropriate derivatives thereof obtained by the methods of Examples 3009-3013.

| Example No. | Method of Example | Ester |
|---|---|---|
| 3045 | 3054 | 2,3-Dihydroxypropyl 4-(allylamino)-phenylacetate |
| 3066 | 3054 | 2,3-Dihydroxypropyl 4-(1-octadec-9-enylamino)hydrocinnamate |
| 3067 | 3054 | 2,3-Dihydroxypropyl 4-(1-pentadec-4-enylamino)cinnamate |
| 3068 | 3054 | 2,3-Dihydroxypropyl 4-(1-pentadec-4,14-dienylamino)phenylpropiolate |

TABLE LXXIX (cont'd)

| Example No. | Method of Example | Ester |
|---|---|---|
| 3069 | 3054 | 2,3-Dihydroxypropyl 4-[1-(3,7,11-trimethyldodeca-2,6,10-trienylamino]-butyrate |
| 3070 | 3055 | Methyl 4-[1-(6-decylhept-6-enyl)-amino]phenylacetate |
| 3071 | 3055 | Methyl 4-[4-(2,3,methanoheptyl)-amino]hydrocinnamate |
| 3072 | 3055 | Methyl 4-(1-pentadeca-4,14-dienyl-amino)cinnamate |
| 3073 | 3055 | Methyl 4-(1-octadec-9-enylamino)-phenylpropiolate |
| 3074 | 3055 | Methyl 4-[4-(allylamino)phenyl]-butyrate |
| 3075 | 3056 | 2-Hydroxypropyl 4-(allylamino)-phenylacetate |
| 3076 | 3056 | 4-Hydroxybutyl 4-[1-(6-decylhept-6-enyl)amino]hydrocinnamate |
| 3077 | 3056 | 2-Hydroxypropyl 4-[1-(3,7,11-trimethyldodeca-2,6,10-trienyl)amino]-cinnamate |
| 3078 | 3056 | 3-Hydroxypropyl 4-[4-(2,3-methano-heptyl)amino)]phenylpropiolate |
| 3079 | 3056 | 2-Hydroxyethyl 4-[1-(6-decylhept-6-enyl)amino]butyrate |
| 3080 | 3057 | 2-Methoxyethyl 4-(propargylamino)-phenylacetate |
| 3081 | 3057 | 2-Ethoxyethyl 4-(1-cyclopropyl-methylamino)propiolate |
| 3082 | 3058 | Methyl 4-(allylamino)hydrocinnamate |
| 3083 | 3058 | Methyl 4-(3-cyclopropylpropyl-amino)cinnamate |
| 3084 | 3059 | 1-Methoxycarbonylpropyl 4-(1-penta-dec-4,14-dienylamino)hydrocinnamate |

TABLE LXXIX (cont'd)

| Example No. | Method of Example | Ester |
|---|---|---|
| 3085 | 3059 | 1-Ethoxycarbonylpropyl 4-[1-(6-decylhept-6-enyl)amino]phenylpropiolate |
| 3086 | 3060 | 1-Ethoxycarbonylethyl 4-(1-pentadec-4-enylamino)phenylpropiolate |
| 3087 | 3061 | 1-Carboxyethyl 4-(1-octadec-9-enylamino)phenylacetate |
| 3088 | 3061 | 1-Carboxyethyl 4-[2-(2-methylcyclopropylethylamino]cinnamate |
| 3089 | 3061 | 1-Carboxybutyl 4-(allylamino)phenylpropiolate |
| 3090 | 3061 | 1-Carboxyethyl 4-[4-(1-pentadec-4-enylamino)phenyl]butyrate |
| 3091 | 3062 | O-[4-(1-pentadec-4,14-dienylamino)-phenylacetyl]tartrate |
| 3092 | 3063 | O-[4-(Allylamino)benzoyl]malic acid |
| 3093 | 3063 | O-[4-(4-cyclopropylmethylbenzoyl]-malic acid |
| 3094 | 3064 | 2-(Ethoxycarbonyl)vinyl 4-(but-2-enylamino)hydrocinnamate |
| 3095 | 3064 | 2-(Ethoxycarbonyl)vinyl 4-(allylamino)cinnamate |
| 3096 | 3064 | 2-(Ethoxycarbonyl)vinyl 4-(tetradec-4-amino)propiolate |
| 3097 | 3064 | 2-(Ethoxycarbonyl)vinyl 4-[4-(1-octadec-9-enylamino)butyrate |

0003663

CLAIMS:

1. A compound of the formula:

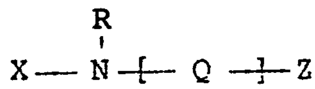 ....I

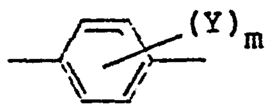

Q is
wherein Y is loweralkyl, loweralkoxy, hydroxy, nitro, acyloxy or halo, and m is an integer of from 0 to 4 inclusive, or a 5- or 6-membered heteroaryl ring containing 1 or more nitrogen, sulfur or oxygen atoms and which may be optionally substituted with one fluorine atom;
R is hydrogen or a group convertible _in vivo_ thereinto;
Z is:

a) $-\overset{O}{\overset{||}{C}}-J$ wherein J is selected from the group consisting of hydroxy, loweralkoxy, loweralkoxyloweralkyloxy, diloweralkylaminoloweralkoxy, hydrogen, loweralkyl, (mono- or polyhydroxy)loweralkoxy, (mono- or polycarboxy)loweralkoxy, (mono- or polycarboxy)hydroxyloweralkoxy, allyloxy, 2,3-epoxypropoxy, substitued or unsubstituted(phenoxy, benzyloxy and 3-pyridyloxy), pyridylmethoxy, tetrahydropyranyloxy, (mono- or polyhydroxy)loweralkylamino, (mono- or polycarboxy)loweralkylamino, (mono- or polycarboalkoxy)loweralkylamino, allylamino, propargylamino, 2-sulfoethylamino, (substituted or unsubstituted aroyl)amino, loweralkanoylamino, loweralkanesulfonylamino, (substituted or unsubstituted arene)sulfonylamino, loweralkanoylhydrazino, hydroxylamino, polymethyleneimino, (4-carboxy- or 4-carboethoxy) thiazolidino, and loweralkyl bearing one or more carboxy, carboloweralkoxy, carbamoyl, acyl, sulfinyl or sulfonyl groups, or

b) $-B-\overset{O}{\overset{||}{C}}-OK$ wherein B is a saturated or unsaturated loweralkylene group and K is selected from the group consist-

ing of hydrogen, loweralkyl, loweralkoxyethyl, diloweralkyl aminoethyl, (mono- or polyhydroxy)loweralkyl, (mono- or polycarboxy)loweralkyl, (mono- or polycarboxy)hydroxyloweralkyl, allyl, 2,3-epoxypropyl, substituted or unsubstituted (phenyl, benzyl or 3-pyridyl) or pyridylmethyl and tetrahydropyranyl; and wherein Q, Y, m and X are defined as follows:

Formula I-A) Q is 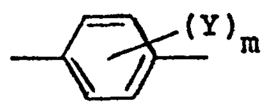 ; m is O; X is $S_a$-$T_a$- wherein $S_a$ is selected from the group consisting of $C_3$-$C_{16}$ cycloalkyl or $C_4$-$C_{17}$ cycloalkenyl and is either unsubstituted or substituted with at least one $C_1$-$C_{13}$ alkyl, $C_4$-$C_8$ cycloalkyl, decahydronaphthyl, methylene, ethylidene, or isopropylidene group; $T_a$ is a bond or a divalent radical selected from the group consisting of unbranched or branched $C_1$-$C_{13}$ alkylene or alkenyl-ene and is either unsubstituted or substituted with at least one $C_1$-$C_4$ alkyl group; with the proviso that the total number of carbon atoms in $S_a$ and $T_a$ shall not exceed twenty; and with the further proviso that when $T_a$ is not a bond, $S_a$ is not an unsubstituted cyclopropyl nor a cyclopropyl substituted with at least one $C_1$-$C_{13}$ alkyl;

Formula I-B) Q is 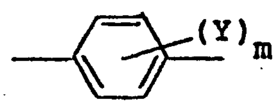 ; m is O; X is $S_b$- wherein $S_b$ is a branched or unbranched mono- or polyunsaturated or cyclopropylated $C_3$-$C_{22}$ alkyl group and can be represented by the formula:

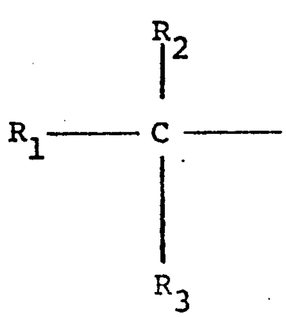

or

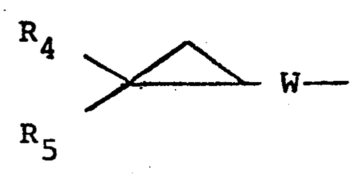

wherein $R_2$ and $R_3$ are the same or different and are hydrogen or a saturated or unsaturated $C_1$-$C_9$ alkyl group; $R_1$ is a:

Formula I-$B_a$) $C_2$ to $C_{21}$ E- or Z-alkenyl group unsubstituted or substituted with at least one loweralkyl group;

Formula I-$B_b$) $C_2$ to $C_{20}$ alkynyl group unsubstituted or substituted with at least one methyl or ethyl group;

Formula I-$B_c$) $C_4$ to $C_{20}$ alkyl group containing at least 2 non-cumulative double bonds, said group being unsubstituted or substituted with at least one methyl or ethyl group;

Formula I-$B_d$) $C_3$ to $C_{12}$ allenyl group unsubstituted or substituted with at least one methyl or ethyl group;

Formula I-$B_e$) vinyl or $C_3$ to $C_8$ E- or Z-alkenyl group said vinyl or alkenyl group being unsubstituted or substituted with at least one methyl group, and in this Formula I-$B_e$, $R_2$ is a vinyl or $C_3$ to $C_8$ E- or Z-alkenyl group unsubstituted or substituted with at least one methyl group;

Formula I-$B_f$) allyl or $C_4$ to $C_8$ E- or Z-alkenyl group said allyl or alkenyl group being substituted with at least one exo-(methylene, ethylidene, or isopropylidene), and either further unsubstituted or substituted with at least one methyl group;

Formula I-$B_g$) vinyl or $C_3$ to $C_8$ E- or Z-alkenyl group substituted between the nitrogen and the double bond with at least one loweralkenyl, said vinyl or alkenyl group being unsubstituted or substituted with at least one methyl group;

Formula I-$B_h$) vinyl or $C_3$ to $C_8$ E- or Z-alkenyl group said vinyl or alkenyl group being unsubstituted or substituted with at least one methyl and in this case, $R_2$

is a $C_2$ to $C_9$ alkynyl group unsubstituted or substituted with at least one methyl group;

Formula I-$B_i$) $C_4$ to $C_{20}$ alkyl group containing at least one carbon-carbon double bond and at least one carbon-carbon triple bond said group being unsubstituted or substituted with at least one loweralkyl group; or wherein $S_b$ is a;

Formula I-$B_j$) group of the formula 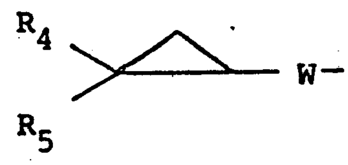

wherein $R_4$ is hydrogen or a $C_1$ to $C_{15}$ alkyl group unsubstituted or substituted with at least one methyl group, $R_5$ is hydrogen or methyl and W is a bond or a $C_1$ to $C_{15}$ branched or unbranched alkylene group unsubstituted or substituted with at least one methyl group;

Formula I-C) Q is 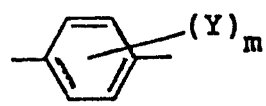 ; m is O; X is $S_c$-$T_c$- wherein $S_c$ is selected from the group consisting of mercapto, lowerakylthio, aralkylthio, arylthio, loweralkylsulinyl, loweralkylsulfonyl, aralkylsulfinyl, aralkylsulfonyl, arysulfinyl, arylsulfonyl, arylamino, acylamino, loweralkylsulfonylamino, arylsulfonylamino, azido, oxo, oximino, chloro, bromo, and iodo; wherein the substituent $S_c$ may contain q carbon atoms with q being an integer from 0 to 8, inclusive; $T_c$ is an unbranched or branched alkylene group, optionally saturated or (mono- or poly-)unsaturated and containing or not containing a cycloalkyl group, represented by the formula $C_nH_{2(n-p)}$ with n being an integer from 7 to 18, inclusive, and p being an integer from 0 to 5, inclusive, both being selected in a manner such that the sum of n and q is greater than 6 but less than 19; provided that when $S_c$ is halogen $T_c$ must be a saturated alkylene group; J may not be loweralkyl bearing one or more carboxy, carboloweralkoxy, carbamoyl, acyl, sulfinyl or sulfonyl

groups, and Z may not be 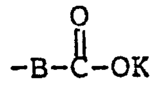 ;

Formula I-D) Q is 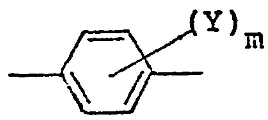 ; m is O; X is $S_d$-$T_d$- wherein $S_d$ is selected from the group consisting of substituted or unsubstituted aryl and heteroaryl groups; $T_d$ is a saturated or unsaturated alkylene group of 1-18 carbon atoms which may be branched or unbranched; such that the total number of carbon atoms in $S_d$ and $T_d$ shall not exceed 24; provided that J may not be hydroxyl, loweralkoxy, loweralkoxyloweralkyloxy, diloweralkylaminoloweralkoxy, or substituted or unsubstituted benzyloxy;

Formula I-E) Q is 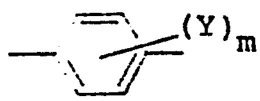 ; m is 1 to 4 inclusive; X is a saturated or unsaturated hydrocarbon radical of 7-19 carbon atoms which may be branched or unbranched and which may contain one or more radicals selected from the group consisting of saturated or unsaturated cycloalkyl, substituted or unsubstituted aryl and substituted or unsubstituted heteroaryl; Y is loweralkyl, loweralkoxy, hydroxy, nitro, acyloxy, or halo;

Formula I-F) Q is a 5- or 6-membered heteroaryl ring containing one or more nitrogen, sulfur, or oxygen atoms and optionally unsubstituted or substituted with one fluoro; X is $S_f$-$T_f$- wherein $S_f$ is selected from the group consisting of hydrogen, $C_3$-$C_8$ cycloalkyl, and substituted or unsubstituted aryl; $T_f$ is a divalent radical selected from the group consisting of branched or unbranched $C_6$-$C_{19}$ alkylene, alkenylene, and alkynylene;

Formula I-G) Q is 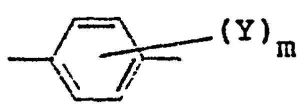 ; m is O; X is $S_g$-$T_g$- wherein $S_g$ is selected from the group consisting of carboxy, loweralkoxycarbonyl, aryloxycarbonyl,

heteroaryloxycarbonyl, carbamyl, mono- or diloweralkylcarbamyl, cyano, sulfo, monoalkyl- or monoarylsulfamyl; $T_g$ is an unbranched or branched alkylene group, optionally saturated or mono- or polyunsaturated, and containing or not containing a cycloalkyl group, represented by the formula $C_rH_{2(r-p)}$ with r being an integer from 1 to 18, inclusive, and p being an integer from 0 to 5, inclusive; with the proviso that when $S_g$ is a carbamyl, cyano, or sulfo group, $S_g$ and the nitrogen atom in Formula I may not be bonded to the same carbon atom of the group $T_g$;

Formula I-H) Q is 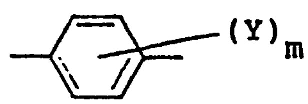 ; m is 0; X is a saturated or unsaturated hydrocarbon radical of 7-19 carbon atoms which may be branched or unbranched and which may contain a saturated or unsaturated cycloalkyl group, said radical containing one or more perfluorinated ($-CF_2$ or $-CF_3$) carbon atoms excluding the carbon adjacent to the nitrogen atom;
and the pharmacologically acceptable acid-addition and cationic salts thereof.

2. A compound of Formula I-A according to Claim 1, of the formula:

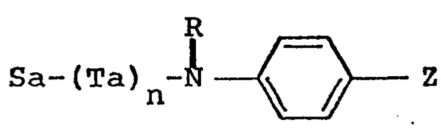

wherein Z is a moiety of the formula:

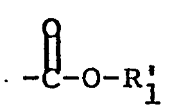

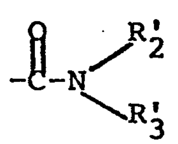

wherein $R_1'$ is selected from the group consisting of hydrogen, $C_1$-$C_6$ alkyl, loweralkoxyloweralkyl, (mono- or polyhydroxy)loweralkyl, allyl, 2,3-epoxypropyl, substituted or unsubstituted-(phenyl, benzyl, or 3-pyridyl), pyridylmethyl, mono- or polycarboxy(loweralkyl or hydroxyloweralkyl), and tetrahydropyranyl;

$R_2'$ is selected from the group consisting of hydrogen, carboxyloweralkyl, carboalkoxyloweralkyl, loweralkanoyl, loweralkanesulfonyl, benzenesulfonyl, sodium sulfo loweralkyl, sulfoloweralkyl, loweralkenyl, loweralkynyl, and phenylloweralkyl;

$R_3'$ is selected from the group consisting of loweralkyl, hydroxy, loweralkoxy, haloloweralkyl, phenyl, carboxyphenyl, chlorophenyl, sodium sulfophenyl, 3-pyridyl, pyridylloweralkyl, (mono- and polyhydroxy)loweralkyl, loweralkoxy loweralkyl, (di-loweralkyl)aminoloweralkyl, piperidino loweralkyl, pyrrolidino loweralkyl, amino, loweralkanoylamino, loweralkanesulfonylamino, N-piperidyl, benzenesulfonylamino, p-toluenesulfonylamino and 4-loweralkylpiperazino; $R_2'$ and $R_3'$ taken together with the associated nitrogen is selected from the group consisting of pyrrolidino, piperidino, morpholino, hexamethyleneimino, loweralkylpiperidino, 4-loweralkyl-1-piperazino, 4-phenylpiperazino, 3-pyrrolinyl, $\Delta^3$-piperidino, 4-(carboethoxy or carboxy)-3-thiazolidinyl, and 4-(carboethoxy)-3-oxazolidinyl;

R is selected from the group consisting of hydrogen, or a group convertible in vivo thereinto, such as methyl, carboxymethyl, acetyl, succinyl, 1-(sodium sulfo)loweralkyl, 1-(sodium sulfo)polyhydroxyalkyl and 1,3-bis-(sodium sulfo) aralkyl

n is either zero or one;

$T_a$ is a divalent radical selected from the group consisting of unbranched or branched $C_1$-$C_{13}$ alkylene or alkenylene and is either unsubstituted or substituted with at least one $C_1$-$C_4$ alkyl group;

and $S_a$ is selected from the group consisting of $C_3$-$C_{16}$ cycloalkyl or $C_4$-$C_{17}$ cycloalkenyl and is either unsubstituted or substituted with at least one $C_1$-$C_{13}$ alkyl, $C_4$-$C_8$ cycloalkyl, decahydronaphthyl, methylene, ethylidene, or isopropylidine group;

with the proviso that the total number of carbon atoms in $S_a$ and $T_a$ shall not exceed twenty; and with the further proviso that when n is 1, $S_a$ is not an unsubstituted cyclopropyl nor a cyclopropyl substituted with at least one $C_1$-$C_{13}$ alkyl;

and the pharmaceutically acceptably non-toxic acid-addition and cationic salts thereof.

3. A compound according to Claim 2, wherein n is 1, $T_a$ is a divalent radical selected from the group consisting of branched or unbranched $C_1$-$C_{13}$ alkylene or alkenylene and is either unsubstituted or substituted with at least one $C_1$-$C_4$ alkyl; $S_a$ is a moiety selected from the group consisting of $C_3$-$C_8$ cycloalkyl which is either unsubstituted or substituted with at least one $C_1$-$C_{13}$ alkyl, a $C_5$-$C_7$ cycloalkyl, or a decahydronaphthyl group; with the proviso that D is not an unsubstituted cyclopropyl nor a cyclopropyl substituted with at least one $C_1$ to $C_{13}$ alkyl.

4. A compound according to Claim 2, wherein n is 1, Ta is a divalent radical selected from the group consisting of branched or unbranched $C_1$-$C_{13}$ alkylene or alkenylene; and is either unsubstituted or substituted with at least one $C_1$-$C_2$ alkyl; and $S_a$ is a moiety selected from the group consisting of $C_4$-$C_9$ cycloalkenyl and is either unsubstituted or substituted with at least one $C_1$-$C_{13}$ alkyl group; and $C_5$-$C_8$ cycloalkyl unsubstituted or substituted with at least one methylene moiety, and/or at least one $C_1$-$C_{13}$ alkyl.

5. A compound according to Claim 2, wherein n is 0, $S_a$ is a moiety selected from the group consisting of $C_4$-$C_7$ cycloalkyl and is either unsubstituted or substituted with at least one $C_4$-$C_7$ cycloalkyl, and decahydronaphthalene unsubstituted or substituted with at least one $C_1$ to $C_4$ alkyl.

6. A compound according to Claim 2, wherein n is 0, and $S_a$ is selected from the group consisting of $C_4$-$C_{16}$

cycloalkyl substituted with at least one $C_1$-$C_5$ alkyl.

7. A compound according to Claim 2, wherein n is 0 and $S_a$ is a moiety selected from the group consisting of $C_4$-$C_{17}$ cycloalkenyl which is either unsubstituted or substituted with at least one $C_1$- $C_4$ alkyl, and $C_4$-$C_{10}$ cycloalkyl substituted with a moiety selected from the group consisting of methylene, ethylidene, and isopropylidene and/or at least one $C_1$-$C_4$.

8. A compound according to Claim 2, wherein Z is

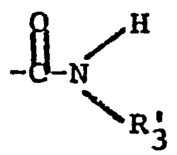 and 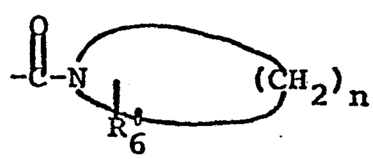

wherein $R'_3$ is a $C_2$-$C_4$ alkyl group unsubstituted or substituted with at least one hydroxyl group, $-CH_2-CH-CH_2$, $-CH_2-C-CH$, $-CH_2CH_2SO_3H$, $-(CH_2)m-COOR'_7$ wherein m is 2-4 and $R'_7$ is hydrogen or a $C_1$-$C_3$ alkyl group, $-\overset{O}{C}-R'_8$ wherein $R_8$ is phenyl or a $C_1$-$C_3$ alkyl group, $-SO_2R'_9$ wherein $R_9$ is a $C_1$-$C_3$ alkyl or phenyl or p-tolyl group, -OH, $OCH_3$ and $OCH_2CH_3$, $NHSO_2-R'_8$ wherein $R'_8$ is a previously defined, and wherein n is one of the integers, 4, 5 and 6 and $R'_6$ is hydrogen or represents at least one methyl group.

9. A compound of formula I-C according to Claim 1 of the formula:

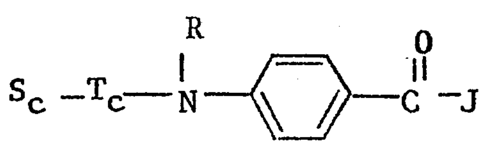

wherein R is hydrogen, methyl, ethyl, carboxymethyl, acetyl, trifluoroacetyl, succinyl, 1-(sodium sulfo)loweralkyl, 1-(sodium sulfo)polyhydroxyalkyl, or 1,3-<u>bis</u>(sodium sulfo) aralkyl; $S_c$ is methylthio, *n*-butylthio, benzylthio, *p*-chlorobenzylsulfinyl, *p*-chlorophenylamino, phenylamino, acetamido,

benzamido, methanesulfonamino, p-toluenesulfonamido;

$T_c$ is octamethylene, undecamethylene, tetradecamethylene, hexadecamethylene, 3-methylheptamethylene, 1,1,6-trimethylheptamethylene, 1-(n-butyl)pentamethylene, 1-ethyl-1-methylpentamethylene, 2,7-dimethyloctamethylene, 1-(n-octyl)decamethylene, 2-undecenylene, 1-(2-butenyl)decamethylene, 1-(1-methyl-2-propenyl)decamethylene, 1-(n-hexyl)-3-decenylene, 7-hexadecenylene, 9-hexadecenylene, 1-(n-butyl)-3-dodecenylene, 12-hexadecenylene, 3,7,7-trimethyl-2-heptenylene, 1-(n-propyl)-2-heptenylene, 2-decenylene, 9-methyl-2-nonenylene, 1-(n-pentyl)-2-undecynylene, 1-ethyl-2-tridecynylene, 1-(n-hexyl)-4-decynylene, 4-hexadecynylene, 2,6,6-trimethyl-1,3-cyclohexylenemethylene, 2,2,4-trimethyl-1,3-cyclopentylenemethylene; and

J is methoxy, isopropoxy, 2-ethoxyethoxy, 2-dimethylaminoethoxy, 1-methyl-4-piperidyloxy, 4-pyridylmethoxy, 2,3-dihydroxypropoxy, 2-hydroxypropoxy, 3-hydroxypropoxy, 4-chlorobenzyloxy, 3-methylbenzyloxy, 4-fluorophenoxy, 4-sulfophenoxy, 2,6-dichlorophenoxy, 3-carboxyphenoxy, 2,6-dimethyl-3-pyridyloxy, 6-methoxy-3-pyridyloxy, 2-hydroxy-3-pyridyloxy, 5-carboxy-3-pyridyloxy, carboxymethoxy, 1-methoxycarbonylpropoxy, 2-methoxycarbonyl-2-propoxy, 2,3-dihydroxypropylamino, carboxymethylamino, acetylamino, benzoylamino, 4-chlorobenzylamino, methanesulfonylamino, p-toluenesulfonylamino, 1-piperidyl.

10. A compound of the formula I-D as defined in Claim 1 wherein moiety $S_d$ is phenyl, 4-methylphenyl, 3-methylphenyl, 4-decylphenyl, 3,4-dimethylphenyl, 4-methoxyphenyl, 4-hexylphenyl, 4-tridecyloxyphenyl, 4-chlorophenyl, 4-fluorophenyl, 2-chloro-6-fluorophenyl, pentafluorophenyl, 1-napthyl, 4-biphenylyl, 2-furyl, 2-thienyl, 2-butyl-4-thiazolyl, 2-imidazolyl, 2-undecyl-r-imidazolyl, 5-ethyl-2-furyl, 4-propyl-2-oxazolyl, or 5-methyl-2-thienyl; $T_d$ is methylene, ethylene, hexamethylene, undecamethylene, hexadecamethylene, 3-methylheptamethylene, 2,7-dimethyloctamethylene, 9-decenylene, 4-hexenylene, 1-methyl-2-propenylene, 13-pentadecenylene, 18-methyl-18-octadecenylene, 6,8-nonadienylene, 10-undecenylene, 8-nonynylene, 16-heptadecynylene, 3-hexynylene, or 1-ethyl-2-tridecynylene; the group J is methoxy; isopropoxy; 2-ethoxyethoxy; 2-dimethylaminothoxy; 1-methyl-4-piperidyloxy; 4-pyridylmethoxy; 2,3-dihydroxypropoxy; 2-hydroxypropoxy; 3-hydroxypropoxy; 4-chlorobenzyloxy; 3-methylbenzyloxy; 4-sulfophenoxy; 4-fluorophenoxy; 2,6-dichlorophenoxy; 3-carboxyphenoxy; 2,6-dimethyl-3-pyridyloxy, 6-methoxy-3-pyridyloxy; 2-hydroxy-3-pyridyloxy; 5-carboxy-3-pyridyloxy; 4-cyano-3-pyridyloxy; carboxymethoxy; 1-methoxycarbonylpropoxy; 2-methoxycarbonyl-2-propyl; 2,3-dihydroxypropylamino; carboxymethylamino, acetylamino, benzoylamino, 4-chlorobenzoylamino; methanesulfonylamino; phenylsulfonylamino, 1-piperidyl, carboxymethyl; carboxyethyl; 2-carboethoxy-2-propyl; dicarboethoxymethyl; carboethoxyvinyl.

11. A compound of the formula I-D as defined in Claim 1 wherein the radical Z is 4-carboxybutyl; 2-carboethoxyethyl; 2-carboxyvinyl or 2-carboethoxyethynyl.

12. A compound of formula I-E as defined in Claim 1 wherein the substituent R is methyl, carboxymethyl, acetyl, succinyl, 1-(sodium sulfo)lower alkyl, 1-(sodium-sulfo) polyhydroxy alkyl, or 1,3-*bis*(sodium sulfo) aralkyl; the substituent X is *n*-octadecyl; 1-methyl-undecyl; 1-methylpentadecyl; 1-methylhexadecyl; 1-ethyltetradecyl; 2-methylundecyl; 2-methylpentadecyl; 6-methylhexadecyl; 14-methylpentadaceyl; 15-methylhexadecyl; 3,7,11-trimethyldodecyl; 3,7,11,15-tetramethylhexadecyl; 1-methyl-1-ethyltetradecyl; 1,1-dimethylundecyl; 1,1-dimethyltetradecyl;

13,13-dimethyltetradecyl; 15,15-dimethylhexadecyl; 10-undecenyl; 3,7-dimethyl-6-octenyl; 2,6,10-trimethyl-11-dodecenyl; 1-methyl-6-heptenyl; 1,1-diisopropyl-2-propenyl; 1,3-dimethyl-1-ethyl-2-pentenyl; 4,14-pentadecadienyl; 3,7,11-trimethyl-2,6,10-dodecatrienyl; 11-hexadecynyl; 6-methyl-6-hepten-2-ynyl; 1-isopropyl-1-methyl-2-heptynyl; 3-(1,3-dimethylcyclohexyl)-2-propyl; 13-cyclopentyltridecyl; 11-cyclohexylundecyl; cyclotetradecyl; 4-(1-cyclohexenyl)butyl; 4-cyclohexyl-2-butenyl; 11-phenylundecyl; 4-chlorobenzyl; 3-(4-fluorophenyl)propyl; 6-(4-methoxyphenyl)-hexyl; 4-(decyloxy)benzyl; 4-methylbenzyl; 3-(3-trifluoromethylphenyl)propyl; 3-fluoro-4-methylbenzyl; 3-(4-chloro-2-methoxyphenyl)propyl; cinnamyl; 4-(2-thienyl)butyl; 6-(2-furyl)hexyl; 3-(5-methyl-2-furyl)propyl; 3-(2,4-dichlorophenyl)propyl; 3-(4-benzyloxyphenyl)propyl; 2-(1-naphthyl)ethyl.

13. A compound of formula I-E as defined in Claim 1 wherein the group $(Y)_m$ is 2-methyl; 3-methyl; 3,5-dimethyl; 2-chloro; 3-chloro; 3,5-dichloro; 3,5-dibromo; 3,5-diiodo; 2-hydroxy; 3-hydroxy; 2-acetoxy; 2-methoxy; 3,5-dimethoxy; 3-bromo; 3-nitro, 2-fluoro; 3-fluoro; 2,5-difluoro; 2,6-difluoro; 3,5-difluoro; 2,3,5-trifluoro; 2,3,6-trifluoro; or 2,3,5,6-tetrafluoro.

14. A compound of the formula I-E as defined in any one of Claims 1, 11 or 12 wherein the group J is methoxy; isopropoxy; 2-ethoxy-ethoxy; 2-dimethylaminoethoxy; 1-methyl-4-piperidyloxy; 4-pyridylmethoxy; 2,3-dihydroxypropoxy; 2-hydroxypropoxy; 3-hydroxypropoxy; 4-chlorobenzyloxy; 3-methylbenzyloxy; 4-sulfophenoxy; 4-fluorophenoxy; 2,6-dichlorophenoxy; 3-carboxyphenoxy; 2,6-dimethyl-3-pyridyloxy; 6-methoxy-3-pyridyloxy; 2-hydroxy-3-pyridyloxy; 4-carboxy-3-pyridyloxy; 4-cyano-3-pyridyloxy; carboxymethoxy; 1-methoxycarbonylpropoxy; 2-methoxycarbonyl-2-propyl, 2-3-di-hydroxypropyl

amino; carboxymethylamino, acetylamino, benzoylamino, 4-chlorobenzoylamino; methanesulfonylamino; phenylsulfonylamino, 1-piperidyl; carboxymethyl; carboxyethyl; 2-carboethoxy-2-propyl; dicarboethoxymethyl; carboethoxyvinyl.

15. A compound of the formula I-E as defined in any one of claims 1, 11 or 12 wherein the radical Z is 4-carboxybutyl; 2-carboethoxyethyl; 2-carboxyvinyl or 2-carboethoxyethynyl.

16. A compound of the formula I-F according to Claim 1 wherein the substituent $S_f$ is hydrogen, cyclopropyl, cyclopentyl, cyclohexyl, phenyl, 4-chlorophenyl, 4-fluorophenyl, 3-methoxyphenyl, 4-methylphenyl, naphthyl; the moiety $T_f$ is, octamethylene, undecamethylene, tetradecamethylene, hexadecamethylene, 3-methylheptamethylene, 1,1,6-trimethylheptamethylene, 1-(n-butyl) pentamethylene, 1-ethyl-1-methylpentamethylene, 2,7-dimethyloctamethylene, 1-(n-octyl)decamethylene, 2-undecenylene, 1-(2-butenyl)decamethylene, 1-(1-methyl-2-propenyl)decamethene, 1-(n-hexyl)-3-decenylene, 7-hexadecenylene, 9-hexadecenylene, 1-(n-butyl)-3-dodecenylene, 12-hexadecenylene, 3,7,7-trimethyl-2-heptenylene, 1-(n-propyl)-2-heptenylene, 2-decenylene, 9-methyl-2-nonenylene, 1-(n-pentyl)-2-undecynylene, 1-ethyl-2-tridecynylene, 1-(n-hexyl)-4-decynylene, 4-hexadecynylene; the moiety Q is furan, pyrazole, pyridine, pyrimidine, oxazole, pyrrole, thiazole, thiophene; the substituent J is methoxy, isopropoxy, 2-ethoxyethoxy, 2-dimethylaminoethoxy, 1-methyl-4-pyperidylyloxy, 4-p ridylmethoxy, 2,3-dihydroxypropoxy, 2-hydroxypropoxy, 3-hydroxypropoxy, 4-chlorobenzyloxy, 3-methylbenzyloxy, 4-fluorophenoxy, 4-sulfophenoxy, 2,6-dichlorophenoxy, 3-carboxyphenoxy, 2,6-dimethyl-3-pyridyloxy, 6-methoxy-3-pyridyloxy, 2-hydroxy-3-pyridyloxy, 5-carboxy-3-pyridyloxy, carboxymethoxy,

1-methoxycarbonylpropoxy, 2-methoxycarbonyl-2-propoxy, 2,3-dihydroxypropylamino, carboxymethylamino, acetylamino, benzoylamino, 4-chlorobenzoylamino, methanesulfonylamino, p-toluenesulfonylamino, 1-piperidyl.

17. A compound according to Claim 15 wherein $S_f$ is hydrogen; Q is furan, pyrrole, or thiophene; and J is hydroxy.

18. A compound of the formula I-G as defined in Claim 1 wherein the substituent $S_g$ is, carbomethoxy, carboethoxy, carbo-*tert*-butoxy, carbophenoxy, carbo-p-chlorophenoxy, carbo-4-pyridylmethoxy, carbo-3-pyridyloxy, N-methylcarbamyl, N,N-diethylcarbamyl, N-methylsulfamyl, N-phenylsulfamyl, N-(p-toluene)sulfamyl; the moiety $T_g$ is octamethylene, undecamethylene, tetradecamethylene, hexadecamethylene, 3-methylhepamethylene; 1,1,6-trimethylheptamethylene, 1-(n-butyl)-pentamethylene, 1-ethyl-1-methylpentamethylene, 2,7-dimethyl-octamethylene, 1-(n-octyl)decamethylene, 2-undecenylene, 1-(2-butenyl)decamethylene, 1-(1-methyl-2-propenyl)decamethylene, 1-(n-hexyl)-3-decenylene, 7-hexadecenylene, 9-hexadecenylene, 1-(n-butyl)-3-dodecenylene, 12-hexadecenylene, 3,7,7-trimethyl-2-heptenylene, 1-(n-propyl)-2-heptenylene, 2-decenylene, 9-methyl-2-nonenylene, 1-(n-pentyl)-2-undecynylene, 1-ethyl-2-tridecynylene, 1-(n-hexyl)-4-decynylene, 4-hexadecynylene, 2,6,6-trimethyl-1,3-cyclohexylenemethylene, 2,2,4-trimethyl-1,3-cyclopentylenemethylene and the substituent J is, methoxy, isopropoxy, 2-ethoxyethoxy, 2-dimethylaminoethoxy, 1-methyl-4-piperidyloxy, 4-pyridylmethoxy, 2,3-dihydroxypropoxy, 2-hydroxypropoxy, 3-hydroxypropoxy, 4-chlorobenzyloxy, 3-methylbenzyloxy, 4-fluorophenoxy, 4-sulfophenoxy, 2,6-dichlorophenoxy, 3-carboxyphenoxy, 2,6-dimethyl-3-pyridyloxy, 6-methoxy-3-pyridyloxy, 2-hydroxy-3-pyridyloxy,

5-carboxy-3-pyridyloxy, carboxymethoxy, 1-methoxycarbonylpropoxy, 2-methoxycarbonyl-2-propoxy, 2,3-dihydroxypropylamino, carboxymethylamino, acetylamino, benzoylamino, 4-chlorobenzoylamino, methanesulfonylamino, p-toluenesulfonylamino, 1-piperidyl.

19. A compound of the formula I-H as defined in Claim 1, wherein the substituent X is 6-(trifluoromethyl)hexyl, 8-(trifluoromethyl)octyl, 10-(trifluoromethyl)decyl, 11-(trifluoromethyl)undecyl, 13-(trifluoromethyl)tridecyl, 15-(trifluoromethyl)pentadecyl, 17-(trifluoromethyl)heptadecyl, 1-(2,2,2-trifluoroethyl)decyl, 1-(2,2,2-trifluoroethyl)pentadecyl, 7-(trifluoromethyl)undecyl, 1-hexyl-11-(trifluoromethyl)undecyl, 3,7-dimethyl-7-(trifluoromethyl)octyl, 11-(pentafluoroethyl)undecyl, 16-(pentafluoroethyl)hexadecyl, 11-(heptafluoropropyl)undecyl, 11-(heptafluoroisopropyl)undecyl, 11-(nonafluorobutyl)undecyl, 11-(undecafluoropentyl)undecyl, 12-fluoro-12-(heptafluoropropyl)-12-(trifluoromethyl)dodecyl, 5-(pentadecafluoroheptyl)pentyl, 7-(pentadecafluoroheptyl)heptyl, 3,3-difluorotetradecyl, 6,6-difluorohexadecyl, 11,11-difluorododecyl, 14,14-difluoropentadecyl, 15,15-difluorohexadecyl, 16,16-difluoroheptadecyl, 11,11-difluoro-16-(trifluoromethyl)hexadecyl, (pentadecafluoroheptyl)methyl, (hentricontafluoropentadecyl)methyl, 11-(trifluoromethyl)-9-undecenyl, 15-(trifluoromethyl)-9-pentadecenyl, 5-(pentadecafluoroheptyl)-4-pentenyl, 6-(pentadecafluoroheptyl)-5-hexenyl, 15-(trifluoromethyl)-7-pentadecenyl, 15-(trifluoromethyl)-12-pentadecenyl, 15-(trifluoromethyl)-4-pentadecynyl, 1-hexyl-9-(trifluoromethyl)-4-

nonynyl, 3-(2,2,2-trifluoroethyl)-2,4,4-trimethylcyclopentyl, 3-(trifluoromethyl)-2,4,4-trimethylcyclohexyl; the group J is methoxy; isopropoxy; 2-ethoxyethoxy; 2-dimethylaminoethoxy; 1-methyl-4-piperidyloxy; 4-pyridylmethoxy; 2,3-dihydroxypropoxy; 2-hydroxypropoxy 3-hydroxypropoxy; 4-chlorobenzyloxy; 3-methylbenzyloxy; 4-sulfophenoxy; 4-fluorophenoxy; 2,6-di chlorophenoxy; 3-carboxyphenoxy; 2,6-dimethyl-3-pyridyloxy; 6-methoxy-3-pyridyloxy;2-hydroxy-3-pyridyloxy; 5-carboxy-3-pyridyloxy; 4-cyano-3-pyridyloxy; carboxymethoxy; 1-methoxy-carbonylpropoxy; 2-methoxycarbonyl-2-propyl, 2,3-dihydroxypropylamino; carboxymethylamino, acetylamino, benzoylamino, 4-chlorobenzoylamino; methanesulfonylamino; phenylsulfonylamino, 1-piperidyl, carboxymethyl; carboxyethyl; 2-carboethoxy-2-propyl; dicarboethoxymethyl; carboethoxyvinyl.

20. A compound of the formula I-H as defined in Claim 1 wherein the radical 2 is 4-carboxybutyl; 2-carboethoxyethyl; 2-carboxyvinyl or 2-carboethoxyethynyl.

21. An antiatherosclerotic composition useful for preventing or diminishing atherosclerotic lesion formation in mammals, comprising a compound according to any preceding claim and a pharmaceutically acceptable carrier or diluent.

22. A process for preparing a compound as defined in Claim 1 which comprises reacting a compound of the formula:

D-E

wherein D is X as defined above, or a group convertible into X;

E is halogen, alkanesulfonyloxy, arenesulfonyloxy, trifluoromethanesulfonyloxy, or $-\overset{O}{\overset{\|}{C}}-G$ wherein G is hydrogen, halogen, acyloxy, 1-imidazolyl, an activated ester moiety,

or a hydrocarbyl group provided that when G is a hydrocarbyl group the moieties D and G may in addition be directly bonded so that $D-\overset{O}{\overset{\|}{C}}-G$ forms a cyclic ketone, and further provided that $D-\overset{O}{\overset{\|}{C}}-G$ comprises the carbon skeleton of a desired X;
with a compound of the formula:

$$L-\!\left[\,Q\,\right]\!-M \qquad ....II$$

wherein Q is defined above;
L is -NHR, -NHV, -NXR or -NXV or a group convertible *in situ* thereinto wherein X and R are as defined above and V is an amine protecting group such as benzyl, trifluoroacetyl, carbo-*t*-butoxy, carbobenzyloxy, trialkylsilyl, and the like;
M is Z which is as defined above or a group convertible to Z; provided that when E is a bromine atom bonded to a saturated carbon atom and L is $-NH_2$ E and L may be interchanged, and further provided that when L is -NXR or -NXV then M is hydrogen and compound II is reacted in the presence of a suitable catalyst such as a Lewis acid, with a compound of the formula $J'-K'$ wherein $J'$ is a suitable leaving group such as halogen, sulfonyloxy, and the like, and $K'$ is Z or a group convertible thereto; and when required either during or after its formation, reducing an amide carbonyl or imino C=N group to produce the -NX group, and in any order or at anytime, before or after reacting compound II with $J'-K'$ or D-E, introducing or removing the protecting group V, or converting a specified M or X group to any other M or X; and/or forming the pharmaceutically acceptable acid-addition or cationic salt thereof.